# EUROPEAN PATENT SPECIFICATION

(11) **EP 2 593 594 B1**
(45) Date of publication and mention of the grant of the patent: **27.09.2017**
(21) Application number: 11807533.2
(22) Date of filing: 14.07.2011
(51) Int. Cl.: C07K 16/00

(54) **ANTIBODY LIBRARIES**
ANTIKÖRPERBIBLIOTHEKEN
BANQUES D'ANTICORPS

(30) Priority: 16.07.2010 US 365194 P
(43) Date of publication of application: 22.05.2013
(73) Proprietor: Adimab, LLC, Lebanon, NH 03766 (US)
(72) Inventor: VASQUEZ, Maximiliano, Palo Alto, CA 94303 (US); SIVASUBRAMANIAN, Arvind, Santa Clara, CA 95051 (US); FELDHAUS, Michael, Grantham, NH 03753 (US)
(74) Representative: Schiweck, Weinzierl & Koch Patentanwälte Partnerschaft mbB
(86) International application number: PCT/US2011/044063
(87) International publication number: WO 2012/009568

(56) References cited:
- WO-A1-2010/105256
- WO-A2-2009/036379
- US-A1- 2003 022 240
- US-A1- 2009 181 855
- US-A1- 2010 056 386

## Description

### BACKGROUND

Antibodies have profound relevance as research tools and in diagnostic and therapeutic applications. However, the identification of useful antibodies is difficult and once identified, antibodies often require considerable redesign or "humanization" before they are suitable for therapeutic applications in humans.

Many methods for identifying antibodies involve display of libraries of antibodies derived by amplification of nucleic acids from B cells or tissues. Some of these methods have utilized synthetic libraries.

However, many of these approaches have limitations. For example, most human antibody libraries known in the art contain only the antibody sequence diversity that can be experimentally captured or cloned from a biological source (*e.g*., B cells). Accordingly, such libraries may over-represent some sequences, while completely lacking or under-representing other sequences, particularly those binding human antigens. Most synthetic libraries known in the art have other limitations, such as the occurrence of unnatural (*i.e*., non-human) amino acid sequence motifs that have the potential to be immunogenic.

Accordingly, a need exists for diverse antibody libraries that contain candidate antibodies that are non-immunogenic (*i.e*., are human) and have desired properties (*e.g*., the ability to recognize a broad variety of antigens). However, obtaining such libraries requires balancing the competing objectives of generating diverse libraries while still maintaining the human character of the sequences within the library. The current invention provides antibody libraries that have these and other desirable features, and methods of making and using such libraries.

### SUMMARY

The invention provides, among other things, improvements in the design and production of synthetic libraries that mimic the diversity of the natural human repertoire of CDRH3, CDRL3, heavy chain, light chain, and/or full-length (intact) antibody sequences. In some embodiments the invention defines and provides methods of generating theoretical segment pools of TN1, DH, N2, and H3-JH segments to consider for inclusion in a physical manifestation of a library (*e.g*., polynucleotide or polypeptide) comprising or encoding CDRH3 sequences (*e.g*., an antibody library). In certain embodiments the invention defines and provides methods of matching the individual members of these theoretical segment pools to a reference set of CDRH3 sequences, to determine the frequency of occurrence (or segment usage weight) of each of the segments in the theoretical segment pool in the reference set. While any set of CDRH3 sequences may be used as a reference set, the invention also defines and provides methods of generating particular reference sets or subsets of interest. For example, among other things, the present invention provides methods for filtering an original reference set to obtain a provided reference set with a preimmune character. Also provided are methods to define and/or identify segments that occur within the CDRH3 sequences in the reference set but not in the theoretical segment pool. Such segments can be added to a theoretical segment pool, for example in order to be considered for inclusion in a physical library. Although the frequency of occurrence of a particular segment in a reference set is useful to select segments for inclusion in a physical library, the invention also provides a number of physicochemical and biological properties that can be used (alone or together with any other criterion or criteria) to select segments for inclusion in a physical library.

In some embodiments the invention provides libraries that differ from certain other libraries known in the art in that they are not sitewise-stochastic in composition or sequence, and are therefore intrinsically less random than these certain other libraries of the art (*see e.g.,* Example 14 of US Pub. No. 2009/0181855, incorporated by reference in its entirety, for a discussion of information content and randomness). In some embodiments, degenerate oligonucleotides may be used to further increase the diversity of the members of a library while further improving matching with a reference set of sequences (e.g., CDRH3, CDRL3, heavy chain, light chain, and/or full-length (intact) antibody sequences).

The invention also provides libraries whose members have sequences that are related to one anotherin that they would be selected for inclusion in a physical library by performing the analyses described herein, for example by generating a CDRH3 reference set as in Example 3; generating theoretical segment pools as in Examples 5-7; matching the members of a theoretical segment pool to the reference set as in Examples 4 and 8; and selecting members of the theoretical segment pool for inclusion in a physical library as in Examples 8-9. Also provided are methods of further increasing diversity in certain sequences by utilizing degenerate oligonucleotides as in Examples 12-16.

In some embodiments, the present invention provides polynucleotide and polypeptide libraries comprising CDRH3, CDRL3, heavy chain, light chain, and/or full-length (intact) antibody sequences, and methods of making and using such libraries.

In some embodiments, the invention provides libraries comprising, consisting essentially of, or consisting of any of the libraries or theoretical segment pools described herein.

In some embodiments, the present invention recognizes that by mimicking the *in vivo* activity of the enzyme TdT computationally, theoretical segment pools can be generated and subsequently matched to large reference datasets of CDR sequences to choose, for inclusion in a library, those theoretical segments that best recapitulate the CDR sequences in the reference data sets.

In certain embodiments, the invention provides libraries of polynucleotides comprising at least about 10⁴ polynucleotides encoding CDRH3 polypeptides with the structure: [TN1]-[DH]-[N2]-[H3-JH], wherein: TN1 is a polypeptide corresponding to any of the TN1 polypeptides of Tables 9-10 and 18-26, or a polypeptide produced by translation of any of the TN1 polynucleotides of Tables 25-26; DH is a polypeptide corresponding to any of the DH polypeptides of Tables 9, 11, 17-25 and 28, or a polypeptide produced by translation of any of the DH-encoding polynucleotides of Tables 16, 25 and 27; N2 is a polypeptide corresponding to any of the N2 polypeptides of Tables 9, 12, 18-25 and 30, or a polypeptide produced by translation of any of the N2-encoding polynucleotides of Tables 25 and 29; and H3-JH is a polypeptide corresponding to any of the H3-JH polypeptides of Tables 9, 13, 15, 18-25 and 32, or a polypeptide produced by translation of any of the H3-JH-encoding polynucleotides of Tables 14, 25 and 31.

In some embodiments, the invention provides libraries wherein at least about 1%, 5%, or 10% of the sequences in the library have the structure provided above, or that of any of the libraries provided herein.

In certain embodiments, the invention provides libraries comprising polynucleotides encoding CDRH3 polypeptides produced by the sets of TN1, DH, N2, and H3-JH polypeptides provided in any one of Tables 23-25.

In some embodiments, the invention provides libraries comprising polynucleotides encoding CDRH3 polypeptides produced by the set of TN1 polypeptides provided in Table 26, the set of DH polypeptides provided in Table 28, the set of N2 polypeptides provided in Table 30 and the set of H3-JH polypeptides provided in Table 32.

In certain embodiments, the invention provides libraries whose members show (or encode polypeptides that show) at least a certain percent identity with the polypeptides described above, for example, a library comprising at least about 10⁴ polynucleotides encoding CDRH3 polypeptides with the structure: [TN1]-[DH]-[N2]-[H3-JH], wherein: TN1 is a polypeptide at least about 80%, 90%, or 95% identical to any of the TN1 polypeptides of Tables 9-10 and 18-26, or a polypeptide at least about 80%, 90%, or 95% identical to a polypeptide produced by translation of any of the TN1 polynucleotides of Tables 25-26; DH is a polypeptide at least about 80%, 90%, or 95% identical to any of the DH polypeptides of Tables 9, 11, 17-25 and 28, or a polypeptide at least about 80%, 90%, or 95% identical to a polypeptide produced by translation of any of the DH-encoding polynucleotides of Tables 16, 25 and 27; N2 is a polypeptide at least about 80%, 90%, or 95% identical to any of the N2 polypeptides of Tables 9, 12, 18-25 and 30, or a polypeptide at least about 80%, 90%, or 95% identical to a polypeptide produced by translation of any of the N2-encoding polynucleotides of Tables 25 and 29; and H3-JH is a polypeptide at least about 80%, 90%, or 95% identical to any of the H3-JH polypeptides of Tables 9, 13, 15, 18-25 and 32, or a polypeptide at least about 80%, 90%, or 95% identical to a polypeptide produced by translation of any of the H3-JH-encoding polynucleotides of Tables 14, 25 and 31.

In some embodiments, the invention provides libraries comprising polynucleotides encoding light chain variable regions, wherein the light chain variable regions are selected from the group consisting of: (a) a VK1-05 sequence varied at one or more of positions 4, 49, and 46; (b) a VK1-12 sequence varied at one or more of positions 4, 49, 46, and 66; (c) a VK1-33 sequence varied at one or more of positions 4, 49, and 66; (d) a VK1-39 sequence varied at one or more of positions 4, 49, and 46; (e) a VK2-28 sequence varied at one or more of positions 2, 4, 46, and 49; (f) a VK3-11 sequence varied at one or more of positions 2, 4, 36, and 49; (g) a VK3-15 sequence varied at one or more of positions 2, 4, 48, and 49; (h) a VK3-20 sequence varied at one or more of positions 2, 4, 48, and 49; and/or (i) a VK4-1 sequence varied at one or more of positions 4, 46, 49, and 66.

In certain embodiments, the invention provides libraries comprising polynucleotides encoding light chain variable regions that comprise polypeptide sequences at least about 80%, 90%, or 95% identical to two or more of the light chain polypeptide sequences provided in Table 3.

In some embodiments, the invention provides libraries wherein the light chain variable regions comprise the polypeptide sequences provided in Table 3.

In certain embodiments, the invention provides libraries comprising polynucleotides encoding light chain variable regions, wherein the L3-VL polypeptide sequences of the light chain variable regions are varied at two or three residues between positions 89 to 94, inclusive, in comparison to an L3-VL germline sequence. In some embodiments, libraries containing a single light chain germline sequence and its variants are provided. In certain embodiments, variants produced from different light chain germline sequences can be combined to produce libraries encoding multiple light chain germline sequences and their variants. Any of the light chain L3-VL germline sequences provided herein may be varied at two or three residues between positions 89 to 94, inclusive, and one of ordinary skill in the art will recognize that any other L3-VL sequence can also be varied according to the principles described herein to produce libraries provided by the invention. In some embodiments, the present invention comprises libraries containing polynucleotides that encode antibody light chain variable regions, wherein the antibody light chain variable regions comprise one or more of the following L3-VL sequences: (i) an amino acid sequence that is identical to an L3-VL germline sequence (e.g., see Table 1); (ii) an amino acid sequence that contains two substitutions between residues 89-94, inclusive, in comparison to an L3-VL germline sequence; and (iii) an amino acid sequence that contains three substitutions between residues 89-94, inclusive, in comparison to an L3-VL germline sequence. In some embodiments, each antibody light chain variable region on a library includes one or more of the above L3-VL sequences. In some embodiments, such a library is combined with one or more sets of other nucleic acids that may or may not encode antibody light chain variable regions, and may or may not contain such L3-VL sequences. In some embodiments, the present invention comprises libraries containing polynucleotides that encode an antibody light chain variable region having an amino acid sequence as set forth in Table 4, or a polynucleotide sequence as set forth in one or more of Tables 5-7, wherein two or three residues at positions 89-94, inclusive, are varied.

In some embodiments, the present invention comprises libraries containing polynucleotides that encode an antibody light chain variable region, wherein, across the library, all encoded antibody light chain variable regions are identical to one another except for substitutions of residues at positions between residue 89 and residue 94, inclusive and further wherein, across the library, sequences of any two encoded antibody light chain variable regions differ from one another at not more than 3 positions.

In some embodiments, the invention provides libraries comprising polynucleotides encoding light chain variable regions comprising polypeptide sequences at least about 80%, 90%, or 95% identical to polypeptides produced by translation of two or more of the polynucleotide sequences provided in Tables 5-7. In certain embodiments all members of the library are at least about 80%, 90%, or 95% identical to polypeptides produced by translation of two or more of the polynucleotide sequences provided in Tables 5-7.

In certain embodiments, the invention provides a library comprising light chain variable regions that comprise the polypeptides produced by translation of the polynucleotide sequences provided in Tables 5-7. In certain embodiments, all members of the library comprise the polypeptides produced by translation of the polynucleotide sequences provided in Tables 5-7.

In some embodiments, any of the libraries described herein as containing or encoding CDRL3 and/or light chain variable regions, contains or encodes such CDRL3 and/or light chain variable regions in the context of complete light chains. Furthermore, in some embodiments, such libraries (and/or complete light chain libraries) further contain or encode one or more heavy chain CDRH3, variable domains, or intact heavy chains. In some embodiments, provided libraries include or encode intact antibodies such as, for example, intact intact IgGs.

In some embodiments, provided libraries include or encode human antibodies or antibody fragments; in some such embodiments, provided libraries include or encode intact human antibodies.

In certain embodiments, the invention provides libraries that comprise nucleic acid vectors containing library nucleic acids described herein. In many embodiments, each such library member comprises the same vector.

In some embodiments, the invention provides host cells containing one or more provided libraries, for example including a vector. In some embodiments the host cell is a yeast, and in certain embodiments the yeast is *Saccharomyces cerevisiae.*

In some embodiments, the invention provides antibodies isolated from the libraries described herein.

In certain embodiments, the invention provides kits containing any of the libraries described herein.

In some embodiments, the invention provides representations of libraries and/or theoretical segment pools in a computer readable format, for example, the TN1 polypeptides of Tables 10, 23-25 and 26; the DH polypeptides of Tables 11, 23-25 and 28; the N2 polypeptides of Tables 12,23-25 and 30; the H3-JH polypeptides of Tables 13, 15, 17,23-25 and 32; the TN1 polynucleotides of Tables 25-26; the DH polynucleotides of Tables 25 and 27; the N2 polynucleotides of Tables 25 and 29; and/or the H3-JH polynucleotides of Tables 25 and 31.

In certain embodiments, the invention provides a representation of the polynucleotide sequences of the Human Preimmune Set (Appendix A), or the polypeptide expression products thereof, in a computer readable format.

In some embodiments, the invention provides a method of making synthetic polynucleotides encoding a CDRH3 library, comprising: (a) providing a theoretical segment pool containing TN1, DH, N2, and H3-JH segments; (b) providing a reference set of CDRH3 sequences; (c) utilizing the theoretical segment pool of (a) to identify the closest match(es) to each CDRH3 sequence in the reference set of (b); (d) selecting segments from the theoretical segment pool for inclusion in a synthetic library; and (e) synthesizing the synthetic CDRH3 library. In certain embodiments, the invention provides libraries made by this method. In some embodiments, the segments selected for inclusion in the synthetic library are selected according to their segment usage weight in the reference set of CDRH3 sequences.

In certain embodiments, the invention provides a method of making synthetic polynucleotides encoding a CDRL3 library, comprising: (i) obtaining a reference set of light chain sequences, wherein the reference set contains light chain sequences with VL segments originating from the same IGVL germline gene and/or its allelic variants; (ii) determining which amino acids occur at each of the CDRL3 positions in the reference set that are encoded by the IGVL gene; (iii) synthesizing light chain variable domain encoding sequences wherein two positions between positions 89 and 94, inclusive, contain degenerate codons encoding two or more of the five most frequently occurring amino acid residues at the corresponding positions in the reference set; and (iv) synthesizing the polynucleotides encoding the CDRL3 library. In certain embodiments, the invention provides libraries made by this method.

In some embodiments, the invention provides a method of using any of the libraries of the invention to isolate an antibody binding an antigen, comprising contacting the polypeptide expression products of said libraries with an antigen and isolating polypeptide expression products that bind to the antigen.

In certain embodiments, the number of N-linked glycosylation sites, deamidation motifs, and/or Cys residues in the libraries of the invention are reduced or eliminated in comparison to libraries produced by amplification of a repertoire from a biological source.

The invention provides a number of polynucleotide and polypeptide sequences and segments that can be used to build larger polynucleotide and polypeptide sequences (e.g., TN1, DH, N2, and H3-JH segments that can be used to build CDRH3). One of ordinary skill in the art will readily recognize that in some instances these sequences can be more succinctly represented by providing consensus sequences after alignment of the sequences provided by the invention, and that these consensus sequences fall within the scope of the invention and may be used to more succinctly represent any of the sequences provided herein.

### BRIEF DESCRIPTION OF THE DRAWING

Figure 1 shows that Vernier residues 4 and 49 (starred) in VK1-39 have a diversity index comparable to or greater than the diversity indices of the CDR positions *(i.e.,* at or above 0.07, in this example).
Figure 2 shows that clinically validated CDRL3 sequences deviate little from germline-like sequences (n=35).
Figure 3 shows the percent of sequences in the jumping dimer CDRL3 libraries of the invention and a previous CDRL3 library, VK-v1.0, with *X* or fewer mutations from germline. Here, *FX* is the percentage of sequences in a library with *X* or fewer mutations from germline.
Figure 4 shows the application of a provided method used to generate nucleotide sequences (SEQ ID NOS 8748-8759, respectively, in order of appearance) encoding the parent H3-JH segments.
Figure 5 shows the general schematic of an approach used to select segments from a theoretical segment pool for inclusion in a theoretical and/or synthetic library.
Figure 6 shows the frequency of "Good" and "Poor" expressing CDRH3 sequences isolated from yeast-based libraries described in US 2009/0181855, and their comparison to the sequences contained in the library design described therein ("Design"), as a function of the DH segment hydrophobicity (increasing to the right).
Figure 7 shows the percentage of CDRH3 sequences in the LUA-141 library and Exemplary Library Design 3 (ELD-3) that match CDRH3 sequences from Lee-666 and Boyd-3000 with zero, one, two, three, or more than three amino acid mismatches.
Figure 8 shows that Exemplary Library Design 3 (ELD-3) and the Extended Diversity Library Design both return better matches to clinically relevant CDRH3 sequences than the LA-141 library.
Figure 9 shows that the combinatorial efficiency of Exemplary Library Design 3 (ELD-3) is greater than that of the LUA-141 library. Specifically, the ELD-3 segments are more likely to yield a unique CDRH3 than the LUA-141 library segments.
Figure 10 shows the amino acid compositions of the Kabat-CDRH3s of LUA-141, Exemplary Library Design 3 (ELD-3), and Human CDRH3 sequences from the HPS (Human H3).
Figure 11 shows the Kabat-CDRH3 length distribution of LUA-141, Exemplary Library Design 3 (ELD-3), and Human CDRH3 sequences from the HPS (Human H3).
Figure 12 shows the percentage of CDRH3 sequences in the Extended Diversity library that match CDRH3 sequences from Boyd *et al.* with zero to thirty-two amino acid mismatches
Figure 13 shows the Kabat-CDRH3 length distribution of Exemplary Library Design 3 ("ELD-3"), the Extended Diversity Library Design ("Extended Diversity") and human CDRH3 sequences from the Boyd *et al.* data set ("Boyd 2009").
Figure 14 shows the amino acid compositions of the Kabat-CDRH3s of the Extended Diversity Library Design ("Extended Diversity") and human CDRH3 sequences from the Boyd *et al.* dataset ("Boyd 2009").
Figure 15 shows the combinatorial efficiency of the Extended Diversity Library Design by matching 20,000 randomly selected sequences from the (same) design. About 65% of the sequences appear only once in the design and about 17% appear twice.

### DETAILED DESCRIPTION OF CERTAIN EMBODIMENTS OF THE INVENTION

The present invention provides, among other things, polynucleotide and polypeptide libraries, methods of producing and using the libraries, kits containing the libraries, and computer readable forms of representations of libraries and/or theoretical segment pools disclosed herein. Libraries taught in this application can be described, at least in part, in terms of components (*e.g*.., polynucleotide or polypeptide "segments") from which they are assembled. Among other things, the present invention specifically provides and contemplates these polynucleotide or polypeptide segments, methods of producing and using such segments, and kits and computer readable forms of representations that include such library segments.

In certain embodiments, the invention provides antibody libraries specifically designed based on sequences and CDR length distribution in a naturally occurring human antibody repertoire. It is estimated that, even in the absence of antigenic stimulation, an individual human makes at least about 10⁷ different antibody molecules (Boyd et al., Science Translational Medicine, 2009, 1: 1). The antigen-binding sites of many antibodies can cross-react with a variety of related but different epitopes. In addition, the human antibody repertoire is large enough to ensure that there is an antigen-binding site to fit almost any potential epitope, albeit potentially with low affinity.

The mammalian immune system has evolved unique genetic mechanisms that enable it to generate an almost unlimited number of different light and heavy chains in a remarkably economical way, by combinatorially joining chromosomally separated gene segments prior to transcription. Each type of immunoglobulin (Ig) chain (*i.e.,* kappa light, lambda light, and heavy) is synthesized by combinatorial assembly of DNA sequences, selected from two or more families of gene segments, to produce a single polypeptide chain. Specifically, the heavy chains and light chains each consist of a variable region and a constant (C) region. The variable regions of the heavy chains are encoded by DNA sequences assembled from three families of gene sequences: variable (IGHV), diversity (IGHD), and joining (IGHJ). The variable regions of light chains are encoded by DNA sequences assembled from two families of gene sequences for each of the kappa and lambda light chains: variable (IGLV) and joining (IGLJ). Each variable region (heavy and light) is also recombined with a constant region, to produce a full-length immunoglobulin chain.

While combinatorial assembly of the V, D and J gene segments make a substantial contribution to antibody variable region diversity, further diversity is introduced *in vivo,* at the pre-B cell stage, via imprecise joining of these gene segments and the introduction of non-templated nucleotides at the junctions between the gene segments (see *e.g.,* U.S. Pub. No. 2009/0181855, for more information).

After a B cell recognizes an antigen, it is induced to proliferate. During proliferation, the B cell receptor locus undergoes an extremely high rate of somatic mutation that is far greater than the normal rate ofgenomic mutation. The mutations that occur are primarily localized to the Ig variable regions and comprise substitutions, insertions and deletions. This somatic hypermutation enables the production of B cells that express antibodies possessing enhanced affinity toward an antigen. Such antigen-driven somatic hypermutation fine-tunes antibody responses to a given antigen.

Synthetic antibody libraries of the instant invention have the potential to recognize any antigen, including antigens of human origin. The ability to recognize antigens of human origin may not be present in other antibody libraries, such as antibody libraries prepared from human biological sources (*e.g*., from human cDNA), because self-reactive antibodies are removed by the donor's immune system via negative selection.

Still further, the present invention provides strategies that streamline and/or simplify certain aspects of library development and/or screening. For example, in some embodiments, the present invention permits use of cell sorting technologies (*e.g*., fluorescence activated cell sorting, FACS) to identify positive clones, and therefore bypasses or obviates the need for the standard and tedious methodology of generating a hybridoma library and supernatant screening.

Yet further, in some embodiments, the present invention provides libraries and/or sublibraries that accommodate multiple screening passes. For example, in some embodiments, provided libraries and/or sublibraries can be screened multiple times. In some such embodiments, individual provided libraries and/or sublibraries can be used to discover additional antibodies against many targets.

Before further description of the invention, certain terms are defined.

### Definitions

Unless defined otherwise, all technical and scientific terms used herein have the meaning commonly understood by one of ordinary skill in the art relevant to the invention. Unless otherwise specified, the Kabat numbering system is used throughout the application. The definitions below supplementthose in the art and are directed to the embodiments described in the current application.

The term "amino acid" or "amino acid residue," as would be understood by one of ordinary skill in the art, typically refers to an amino acid having its art recognized definition such as an amino acid selected from the group consisting of: alanine (Ala or A); arginine (Arg or R); asparagine (Asn or N); aspartic acid (Asp or D); cysteine (Cys or C); glutamine (Gln or Q); glutamic acid (Glu or E); glycine (Gly or G); histidine (His or H); isoleucine (Ile or I): leucine (Leu or L); lysine (Lys or K); methionine (Met or M); phenylalanine (Phe or F); proline (Pro or P); serine (Ser or S); threonine (Thr or T); tryptophan (Trp or W); tyrosine (Tyr or Y); and valine (Val or V), although modified, synthetic, or rare amino acids may be used as desired. Generally, amino acids can be grouped as having a non-polar side chain (*e.g*., Ala, Cys, Ile, Leu, Met, Phe, Pro, Val); a negatively charged side chain (*e.g.,* Asp, Glu); a positively charged side chain (*e.g.,* Arg, His, Lys); or an uncharged polar side chain (*e.g*., Asn, Cys, Gln, Gly, His, Met, Phe, Ser, Thr, Trp, and Tyr).

As would be understood by those of ordinary skill in the art, the term "antibody" is used herein in the broadest sense and specifically encompasses at least monoclonal antibodies, polyclonal antibodies, multi-specific antibodies (*e.g*., bispecific antibodies), chimeric antibodies, humanized antibodies, human antibodies, and antibody fragments. An antibody is a protein comprising one or more polypeptides substantially or partially encoded by immunoglobulin genes or fragments of immunoglobulin genes. The recognized immunoglobulin genes include the kappa, lambda, alpha, gamma, delta, epsilon and mu constant region genes, as well as myriad immunoglobulin variable region genes.

The term "antibody binding region" refers to one or more portions of an immunoglobulin or antibody variable region capable of binding an antigen(s). Typically, the antibody binding region is, for example, an antibody light chain (or variable region or one or more CDRs thereof), an antibody heavy chain (or variable region or one or more CDRs thereof), a heavy chain Fd region, a combined antibody light and heavy chain (or variable regions thereof) such as a Fab, F(ab')₂, single domain, or single chain antibodies (scFv), or any region of a full length antibody that recognizes an antigen, for example, an IgG *(e.g.,* an IgG1, IgG2, IgG3, or IgG4 subtype), IgA1, IgA2, IgD, IgE, or IgM antibody.

"Antibody fragments" comprise a portion of an intact antibody, for example, one or more portions of the antigen-binding region thereof. Examples of antibody fragments include Fab, Fab', F(ab')₂, and Fv fragments, diabodies, linear antibodies, single-chain antibodies, and multi-specific antibodies formed from intact antibodies and antibody fragments.

The term "antibody of interest" refers to an antibody that has a property of interest that is identified and/or isolated from a library of the invention. Exemplary properties of interest include, for example, but are not limited to, binding to a particular antigen or epitope, binding with a certain affinity, cross-reactivity, blocking a binding interaction between two molecules, and/or eliciting a certain biological effect.

The term "canonical structure," as understood by those of ordinary skill in the art, refers to the main chain conformation that is adopted by the antigen binding (CDR) loops. From comparative structural studies, it has been found that five of the six antigen binding loops have only a limited repertoire of available conformations. Each canonical structure can be characterized by the torsion angles of the polypeptide backbone. Correspondent loops between antibodies may, therefore, have very similar three dimensional structures, despite high amino acid sequence variability in most parts of the loops (Chothia and Lesk, J. Mol. Biol., 1987, 196: 901; Chothia et al., Nature, 1989, 342: 877; Martin and Thornton,J. Mol. Biol., 1996,263: 800). Furthermore, there is a relationship between the adopted loop structure and the amino acid sequences surrounding it. As is known in the art, the conformation of a particular canonical class is determined by the length of the loop and the amino acid residues residing at key positions within the loop, as well as within the conserved framework (*i.e.,* outside of the loop). Assignment to a particular canonical class can therefore be made based on the presence of these key amino acid residues. The term "canonical structure" may also include considerations as to the linear sequence of the antibody, for example, as catalogued by Kabat (Kabat et al., in "Sequences of Proteins of Immunological Interest," 5th Edition, U.S. Department of Heath and Human Services, 1992). The Kabat numbering scheme is a widely adopted standard for numbering the amino acid residues of an antibody variable domain in a consistent manner, and is used herein unless indicated otherwise. Additional structural considerations can also be used to determine the canonical structure of an antibody. For example, those differences not fully reflected by Kabat numbering can be described by the numbering system of Chothia *et al.* and/or revealed by other techniques, for example, crystallography and two or three-dimensional computational modeling. Accordingly, a given antibody sequence may be placed into a canonical class which allows for, among other things, identifying appropriate chassis sequences (*e.g*., based on a desire to include a variety of canonical structures in a library). Kabat numbering of antibody amino acid sequences and structural considerations as described by Chothia *et al*., and their implications for construing canonical aspects of antibody structure, are described in the literature.

The terms "CDR", and its plural "CDRs", refer to a complementarity determining region (CDR) of which three make up the binding character of a light chain variable region (CDRL1, CDRL2 and CDRL3) and three make up the binding character of a heavy chain variable region (CDRH1, CDRH2 and CDRH3). CDRs contribute to the functional activity of an antibody molecule and are separated by amino acid sequences that comprise framework regions. The exact definitional CDR boundaries and lengths are subject to different classification and numbering systems. CDRs may therefore be referred to by Kabat, Chothia, contact or other boundary definitions, including for example the CDRH3 numbering system described below. Despite differing boundaries, each of these systems has some degree of overlap in what constitutes the so called "hypervariable regions" within the variable region. CDR definitions according to these systems may therefore differ in length and boundary areas with respect to the adjacent framework region. See, for example Kabat et al., in "Sequences of Proteins of Immunological Interest," 5th Edition, U.S. Department of Health and Human Services, 1992; Chothia et al., J. Mol. Biol., 1987, 196: 901; and MacCallum et al., J. Mol. Biol., 1996, 262: 732. The "CDRH3 numbering system" used herein defines the first amino acid of CDRH3 as being at position 95 and the last amino acid of CDRH3 as position 102. Note that this is a custom numbering system that is not according to Kabat. The amino acid segment, beginning at position 95 is called "TN1" and, when present, is assigned numbers 95, 96, 96A, 96B, etc. Note that the nomenclature used in the current application is slightly different from that used in U.S. Publication Nos. 2009/0181855 and 2010/0056386, and WO/2009/036379. In those applications, position 95 was designated a "Tail" residue, while here, the Tail (T) has been combined with the N1 segment, to produce one segment, designated "TN1." The TN1 segment is followed by the "DH" segment, which is assigned numbers 97, 97A, 97B, 97C, etc. The DH segment is followed by the "N2" segment, which, when present, is numbered 98, 98A, 98B, etc. Finally, the most C-terminal amino acid residue of the "H3-JH" segment is designated as number 102. The residue directly before (N-terminal) it, when present, is 101, and the one before (if present) is 100. The rest of the H3-JH amino acids are numbered in reverse order, beginning with 99 for the amino acid just N-terminal to 100, 99A for the residue N-terminal to 99, and so forth for 99B, 99C, etc. Examples of CDRH3 sequence residue numbers may therefore include the following:

13 Amino Acid CDR-H3 with TN1 and N2
(95)(96)(96A)(97)(97A)(97B)(97C)(97D) (98) (99)(100)(101)(102)
|------------|-----------------------|----|------------------|
TN1 DH N2 H3-JH

10 Amino Acid CDR-H3 without TN1 and N2
(97)(97A)(97B)(97C)(97D)(97E)(97F)(97G)(101)(102)
|-----------------------------------|---------|
DH H3-JH

"Chassis" of the invention are portions of the antibody heavy chain variable (IGHV) or light chain variable (IGLV) domains that are not part of CDRH3 or CDRL3, respectively. A chassis of the invention is defined as the portion of the variable region of an antibody beginning with the first amino acid of FRM1 and ending with the last amino acid of FRM3. In the case of the heavy chain, the chassis includes the amino acids including from position 1 to position 94. In the case of the light chains (kappa and lambda), the chassis are defined as including from position 1 to position 88. The chassis of the invention may contain certain modifications relative to the corresponding germline variable domain sequences. These modifications may be engineered (*e.g.,* to remove N-linked glycosylation sites) or naturally occurring (*e.g.,* to account for naturally occurring allelic variation). For example, it is known in the art that the immunoglobulin gene repertoire is polymorphic (Wang et al., Immunol. Cell. Biol., 2008, 86: 111; Collins et al., Immunogenetics, 2008, 60: 669); chassis, CDRs and constant regions representative of these allelic variants are also encompassed by the invention. In some embodiments, the allelic variant(s) used in a particular embodiment of the invention may be selected based on the allelic variation present in different patient populations, for example, to identify antibodies that are non-immunogenic in these patient populations. In certain embodiments, the immunogenicity of an antibody of the invention may depend on allelic variation in the major histocompatibility complex (MHC) genes of a patient population. Such allelic variation may also be considered in the design of libraries of the invention. In certain embodiments of the invention, the chassis and constant regions are contained in a vector, and a CDR3 region is introduced between them via homologous recombination.

As used herein, a sequence designed with "directed diversity" has been specifically designed to contain both sequence diversity and length diversity. Directed diversity is not stochastic.

As used herein, the term "diversity" refers to a variety or a noticeable heterogeneity. The term "sequence diversity" refers to a variety of sequences which are collectively representative of several possibilities of sequences, for example, those found in natural human antibodies. For example, CDRH3 sequence diversity may refer to a variety of possibilities of combining the known human TN1, DH, N2, and H3-JH segments to form CDRH3 sequences. The CDRL3 sequence diversity (kappa or lambda) may refer to a variety of possibilities of combining the naturally occurring light chain variable region contributing to CDRL3 (*i.e.,* "L3-VL") and joining (*i.e.,* "L3-JL") segments, to form CDRL3 sequences. As used herein, "H3-JH" refers to the portion of the IGHJ gene contributing to CDRH3. As used herein, "L3-VL" and "L3-JL" refer to the portions of the IGLV and IGLJ genes (kappa or lambda) contributing to CDRL3, respectively.

As used herein, the term "expression" refers to steps involved in the production of a polypeptide including, but not limited to, transcription, post-transcriptional modification, translation, post-translational modification, and secretion.

The term "framework region" refers to the art-recognized portions of an antibody variable region that exist between the more divergent (*i*.*e*., hypervariable) CDRs. Such framework regions are typically referred to as frameworks 1 through 4 (FRM1, FRM2, FRM3, and FRM4) and provide a scaffold for the presentation of the six CDRs (three from the heavy chain and three from the light chain) in three dimensional space, to form an antigen-binding surface.

The term "full-length heavy chain" refers to an immunoglobulin heavy chain that contains each of the canonical structural domains of an immunoglobulin heavy chain, including the four framework regims, the three CDRs, and the constant region.

The term "full-length light chain" refers to an immunoglobulin light chain that contains each of the canonical structural domains of an immunoglobulin light chain, including the four framework regions, the three CDRs, and the constant region.

The term "germline-like," when used with respect to the CDRL3 sequences of the light chains of the invention, means those sequences consisting of combinations of: (i) the first six wild-type residues contributed to CDRL3 by the IGVL germline gene (*i*.*e*., positions 89 to 94 in the Kabat numbering system; "L" is kappa or lambda); and (ii) one of several amino acid sequences, two one to four amino acids in length, largely, but not exclusively, derived from the JL segment ("L," again is kappa or lambda). For kappa CDRL3 sequences of the most common lengths (*i.e.,* 8, 9, and 10 residues), the sequences of (ii) number twenty and are: FT, LT, IT, RT, WT, YT, [X]T, [X]PT, [X]FT, [X]LT, [X]IT, [X]RT, [X]WT, [X]YT, [X]PFT, [X]PLT, [X]PIT, [X]PRT, [X]PWT and [X]PYT, where [X] corresponds to the amino acid residue found at position 95 (Kabat) in the respective VK germline sequence. X is most commonly P, but may also be S or any other amino acid residue found at position 95 of a VK germline sequence. For eight exemplified VK chassis exemplified herein, the corresponding 160 germline-like sequences, (*i*.*e*., 20 sequences of two to four amino acids in length combined with positions 89 to 94 of each of eight VK germline sequences) are provided in Table 1. A similar approach is applied to define germline-like CDRL3 sequences for lambda light chains. As for the kappa sequences described above, the intact, un-mutated portions of CDRL3 encoded by the IGVL genes (in this case, IGVλ) would be combined with the sequences largely, but not exclusively, derived from the Jλ, segment. Here, the latter sequences (corresponding to (ii), above), number five and are: YV, VV, WV, AV or V. In addition, and as described in Example 7 of US 2009/0818155, one could further allow for variation at the last position of the Vλ-gene-encoded portion of CDRL3 by considering partial codons, while still considering the resulting sequences "germline-like." More specifically, the entire "minimalist library" of Example 7 in US 2009/0818155 would be defined as "germline-like." One of ordinary skill in the art will readily recognize that these methods can be extended to other VK and Vλ sequences.

The term "genotype-phenotype linkage," as understood by those of ordinary skill in the art, refers to the fact that the nucleic acid (genotype) encoding a protein with a particular phenotype (*e.g*., binding an antigen) can be isolated from a library. For the purposes of illustration, an antibody fragment expressed on the surface of a phage can be isolated based on its binding to an antigen (*e.g*., U.S. Patent No. 5,837,500). The binding of the antibody to the antigen simultaneously enables the isolation of the phage containing the nucleic acid encoding the antibody fragment. Thus, the phenotype (antigen-binding characteristics of the antibody fragment) has been "linked" to the genotype (nucleic acid encoding the antibody fragment). Other methods of maintaining a genotype-phenotype linkage include those of Wittrup et al. (U.S. Patent Nos. 6,300,065, 6,331,391, 6,423,538, 6,696,251, 6,699,658, and U.S. Pub. No. 20040146976), Miltenyi (US Patent No. 7,166,423, incorporated by reference in its entirety), Fandl (U.S. Patent No. 6,919,183, US Pub No. 20060234311), Clausell-Tormos et al. (Chem. Biol., 2008, 15: 427), Love et al. (Nat. Biotechnol., 2006, 24: 703), and Kelly et al. (Chem. Commun., 2007, 14: 1773). The term can be used to refer to any method which localizes an antibody protein together with the gene encoding the antibody protein, in a way in which they can both be recovered while the linkage between them is maintained.

The term "heterologous moiety" is used herein to indicate the addition of a moiety to an antibody wherein the moiety is not part of a naturally-occurring antibody. Exemplary heterologous moieties include drugs, toxins, imaging agents, and any other compositions which might provide an activity that is not inherent in the antibody itself.

As used herein, the term "host cell" is intended to refer to a cell comprising a polynucleotide of the invention. It should be understood that such terms refer not only to the particular subject cell but to the progeny or potential progeny of such a cell. Because certain modifications may occur in succeeding generations due to either mutation or environmental influences, such progeny may not, in fact, be identical to the parent cell, but are still included within the scope of the term as used herein.

As used herein, the term "human antibody CDR library" includes at least a polynucleotide or polypeptide library which has been designed to represent the sequence diversity and length diversity of naturally occurring CDRs in human antibodies (*e.g*., the term "CDR" in "human antibody CDR library" may be substituted with "CDRL1," "CDRL2," "CDRL3," "CDR1," "CDRH2," and/or "CDRH3"). Known human CDR sequences are represented in various data sets, including Jackson et al., J. Immunol Methods, 2007, 324: 26; Martin, Proteins, 1996,25: 130; Lee et al., Immunogenetics, 2006, 57: 917, Boyd et al., Science Translational Medicine, 2009, 1: 1, and WO/2009/036379, and the HPS, which is provided in Appendix A. The term "Human Preimmune Set," or "HPS," refers to a reference set of 3,571 curated human preimmune heavy chain sequences corresponding to the GI Nos. provided in Appendix A.

An "intact antibody" is one comprising full-length heavy- and light- chains (*i.e*., four frameworks, three CDRs, and a constant region for each of the heavy and light chains). An intact antibody is also referred to as a "full-length" antibody.

The term "length diversity" refers to a variety in the length of a family of nucleotide or amino acid sequence. For example, in naturally occurring human antibodies, the heavy chain CDR3 sequence varies in length, for example, from about 2 amino acids to over about 35 amino acids, and the light chain CDR3 sequence varies in length, for example, from about 5 to about 16 amino acids.

The term "library" refers to a set of entities comprising two or more entities having diversity as described herein, and/or designed according to the methods of the invention. For example, a "library of polynucleotides" refers to a set of polynucleotides comprising two or more polynucleotides having diversity as described herein, and/or designed according to the methods of the invention. A "library of polypeptides" refers to a set of polypeptides comprising two or more polypeptides having diversity as described herein, and/or designed according to the methods of the invention. A "library of synthetic polynucleotides" refers to a set of polynucleotides comprising two or more synthetic polynucleotides having diversity as described herein, and/or designed according to the methods of the invention. Libraries where all members are synthetic are also encompassed by the invention. A "human antibody library" refers to a set of polypeptides comprising two or more polypeptides having diversity as described herein, and/or designed according to the methods of the invention, for example a library designed to represent the sequence diversity and length diversity of naturally occurring human antibodies. In some embodiments, the term "library" may refer to a set of entities sharing similar structural or sequence characteristics, for example, a "heavy chain library," "light chain library," "antibody library," and/or "CDRH3 library."

The term "physical realization" refers to a portion of a theoretical (*e.g*., computer-based) or synthetic (e.g., oligonucleotide-based) diversity that can actually be physically sampled, for example, by any display methodology. Exemplary display methodology include: phage display, ribosomal display, and yeast display. For synthetic sequences, the size of the physical realization of a library depends on (1) the fraction of the theoretical diversity that can actually be synthesized, and (2) the limitations of the particular screening method. Exemplary limitations of screening methods include the number of variants that can be screened in a particular assay (*e.g*., ribosome display, phage display, yeast display) and the transformation efficiency of the host cells (*e.g*., yeast, mammalian cells, bacteria) which are used in a screening assay. For the purposes of illustration, given a library with a theoretical diversity of 10¹² members, an exemplary physical realization of the library *(e.g.,* in yeast, bacterial cells, or ribosome display) that can maximally include 10¹¹ members will, therefore, sample about 10% of the theoretical diversity of the library. However, if fewer than 10¹¹ members of the library with a theoretical diversity of 10¹² are synthesized, and the physical realization of the library can maximally include 10¹¹ members, less than 10% of the theoretical diversity of the library is sampled in the physical realization of the library. Similarly, a physical realization of the library that can maximally include more than 10¹² members would "oversample" the theoretical diversity, meaning that each member may be present more than once (assuming that the entire 10¹² theoretical diversity is synthesized).

The term "polynucleotide(s)" refers to nucleic acids such as DNA molecules and RNA molecules and analogs thereof (*e.g*., DNA or RNA generated using nucleotide analogs or using nucleic acid chemistry). As desired, the polynucleotides may be made synthetically, *e.g*., using art-recognized nucleic acid chemistry or enzymatically using, *e.g*., a polymerase, and, if desired, be modified. Typical modifications include methylation, biotinylation, and other art-known modifications. In addition, the nucleic acid molecule can be single-stranded or double-stranded and, where desired, linked to a detectable moiety. The representation of nucleotide bases herein follows International Union of Pure and Applied Chemistry (IUPAC) nomenclature (see U.S. Pub. No. 2009/0181855, incorporated by reference in its entirety).

"Preimmune" antibody libraries have sequence diversities and length diversities similar to naturally occurring human antibody sequences before these sequences have undergone negative selection and/or somatic hypermutation. For example, the set of sequences described in Lee et al. (Immunogenetics, 2006, 57: 917,) and the Human Preimmune Set (HPS) described herein (see Appendix A) are believed to represent sequences from the preimmune repertoire. In certain embodiments of the invention, the sequences of the invention will be similar to these sequences (*e.g*., in terms of composition and length).

As used herein, the term "sitewise stochastic" describes a process of generating a sequence of amino acids, where only the amino acid occurrences at the individual positions are considered, and higher order motifs (*e.g.,* pair-wise correlations) are not accounted for (*e.g.,* see Knappik, et al., J Mol Biol, 2000, 296: 57, and analysis provided in U.S. Publication No. 2009/0181855). The term "split-pool synthesis" refers to a procedure in which the products of a plurality of individual first reactions are combined (pooled) and then separated (split) before participating in a plurality of second reactions. For example, U.S. Publication No. 2009/0181855 describes the synthesis of 278 DH segments (products), each in a separate reaction. After synthesis, these 278 segments are combined (pooled) and then distributed (split) amongst 141 columns for the synthesis of the N2 segments. This enables the pairing of each of the 278 DH segments with each of 141 N2 segments.

As used herein, "stochastic" describes a process of generating a random sequence of nucleotides or amino acids, which is considered as a sample of one element from a probability distribution (*e.g*., see U.S. Patent No. 5,723,323).

As used herein, the term "synthetic polynucleotide" refers to a molecule formed through a chemical process, as opposed to molecules of natural origin, or molecules derived via template-based amplification of molecules of natural origin (*e.g*., immunoglobulin chains cloned from populations of B cells via PCR amplification are not "synthetic" as used herein). In some instances, for example, when referring to libraries of the invention that comprise multiple segments (*e.g*., TN1, DH, N2, and/or H3-JH), the invention encompasses libraries in which at least one, two, three, or four of the aforementioned components is synthetic. By way of illustration, a library in which certain components are synthetic, while other components are of natural origin or derived via template-based amplification of molecules of natural origin, would be encompassed by the invention. Libraries that are fully synthetic would, of course, also be encompassed by the invention.

The term "theoretical diversity" refers to the maximum number of variants in a library design. For example, given an amino acid sequence of three residues, where residues one and three may each be any one of five amino acid types and residue two may be any one of 20 amino acid types, the theoretical diversity is 5×20×5=500 possible sequences. Similarly if sequence X is constructed by combination of 4 amino acid segments, where segment 1 has 100 possible sequences, segment 2 has 75 possible sequences, segment 3 has 250 possible sequences, and segment 4 has 30 possible sequences, the theoretical diversity of fragment X would be 100x75x200x30, or 5.6×10⁵ possible sequences.

The term "theoretical segment pool" refers to a set of polynucleotide or polypeptide segments that can be used as building blocks to assemble a larger polynucleotide or polypeptide. For example, a theoretical segment pool containing TN1, DH, N2, and H3-JH segments can be used to assemble a library of CDRH3 sequences by concatenating them combinatorially to form a sequence represented by [TN1]-[DH]-[N2]-[H3-JH], and synthesizing the corresponding oligonucleotide(s). The term "theoretical segment pool" can apply to any set of polynucleotide or polypeptide segments. Thus, while a set of TN1, DH, N2, and H3-JH segments are collectively considered a theoretical segment pool, each of the individual sets of segments also comprise a theoretical segment pool, specifically a TN1 theoretical segment pool, a DH theoretical segment pool, an N2 theoretical segment pool, and an H3-JH theoretical segment pool. Any subsets of these theoretical segment pools containing two or more sequences can also be considered theoretical segment pools.

The term "unique," as used herein, refers to a sequence that is different (*e.g*., has a different chemical structure) from every other sequence within the designed set (*e.g.,* the theoretical diversity). It should be understood that there are likely to be more than one copy of many unique sequences from the theoretical diversity in a particular physical realization. For example, a library comprising three unique sequences at the theoretical level may comprise nine total members if each sequence occurs three times in the physical realization of the library. However, in certain embodiments, each unique sequence may occur only once, less than once, or more than once.

The term "variable" refers to the portions of the immunoglobulin domains that exhibit variability in their sequence and that are involved in determining the specificity and binding affinity of a particular antibody (*i.e.,* the "variable domain(s)"). Variability is not evenly distributed throughout the variable domains of antibodies; it is concentrated in sub-domains of each of the heavy and light chain variable regions. These sub-domains are called "hypervariable" regions or "complementarity determining regions" (CDRs). The more conserved (*i.e.,* non-hypervariable) portions of the variable domains are called the "framework" regions (FRM). The variable domains of naturally occurring heavy and light chains each comprise four FRM regions, largely adopting a β-sheet configuration, connected by three hypervariable regions, which form loops connecting, and in some cases forming part of, the β-sheet structure. The hypervariable regions in each chain are held together in close proximity by the FRM and, with the hypervariable regions from the other chain, contribute to the formation of the antigen-binding site (see Kabat et al. Sequences of Proteins of Immunological Interest, 5th Ed. Public Health Service, National Institutes of Health, Bethesda, Md., 1991, incorporated by reference in its entirety). The constant domains are not directly involved in antigen binding, but exhibit various effector functions, such as, for example, antibody-dependent, cell-mediated cytotoxicity and complement activation.

Libraries of the invention containing "VKCDR3" sequences and "VλCDR3" sequences refer to the kappa and lambda sub-sets of the light chain CDR3 (CDRL3) sequences, respectively. Such libraries may be designed with directed diversity, to collectively represent the length and sequence diversity of the human antibody CDRL3 repertoire. "Preimmune" versions of these libraries have similar sequence diversities and length diversities as naturally occurring human antibody CDRL3 sequences before these sequences undergo negative selection and/or somatic hypermutation. Known human CDRL3 sequences are represented in various data sets, including the NCBI database, WO/2009/036379, and Martin, Proteins, 1996, 25: 130.

### General Design of Libraries

Antibody libraries provided by the present invention may be designed to reflect certain aspects of the preimmune repertoire as created by the human immune system. Certain libraries of the invention are based on rational design informed by collections of human V, D, and J genes, and large databases of human heavy and light chain sequences (*e.g.,* publicly known germline sequences and sequences from Jackson et al., J. Immunol Methods, 2007, 324: 26; Lee et al., Immunogenetics, 2006, 57: 917; Boyd et al., Science Translational Medicine, 2009, 1: 1-8, and sequences compiled from rearranged VK and Vλ sequences (see WO/2009/036379, also incorporated by reference in its entirety). Additional information may be found, for example, in Scaviner et al., Exp. Clin. Immunogenet., 1999, 16: 234; Tomlinson et al., J. Mol. Biol., 1992,227: 799; and Matsuda et al., J. Exp. Med., 1998, 188: 2151. In certain embodiments of the invention, segments representing the possible V, D, and J diversity found in the human repertoire, as well as junctional diversity (*i.e.,* TN1 and N2), are synthesized *de novo* as single or double-stranded DNA oligonucleotides. In certain embodiments of the invention, oligonucleotides encoding CDR sequences are introduced into yeast along with one or more acceptor vectors containing heavy or light chain chassis sequences and constant domains. No primer-based PCR amplification or template-directed cloning steps from mammalian cDNA or mRNA are employed. Through standard homologous recombination, the recipient yeast recombines the CDR segments with the acceptor vectors containing the chassis sequences and constant regions, to create a properly ordered synthetic, full-length human heavy chain and/or light chain immunoglobulin library that can be genetically propagated, expressed, presented, and screened. One of ordinary skill in the art will readily recognize that the acceptor vector can be designed so as to produce constructs other than full-length human heavy chains and/or light chains. For example, in certain embodiments of the invention, the chassis may be designed to encode portions of a polypeptide encoding an antibody fragment or subunit of an antibody fragment, so that a sequence encoding an antibody fragment, or subunit thereof, is produced when the oligonucleotide cassette containing the CDR is recombined with the acceptor vector.

Thus, in certain embodiments, the invention provides a synthetic, preimmune human antibody repertoire the repertoire comprising:
(a) one or more selected human antibody heavy chain chassis (*i.e.,* amino acids 1 to 94 of the heavy chain variable region, using Kabat's definition);
(b) a CDRH3 repertoire (described more fully below), designed based on the human IGHD and IGHJ germline sequences, and the extraction of TN1 and N2 sequences from reference sets of human CDRH3 sequences, the CDRH3 repertoire comprising (i) a TN1 segment; (ii) a DH segment; (iii) anN2 segment; (iv) an H3-JH segment.
(c) one or more selected human antibody kappa and/or lambda light chain chassis; and
(d) a CDRL3 repertoire designed based on the human IGLV and IGLJ germline sequences, wherein "L" may be a kappa or lambda light chain.

The instant invention also provides methods for producing and using such libraries, as well as libraries comprising one or more immunoglobulin domains or antibody fragments. Design and synthesis of each component of the antibody libraries of the invention is provided in more detail below.

### Design of Antibody Library Chassis Sequences

In certain embodiments, provided libraries are constructed from selected chassis sequences that are based on naturally occurring variable domain sequences (*e.g*., IGHV and IGLV genes). The selection of such chassis sequences can be done arbitrarily, or through the definition of certain pre-determined criteria. For example, the Kabat database, an electronic database containing non-redundant rearranged antibody sequences, can be queried for those heavy and light chain germline sequences that are most frequently represented. An algorithm such as BLAST, or a more specialized tool such as SoDA (Volpe et al., Bioinformatics, 2006, 22: 438-44, incorporated by reference in its entirety), can be used to compare rearranged antibody sequences with germline sequences (*e.g.,* using the V BASE2 database; see, for example, Retter et al., Nucleic Acids Res., 2005, 33: D671-D674), or similar collections of human V, D, and J genes, to identify germline families that are most frequently used to generate functional antibodies.

Several criteria can be utilized for the selection of chassis for inclusion in the libraries of the invention. For example, sequences that are known (or have been determined) to express poorly in yeast, or other organisms used in the invention (*e.g*., bacteria, mammalian cells, fungi, or plants) can be excluded from the libraries. Chassis may also be chosen based on the representation of their corresponding germline genes in the peripheral blood of humans. In certain embodiments of the invention, it may be desirable to select chassis that correspond to germline sequences that are highly represented in the peripheral blood of humans. In some embodiments, it may be desirable to select chassis that correspond to germline sequences that are less frequently represented, for example, to increase the canonical diversity of the library. Therefore, chassis may be selected to produce libraries that represent the largest and most structurally diverse group of functional human antibodies.

In certain embodiments of the invention, less diverse chassis may be utilized, for example, if it is desirable to produce a smaller, more focused library with less chassis variability and greater CDR variability. In some embodiments of the invention, chassis may be selected based on both their expression in a cell of the invention (*e.g*., a yeast cell) and the diversity of canonical structures represented by the selected sequences. One may therefore produce a library with a diversity of canonical structures that express well in a cell of the invention.

### Design of Heavy Chain Chassis Sequences

The design and selection of heavy chain chassis sequences that can be used in the current invention is described in detail in U.S. Publication Nos. 2009/0181855 and 2010/0056386, and WO/2009/036379, and is therefore described only briefly here.

In general, VH domains of the library comprise three components: (1) a VH "chassis," which includes amino acids 1 to 94 (using Kabat numbering), (2) the CDRH3, which is defined herein to include the Kabat CDRH3 proper (positions 95-102), and (3) the FRM4 region, including amino acids 103 to 113 (Kabat numbering). The overall VH domain structure may therefore be depicted schematically (not to scale) as:

In certain embodiments of the invention, the VH chassis of the libraries may comprise from about Kabat residue 1 to about Kabat residue 94 of one or more of the following IGHV germline sequences: IGHV1-2, IGHV1-3, IGHV1-8, IGHV1-18, IGHV1-24, IGHV1-45, IGHV1-46, IGHV1-58, IGHV1-69, IGH8, IGH56, IGH100, IGHV3-7, IGHV3-9, IGHV3-11, IGHV3-13, IGHV3-15, IGHV3-20, IGHV3-21, IGHV3-23, IGHV3-30, IGHV3-33, IGHV3-43, IGHV3-48, IGHV3-49, IGHV3-53, IGHV3-64, IGHV3-66, IGHV3-72, IGHV3-73, IGHV3-74, IGHV4-4, IGHV4-28, IGHV4-31, IGHV4-34, IGHV4-39, IGHV4-59, IGHV4-61, IGHV4-B, IGHV5-51, IGHV6-1, and/or IGHV7-4-1. In some embodiments of the invention, a library may contain one or more of these sequences, one or more allelic variants of these sequences, or encode an amino acid sequence at least about 99.9%, 99.5%, 99%, 98.5%, 98%, 97.5%, 97%, 96.5%, 96%, 95.5%, 95%, 94.5%, 94%, 93.5%, 93%, 92.5%, 92%, 91.5%, 91%, 90.5%, 90%, 89%, 88%, 87%, 86%, 85%, 84%, 83%, 82%, 81%, 80%, 77.5%, 75%, 73.5%, 70%, 65%, 60%, 55%, or 50% identical to one or more of these sequences. One of ordinary skill in the art will recognize that given the chassis definition provided above, any IGHV-encoding sequence can be adapted for use as a chassis of the invention. As exemplified in U.S. Publication Nos. 2009/0181855 and 2010/0056386, and WO/2009/036379 these chassis can also be varied, particularly by altering the amino acid residues in the CDRH1 and CDRH2 regions, further increasing the diversity of the library.

### Design of Light Chain Chassis Sequences

The design and selection of light chain chassis sequences that can be used in the current invention is described in detail in U.S. Publication Nos. 2009/0181855 and 2010/0056386, and WO/2009/036379, and is therefore described only briefly here. The light chain chassis of the invention may be based on kappa and/or lambda light chain sequences.

The VL domains of the library comprise three primary components: (1) a VL "chassis", which includes amino acids 1 to 88 (using Kabat numbering), (2) the CDRL3, which is defined herein to include the Kabat CDRL3 proper (positions 89-97), and (3) the FRM4 region, including amino acids 98 to 107 (Kabat numbering). The overall VL domain structure may therefore be depicted schematically (not to scale) as:

In certain embodiments of the invention, the VL chassis of the libraries include one or more chassis based on IGKV germline sequences. In certain embodiments of the invention, the VL chassis of the libraries may comprise from about Kabat residue 1 to about Kabat residue 88 of one or more of the following IGKV germline sequences: IGV1-05, IGKV1-06, IGKV1-08, IGKV1-09, IGKV1-12, IGKV1-13, IGKV1-16, IGKV1-17, IGKV1-27, IGKV1-33, IGKV1-37, IGKV1-39, IGKV1D-16, IGKV1D-17, IGKV1D-43, IGKV1D-8, IGK54, IGK58, IGK59, IGK60, IGK70, IGKV2D-26, IGKV2D-29, IGKV2D-30, IGKV3-11, IGKV3-15, IGKV3-20, IGKV3D-07, IGKV3D-11, IGKV3D-20, IGKV4-1, IGKV5-2, IGKV6-21, and/or IGKV6D-41. In some embodiments of the invention, a library may contain one or more of these sequences, one or more allelic variants of these sequences, or encode an amino acid sequence at least about 99.9%, 99.5%, 99%, 98.5%, 98%, 97.5%, 97%, 96.5%, 96%, 95.5%, 95%, 94.5%, 94%, 93.5%, 93%, 92.5%, 92%, 91.5%, 91%, 90.5%, 90%, 89%, 88%, 87%, 86%, 85%, 84%, 83%, 82%, 81%, 80%, 77.5%, 75%, 73.5%, 70%, 65%, 60%, 55%, or 50% identical to one or more of these sequences.

In certain embodiments of the invention, the VL chassis of the libraries include one or more chassis based on IGλV germline sequences. In certain embodiments of the invention, the VL chassis of the libraries may comprise from about Kabat residue 1 to about Kabat residue 88 of one or more of the following IGλV germline sequences: IGλV3-1, IGλV3-21, IGλ44, IGλV1-40, IGλV3-19, IGλV1-51, IGλV1-44, IGλV6-57, IGλ11, IGλV3-25, IGλ53, IGλV3-10, IGλV4-69, IGλV1-7, IGλ41, IGλV7-43, IGλV7-46, IGλV5-45, IGλV4-60, IGλV10-54, IGλV8-61, IGλV3-9, IGλV1-36, IGλ48, IGλV3-16, IGλV3-27, IGλV4-3, IGλV5-39, IGλV9-49, and/or IGλV3-12. In some embodiments of the invention, a library may contain one or more of these sequences, one or more allelic variants of these sequences, or encode an amino acid sequence at least about 99.9%, 99.5%, 99%, 98%, 97%, 96%, 95%, 94%, 93%, 92%, 91%, 90%, 85%, 80%, 75%, 70%, 65%, 60%, 55%, or 50% identical to one or more of these sequences.

One of ordinary skill in the art will recognize that given the chassis definition provided above, any IGKV- or IGλV-encoding sequence can be adapted for use as a chassis of the invention.

### Design and Selection of TN1, DH, N2, and H3-JH Segments

The human germline repertoire contains at least six IGHJ genes (IGHJ1, IGHJ2, IGHJ3, IGHJ4, IGHJ5, and IGHJ6; included in Table 14, where the primary allele is designated "01," and selected allelic variants are designated "02" or "03"), and at least 27 IGHD genes (Table 16, including allelic variants). In some embodiments, the invention comprises a library of CDRH3 polypeptide sequences, or polynucleotide sequences encoding CDRH3 sequences, the library comprising members of any of the theoretical segment pools disclosed herein.

A person of ordinary skill in the art will recognize that not every segment in a theoretical segmentpool provided herein is necessary to produce a functional CDRH3 library of the invention. Therefore, in certain embodiments, a CDRH3 library of the invention will contain a subset of the segments of any of the theoretical segment pools described herein. For example, in certain embodiments of the invention, at least about 15, 30, 45, 60, 75, 90, 100, 105, 120, 135, 150, 165, 180, 195, 200, 210, 225, 240, 255, 270, 285, 300, 320, 340, 360, 380, 400, 420, 440, 460, 480, 500, 520, 540, 560, 580, 600, 620, 640, or 643 of the H3-JH segments of any of the theoretical segment pools provided herein, or generated by the methods described herein, are included in a library. In some embodiments of the invention, at least about 15, 30, 45, 60, 75, 90, 100, 105, 120, 135, 150, 165, 180, 195, 200, 250, 300, 350, 400, 450, 500, 550, 600, 650, 700, 750, 800, 850, 900, 950, 1000, 1050, 1100, 1111, 2000, 3000, 4000, 5000, 6000, 7000, 14000, 21000, 28000, 35000, 42000, 49000, 56000, 63000, or 68374 of the DH segments of any of the theoretical segment pools provided herein, or generated by the methods described herein, are included in a library. In some embodiments of the invention, at least about 10, 20, 30, 40, 50, 60, 70, 80, 90, 100, 110, 120, 130, 140, 141, 150, 160, 170,180, 190, or 200, 220, 240, 260, 280, 300, 320, 340, 360, 380, 400, 420, 424, 440, 460, 480, 500, 550, 600, 650, 700, 727, 750, 800, 850, 900, 950, or 1000 of the TN1 and/or N2 segments of any of the theoretical segment pools provided herein, or generated by the methods described herein, are included in a library. In certain embodiments, a library of the invention may contain less than a particular number of polynucleotide or polypeptide segments, where the number of segments is defined using any one of the integers provided above for the respective segment. In some embodiments of the invention, a particular numerical range is defined, using any two of the integers provided above as lower and upper boundaries of the range, inclusive or exclusive. All combinations of the integers provided, which define an upper and lower boundary, are contemplated.

In certain embodiments, the invention provides CDRH3 libraries comprising at least about 1%, 2.5%, 5%, 10%, 15%, 20%, 25%, 30%, 40%, 45%, 50%, 55%, 60%, 65%, 70%, 75%, 80%, 85%, 90%, 95%, or 99% of the segments from any of the theoretical segment pools provided herein. For example, the invention provides libraries comprising at least about 1%, 2.5%, 5%, 10%, 15%, 20%, 25%, 30%, 40%, 45%, 50%, 55%, 60%, 65%, 70%, 75%, 80%, 85%, 90%, 95%, or 99% of the TN1, DH, N2, and/or H3-JH segments from any of the theoretical segment pools provided herein. In some embodiments of the invention, a particular percentage range is defined, using any two of the percentages provided above as lower and upper boundaries of the range, inclusive or exclusive. All combinations of the percentages provided, which define an upper and lower boundary, are contemplated.

In some embodiments of the invention, at least about 1%, 2.5%, 5%, 10%, 15%, 20%, 25%, 30%, 40%, 45%, 50%, 55%, 60%, 65%, 70%, 75%, 80%, 85%, 90%, 95%, or 99% of the H3-JH DD, TN1, and/or N2 segments in a CDRH3 library are H3-JH, DH, TN1, and/or N2 segments of any of the theoretical segment pools provided herein, or generated by the methods described herein,. In some embodiments of the invention, at least about 1%, 2.5%, 5%, 10%, 15%, 20%, 25%, 30%, 40%, 45%, 50%, 55%, 60%, 65%, 70%, 75%, 80%, 85%, 90%, 95%, or 99% of the H3-JH, DH, TN1, and/or N2 segments of antibodies isolated from a CDRH3 library *(e.g.,* by binding to a particular antigen and/or generic ligand through one or more rounds of selection) are H3-JH, DH, TN1, and/or N2 segments of any of the theoretical segment pools provided herein, or generated by methods described herein. In certain embodiments, a CDRH3 library of the invention may contain less than a particular percentage of H3-JH, DH, TN1, and/or N2 segments of any of the theoretical segment pools provided herein, or generated by the methods described herein, where the number of segments is defined using any one of the percentages provided above for the respective segment. In some embodiments of the invention, a particular percentage range is defined, using any two of the percentages provided above as lower and upper boundaries of the range, inclusive or exclusive. All combinations of the percentages provided, which define an upper and lower boundary, are contemplated.

One of ordinary skill in the art will appreciate, upon reading the disclosure herein. that given the TN1, DH, N2, and/or H3-JH segments of any of the theoretical segment pools provided herein, or generated by the methods described herein, similar TN1, DH, N2, and/or H3-JH segments, and corresponding CDRH3 libraries, could be produced which, while not 100% identical to those provided in terms of their sequences, may be functionally very similar. Such theoretical segment pools and CDRH3 libraries also fall within the scope of the invention. A variety of techniques well-known in the art could be used to obtain these additional sequences, including the mutagenesis techniques provided herein. Therefore, each of the explicitly enumerated embodiments of the invention can also be practiced using segments that share a particular percent identity to any of the segments of any of the theoretical segment pools provided herein, or generated by the methods described herein. For example, each of the previously described embodiments of the invention can be practiced using TN1, DH, N2, and/or H3-JH segments that are at least about 50%, 55%, 60%, 65%, 70%, 75%, 80%, 85%, 90% 91%, 92%, 93%, 94%, 95%, 96%, 97%, 98%, 99%, 99.5%, or 99.9% identical to the TN1 DH, N2, and/or H3-JH segments of any of the theoretical segment pools provided herein, or generated by the methods described herein.

In some embodiments, the invention provides libraries produced from one or more VH chassis sequences combined with one or more TN1 segments, one or more DH segments, one or more N2 segments, and one or more H3-JH segments. In certain embodiments at least 1, 2, 5, 10, 20, 50, 75, or 100, of each chassis, TN1, DH, N2, or H3-JH segments are included in a library of the invention.

In some embodiments, the invention provides a method of selecting TN1, DH, N2, and H3-JH segments from a theoretical segment pool for inclusion in a synthetic CDRH3 library, comprising:
(i) providing a theoretical segment pool containing one or more of TN1, DH, N2, and H3-JH segments;
(ii) providing a reference set of CDRH3 sequences;
(iii) utilizing the theoretical segment pool of (i) to identify the closest match(es) to each CDRH3 sequence in the reference set of (ii); and
(iv) selecting segments from the theoretical segment pool for inclusion in a synthetic library.

In some embodiments, the selection process of (iv) can optionally involve any number of additional criteria, including the frequency of occurrence of the segments of (i) in the reference set of (ii); the corresponding segmental usage weights; and any physicochemical properties (see all numerical indices at www.genome.jp/aaindex/) of the segments (*e.g*., hydrophobicity, alpha-helical propensity, and/or isoelectric point). Optionally, TN1 and/or N2 segments that do not occur in the theoretical segment pool of (i) but that are found in the reference set of (ii) may be identified and added to prospective theoretical segment pools to produce theoretical segment pools with increased TN1 and/or N2 diversity in the prospective theoretical segment pools and/or synthetic libraries of the invention.

Any characteristic or set of characteristics of the segments can be used to choose them for inclusion in the library, including for example one or more biological properties (e.g., immunogenicity, stability, half-life) and/or one or more physicochemical properties such as the numerical indices provided above. In some embodiments, at least 1, 2, 3, 4, 5, 6, 7, 8, 9, 10, 11, 12, 13, 14, 15 or more such properties is used to select segments for inclusion in a library of the invention.
Physiochemical properties included in the index provided above can include, for example, ANDN920101 alpha-CH chemical shifts (Andersen et al., 1992); ARGP820101 Hydrophobicity index (Argos et al., 1982); ARGP820102 Signal sequence helical potential (Argos et al., 1982); ARGP820103 Membrane-buried preference parameters (Argos et al., 1982); BEGF750101 Conformational parameter of inner helix (Beghin-Dirkx, 1975); BEGF750102 Conformational parameter of beta-structure (Beghin-Dirkx, 1975); BEGF750103 Conformational parameter of beta-turn (Beghin-Dirkx, 1975); BHAR880101 Average flexibility indices (Bhaskaran-Ponnuswamy, 1988); BIGC670101 Residue volume (Bigelow, 1967); BIOV880101 Information value for accessibility; average fraction 35% (Biou et al., 1988); BIOV880102 Information value for accessibility; average fraction 23% (Biou et al., 1988); BROC820101 Retention coefficient in TFA (Browne et al., 1982); BROC820102 Retention coefficient in HFBA (Browne et al., 1982); BULH740101 Transfer free energy to surface (Bull-Breese, 1974); BULH740102 Apparent partial specific volume (Bull-Breese, 1974); BUNA790101 alpha-NH chemical shifts (Bundi-Wuthrich, 1979); BUNA790102 alpha-CH chemical shifts (Bundi-Wuthrich, 1979); BUNA790103 Spin-spin coupling constants 3JHalpha-NH (Bundi-Wuthrich, 1979); BURA740101 Normalized frequency of alpha-helix (Burgess et al., 1974); BURA740102 Normalized frequency of extended structure (Burgess et al., 1974); CHAM810101 Steric parameter (Charton, 1981); CHAM820101 Polarizability parameter (Charton-Charton, 1982); CHAM820102 Free energy of solution in water, kcal/mole (Charton-Charton, 1982); CHAM830101 The Chou-Fasman parameter of the coil conformation (Charton-Charton, 1983); CHAM830102 A parameter defined from the residuals obtained from the best correlation of the Chou-Fasman parameter of beta-sheet (Charton-Charton, 1983); CHAM830103 The number of atoms in the side chain labelled 1+1 (Charton-Charton, 1983); CHAM830104 The number of atoms in the side chain labelled 2+1 (Charton-Charton, 1983); CHAM830105 The number of atoms in the side chain labelled 3+1 (Charton-Charton, 1983); CHAM830106 The number of bonds in the longest chain (Charton-Charton, 1983); CHAM830107 A parameter of charge transfer capability (Charton-Charton, 1983); CHAM830108 A parameter of charge transfer donor capability (Charton-Charton, 1983); CHOC750101 Average volume of buried residue (Chothia, 1975); CHOC760101 Residue accessible surface area in tripeptide (Chothia, 1976); CHOC760102 Residue accessible surface area in folded protein (Chothia, 1976); CHOC760103 Proportion of residues 95% buried (Chothia, 1976); CHOC760104 Proportion of residues 100% buried (Chothia, 1976); CHOP780101 Normalized frequency of beta-turn (Chou-Fasman, 1978a); CHOP780201 Normalized frequency of alpha-helix (Chou-Fasman, 1978b); CHOP780202 Normalized frequency of beta-sheet (Chou-Fasman, 1978b); CHOP780203 Normalized frequency of beta-turn (Chou-Fasman, 1978b); CHOP780204 Normalized frequency of N-terminal helix (Chou-Fasman, 1978b); CHOP780205 Normalized frequency of C-terminal helix (Chou-Fasman, 1978b); CHOP780206 Normalized frequency of N-terminal non helical region (Chou-Fasman, 1978b); CHOP780207 Normalized frequency of C-terminal non helical region (Chou-Fasman, 1978b); CHOP780208 Normalized frequency of N-terminal beta-sheet (Chou-Fasman, 1978b); CHOP780209 Normalized frequency of C-terminal beta-sheet (Chou-Fasman, 1978b); CHOP780210 Normalized frequency of N-terminal non beta region (Chou-Fasman, 1978b); CHOP780211 Normalized frequency of C-terminal non beta region (Chou-Fasman, 1978b); CHOP780212 Frequency of the 1 st residue in turn (Chou-Fasman, 1978b); CHOP780213 Frequency of the 2nd residue in turn (Chou-Fasman, 1978b); CHOP780214 Frequency of the 3rd residue in turn (Chou-Fasman, 1978b); CHOP780215 Frequency of the 4th residue in turn (Chou-Fasman, 1978b); CHOP780216 Normalized frequency of the 2nd and 3rd residues in turn (Chou-Fasman, 1978b); CIDH920101 Normalized hydrophobicity scales for alpha-proteins (Cid et al., 1992); CIDH920102 Normalized hydrophobicity scales for beta-proteins (Cid et al., 1992); CIDH920103 Normalized hydrophobicity scales for alpha+beta-proteins (Cid et al., 1992); CIDH920104 Normalized hydrophobicity scales for alpha/beta-proteins (Cid et al., 1992); CIDH920105 Normalized average hydrophobicity scales (Cid et al., 1992); COHE430101 Partial specific volume (Cohn-Edsall, 1943); CRAJ730101 Normalized frequency of middle helix (Crawford et al., 1973); CRAJ730102 Normalized frequency of beta-sheet (Crawford et al., 1973); CRAJ730103 Normalized frequency of turn (Crawford et al., 1973); DAWD720101 Size (Dawson, 1972); DAYM780101 Amino acid composition (Dayhoff et al., 1978a); DAYM780201 Relative mutability (Dayhoff et al., 1978b); DESM900101 Membrane preference for cytochrome b: MPH89 (Degli Esposti et al., 1990); DESM900102 Average membrane preference: AMP07 (Degli Esposti et al., 1990); EISD840101 Consensus normalized hydrophobicity scale (Eisenberg, 1984); EISD860101 Solvation free energy (Eisenberg-McLachlan, 1986); EISD860102 Atom-based hydrophobic moment (Eisenberg-McLachlan, 1986); EISD860103 Direction of hydrophobic moment (Eisenberg-McLachlan, 1986); FASG760101 Molecular weight (Fasman, 1976); FASG760102 Melting point (Fasman, 1976); FASG760103 Optical rotation (Fasman, 1976); FASG760104 pK-N (Fasman, 1976); FASG760105 pK-C (Fasman, 1976); FAUJ830101 Hydrophobic parameter pi (Fauchere-Pliska, 1983); FAUJ880101 Graph shape index (Fauchere et al., 1988); FAUJ880102 Smoothed upsilon steric parameter (Fauchere et al., 1988); FAUJ880103 Normalized van der Waals volume (Fauchere et al., 1988); FAUJ880104 STERIMOL length of the side chain (Fauchere et al., 1988); FAUJ880105 STERIMOL minimum width of the side chain (Fauchere et al., 1988); FAUJ880106 STERIMOL maximum width of the side chain (Faudiere et al., 1988); FAUJ880107 N.m.r. chemical shift of alpha-carbon (Fauchere et al., 1988); FAUJ880108 Localized electrical effect (Fauchere et al., 1988); FAUJ880109 Number of hydrogen bond donors (Fauchere et al., 1988); FAUJ880110 Number of full nonbonding orbitals (Fauchere et al., 1988); FAUJ880111 Positive charge (Fauchere et al., 1988); FAUJ880112 Negative charge (Fauchere et al., 1988); FAUJ880113 pK-a(RCOOH) (Fauchere et al., 1988); FINA770101 Helix-coil equilibrium constant (Finkelstein-Ptitsyn, 1977); FINA910101 Helix initiation parameter at posision i-1 (Finkelstein et al., 1991); FINA910102 Helix initiation parameter at posision i,i+1,i+2 (Finkelstein et al., 1991); FINA910103 Helix termination parameter at posision j-2,j-1,j (Finkelstein et al., 1991); FINA910104 Helix termination parameter at posision j+1 (Finkelstein et al., 1991); GARJ730101 Partition coefficient (Garel et al., 1973); GEIM800101 Alpha-helix indices (Geisow-Roberts, 1980); GEIM800102 Alpha-helix indices for alpha-proteins (Geisow-Roberts, 1980); GEIM800103 Alpha-helix indices for beta-proteins (Geisow-Roberts, 1980); GEIM800104 Alpha-helix indices for alpha/beta-proteins (Geisow-Roberts, 1980); GEIM800105 Beta-strand indices (Geisow-Roberts, 1980); GEIM800106 Beta-strand indices for beta-proteins (Geisow-Roberts, 1980); GEIM800107 Beta-strand indices for alpha/beta-proteins (Geisow-Roberts, 1980)
GEIM800108 Aperiodic indices (Geisow-Roberts, 1980);GEI M800109 Aperiodic indices for alphaproteins (Geisow-Roberts, 1980); GEIM800110 Aperiodic indices for beta-proteins (Geisow-Roberts, 1980);
GEIM800111 Aperiodic indices for alpha/beta-proteins (Geisow-Roberts, 1980); GOLD730101 Hydrophobicity factor (Goldsack-Chalifoux, 1973); GOLD730102 Residue volume (Goldsack-Chalifoux, 1973); GRAR740101 Composition (Grantham, 1974); GRAR740102 Polarity (Grantham, 1974)
GRAR740103 Volume (Grantham, 1974); GUYH850101 Partition energy (Guy, 1985); HOPA770101 Hydration number (Hopfinger, 1971), Cited by Charton-Charton (1982)
HOPT810101 Hydrophilicity value (Hopp-Woods, 1981); HUTJ700101 Heat capacity (Hutchens, 1970); HUTJ700102 Absolute entropy (Hutchens, 1970); HUTJ700103 Entropy of formation (Hutchens, 1970); ISOY800101 Normalized relative frequency of alpha-helix (Isogai et al., 1980); ISOY800102 Normalized relative frequency of extended structure (Isogai et al., 1980); ISOY800103 Normalized relative frequency of bend (Isogai et al., 1980); ISOY800104 Normalized relative frequency of bend R (Isogai et al., 1980); ISOY800105 Normalized relative frequency of bend S (Isogai et al., 1980); ISOY800106 Normalized relative frequency of helix end (Isogai et al., 1980); ISOY800107 Normalized relative frequency of double bend (Isogai et al., 1980); ISOY800108 Normalized relative frequency of coil (Isogai et al., 1980); JANJ780101 Average accessible surface area (Janin et al., 1978); JANJ780102 Percentage of buried residues (Janin et al., 1978); JANJ780103 Percentage of exposed residues (Janin et al., 1978); JANJ790101 Ratio of buried and accessible molar fractions (Janin, 1979); JANJ790102 Transfer free energy (Janin, 1979); JOND750101 Hydrophobicity (Jones, 1975); JOND750102 pK (-COOH) (Jones, 1975); JOND920101 Relative frequency of occurrence (Jones et al., 1992); JOND920102 Relative mutability (Jones et al., 1992)
JUKT750101 Amino acid distribution (Jukes et al., 1975); JUNJ780101 Sequence frequency (Jungck, 1978); KANM800101 Average relative probability of helix (Kanehisa-Tsong, 1980); KANM800102 Average relative probability of beta-sheet (Kanehisa-Tsong, 1980); KANM800103 Average relative probability of inner helix (Kanehisa-Tsong, 1980); KANM800104 Average relative probability of inner beta-sheet (Kanehisa-Tsong, 1980); KARP850101 Flexibility parameter for no rigid neighbors (Karplus-Schulz, 1985); KARP850102 Flexibility parameter for one rigid neighbor (Karplus-Schulz, 1985); KARP850103 Flexibility parameter for two rigid neighbors (Karplus-Schulz, 1985); KHAG800101 The Kerr-constant increments (Khanarian-Moore, 1980); KLEP840101 Net charge (Klein et al., 1984); KRIW710101 Side chain interaction parameter (Krigbaum-Rubin, 1971); KRIW790101 Side chain interaction parameter (Krigbaum-Komoriya, 1979); KRIW790102 Fraction of site occupied by water (Krigbaum-Komoriya, 1979); KRIW790103 Side chain volume (Krigbaum-Komoriya, 1979); KYTJ820101 Hydropathy index (Kyte-Doolittle, 1982); LAWE840101 Transfer free energy, CHP/water (Lawson et al., 1984); LEVM760101 Hydrophobic parameter (Levitt, 1976); LEVM760102 Distance between C-alpha and centroid of side chain (Levitt, 1976); LEVM760103 Side chain angle theta(AAR) (Levitt, 1976); LEVM760104 Side chain torsion angle phi(AAAR) (Levitt, 1976); LEVM760105 Radius of gyration of side chain (Levitt, 1976); LEVM760106 van der Waals parameter R0 (Levitt, 1976)
LEVM760107 van der Waals parameter epsilon (Levitt, 1976); LEVM780101 Normalized frequency of alpha-helix, with weights (Levitt, 1978); LEVM780102 Normalized frequency of beta-sheet, with weights (Levitt, 1978); LEVM780103 Normalized frequency of reverse turn, with weights (Levitt, 1978); LEVM780104 Normalized frequency of alpha-helix, unweighted (Levitt, 1978); LEVM780105 Normalized frequency of beta-sheet, unweighted (Levitt, 1978); LEVM780106 Normalized frequency of reverse turn, unweighted (Levitt, 1978); LEWP710101 Frequency of occurrence in beta-bends (Lewis et al., 1971); LIFS790101 Conformational preference for all beta-strands (Lifson-Sander, 1979); LIFS790102 Conformational preference for parallel beta-strands (Lifson-Sander, 1979); LIFS790103 Conformational preference for antiparallel beta-strands (Lifson-Sander, 1979); MANP780101 Average surrounding hydrophobicity (Manavalan-Ponnuswamy, 1978); MAXF760101 Normalized frequency of alpha-helix (Maxfield-Scheraga, 1976); MAXF760102 Normalized frequency of extended structure (Maxfield-Scheraga, 1976); MAXF760103 Normalized frequency of zeta R (Maxfield-Scheraga, 1976); MAXF760104 Normalized frequency of left-handed alpha-helix (Maxfield-Scheraga, 1976); MAXF760105 Normalized frequency of zeta L (Maxfield-Scheraga, 1976); MAXF760106 Normalized frequency of alpha region (Maxfield-Scheraga, 1976); MCMT640101 Refractivity (McMeekin et al., 1964), Cited by Jones (1975); MEEJ800101 Retention coefficient in HPLC, pH7.4 (Meek, 1980); MEEJ800102 Retention coefficient in HPLC, pH2.1 (Meek, 1980); MEEJ810101 Retention coefficient in NaClO4 (Meek-Rossetti, 1981); MEEJ810102 Retention coefficient in NaH2PO4 (Meek-Rossetti, 1981); MEIH800101 Average reduced distance for C-alpha (Meirovitch et al., 1980); MEIH800102 Average reduced distance for side chain (Meirovitch et al., 1980); MEIH800103 Average side chain orientation angle (Meirovitch et al., 1980); MIYS850101 Effective partition energy (Miyazawa-Jernigan, 1985); NAGK730101 Normalized frequency of alpha-helix (Nagano, 1973); NAGK730102 Normalized frequency of bata-structure (Nagano, 1973)
NAGK730103 Normalized frequency of coil (Nagano, 1973); NAKH900101 AA composition of total proteins (Nakashima et al., 1990); NAKH900102 SD of AA composition of total proteins (Nakashima et al., 1990); NAKH900103 AA composition of mt-proteins (Nakashima et al., 1990); NAKH900104 Normalized composition of mt-proteins (Nakashima et al., 1990); NAKH900105 AA composition of mt-proteins from animal (Nakashima et al., 1990); NAKH900106 Normalized composition from animal (Nakashima et al., 1990); NAKH900107 AA composition of mt-proteins from fungi and plant (Nakashima et al., 1990); NAKH900108 Normalized composition from fungi and plant (Nakashima et al., 1990); NAKH900109 AA composition of membrane proteins (Nakashima et al., 1990); NAKH900110 Normalized composition of membrane proteins (Nakashima et al., 1990); NAKH900111 Transmembrane regions of non-mt-proteins (Nakashima et al., 1990); NAKH900112 Transmembrane regions of mt-proteins (Nakashima et al., 1990); NAKH900113 Ratio of average and computed composition (Nakashima et al., 1990); NAKH920101 AA composition of CYT of single-spanning proteins (Nakashima-Nishikawa, 1992); NAKH920102 AA composition of CYT2 of single-spanning proteins (Nakashima-Nishikawa, 1992); NAKH920103 AA composition of EXT of single-spanning proteins (Nakashima-Nishikawa, 1992); NAKH920104 AA composition of EXT2 of single-spanning proteins (Nakashima-Nishikawa, 1992); NAKH920105 AA composition of MEM of single-spanning proteins (Nakashima-Nishikawa, 1992); NAKH920106 AA composition of CYT of multi-spanning proteins (Nakashima-Nishikawa, 1992); NAKH920107 AA composition of EXT of multi-spanning proteins (Nakashima-Nishikawa, 1992); NAKH920108 AA composition of MEM of multi-spanning proteins (Nakashima-Nishikawa, 1992); NISK800101 8 A contact number (Nishikawa-Ooi, 1980); NISK860101 14 A contact number (Nishikawa-Ooi, 1986); NOZY710101 Transfer energy, organic solvent/water (Nozaki-Tanford, 1971); OOBM770101 Average non-bonded energy per atom (Oobatake-Ooi, 1977); OOBM770102 Short and medium range non-bonded energy per atom (Oobatake-Ooi, 1977); OOBM770103 Long range non-bonded energy per atom (Oobatake-Ooi, 1977)
OOBM770104 Average non-bonded energy per residue (Oobatake-Ooi, 1977); OOBM770105 Short and medium range non-bonded energy per residue (Oobatake-Ooi, 1977); OOBM850101 Optimized beta-structure-coil equilibrium constant (Oobatake et al., 1985); OOBM850102 Optimized propensity to form reverse turn (Oobatake et al., 1985); OOBM850103 Optimized transfer energy parameter (Oobatake et al., 1985); OOBM850104 Optimized average non-bonded energy per atom (Oobatake et al., 1985); OOBM850105 Optimized side chain interaction parameter (Oobatake et al., 1985); PALJ810101 Normalized frequency of alpha-helix from LG (Palau et al., 1981); PALJ810102 Normalized frequency of alphahelix from CF (Palau et al., 1981); PALJ810103 Normalized frequency of beta-sheet from LG (Palau et al., 1981); PALJ810104 Normalized frequency of beta-sheet from CF (Palau et al., 1981); PALJ810105 Normalized frequency of turn from LG (Palau et al., 1981); PALJ810106 Normalized frequency of turn from CF (Palau et al., 1981); PALJ810107 Normalized frequency of alpha-helix in all-alpha class (Palau et al., 1981); PALJ810108 Normalized frequency of alpha-helix in alpha+beta class (Palau et al., 1981); PALJ810109 Normalized frequency of alpha-helix in alpha/beta class (Palau et al., 1981); PALJ810110 Normalized frequency of beta-sheet in all-beta class (Palau et al., 1981); PALJ810111 Normalized frequency of beta-sheet in alpha+beta class (Palau et al., 1981); PALJ810112 Normalized frequency of beta-sheet in alpha/beta class (Palau et al., 1981); PALJ810113 Normalized frequency of turn in all-alpha class (Palau et al., 1981); PALJ810114 Normalized frequency of turn in all-beta class (Palau et al., 1981); PALJ810115 Normalized frequency of turn in alpha+beta class (Palau et al., 1981); PALJ810116 Normalized frequency of turn in alpha/beta class (Palau et al., 1981); PARJ860101 HPLC parameter (Parker et al., 1986); PLIV810101 Partition coefficient (Pliska et al., 1981); PONP800101 Surrounding hydrophobicity in folded form (Ponnuswamy et al., 1980); PONP800102 Average gain in surrounding hydrophobicity (Ponnuswamy et al., 1980); PONP800103 Average gain ratio in surrounding hydrophobicity (Ponnuswamy et al., 1980); PONP800104 Surrounding hydrophobicity in alpha-helix (Ponnuswamy et al., 1980); PONP800105 Surrounding hydrophobicity in beta-sheet (Ponnuswamy et al., 1980); PONP800106 Surrounding hydrophobicity in turn (Ponnuswamy et al., 1980); PONP800107 Accessibility reduction ratio (Ponnuswamy et al., 1980); PONP800108 Average number of surrounding residues (Ponnuswamy et al., 1980); PRAM820101 Intercept in regression analysis (Prathakaran-Ponnuswamy, 1982); PRAM820102 Slope in regression analysis x 1.0E1 (Prabhakaran-Ponnuswamy, 1982); PRAM820103 Correlation coefficient in regression analysis (Prabhakaran-Ponnuswamy, 1982); PRAM900101 Hydrophobicity (Prabhakaran, 1990); PRAM900102 Relative frequency in alpha-helix (Prabhakaran, 1990); PRAM900103 Relative frequency in beta-sheet (Prabhakaran, 1990); PRAM900104 Relative frequency in reverse-turn (Prabhakaran, 1990); PTI0830101 Helix-coil equilibrium constant (Ptitsyn-Finkelstein, 1983); PTIO830102 Beta-coil equilibrium constant (Ptitsyn-Finkelstein, 1983); QIAN880101 Weights for alpha-helix at the window position of -6 (Qian-Sejnowski, 1988); QIAN880102 Weights for alpha-helix at the window position of -5 (Qian-Sejnowski, 1988); QIAN880103 Weights for alpha-helix at the window position of -4 (Qian-Sejnowski, 1988); QIAN880104 Weights for alpha-helix at the window position of -3 (Qian-Sejnowski, 1988); QIAN880105 Weights for alpha-helix at the window position of -2 (Qian-Sejnowski, 1988); QIAN880106 Weights for alpha-helix at the window position of -1 (Qian-Sejnowski, 1988); QIAN880107 Weights for alpha-helix at the window position of 0 (Qian-Sejnowski, 1988); QIAN880108 Weights for alpha-helix at the window position of 1 (Qian-Sejnowski, 1988); QIAN880109 Weights for alpha-helix at the window position of 2 (Qian-Sejnowski, 1988); QIAN880110 Weights for alpha-helix at the window position of 3 (Qian-Sejnowski, 1988); QIAN880111 Weights for alpha-helix at the window position of 4 (Qian-Sejnowski, 1988); QIAN880112 Weights for alpha-helix at the window position of 5 (Qian-Sejnowski, 1988); QIAN880113 Weights for alpha-helix at the window position of 6 (Qian-Sejnowski, 1988); QIAN880114 Weights for beta-sheet at the window position of -6 (Qian-Sejnowski, 1988); QIAN880115 Weights for beta-sheet at the window position of -5 (Qian-Sejnowski, 1988); QIAN880116 Weights for beta-sheet at the window position of -4 (Qian-Sejnowski, 1988); QIAN880117 Weights for beta-sheet at the window position of-3 (Qian-Sejnowski, 1988); QIAN880118 Weights for beta-sheet at the window position of -2 (Qian-Sejnowski, 1988); QIAN880119 Weights for beta-sheet at the window position of -1 (Qian-Sejnowski, 1988); QIAN880120 Weights for beta-sheet at the window position of 0 (Qian-Sejnowski, 1988); QIAN880121 Weights for beta-sheet at the window position of 1 (Qian-Sejnowski, 1988); QIAN880122 Weights for beta-sheet at the window position of 2 (Qian-Sejnowski, 1988); QIAN880123 Weights for beta-sheet at the window position of 3 (Qian-Sejnowski, 1988); QIAN880124 Weights for beta-sheet at the window position of 4 (Qian-Sejnowski, 1988); QIAN880125 Weights for beta-sheet at the window position of 5 (Qian-Sejnowski, 1988); QIAN880126 Weights for beta-sheet at the window position of 6 (Qian-Sejnowski, 1988); QIAN880127 Weights for coil at the window position of -6 (Qian-Sejnowski, 1988); QIAN880128 Weights for coil at the window position of-5 (Qian-Sejnowski, 1988); QIAN880129 Weights for coil at the window position of -4 (Qian-Sejnowski, 1988); QIAN880130 Weights for coil at the window position of -3 (Qian-Sejnowski, 1988); QIAN880131 Weights for coil at the window position of -2 (Qian-Sejnowski, 1988); QIAN880132 Weights for coil at the window position of -1 (Qian-Sejnowski, 1988); QIAN880133 Weights for coil at the window position of 0 (Qian-Sejnowski, 1988); QIAN880134 Weights for coil at the window position of 1 (Qian-Sejnowski, 1988); QIAN880135 Weights for coil at the window position of 2 (Qian-Sejnowski, 1988); QIAN880136 Weights for coil at the window position of 3 (Qian-Sejnowski, 1988); QIAN880137 Weights for coil at the window position of 4 (Qian-Sejnowski, 1988); QIAN880138 Weights for coil at the window position of 5 (Qian-Sejnowski, 1988); QIAN880139 Weights for coil at the window position of 6 (Qian-Sejnowski, 1988); RACS770101 Average reduced distance for C-alpha (Rackovsky-Scheraga, 1977); RACS770102 Average reduced distance for side chain (Rackovsky-Scheraga, 1977); RACS770103 Side chain orientational preference (Rackovsky-Scheraga, 1977); RACS820101 Average relative fractional occurrence in A0(i) (Rackovsky-Scheraga, 1982); RACS820102 Average relative fractional occurrence in AR(i) (Rackovsky-Scheraga, 1982); RACS820103 Average relative fractional occurrence in AL(i) (Rackovsky-Scheraga, 1982); RACS820104 Average relative fractional occurrence in EL(i) (Rackovsky-Scheraga, 1982); RACS820105 Average relative fractional occurrence in E0(i) (Rackovsky-Scheraga, 1982); RACS820106 Average relative fractional occurrence in ER(i) (Rackovsky-Scheraga, 1982); RACS820107 Average relative fractional occurrence in AO(i-1) (Rackovsky-Scheraga, 1982); RACS820108 Average relative fractional occurrence in AR(i-1) (Rackovsky-Scheraga, 1982); RACS820109 Average relative fractional occurrence in AL(i-1) (Rackovsky-Scheraga, 1982); RACS820110 Average relative fractional occurrence in EL(i-1) (Rackovsky-Scheraga, 1982); RACS820111 Average relative fractional occurrence in E0(i-1) (Rackovsky-Scheraga, 1982); RACS820112 Average relative fractional occurrence in ER(i-1) (Rackovsky-Scheraga, 1982); RACS820113 Value oftheta(i) (Rackovsky-Scheraga, 1982); RACS820114 Value oftheta(i-1) (Rackovsky-Scheraga, 1982); RADA880101 Transfer free energy from chx to wat (Radzicka-Wolfenden, 1988); RADA880102 Transfer free energy from oct to wat (Radzicka-Wolfenden, 1988); RADA880103 Transfer free energy from vap to chx (Radzicka-Wolfenden, 1988); RADA880104 Transfer free energy from chx to oct (Radzicka-Wolfenden, 1988); RADA880105 Transfer free energy from vap to oct (Radzicka-Wolfenden, 1988); RADA880106 Accessible surface area (Radzicka-Wolfenden, 1988); RADA880107 Energy transfer from out to in(95%buried) (Radzicka-Wolfenden, 1988); RADA880108 Mean polarity (Radzicka-Wolfenden, 1988); RICJ880101 Relative preference value at N" (Richardson-Richardson, 1988); RICJ880102 Relative preference value at N' (Richardson-Richardson, 1988); RICJ880103 Relative preference value at N-cap (Richardson-Richardson, 1988); RICJ880104 Relative preference value at N1 (Richardson-Richardson, 1988); RICJ880105 Relative preference value at N2 (Richardson-Richardson, 1988); RICJ880106 Relative preference value at N3 (Richardson-Richardson, 1988); RICJ880107 Relative preference value at N4 (Richardson-Richardson, 1988); RICJ880108 Relative preference value at N5 (Richardson-Richardson, 1988); RICJ880109 Relative preference value at Mid (Richardson-Richardson, 1988); RICJ880110 Relative preference value at C5 (Richardson-Richardson, 1988); RICJ880111 Relative preference value at C4 (Richardson-Richardson, 1988); RICJ880112 Relative preference value at C3 (Richardson-Richardson, 1988); RICJ880113 Relative preference value at C2 (Richardson-Richardson, 1988); RICJ880114 Relative preference value at C1 (Richardson-Richardson, 1988); RICJ880115 Relative preference value at C-cap (Richardson-Richardson, 1988); RICJ880116 Relative preference value at C' (Richardson-Richardson, 1988); RICJ880117 Relative preference value at C" (Richardson-Richardson, 1988); ROBB760101 Information measure for alpha-helix (Robson-Suzuki, 1976); ROBB760102 Information measure for N-terminal helix (Robson-Suzuki, 1976); ROBB760103 Information measure for middle helix (Robson-Suzuki, 1976); ROBB760104 Information measure for C-terminal helix (Robson-Suzuki, 1976); ROBB760105 Information measure for extended (Robson-Suzuki, 1976); ROBB760106 Information measure for pleated-sheet (Robson-Suzuki, 1976); ROBB760107 Information measure for extended without H-bond (Robson-Suzuki, 1976); ROBB760108 Information measure for turn (Robson-Suzuki, 1976); ROBB760109 Information measure for N-terminal turn (Robson-Suzuki, 1976); ROBB760110 Information measure for middle turn (Robson-Suzuki, 1976); ROBB760111 Information measure for C-terminal turn (Robson-Suzuki, 1976); ROBB760112 Information measure for coil (Robson-Suzuki, 1976); ROBB760113 Information measure for loop (Robson-Suzuki, 1976); ROBB790101 Hydration free energy (Robson-Osguthorpe, 1979); ROSG850101 Mean area buried on transfer (Rose et al., 1985); ROSG850102 Mean fractional area loss (Rose et al., 1985); ROSM880101 Side chain hydropathy, uncorrected for solvation (Roseman, 1988); ROSM880102 Side chain hydropathy, corrected for solvation (Roseman, 1988); ROSM880103 Loss of Side chain hydropathy by helix formation (Roseman, 1988); SIMZ760101 Transfer free energy (Simon, 1976), Cited by Charton-Charton (1982); SNEP660101 Principal component I (Sneath, 1966); SNEP660102 Principal component II (Sneath, 1966); SNEP660103 Principal component III (Sneath, 1966); SNEP660104 Principal component IV (Sneath, 1966); SUEM840101 Zimm-Bragg parameter s at 20 C (Sueki et al., 1984); SUEM840102 Zimm-Bragg parameter sigma x 1.0E4 (Sueki et al., 1984); SWER830101 Optimal matching hydrophobicity (Sweet-Eisenberg, 1983); TANS770101 Normalized frequency of alpha-helix (Tanaka-Scheraga, 1977); TANS770102 Normalized frequency of isolated helix (Tanaka-Scheraga, 1977); TANS770103 Normalized frequency of extended structure (Tanaka-Scheraga, 1977); TANS770104 Normalized frequency of chain reversal R (Tanaka-Scheraga, 1977); TANS770105 Normalized frequency of chain reversal S (Tanaka-Scheraga, 1977); TANS770106 Normalized frequency of chain reversal D (Tanaka-Scheraga, 1977); TANS770107 Normalized frequency of left-handed helix (Tanaka-Scheraga, 1977); TANS770108 Normalized frequency of zeta R (Tanaka-Scheraga, 1977); TANS770109 Normalized frequency of coil (Tanaka-Scheraga, 1977)
TANS770110 Normalized frequency of chain reversal (Tanaka-Scheraga, 1977); VASM830101 Relative population of conformational state A (Vasquez et al., 1983); VASM830102 Relative population of conformational state C (Vasquez et al., 1983); VASM830103 Relative population of conformational state E (Vasquez et al., 1983); VELV850101 Electron-ion interaction potential (Veljkovic et al., 1985); VENT840101 Bitterness (Venanzi, 1984); VHEG790101 Transfer free energy to lipophilic phase (von Heijne-Blomberg, 1979); WARP780101 Average interactions per side chain atom (Warme-Morgan, 1978); WEBA780101 RF value in high salt chromatography (Weber-Lacey, 1978); WERD780101 Propensity to be buried inside (Wertz-Scheraga, 1978); WERD780102 Free energy change of epsilon(i) to epsilon(ex) (Wertz-Scheraga, 1978); WERD780103 Free energy change of alpha(Ri) to alpha(Rh) (Wertz-Scheraga, 1978); WERD780104 Free energy change of epsilon(i) to alpha(Rh) (Wertz-Scheraga, 1978); WOEC730101 Polar requirement (Woese, 1973); WOLR810101 Hydration potential (Wolfenden et al., 1981); WOLS870101 Principal property value z1 (Wold et al., 1987); WOLS870102 Principal property value z2 (Wold et al., 1987); WOLS870103 Principal property value z3 (Wold et al., 1987); YUTK870101 Unfolding Gibbs energy in water, pH7.0 (Yutani et al., 1987); YUTK870102 Unfolding Gibbs energy in water, pH9.0 (Yutani et al., 1987); YUTK870103 Activation Gibbs energy of unfolding, pH7.0 (Yutani et al., 1987); YUTK870104 Activation Gibbs energy of unfolding, pH9.0 (Yutani et al., 1987); ZASB820101 Dependence of partition coefficient on ionic strength (Zaslavsky et al., 1982); ZIMJ680101 Hydrophobicity (Zimmerman et al., 1968); ZIMJ680102 Bulkiness (Zimmerman et al., 1968); ZIMJ680103 Polarity (Zimmerman et al., 1968); ZIMJ680104 Isoelectric point (Zimmerman et al., 1968); ZIMJ680105 RF rank (Zimmerman et al., 1968); AURR980101 Normalized positional residue frequency at helix termini N4'(Aurora-Rose, 1998); AURR980102 Normalized positional residue frequency at helix termini N'" (Aurora-Rose, 1998); AURR980103 Normalized positional residue frequency at helix termini N" (Aurora-Rose, 1998); AURR980104 Normalized positional residue frequency at helix termini N'(Aurora-Rose, 1998); AURR980105 Normalized positional residue frequency at helix termini Nc (Aurora-Rose, 1998); AURR980106 Normalized positional residue frequency at helix termini N1 (Aurora-Rose, 1998); AURR980107 Normalized positional residue frequency at helix termini N2 (Aurora-Rose, 1998); AURR980108 Normalized positional residue frequency at helix termini N3 (Aurora-Rose, 1998); AURR980109 Normalized positional residue frequency at helix termini N4 (Aurora-Rose, 1998); AURR980110 Normalized positional residue frequency at helix termini N5 (Aurora-Rose, 1998); AURR980111 Normalized positional residue frequency at helix termini C5 (Aurora-Rose, 1998); AURR980112 Normalized positional residue frequency at helix termini C4 (Aurora-Rose, 1998); AURR980113 Normalized positional residue frequency at helix termini C3 (Aurora-Rose, 1998); AURR980114 Normalized positional residue frequency at helix termini C2 (Aurora-Rose, 1998); AURR980115 Normalized positional residue frequency at helix termini C1 (Aurora-Rose, 1998); AURR980116 Normalized positional residue frequency at helix termini Cc (Aurora-Rose, 1998); AURR980117 Normalized positional residue frequency at helix termini C' (Aurora-Rose, 1998); AURR980118 Normalized positional residue frequency at helix termini C" (Aurora-Rose, 1998); AURR980119 Normalized positional residue frequency at helix termini C'" (Aurora-Rose, 1998); AURR980120 Normalized positional residue frequency at helix termini C4' (Aurora-Rose, 1998); ONEK900101 Delta G values for the peptides extrapolated to 0 M urea (O'Neil-DeGrado, 1990); ONEK900102 Helix formation parameters (delta delta G) (O'Neil-DeGrado, 1990); VINM940101 Normalized flexibility parameters (B-values), average (Vihinen et al., 1994); VINM940102 Normalized flexibility parameters (B-values) for each residue surrounded by none rigid neighbours (Vihinen et al., 1994); VINM940103 Normalized flexibility parameters (B-values) for each residue surrounded by one rigid neighbours (Vihinen et al., 1994); VINM940104 Normalized flexibility parameters (B-values) for each residue surrounded by two rigid neighbours (Vihinen et al., 1994); MUNV940101 Free energy in alpha-helical conformation (Munoz-Serrano, 1994); MUNV940102 Free energy in alpha-helical region (Munoz-Serrano, 1994); MUNV940103 Free energy in beta-strand conformation (Munoz-Serrano, 1994); MUNV940104 Free energy in beta-strand region (Munoz-Serrano, 1994); MUNV940105 Free energy in beta-strand region (Munoz-Serrano, 1994)
WIMW960101 Free energies of transfer of AcWl-X-LL peptides from bilayer interface to water (Wimley-White, 1996); KIMC930101 Thermodynamic beta sheet propensity (Kim-Berg, 1993); MONM990101 Turn propensity scale for transmembrane helices (Monne et al., 1999); BLAM930101 Alpha helix propensity of position 44 in T4 lysozyme (Blaber et al., 1993); PARS000101 p-Values of mesophilic proteins based on the distributions of B values (Parthasarathy-Murthy, 2000); PARS000102 p-Values of thermophilic proteins based on the distributions of B values (Parthasarathy-Murthy, 2000); KUMS000101 Distribution of amino acid residues in the 18 non-redundant families of thermophilic proteins (Kumar et al., 2000); KUMS000102 Distribution of amino acid residues in the 18 non-redundant families of mesophilic proteins (Kumar et al., 2000); KUMS000103 Distribution of amino acid residues in the alpha-helices in thermophilic proteins (Kumar et al., 2000); KUMS000104 Distribution of amino acid residues in the alpha-helices in mesophilic proteins (Kumar et al., 2000); TAKK010101 Side-chain contribution to protein stability (kJ/mol) (Takano-Yutani, 2001); FODM020101 Propensity of amino acids within pi-helices (Fodje-Al-Karadaghi, 2002); NADH010101 Hydropathy scale based on self-information values in the two-state model (5% accessibility) (Naderi-Manesh et al., 2001); NADH010102 Hydropathy scale based on self-information values in the two-state model (9% accessibility) (Naderi-Manesh et al., 2001); NADH010103 Hydropathy scale based on self-information values in the two-state model (16% accessibility) (Naderi-Manesh et al., 2001); NADH010104 Hydropathy scale based on self-information values in the two-state model (20% accessibility) (Naderi-Manesh et al., 2001); NADH010105 Hydropathy scale based on self-information values in the two-state model (25% accessibility) (Naderi-Manesh et al., 2001); NADH010106 Hydropathy scale based on self-information values in the two-state model (36% accessibility) (Naderi-Manesh et al., 2001); NADH010107 Hydropathy scale based on self-information values in the two-state model (50% accessibility) (Naderi-Manesh et al., 2001); MONM990201 Averaged turn propensities in a transmembrane helix (Monne et al., 1999); KOEP990101 Alpha-helix propensity derived from designed sequences (Koehl-Levitt, 1999); KOEP990102 Beta-sheet propensity derived from designed sequences (Koehl-Levitt, 1999); CEDJ970101 Composition of amino acids in extracellular proteins (percent) (Cedano et al., 1997); CEDJ970102 Composition of amino acids in anchored proteins (percent) (Cedano et al., 1997); CEDJ970103 Composition of amino acids in membrane proteins (percent) (Cedano et al., 1997); CEDJ970104 Composition of amino acids in intracellular proteins (percent) (Cedano et al., 1997); CEDJ970105 Composition of amino acids in nuclear proteins (percent) (Cedano et al., 1997); FUKS0 10101 Surface composition of amino acids in intracellular proteins of thermophiles (percent) (Fukuchi-Nishikawa, 2001); FUKS010102 Surface composition of amino acids in intracellular proteins of mesophiles (percent) (Fukuchi-Nishikawa, 2001); FUKS010103 Surface composition of amino acids in extracellular proteins of mesophiles (percent) (Fukuchi-Nishikawa, 2001); FUKS010104 Surface composition of amino acids in nuclear proteins (percent) (Fukuchi-Nishikawa, 2001); FUKS010105 Interior composition of amino acids in intracellular proteins of thermophiles (percent) (Fukuchi-Nishikawa, 2001); FUKS010106 Interior composition of amino acids in intracellular proteins of mesophiles (percent) (Fukuchi-Nishikawa, 2001); FUKS010107 Interior composition of amino acids in extracellular proteins of mesophiles (percent) (FukuchrNishikawa, 2001); FUKS010108 Interior composition of amino acids in nuclear proteins (percent) (Fukuchi-Nishikawa, 2001); FUKS010109 Entire chain composition of amino acids in intracellular proteins of thermophiles (percent) (Fukuchi-Nishikawa, 2001); FUKS010110 Entire chain composition of amino acids in intracellular proteins of mesophiles (percent) (Fukuchi-Nishikawa, 2001); FUKS010111 Entire chain composition of amino acids in extracellular proteins of mesophiles (percent) (Fukuchi-Nishikawa, 2001); FUKS010112 Entire chain compositino of amino acids in nuclear proteins (percent) (Fukuchi-Nishikawa, 2001); AVBF000101 Screening coefficients gamma, local (Avbelj, 2000); AVBF000102 Screening coefficients gamma, non-local (Avbelj, 2000); AVBF000103 Slopes tripeptide, FDPB VFF neutral (Avbelj, 2000); AVBF000104 Slopes tripeptides, LD VFF neutral (Avbelj, 2000); AVBF000105 Slopes tripeptide, FDPB VFF noside (Avbelj, 2000); AVBF000106 Slopes tripeptide FDPB VFF all (Avbelj, 2000); AVBF000107 Slopes tripeptide FDPB PARSE neutral (Avbelj, 2000); AVBF000108 Slopes dekapeptide, FDPB VFF neutral (Avbelj, 2000); AVBF000109 Slopes proteins, FDPB VFF neutral (Avbelj, 2000); YANJ020101 Side-chain conformation by gaussian evolutionary method (Yang et al., 2002); MITS020101 Amphiphilicity index (Mitaku et al., 2002); TSAJ990101 Volumes including the crystallographic waters using the ProtOr (Tsai et al., 1999); TSAJ990102 Volumes not including the crystallographic waters using the ProtOr (Tsai et al., 1999); COSI940101 Electron-ion interaction potential values (Cosic, 1994); PONP930101 Hydrophobicity scales (Ponnuswamy, 1993); WILM950101 Hydrophobicity coefficient in RP-HPLC, C18 with 0.1%TFA/MeCN/H2O (Wilce et al. 1995); WILM950102 Hydrophobicity coefficient in RP-HPLC, C8 with 0.1%TFA/MeCN/H2O (Wilce et al. 1995); WILM950103 Hydrophobicity coefficient in RP-HPLC, C4 with 0.1%TFA/MeCN/H2O (Wilce et al. 1995); WILM950104 Hydrophobicity coefficient in RP-HPLC, C18 with 0.1%TFA/2-PrOH/MeCN/H2O (Wilce et al. 1995); KUHL950101 Hydrophilicity scale (Kuhn et al., 1995); GUOD860101 Retention coefficient at pH 2 (Guo et al., 1986); JURD980101 Modified Kyte-Doolittle hydrophobicity scale (Juretic et al., 1998); BASU050101 Interactivity scale obtained from the contact matrix (Bastolla et al., 2005); BASU050102 Interactivity scale obtained by maximizing the mean of correlation coefficient over single-domain globular proteins (Bastolla et al., 2005); BASU050103 Interactivity scale obtained by maximizing the mean of correlation coefficient over pairs of sequences sharing the TIM barrel fold (Bastolla et al., 2005); SUYM030101 Linker propensity index (Suyama-Ohara, 2003); PUNT030101 Knowledge-based membrane-propensity scale from 1D_elix in MPtopo databases (Punta-Maritan, 2003); PUNT030102 Knowledge-based membrane-propensity scale from 3D_Helix in MPtopo databases (Punta-Maritan, 2003); GEOR030101 Linker propensity from all dataset (George-Heringa, 2003); GEOR030102 Linker propensity from 1-linker dataset (George-Heringa, 2003); GEOR030103 Linker propensity from 2-linker dataset (George-Heringa, 2003); GEOR030104 Linker propensity from 3-linker dataset (George-Heringa, 2003); GEOR030105 Linker propensity from small dataset (linker length is less than six residues) (George-Heringa, 2003); GEOR030106 Linker propensity from medium dataset (linker length is between six and 14 residues) (George-Heringa, 2003); GEOR030107 Linker propensity from long dataset (linker length is greater than 14 residues) (George-Heringa, 2003); GEOR030108 Linker propensity from helical (annotated by DSSP) dataset (George-Heringa, 2003); GEOR030109 Linker propensity from non-helical (annotated by DSSP) dataset (George-Heringa, 2003); ZHOH040101 The stability scale from the knowledge-based atom-atom potential (Zhou-Zhou, 2004); ZHOH040102 The relative stability scale extracted from mutation experiments (Zhou-Zhou, 2004); ZHOH040103 Buriability (Zhou-Zhou, 2004); BAEK050101 Linker index (Bae et al., 2005); HARY940101 Mean volumes of residues buried in protein interiors (Harpaz et al., 1994); PONJ960101 Average volumes of residues (Pontius et al., 1996); DIGM050101 Hydrostatic pressure asymmetry index, PAI (Di Giulio, 2005); WOLR790101 Hydrophobicity index (Wolfenden et al., 1979); OLSK800101 Average internal preferences (Olsen, 1980); KIDA850101 Hydrophobicity-related index (Kidera et al., 1985); GUYH850102 Apparent partition energies calculated from Wertz-Scheraga index (Guy, 1985); GUYH850103 Apparent partition energies calculated from Robson-Osguthorpe index (Guy, 1985); GUYH850104 Apparent partition energies calculated from Janin index (Guy, 1985); GUYH850105 Apparent partition energies calculated from Chothia index (Guy, 1985); ROSM880104 Hydropathies of amino acid side chains, neutral form (Roseman, 1988); ROSM880105 Hydropathies of amino acid side chains, pi-values in pH 7.0 (Roseman, 1988); JACR890101 Weights from the IFH scale (Jacobs-White, 1989); COWR900101 Hydrophobicity index, 3.0 pH (Cowan-Whittaker, 1990)
BLAS910101 Scaled side chain hydrophobicity values (Black-Mould, 1991); CASG920101 Hydrophobicity scale from native protein structures (Casari-Sippl, 1992); CORJ870101 NNEIG index (Cornette et al., 1987); CORJ870102 SWEIG index (Cornette et al., 1987); CORJ870103 PRIFT index (Cornette et al., 1987); CORJ870104 PRILS index (Cornette et al., 1987); CORJ870105 ALTFT index (Cornette et al., 1987) CORJ870106 ALTLS index (Cornette et al., 1987); CORJ870107 TOTFT index (Cornette et al., 1987); CORJ870108 TOTLS index (Cornette et al., 1987); MIYS990101 Relative partition energies derived by the Bethe approximation (Miyazawa-Jernigan, 1999); MIYS990102 Optimized relative partition energies - method A (Miyazawa-Jernigan, 1999); MIYS990103 Optimized relative partition energies - method B (Miyazawa-Jernigan, 1999); MIYS990104 Optimized relative partition energies - method C (Miyazawa-Jernigan, 1999); MIYS990105 Optimized relative partition energies - method D (Miyazawa-Jernigan, 1999); ENGD860101 Hydrophobicity index (Engelman et al., 1986); and FASG890101 Hydrophobicity index (Fasman, 1989)

In some embodiments of the invention, degenerate oligonucleotides are used to synthesize one or more of the TN1, DH, N2, and/or H3-JH segments of the invention. In certain embodiments of the invention, the codon at or near the 5' end of the oligonucleotide encoding the H3-JH segment is a degenerate codon. Such degenerate codons may be the first codon from the 5' end, the second codon from the 5' end, the third codon from the 5' end, the fourth codon from the 5' end, the fifth codon from the 5' end, and/or any combination of the above. In some embodiments of the invention, one or more of the codons at or near the 5' and/or 3' ends of the DH segment are degenerate. Such degenerate codons may be the first codon from the 5' and/or 3' end(s), the second codon from the 5' and/or 3' end(s), the third codons from the 5' and/or 3' end(s), the fourth codon from the 5' and/or 3' end(s), the fifth codon from the 5' and/or 3' end(s), and/or any combination of the above. Degenerate codons used in each of the oligonucleotides encoding the segments may be selected for their ability to optimally recapitulate sequences in a theoretical segment pool and/or CDRH3 reference set.

In some embodiments, the invention provides methods of producing a theoretical segment pool of H3-JH segments, as described in the Examples. Theoretical segment pools generated utilizing NNN triplets, instead of or in addition to the NN doublets described in Example 5 also fall within the scope of the invention, as do synthetic libraries incorporating segments from these theoretical segment pools.

In some embodiments, the invention provides methods of producing a theoretical segment pool of DH segments, as described in the Examples. In particular, for example, the invention provides methods of producing a theoretical segment pool of DH segments described by the PYTHON program of Example 6. Example 6 describes the application of this program to produce the 68K theoretical segment pool (minimum length of DNA sequences after progressive deletions = 4 bases; and minimum length ofpeptide sequences for inclusion in the theoretical segment pool = 2). An alternative example is provided wherein the minimum length of the DNA sequences after progressive deletions was one base and the minimum length of the peptide sequence is one amino acid. It is also contemplated that other values could be used for these parameters. For example, the minimum length of the DNA sequences after progressive deletions could be set as 1, 2, 3, 4, 5, 6, 7, 8, 9, 10, 11, 12, 13, 14, or 15, and the minimum length of the peptide sequences in the theoretical segment pool could be set as 1, 2, 3, 4, or 5.

### Design of CDRH3 Libraries Using the TN1, DH, N2, and H3-JH Segments

The CDRH3 libraries of the invention comprise TN1, DH, N2, and H3-JH segments. Thus, in certain embodiments of the invention, the overall design of the CDRH3 libraries can be represented by the following formula:

[TN1]-[DH]-[N2]-[H3-JH].

In certain embodiments of the invention, a synthetic CDRH3 repertoire is combined with selected VH chassis sequences and heavy chain constant regions, via homologous recombination. Therefore, in certain embodiments of the invention, it may be desirable to include DNA sequences flanking the 5' and 3' ends of the synthetic CDRH3 libraries, to facilitate homologous recombination between the synthetic CDRH3 libraries and vectors containing the selected chassis and constant regions. In certain embodiments, the vectors also contain a sequence encoding at least a portion of the non-truncated region of the IGHJ gene (*i.e.,* FRM4-JH). Thus, a polynucleotide encoding an N-terminal sequence (*e.g.,* CA(K/R/T)) may be added to the synthetic CDRH3 sequences, wherein the N-terminal polynucleotide is homologous with FRM3 of the chassis, while a polynucleotide encoding a C-terminal sequence (*e.g.,* WG(Q/R/K)G) may be added to the synthetic CDRH3, wherein the C-terminal polynucleotide is homologous with FRM4-JH. Although the sequence WG(Q/R)G is presented in this exemplary embodiment, additional amino acids, Terminal to this sequence in FRM4-JH may also be included in the polynucleotide encoding the Terminal sequence. The purpose of the polynucleotides encoding the N-terminal and C-terminal sequences, in this case, is to facilitate homologous recombination, and one of ordinary skill in the art would recognize that these sequences may be longer or shorter than depicted below. Accordingly, in certain embodiments of the invention, the overall design of the CDRH3 repertoire, including the sequences required to facilitate homologous recombination with the selected chassis, can be represented by the following formula (regions homologous with vector underlined):

CA[R/K/T]-[TN1]-[DH]-[N2]-[H3-JH]-[WG(Q/R/K)G].

In some embodiments of the invention, the CDRH3 repertoire can be represented by the following formula, which excludes the T residue presented in the schematic above:

CA[R/K]-[TN1]-[DH]-[N2]-[H3-JH]-[WG(Q/R/K)G].

References describing collections of V, D, and J genes include Scaviner *et al.,* Exp. Clin, Immunogenet., 1999, 16: 243 and Ruiz *et al.,* Exp. Clin. Immunogenet, 1999, 16: 173, each incorporated by reference in its entirety.

Although homologous recombination is one method of producing the libraries of the invention, a person of ordinary skill in the art will readily recognize that other methods of DNA assembly, such as ligation or site-specific recombination, and/or DNA synthesis, can also be used to produce the libraries of the invention.

### CDRH3 Lengths

The lengths of the segments may also be varied, for example, to produce libraries with a particular distribution of CDRH3 lengths. In one embodiment of the invention, the H3-JH segments are about 0 to about 10 amino acids in length, the DH segments are about 0 to about 12 amino acids in length, the TN1 segments are about 0 to about 4 amino acids in length, and the N2 segments are about 0 to about 4 amino acids in length. In certain embodiments, the H3-JH segments are at least about 0, 1, 2, 3, 4, 5, 6, 7, 8, 9, and/or 10 amino acids in length. In some embodiments, the DH segments are at least about 0, 1, 2, 3, 4, 5, 6, 7, 8, 9, 10, 11, and/or 12 amino acids in length. In certain embodiments, the TN1 segments are at least about 0, 1, 2, 3, or 4 amino acids in length. In some embodiments, the N2 amino acids are at least about 0, 1, 2, 3, or 4 amino acids in length. In certain embodiments of the invention, the CDRH3 is about 2 to about 35, about 2 to about 28, or about 5 to about 26 amino acids in length. In some embodiments, the CDRH3 is at least about 2, 3, 4, 5,6, 7, 8, 9, 10, 11, 12, 13, 14, 15, 16, 17, 18, 19, 20, 21, 22, 23, 24, 25, 26, 27, 28, 29, 30, 31, 32, 33, 34, and/or 35 amino acids in length. In some embodiments, the length of any of the segments or CDRH3s of the invention may be less than a particular number of amino acids, where the number of amino acids is defined using any one of the integers provided above for the respective segment or CDRH3. In certain embodiments of the invention, a particular numerical range is defined, using any two of the integers provided above as lower and upper boundaries of the range, inclusive or exclusive. All combinations of the integers provided, which define an upper and lower boundary, are contemplated.

### Design of CDRL3 Libraries

The design of CDRL3 libraries, and light chain sequences, is described in detail in U.S. Publication Nos. 2009/0181855 and 2010/0056386, and WO/2009/036379, each of which is incorporated by reference in its entirety, and is therefore only described briefly herein. Libraries described herein are designed according to similar principles, with three important differences, namely that the libraries of the current invention contain (1) variability in CDRL1 and CDRL2; (2) variability in the framework regions; and/or (3) variability in CDRL3 that is designed to produce light chain libraries with CDRL3s that closely resemble human germline-like CDRL3 sequences, as defined above (Table 1).

A CDRL3 library of the invention may be a VKCDR3 library and/or a VλCDR3 library. In certain embodiments of the invention, patterns of occurrence of particular amino acids at defined positions within VL sequences are determined by analyzing data available in public or other databases, for example, the NCBI database (see, for example, WO/2009/036379). In certain embodiments of the invention, these sequences are compared on the basis of identity and assigned to families on the basis of the germline genes from which they are derived. The amino acid composition at each position of the sequence, in each germline family, may then be determined. This process is illustrated in the Examples provided herein.

### Light Chains with Framework Variability

In some embodiments, the invention provides a library of light chain variable domains wherein the light chain variable domains are varied at one or more of framework positions 2, 4, 36, 46, 48, 49, and 66. In some embodiments, the invention provides a library of light chain variable domains comprising at least a plurality of light chain variable domains whose amino acid sequences are identical to one another except for substitutions at one or more of positions 2, 4, 36, 46, 48, 49, and 66. In certain embodiments, the invention provides a library of light chain variable domains comprising at least a plurality of light chain variable domains whose amino acid sequences are at least about 70%, 75%, 80%, 85%, 90%, 95%, 96%, 97%, 98%, 99%, and/or 99.5% to any of the light chain variable domain sequences disclosed herein, and further have substitutions at one or more of positions 2, 4, 36, 46, 48, 49, and 66. In some embodiments, the amino acids selected for inclusion in these positions are selected from amongst about the most 2, 3, 4, 5, 6, 7, 8, 9, and/or 10 most frequently occurring amino acids at the corresponding position in a reference set of light chain variable domains.

In some embodiments, the invention provides systems and methods of selecting framework positions to be varied in a light chain variable domain, comprising:
(i) obtaining a reference set of light chain sequences, wherein the reference set contains light chain sequences with VL segments selected from the group consisting of sequences found in, or encoded by, a single IGVL germline gene and/or sequences found in, or encoded by, allelic variants of the single IGVL germline gene;
(ii) determining which framework positions within the reference set have a degree of variability that is similar to the degree of variability occurring in one more CDR positions of the sequences in the reference set (*e.g.,* the variability in a framework position is at least about 70%, 80%, 90%, or 95%, 100%, or more of the variability found in a CDR position of the sequences in the reference set);
(iii) determining the frequency of occurrence of amino acid residues for each of the framework positions identified in (ii);
(iv) synthesizing light chain variable domain encoding sequences wherein the framework positions identified in (ii) are varied to include the 2, 3, 4, 5, 6, 7, 8, 9, 10, 11, 12, 13, 14, 15, 16, 17, 18, 19, or 20 most frequently occurring amino acid residues (identified in (iii)) at the corresponding position.

One of ordinary skill in the art, reading the present disclosure will appreciate that the present invention provides analogous methods for developing framework variants of heavy chain sequences.

### Light Chains with CDR1 and/or CDR2 Variability

In some embodiments, the invention provides a library of light chain variable domains wherein the light chain variable domains are varied at one or more of CDRL1 positions 28, 29, 30, 30A, 30B, 30E, 31, and 32 (Chothia-Lesk numbering scheme; Chothia and Lesk, J. Mol. Biol., 1987, 196: 901). In some embodiments, the invention provides a library of light chain variable domains wherein the light chain variable domains are varied at one or more of CDRL2 positions 50, 51, 53, and 55. In some embodiments, the amino acids selected for inclusion in these CDRL1 and/or CDRL2 positions are selected from amongst about the most 2, 3, 4, 5, 6, 7, 8, 9, 10, 11, 12, 13, 14, 15, 16, 17, 18, 19, and/or 20 most frequently occurring amino acids at the corresponding position in a reference set of light chain variable domains.

In some embodiments, the invention provides systems and methods for selecting CDRL1 and/or CDRL2 positions to be varied in a light chain variable domain, comprising:
(i) obtaining a reference set of light chain sequences, wherein the reference set contains light chain sequences with VL segments selected from the group consisting of sequences found in, or encoded by, a single IGVL germline gene and sequences found in, or encoded by, allelic variants of the single IGVL germline gene;
(ii) determining which CDRL1 and/or CDRL2 positions are variable within the reference set;
(iii) synthesizing light chain variable domain encoding sequences wherein the CDRL1 and/or CDRL2 positions identified in (ii) are varied to include the 2, 3, 4, 5, 6, 7, 8, 9, 10, 11, 12, 13, 14, 15, 16, 17, 18, 19, or 20 most frequently occurring amino acid residues at the corresponding position.

One of ordinary skill in the art, reading the present disclosure will appreciate that the present invention provides analogous methods for developing CDRH2 and/or CDRH2 variants of heavy chain sequences.

### Light Chain Sequences

In some embodiments, the invention provides a light chain library comprising one or more of any of the light chain sequences provided herein, for example, the polypeptide sequences of Table 3 and/or Table 4 and/or the polynucleotide sequences of Table 5, Table 6, and/or Table 7. A person of ordinary skill in the art will recognize that not every light chain sequence provided herein is necessary to produce a functional light chain library of the invention. Therefore, in certain embodiments, a light chain library of the invention will contain a subset of the sequences described above. For example, in certain embodiments of the invention, at least about 10, 100, 200, 300, 400, 500, 600, 700, 800, 900, 10³, 10⁴, and/or 10⁵ of the light chain polynucleotide and/or polypeptide sequences provided herein are included in a library. In some embodiments, a library of the invention may contain less than a particular number of polynucleotide or polypeptide segments, where the number of segments is defined using any one of the integers provided above for the respective segment. In certain embodiments of the invention, a particular numerical range is defined, using any two of the integers provided above as lower and upper boundaries of the range, inclusive or exclusive. All combinations of the integers provided, which define an upper and lower boundary, are contemplated.

In certain embodiments, the invention provides light chain libraries comprising at least about 1%, 2.5%, 5%, 10%, 15%, 20%, 25%, 30%, 40%, 45%, 50%, 55%, 60%, 65%, 70%, 75%, 80%, 85%, 90%, 95%, or 99% of the sequences from any of the sets of light chain sequences provided herein. For example, the invention provides libraries comprising at least about 1%, 2.5%, 5%, 10%, 15%, 20%, 25%, 30%, 40%, 45%, 50%, 55%, 60%, 65%, 70%, 75%, 80%, 85%, 90%, 95%, or 99% of the light chain sequences provided in Table 3, Table 4, Table 5, Table 6, and/or Table 7. In some embodiments of the invention, a particular percentage range is defined, using any two of the percentages provided above as lower and upper boundaries of the range, inclusive or exclusive. All combinations of the percentages provided, which define an upper and lower boundary, are contemplated.

In some embodiments of the invention, at least about 1%, 2.5%, 5%, 10%, 15%, 20%, 25%, 30%, 40%, 45%, 50%, 55%, 60%, 65%, 70%, 75%, 80%, 85%, 90%, 95%, or 99% of the light chain sequences in a library are light chain sequences provided herein. In certain embodiments of the invention, at least about 1%, 2.5%, 5%, 10%, 15%, 20%, 25%, 30%, 40%, 45%, 50%, 55%, 60%, 65%, 70%, 75%, 80%, 85%, 90%, 95%, or 99% of the light chain sequences isolated from a light chain library (*e*.*g*., by binding to a particular antigen and/or generic ligand) are light chain sequences provided herein. In some embodiments, a light chain library of the invention may contain less than a particular percentage of light chain sequences provided herein, where the percentage of light chain sequences is defined using any one of the percentages provided above. In certain embodiments of the invention, a particular percentage range is defined, using any two of the percentages provided above as lower and upper boundaries of the range, inclusive or exclusive. All combinations of the percentages provided, which define an upper and lower boundary, are contemplated.

One of ordinary skill in the art will further recognize that given the light chain sequences provided herein, similar light chain sequences could be produced which share a designated level of overall sequence identity and/or one or more characteristic sequence elements described herein, which overall degree of sequence identity and/or characteristic sequence elements may confer common functional attributes. Those of ordinary skill in the art will be well familiar with a variety of techniques for preparing such related sequences, , including the mutagenesis techniques provided herein. Therefore, each of the explicitly enumerated embodiments of the invention can also be practiced using light chain sequences that share a particular percent identity to any of the light chain sequences provided herein. For example, each of the previously described embodiments of the invention can be practiced using light chain sequences that are at least about 70%, 75%, 80%, 85%, 90%, 91%, 92%, 93%, 94%, 95%, 96%, 97%, 98%, 99%, 99.5%, or 99.9% identical to the light chain sequences provided herein. For example, in some embodiments, light chain libraries provided by the invention comprise light chain variable domains at least about 70%, 75%, 80%, 85%, 90%, 91%, 92%, 93%, 94%, 95%, 96%, 97%, 98%, 99%, 99.5%, or 99.9% identical to the light chain sequences provided herein, with substitutions in one or more of framework positions 2, 4, 36, 46, 48, 49, and 66, CDRL1 positions 28, 29, 30, 30A, 30B, 30E, 31, and 32 (Chothia-Lesk numbering scheme), and/or CDRL2 positions 50, 51, 53, and 55.

In some embodiments, the invention provides systems and methods for varying positions within the portion of CDRL3s encoded by a particular IGVL germline gene, comprising:
(i) obtaining a reference set of light chain sequences, wherein the reference set contains light chain sequences with VL segments originating from the same IGVL germline gene and/or its allelic variants;
(ii) determining which amino acids occur at each of the CDRL3 positions in the reference set that are encoded by the IGVL gene (*i.e.,* positions 89-94, inclusive);
(iii) synthesizing light chain variable domain encoding sequences wherein two positions in each light chain variable domain encoding sequence contain degenerate codons encoding the 2, 3, 4, 5, 6, 7, 8, 9, 10, 11, 12, 13, 14, 15, 16, 17, 18, 19, or 20 most frequently occurring amino acid residues at the corresponding positions in the reference set.

As described in the examples, the degenerate codons of (iii) can be chosen to best reproduce the amino acid diversity contained in the reference set for each of the two positions varied in each light chain. Finally, while the methods and systems described above are described with respect to CDRL3, one of ordinary skill in the art will readily recognize that the same principles can be applied to CDRH1 and/or CDRH2 of the heavy chain, which are encoded entirely by the IGHV gene.

### CDRL3 Lengths

In some embodiments, as an alternative or in addition to other features described herein, the present invention provides libraries in which lengths of CDRL3s may be varied. The present invention therefore provides, among other things, libraries with a particular distribution of CDRL3 lengths. Although CDRL3 libraries of lengths 8, 9, and 10 are exemplified, one of ordinary skill in the art will readily recognize that the methods described herein can be applied to produce light chains with CDRL3s of different lengths (*e.g.,* about 5, 6, 7, 11, 12, 13, 14, 15, and/or 16) that also fall within the scope of the invention. In some embodiments, the length of any of the CDRL3s of the invention may be less than a particular number of amino acids, where the number of amino acids is defined using any one of the integers provided above. In some embodiments of the invention, a particular numerical range is defined, using any two of the integers provided above as lower and upper boundaries of the range, inclusive or exclusive. All combinations of the integers provided, which define an upper and lower boundary, are contemplated.

### Synthetic Antibody Libraries

In some embodiments of the invention, provided libraries include one or more synthetic polynucleotides. In some embodiments, provided libraries may comprise synthetic polynucleotides selected from (a) heavy chain chassis polynucleotides; (b) light chain chassis polynucleotides; (c) CDR3 polynucleotides; (d) constant domain polynucleotides; and (e) combinations thereof. Those of ordinary skill in the art will appreciate that such synthetic polynucleotides may be linked to other synthetic or non-synthetic polynucleotides in provided libraries.

Synthetic polynucleotides provided herein may be prepared by any available method. For example, in some embodiments, synthetic polynucleotides can be synthesized by split pool DNA synthesis as described in Feldhaus et al., Nucleic Acids Research, 2000, 28: 534; Omstein et al., Biopolymers, 1978, 17: 2341; Brenner and Lerner, PNAS, 1992, 87: 6378, U.S. Publication Nos. 2009/0181855 and 2010/0056386, and WO/2009/036379 (each incorporated by reference in its entirety).

In some embodiments of the invention, segments representing the possible TN1, DH, N2, and JH diversity found in the human repertoire are synthesized *de novo* either as double-stranded DNA oligonucleotides, single-stranded DNA oligonucleotides representative of the coding strand, or single-stranded DNA oligonucleotides representative of the non-coding strand. Such sequences can then be introduced into a host cell along with an acceptor vector containing a chassis sequence and, in some cases a portion of FRM4 and a constant region. No primer-based PCR amplification from mammalian cDNA or mRNA or template-directed cloning steps from mammalian cDNA or mRNA need be employed.

### Construction of Libraries by Yeast Homologous Recombination

In certain embodiments, the invention exploits the inherent ability of yeast cells to facilitate homologous recombination at high efficiency. The mechanism of homologous recombination in yeast and its applications are briefly described below (also see *e.g.,* U.S. Patent Nos. 6,406,863; 6,410,246; 6,410,271; 6,610,472; and 7,700,302, each of which is incorporated by reference in its entirety).

As an illustrative embodiment, homologous recombination can be carried out in, for example, *Saccharomyces cerevisiae*, which has genetic machinery designed to carry out homologous recombination with high efficiency. Exemplary *S. cerevisiae* strains include EM93, CEN.PK2, RM11-1a, YJM789, and BJ5465. This mechanism is believed to have evolved for the purpose of chromosomal repair, and is also called "gap repair" or "gap filling". By exploiting this mechanism, mutations can be introduced into specific loci of the yeast genome. For example, a vector carrying a mutant gene can contain two sequence segments that are homologous to the 5' and 3' open reading frame (ORF) sequences of a gene that is intended to be interrupted or mutated. The vector may also encode a positive selection marker, such as a nutritional enzyme allele (*e.g.,* URA3) and/or an antibiotic resistant marker (*e.g.,* Geneticin / G418), flanked by the two homologous DNA segments. Other selection markers and antibiotic resistance markers are known to one of ordinary skill in the art.

In some embodiments of the invention, this vector (*e.g.,* a plasmid) is linearized and transformed into the yeast cells. Through homologous recombination between the plasmid and the yeast genome, at the two homologous recombination sites, a reciprocal exchange of the DNA content occurs between the wild type gene in the yeast genome and the mutant gene (including the selection marker gene(s)) that is flanked by the two homologous sequence segments. By selecting for the one or more selection markers, the surviving yeast cells will be those cells in which the wild-type gene has been replaced by the mutant gene (Pearson et al., Yeast, 1998, 14: 391, incorporated by reference in its entirety). This mechanism has been used to make systematic mutations in all 6,000 yeast genes, or open reading frames (ORFs), for functional genomics studies. Because the exchange is reciprocal, a similar approach has also been used successfully to clone yeast genomic DNA fragments into a plasmid vector (Iwasaki et al., Gene, 1991, 109: 81).

By utilizing the endogenous homologous recombination machinery present in yeast, gene fragments or synthetic oligonucleotides can also be cloned into a plasmid vector without a ligation step. In this application of homologous recombination, a target gene fragment (*i.e.,* the fragment to be inserted into a plasmid vector, *e.g.,* a CDR3) is obtained (*e*.*g*., by oligonucleotides synthesis, PCR amplification, restriction digestion out of another vector, etc.). DNA sequences that are homologous to selected regions of the plasmid vector are added to the 5' and 3' ends of the target gene fragment. These homologous regions may be fully synthetic, or added via PCR amplification of a target gene fragment with primers that incorporate the homologous sequences. The plasmid vector may include a positive selection marker, such as a nutritional enzyme allele (*e.g.,* URA3), or an antibiotic resistance marker (*e.g.,* Geneticin / G418). The plasmid vector is then linearized by a unique restriction cut located in-between the regions of sequence homology shared with the target gene fragment, thereby creating an artificial gap at the cleavage site. The linearized plasmid vector and the target gene fragment flanked by sequences homologous to the plasmid vector are co-transformed into a yeast host strain. The yeast is then able to recognize the two stretches of sequence homology between the vector and target gene fragment and facilitate a reciprocal exchange of DNA content through homologous recombination at the gap. As a consequence, the target gene fragment is inserted into the vector without ligation.

The method described above has also been demonstrated to work when the target gene fragments are in the form of single stranded DNA, for example, as a circular M13 phage derived form, or as single stranded oligonucleotides (Simon and Moore, Mol. Cell Biol., 1987, 7: 2329; Ivanov et al., Genetics, 1996, 142: 693; and DeMarini *et al.,* 2001, 30: 520., each incorporated by reference in its entirety). Thus, the form of the target that can be recombined into the gapped vector can be double stranded or single stranded, and derived from chemical synthesis, PCR, restriction digestion, or other methods.

Several factors may influence the efficiency of homologous recombination in yeast. For example, the efficiency of the gap repair is correlated with the length of the homologous sequences flanking both the linearized vector and the target gene. In certain embodiments, about 20 or more base pairs may be used for the length of the homologous sequence, and about 80 base pairs may give a near-optimized result (Hua et al., Plasmid, 1997, 38:91; Raymond et al., Genome Res., 2002, 12: 190, each incorporated by reference in its entirety). In certain embodiments of the invention, at least about 5, 10, 15, 20, 21, 22, 23, 24, 25, 26, 27, 28, 29, 30, 31, 32, 33, 34 35, 36, 37, 38, 39, 40, 45, 50, 55, 60, 65, 70, 75, 80, 85, 90, 95, 100, 110, 120, 130, 140, 150, 160, 170, 180, 187, 190, or 200 homologous base pairs may be used to facilitate recombination. In certain embodiments, between about 20 and about 40 base pairs are utilized. In addition, the reciprocal exchange between the vector and gene fragment is strictly sequence-dependent, *i*.*e*. it does not cause a frame shift. Therefore, gap-repair cloning assures the insertion of gene fragments with both high efficiency and precision. The high efficiency makes it possible to clone two, three, or more targeted gene fragments simultaneously into the same vector in one transformation attempt (Raymond et al., Biotechniques, 1999, 26: 134, incorporated by reference in its entirety). Moreover, the nature of precision sequence conservation through homologous recombination makes it possible to clone selected genes or gene fragments into expression or fusion vectors for direct functional examination (El-Deiry et al., Nature Genetics, 1992, 1: 4549; Ishioka et al., PNAS, 1997, 94: 2449). Libraries of gene fragments have also been constructed in yeast using homologous recombination. For example, a human brain cDNA library was constructed as a two-hybrid fusion library in vector pJG4-5 (Guidotti and Zervos, Yeast, 1999, 15: 715). It has also been reported that a total of 6,000 pairs of PCR primers were used for amplification of 6,000 known yeast ORFs for a study of yeast genomic protein interactions (Hudson et al., Genome Res., 1997, 7: 1169). In 2000, Uetz *et al.* conducted a comprehensive analysis-of protein-protein interactions in *Saccharomyces cerevisiae* (Uetz et al., Nature, 2000, 403: 623). The protein-protein interaction map of the budding yeast was studied by using a comprehensive system to examine two-hybrid interactions in all possible combinations between the yeast proteins (Ito et al., PNAS, 2000, 97: 1143), and the genomic protein linkage map of Vaccinia virus was studied using this system (McCraith et al., PNAS, 2000, 97: 4879, incorporated by reference in its entirety).

In certain embodiments of the invention, a synthetic CDR3 (heavy or light chain) may be joined by homologous recombination with a vector encoding a heavy or light chain chassis, a portion of FRM4, and a constant region, to form a full-length heavy or light chain. In certain embodiments of the invention, the homologous recombination is performed directly in yeast cells. In some embodiments, such a method comprises:
(a) transforming into yeast cells:
   (i) a linearized vector encoding a heavy or light chain chassis, a portion of FRM4, and a constant region, wherein the site of linearization is between the end of FRM3 of the chassis and the beginning of the constant region; and
   (ii) a library of CDR3 insert nucleotide sequences that are linear and double stranded, wherein each of the CDR3 insert sequences comprises a nucleotide sequence encoding CDR3 and 5'- and 3'-flanking sequences that are sufficiently homologous to the termini of the vector of (i) at the site of linearization to enable homologous recombination to occur between the vector and the library of CDR3 insert sequences; and
(b) allowing homologous recombination to occur between the vector and the CDR3 insert sequences in the transformed yeast cells, such that the CDR3 insert sequences are incorporated into the vector, to produce a vector encoding full-length heavy chain or light chain.

As specified above, CDR3 inserts may have a 5' flanking sequence and a 3' flanking sequence that are homologous to the termini of the linearized vector. When the CDR3 inserts and the linearized vectors are introduced into a host cell, for example, a yeast cell, the "gap" (the linearization site) created by linearization of the vector is filled by the CDR3 fragment insert through recombination of the homologous sequences at the 5' and 3' termini of these two linear double-stranded DNAs (*i.e.,* the vector and the insert). Through this event of homologous recombination, libraries of circular vectors encoding full-length heavy or light chains comprising variable CDR3 inserts is generated. Particular instances of these methods are presented in the Examples.

Subsequent analysis may be carried out to determine, for example, the efficiency of homologous recombination that results in correct insertion of the CDR3 sequences into the vectors. For example, PCR amplification of the CDR3 inserts directly from selected yeast clones may reveal how many clones are recombinant. In certain embodiments, libraries with minimum of about 90% recombinant clones are utilized. In certain embodiments libraries with a minimum of about 1%, 5% 10%, 15%, 20%, 25%, 30%, 35%, 40%, 45%, 50%, 55%, 60%, 65%, 70%, 75%, 80%, 85%, 86%, 87%, 88%, 89%, 90%, 91%, 92%, 93%, 94%, 95%, 96%, 97%, 98%, or 99% recombinant clones are utilized. The same PCR amplification of selected clones may also reveal the insert size.

To verify the sequence diversity of the inserts in the selected clones, a PCR amplification product with the correct size of insert may be "fingerprinted" with restriction enzymes known to cut or not cut within the amplified region. From a gel electrophoresis pattern, it may be determined whether the clones analyzed are of the same identity or of the distinct or diversified identity. The PCR products may also be sequenced directly to reveal the identity of inserts and the fidelity of the cloning procedure, and to prove the independence and diversity of the clones.

### Expression and Screening Systems

Libraries of polynucleotides generated by any of the techniques described herein, or other suitable techniques, can be expressed and screened to identify antibodies having desired structure and/or activity. Expression of the antibodies can be carried out, for example, using cell-free extracts (and *e*.*g*., ribosome display), phage display, prokaryotic cells (*e.g.,* bacterial display), or eukaryotic cells (*e.g.,* yeast display). In certain embodiments of the invention, the antibody libraries are expressed in yeast.

In some embodiments, polynucleotides are engineered to serve as templates that can be expressed in a cell-free extract. Vectors and extracts as described, for example in U.S. Patent Nos. 5,324,637; 5,492,817; 5,665,563 can be used and many are commercially available. Ribosome display and other cell-free techniques for linking a polynucleotide (*i*.*e*., a genotype) to a polypeptide (*i.e.,* aphenotype) can be used, *e.g.,* Profusion™ (see, *e.g.,* U.S. Patent Nos. 6,348,315; 6,261,804; 6,258,558; and 6,214,553). Alternatively or additionally, polynucleotides of the invention can be expressed in an *E. coli* expression system, such as that described by Pluckthun and Skerra. (Meth. Enzymol., 1989, 178: 476; Biotechnology, 1991, 9: 273). Mutant proteins can be expressed for secretion in the medium and/or in the cytoplasm of the bacteria, as described by Better and Horwitz, Meth. Enzymol., 1989, 178: 476, incorporated by reference in its entirety. In some embodiments, the single domains encoding VH and VL are each attached to the 3' end of a sequence encoding a signal sequence, such as the ompA, phoA or pelB signal sequence (Lei et al., J. Bacteriol., 1987, 169: 4379). These gene fusions are assembled in a dicistronic construct, so that they can be expressed from a single vector, and secreted into the periplasmic space of *E. coli* where they will refold and can be recovered in active form. (Skerra et al., Biotechnology, 1991, 9: 273). For example, antibody heavy chain genes can be concurrently expressed with antibody light chain genes to produce antibodies or antibody fragments.

In some embodiments of the invention, antibody sequences are expressed on the membrane surface of a prokaryote, *e*.*g*., *E. coli*, using a secretion signal and lipidation moiety as described, *e.g.,* in US2004/0072740; US2003/0100023; and US2003/0036092). Higher eukaryotic cells, such as mammalian cells, for example myeloma cells (*e.g.,* NS/0 cells), hybridoma cells, Chinese hamster ovary (CHO), and human embryonic kidney (HEK) cells, can also be used for expression of the antibodies of the invention. Typically, antibodies expressed in mammalian cells are designed to be secreted into the culture medium, or expressed on the surface of the cell. Antibody or antibody fragments can be produced, for example, as intact antibody molecules or as individual VH and VL fragments, Fab fragments, single domains, or as single chains (scFv) (Huston et al., PNAS, 1988, 85: 5879). Alternatively or additionally, antibodies can be expressed and screened by anchored periplasmic expression (APEx 2-hybrid surface display), as described, for example, in Jeong et al., PNAS, 2007, 104: 8247 or by other anchoring methods as described, for example, in Mazor et al., Nature Biotechnology, 2007, 25: 563. In some embodiments of the invention, antibodies can be selected using mammalian cell display (Ho et al., PNAS, 2006, 103: 9637). Screening of the antibodies derived from the libraries of the invention can be carried out by any appropriate means. For example, binding activity can be evaluated by standard immunoassay and/or affinity chromatography. Screening of antibodies of the invention for catalytic function, *e*.*g*., proteolytic function can be accomplished using a standard assays, *e*.*g*., the hemoglobin plaque assay as described in U.S. Patent No. 5,798,208. Determining the ability of candidate antibodies to bind therapeutic targets can be assayed *in vitro* using, *e.g.,* a BIACORE™ instrument, which measures binding rates of an antibody to a given target or antigen based on surface plasmon resonance. *In vivo* assays can be conducted using any of a number of animal models and then subsequently tested, as appropriate, in humans. Cell-based biological assays are also contemplated.

One feature of the instant invention is the speed at which the antibodies of the library can be expressed and screened. In certain embodiments of the invention, the antibody library can be expressed in yeast, which have a doubling time of less than about 3, 4, 5, 6, 7, 8, 9,10, 11, 12, 13, 14, 15, 16, 17, 18, 19, 20, 21, 22, 23, or 24 hours. In some embodiments, the doubling times are about 1 to about 3 hours, about 2 to about 4, about 3 to about 8 hours, about 3 to about 24, about 5 to about 24, about 4 to about 6 about 5 to about 22, about 6 to about 8, about 7 to about 22, about 8 to about 10 hours, about 7 to about 20, about 9 to about 20, about 9 to about 18, about 11 to about 18, about 11 to about 16, about 13 to about 16, about 16 to about 20, or about 20 to about 30 hours. In certain embodiments of the invention, an antibody library is expressed in yeast with a doubling time of about 16 to about 20 hours, about 8 to about 16 hours, or about 4 to about 8 hours. Thus, an antibody library of the instant invention can be expressed and screened in a matter of hours, as compared to previously known techniques which take several days to express and screen antibody libraries. A limiting step in the throughput of such screening processes in mammalian cells is typically the time required to iteratively regrow populations of isolated cells, which, in some cases, have doubling times greater than the doubling times of the yeast used in the current invention.

In certain embodiments of the invention, the composition of a library may be defined after one or more enrichment steps (for example by screening for antigen binding, binding to a generic ligand, or other properties). For example, a library with a composition comprising about x% sequences or libraries of the invention may be enriched to contain about 2x% 3x%, 4x%, 5x%, 6x%, 7x%, 8x%, 9x%, 10x%, 20x%, 25x%, 40x%, 50x%, 60x% 75x%, 80x%, 90x%, 95x%,or 99x% sequences or libraries of the invention, after one or more screening steps. In some embodiments of the invention, the sequences or libraries of the invention may be enriched about 2-fold, 3-fold, 4-fold, 5-fold, 6-fold, 7-fold, 8-fold, 9-fold, 10-fold, 100-fold, 1,000-fold, or more, relative to their occurrence prior to the one or more enrichment steps. In certain embodiments of the invention, a library may contain at least a certain number of a particular type of sequence(s), such as CDRH3s, CDRL3s, heavy chains, light chains, or whole antibodies (*e*.*g*., at least about 10³, 10⁴, 10⁵, 10⁶, 10⁷, 10⁸, 10⁹, 10¹⁰, 10¹¹, 10¹², 10¹³, 10¹⁴, 10¹⁵, 10¹⁶, 10¹⁷, 10¹⁸, 10¹⁹, or 10²⁰). In certain embodiments, these sequences may be enriched during one or more enrichment steps, to provide libraries comprising at least about 10², 10³, 10⁴, 10⁵, 10⁶, 10⁷, 10⁸, 10⁹, 10¹⁰, 10¹¹, 10¹², 10¹³, 10¹⁴, 10¹⁵, 10¹⁶, 10¹⁷, 10¹⁸, or 10¹⁹ of the respective sequence(s).

### Mutagenesis Approaches for Affinity Maturation

As described above, antibody leads can be identified through a selection process that involves screening the antibodies of a library of the invention for binding to one or more antigens, or for a biological activity. Coding sequences of these antibody leads may be further mutagenized *in vitro* or *in vivo* to generate secondary libraries with diversity introduced in the context of the initial antibody leads. Such mutagenized antibody leads can then be further screened for binding to target antigens or biological activity, *in vitro* or *in vivo,* following procedures similar to those used for the selection of the initial antibody lead from the primary library. Such mutagenesis and selection of primary antibody leads effectively mimics the affinity maturation process naturally occurring in a mammal that produces antibodies with progressive increases in the affinity to an antigen.

In some embodiments of the invention, only the CDRH3 region is mutagenized. In some embodiments of the invention, the whole variable region is mutagenized. In some embodiments of the invention one or more of CDRH1, CDRH2, CDRH3, CDRL1, CDRL2, and/ CDRL3 may be mutagenized. In some embodiments of the invention, "light chain shuffling" may be used as part of the affinity maturation protocol. In certain embodiments, this may involve pairing one or more heavy chains with a number of light chains, to select light chains that enhance the affinity and/or biological activity of an antibody. In certain embodiments of the invention, the number of light chains to which the one or more heavy chains can be paired is at least about 2, 5, 10, 100, 10³, 10⁴, 10⁵, 10⁶, 10⁷, 10⁸, 10⁹, or 10¹⁰. In certain embodiments of the invention, these light chains are encoded by plasmids. In some embodiments of the invention, the light chains may be integrated into the genome of the host cell.

Coding sequences of antibody leads may be mutagenized using any of wide variety of methods. Examples of methods of mutagenesis include, but are not limited to site-directed mutagenesis, error-prone PCR mutagenesis, cassette mutagenesis, and random PCR mutagenesis. Alternatively or additionally, oligonucleotides encoding regions with the desired mutations can be synthesized and introduced into the sequence to be mutagenized, for example, via recombination or ligation.

Site-directed mutagenesis or point mutagenesis may be used to gradually change the CDR sequences in specific regions. For example, this may be accomplished by using oligonucleotide-directed mutagenesis or PCR. For example, a short sequence of an antibody lead may be replaced with a synthetically mutagenized oligonucleotide in either the heavy chain or light chain region, or both. Such a method may not be efficient for mutagenizing large numbers of CDR sequences, but may be used for fine tuning of a particular lead to achieve higher affinity toward a specific target protein.

Cassette mutagenesis may alternatively or additionally be used to mutagenize the CDR sequences in specific regions. In a typical cassette mutagenesis, a sequence block, or a region, of a single template is replaced by a completely or partially randomized sequence. However, the maximum information content that can be obtained may be statistically limited by the number of random sequences of the oligonucleotides. Similar to point mutagenesis, this method may also be used for fine tuning of a particular lead to achieve higher affinity towards a specific target protein.

Error-prone PCR, or "poison" PCR, may be used to mutagenize the CDR sequences, for example, by following protocols described in U.S. Patent No. 6,153,745; Caldwell and Joyce, PCR Methods and Applications, 1992, 2: 28; Leung et al., Technique, 1989, 1: 11; Shafikhani et al., Biotechniques, 1997, 23: 304; and Stemmer et al., PNAS, 1994, 91: 10747.

Conditions for error prone PCR may include, for example, (a) high concentrations of Mn²⁺ (*e.g.,* about 0.4 to about 0.6 mM) that efficiently induces malfunction of Taq DNA polymerase; and/or (b) a disproportionally high concentration of one nucleotide substrate (*e.g.,* dGTP) in the PCR reaction that causes incorrect incorporation of this high concentration substrate into the template and produces mutations. Alternatively or additionally, other factors such as, the number of PCR cycles, the species of DNA polymerase used, and the length of the template, may affect the rate of misincorporation of "wrong" nucleotides into the PCR product. Commercially available kits may be utilized for the mutagenesis of the selected antibody library, such as the "Diversity PCR random mutagenesis kit" (CLONTECH™).

Primer pairs used in PCR-based mutagenesis may, in certain embodiments, include regions matched with the homologous recombination sites in the expression vectors. Such a design allows facile re-introduction of the PCR products back into the heavy or light chain chassis vectors, after mutagenesis, via homologous recombination.

Other PCR-based mutagenesis methods can also be used, alone or in conjunction with the error prone PCR described above. For example, the PCR amplified CDR segments may be digested with DNase to create nicks in the double stranded DNA. These nicks can be expanded into gaps by other exonucleases such as Bal 31. Gaps may then be filled by random sequences by using DNA Klenow polymerase at a low concentration of regular substrates dGTP, dATP, dTTP, and dCTP with one substrate (e.g., dGTP) at a disproportionately high concentration. This fill-in reaction should produce high frequency mutations in the filled gap regions. Such methods of DNase digestion may be used in conjunction with error prone PCR to create a high frequency of mutations in the desired CDR segments.

CDR or antibody segments amplified from the primary antibody leads may also be mutagenized *in vivo* by exploiting the inherent ability of mutation in pre-B cells. The Ig genes in pre-B cells are specifically susceptible to a high-rate of mutation. The Ig promoter and enhancer facilitate such high rate mutations in a pre-B cell environment while the pre-B cells proliferate. Accordingly, CDR gene segments may be cloned into a mammalian expression vector that contains a human Ig enhancer and promoter. Such a construct may be introduced into a pre-B cell line, such as 38B9, which allows the mutation of the VH and VL gene segments naturally in the pre-B cells (Liu and Van Ness, Mol. Immunol., 1999, 36: 461). The mutagenized CDR segments can be amplified from the cultured pre-B cell line and re-introduced back into the chassis-containing vector(s) via, for example, homologous recombination.

In some embodiments, a CDR "hit" isolated from screening the library can be re-synthesized, for example using degenerate codons or trinucleotides, and re-cloned into the heavy or light chain vector using gap repair.

### Other Variants of Polynucleotide Sequences of the Invention

In certain embodiments, the invention provides a polynucleotide that hybridizes with a polynucleotide taught herein, or that hybridizes with the complement of a polynucleotide taught herein. For example, an isolated polynucleotide that remains hybridized after hybridization and washing under low, medium, or high stringency conditions to a polynucleotide taught herein or the complement of a polynucleotide taught herein is encompassed by the present invention.

Exemplary low stringency conditions include hybridization with a buffer solution of about 30% to about 35% formamide, about 1 M NaCl, about 1% SDS (sodium dodecyl sulphate) at about 37°C, and a wash in about 1X to about 2X SSC (20X SSC=3.0 M NaCl/0.3 M trisodium citrate) at about 50°C to about 55°C.

Exemplary moderate stringency conditions include hybridization in about 40% to about 45% formamide, about 1 M NaCl, about 1% SDS at about 37°C, and a wash in about 0.5X to about 1X SSC at abut 55°C to about 60°C.

Exemplary high stringency conditions include hybridization in about 50% formamide, about 1 M NaCl, about 1% SDS at about 37°C, and a wash in about 0.1X SSC at about 60° C to about 65° C.

Optionally, wash buffers may comprise about 0.1% to about 1% SDS.

The duration of hybridization is generally less than about 24 hours, usually about 4 to about 12 hours.

### Sublibraries and Larger Libraries Comprising Libraries or Sub-Libraries of the Invention

Libraries comprising combinations of the libraries described herein (*e.g.,* CDRH3 and CDRL3 libraries) are encompassed by the invention. Sublibraries comprising portions of the libraries described herein are also encompassed by the invention (*e.g.,* a CDRH3 library in a particular heavy chain chassis or a sub-set of the CDRH3 libraries, for example based on length).

Moreover, libraries containing one of the libraries or sublibraries of the invention also fall within the scope of the invention. For example, in certain embodiments of the invention, one or more libraries or sublibraries of the invention may be contained within a larger library (theoretical or physical), which may include sequences derived by other means, for example, non-human or human sequence derived by stochastic or sitewise-stochastic synthesis. In certain embodiments of the invention, at least about 1% of the sequences in a polynucleotide library may be those of the invention (*e.g.,* CDRH3 sequences, CDRL3 sequences, VH sequences, VL sequences), regardless of the composition of the other 99% of sequences. For the purposes of illustration only, one of ordinary skill in the art would readily recognize that a library containing 10⁹ total members, where 10⁷ members are members of the libraries of the invention (*i*.*e*., 1%) would have utility, and that members of the libraries of the invention could be isolated from such a library. In some embodiments of the invention, at least about 0.001%, 0.01%, 0.1%, 2%, 5%, 10%, 20%, 30%, 40%, 50%, 60%, 70%, 80%, 81%, 82%, 83%, 84%, 85%, 86%, 87%, 88%, 89%, 90%, 91,%, 92%, 93%, 94%, 95%, 96%, 97%, 98% or 99% of the sequences in any polynucleotide library may be those of the invention, regardless of the composition of the other sequences. In some embodiments, the sequences of the invention may comprise about 0.001% to about 1%, about 1% to about 2%, about 2% to about 5%, about 5% to about 10%, about 10% to about 15%, about 15% to about 20%, about 20% to about 25%, about 25% to about 30%, about 30% to about 35%, about 35% to about 40%, about 40% to about 45%, about 45% to about 50%, about 50% to about 55%, about 55% to about 60%, about 60% to about 65%, about 65% to about 70%, about 70% to about 75%, about 75% to about 80%, about 80% to about 85%, about 85% to about 90%, about 90% to about 95%, or about 95% to about 99% of the sequences in any polynucleotide library, regardless of the composition of the other sequences. Thus, libraries more diverse than one or more libraries or sublibraries of the invention, but yet still comprising one or more libraries or sublibraries of the invention, in an amount in which the one or more libraries or sublibraries of the invention can be effectively screened and from which sequences encoded by the one or more libraries or sublibraries of the invention can be isolated, also fall within the scope of the invention.

### Alternative Scaffolds

As would be evident to one of ordinary skill in the art, the CDRH3 and/or CDRL3 polypeptides provided by the invention may also be displayed on alternative scaffolds. Several such scaffolds have been shown to yield molecules with specificities and affinities that rival those of antibodies. Exemplary alternative scaffolds include those derived from fibronectin (*e.g.,* AdNectin), the β-sandwich (*e.g.,* iMab), lipocalin (*e.g.,* Anticalin), EETI-II/AGRP, BPTI/LACI-D1/ITI-D2 (*e.g.,* Kunitz domain), thioredoxin *(e.g.,* peptide aptamer), protein A (*e.g.,* Affibody), ankyrin repeats (*e.g.,* DARPin), γB-crystallin/ubiquitin (*e.g.,* Affilin), CTLD₃ (*e.g.,* Tetranectin), and (LDLR-A module)₃ (*e.g.,* Avimers). Additional information on alternative scaffolds is provided, for example, in Binz et al., Nat. Biotechnol., 2005 23: 1257 and Skerra, Current Opin. in Biotech., 2007 18: 295-304, each of which is incorporated by reference in its entirety.

### Additional Embodiments of the Invention

### Library Sizes

In some embodiments of the invention, a library comprises about 10¹ to about 10²⁰ different polynucleotide or polypeptide sequences (encoding or comprising *e.g.,* antibodies, heavy chains, CDRH3s, light chains, and/or CDRL3s). In some embodiments, the libraries of the invention are designed to include at least about 10¹, 10², 10³, 10⁴, 10⁵, 10⁶, 10⁷, 10⁸, 10⁹, 10¹⁰, 10¹¹, 10¹², 10¹³, 10¹⁴, 10¹⁵, 10¹⁶, 10¹⁷, 10¹⁸, 10¹⁹, or 10²⁰, or more different antibody, heavy chain, CDRH3, light chain, and/or CDRL3 polynucleotide orpolypeptide sequences. In some embodiments, a library of the invention may contain less than a particular number of polynucleotide or polypeptide sequences, where the number of sequences is defined using any one of the integers provided above. In certain embodiments of the invention, a particular numerical range is defined, using any two of the integers provided above as lower and upper boundaries of the range, inclusive or exclusive. All combinations of the integers provided, which define an upper and lower boundary, are contemplated.

In some embodiments, the invention provides libraries wherein a fraction of the members of the library are members produced according to the methods, systems, and compositions provided herein. One important property of the libraries of the invention is that they favorably mimic certain aspects of the human preimmune repertoire, including length diversity and sequence diversity. One or ordinary skill in the art will readily recognize that libraries provided by the invention include libraries where a subset of the members of the library are members produced according to the methods, systems, and compositions provided herein. For example, a library containing 10⁸ members wherein 10⁶ members are produced according to the methods, systems, and compositions provided herein, would contain 1% sequences produced according to the methods, systems, and compositions provided herein. One of ordinary skill in the art would recognize that one or more of the 10⁶ members could readily be isolated using screening techniques known in the art. Therefore, said libraries fall within the scope of the invention. More specifically, libraries comprising at least about 1%, 2.5%, 5%, 10%, 15%, 20%, 25%, 30%, 40%, 45%, 50%, 55%, 60%, 65%, 70%, 75%, 80%, 85%, 90%, 95%, or 99% CDRH3, CDRL3, light chain, or heavy chain, and/or full-length antibody sequences provided herein fall within the scope of the invention. Libraries comprising at least about 10³, 10⁴, 10⁵, 10⁶, 10⁷, 10⁸, 10⁹, 10¹⁰, 10¹¹, 10¹², 10¹³, 10¹⁴, 10¹⁵ CDRH3, CDRL3, light chain, heavy chain, and/or full-length antibody sequences provided herein also fall within the scope of the invention.

### Human Preimmune Set

In some embodiments, the invention comprises the set of 3,571 curated human preimmune antibody sequences contained within the HPS, their corresponding CDRH3 sequences (Appendix A), and/or a representation of these CDRH3 sequences (and/or TN1, DH, N2, and/or H3-JH segments thereof) in a computer readable format. In certain embodiments, the invention comprises a method of producing a CDRH3 library, the method comprising matching candidate segments (*i.e.,* TN1, DH, N2, and H3-JH) from a theoretical segment pool with CDRH3 sequences in the HPS and/or any other repertoire of CDRH3 sequences. In some embodiments, the invention comprises the candidate segments from the theoretical segment pools disclosed herein and/or the segments selected for inclusion in a physical library.

### Embodiments

While the methods described herein demonstrate the production of theoretical segment pools of H3-JH and DH segments using a limited number of allelic variants, one of ordinary skill in the art will recognize that methods taught herein may be applied to any IGHJ and IGHD genes, including any other allelic variants and all non-human IGHJ and IGHD genes. Alternatively or additionally, methods described herein may be applied to any reference set of CDRH3 sequences, for example to extract additional TN1 and/or N2 segments. Alternatively or additionally, one of ordinary skill in the art will recognize that each of the described embodiments of the invention may be in polynucleotide or polypeptide form, within a vector, virus, or microorganism (*e.g.,* a yeast or bacteria). Furthermore, since the invention involves synthetic libraries that are fully enumerated, certain embodiments of the invention relate to any of the embodiments described above in a computer readable format, and uses thereof.

Non-human antibody libraries also fall within the scope of the invention.

The present disclosure describes the removal of sequences containing Cys residues, N-linked glycosylation motifs, deamidation motifs, and highly hydrophobic sequences from the libraries of the invention. One of ordinary skill in the art will recognize that one or more of these criteria (*i.e.,* not necessarily all) can be applied to remove undesirable sequences from any library of the invention. However, libraries containing one or more of these types of sequences also fall within the scope of the invention. Other criteria can also be used; those described herein are not limiting.

In certain embodiments, the invention provides libraries in which the number of times a particular sequence is repeated within the library (either theoretical, synthetic, or physical realization) is limited. For example, in some embodiments, the invention provides libraries wherein the frequency of occurrence of any of the sequences in the library (*e.g.,* CDRH3, CDRL3, heavy chain, light chain, full-length antibody) is less than about 2, 5, 10, 15, 20, 25, 30, 35, 40, 45, 50, 55, 60, 65, 70, 75, 80, 85, 90, 95, 100, 200, 300, 400, 500, 600, 700, 800, 900, or 1000. In some embodiments, the frequency of occurrence of any of the sequences in the library is less than a multiple of the frequency of occurrence of any other sequence in the library, for examples less than about 2, 5, 10, 15, 20, 25, 30, 35, 40, 45, 50, 55, 60, 65, 70, 75, 80, 85, 90, 95, 100, 200, 300, 400, 500, 600, 700, 800, 900, or 1000 times the frequency of occurrence of any other sequence in the library.

In some embodiments, libraries are defined by the combinatorial diversity of the segments used to produce CDRH3 sequences, in particular the number of non-degenerate segment combinations that can be used to produce a particular CDRH3 sequence. In some embodiments, this metric may be calculated using, for example, a sample of about 2000, 5000, 10000, 20000, 50000, 100000, or more sequences from the CDRH3 library and "self-matching" using the segments used to generate the CDRH3 sequences of that library. In certain embodiments, the invention provides libraries wherein at least about 95%, 90%, 85%, 80%, 75%, 70%, 65%, 60% 55%, 50%, 45%, 40%, 35%, 30%, 25%, 20%, 15%, 10%, or 5% of the CDRH3 sequences in the library may be formed by a single combination of segments.

In certain embodiments of the invention, a statistical bootstrap analysis was used to generate CDRH3 reference sets. While it may be advantageous to use this method, it is not required for every embodiment of the invention.

In some embodiments, the invention provides methods and systems of selecting polynucleotides to encode polypeptides of the invention, comprising selecting polynucleotide segments lacking (or containing) certain restriction sites individually and/or after combinatorial concatenation with other segments *(e.g.,* see Example 9.3.7).

The exemplary libraries provided herein are not limiting and provided for exemplification only.

### EXAMPLES

This invention is further illustrated by the following examples which should not be construed as limiting. The contents of all references, patents and published patent applications cited throughout this application are hereby incorporated by reference.

In general, the practice of the present invention employs, unless otherwise indicated, conventional techniques of chemistry, molecular biology, recombinant DNA technology, PCR technology, immunology (especially, *e.g.,* antibody technology), expression systems (*e.g.,* yeast expression, cell-free expression, phage display, ribosome display, and PROFUSION™), and any necessary cell culture that are within the skill of the art and are explained in the literature. See, *e.g.,* Sambrook, Fritsch and Maniatis, Molecular Cloning: Cold Spring Harbor Laboratory Press (1989); DNA Cloning, Vols. 1 and 2, (D.N. Glover, Ed. 1985); Oligonucleotide Synthesis (M.J. Gait, Ed. 1984); PCR Handbook Current Protocols in Nucleic Acid Chemistry, Beaucage, Ed. John Wiley & Sons (1999) (Editor); Oxford Handbook of Nucleic Acid Structure, Neidle, Ed., Oxford Univ Press (1999); PCR Protocols: A Guide to Methods and Applications, Innis et al., Academic Press (1990); PCR Essential Techniques: Essential Techniques, Burke, Ed., John Wiley & Son Ltd (1996); The PCR Technique: RT-PCR, Siebert, Ed., Eaton Pub. Co. (1998); Antibody Engineering Protocols (Methods in Molecular Biology), 510, Paul, S., Humana Pr (1996); Antibody Engineering: A Practical Approach (Practical Approach Series, 169), McCafferty, Ed., Irl Pr (1996); Antibodies: A Laboratory Manual, Harlow et al., C.S.H.L. Press, Pub. (1999); Current Protocols in Molecular Biology, eds. Ausubel et al., John Wiley & Sons (1992); Large-Scale Mammalian Cell Culture Technology, Lubiniecki, A., Ed., Marcel Dekker, Pub., (1990); Phage Display: A Laboratory Manual, C. Barbas (Ed.), CSHL Press, (2001); Antibody Phage Display, P O'Brien (Ed.), Humana Press (2001); Border et al., Nature Biotechnology, 1997, 15: 553; Border et al., Methods Enzymol., 2000, 328: 430; ribosome display as described by Pluckthun et al. in U. S. Patent No. 6,348,315, and Profusion™ as described by Szostak et al. in U.S. Patent Nos. 6,258,558; 6,261,804; and 6,214,553; and bacterial periplasmic expression as described in US20040058403A1.

Further details regarding antibody sequence analysis using Kabat conventions and programs to analyze aligned nucleotide and amino acid sequences may be found, *e.g.,* in Johnson et al., Methods Mol. Biol., 2004, 248: 11; Johnson et al., Int. Immunol., 1998, 10: 1801; Johnson et al., Methods Mol. Biol., 1995, 51: 1; Wu et al., Proteins, 1993, 16: 1; and Martin, Proteins, 1996,25: 130.

Further details regarding antibody sequence analysis using Chothia conventions may be found, *e*.*g*., in Chothia et al., J. Mol. Biol., 1998, 278: 457; Morea et al., Biophys. Chem., 1997, 68: 9; Morea et al., J. Mol. Biol., 1998, 275: 269; Al-Lazikani et al., J. Mol. Biol., 1997, 273: 927. Barre et al., Nat. Struct. Biol., 1994, 1: 915; Chothia et al., J. Mol. Biol., 1992, 227: 799; Chothia et al., Nature, 1989, 342: 877; and Chothia et al., J. Mol. Biol., 1987, 196: 901. Further analysis of CDRH3 conformation may be found in Shirai et al., FEBS Lett., 1999, 455: 188 and Shirai et al., FEBS Lett., 1996, 399: 1. Further details regarding Chothia analysis are described, for example, in Chothia et al., Cold Spring Harb. Symp. Quant Biol., 1987, 52: 399.

Further details regarding CDR contact considerations are described, for example, in MacCallum et al., J. Mol. Biol., 1996, 262: 732. Further details regarding the antibody sequences and databases referred to herein are found, *e*.*g*., in Tomlinson et al., J. Mol. Biol., 1992, 227: 776, VBASE2 (Retter et al., Nucleic Acids Res., 2005, 33: D671); BLAST (www.ncbi.nlm.nih.gov/BLAST/); CDHIT (bioinformatics.ljcrf.edu/cd-hi/); EMBOSS (www.hgmp.mrc.ac.uk/Software/EMBOSS/); PHYLIP (evolution.genetics.washington.edu/phylip.html); and FASTA (fasta.bioch.virginia.edu).

### LIGHT CHAIN LIBRARIES

### Example 1. Light Chain Libraries with Framework and/or CDRL1 and/or CDRL2 Variability

Although the diversity in antibody sequences is concentrated in the CDRs, certain residues in the framework regions can also influence antigen recognition and/or modulate affinity (Queen et al., Proc. Natl. Acad. Sci. USA, 1989, 86: 10029; Carter et al., Proc. Natl. Acad. Sci. USA, 1992, 89: 4285). These residues have been cataloged and used to make framework substitutions that improve antibody affinity, for example, during the process of antibody humanization (*e.g.,* see the "Vernier" residues in Foote and Winter, J. Mol. Biol., 1992, 224: 487). In the heavy chain,
the Vernier residues include Kabat-numbered residues 2, 27-30, 47-49, 67, 69, 71, 73, 78, 93-94, and 103. In the light chain, the Vernier residues include Kabat residues 2, 4, 35-36, 46-49, 64, 66, 68-69, 71, and 98. The Vernier residue numbers are the same for kappa and lambda light chain sequences (*see* Table 4 in Chothia et al., J. Mol. Biol., 1985, 186: 651). Additionally, framework
positions at the VL-VH interface may also influence affinity. In the heavy chain, the interface residues include Kabat residues 35, 37, 39, 45, 47, 91, 93, 95, 100, and 103 (Chothia et al., J. Mol. Biol., 1985, 186: 651). In the light chain, the interface residues include Kabat residues 34, 36, 38, 44, 46, 87, 89, 91, 96, and 98.

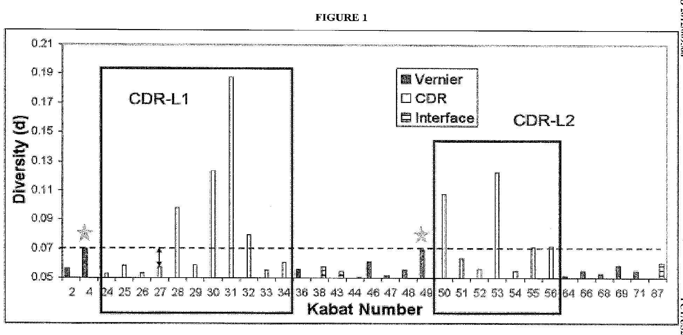

The following procedure was used to select the framework residues to be varied and the amino acids to which they should be varied:
a. A collection of human VK light chain DNA sequences was obtained from NCBI (*see* Appendix A of WO/2009/036379 for GI Nos.). These sequences were classified according to the germline origin of their VK germline segment.
b. Patterns of variation at each of the Vernier and interface positions were examined as follows:
   i. Equation 1 (from Makowski & Soares, Bioinformatics, 2003, 19: 483, incorporated by reference in its entirety) was used to calculate a diversity index for the Vernier positions, interface positions, CDRL1, and CDR2. $d = \frac{1}{N \sum {p}_{i}^{2}}$ Here, *d* is the diversity index, *N* is 20, the total number of amino acid types, and *pᵢ* is the fraction of amino acid of type "i" at the position of interest The sum is carried out over the 20 amino acid types. The parameter *d* will attain its minimum value of 0.05 or 1/20, when a single amino acid type is observed at a given position: *pᵢ* is I for one type and zero for all the rest. Conversely, when all the amino acid types arc equally probable (*e.g., pᵢ* is 0.05 for all i), *d* will attain its maximum value of 1.0.
   ii. The diversity index for each of the Vernier and interface positions were compared to the diversity index for the positions in CDRL1 and CDRL2.
   iii. The interface positions were found to be relatively invariant, with *d* values very close to the minimum of 0.05, and were thus not altered. The Vernier residues with a diversity index comparable to or larger than that of the CDR positions (*i.e*., at or above 0.07 for the particular example provided in Figure 1 were selected as candidates for variance (see Figure 1). The amino acid residues included in these positions were selected from amongst the two to three amino acids most frequently occurring in that position in the sequences in the collection of human VK light chains, for each particular VK germline.
   iv. Table 2 shows the positions selected for variance in each of nine exemplified light chain germlines. The alternative framework positions represent positions with a diversity index less than the primary framework positions, but where variability may still be incorporated to influence antigen binding.
   v. The amino acid residues in the framework positions selected for variance were varied as follows (Table 3 provides the polypeptide sequences of these variants):
      1. **Position 2**: Germline I was optionally changed to V.
      2. **Position 4:** Germline M or L was optionally changed to L or M. In some embodiments, changes from M to L, but not the reverse, may be preferred, because M may undergo oxidation during production, processing, or storage, potentially altering the properties of the antibody.
      **3. Position 36:** Germline Y was optionally changed to F and H.
      **4. Position 46:** Germline L was optionally changed to V.
      **5. Position 48:** Germline I was optionally changed to L.
      **6. Position 49:** Germline Y was optionally changed to S, F, and H.
      **7. Position 66:** Germline G was optionally changed to R and E.

One of ordinary skill in the art would readily recognize that the procedure outlined above could also be used to select positions to vary in Vλ germline sequences, and that libraries containing Vλ chains also fall within the scope of the invention.

In addition to the framework mutations, variability was also introduced into CDRL1 and CDRL2. This was performed by determining which residues in CDRL1 and CDRL2 were variable, within a particular germline, in the VK dataset used above and incorporating the most frequently occurring 2 to 4 variants into CDRL1 and CDRL2 in the synthetic libraries of the invention. With the exception of position 50 of CDRL2 of the VK1-5 germline, these alternatives did not arise from allelic variation. Table 3 shows the polypeptide sequences of nine light chain chassis and their framework and CDR L1/L2 variants for the currently exemplified embodiment of the invention. The amino acid residues in the CDRL1/L2 positions selected for variance were varied as follows (using the Chothia-Lesk numbering system; Chothia and Lesk, J. Mol. Biol., 1987, 196: 901):
1. **Position 28:** Germline S or G were optionally changed to G, A, or D.
2. **Position 29:** Germline V was optionally changed to I.
3. **Position 30:** Germline S was optionally changed to N, D, G, T, A, or R.
4. **Position 30A:** Germline H was optionally changed to Y
5. **Position 30B:** Germline S was optionally changed to R or T.
6. **Position 30E:** Germline Y was optionally changed to N.
7. **Position 31:** Germline S was optionally changed to D, R, I, N, or T.
8. **Position 32:** Germline Y or N were optionally changed to F, S, or D.
9. **Position 50:** Germline A, D, or G were optionally changed to G, S, E, K, or D.
10. **Position 51:** Germline G or A were optionally changed to A, S, or T.
11. **Position 53:** Germline S or N were optionally changed to N, H, S, K, or R.
12. **Position 55:** Germline E was optionally changed to A or Q.

### Example 2. Light Chain Libraries with Enhanced Diversity in CDRL3

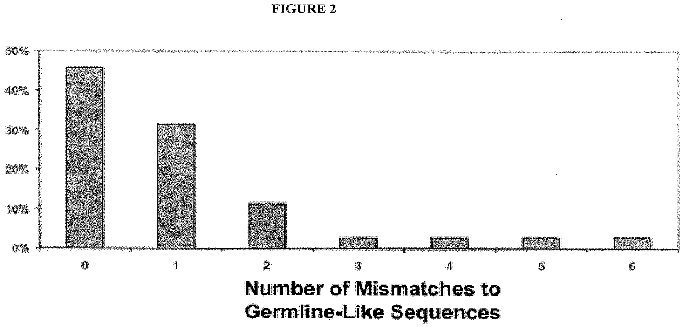

A variety of methods of producing light chain libraries are known in the art (*e.g.,* see U.S. Publication Nos. 2009/0181855, 2010/0056386, and WO/2009/036379). An analysis of clinically validated antibody sequences indicated that these sequences have very little deviation from germline-like VL-JL (where "L" can be a kappa or lambda germline sequence) rearrangements prior to somatic mutation (Figure 2). Here, a germline-like rearrangement is one where neither the V nor J portion differ from the respective germline genes and, for the purposes of this particular example, where the length of CDRL3 is restricted to 8, 9 or 10 amino acids (*see* U.S. Publication Nos. 2009/0181855, 2010/0056386, and WO/2009/036379). For the IGHJK1 gene, however, both WT (Trp-Thr) and RT (Arg-Thr) sequences (the first two N-terminal residues) are considered "germline-like" and so are full L3 rearrangements containing such sequences. Therefore, new light chain libraries were designed and constructed with the objectives of simultaneously (1) minimizing deviation from germline-like sequences, as defined above; and (2) generating maximal diversity. In particular, the overarching goal was to maximize the *type* of diversity that is indicated to be most favorable by clinically validated antibody sequences. In particular, the designed library sought to maximize the diversity of CDRL3 sequences that differ from length-matched germline sequences by two amino acids or fewer.

This was accomplished by utilizing a "jumping dimer" or "jumping trimer" approach to light chain oligonucleotide design. The jumping dimer approach involves the incorporation of degenerate codons at each of the six positions of CDRL3 encoded by the VL segment (L3-VL). At most two positions vary from germline in each individual L3-VL sequence, but the two positions do not have to be adjacent to one another. Thus, the total number of designed degenerate oligonucleotides synthesized per VL chassis is 6!/(4!2!), or fifteen (accounting for six of the most commonly occurring amino acids at the junction (position 96) between VL and JL for each kappa germline chassis (namely F, L, I, R, W, Y, and P; see U.S. Publication Nos. 2009/0181855 and 2010/0056386, and WO/2009/036379, each of which is incorporated by reference in its entirety, for more details on the junctional amino acids at position 96). The jumping trimer approach is analogous to the jumping dimer approach, but with three positions varying from germline in each individual L3-VL sequence, instead of two as in the jumping dimer. The degenerate codons selected for each position in the jumping dimer and trimer approaches were chosen to (1) to reproduce the diversity contained in the known repertoire of publicly available human VK sequences (see Appendix A of WO/2009/036379); and (2) to minimize or eliminate undesirable sequences within the CDRL3s of the resulting synthetic light chains, such as N-linked glycosylation motifs (NXS/NXT), Cys residues, stop codons, and deamidation-prone NG motifs. Table 4 shows the fifteen degenerate oligonucleotides encoding the VK1-39 CDRL3 sequences with a length of nine amino acids and F or Y as the junctional amino acid, and the corresponding degenerate polypeptide sequences. Table 5, Table 6, and Table 7 provide the oligonucleotide sequences for each of the VK sequences of the exemplary jumping dimer and trimer libraries, for CDRL3 lengths of 8, 9, and 10, respectively, and the sequences for the corresponding CDRL3s.

The number of unique CDRL3 sequences within each germline library was then enumerated and compared to the number of unique CDRL3 sequences in a different light chain library, designated "VK-v1.0" (see Example 6.2 in US Publication No. 2009/0181855), for each of the three lengths. Table 8 provides the number of unique CDRL3 sequences in each of the respective germline libraries.

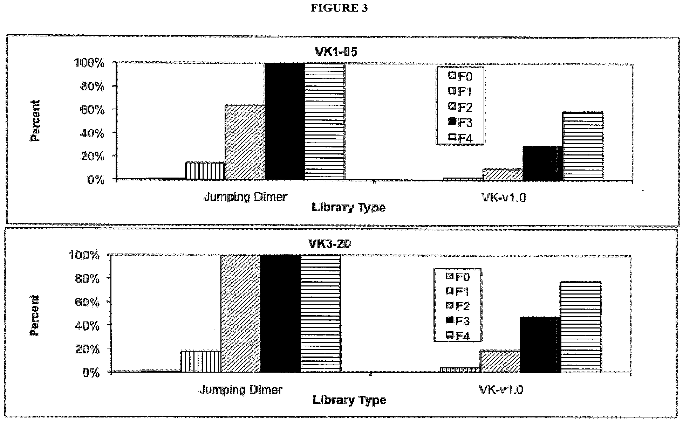

Figure 3 provides the percentage of sequences in the jumping dimer and VK-v1.0 libraries with CDRL3 length of nine amino acids that contain no mutations from germline-like sequences (Table 1) or 1, 2, 3, or 4 or fewer mutations from germline-like sequences. Naturally-occurring VK1-05 sequences are almost as likely to have Ser (germline amino acid type) as Pro at Kabat position 95, thus both residues (S and P) were incorporated in the synthetic libraries representing VK1-05 repertoires. However, as indicated in Table 1, only Ser was considered to be a germline-like residue at position 95 for the purposes of this analysis when the VK gene is VK-05. The plot for VK3-20 is representative of the remaining chassis in the library for a length nine. All of the sequences in the VK1-05 library were within three amino acids of human germline sequences, and approximately 63% of the sequences were within two amino acids of human germline-like sequences. For the rest of the libraries, and as designed, 100% of the sequences were within two amino acids of human germline-like sequences; thus, over 95% of the sequences of length 9 in the jumping dimer library considered as a whole were within 2 amino acids of germline-like sequences. By comparison, only 16% of the members of the VK-v1.0 libraries of length nine amino acids are within two amino acids of the corresponding human germline-like sequences. For length 8, about 98% of the sequences in the jumping dimer libraries were within two amino acids of germline-like, versus about 19% for VK-v1.0. For length 10, more than 95% of the sequences of the jumping dimer library were within two amino acids of germline-like, versus about 8% for VK-v1.0.

In some embodiments, to concentrate the diversity in positions most likely to be solvent-exposed in the folded antibody, positions 89 and 90 (Kabat numbering) are not modified from germline- these are most often QQ, but the sequence is MQ for the VK2-28 chassis. Other VK germline genes have different sequences at positions 88-89, and the use of these genes as chassis also falls within the scope of the invention. For example, VK1-27 has QK, VK1-17 and VK1-6 both have LQ, and so on. The sequences in these positions are known in the art and can be obtained, for example, from Scaviner et al., Exp. Clin. Immunogenet., 1999, 16: 234 (see Figure 2).

### CDRH3 LIBRARIES

The following examples describe methods and compositions useful for the design and synthesis of antibody libraries with improved CDRH3 sequences in comparison to libraries known in the art. The CDRH3 sequences of the invention have enhanced diversity in comparison to libraries known in the art, while retaining the character of human sequences, improving combinatorial efficiency of the synthetic CDRH3 segments, and/or improving the matching between synthetic CDRH3 sequences and human CDRH3 sequences in one or more reference sets.

### Example 3. Generating a Curated Reference Set of Human Preimmune CDRH3 Sequences

A file containing approximately 84,000 human and mouse heavy chain DNA sequences was downloaded from the BLAST public resource (ftp.ncbi.nih.gov/blast/db/FASTA/; filename: igSeqNt.gz; download date: Aug 29, 2008). Of these approximately 84,000 sequences, approximately 34,000 sequences were identified as human heavy chain sequences based on analysis of the sequence header annotation. These sequences were then filtered as follows: First, all sequences were classified, via their VH-region, according to their corresponding (closest matched) VH germline. Sequences that were of an incorrect or insufficient length, or that could not be matched due to extensive mutation, were discarded. Second, any sequences containing more than five mutations, at the DNA level, when compared to their corresponding germline VH sequence were also discarded. It was assumed, consistent with Rada and Milstein, EMBO J., 2001, 20: 4570, that mutations (or lack thereof) in the N-terminal portion of the variable region may be used as conservative surrogates for mutations (or lack thereof) in the C-terminal portion of the variable region, in particular in CDRH3. Therefore, selecting only sequences with five or fewer nucleotide mutations in VH, which is N-terminal to CDRH3 is highly likely to also select for CDRH3 sequences that are either lightly mutated or not mutated at all (*i.e.,* having preimmune character).

After translating the remaining DNA sequences into their amino acid counterparts, the appropriate reading frame containing the heavy chain germline amino acid sequence was identified and used to identify the sequences of the CDRs, including that of the CDRH3. The list of CDRH3 sequences obtained at this point was further filtered to eliminate members that did not differ from any other sequence in the set by at least three amino acids (after matching for length). This process yielded 11,411 CDRH3 sequences, with 3,571 sequences annotated as originating from healthy adults ("Healthy Preimmune Set" or "HPS"; *see* Appendix A for GI Nos.) and the other 7,840 sequences annotated as originating from individuals suffering from disease, of fetal origin, or of antigen-specific origin. The methods described below were then used to deconvolute each of the sequences in the HPS into the four segments that constitute the CDRH3: (1) TN1, (2) DH, (3) N2, and (4) H3-JH.

### Example 4. Method to Match Segments from a Theoretical Segment Pool to CDRH3s in a Reference Set

This example describes the method used to identify the TN1, DH, N2, and H3-JH segments of the CDRH3s in the HPS. The currently exemplified approach to the design and synthesis of human CDRH3 sequences mimics the segmental V-D-J gene recombination processes by which the human immune system generates the preimmune CDRH3 repertoire. The matching method described here determines which TN1, DH, N2 and H3-JH segments have been used to produce a particular CDRH3 across a reference set of CDRH3s (*e.g.,* the HPS). This information is then used, optionally in conjunction with other information described below (*e.g.,* physicochemical properties), to determine which TN1, DH, N2, and H3-JH segments from a theoretical segment pool (or segments extracted from the CDRH3 sequences in the reference set, in the case of the TN1 andN2) should be included in a synthetic CDRH3 library.

The inputs to the matching method are: (1) a reference set of CDRH3 sequences (*e.g.,* the human CDRH3 sequences in the HPS), and (2) a theoretical segment pool, containing a plurality of TN1, DH, N2 and/or H3-JH segments. Methods by which the members of the theoretical segment pool are generated are more fully described below. For each CDRH3 in the reference set, the matching method generates two outputs: (i) a list of the closest matched CDRH3 sequences that can be generated using the segments of the theoretical segment pool, and (ii) the one or more segment combinations from the theoretical segment pool that can be used to create these closest matched CDRH3 sequences.

The matching method was performed as follows: Each TN1 segment in the theoretical segment pool was aligned at its first amino acid with the first amino acid (position 95) of the CDRH3 sequence from the reference set. For each segment length, all (*i.e.,* one or more) of the segments returning the best matches are retained, and the remaining segments are discarded. The retained TN1 segments are then concatenated with all DH segments from the theoretical segment pool, to create [TN1]-[DH] segments. These segments are then aligned as above, and all the best matches for each of the [TN1]-[DH] segments are retained. The procedure is repeated with [TN1]-[DH]-[N2] and [TN1]-[DH]-[N2]-[H3-JH] segments until the length of the CDRH3 sequence from the reference set is identically recapitulated by the segment combinations from the theoretical segment pool. All segment combinations returning the best match to the CDRH3s in the reference set are retained as the output of the matching method.

Table 9 provides an example of the output of the matching method, specifically the output for four individual sequences from the HPS, using a theoretical segment pool designated "Theoretical Segment Pool 1," or "TSP1". TSP1 contains several theoretical segment pools, namely: 212 TN1 segments (Table 10), 1,111 DH segments (Table 11), 141 N2 segments (Table 12), and 285 H3-JH segments (Table 13). The CDRH3 sequence in Test Case 1 contains an identical match in TSP1 that is reached via a unique combination of the four segments. Test Cases 2.1 and 2.2 each return an identical match, but via two distinct combinations that differ in the TN1 and DH segments. In Test Cases 3.1, 4.1, and 4.2, the closest matches are all a single amino acid away from the reference CDRH3, and can be reached via one (3.1) or two (4.1 and 4.2) combinations of segments from TSP1. This approach can be generalized to find all of the closest matches to any reference CDRH3 sequence within any theoretical segment pool and all combinations of the segments within the theoretical segment pool that can produce the reference CDRH3 sequence exactly and/or its closest matches.

### Example 5. Deriving Theoretical Segment Pools of H3-JH Segments

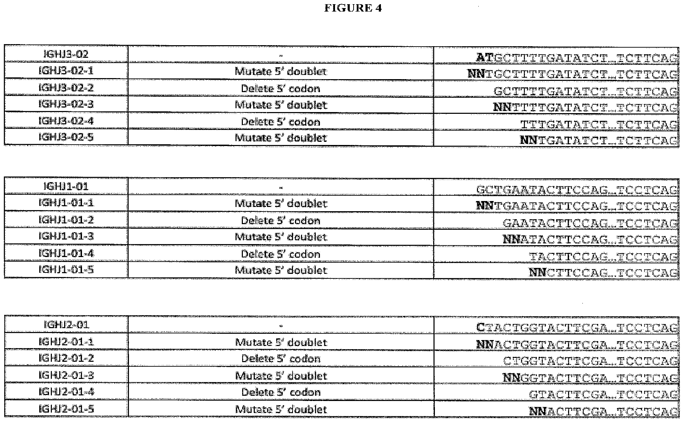

In order to produce theoretical segment pools of H3-JH segments for consideration for inclusion in a synthetic CDRH3 library, the following method was applied to generate mutants of seven (IGHJ1-01, IGHJ2-01, IGHJ3-02, IGHJ4-02, IGHJ5-02, IGHJ6-02 and IGHJ6-03) of the twelve germline IGHJ sequences of Table 14. These seven alleles were chosen because they were among the most commonly occurring alleles in human sequences. Libraries where all sequences of Table 14 (some differing only in FRM4) are used to generate H3-JH and/or JH (*i.e.,* H3-JH and FRM4) also fall within the scope of the invention. The method is intended to simulate the creation of junctional diversity during the V-D-J recombination process *in vivo,* which occurs via enzyme-mediated addition and deletion of nucleotides to the germline gene segments. The method proceeds as follows, and results in a fully enumerated theoretical segment pool of H3-JH segments:
1. A pre-treatment was applied to the IGHJ genes that contain a partial codon consisting of two nucleotide bases at their 5' terminus (IGHJ3-02, IGHJ4-02, IGHJ5-02, IGHJ6-02 and IGHJ6-03), prior to the first nucleotide encoding the translation of the JH segment that produces the well-known JH framework regions. For example, the IGHJ3-02 gene contains an AT dinucleotide sequence prior to the first nucleotide encoding the translation of the JH segment that produces the JH framework region (Figure 4, top). All partial codons consisting of two nucleotide bases were completed, using all possible nucleotide doublets (*i.e.,* NN) at their two most 5' positions (Figure 4, top, second row for IGHJ3-02). More specifically, the most 5' nucleotide in the germline sequence was mutated to N and an additional N was added 5' to that nucleotide.
2. IGHJ genes IGHJ1-01 (Figure 4, center) and IGHJ2-01 (Figure 4, bottom) contain zero and one nucleotide base(s) at their 5' termini, prior to the first nucleotide encoding the translation of the JH segment that produces the JH framework region. For these genes, the pre-treatment described in step 1 was not performed. Instead, the 5' doublets were mutated to NN (Figure 4, middle and bottom, second row of each). Therefore, after performing this step, each of the seven IGHJ genes enumerated above was converted to a variant with an NN doublet as its first two 5' positions.
3. The 5' codons of the sequences produced via steps 1 and 2 were then deleted, and the first two bases of the resulting DNA sequence were subsequently mutated to an NN doublet (Figure 4, rows 3-4 for all).
4. The 5' codons of the sequences produced in step 3 were then deleted, and the first two bases of the resulting DNA sequence were subsequently mutated to an NN doublet (Figure 4, rows 5-6 for all).
5. Each of the polynucleotide sequences generated by steps (1)- (4) were then translated, to obtain a theoretical segment pool consisting of 248 parent H3-JH polypeptide segments (Table 15) from the reading frame for each sequence that produced the JH framework region.
6. The parent H3-JH polypeptide segments were truncated at their N-termini, by removing one amino acid at a time until only the portion of the JH segment comprising FW4 remains (*i.e.,* an H3-JH segment with a length of zero amino acids).

The methods described above resulted in the production of a theoretical segment pool of 285 H3-JH segments (Table 13).

### Example 6. Deriving Theoretical Segment Pools of DH Segments

Two theoretical pools of DH segments were generated, using one or more of two translation methods, designated "Translation Method 0" (TM0), or "Translation Method 1" ("TM1"), each performed in the three forward reading frames of 27 human germline IGHD DNA sequences or segments derived therefrom (Table 16).

### The 1K DH Theoretical Segment Pool (1K DH)

TM1 was used to generate the "1K DH Theoretical Segment Pool" ("1K DH"; see the 1,111 DH segments of Table 11). In TM1, IGHD sequences that had a partial codon containing two untranslated bases after translation in any of the three forward reading frames were completed to produce a full codon only if the two bases could encode only a single amino acid upon completion. For example, a DNA sequence such as TTA-GCT-CG has two full codons that would be translated to LA, and a remaining partial codon (CG) that can only encode R, as any of CGA, CGC, CGG, or CGT will encode R. Thus, applying TM1 to this sequence will yield LAR. For sequences with partial codons that could encode more than one amino acid (*e.g.,* GA or AG), the partial codons were ignored. Applying TM1 to the 27 IGHD sequences of Table 16 generated a theoretical segment pool containing the 73 DH parent segments of Table 17 (some containing stop codons ("Z") and unpaired Cys residues). These sequences were then progressively deleted at the amino acid level, at their N- and C-termini, until only two amino acids remained. Truncated segments were discarded if they contained a stop codon, unpaired Cys residues, N-linked glycosylation motifs, or deamidation motifs. This process yielded the 1,111 DH segments of Table 11.

### The 68K DH Theoretical Segment Pool (68K DH)

The 27 IGHD genes and alleles of Table 16 were progressively deleted on either or both of their 5' and 3' ends until four bases remained, yielding 5,076 unique polynucleotide sequences of four or more nucleotides. These 5,076 sequences were subjected to systematic addition of 0, 1 and/or 2 N nucleotides their 5' and/or 3' ends. The resulting sequences were translated using TM0. In TM0, only full codons are translated; partial codons (*i.e.,* 1 or 2 bases) are ignored. This method yielded 68,374 unique DH polypeptide segments after elimination of segments with stop codons, unpaired Cys residues, Asn in the last or next to last position that can lead to N-linked glycosylation motifs, and deamidation motifs (the "68K DH Theoretical Segment Pool"). Using the IGHD genes of Table 16 as an input for the PYTHON computer code provided below will reproduce the exact theoretical segment pool of 68,374 DH segments. There are two free parameters in this program: (1) the minimum length of the DNA sequences remaining after progressive deletions (4 bases in this example), and (2) the minimum length of the peptide sequences (2 amino acids in this example) acceptable for inclusion in the theoretical segment pool. These parameters can be changed to alter the output of the program. For example, changing the first parameter to one base and the second parameter to one amino acid would lead to a larger theoretical segment pool with 68,396 unique sequences, including 18 single-amino acid segments. DH segments progressively truncated to different lengths also fall within the scope of the invention; for example those truncated to 1, 2, 3, or 4 or more amino acids, or 1, 2, 3, 4, 5, 6, 7, 8, 9, 10, or more nucleotides prior to translation.

### PYTHON Computer Program to Generate 68,374 DH Segments

```
                import math, sys, string
               class genes:
                        name = 'x'
                        seq = 'x'
                        progdel = set()
               class table:
                   name = 'x'
                   dna = 'x'
                   dna_n = 20 * ['x']
                   prot = 60 * ['x']
                   uprot = set()
               pepcod = { 'A':0, 'C':1, 'D':2, 'E': 3, 'F': 4, 'G': 5, 'H': 6,
                'I':7, 'K':8, 'L': 9, 'M': 10,
                'N':11, 'P':12, 'Q':13, 'R': 14, 'S': 15, 'T': 16, 'V':17, 'W':18,
                'Y': 19, 'Z': 20}
               codpep = 21 * ['']
               codpep[0] = 'A'
               codpep[1] = 'C'
               codpep[2] = 'D'
               codpep[3] = 'E'
               codpep[4] = 'F'
               codpep[5] = 'G'
               codpep[6] = 'H'
               codpep[7] = 'I'
               codpep[8] = 'K'
               codpep[9] = 'L'
               codpep[10] = 'M'
               codpep[11] = 'N'
               codpep[12] = 'P'
               codpep[13] = 'Q'
               codpep[14] = 'R'
               codpep[15] = 'S'
               codpep[16] = 'T'
               codpep[17] = 'V'
               codpep[18] = 'W'
               codpep[19] = 'Y'
                # Z represents a stop codon
               codpep[20] = 'Z'
               bases = 'ACGT'
               def translate_dna(sequence):
                # Translation of input DNA sequence using standard genetic code
                # Only full codons are considered with any remaining 1 or 2 bp being
                ignored
                # Z represents a stop codon
                             code = {
                             'ATA':'I', 'ATC':'I', 'ATT':'I', 'ATG':'M',
                             'ACA':'T', 'ACC':'T', 'ACG':'T', 'ACT':'T',
                             'AAC':'N', 'AAT':'N', 'AAA':'K', 'AAG':'K',
                             'AGC':'S', 'AGT':'S', 'AGA':'R', 'AGG':'R',
                             'CTA':'L', 'CTC':'L', 'CTG':'L', 'CTT':'L',
                             'CCA':'P', 'CCC':'P', 'CCG':'P', 'CCT':'P',
                             'CAC':'H', 'CAT':'H', 'CAA':'Q', 'CAG':'Q',
                             'CGA':'R', 'CGC':'R', 'CGG':'R', 'CGT':'R',
                             'GTA':'V', 'GTC':'V', 'GTG':'V', 'GTT':'V',
                             'GCA':'A', 'GCC':'A', 'GCG':'A', 'GCT':'A',
                             'GAC':'D', 'GAT':'D', 'GAA':'E', 'GAG':'E',
                             'GGA':'G', 'GGC':'G', 'GGG':'G', 'GGT':'G',
                             'TCA':'S', 'TCC':'S', 'TCG':'S', 'TCT':'S',
                             'TTC':'F', 'TTT':'F', 'TTA':'L', 'TTG':'L',
                             'TAC':'Y', 'TAT':'Y', 'TAA':'Z', 'TAG':'Z',
                             'TGC':'C', 'TGT':'C', 'TGA':'Z', 'TGG':'W',
                             }
                             proteinseq = "
                             for n in range(0,len(sequence),3):
                                  if code.has_key(sequence[n:n+3]) == True:
                                        proteinseq += code[sequence[n:n+3]]
                             return proteinseq
                # main body starts here
                # open input and output files
                in1 = open(sys.argv[1], 'r')
               ou1 = open(sys.argv[2], 'w')
                # read DNA sequences for input DH segments
               data = in1.readlines()
               nseg = len(data)
                seqs = [ genes() for i in range(nseg) ]
                for i in range(nseg):
                  line = data[i]
                  words = string.split(line)
                  seqs[i].name = words[0]
                  seqs[i].seq = words[1]
                  seqs[i].progdel = set()
                # Define here minimum length for DNA (4) and for protein (2)
               minlen = 4
               minp = 2
                # Implement progressive base by base deletion from 5' or 3' or both
               ends
               alln = 0
                for i in range(nseg):
                        seq = seqs[i].seq
                        lseq = len(seq)
                        nt = ct = lseq
                        for n in range(nt):
                                for c in range(ct):
                                        nseq = seq[n:lseq-c]
                                         if (len(nseq) >= minlen):
                                                 seqs[i].progdel.add(nseq)
                        alln += len(seqs[i].progdel)
                # Collect unique DNA sequences across all DH genes of origin and
                ignore redundant ones
               progdel = [ table() for i in range(alln) ]
               n = 0
                for i in range(nseg):
                        for kk in seqs[i].progdel:
                                unix = 1
                                for j in range(n):
                                         if (kk == progdel[j].dna):
                                                 unix = 0
                                                 break
                                if (unix == 1):
                                        progdel[n].name = seqs[i].name
                                        progdel[n].dna = kk
                                        n +=1
                # Add none, 1 or 2 bp on one or both ends
                # extras 20 + 20 * (21) = 20 + 420 = 440
                # allocate memory for all variants
                for i in range(n):
                        progdel[i].dna_n = 440 * ['x']
                        progdel[i].prot = 441 *3 * ['x']
                        progdel[i].uprot = set()
                # add 1 or 2 bases at each end of input segment
               tot = 0
                for i in range(n):
                # Step over each unique DNA sequence
                  k = 0
                # One base on 5' end combined with 1 or 2 bases added to 3' end
                  for 15 in range(4):
                     progdel[i].dna_n[k] = bases[15] + progdel[i].dna
                     k +=1
                     for 13 in range(4):
                       progdel[i].dna_n[k] = bases[15] + progdel[i].dna + bases[13]
                        k +=1
                     for 13 in range(4):
                        for m3 in range(4):
                          progdel[i].dna_n[k] = bases[15] + progdel[i].dna + bases[13]
               + bases[m3]
                          k +=1
                # One or two bases added to 3' only in this part
                  for 13 in range(4):
                     progdel[i].dna_n[k] = progdel[i].dna + bases[13]
                     k +=1
                  for 13 in range(4):
                     for m3 in range(4):
                       progdel[i].dna_n[k] = progdel[i].dna + bases[13] + bases[m3]
                        k +=1
                # Two bases on 5' end combined with 1 or 2 bp on 3' end
                  for 15 in range(4):
                     for m5 in range(4):
                       progdel[i].dna_n[k] = bases[15] + bases[m5] + progdel[i].dna
                        k +=1
                        for 13 in range(4):
                          progdel[i].dna_n[k] = bases[15] + bases[m5] + progdel[i].dna
               + bases[13]
                          k +=1
                        for 13 in range(4):
                          for m3 in range(4):
                             progdel[i].dna_n[k] = bases[15] + bases[m5] +
               progdel[i].dna + bases[13] + bases[m3]
                             k +=1
                # Now translate in all 3 forwared reading frames
                # Save unique peptide sequences
                  for fr in range(3):
                     piece = progdel[i].dna
                     piece = piece[fr:]
                     tpiece = translate_dna(piece)
                     progdel[i].prot[fr] = tpiece
                     progdel[i].uprot.add(tpiece)
                     for k in range(440):
                       piece = progdel[i].dna_n[k]
                       piece = piece[fr:]
                        tpiece = translate_dna(piece)
                       progdel[i].uprot.add(tpiece)
                       progdel[i].prot[3+440*fr +k] = tpiece
                  tot += len(progdel[i].uprot)
                # Collect unique sequences with no ASN at last or next to last
               position, no unpaired or consecutive CYS, no stops
               unset = set()
                segm = [ genes() for i in range(tot) ]
                lux = 0
               nn = 0
                for i in range(n):
                        k = 0
                        for kk in progdel[i].uprot:
                # Filter out sequences with undesired features, including length
               being too short (under "minp" defined above)
                                if (len(kk) < minp): continue
                                if (kk[len(kk)-1] == "N" or kk[len(kk)-2] == "N"):
               continue
                                if (kk.count("Z") > 0 or kk.count("CC") >0 or
                kk.count("C") % 2 >0): continue
                                unset.add(kk)
                                lux1 = len(unset)
                                if (lux1 > lux):
                                         segm[nn].name = progdel[i].name + "_" + str(nn)
                                         segm[nn].seq = kk
                                        nn += 1
                                lux = lux1
                                k +=1
                # Print out unique peptide sequences that pass all the filters
                for i in range(nn):
                        ou1.write("%s\t%s\n" % (segm[i].name, segm[i].seq))
```

### Example 7. Deriving Theoretical Segment Pools of TN1 and N2 Segments

The libraries of this example are designed to, in some instances, have a greater diversity in their TN1 and N2 segments in comparison to other libraries known in the art. The diversity of the TN1 and N2 segments was increased by using the matching method described in Example 4 to deconvolute the CDRH3 sequences in the HPS into their constituent segments *(i.e.,* TN1, DH, N2, and H3-JH), followed by extraction of "novel" TN1 and N2 segments in the manner described below. For the purposes of the invention, "novel" TN1 and N2 segments are TN1 and N2 segments that do not appear in a theoretical segment pool that is matched to a reference set of CDRH3 sequences. Following is an example of the method used to extract novel TN1 and N2 segments from the HPS. This method can be generalized to extract novel TN1 and N2 segments from any reference set of CDRH3 sequences, using any theoretical segment pool containing TN1, DH, N2, and/or H3-JH segments.

Table 9 provides the matching results for the reference CDRH3 sequence ERTINWGWGVYAFDI (SEQ ID NO: 8760) (Test Cases 5.1-5.4) from the HPS, using Theoretical Segment Pool 1 ("TSP1"). The best matches to the reference CDRH3 are four CDRH3 sequences, each within three amino acids of the reference CDRH3 sequence. In each of these matches, the TN1, DH, N2 and H3-JH segments are of length 4, 3, 3 and 5 amino acids, respectively. Thus the reference CDRH3 can be deconvoluted into the following segments: ERTI-NWG-WGW-YAFDI (SEQ ID NO: 8761) (*i.e.* [TN1]-[DH]-[N2]-[H3-JH], respectively). The DH and H3-JH segments from the reference CDRH3, NWG and YAFDI (SEQ ID NO: 4540) respectively, are identically present in TSP1. However, the TN1 (ERTI) (SEQ ID NO: 8718) and N2 (WGW) segments from the reference CDRH3 are absent in TSP1 and match TSP1 segments with one or more amino acid mismatches. These "novel" TN1 and N2 segments are extracted from the reference CDRH3 and considered for inclusion prospective theoretical segment pools and/or synthetic libraries. Additional novel TN1 and N2 segments were accumulated by applying this analysis to all members of the HPS. In order to robustly identify TN1 and N2 sequences, the extraction was performed only for those CDRH3 sequences in which the DH and H3-JH segments in the reference CDRH3 and TSP1 cumulatively return no more than 3 amino acid mismatches, implying that the DH and H3-JH segments of the reference CDRH3 had been reliably assigned.

### Example 8. Calculation of Segment Usage Weights

Segment usage weights were calculated for their utility in determining which segments from the theoretical segment pools (*e.g.,* TSP1 and TSP1 plus novel TN1 and N2 segments identified as described in Example 7) should be included in a synthetic library. Segment usage weights were obtained by utilization of the matching method described above and Equation 2: $w \left(i\right) = \frac{1}{{S}_{m}} {\sum }_{j = 1}^{{S}_{m}} \frac{1}{g \left(j\right)} {\sum }_{k = 1}^{g \left(j\right)} {f}_{i} \left(k\right)$ where,
- w(i): Weight for segment i. 0 ≤ *w*(*i*) ≤ 1.
- *Sₘ*: Number of sequences (out of total S in the reference CDRH3 set) which contain one or more best matches with no more than m amino acid mismatches in the specified region of the reference CDRH3 sequence. Here, the mismatches are computed over the Kabat-CDRH3 region, but other fragments of the CDRH3 sequences may also be considered. A constant value of *m*=3 was used here, but other values may be used, or the value may depend on the length of the reference CDRH3 sequence.
- g(j): Total number of degenerate segment combinations producing the best match to the reference CDRH3 sequence *j*.
- *fᵢ*(*k*): Fractional amino acid identity of TN1, DH, N2 or H3-JH segment in degenerate match *k*, relative to the corresponding sequence fragment in the reference CDRH3 sequence *j*. The fractional amino acid identity equals zero if the segment does not appear in match *k.* Other definitions of *f*, such as amino acid similarity (e.g., based on physicochemical properties of the amino acids such as hydrophobicity), instead of identity, may be also used.

The procedure for calculating segment usage weights will be further exemplified below. In each of these examples, the best match combinations from TSP1 are provided for a single CDRH3 sequence (*Sₘ* = 1) and the degeneracy (k) and fractional mismatch (*f*) dependent weight calculations are explained.

### Example 8.1. Segment usage weights for Test Case 1 in Table 9

Refer to Test Case 1 in Table 9. The CDRH3 sequence RTAHHFDY (SEQ ID NO: 3660) is identically located in TSP1 (f= 1, subscripts dropped for simplicity) via a unique segment combination (g = 1). Table 18 provides the usage weights for the segments corresponding to the best match from TSP1 for the CDRH3 of Test Case 1.

### Example 8.2. Segment usage weights for Test Cases 2.1 and 2.2 in Table 9

Refer to Test Cases 2.1 and 2.2 in Table 9. The CDRH3 sequence VGIVGAASY(SEQ ID NO: 3661) may be identically located in TSP1 (*f*= 1) via two distinct segment combinations (*g* = 2). Table 19 provides the usage weights for the segments corresponding to the best match from TSP1 for the CDRH3 of Test Cases 2.1 and 2.2.

### Example 8.3. Segment usage weights for Test Case 3.1 in Table 9

Refer to Test Case 3.1 in Table 9. The CDRH3 sequence DRYSGHDLGY (SEQ ID NO: 3662) may be identically located in TSP1 via a unique segment combination (*g* = 1) with a single amino acid difference. As provided below, the TN1, N2 and H3-JH segments match the corresponding reference sequence fragments identically, while four of the five DH amino acids match identically.
*Sequence from the HPS:* DR-YSG**H**D-LG-Y (SEQ ID NO: 3662)
*Nearest Neighbor in TSP1:* DR-YSG**Y**D-LG-Y (SEQ ID NO: 8719)
Thus, here
*f* = 4/5 for the DH segment; and
= 1 for the TN1, N2, and H3-JH segments (Table 20).

### Example 8.4. Matching of Test Cases 4.1 and 4.2 in Table 9.

Refer to test cases 4.1 and 4.2 in Table 9. The CDRH3 sequence GIAAADSNWLDP (SEQ ID NO: 3663) may be located in TSP1 via two distinct segment combinations (*g* = 2), each with a single amino acid difference. As provided below, the TN1, DH and N2 segments match the corresponding reference sequence fragments identically, while five of the six H3-JH amino acids match identically.

| | |
|---|---|
| *Sequence from HPS:* | (-)-GIAAA-D-SNW**L**DP (SEQ ID NO: 3663) |
| *Nearest Neighbor in TSP1:* | (-)-GIAAA-D-SNW**F**DP (SEQ ID NO: 8720) |
| *Sequence from HPS:* | G-IAAA-D-SNW**L**DP (SEQ ID NO: 3663) |
| *Nearest Neighbor in TSP1:* | G-IAAA-D-SNW**F**DP (SEQ ID NO: 8720) |
| | *Here, (*-*) represents the "empty" TN1 segment.* |

Applying Equation 2 results in the segment usage weights provided in Table 21.

### Example 8.5. Calculating the Segment usage weights for Test Cases 1 to 4.2 of Table 9

Extending the individual calculations described above to simultaneously include all of Test Cases 1 to 4.2 of Table 9 results in the segment usage weights of Table 22.

### Example 8.6. Calculating the Segment usage weights for Test Cases 5.1 to 5.4 of Table 9

Refer to the CDRH3 sequence ERTINWGWGVYAFDI (SEQ ID NO: 8760) and the novel TN1 and N2 segments extracted from the CDRH3 sequence, in Example 7. In this case, the novel TN1 and N2 segments (ERTI (SEQ ID NO: 8718) and WGV respectively), and the DH and H3-JH segments from TSP1 (NWG and YAFDI (SEQ ID NO: 4540) respectively) are each assigned usage weights of unity.

### Example 9. Selection of TN1, DH, N2 and JH Segments for Inclusion in Synthetic Libraries

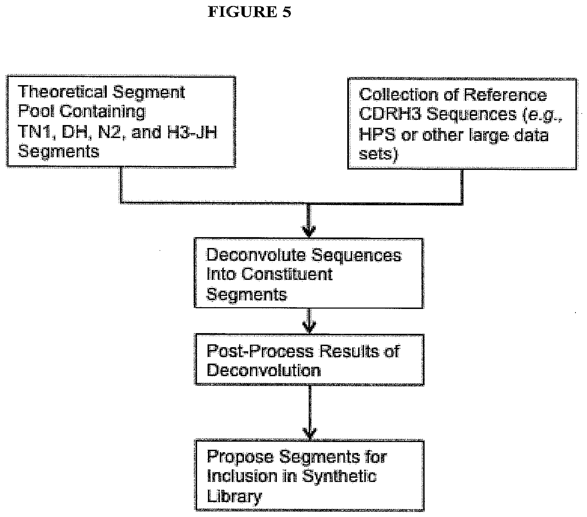

Figure 5 provides the general method used for the design of synthetic CDRH3 libraries. The method uses as input: (1) a theoretical segment pool containing TN1, DH, N2, and H3-JH segments (e.g., TSP plus novel TN1 and N2 segments); and (2) a collection of reference CDRH3 sequences (e.g., the HPS). From these inputs, a particular subset of segments from the theoretical segment pool is selected for inclusion in a physical CDRH3 library.

First, the best matches to the CDRH3s of the HPS were obtained, from within the TSP1 set, with or without the novel TN1 and N2 segments, using the matching method described above. This data was then used to compute the segment usage weights via Equation 2. Segments were prioritized for inclusion in the physical library based on their relative frequency of occurrence in the CDRH3 sequences of the HPS (as indicated by the segment usage weights), as well as other factors (more fully described below), such as hydrophobicity, alpha-helical propensity, and expressibility in yeast.

### Example 9.1. Exemplary Library Design (ELD-1)

ELD-1 uses the HPS and the segments from TSP1 1 (9.5 x 10⁹ members) as inputs and produces an output of 100 TN1, 200 DH, 141 N2 and 100 H3-JH segments, each from TSP1, ranked in order by their usage weights in the HPS, to produce a library with theoretical complexity of 2.82 x 10⁸. The segments corresponding to ELD-1 are provided in Table 23. Note that here the combination of all of the segments (*i*.*e*., TN1, DH, N2, and H3-JH), and the individual sets of segments *(i.e.,* TN1 only, DH only, N2 only, and H3-JH only) each constitute theoretical segment pools.

### Example 9.2. Exemplary Library Design 2 (ELD-2)

The inputs for this design are the HPS and the segments from TSP1 plus the novel TN1 and N2 segments extracted from the HPS (Example 7). The outputs are (1) 200 DH and 100 H3-JH segments, each from TSP1; and (2) 100 TN1 and 200 N2 segments including TN1 and N2 segments originally in TSP1 and those extracted from the sequences in the HPS. Applying the method described in Example 7 to extract novel TN1 and N2 segments (*i.e*., those not included in TSP1) resulted in the identification of 1,710 novel TN1 segments and 1,024 novel N2 segments. The segments corresponding to ELD-2 are provided in Table 24. Note that here the combination of all of the segments *(i.e.,* TN1, DH, N2, and H3-JH), and the individual sets of segments *(i.e.,* TN1 only, DH only, N2 only, and H3-JH only) each constitute theoretical segment pools. As in ELD-1, all segments in ELD-2 were selected for inclusion based solely on their usage weights in the HPS.

### Example 9.3. Exemplary Library Design 3 (ELD-3)

The inputs for this design are identical to those for ELD-2. As in ELD-2, the outputs are (1) a set of 200 DH and 100 H3-JH segments,, each from TSP1; and (2) a set of 100 TN1 and 200 N2 segments, including TN1 and N2 segments originally in TSP1 and those extracted from the sequences in the HPS (Example 7). However, the approach used for the selection of the segments for ELD-3 differs in two respects. First, selected physicochemical properties of the segments (hydrophobicity, isoelectric point, and alpha-helix propensity) were used, in addition to the segment usage weights, to prioritize segments for inclusion in the physical library. Hydrophobicity was used to de-prioritize hydrophobic DH segments that are empirically over-represented in poorly expressed antibodies isolated from yeast-based libraries. Isoelectric point and propensity for alpha-helix formation were utilized to identify segments located in regions of physicochemical property space that were relatively unexplored in CDRH3 libraries known in the art (*e.g*., U.S. Publication Nos. 2009/0181855 and 2010/0056386, and WO/2009/036379). Second, the segment usage weights were calculated via a bootstrap analysis of the HPS dataset. These methods are more fully described below. The segments corresponding to ELD-3 are provided in Table 25. Note that here the combination of all of the segments (*i.e*., TN1, DH, N2, and H3-JH), and the individual sets of segments *(i.e.,* TN1 only, DH only, N2 only, and H3-JH only) each constitute theoretical segment pools.

### Example 9.3.1. Generation of Segment Usage Weights via Bootstrap Analysis

Bootstrap analysis (Efron & Tibshirani, An Introduction to the Bootstrap, 1994 Chapman Hill, New York) is a widely used statistical procedure for estimating the variability of a statistic of a given sample. This estimate is based on the value of the statistic calculated for several sub-samples, equal in size to the original sample and derived from it via sampling with replacement. Members of the original sample are chosen at random to form the sub-samples, and are typically included multiple times in each sub-sample (hence, "sampling with replacement").

Here, the original sample is the HPS dataset with *n*= 3,571 members and the statistic is the segment usage weight. One-thousand sub-samples, each with 3,571 members, were generated by randomly choosing sequences from the HPS dataset (no more than 10 repeats of a given sequence were allowed in each sub-sample). The matching method described above was then applied to each sub-sample, and the final segment usage weights were calculated as the average of the values obtained for the individual sub-samples. Average values derived via this bootstrap procedure are more robust than values calculated from the parent HPS dataset alone. Unless indicated otherwise, these average values of the 1,000 sub-samples were used in the selection of segments for ELD-3.

### Example 9.3.2. Amino Acid Property Indices

The *AAindex* database, available online at www.genome.jp/aaindex/, provides more than 500 numerical indices representing various physicochemical and biochemical properties of amino acids and pairs of amino acids. These properties include hydrophobicity, electrostatic behavior, secondary structure propensities and other characteristics, with several indices often available for a given property. The following three indices were chosen by starting with the well-understood Kyte-Doolittle hydropathy index (KYJT820101) and adding the indices most numerically de-correlated from it and each other. They thus potentially describe non-overlapping regions of amino acid property space and were used for analysis and selection of the DH and H3-JH segments for ELD-3:
1. KYTJ820101 (hydropathy index)
2. LEVM780101 (normalized frequency of alpha helix)
3. ZIMJ680104 (isoelectric point)

### Example 9.3.3. Hydrophobic DH Segments are Over-Represented in Poorly Expressed

### Antibodies Isolated from Yeast-Based Libraries

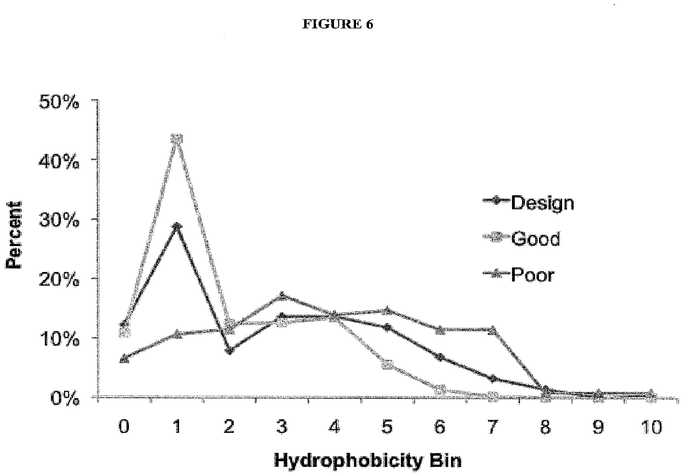

Based on protein expression levels from approximately 1200 antibodies expressed inS. *cerevisiae*, antibodies were classified as either "Good" or "Poor" expressors. The CDRH3 sequence of each antibody in each category was examined to identify sequence features that correlated with the expression level. One such sequence feature is the hydrophobicity of the DH segments calculated using the KYTJ8201 01 index. Figure 6 provides the frequency of "Good" and "Poor" expressors as a function of the DH segment hydrophobicity (increasing to the right). The distribution expected from the synthetic library used to isolate these antibodies is also provided as a reference ("Design"). DH segments with the highest hydrophobicity values (far right of the plot) are over-represented (relative to the expectation based on the design) among "Poor" expressors and under-represented among "Good" expressors. Similarly, hydrophilic DH segments (far left) are over-represented among "Good" expressors and under-represented among "Poor" expressors. From this data, it was inferred that the overall expressibility of the antibodies of the library may be improved by synthesizing CDRH3 sequences with fewer hydrophobic DH segments.

### Example 9.3.4. Selection of the 200 DH Segments for Inclusion in ELD-3

1. A set of 71 DH segments from TSP1 were designated as "core" DH segments for automatic inclusion in ELD-3. These segments had the following desirable properties:
2. Fifty-three of seventy-one were present within the top 7% of DH segments rank-ordered by segment usage weights from the bootstrap analysis.
3. Eighteen of seventy-one were present within the top 7% of DH segments rank-ordered by usage weights derived from antibodies isolated from libraries expressed in *S*. *cerevisiae.*

The remaining 1,040 segments were designated as "non-core." To complete the set of 200 segments in ELD-3, 129 segments were chosen from the "non-core" pool of segments in the following manner:
1. Sixty-five segments were eliminated because they contain either (a) an Asn residue at the last or next-to-last position with the potential to form N-linked glycosylation motifs via combination with N2 amino acids or (b) the amino acid sequence NG, implicated in de-amidation.
2. Segments with higher than median values for the KYTJ820101 hydropathy index (median=2.9 for 1K DH) were eliminated from further consideration. In view of the known importance of Tyr for antigen recognition (Fellouse et al., PNAS, 2004, 101: 12467; and Hofstadter et al., J. Mol. Biol., 1999, 285: 805), segments containing at least one Tyr residue were retained unless located in the highest hydrophobicity quartile (KYTJ820101 value higher than 9.4). This eliminated 443 segments.
3. The final set of 129 segments was obtained by using an objective function that aimed to maximize the Euclidean distance, between the "core" and the remaining 443 "non-core" segments, in a multidimensional space defined by the following variables: (1) amino acid mismatches to nearest neighbor; and (2) values of the three physicochemical property indices.

### Example 9.3.5. Selection of the 100 H3-JH Segments for Inclusion in ELD-3

One-hundred H3-JH segments were chosen for inclusion in ELD-3 in the following manner.
1. Twenty-eight H3-JH segments were selected after being experimentally validated in other libraries containing only these H3-JH segments (*see* U.S. Publication Nos. 2009/0181855 and 2010/0056386, and WO/2009/036379).
2. Fifty-seven segments were selected based on their presence within the top 25% of H3-JH segments rank-ordered by usage weights from the bootstrap analysis described above. These 57 H3-JH segments, plus the 28 H3-JH segments of (1) (*i.e*., 85 segments total) were designated as the "core" H3-JH segments, which, like the core DH segments, were automatically included in ELD-3.
4. Fifteen additional segments were chosen by using an objective function that aimed to maximize the Euclidian distance, between the "core" and the remaining 200 "non-core" segments, in a multidimensional space defined by the following variables: (1) amino acid mismatches to nearest neighbor; and (2) values of the three physicochemical property indices.

### Example 9.3.6. Selection of 100 TN1 and 200 N2 segments for Inclusion in ELD-3

TN1 and N2 segments were extracted from the sequences in each sub-sample of the bootstrap procedure, and the 100 TN1 and 200 N2 segments with the highest average segment usage weights were chosen for inclusion into the library, after elimination of sequences with undesirable motifs, namely Cys and Asn residues.

### Example 9.3.7. Selection of Nucleotide Sequences to Encode the Segments Chosen for Inclusion in ELD-3

Each of the polypeptide segments chosen for inclusion in the library must be back translated (polypeptide to DNA) into a corresponding oligonucleotide sequence. While a large number of oligonucleotides could possibly encode each polypeptide segment, due to the degeneracy of the genetic code, certain constraints were imposed to select oligonucleotides that were more desirable. First, since ELD-3 was expressed in yeast (*S*. *cerevisiae*), codons that are rarely used in yeast were avoided. For example, of the six possible codons for Arg, three: CGA, CGC and CGG are used to encode yeast proteins at rates of under 10% (see, for example, Nakamura et al., Nucleic Acids Res., 2000, 28:292), and therefore those three codons were avoided to the extent possible. Second, since many antibodies are produced in Chinese Hamster Ovary (CHO) cells (after discovery *e.g.*, in yeast), the CCG codon (encoding Pro) was also avoided, since it is rarely used by hamsters (Nakamura et al.)

A number of restriction enzymes are employed during the actual construction of the CDRH3 oligonucleotide library (see Example 10 of U.S. Pub. No. 2009/0181855). It is thus desirable to avoid the occurrence of recognition motifs for these restriction enzymes within the CDRH3 polynucleotide sequences. Codons are selected at the individual segment level to avoid introducing recognition motifs for restriction enzymes that may be used downstream. Since such motifs may also be generated by combinatorial assembly of the segments, the segment combinations are also checked and, whenever possible, codons are changed to eliminate the occurrence of such motifs. Specifically, three restriction enzymes were used during the construction of the currently exemplified CDRH3 library: BsrDI, BbsI, and AvrII. The first two are type II enzymes with non-palindromic recognition sites. The reverse strand of the oligonucleotides encoding the segments was checked explicitly for recognition sites for these two enzymes. In particular, the reverse strands were checked for the motifs GCAATG and CATTGC (for BsrDI) and GAAGAC and GTCTTC (for BbsI). The recognition motif for AvrII is palindromic so the oligonucleotides were only checked for the sequence CCTAGG. However, AvrII is used only to treat TN1 segments, and thus it is not necessary to evaluate its presence in the other segments or their combinations.

An additional constraint that was imposed to improve engineering of the polypeptide to polynucleotide conversion was avoidance of consecutive runs of 6 or more of the same type of base, as this is believed to increase errors during solid phase oligonucleotide synthesis. Therefore, DNA sequences for the segments of ELD-3 were chosen to avoid such motifs. The DNA sequences for the ELD-3 segments are included, with the respective polypeptide sequences, in Table 25. One of ordinary skill in the art will readily recognize that these methods can also be applied to any other library, any restriction sites, any number of nucleotide repeats, and/or to avoid the occurrence of any codons considered undesirable in any organism.

### Example 10. Matching of ELD-3 to Human CDRH3 Datasets and Clinically Relevant Antibodies

Among the objectives of the invention is to mimic the V-D-J recombination processes underlying the creation of the human CDRH3 repertoire *in vivo*, thereby increasing the diversity of the CDRH3 library in comparison to other libraries known in the art, while maintaining the human character of CDRH3. One measure of success is the extent to which collections of human reference CDRH3 sequences are represented identically, or via close matches (*e.g.*, less than about 5, 4, 3, or 2 amino acid differences) in any library of the invention. We evaluated this metric using two human CDRH3 sequence reference datasets, both non-overlapping with each other and the HPS: (1) a collection of 666 human CDRH3 sequences (Lee et al., Immunogenetics, 2006, 57: 917; "Lee-666"); and (2) a collection of 3,000 human CDRH3 sequences randomly chosen from the over 200,000 sequences disclosed in Boyd et al., Science Translational Medicine, 2009, 1: 1-8 ("Boyd-3000"). The results of the random sample of the 3,000 human CDRH3 sequences from Boyd *et al.* was representative of the results of the same analysis as applied to all members of the Boyd *et al.* set (>200,000 CDRH3 sequences).

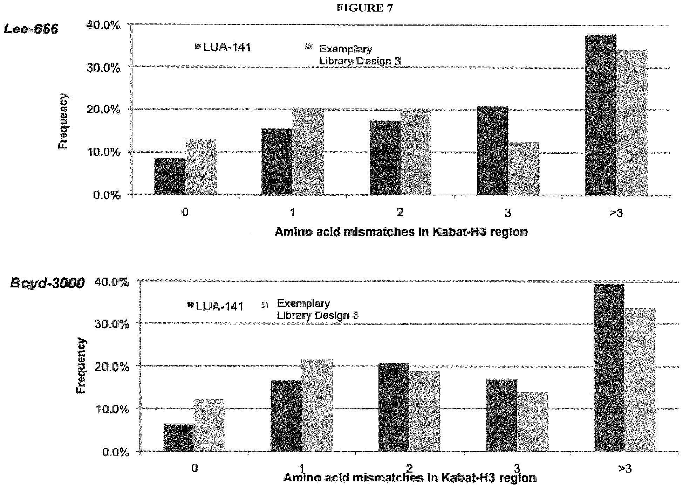

Figure 7 provides the percentage of CDRH3 sequences in two synthetic libraries, "LUA-141" and ELD-3, that match a sequence from the Lee-666 or Boyd-3000 sets with zero, one, two, three, or more than three amino acid mismatches. Here, "LUA-141" represents a library containing 212 TN1, 278 DH, 141 N2, and 28 H3-JH (*see* U.S. Publication No. 2009/0181855 for details). In particular, it is notable that ELD-3 exhibits a higher percentage of sequences (12.9% and 12.1% for the Lee-666 and Boyd-3000 sets, respectively) that identically match a reference CDRH3 sequence than LUA-141 (8.4% and 6.3% for the Lee-666 and Boyd-3000 sets, respectively). It is also notable that ELD-3 exhibits a higher cumulative percentage of human CDRH3 sequences found with no more than two amino acid mismatches (54.1% and 52.5% for the Lee-666 and Boyd-3000 sets, respectively) relative to LUA-141 (41.2% and 43.7% for the Lee-666 and Boyd-3000 sets, respectively).

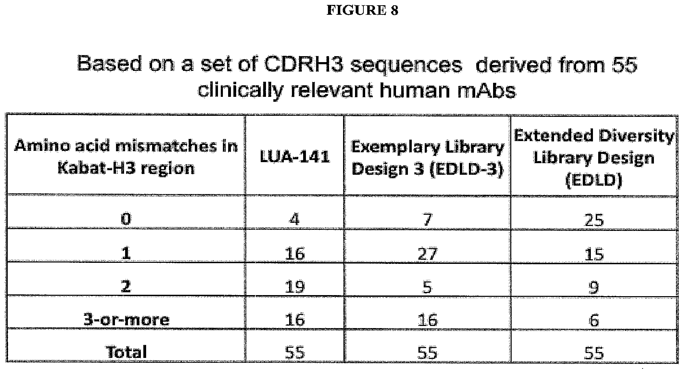

Another metric by which antibody libraries can be evaluated is their ability to match "clinically relevant" reference CDRH3 sequences. Figure 8 demonstrates that ELD-3 returns better matches to clinically relevant CDRH3 sequences than the LUA-141 library. Specifically, ELD-3 matches 34 of 55 (62%) clinically validated antibodies within one amino acid, while the LUA-141 library only matches 20 of 55 (37%).

### Example 11. Comparison of ELD-3 to LUA-141

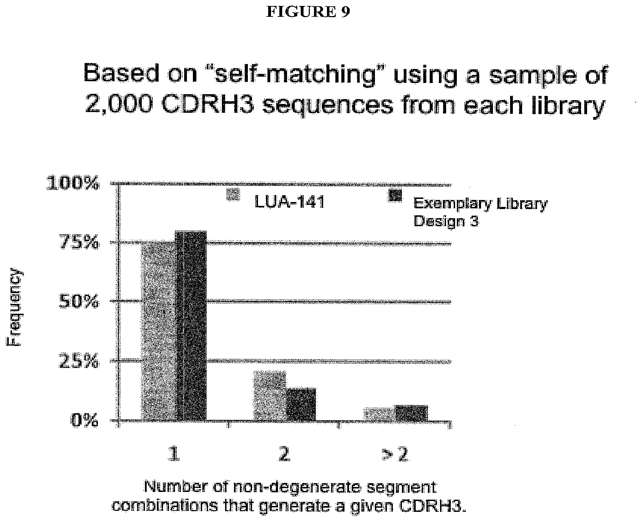

ELD-3 has 73 TN1, 92 DH, 119 N2, and 28 H3-JH in common with LUA-141. Thus, 94.5% of the sequences in ELD-3 (4.0 × 10⁸ members) are different from the LUA-141 library (2.3 × 10⁸ members). Figure 9 demonstrates that the combinatorial efficiency of the segments in ELD-3 is greater than that of the segments in LUA-141. Specifically, the ELD-3 segments are more likely to yield a unique CDRH3 than the LUA-141 library segments. This is advantageous, because it allows one to synthesize libraries with increased CDRH3 diversity using fewer segments.

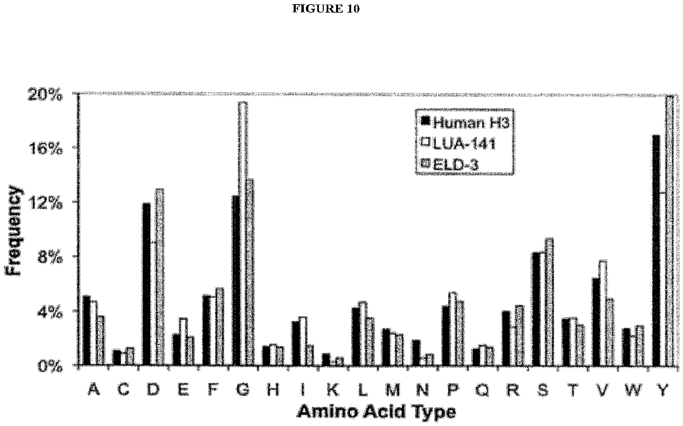

Figure 10 provides the amino acid compositions of the Kabat-CDRH3s of LUA-141, ELD-3, and Human CDRH3 sequences from the HPS.

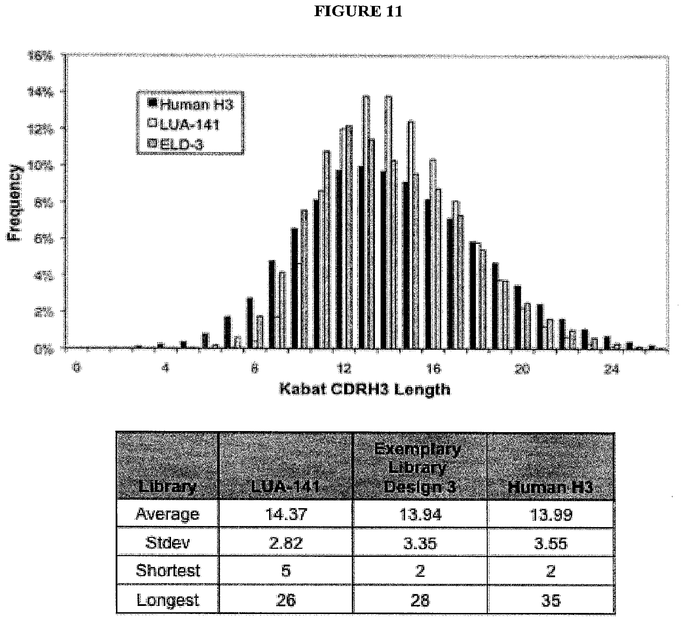

Figure 11 provides the Kabat-CDRH3 length distribution of LUA-141, ELD-3, and Human CDRH3 sequences from the HPS.

### CDRH3 Libraries Synthesized with Degenerate Oligonucleotides

### Example 12. Further Increasing CDRH3 Diversity by Utilizing Degenerate Oligonucleotides

The methods described in this example extend the methods taught above, to produce CDRH3 libraries with more members than those of the libraries described above. In particular, one or two degenerate codons were introduced into the DH and or N2 polynucleotide segments, and (generally) no degenerate codon or one degenerate codon were introduced into the H3-JH segments. Segments with different numbers of degenerate codons are also contemplated; for example DH segments with 0, 1, 2, 3, 4, 5, 6, 7, 8, or more degenerate codons, and H3-JH segments with 0, 1, 2, 3, 4, 5, or more degenerate codons. This results in CDRH3 libraries containing greater than about 10¹¹ (about 2×10¹¹) distinct CDRH3 amino acid sequences that closely reflect properties, such as length and composition among others, of a reference set of human CDRH3 sequences. As described below, the degenerate positions in the DH segments are usually, but not always, the very N- and/or C-terminal positions, or 5' and 3' end codons *(i.e.,* not necessarily only the first or last base), respectively, when considering the corresponding oligonucleotide sequences. Degenerate codons were similarly used to synthesize N2 segments. Two hundred of the TN1 segments were as described in ELD-3, although libraries with degenerate TN1 segments, or with alternative choices of TN1 segment sequences, fall within the scope of the invention. An additional one hundred TN1 segments complete the set of 300 TN1 segments for this library. The amino acid and nucleotide sequences are listed in Table 26. It is also possible to use mixtures of trinucleotides instead of, or in addition to, degenerate oligonucleotides in order to allow amino acid type variation at one or more selected positions within "base" or "seed" segment sequences (defined below).

### Example 13. Selection of DH Segments for Synthesis by Degenerate Oligonucleotides

The segment usage weights were calculated for the 68K DH Theoretical Segment Pool by comparison to the sequences contained in Boyd *et al.* The DH segments with a length of three or more amino acids were ranked according to their segment usage weights (as described above), and the top 201 were designated as "seed" sequences. These seed sequences were then varied by selecting certain positions to incorporate degenerate codons. The positions selected for variance, the amino acids types to which they were varied, were determined by comparing the seed sequences to a reference set of 9,171 DH segments that were a subset of the 68K DH Theoretical Segment Pool. These 9,171 DH segments were selectedbecause their segment usage weight in Boyd *et al.* was significant, meaning that the cumulative segment usage weight (Example 8) is at least 1.0.

Each of the 201 seed sequences was compared to each of the sequences in the reference set of 9,171 DH segments, and those of identical length and differing at a single position were further considered to inform possible variants of the seed. In this manner, the most variable position for each seed was identified and a set of candidate amino acid types was also identified for each position. Finally, a set of degenerate codons was considered, to identify the codon that most faithfully represented the set of candidate amino acid types for each particular position. Degenerate codons encoding stop codons, Cys residues, N-linked glycosylation motifs (*i.e*., NXS or NXT, where X is any amino acid type), or deamidation motifs (NG) were eliminated from consideration. This process generated 149 unique degenerate oligonucleotide sequences, which collectively encode 3,566 unique polypeptide sequences. Alternative designs generated according to the same principles were also considered, and those having a larger diversity (in terms of the number of unique polypeptide sequences) and smaller RMAX values (see below) were given preference for inclusion in the libraries of the invention. However, it is also conceivable that different criteria could be used to select DH segments from the 68K DH Theoretical Segment Pool, and that libraries including DH segments selected by these different criteria would also fall within the scope of the invention.

Because not all degenerate oligonucleotides encode an identical number of polypeptides, the latter do not occur with uniformly identical weights over the entirety of a given theoretical segment pool *(i.e.,* TN1, DH, N2 and H3-JH) contained within a CDRH3 library of the invention. For example, an individual amino acid sequence X encoded by an oligonucleotide of total degeneracy 4 will have a "weight" of 1/4, while another individual amino acid sequence, Y, encoded by an oligonucleotide of degeneracy 6 will have a weight of 1/6. Moreover, certain amino acid sequences could be encoded by more than one degenerate oligonucleotide, so their weights will be the sum of the individual contributions by each oligonucleotide. Within a given theoretical segment pool, the ratio of the weight of the most heavily weighted polypeptide to that of the least heavily weighted one, RMAX, is an important design criterion that one would ideally like to minimize. The RMAX value may be defined by length, or overall for all of the segments of any given type (*i*.*e*., all the DH segments, or all the H3-JH segments, and so on for the TN1, and/or the N2 segments). Table 27 lists the degenerate oligonucleotide sequences, while Table 28 lists the unique polypeptide sequences resulting from these oligonucleotides. These two tables include the DH dimer segments the design of which is detailed below.

### Example 13.1. Selection of DH Dimer Segments

A different method was employed to design a set of degenerate oligonucleotides encoding DH dimer sequences. The method aimed to include all of the 45 dimer sequences in ELD-3 plus as many of the other 400 theoretically possible dimer sequences *(i.e.,* 20 residues possible in each of 2 positions = 20 * 20), minus segments containing Asn (N) residues and excessively hydrophobic dimers *(i.e.,* any dimer combination comprising only F, I, L, M, and/or V residues). This design process ultimately yielded 35 degenerate oligonucleotide sequences encoding 213 unique peptide dimer sequences. As with the selection processes used for all of the other segments of the invention, one or ordinary skill in the art will readily recognize that other criteria could be used to select the DH dimer segments, and that libraries including these segments also fall within the scope of the invention.

Combining the DH dimer segments with the longer DH segments of Example 13, yielded the final set of DH segments of the currently exemplified library, encoded by a total of 184 oligonucleotides (35 encoding dimers and 149 encoding segments having three or more amino acids) versus the 200 oligonucleotides of ELD-3. The 184 oligonucleotides encode a total of 3,779 unique polypeptide sequences: 213 dimers and 3,566 longer segments of three amino acids or greater.

### Example 14. Generation of Expanded N2 Diversity

As described above, ELD-3 contains 200 N2 segments. In the currently exemplified library, the empty N2 segment *(i.e.,* no N2, so that the DH segments are joined directly to the H3-JH segments) and monomer N2 segments were the same as in ELD-3. However, degenerate oligonucleotides were used to generate sets of two-, three-, and four-mers that not only recapitulated all of the corresponding sequences in ELD-3 but also resulted in additional diversity. As with the DH segments, these degenerate oligonucleotides were designed to eliminate Asn (in unsuitable positions) and Cys residues, and stop codons. More specifically, Asn residues were allowed at the first position of trimers and at the first or second position of tetramers whenever the subsequent amino acid was not Gly and the next amino acid was not Ser or Thr, thus avoiding deamidation or N-linked glycosylation motifs within the candidate N2 segments. The N2 theoretical segment pool for the currently exemplified library contains one zero-mer *(i.e.,* no N2 segment), 18 monomer, 279 dimer, 339 trimers, and 90 tetramer N2 amino acid sequences, or 727 segments in total. These amino acid sequences are encoded by 1, 18, 81, 36, and 10 oligonucleotides, respectively, for a total of 146 oligonucleotides. All but the first 19 oligonucleotides, those encoding the zero- and one-mers, are degenerate. Table 29 lists the 146 oligonucleotide sequences, while Table 30 lists the resulting 727 unique polypeptide sequences.

### Example 15. Generation of Expanded H3-JH Diversity

Application of nucleotide-level progressive deletions on the 5' end of the human IGHJ polynucleotide segments down to the point where only the DNA sequence corresponding to FRM4 remained *(i.e.,* no H3-JH remained), followed by systematic 1- or 2-bp completions on the same 5' end, resulted in 643 unique H3-JH peptide segments after translation ("643 H3-JH Set"). As done with the DH segments, it is possible to rank order each of the 643 segments by their usage weights obtained after comparison to the approximately 237,000 human sequences from Boyd *et al*., and the top 200 individual sequences, from those devoid of the undesired motifs described above, were chosen to provide the set of H3-JH segments for the currently exemplified library.

In an alternatively exemplified embodiment, 46 of the 200 H3-JH segments were designed with a twofold degenerate codon in the first position (*i.e*., N-terminal or 5' end, respectively, at the peptide and oligonucleotide level), so that, overall, 200 oligonucleotides would encode 246 unique peptide sequences.

In yet other alternatively exemplified embodiments, further use of degenerate codons may be conceived to produce libraries encoded by 90, 100, 200 or more oligonucleotides representing up to 500 distinct polypeptide sequences. Preferably, but not necessarily, these up to 500 unique sequences could be a subset of the sequences in the 643 H3-JH reference set described above, or a subset of variants of these sequences. As exemplified above, H3-JH segments containing undesirable polypeptide motifs may be eliminated from the design. The oligonucleotide sequences for the JH segments are listed on Table 31, while the resulting unique polypeptide sequences are provided in Table 32. In Table 31, nucleotide sequences corresponding to the FRM4 region are also provided, but the "peptide length" value refers to the H3-JH portion only. For simplicity, only the H3-JH peptide sequences are included in Table 32.

### Example 16. Extended Diversity Library Design (EDLD)

The TN1, DH, H3-JH, and N2 segments selected above, and provided in Tables 26 to 32, were combined to generate an Extended Diversity Library Design (EDLD) with theoretical diversity of about 2×10¹¹ (300 TN1 × 3,779 DH × 727 N2 × 246 H3-JH). The oligonucleotides encoding the selected segments were chosen according to the principles of Example 9.3.7.

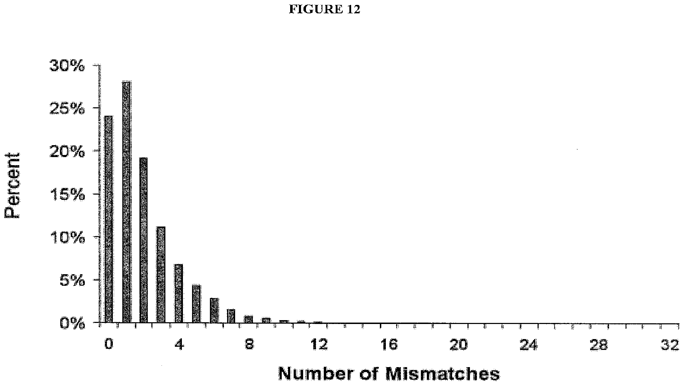

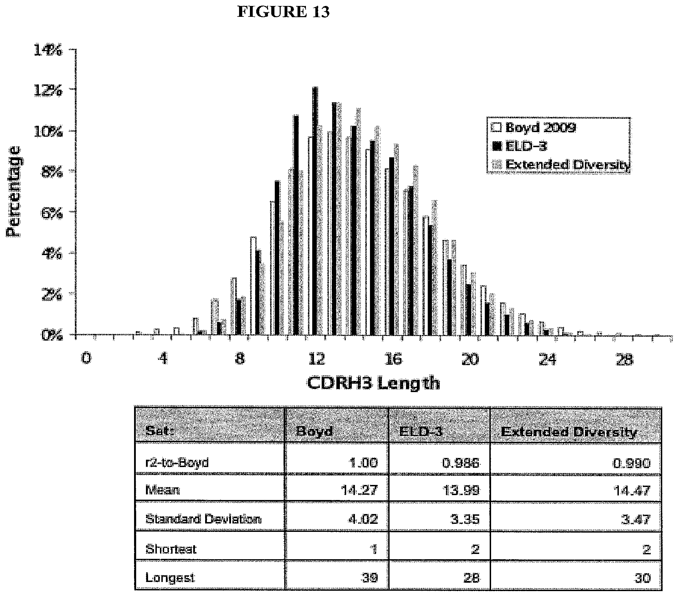

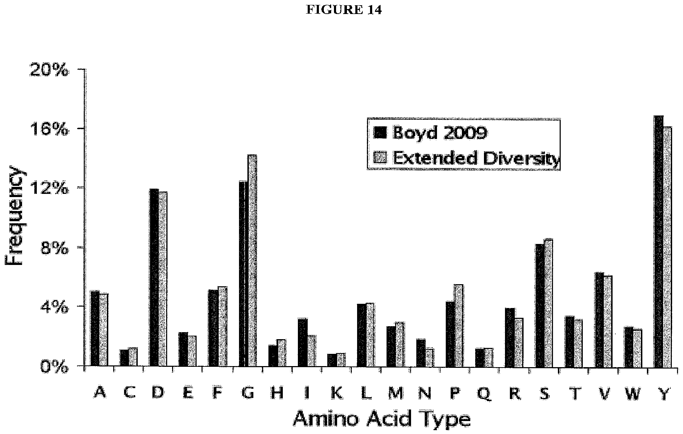

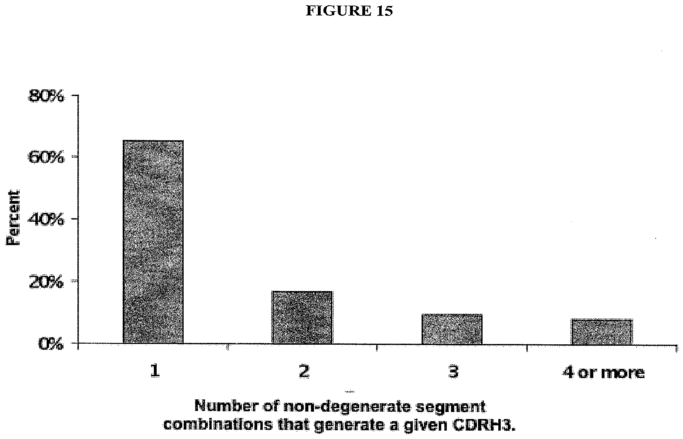

Figures 12 - 15 illustrate certain characteristics of this design indicating, for example, that about 50% of the approximately 237,000 CDRH3 sequences in Boyd *et al.* may be recapitulated by library sequences with either one or no mismatches (*i.e*., by summing the "0" and "1" bins of Figure 12). The theoretical length distributions (Figure 13) and amino acid compositions (Figure 14) of these libraries also match closely the respective characteristics observed in the same set of human CDRH3 sequences. Figure 15 shows the combinatorial efficiency of the Extended Diversity Library Design. Approximately 65% of the sequences appear only once in the design (*i.e*., are generated via one non-degenerate combination of segments). Figure 8, previously presented, shows that the Extended Diversity Library Design outperforms both LUA-141 and ELD-3 in terms of matching to clinically relevant human antibody sequences.

**Table 1. Germline-like sequences for eight of the VK chassis provided by the invention.**

| **Germline** | **Junction** | **Germline-Like CDRL3 Sequence** | **SEQ ID NO** | **Germline** | **Junction** | **Germline-Like CDRL3 Sequence** | **SEQ ID NO** |
|---|---|---|---|---|---|---|---|
| VK1-05 | 1 | QQYNSYST | 1 | VK2-28 | 1 | MQALQTPT | 101 |
| VK1-05 | 2 | QQYNSYFT | 2 | VK2-28 | 2 | MQALQTFT | 102 |
| VK1-05 | 3 | QQYNSYLT | 3 | VK2-28 | 3 | MQALQTLT | 103 |
| VK1-05 | 4 | QQYNSYIT | 4 | VK2-28 | 4 | MQALQTIT | 104 |
| VK1-05 | 5 | QQYNSYRT | 5 | VK2-28 | 5 | MQALQTRT | 105 |
| VK1-05 | 6 | QQYNSYWT | 6 | VK2-28 | 6 | MQALQTWT | 106 |
| VK1-05 | 7 | QQYNSYYT | 7 | VK2-28 | 7 | MQALQTYT | 107 |
| VK1-05 | 8 | QQYNSYSPT | 8 | VK2-28 | 8 | MQALQTPPT | 108 |
| VK1-05 | 9 | QQYNSYSFT | 9 | VK2-28 | 9 | MQALQTPFT | 109 |
| VK1-05 | 10 | QQYNSYSLT | 10 | VK2-28 | 10 | MQALQTPLT | 110 |
| VK1-05 | 11 | QQYNSYSIT | 11 | VK2-28 | 11 | MQALQTPIT | 111 |
| VK1-05 | 12 | QQYNSYSRT | 12 | VK2-28 | 12 | MQALQTPRT | 112 |
| VK1-05 | 13 | QQYNSYSWT | 13 | VK2-28 | 13 | MQALQTPWT | 113 |
| VK1-05 | 14 | QQYNSYSYT | 14 | VK2-28 | 14 | MQALQTPYT | 114 |
| VK1-05 | 15 | QQYNSYSPFT | 15 | VK2-28 | 15 | MQALQTPPFT | 115 |
| VK1-05 | 16 | QQYNSYSPLT | 16 | VK2-28 | 16 | MQALQTPPLT | 116 |
| VK1-05 | 17 | QQYNSYSPIT | 17 | VK2-28 | 17 | MQALQTPPIT | 117 |
| VK1-05 | 18 | QQYNSYSPRT | 18 | VK2-28 | 18 | MQALQTPPRT | 118 |
| VK1-05 | 19 | QQYNSYSPWT | 19 | VK2-28 | 19 | MQALQTPPWT | 119 |
| VK1-05 | 20 | QQYNSYSPYT | 20 | VK2-28 | 20 | MQALQTPPYT | 120 |
| VK1-12 | 1 | QQANSFPT | 21 | VK3-11 | 1 | QQRSNWPT | 121 |
| VK1-12 | 2 | QQANSFFT | 22 | VK3-11 | 2 | QQRSNWFT | 122 |
| VK1-12 | 3 | QQANSFLT | 23 | VK3-11 | 3 | QQRSNWLT | 123 |
| VK1-12 | 4 | QQANSFIT | 24 | VK3-11 | 4 | QQRSNWIT | 124 |
| VK1-12 | 5 | QQANSFRT | 25 | VK3-11 | 5 | QQRSNWRT | 125 |
| VK1-12 | 6 | QQANSFWT | 26 | VK3-11 | 6 | QQRSNWWT | 126 |
| VK1-12 | 7 | QQANSFYT | 27 | VK3-11 | 7 | QQRSNWYT | 127 |
| VK1-12 | 8 | QQANSFPPT | 28 | VK3-11 | 8 | QQRSNWPPT | 128 |
| VK1-12 | 9 | QQANSFPFT | 29 | VK3-11 | 9 | QQRSNWPFT | 129 |
| VK1-12 | 10 | QQANSFPLT | 30 | VK3-11 | 10 | QQRSNWPLT | 130 |
| VK1-12 | 11 | QQANSFPIT | 31 | VK3-11 | 11 | QQRSNWPIT | 131 |
| VK1-12 | 12 | QQANSFPRT | 32 | VK3-11 | 12 | QQRSNWPRT | 132 |
| VK1-12 | 13 | QQANSFPWT | 33 | VK3-11 | 13 | QQRSNWPWT | 133 |
| VK1-12 | 14 | QQANSFPYT | 34 | VK3-11 | 14 | QQRSNWPYT | 134 |
| VK1-12 | 15 | QQANSFPPFT | 35 | VK3-11 | 15 | QQRSNWPPFT | 135 |
| VK1-12 | 16 | QQANSFPPLT | 36 | VK3-11 | 16 | QQRSNWPPLT | 136 |
| VK1-12 | 17 | QQANSFPPIT | 37 | VK3-11 | 17 | QQRSNWPPIT | 137 |
| VK1-12 | 18 | QQANSFPPRT | 38 | VK3-11 | 18 | QQRSNWPPRT | 138 |
| VK1-12 | 19 | QQANSFPPWT | 39 | VK3-11 | 19 | QQRSNWPPWT | 139 |
| VK1-12 | 20 | QQANSFPPYT | 40 | VK3-11 | 20 | QQRSNWPPYT | 140 |
| VK1-33 | 1 | QQYDNLPT | 41 | VK3-15 | 1 | QQYNNWPT | 141 |
| VK1-33 | 2 | QQYDNLFT | 42 | VK3-15 | 2 | QQYNNWFT | 142 |
| VK1-33 | 3 | QQYDNLLT | 43 | VK3-15 | 3 | QQYNNWLT | 143 |
| VK1-33 | 4 | QQYDNLIT | 44 | VK3-15 | 4 | QQYNNWIT | 144 |
| VK1-33 | 5 | QQYDNLRT | 45 | VK3-15 | 5 | QQYNNWRT | 145 |
| VK1-33 | 6 | QQYDNLWT | 46 | VK3-15 | 6 | QQYNNWWT | 146 |
| VK1-33 | 7 | QQYDNLYT | 47 | VK3-15 | 7 | QQYNNWYT | 147 |
| VK1-33 | 8 | QQYDNLPPT | 48 | VK3-15 | 8 | QQYNNWPPT | 148 |
| VK1-33 | 9 | QQYDNLPFT | 49 | VK3-15 | 9 | QQYNNWPFT | 149 |
| VK1-33 | 10 | QQYDNLPLT | 50 | VK3-15 | 10 | QQYNNWPLT | 150 |
| VK1-33 | 11 | QQYDNLPIT | 51 | VK3-15 | 11 | QQYNNWPIT | 151 |
| VK1-33 | 12 | QQYDNLPRT | 52 | VK3-15 | 12 | QQYNNWPRT | 152 |
| VK1-33 | 13 | QQYDNLPWT | 53 | VK3-15 | 13 | QQYNNWPWT | 153 |
| VK1-33 | 14 | QQYDNLPYT | 54 | VK3-15 | 14 | QQYNNWPYT | 154 |
| VK1-33 | 15 | QQYDNLPPFT | 55 | VK3-15 | 15 | QQYNNWPPFT | 155 |
| VK1-33 | 16 | QQYDNLPPLT | 56 | VK3-15 | 16 | QQYNNWPPLT | 156 |
| VK1-33 | 17 | QQYDNLPPIT | 57 | VK3-15 | 17 | QQYNNWPPIT | 157 |
| VK1-33 | 18 | QQYDNLPPRT | 58 | VKQ-15 | 18 | QQYNNWPPRT | 158 |
| VK1-33 | 19 | QQYDNLPPWT | 59 | VK3-15 | 19 | QQYNNWPPWT | 159 |
| VK1-33 | 20 | QQYDNLPPYT | 60 | VK3-15 | 20 | QQYNNWPPYT | 160 |
| VK1-39 | 1 | QQSYSTPT | 61 | VK3-20 | 1 | QQYGSSPT | 161 |
| VK1-39 | 2 | QQSYSTFT | 62 | VK3-20 | 2 | QQYGSSFT | 162 |
| VK1-39 | 3 | QQSYSTLT | 63 | VK3-20 | 3 | QQYGSSLT | 163 |
| VK1-39 | 4 | QQSYSTIT | 64 | VK3-20 | 4 | QQYGSSIT | 164 |
| VK1-39 | 5 | QQSYSTRT | 65 | VK3-20 | 5 | QQYGSSRT | 165 |
| VK1-39 | 6 | QQSYSTWT | 66 | VK3-20 | 6 | QQYGSSWT | 166 |
| VK1-39 | 7 | QQSYSTYT | 67 | VK3-20 | 7 | QQYGSSYT | 167 |
| VK1-39 | 8 | QQSYSTPPT | 68 | VK3-20 | 8 | QQYGSSPPT | 168 |
| VK1-39 | 9 | QQSYSTPFT | 69 | VK3-20 | 9 | QQYGSSPFT | 169 |
| VK1-39 | 10 | QQSYSTPLT | 70 | VK3-20 | 10 | QQYGSSPLT | 170 |
| VK1-39 | 11 | QQSYSTPIT | 71 | VK3-20 | 11 | QQYGSSPIT | 171 |
| VK1-39 | 12 | QQSYSTPRT | 72 | VK3-20 | 12 | QQYGSSPRT | 172 |
| VK1-39 | 13 | QQSYSTPWT | 73 | VK3-20 | 13 | QQYGSSPWT | 173 |
| VK1-39 | 14 | QQSYSTPYT | 74 | VK3-20 | 14 | QQYGSSPYT | 174 |
| VK1-39 | 15 | QQSYSTPPFT | 75 | VK3-20 | 15 | QQYGSSPPFT | 175 |
| VK1-39 | 16 | QQSYSTPPLT | 76 | VK3-20 | 16 | QQYGSSPPLT | 176 |
| VK1-39 | 17 | QQSYSTPPIT | 77 | VK3-20 | 17 | QQYGSSPPIT | 177 |
| VK1-39 | 18 | QQSYSTPPRT | 78 | VK3-20 | 18 | QQYGSSPPRT | 178 |
| VK1-39 | 19 | QQSYSTPPWT | 79 | VK3-20 | 19 | QQYGSSPPWT | 179 |
| VK1-39 | 20 | QQSYSTPPYT | 80 | VK3-20 | 20 | QQYGSSPPYT | 180 |
| VK4-01 | 1 | QQYYSTPT | 81 | | | | |
| VK4-01 | 2 | QQYYSTFT | 82 | | | | |
| VK4-01 | 3 | QQYYSTLT | 83 | | | | |
| VK4-01 | 4 | QQYYSTIT | 84 | | | | |
| VK4-01 | 5 | QQYYSTRT | 85 | | | | |
| VK4-01 | 6 | QQYYSTWT | 86 | | | | |
| VK4-01 | 7 | QQYYSTYT | 87 | | | | |
| VK4-01 | 8 | QQYYSTPPT | 88 | | | | |
| VK4-01 | 9 | QQYYSTPFT | 89 | | | | |
| VK4-01 | 10 | QQYYSTPLT | 90 | | | | |
| VK4-01 | 11 | QQYYSTPIT | 91 | | | | |
| VK4-01 | 12 | QQYYSTPRT | 92 | | | | |
| VK4-01 | 13 | QQYYSTPWT | 93 | | | | |
| VK4-01 | 14 | QQYYSTPYT | 94 | | | | |
| VK4-01 | 15 | QQYYSTPPFT | 95 | | | | |
| VK4-01 | 16 | QQYYSTPPLT | 96 | | | | |
| VK4-01 | 17 | QQYYSTPPIT | 97 | | | | |
| VK4-01 | 18 | QQYYSTPPRT | 98 | | | | |
| VK4-01 | 19 | QQYYSTPPWT | 99 | | | | |
| VK4-01 | 20 | QQYYSTPPYT | 100 | | | | |

**Table 2. Summary of framework variants for exemplified light chain germlines.**

| **Light Chain Germline** | **Number of Sequences Analyzed** | **Primary Framework Positions Selected for Variance** | **Alternative Framework Positions Selected for Variance** |
|---|---|---|---|
| VK1-5 | 307 | 4, 49 | 46 |
| VK1-12 | 113 | 4, 49 | 46, 66 |
| VK1-33 | 188 | 4, 66 | 49 |
| VK1-39 | 656 | 4, 49 | 46 |
| VK2-28 | 275 | 46, 49 | 2, 4 |
| VK3-11 | 375 | 4, 36 | 2, 49 |
| VK3-15 | 202 | 4, 49 | 2, 48 |
| VK3-20 | 867 | 4, 49 | 2, 48 |
| VK4-1 | 368 | 4, 49 | 46, 66 |

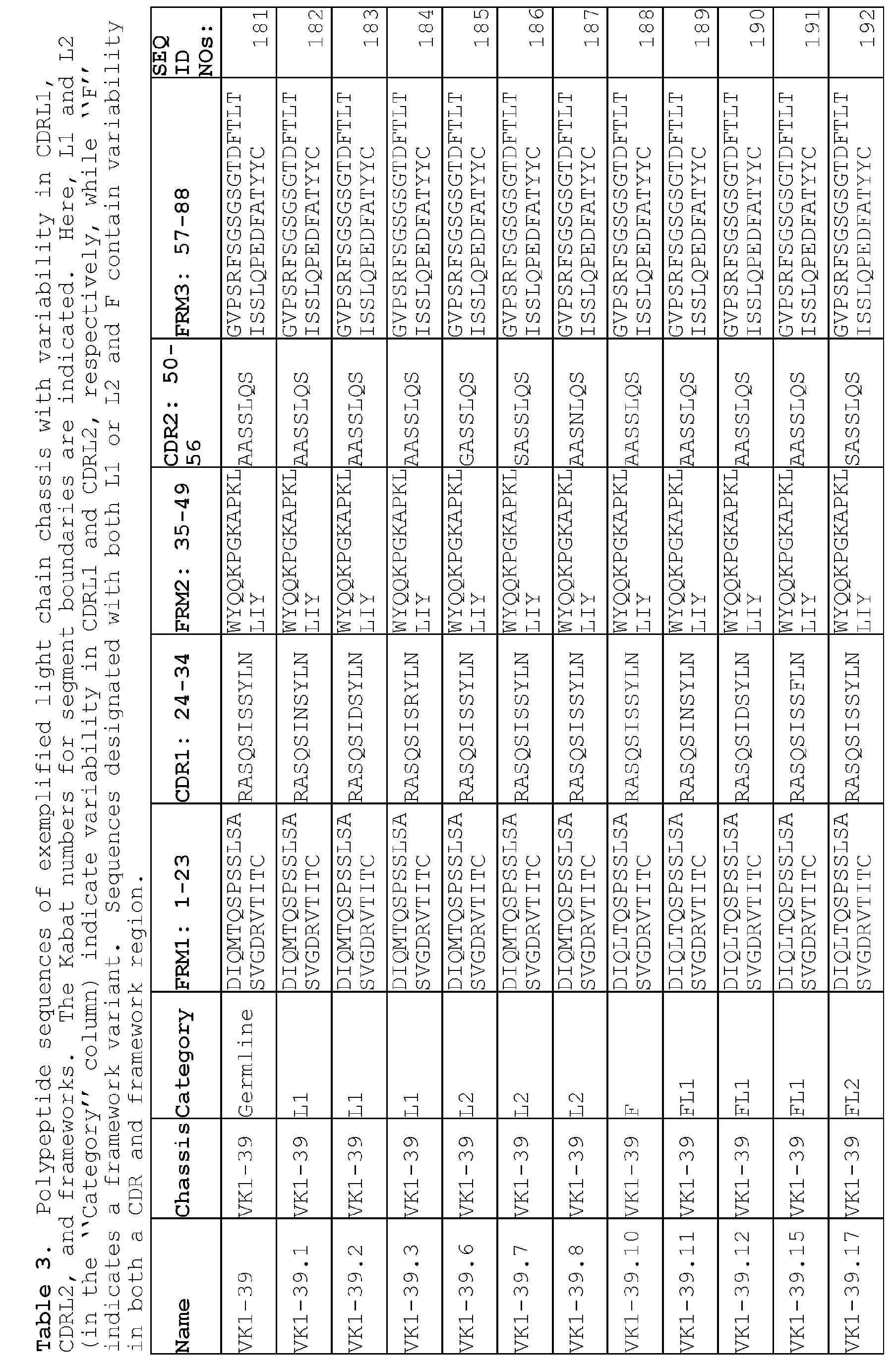

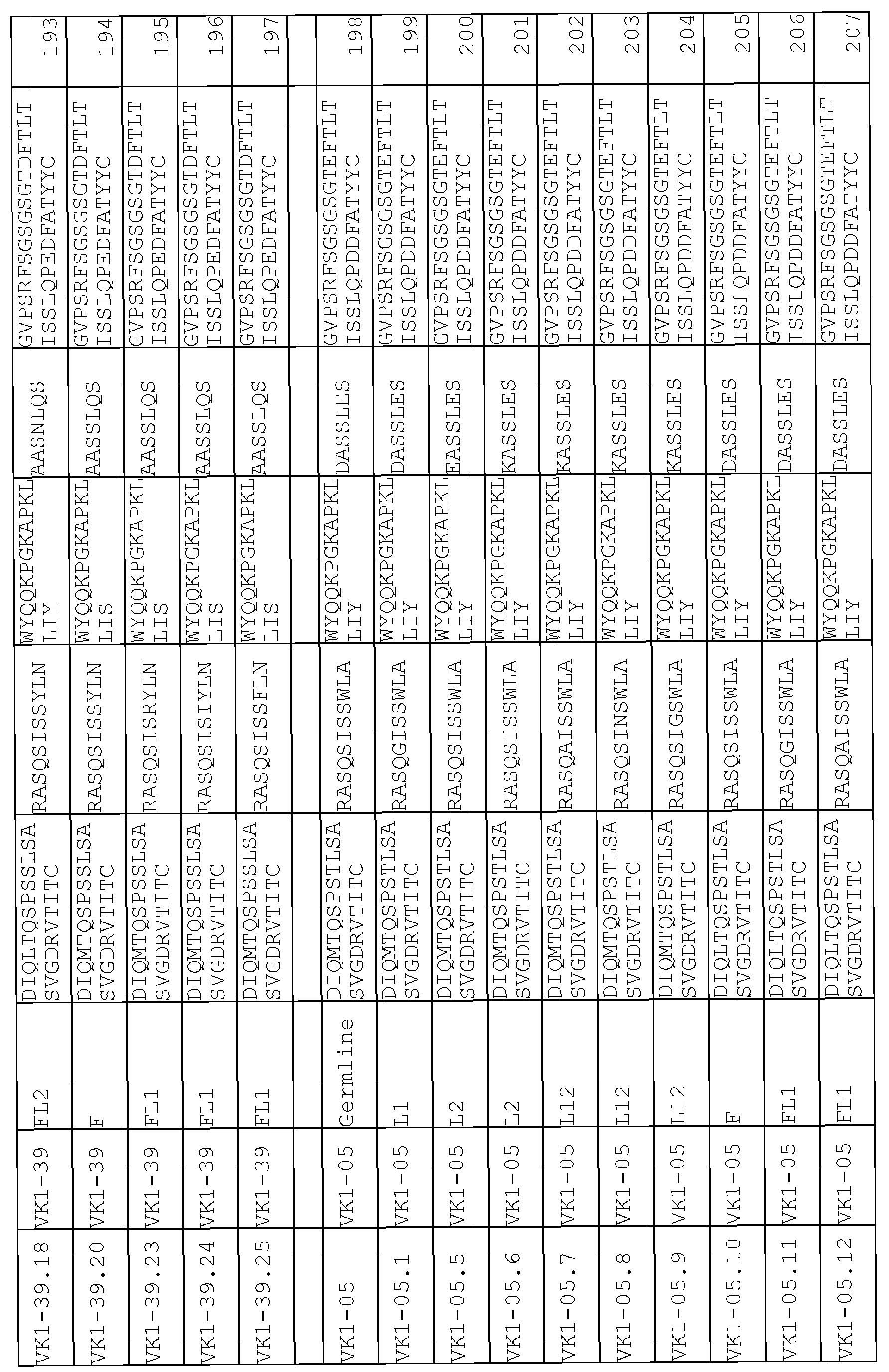

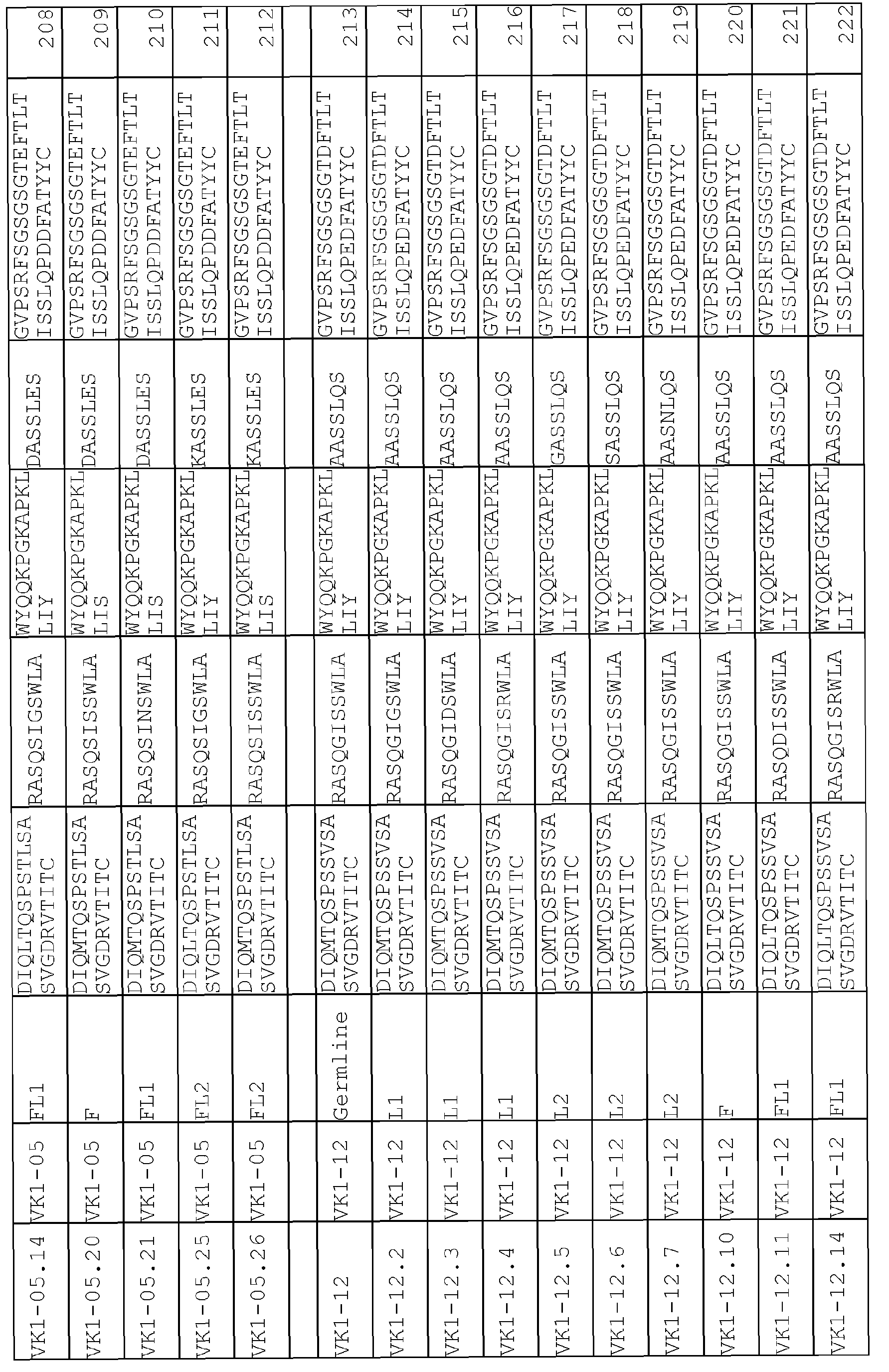

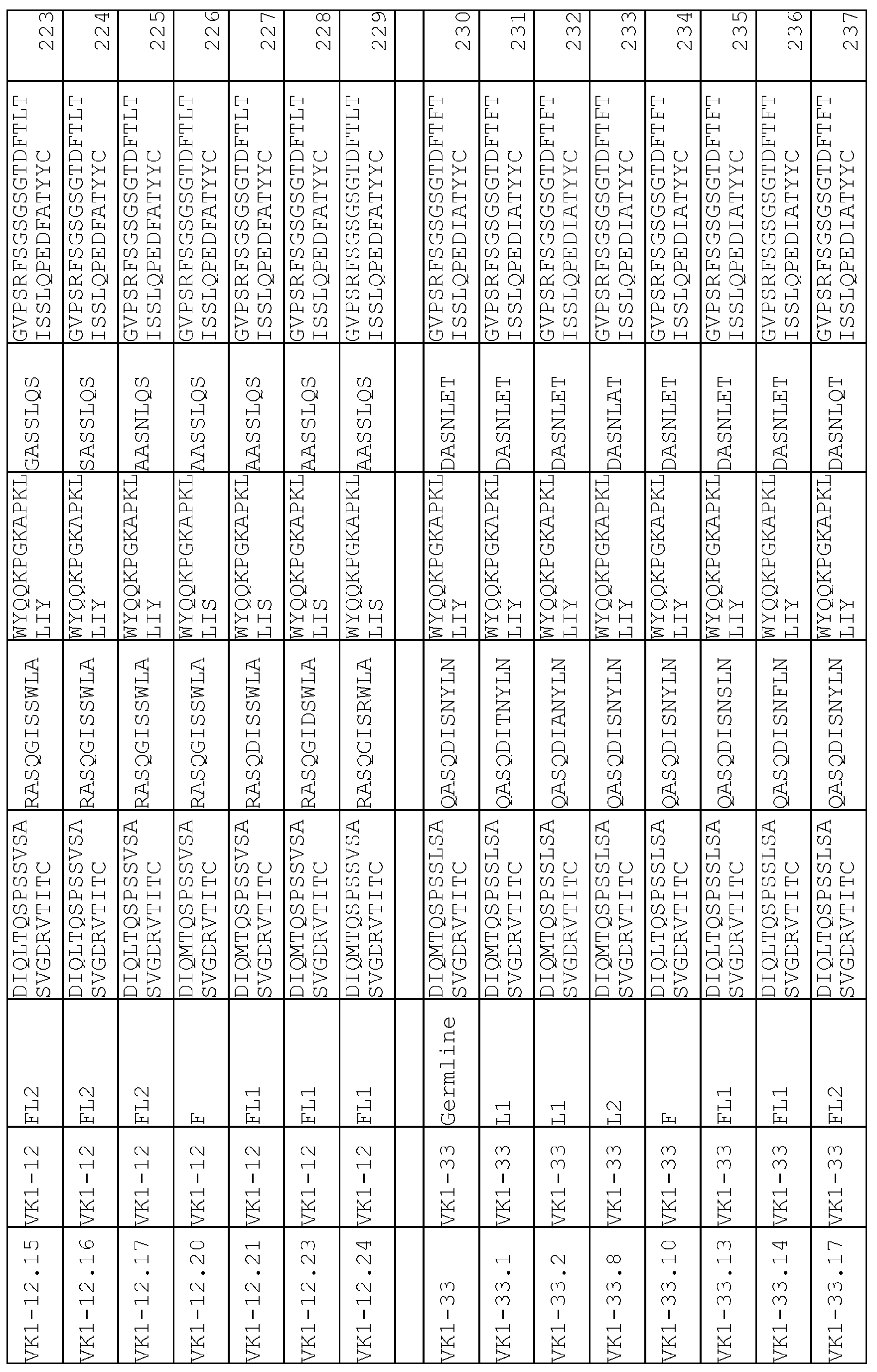

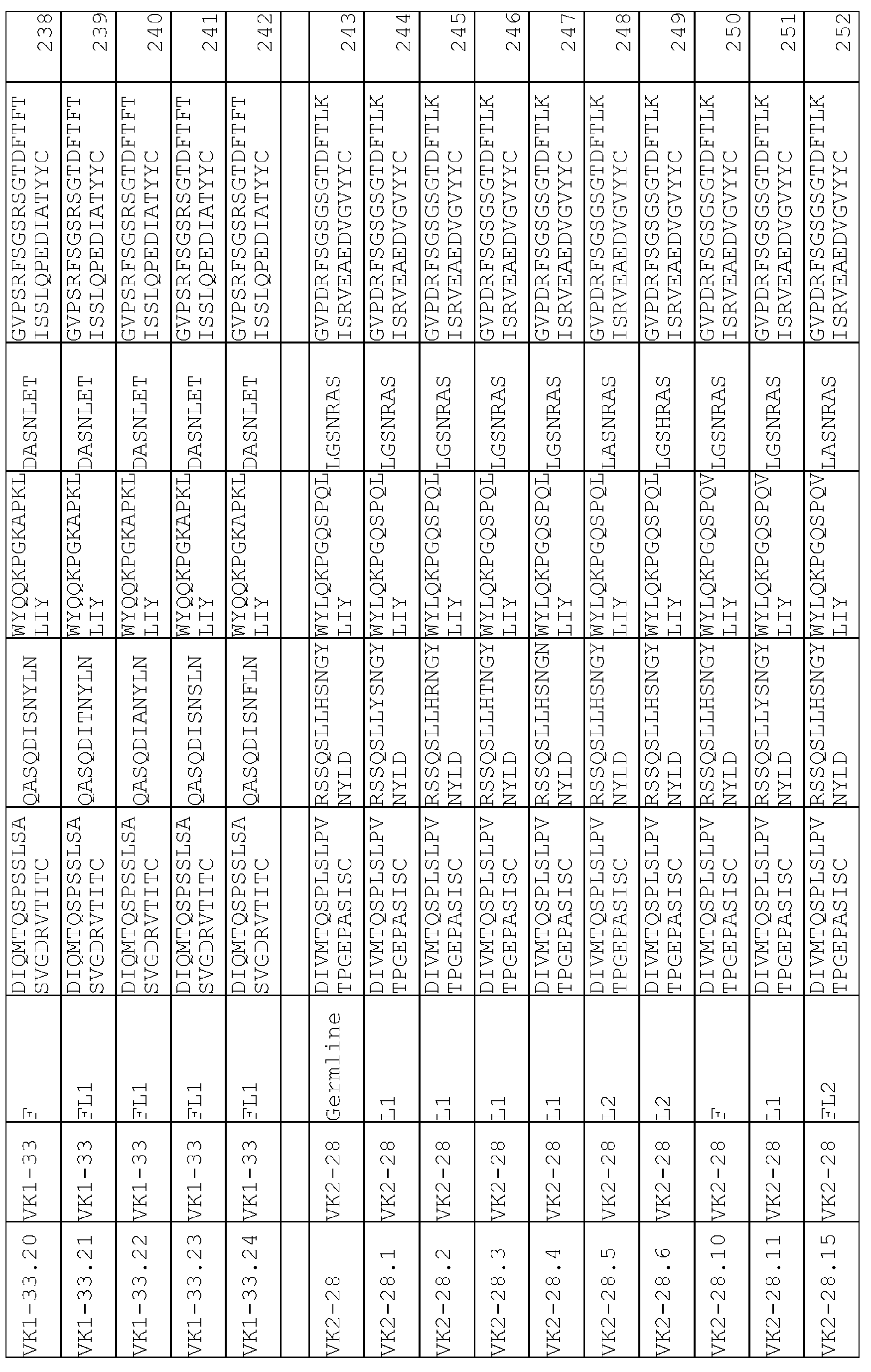

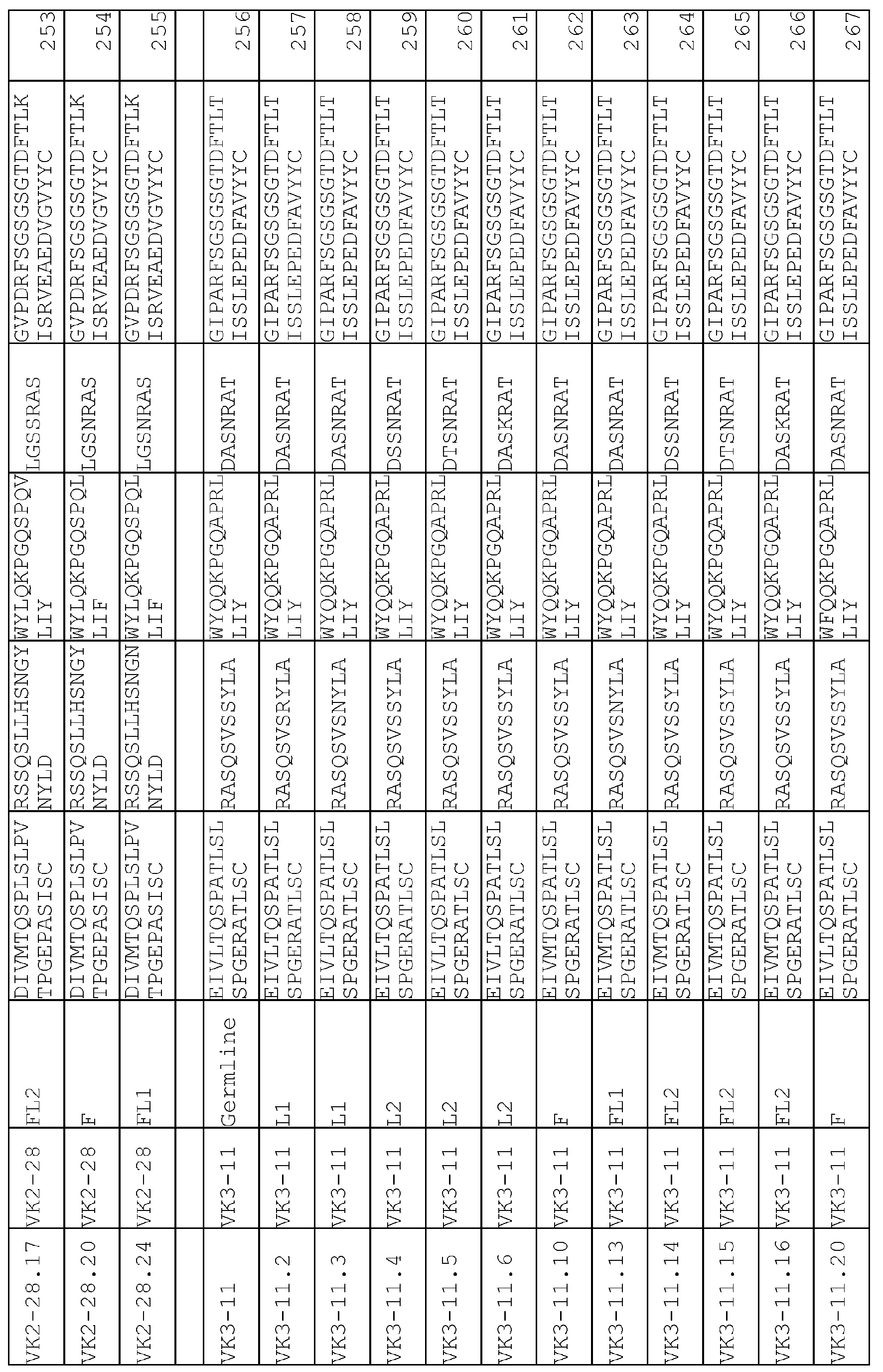

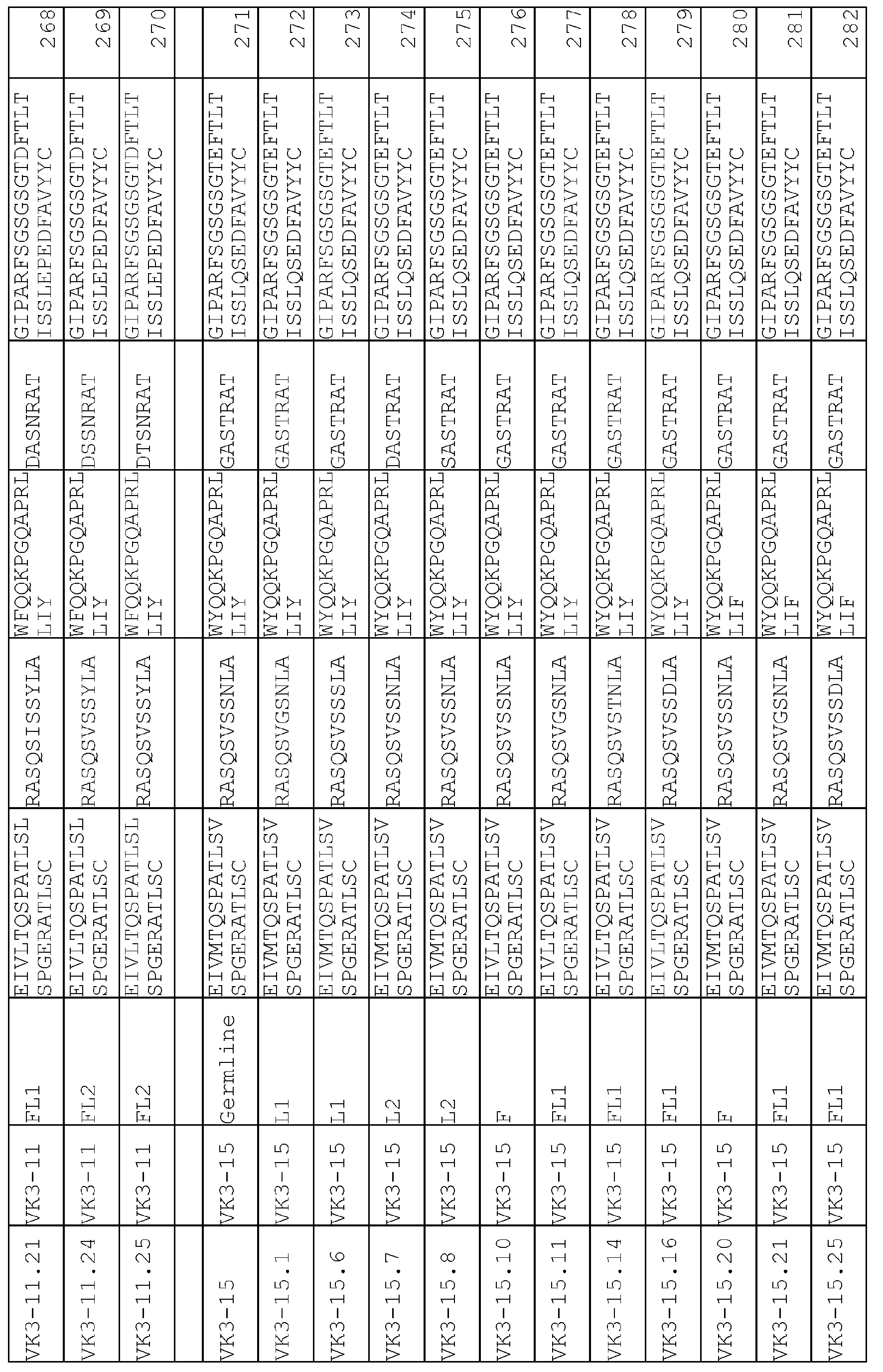

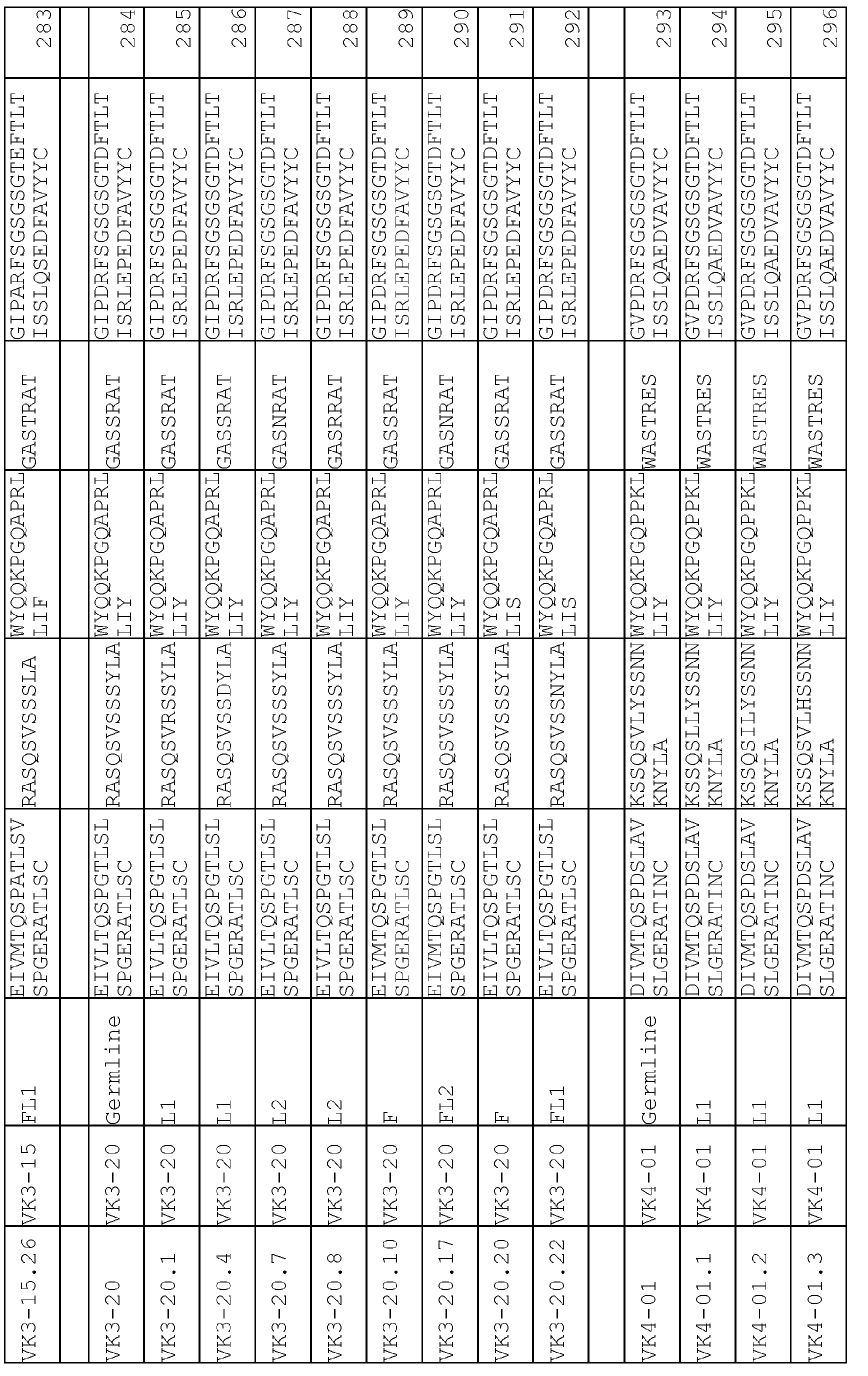

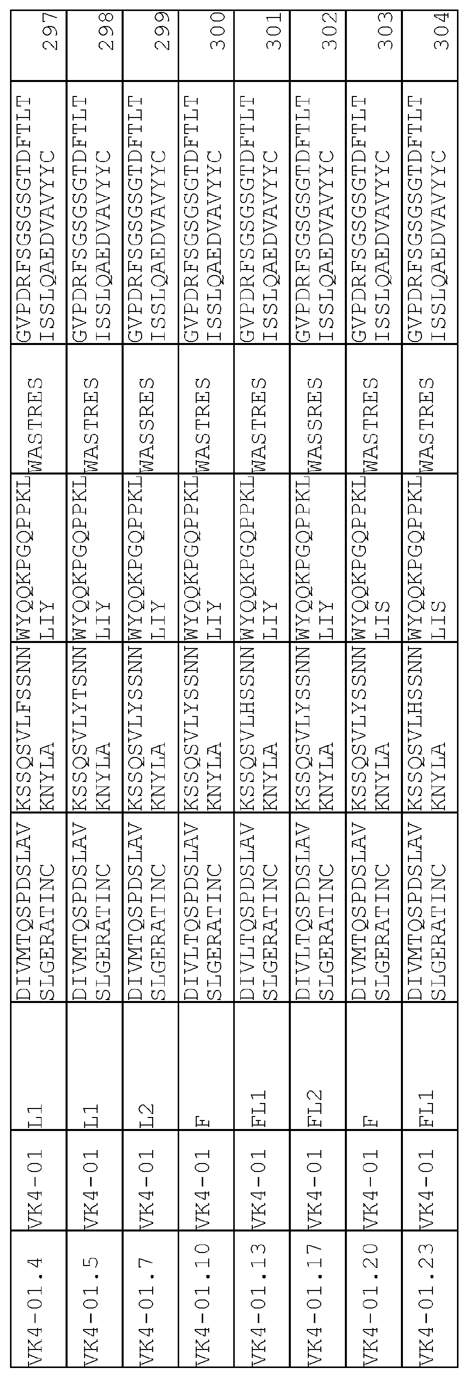

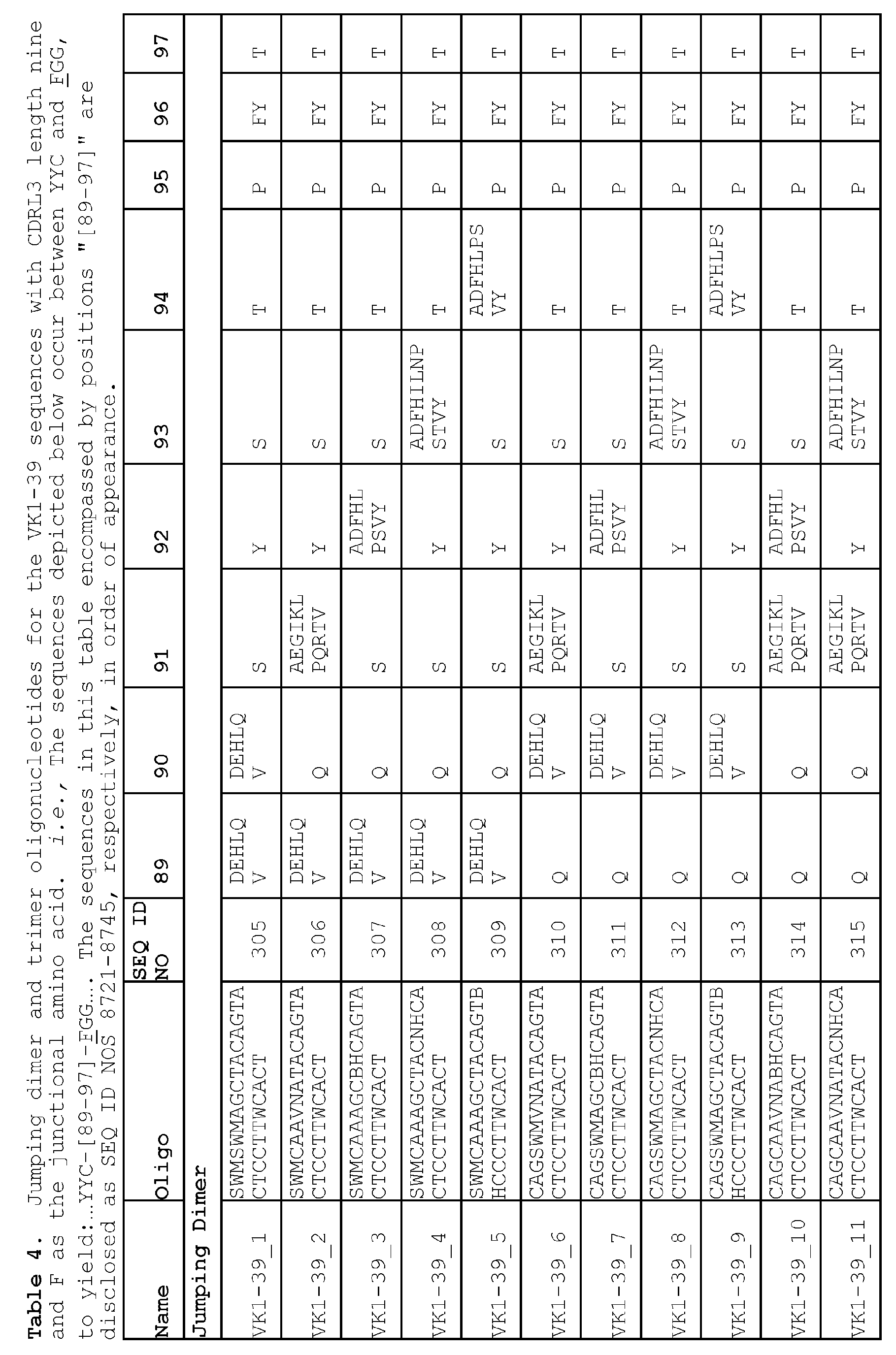

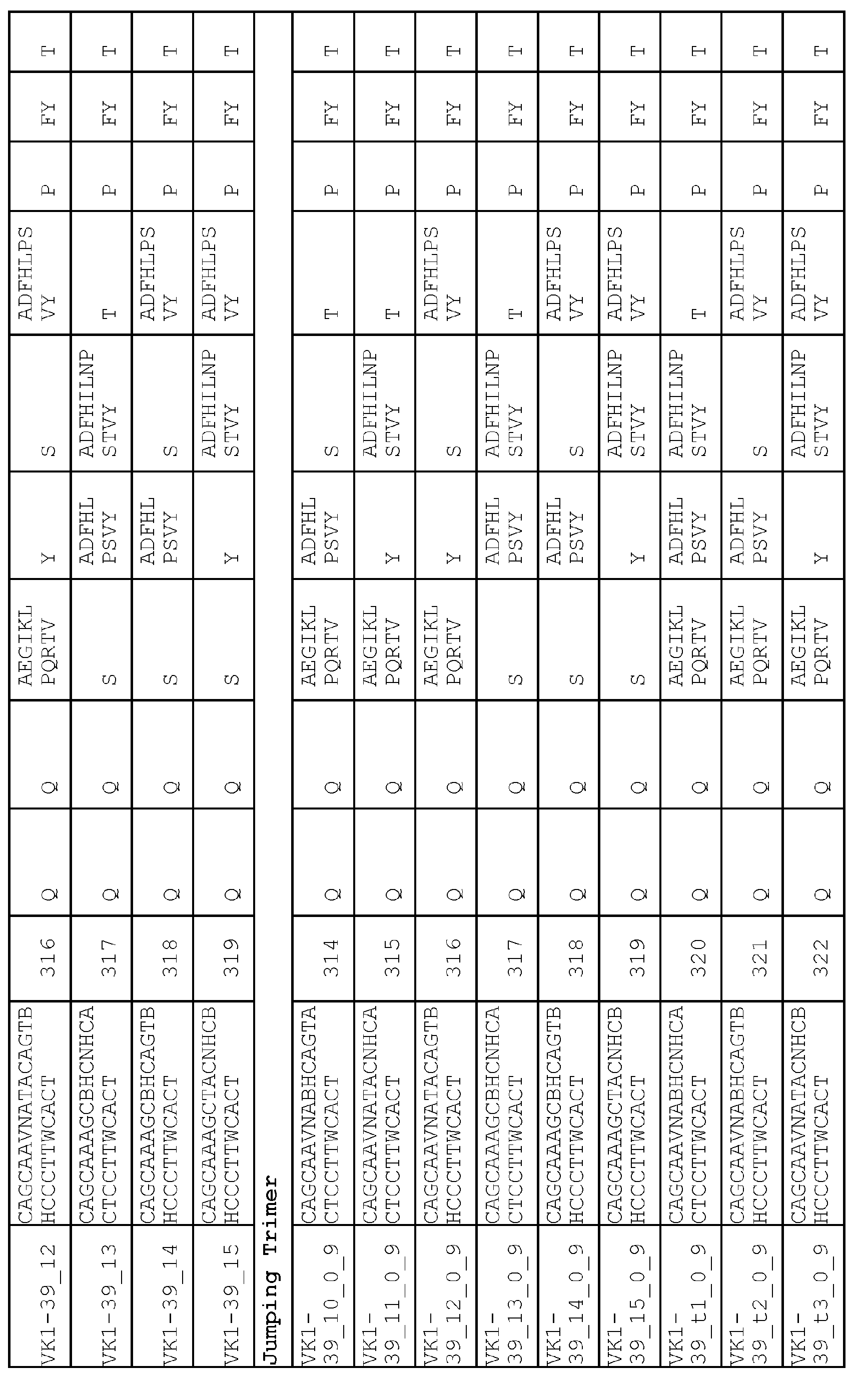

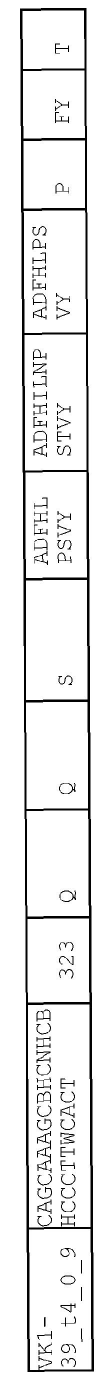

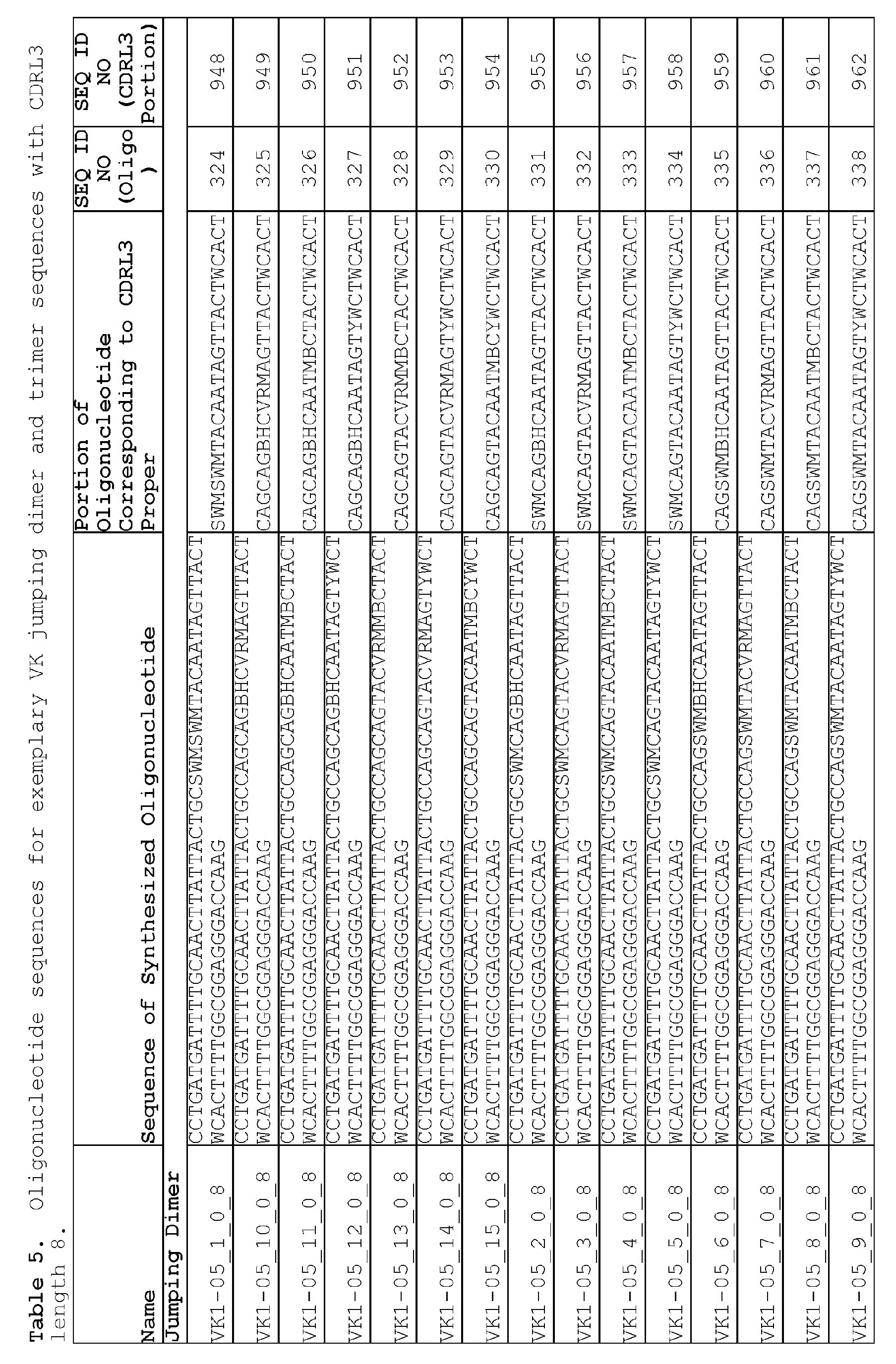

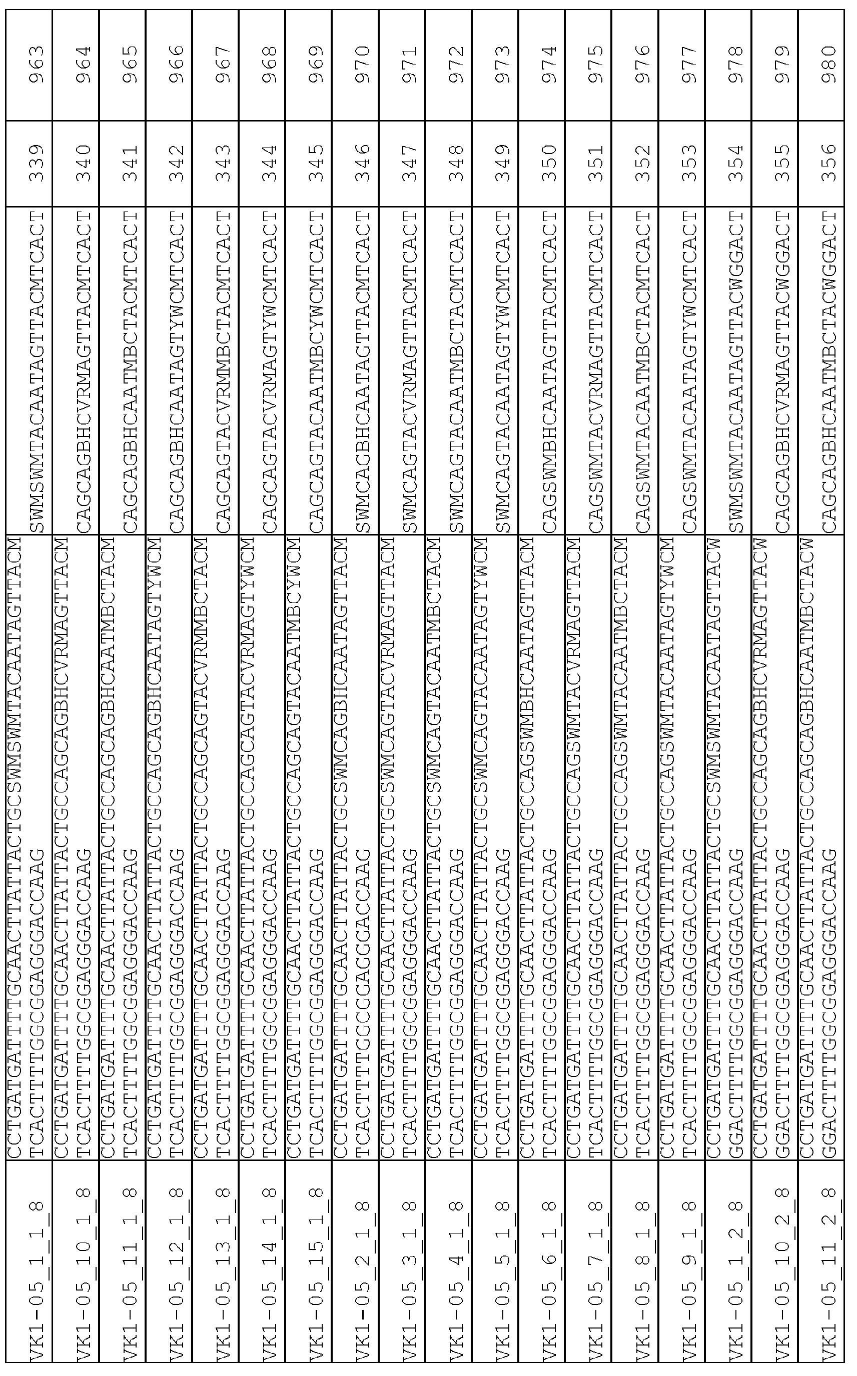

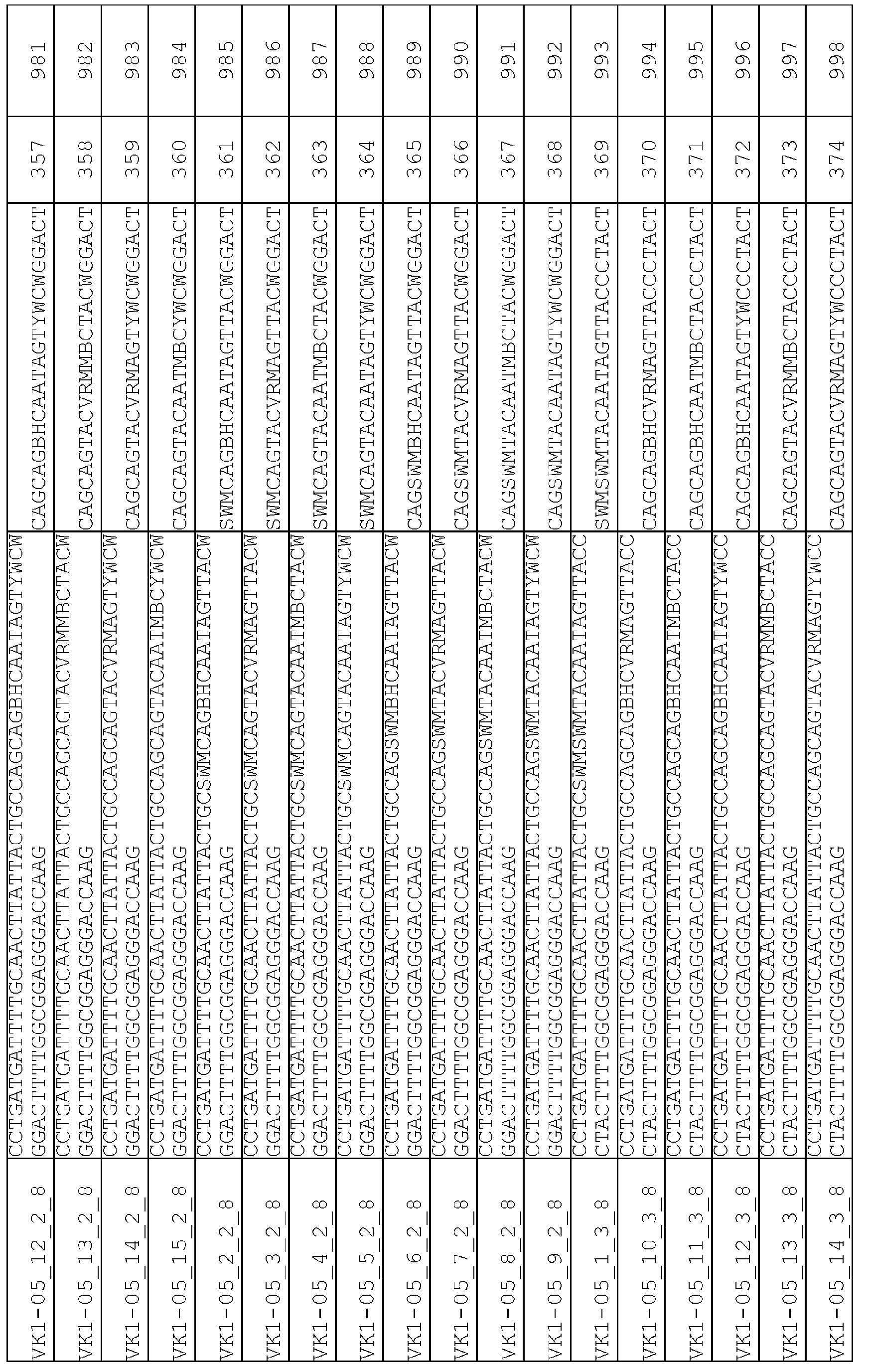

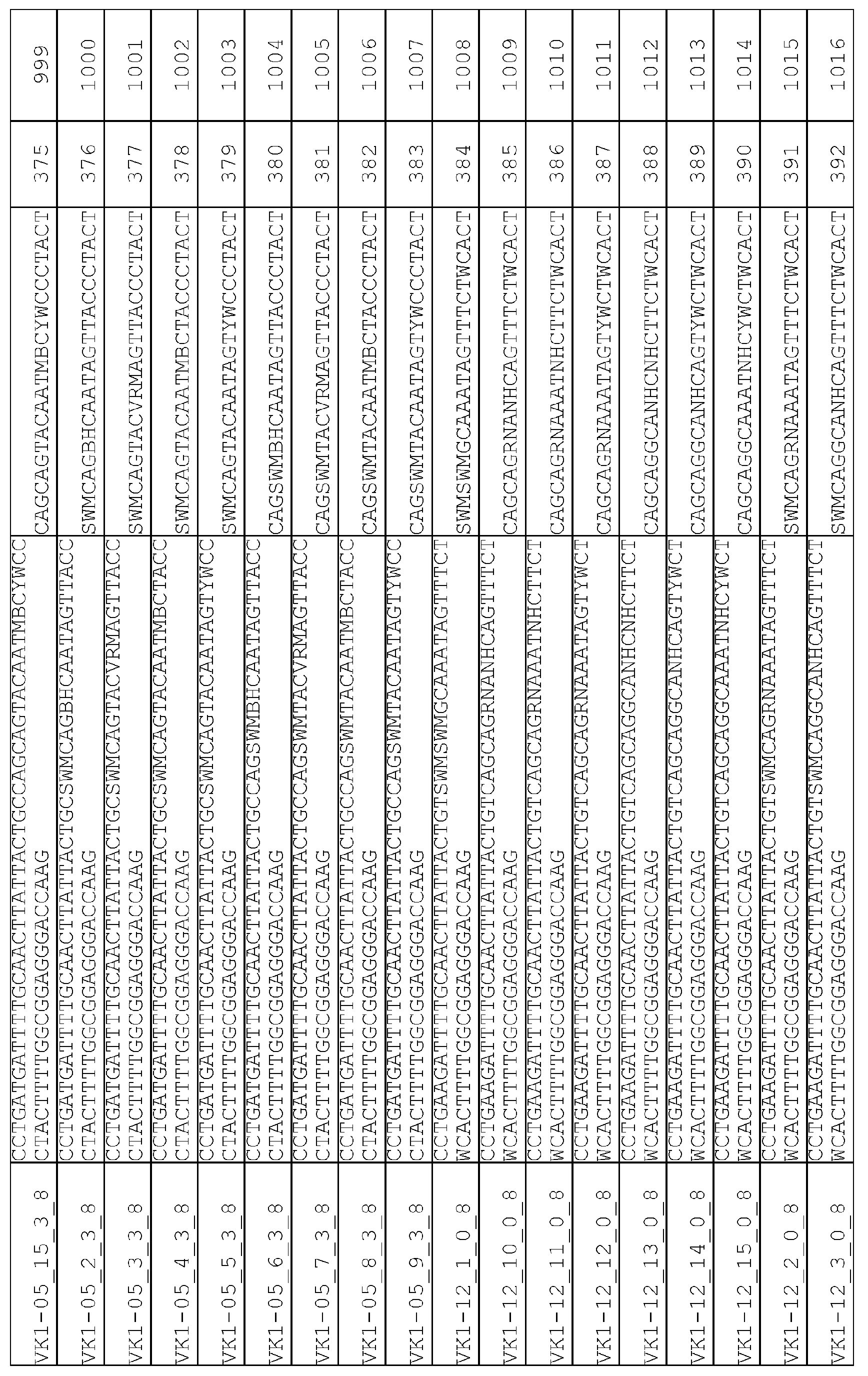

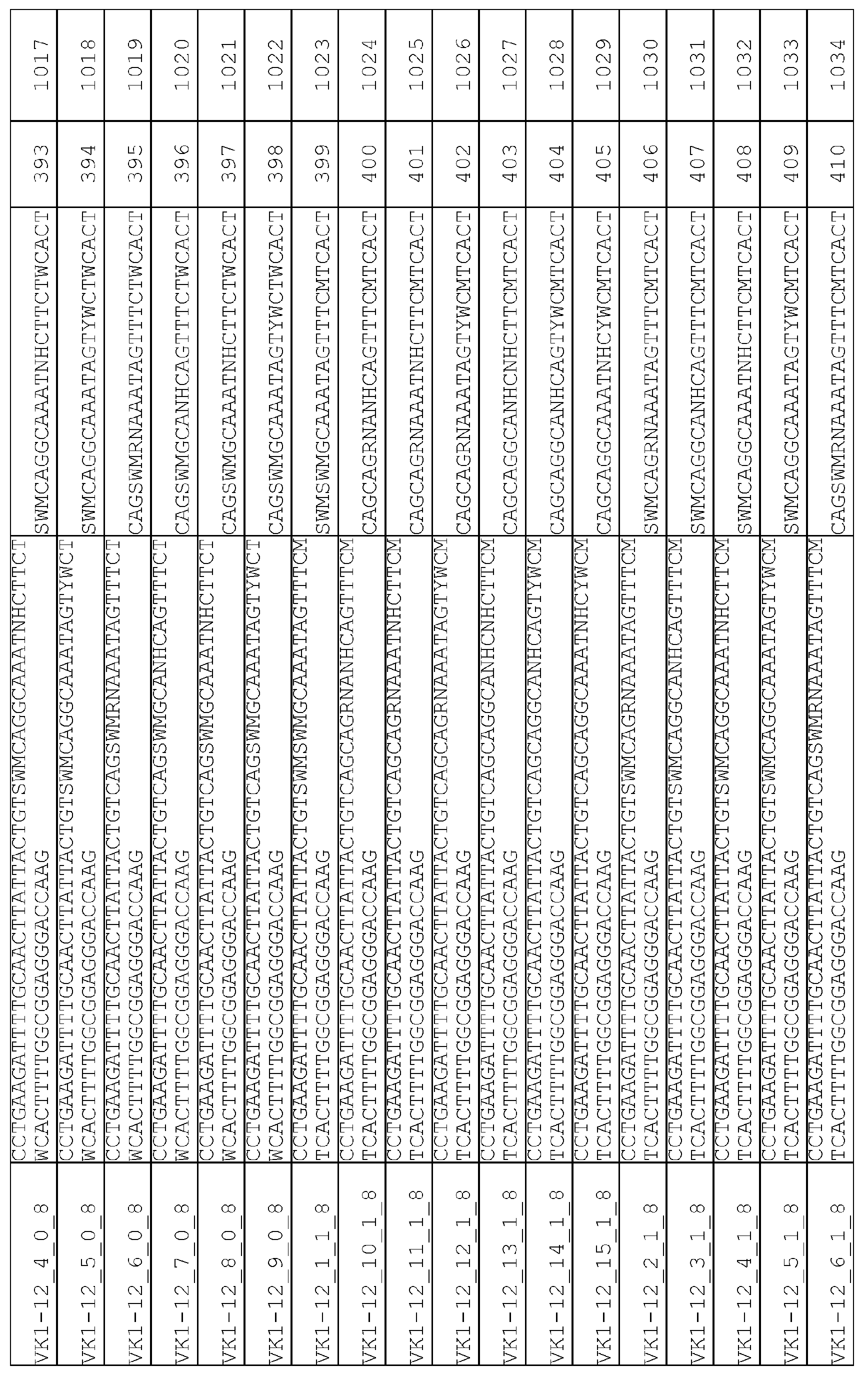

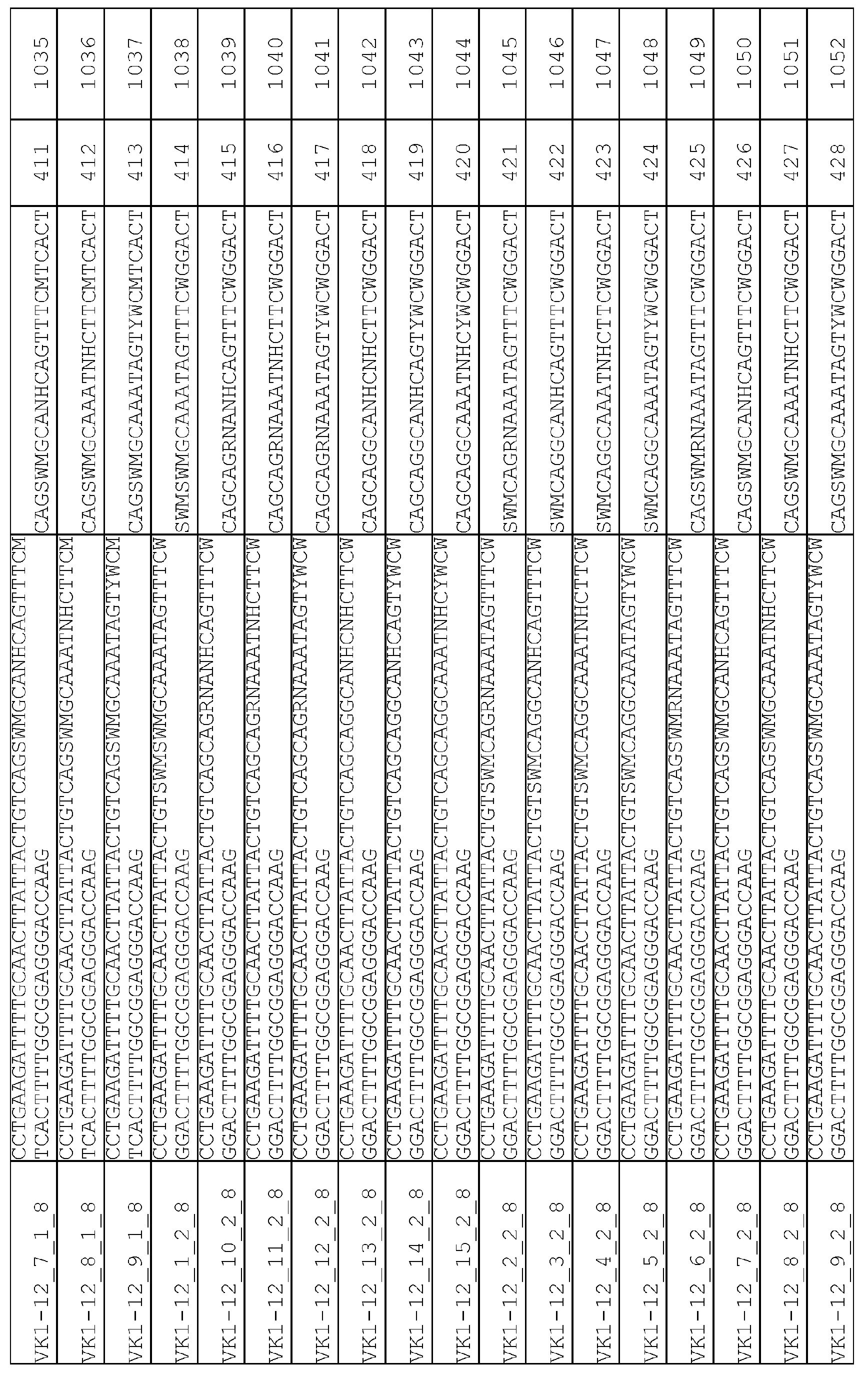

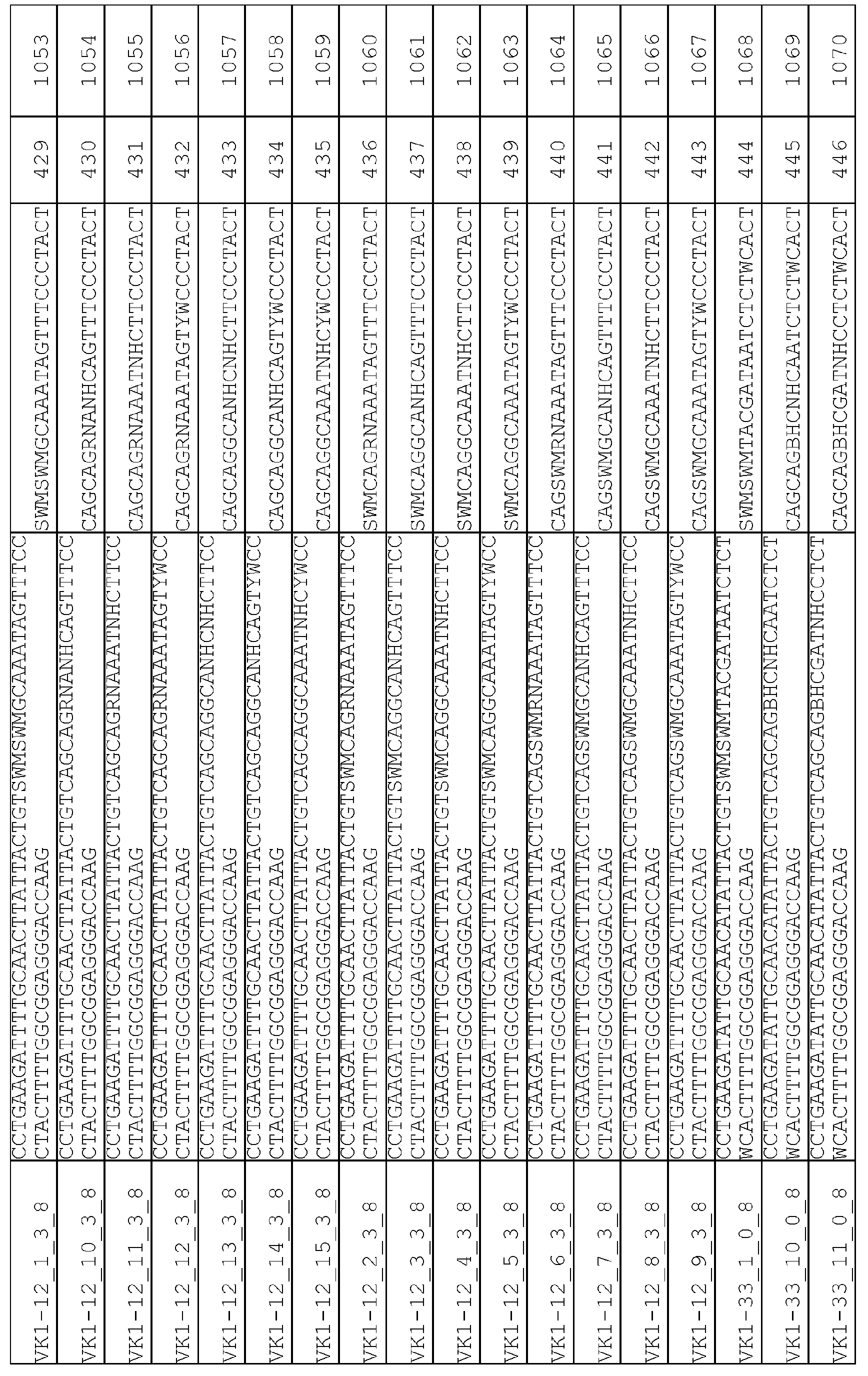

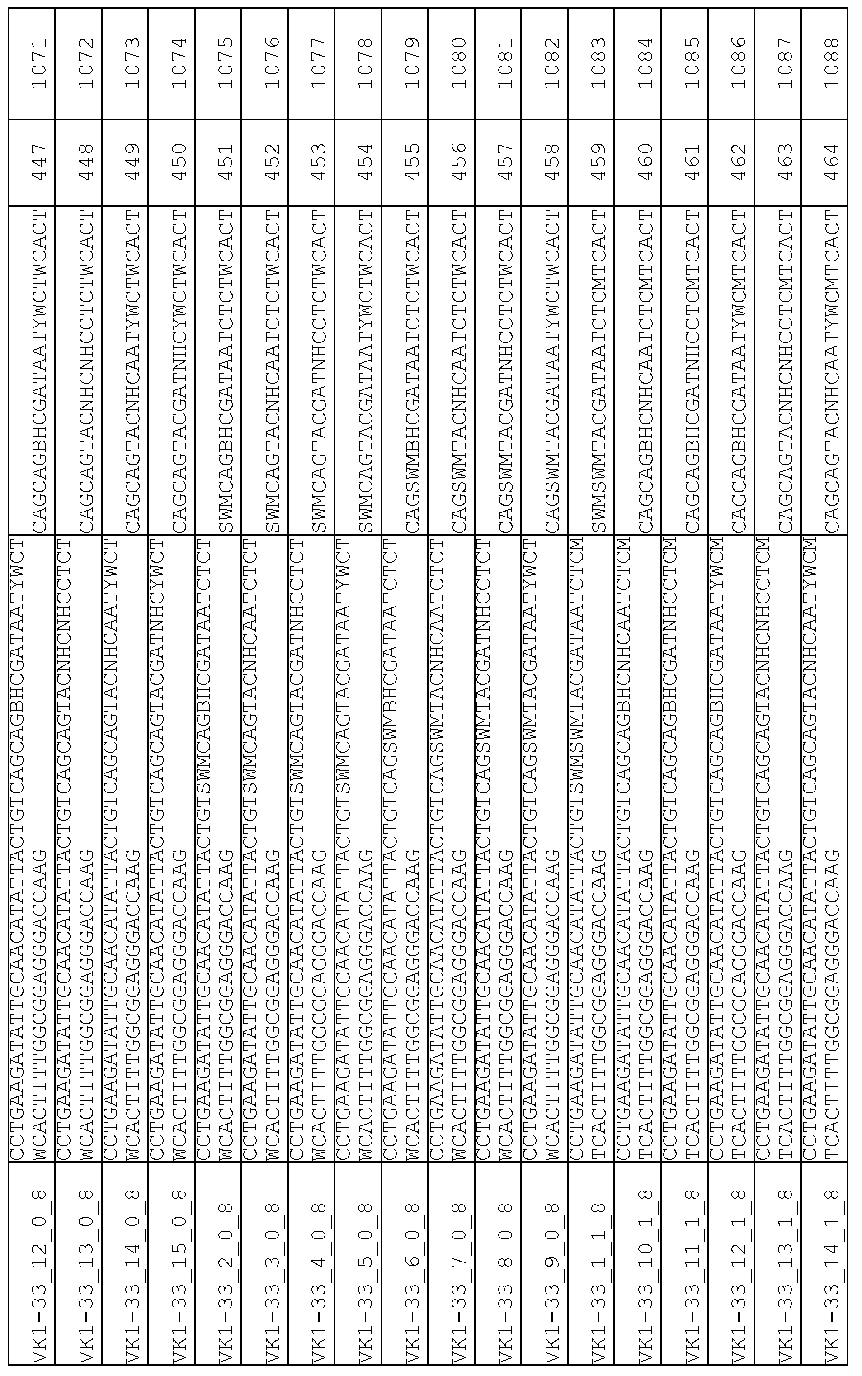

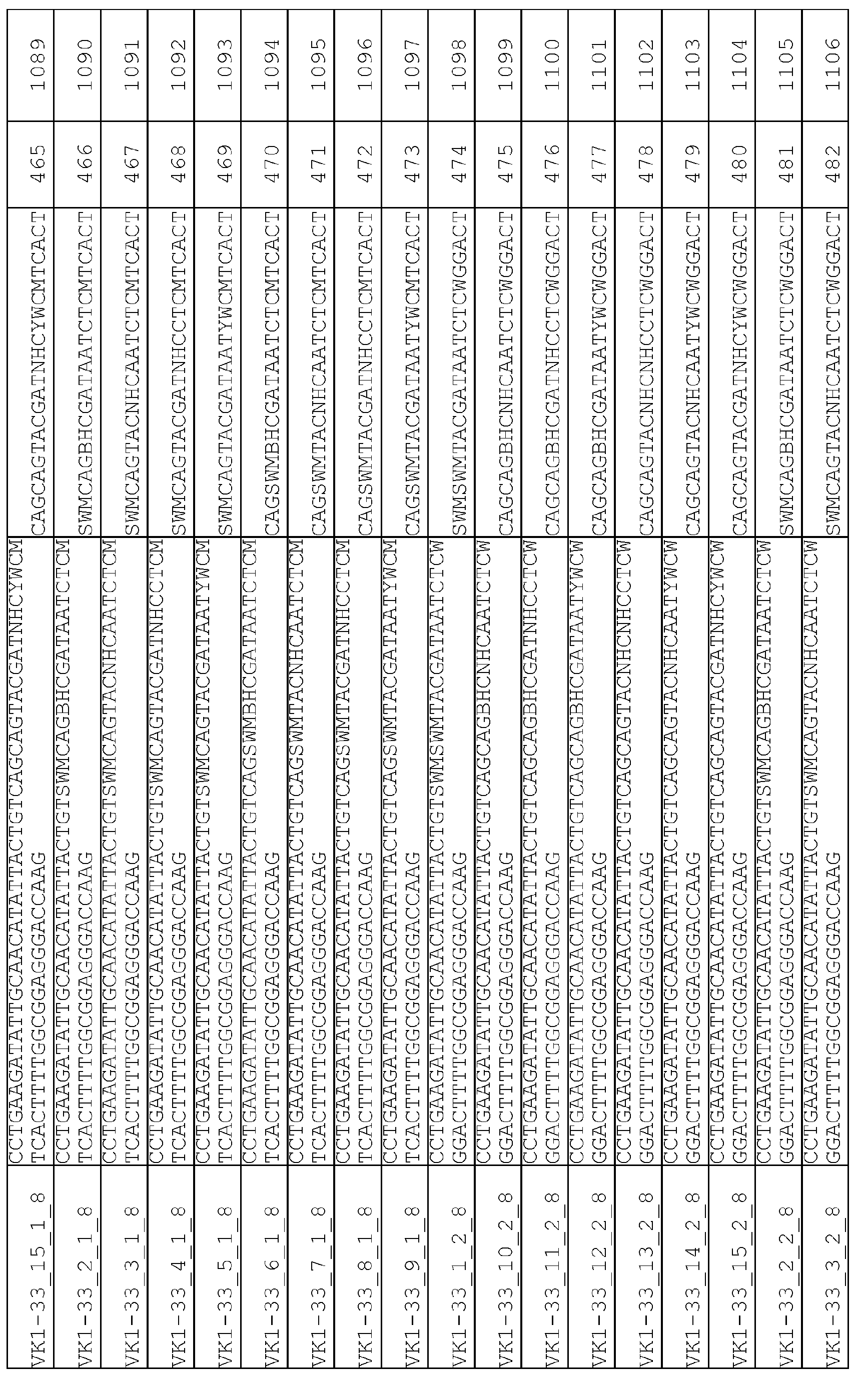

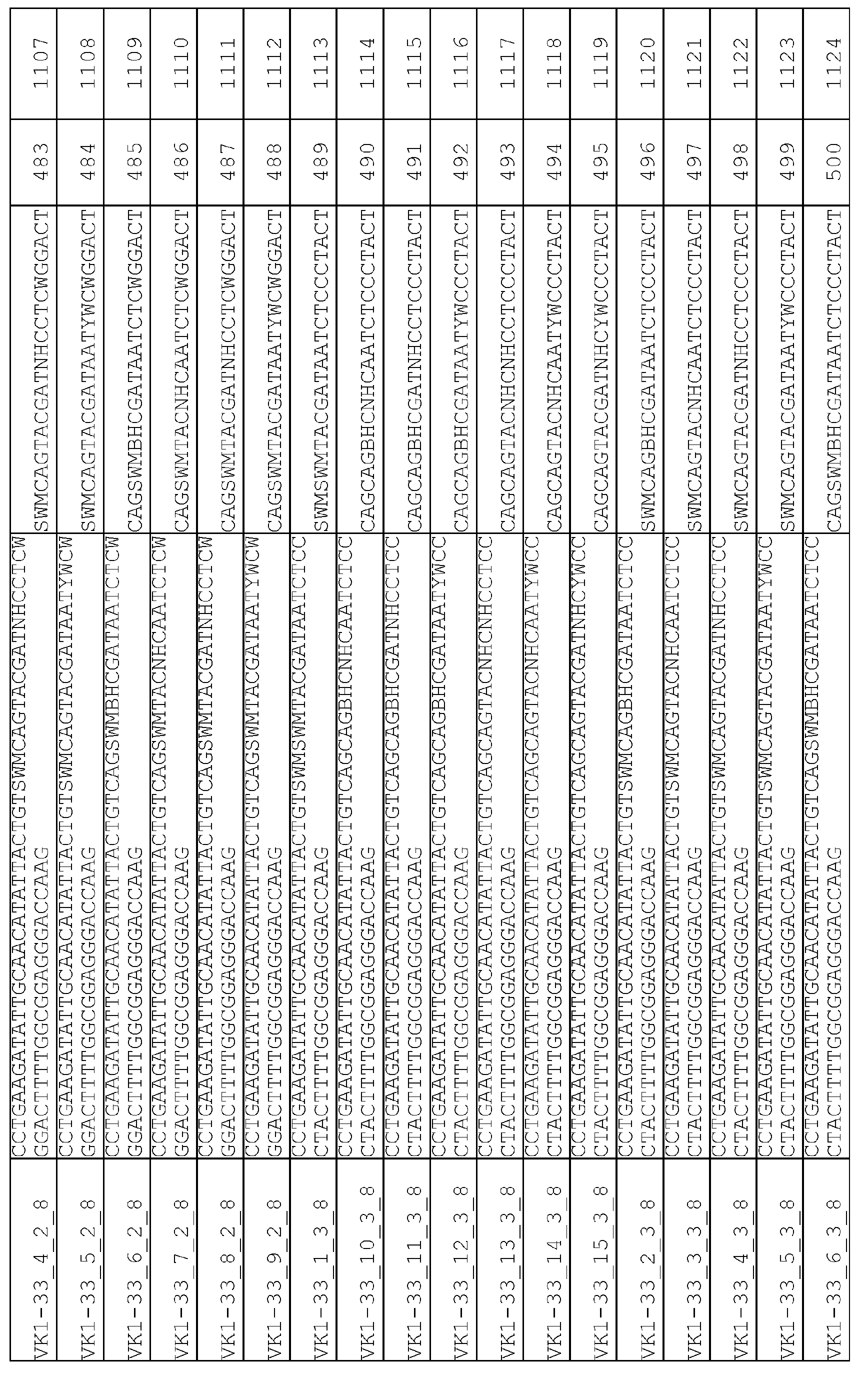

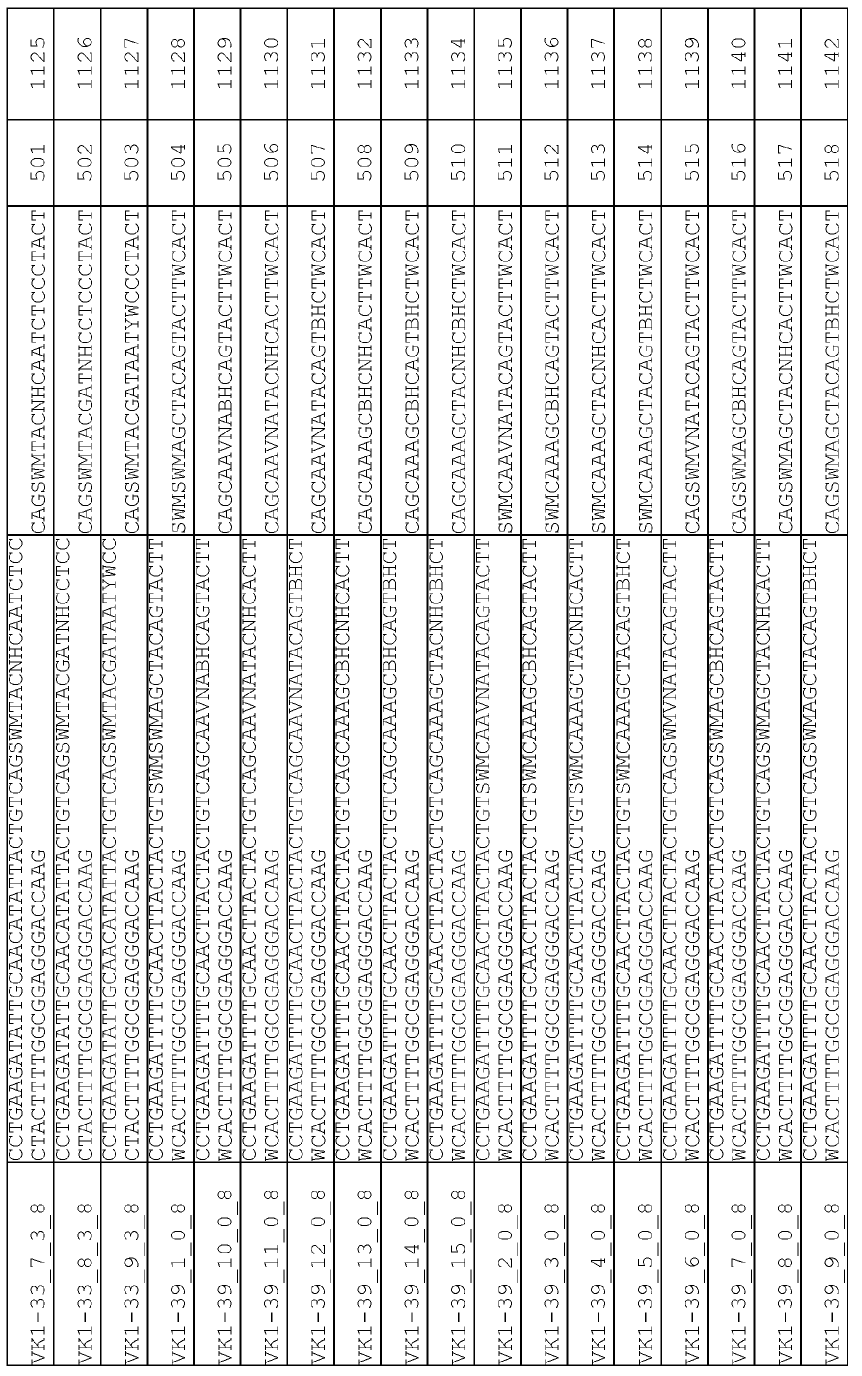

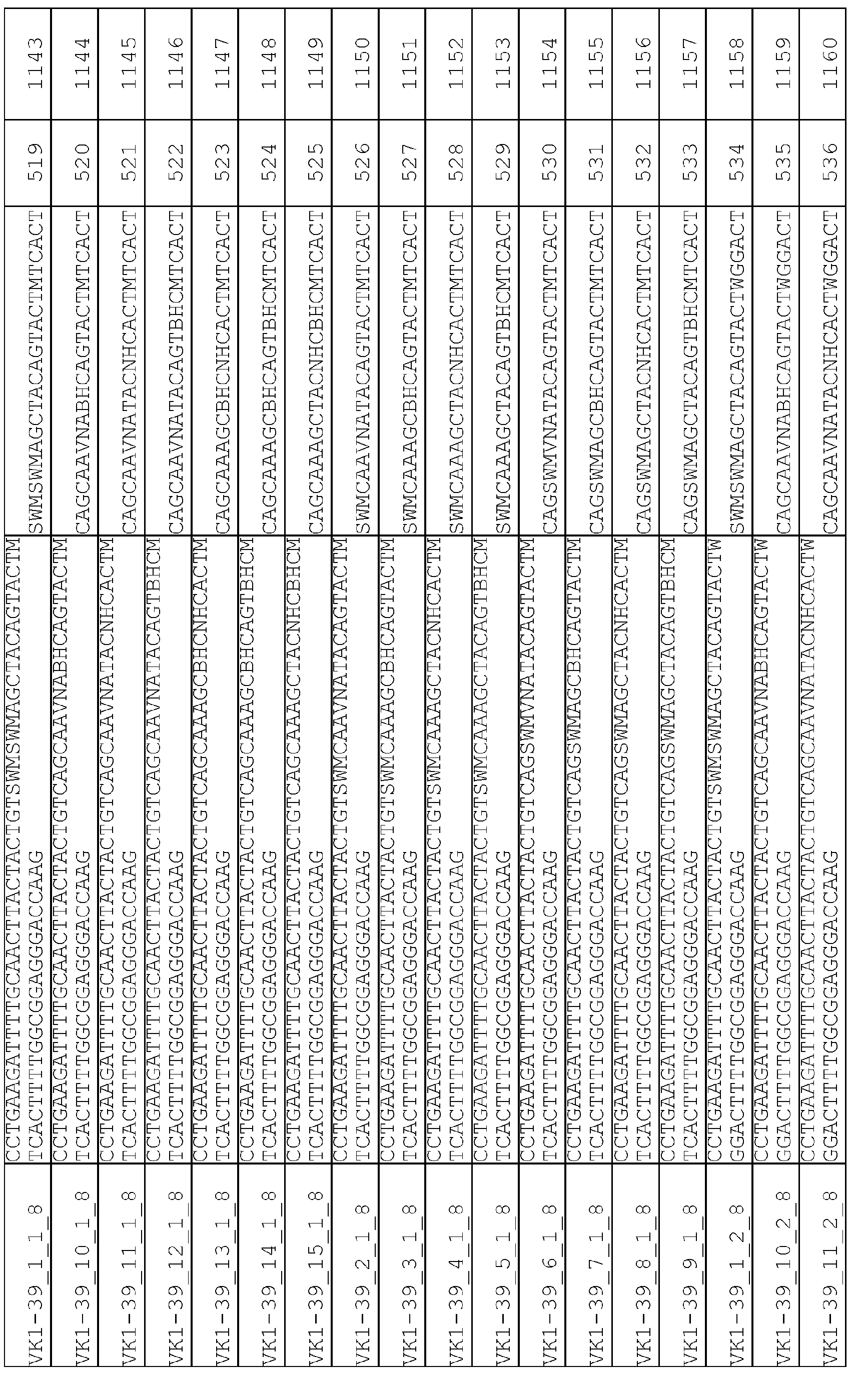

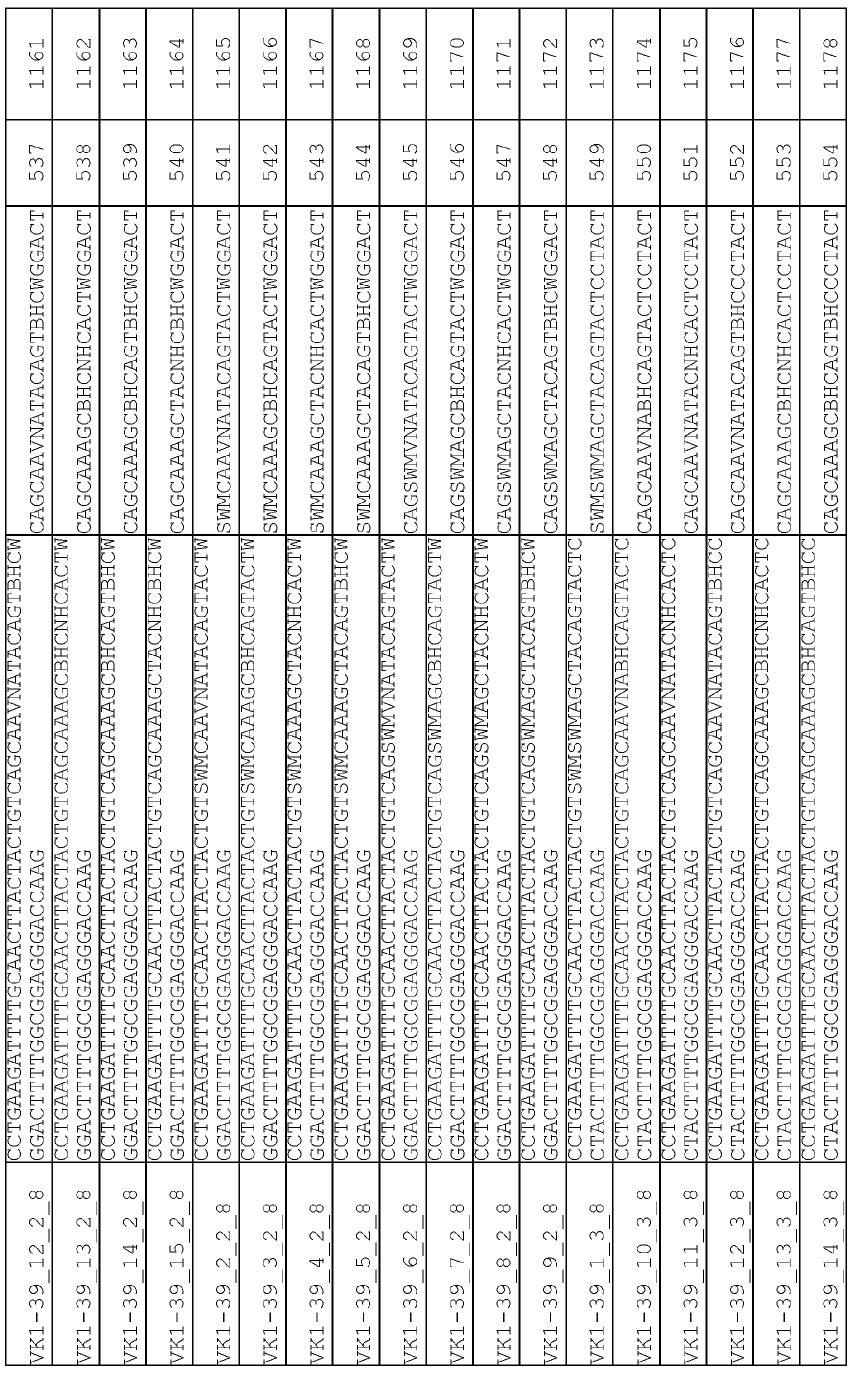

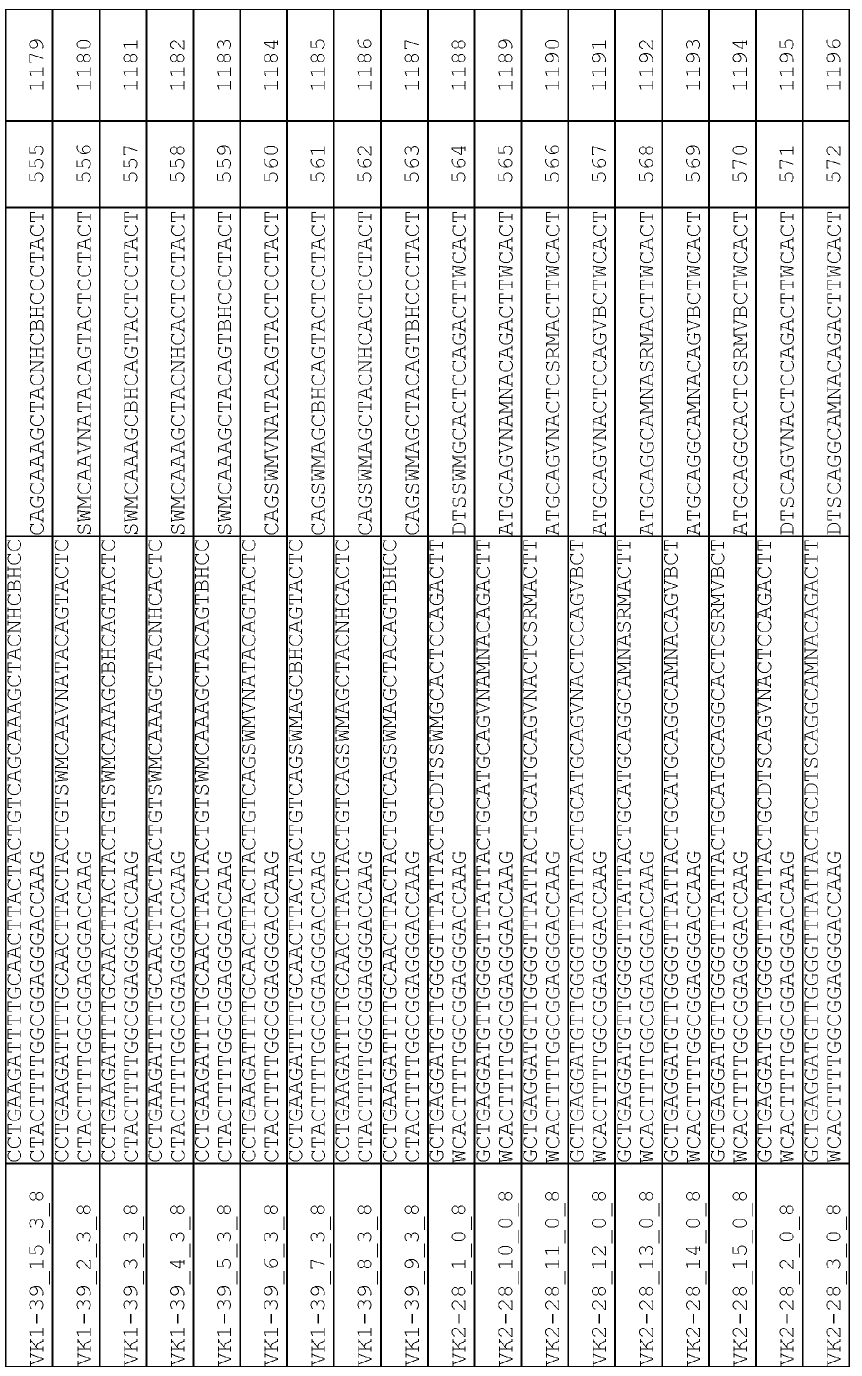

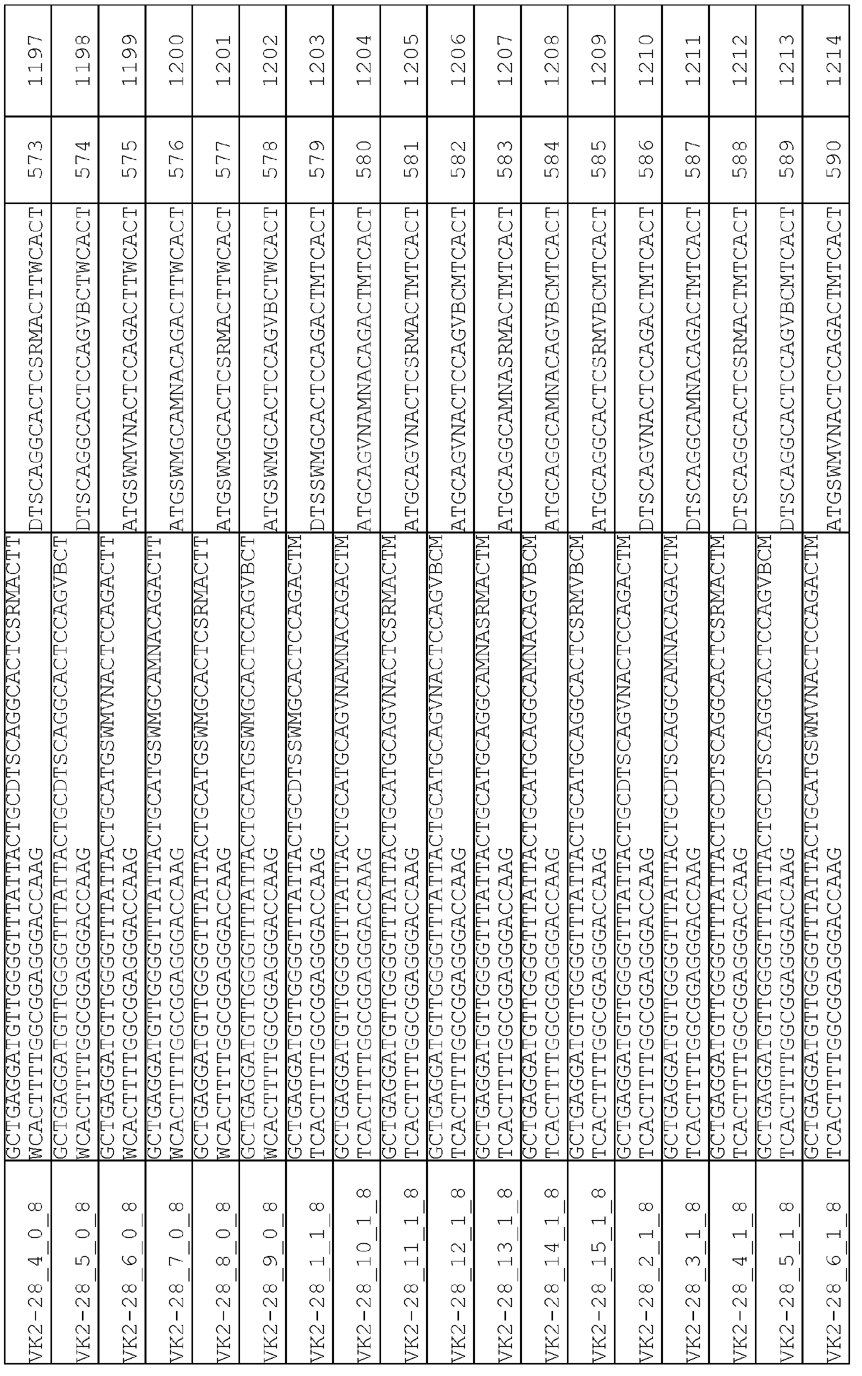

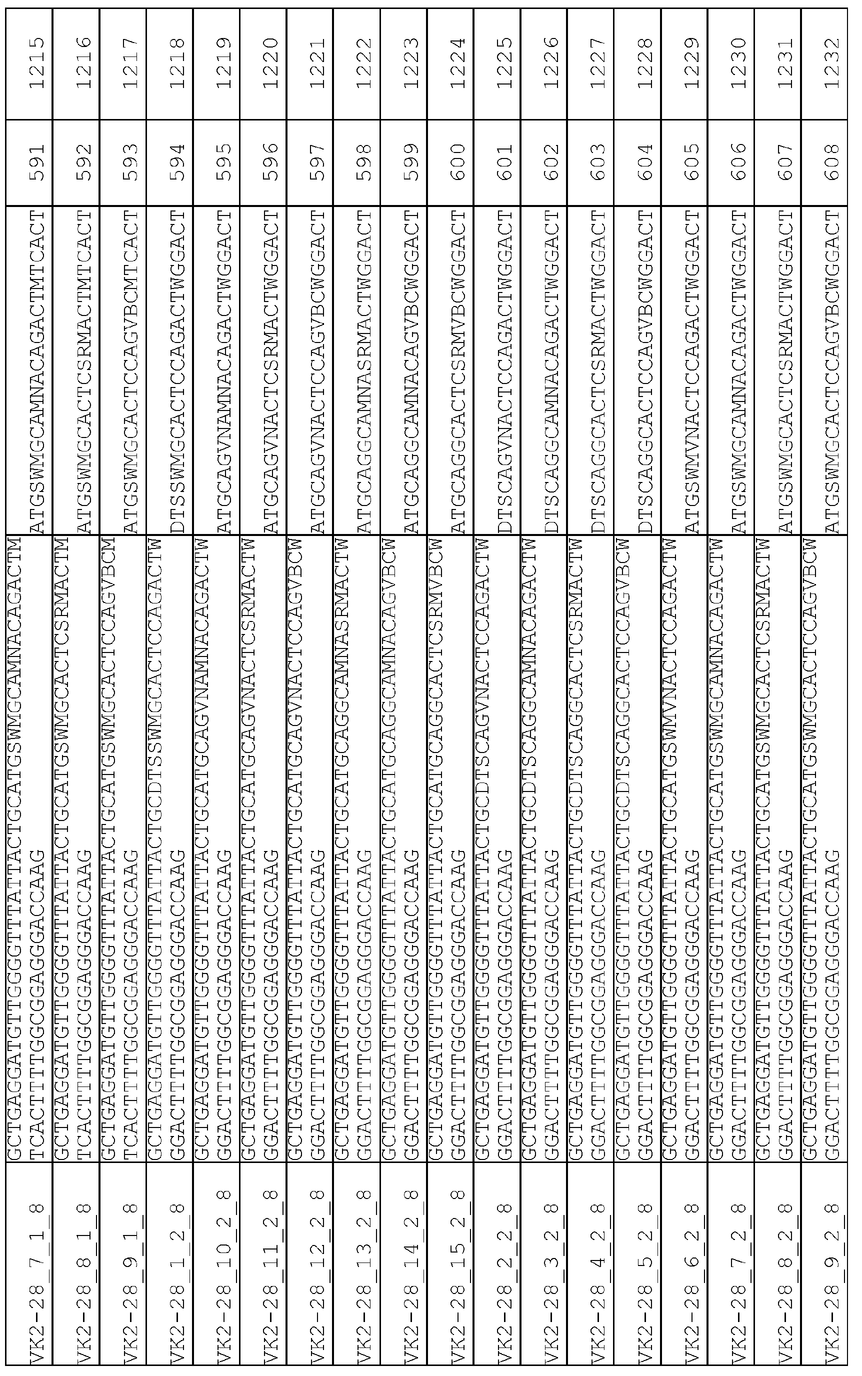

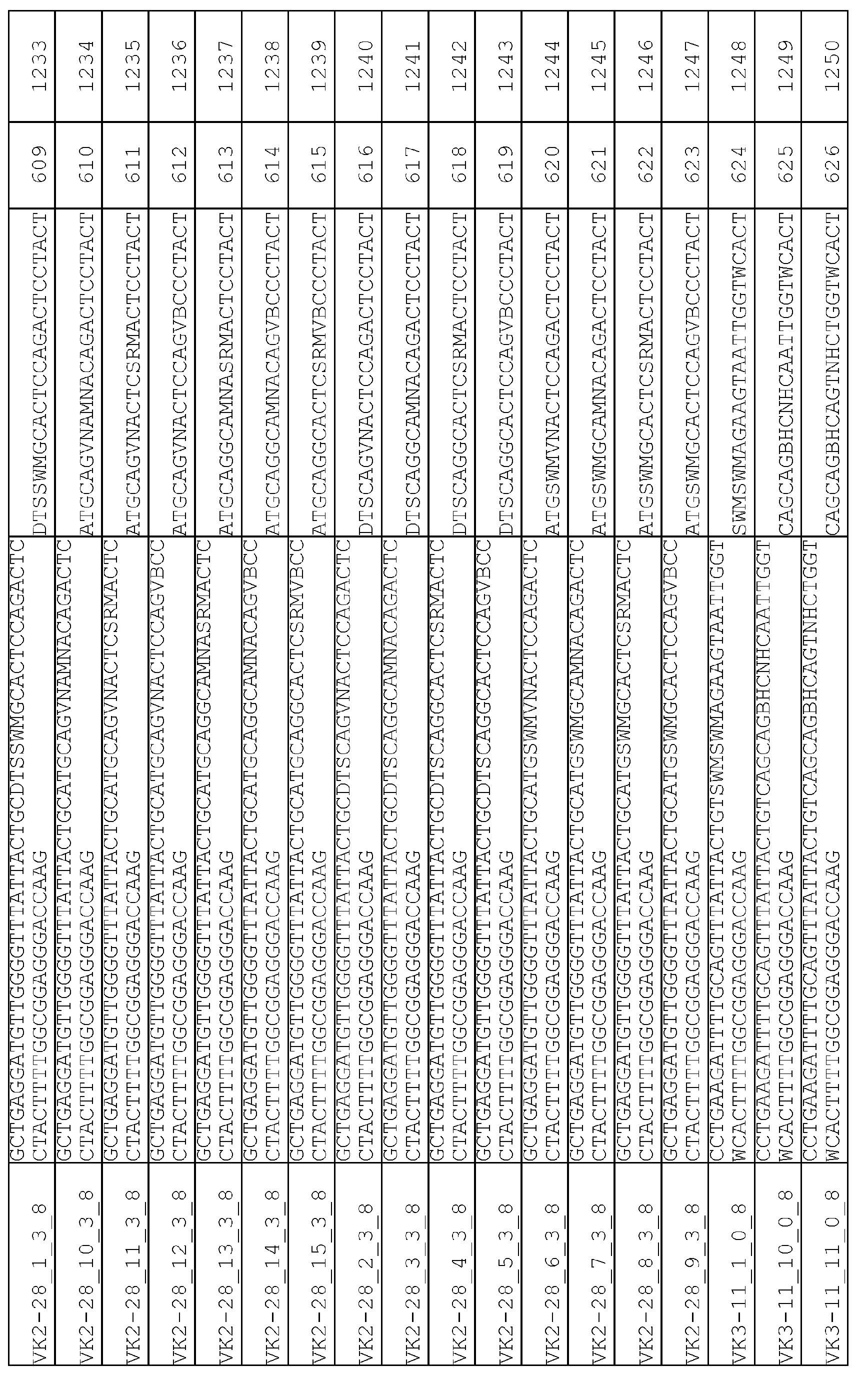

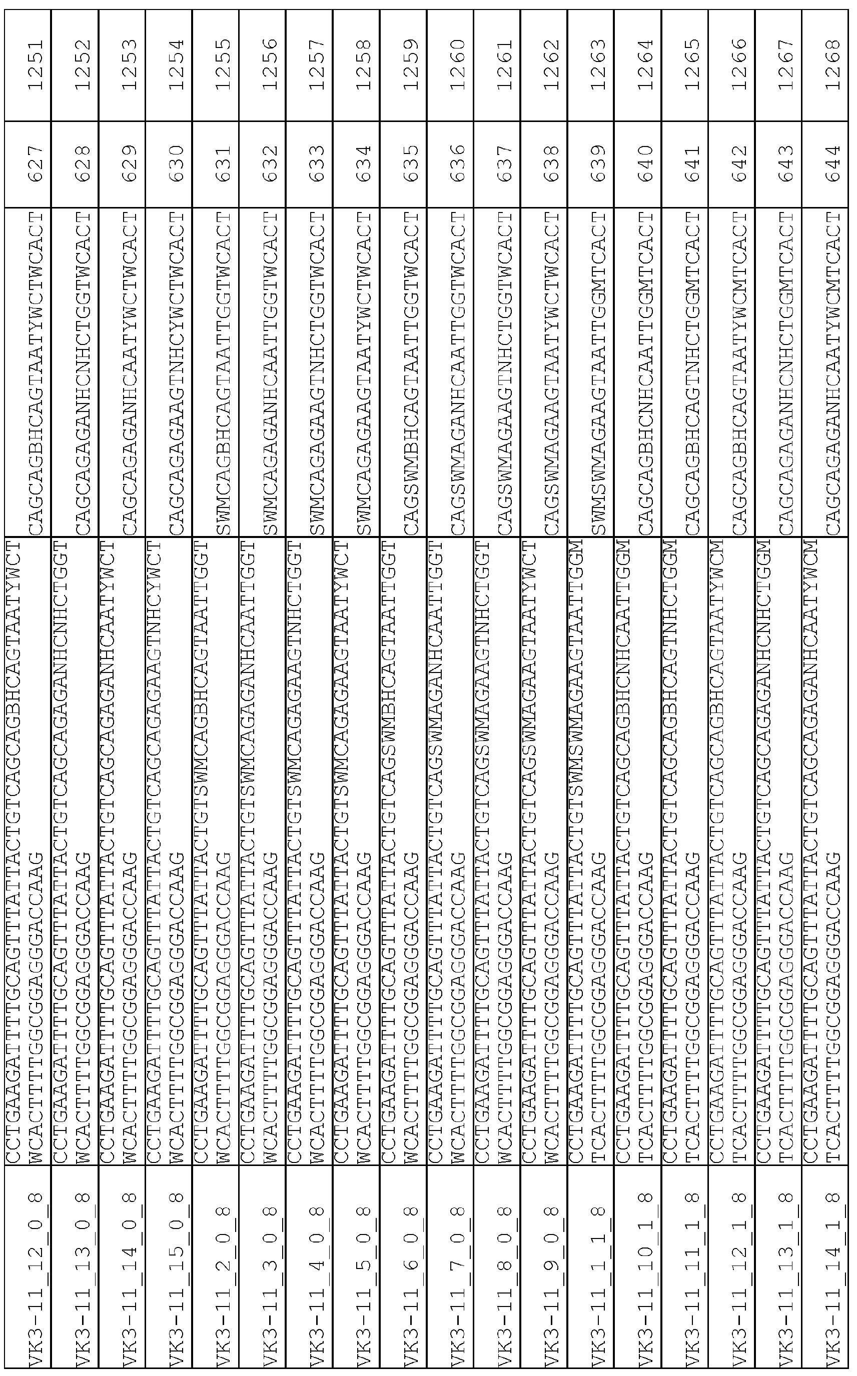

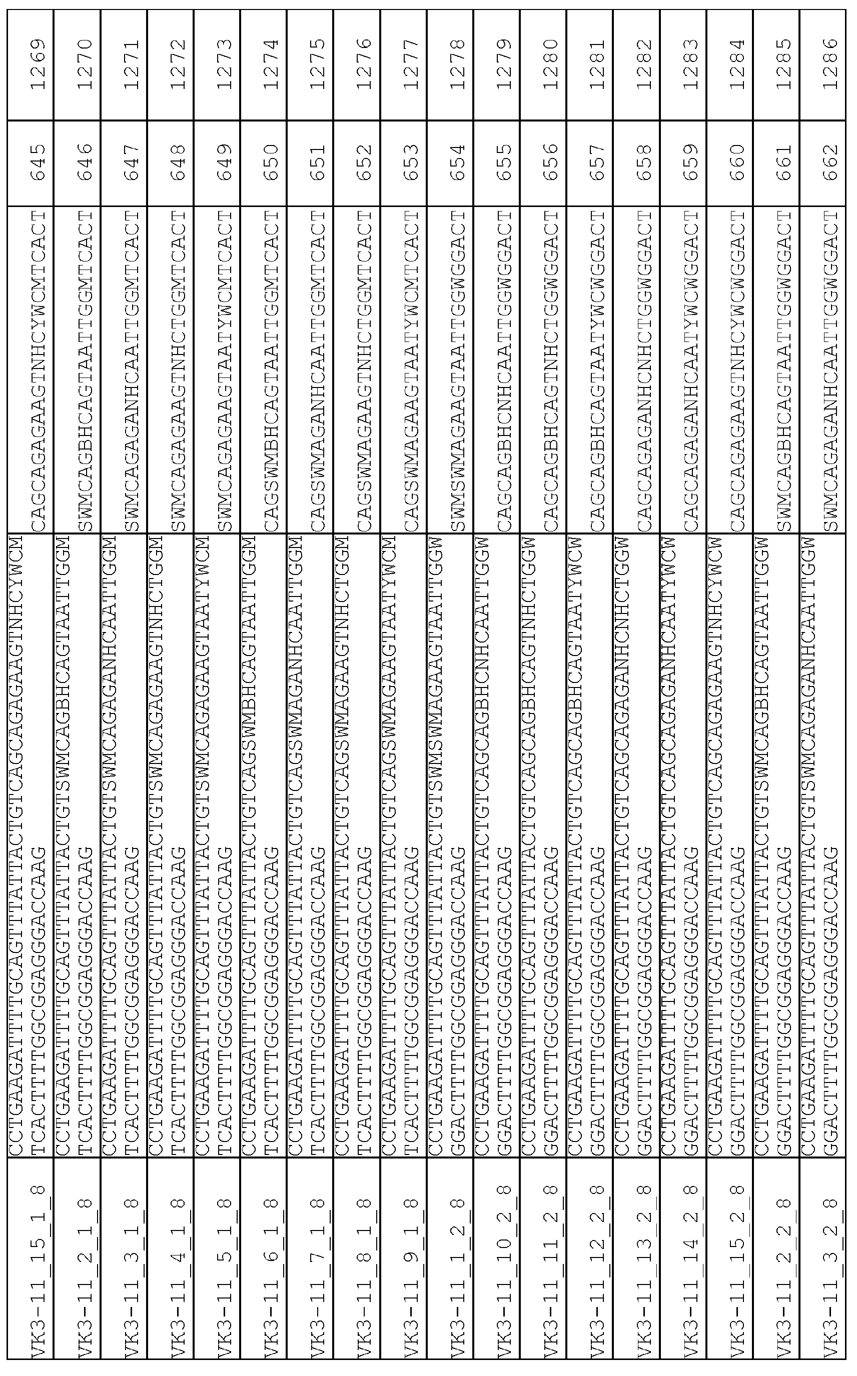

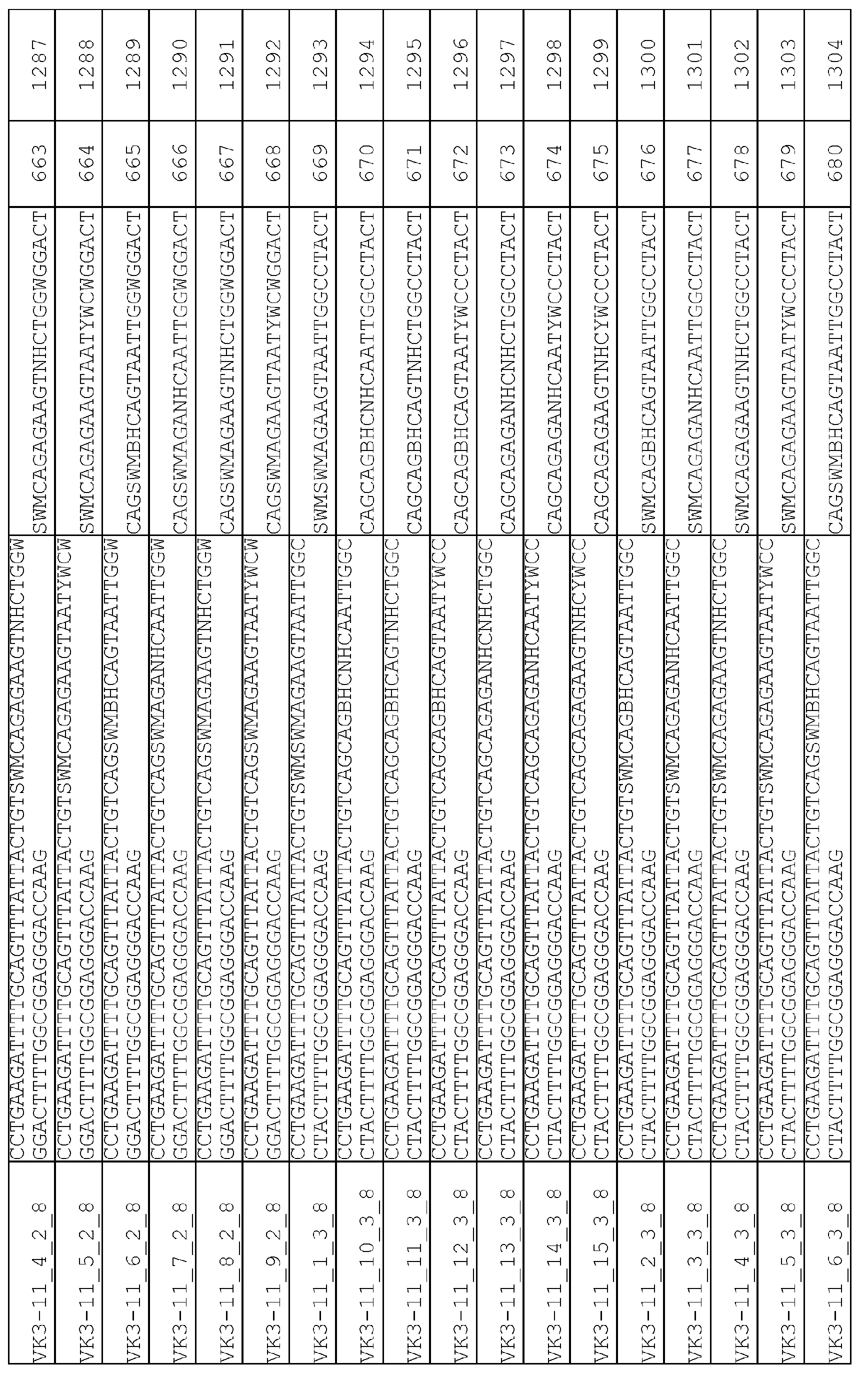

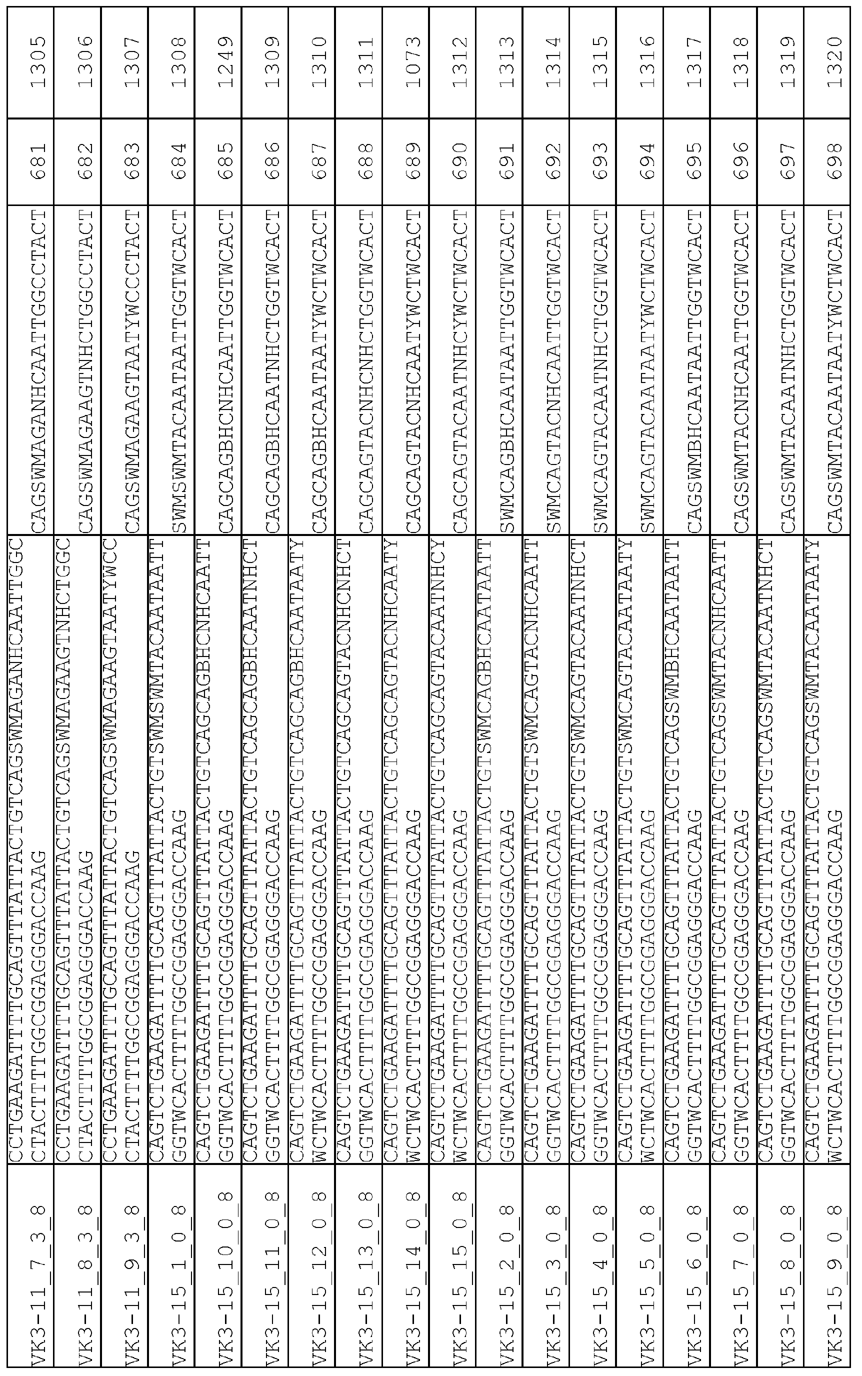

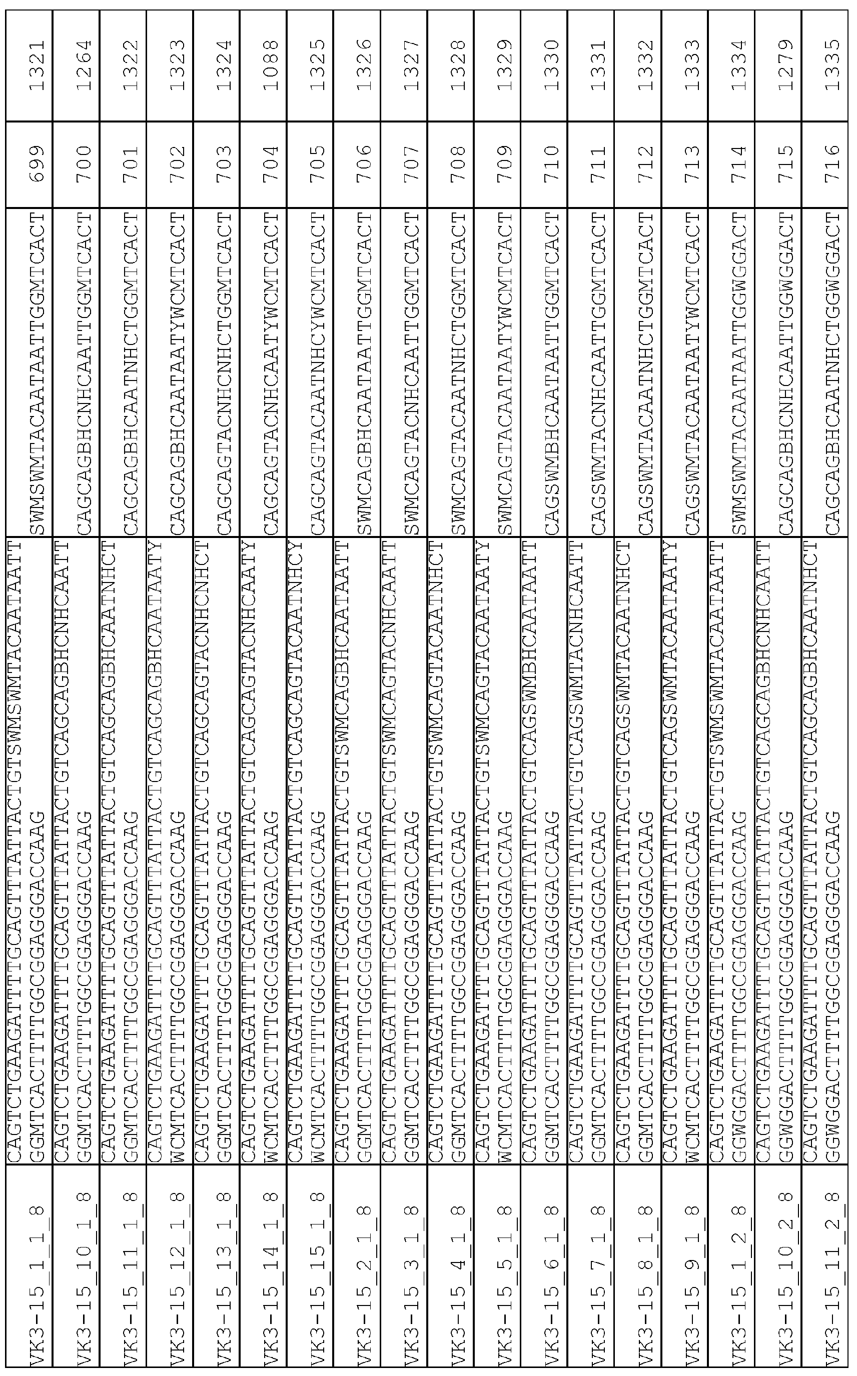

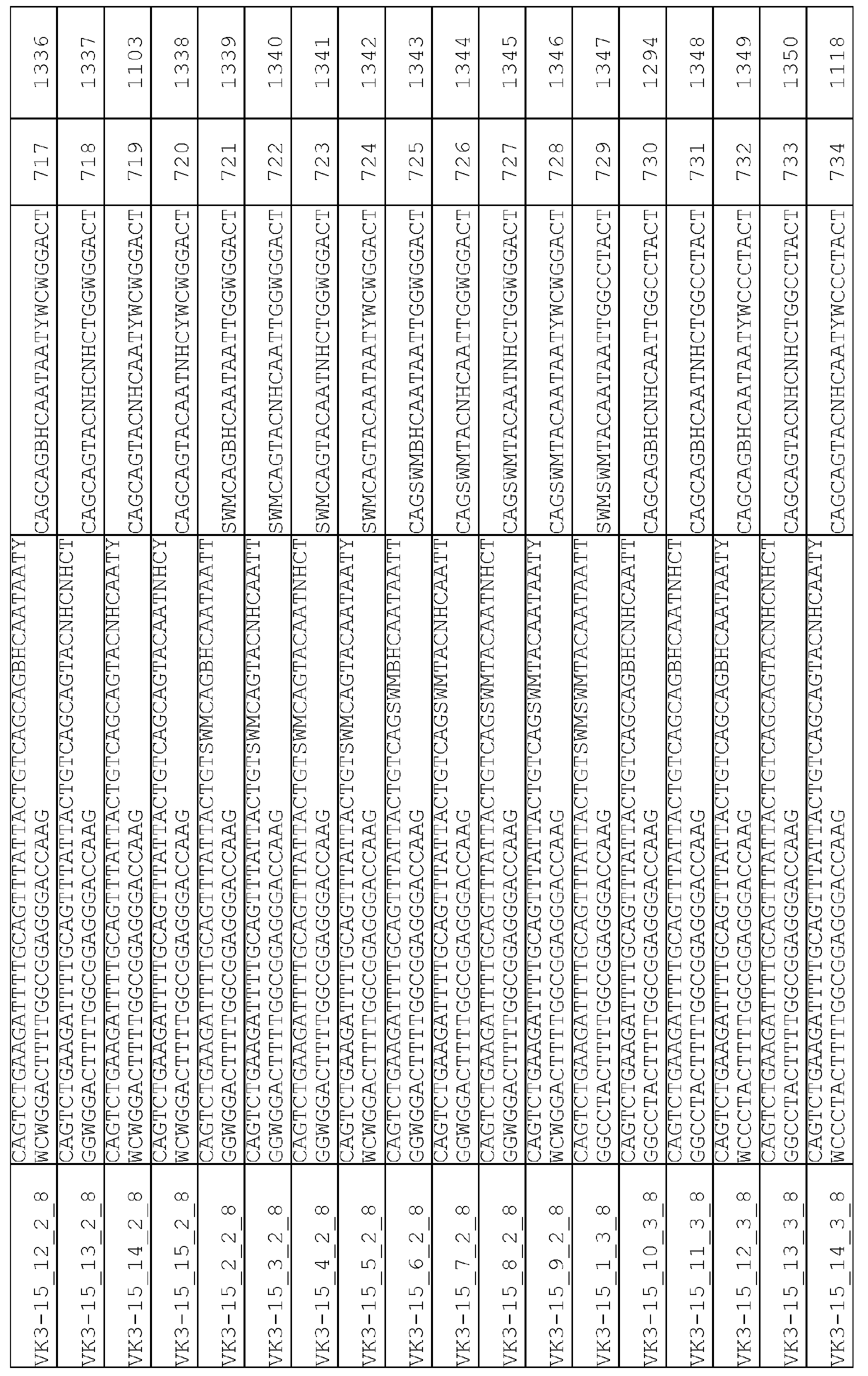

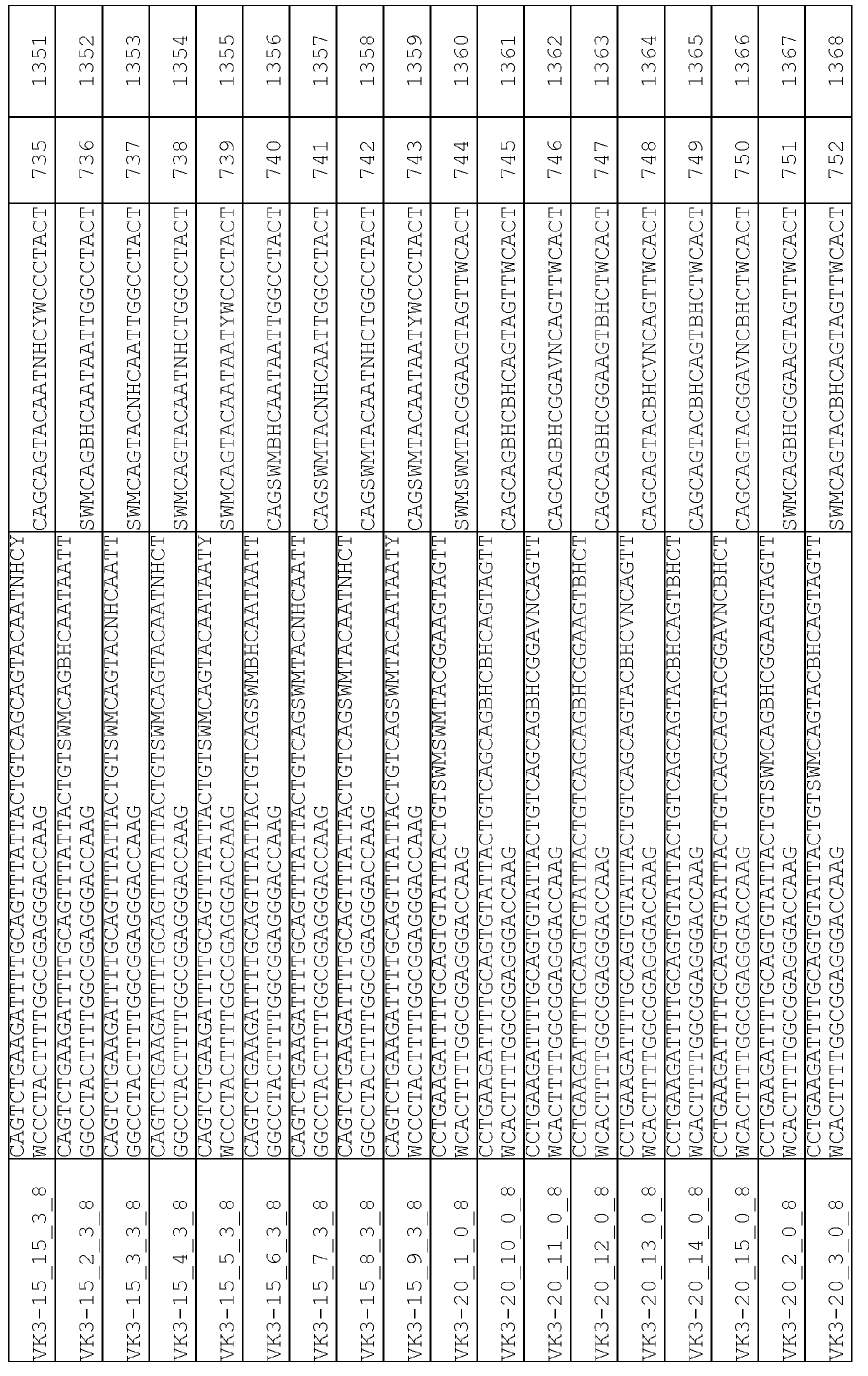

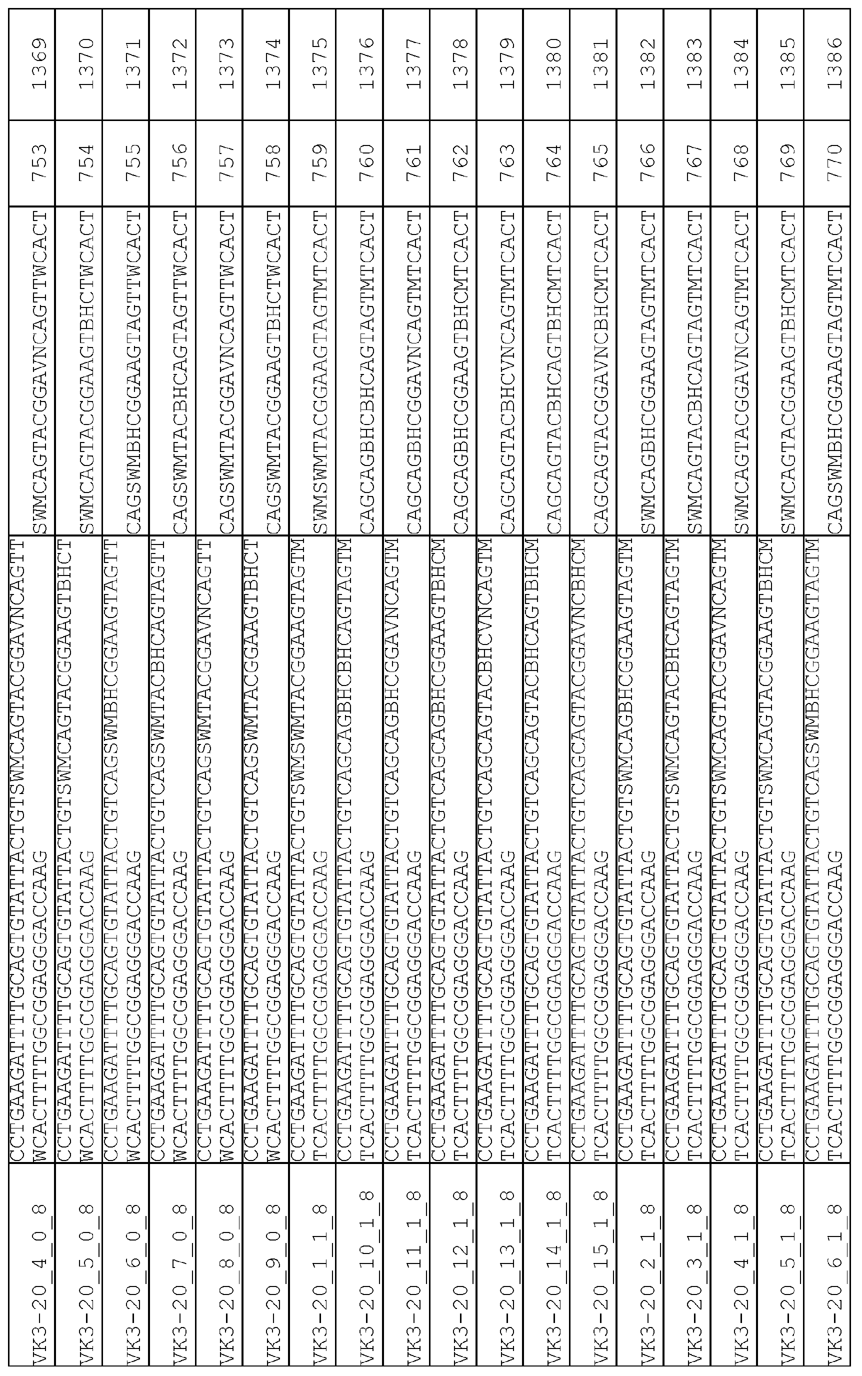

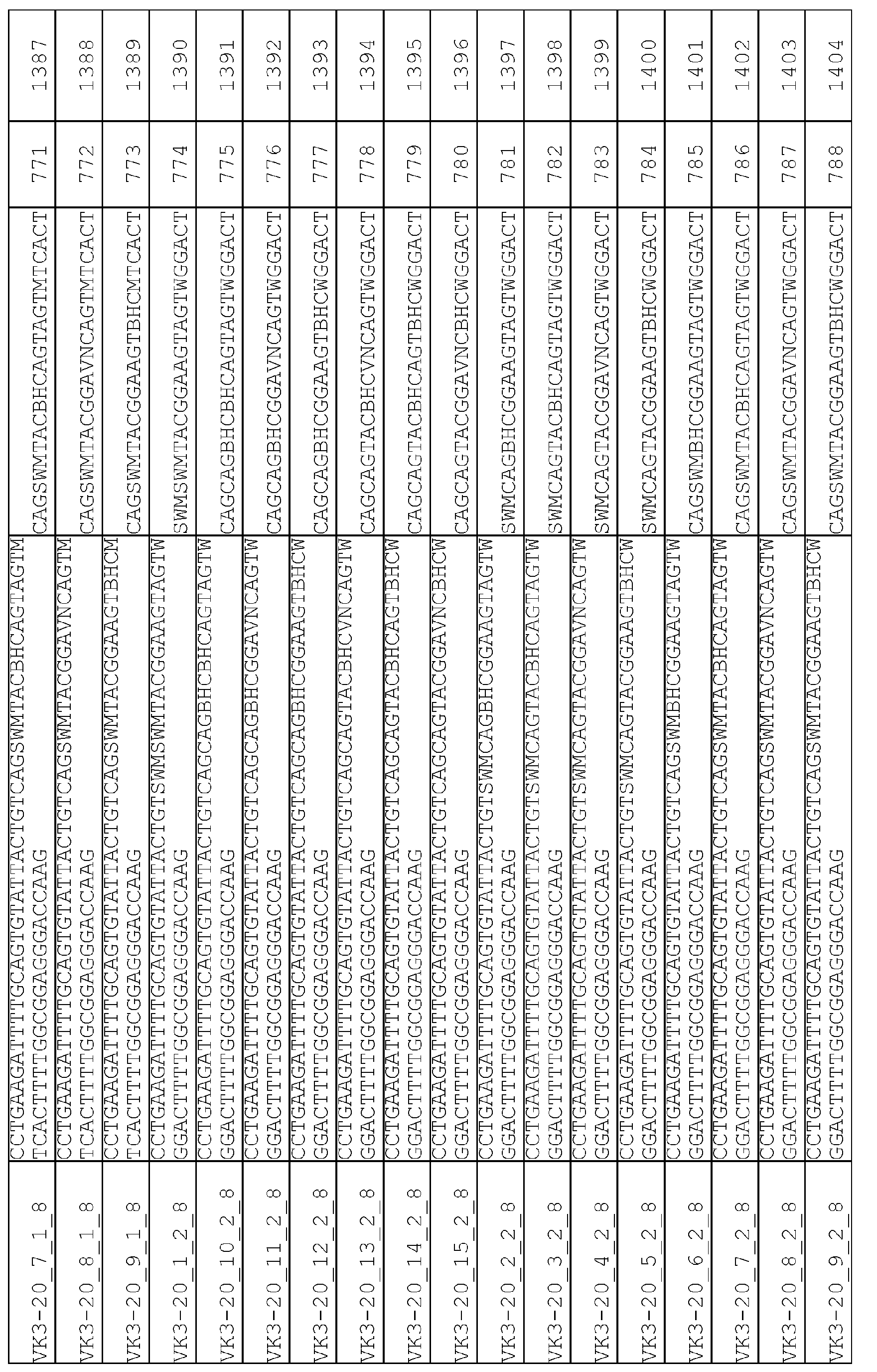

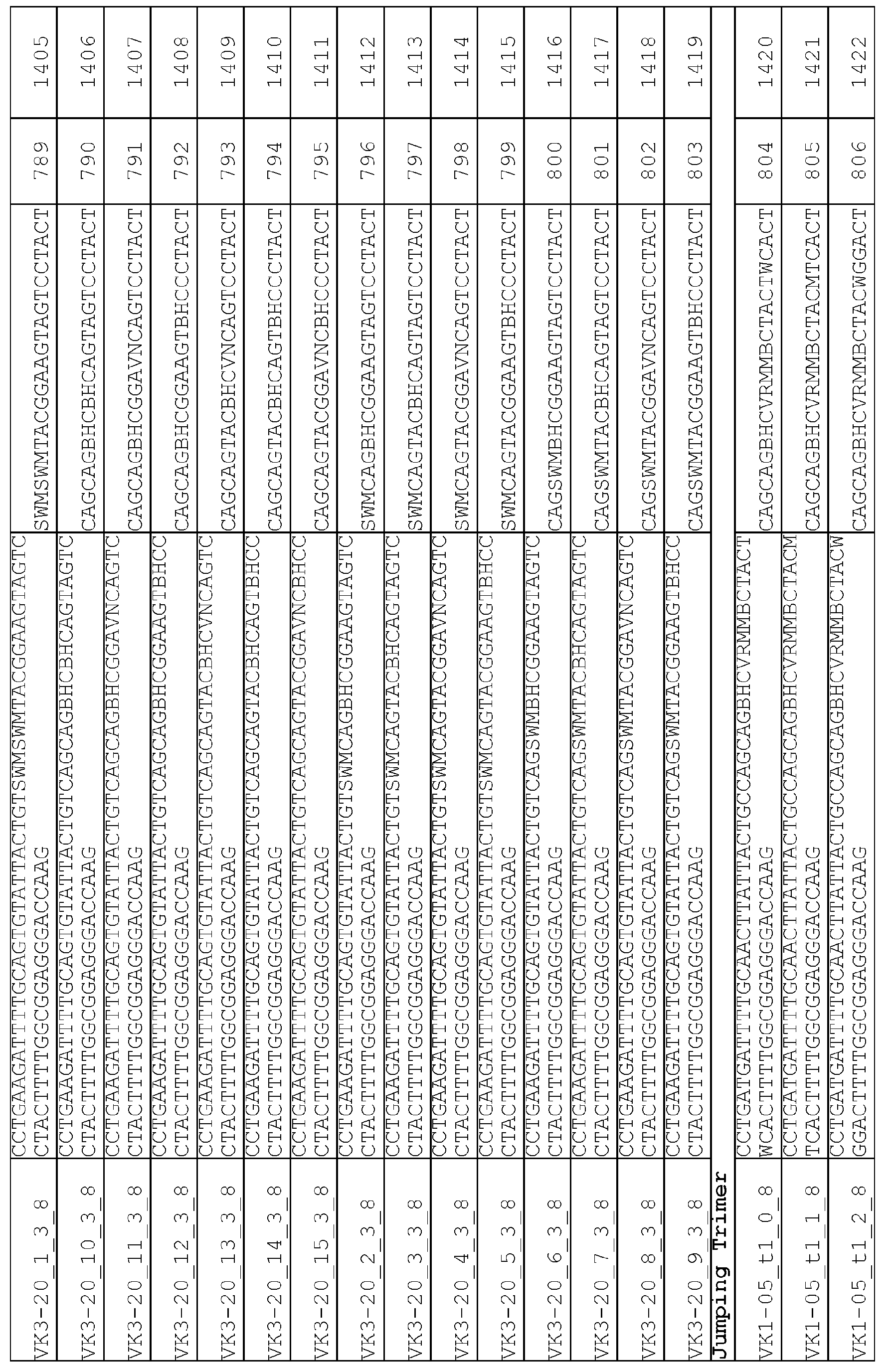

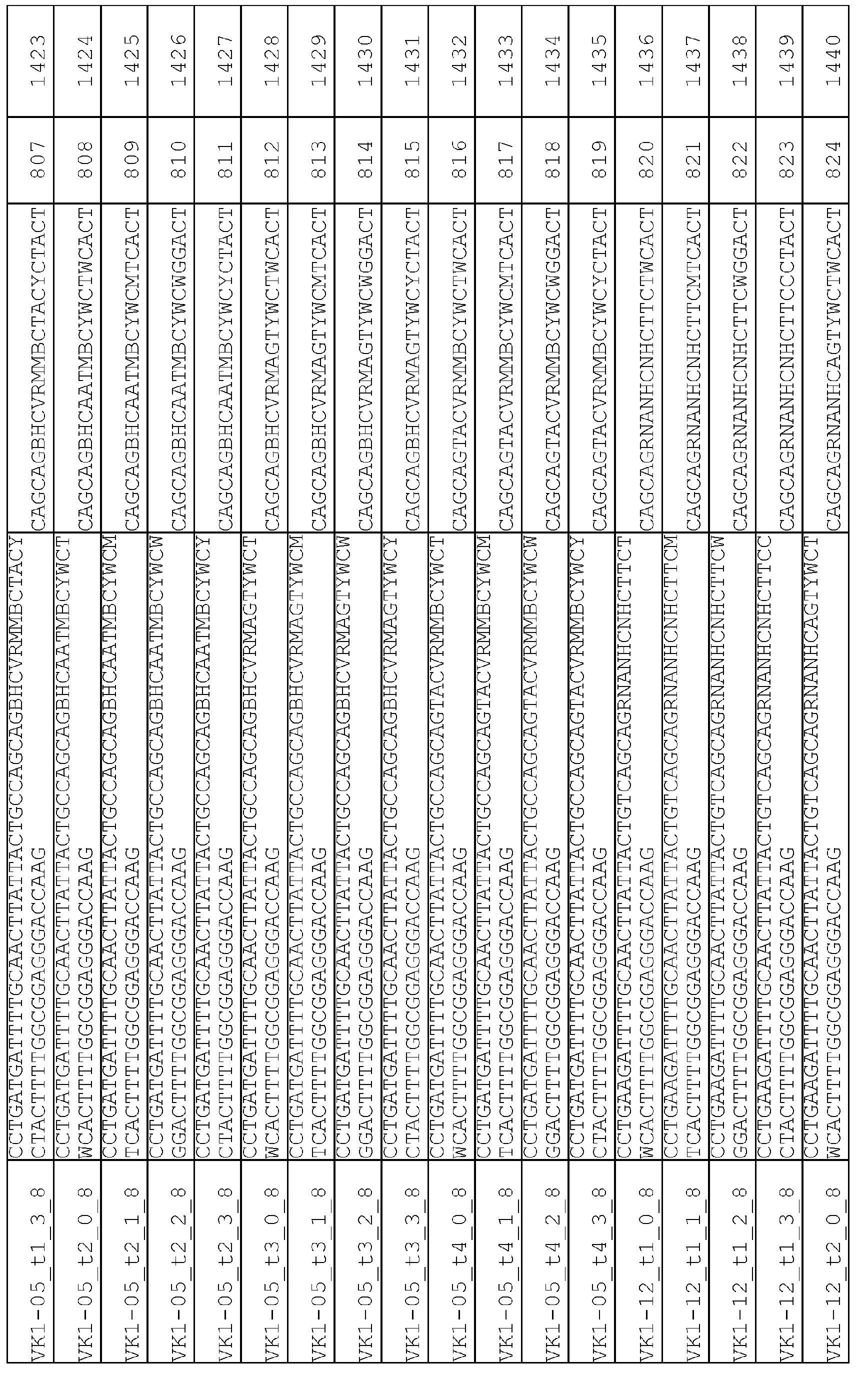

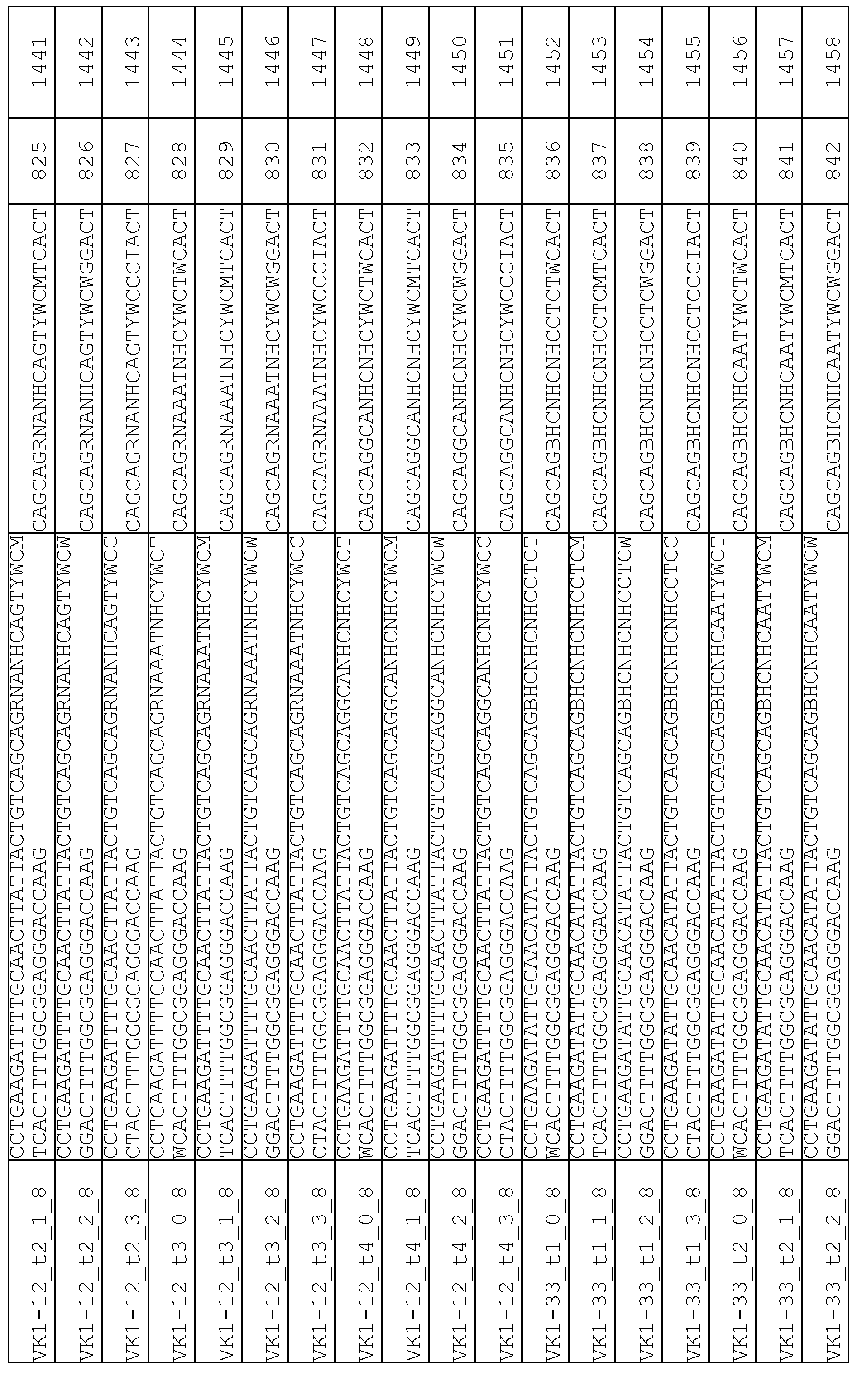

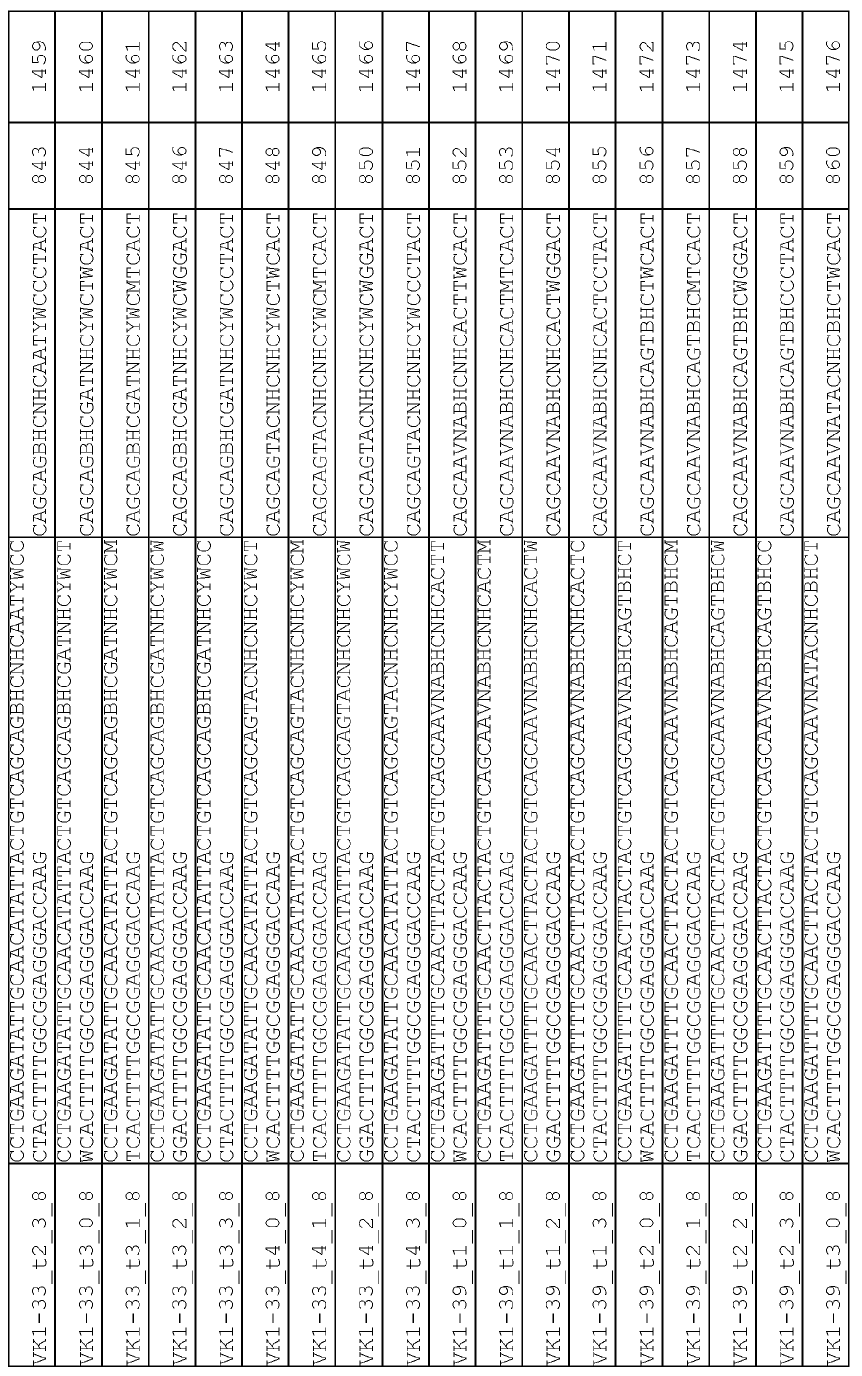

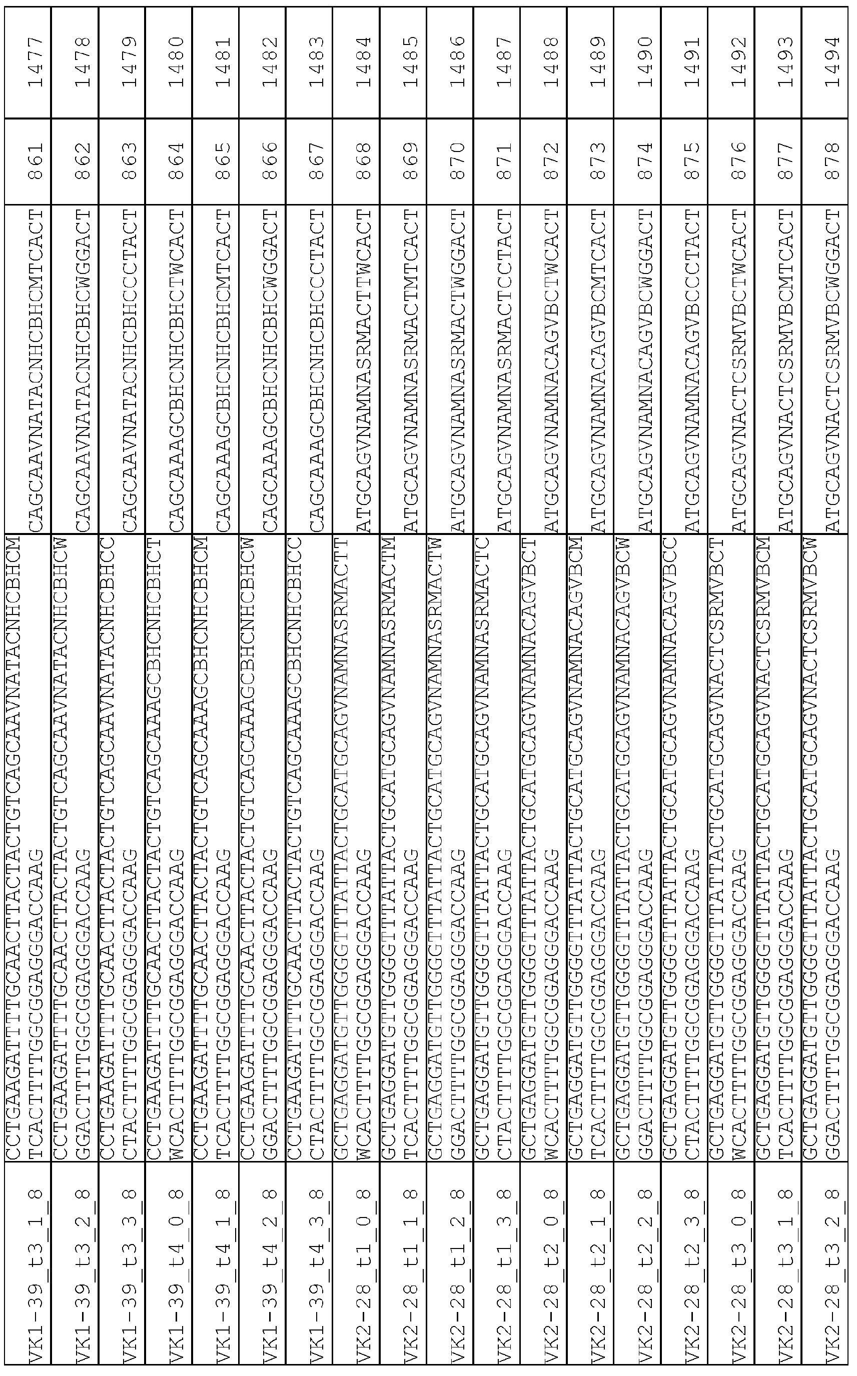

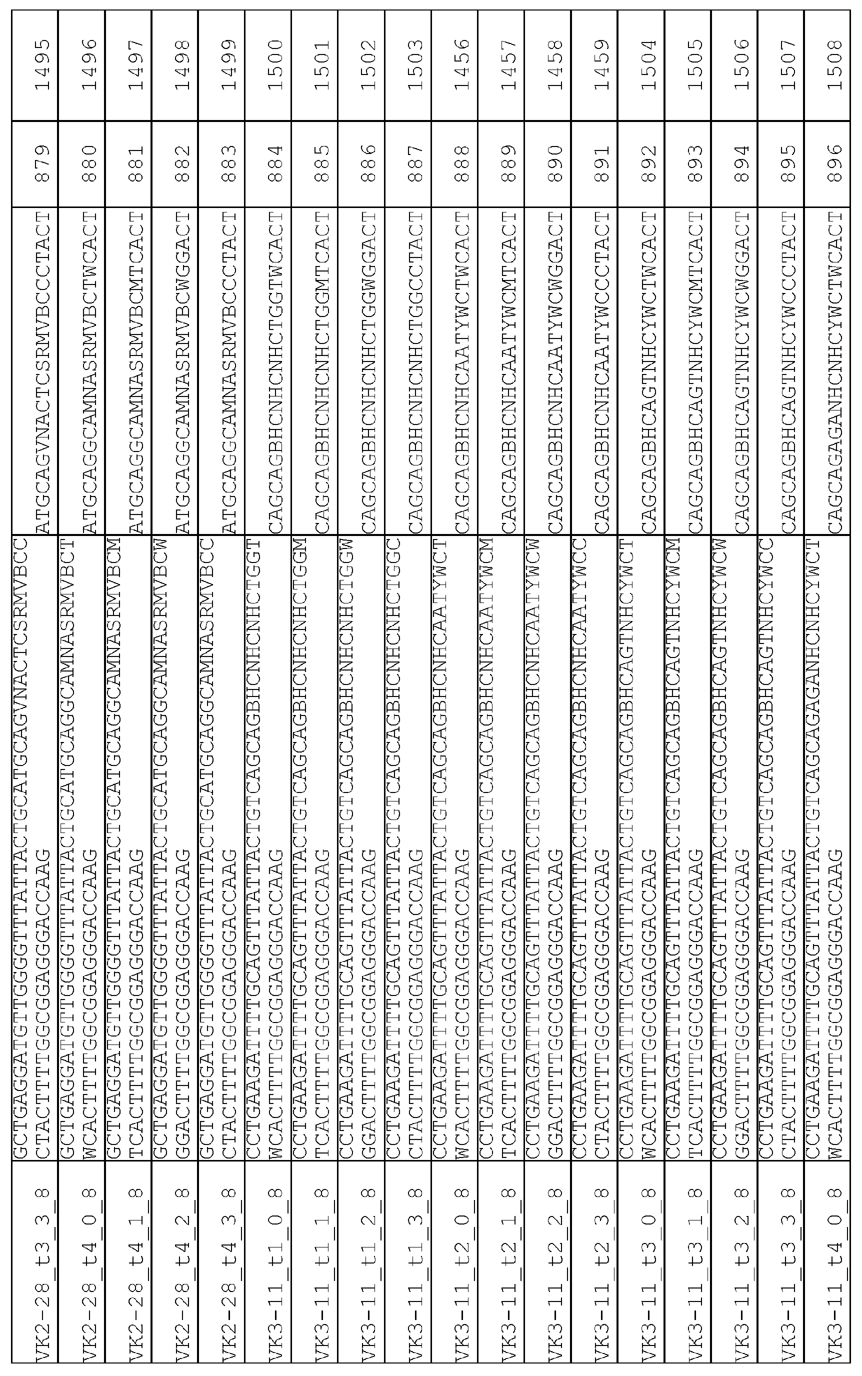

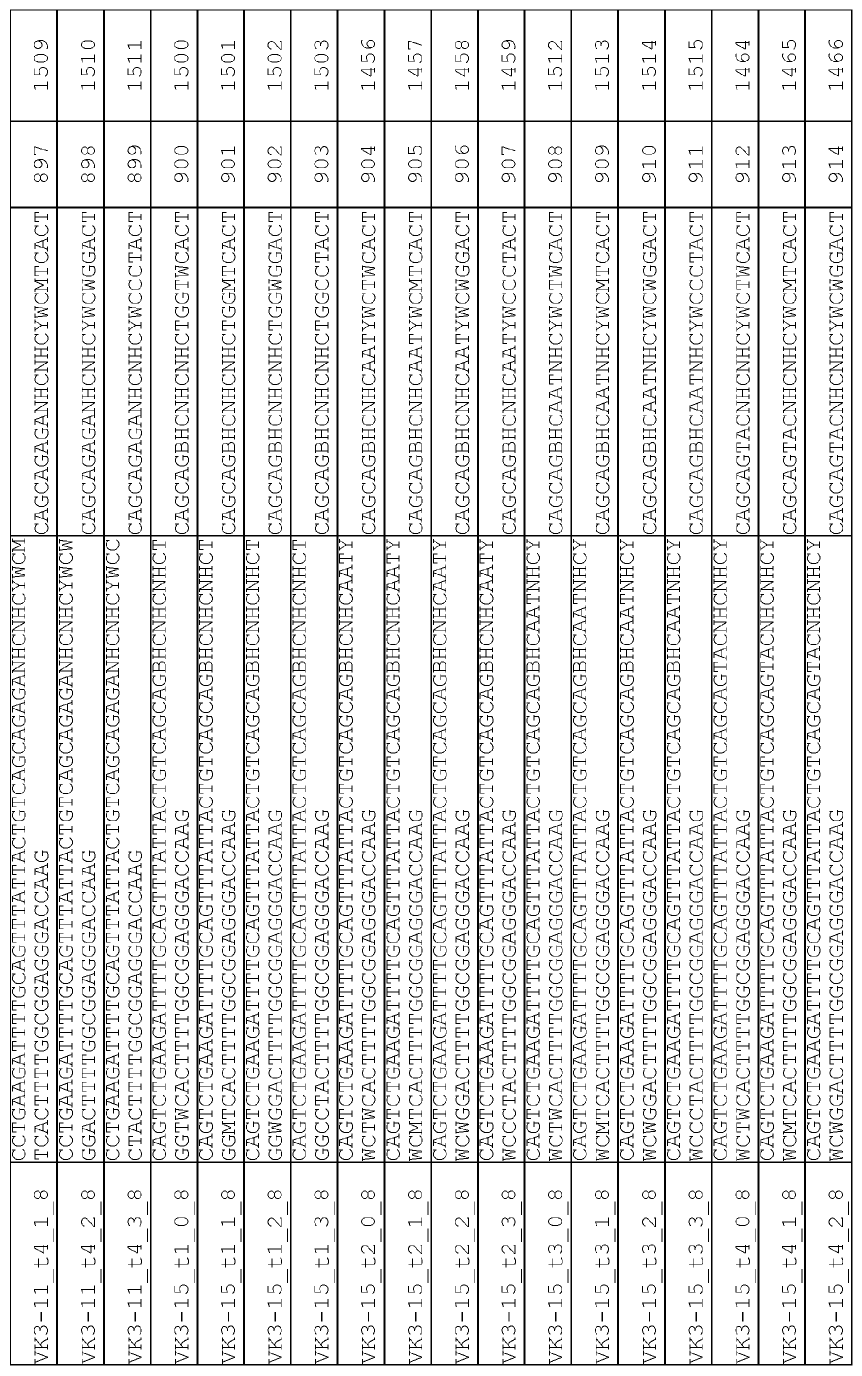

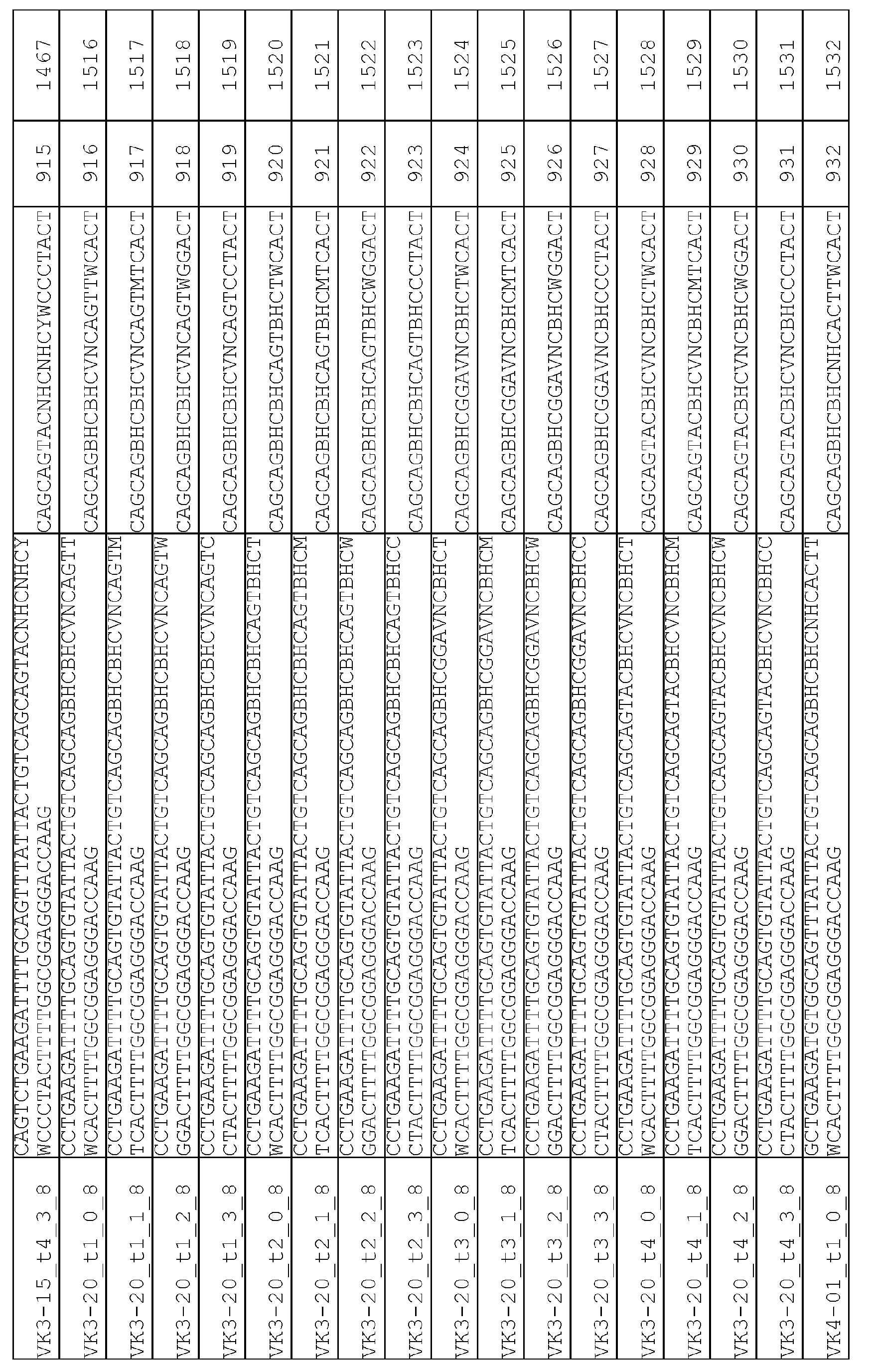

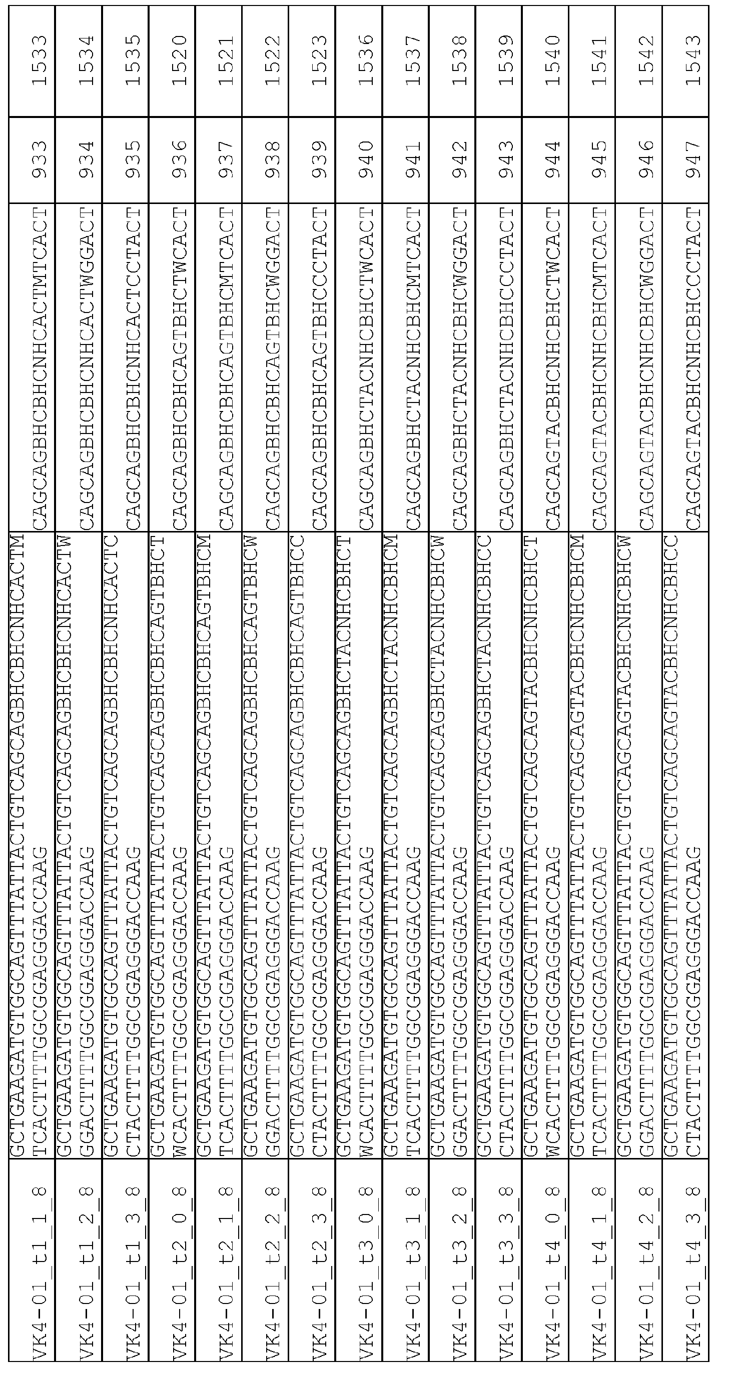

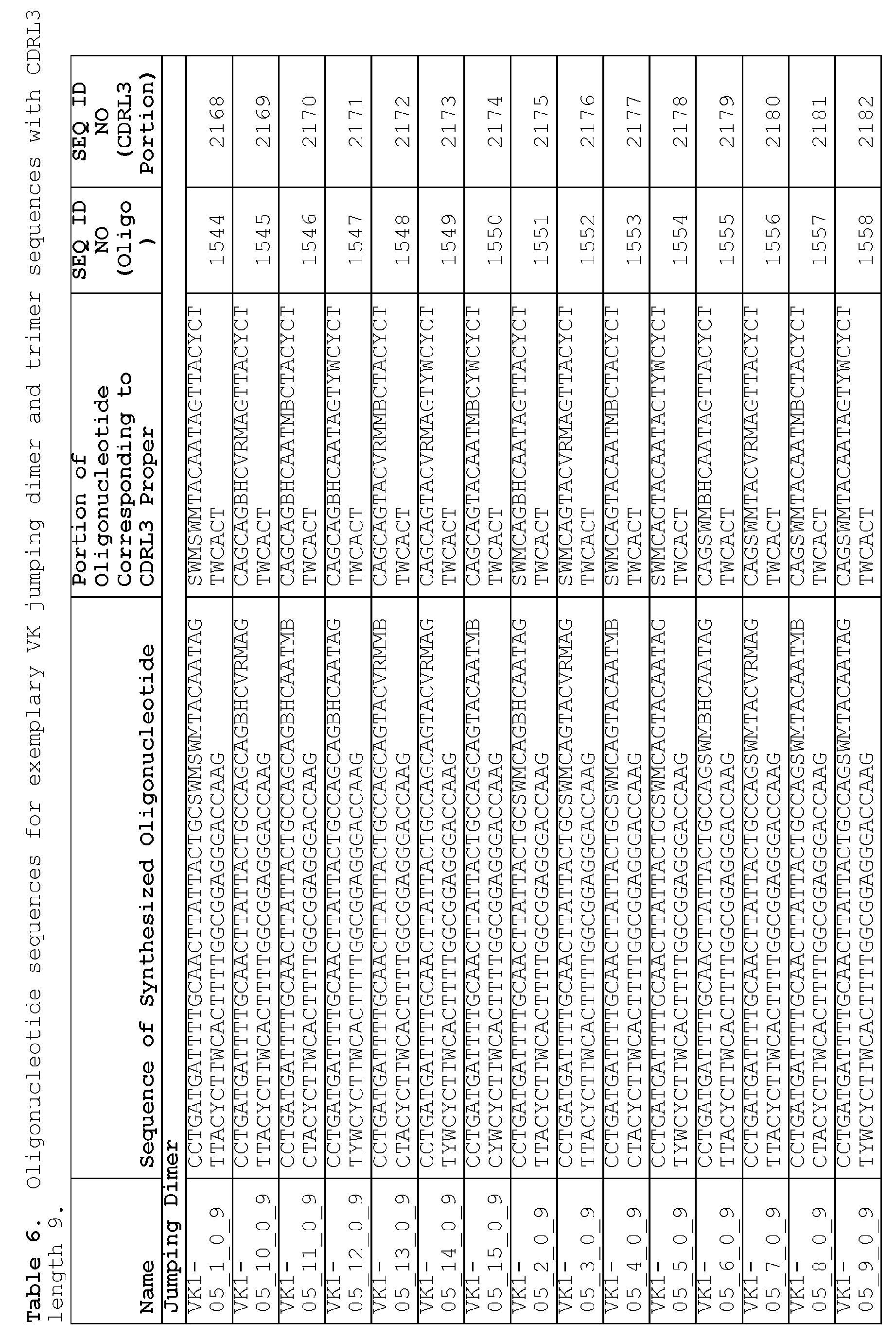

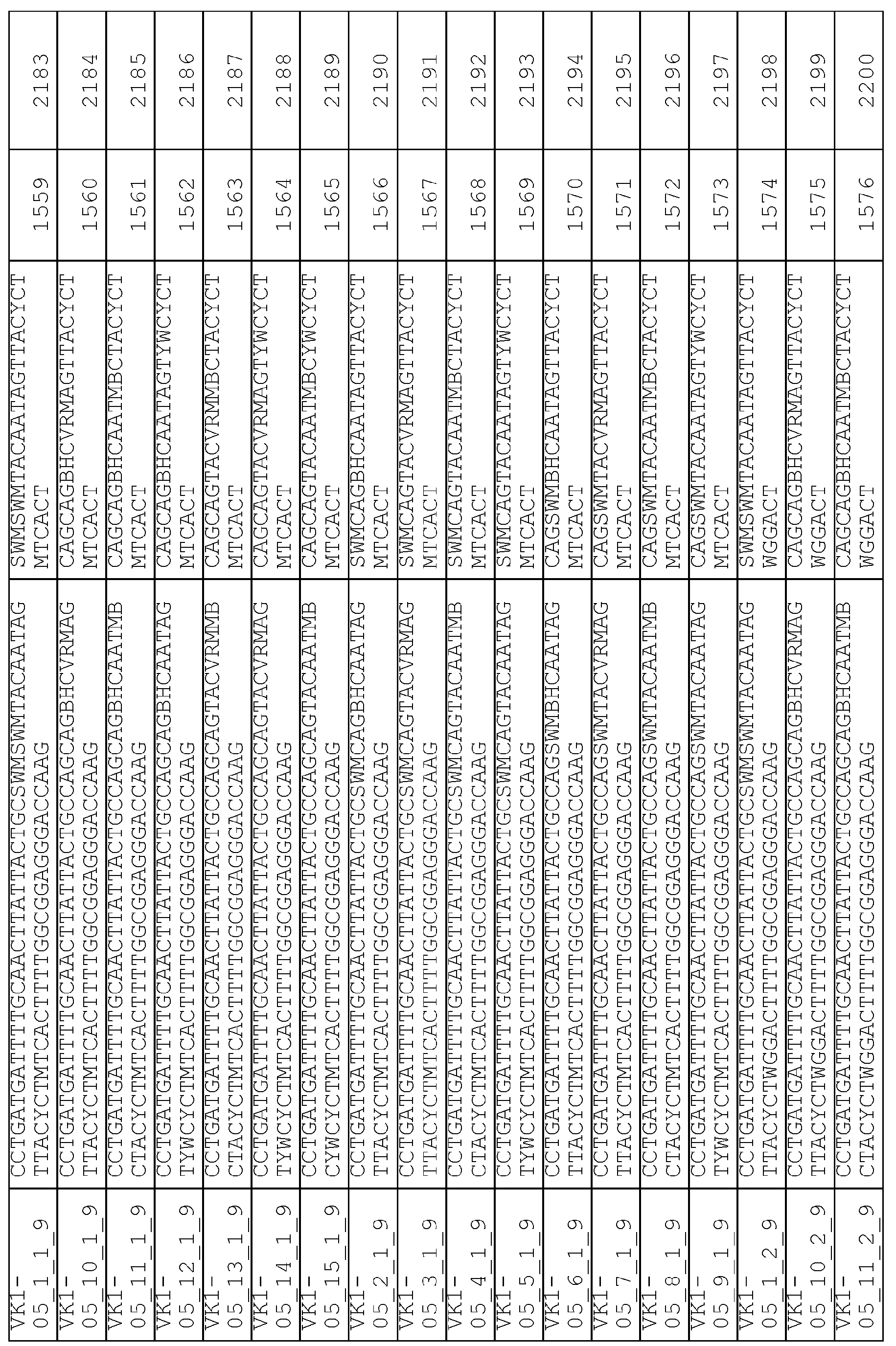

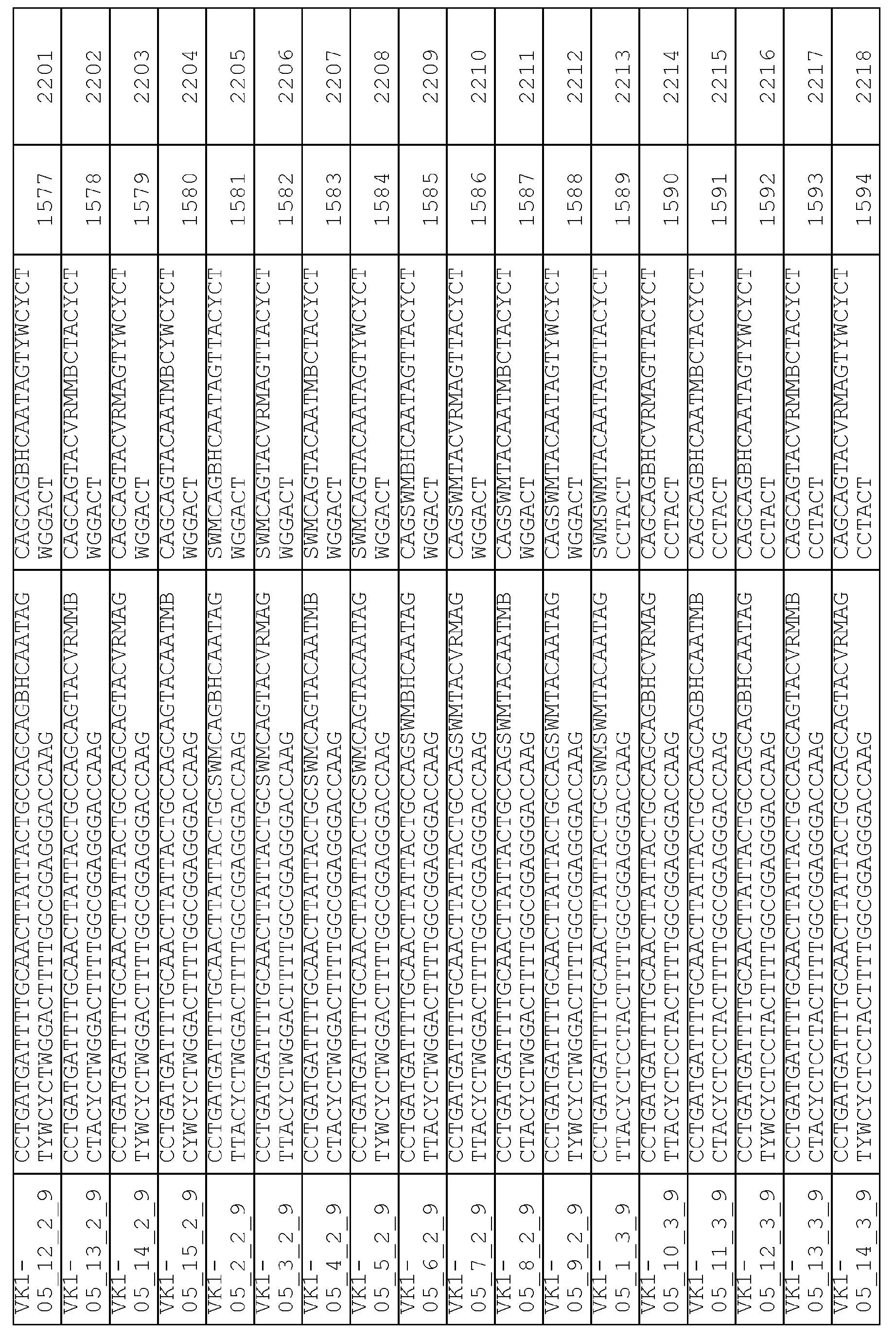

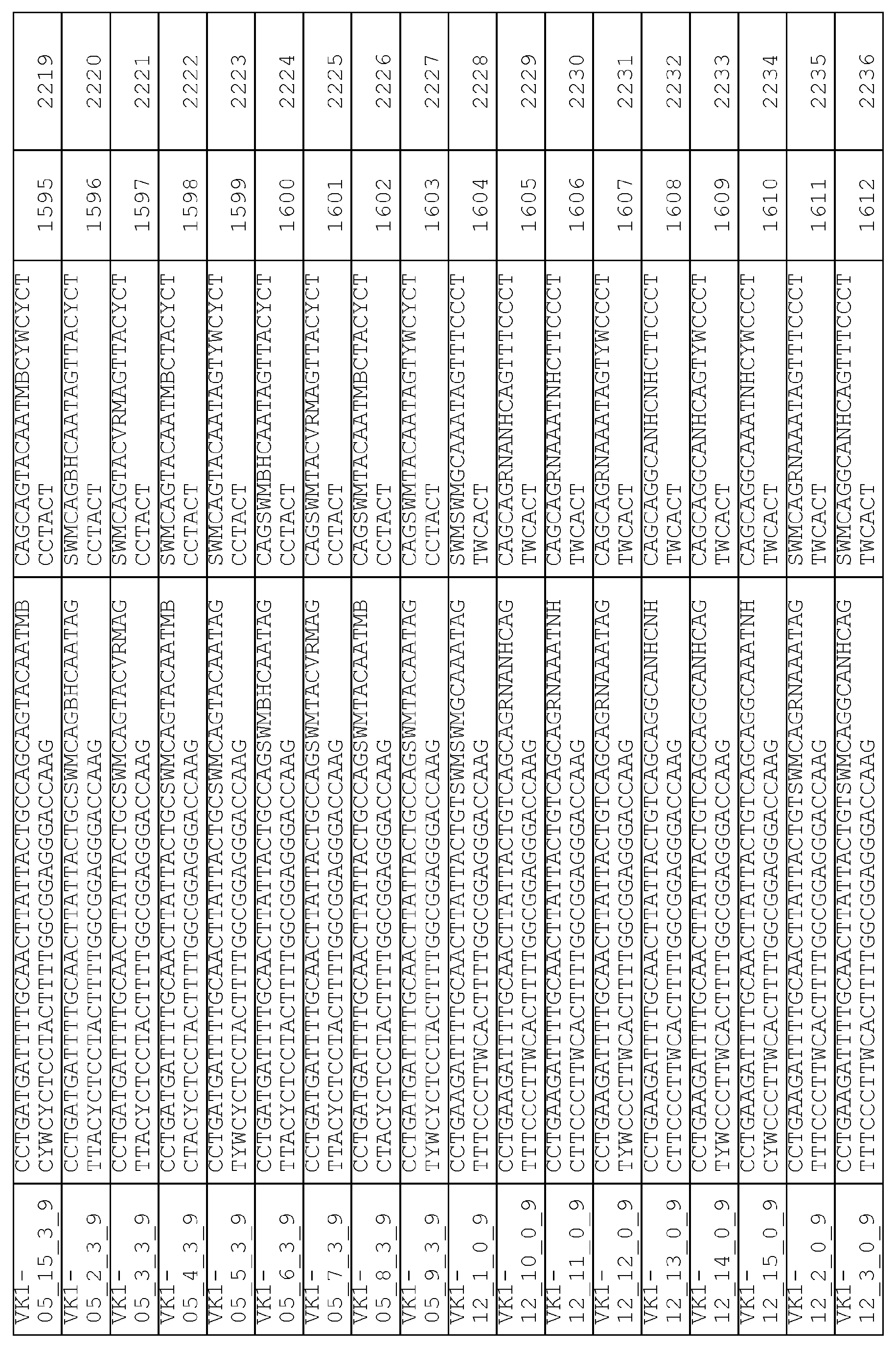

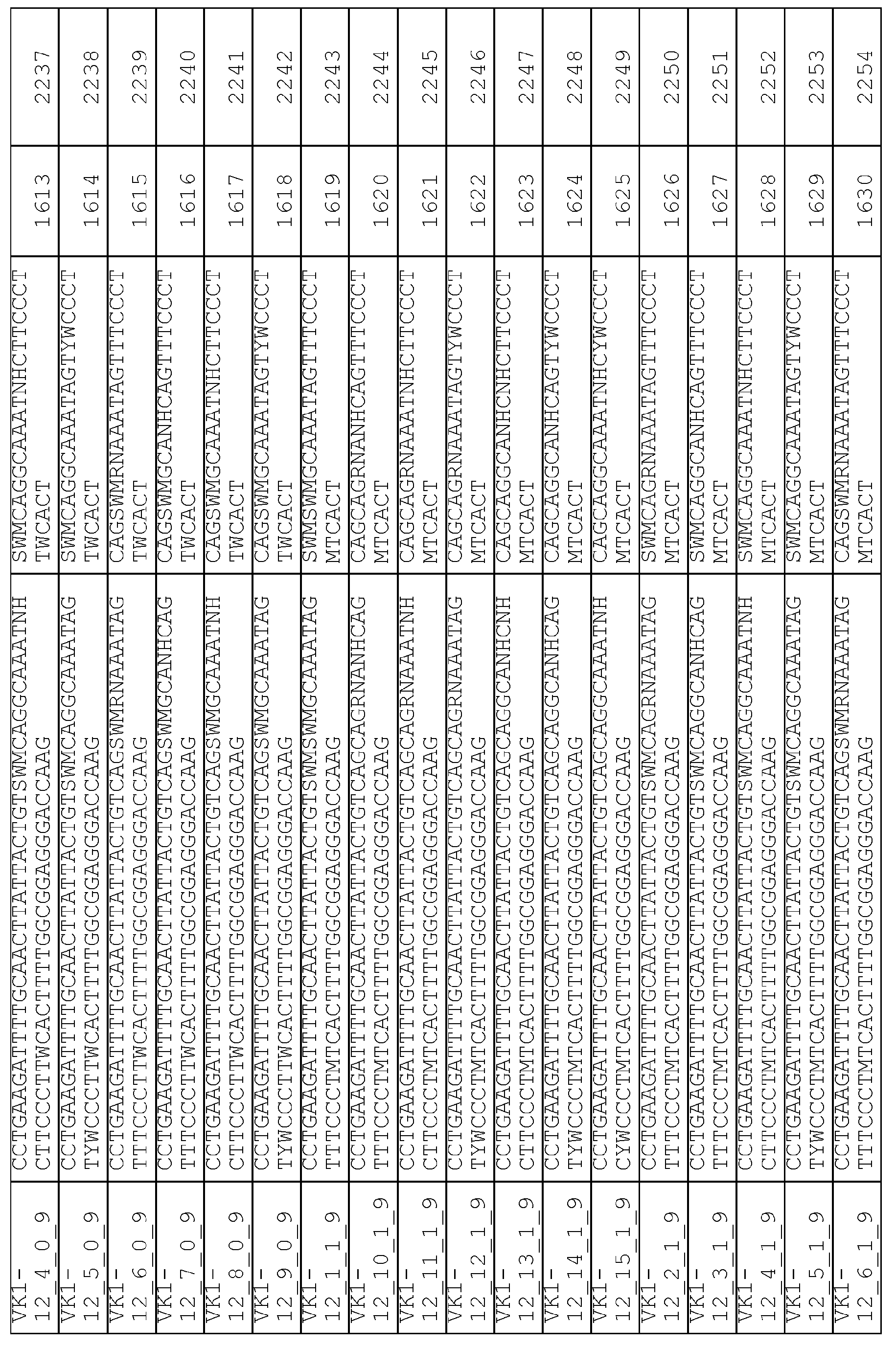

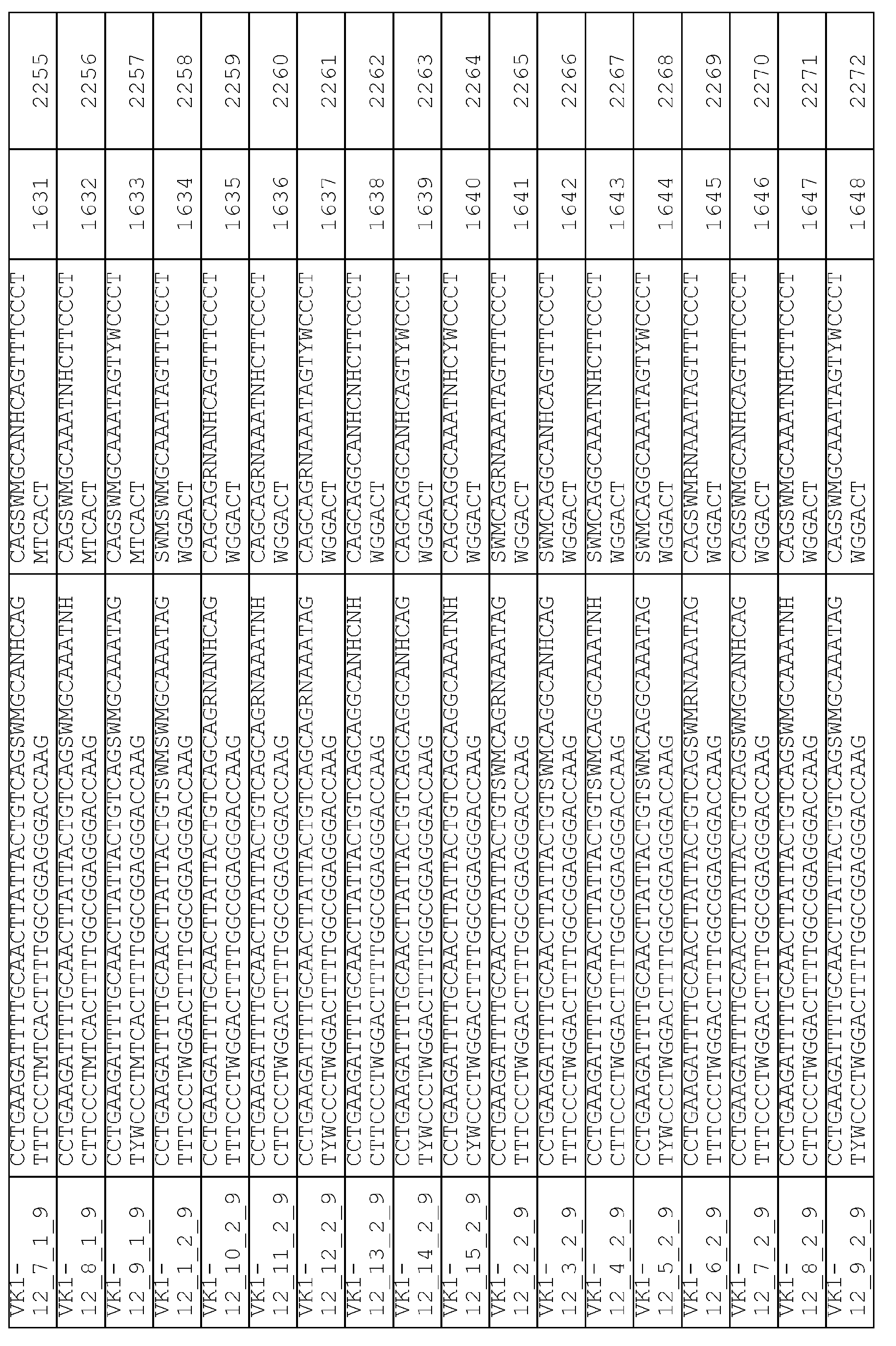

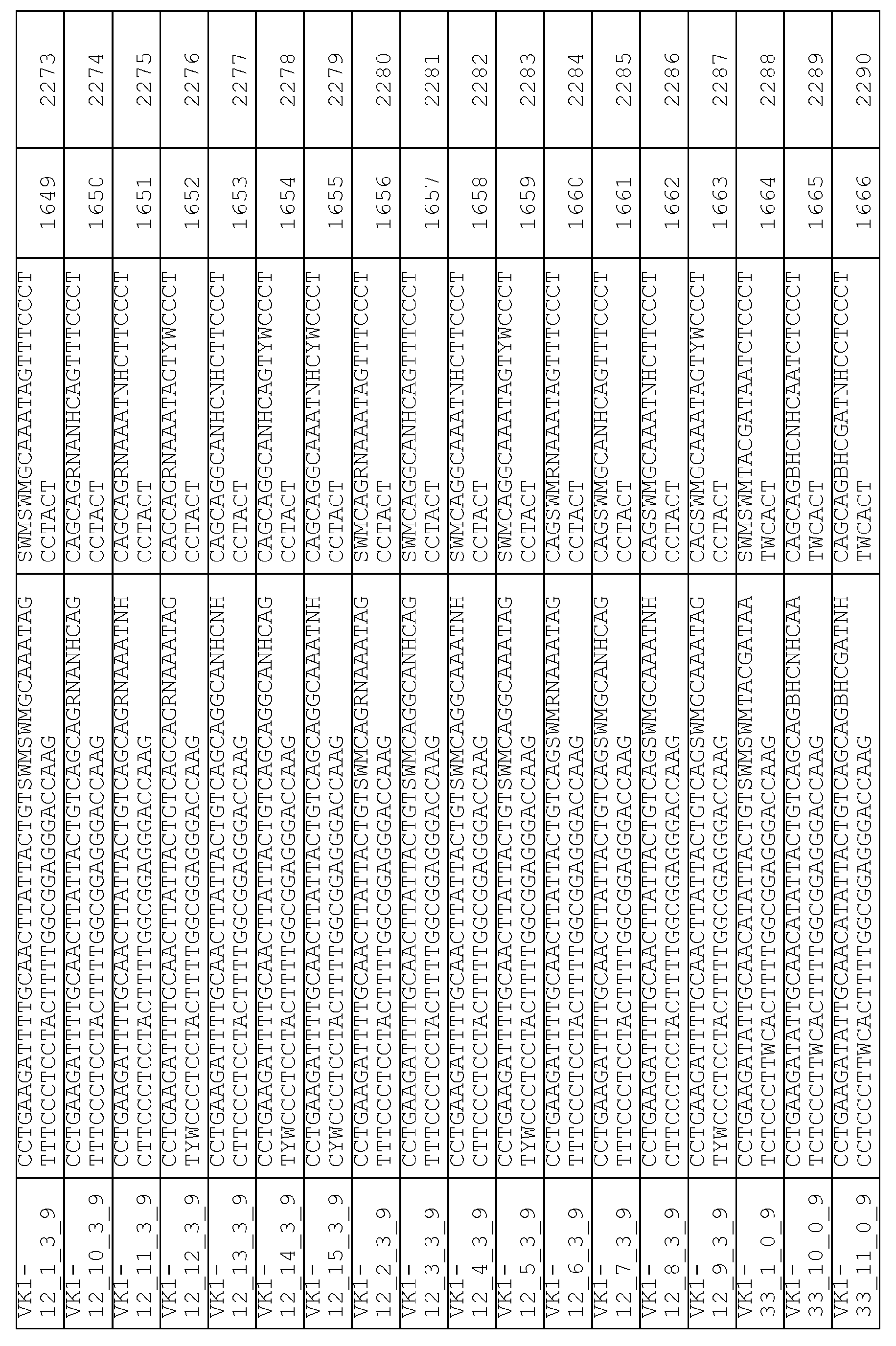

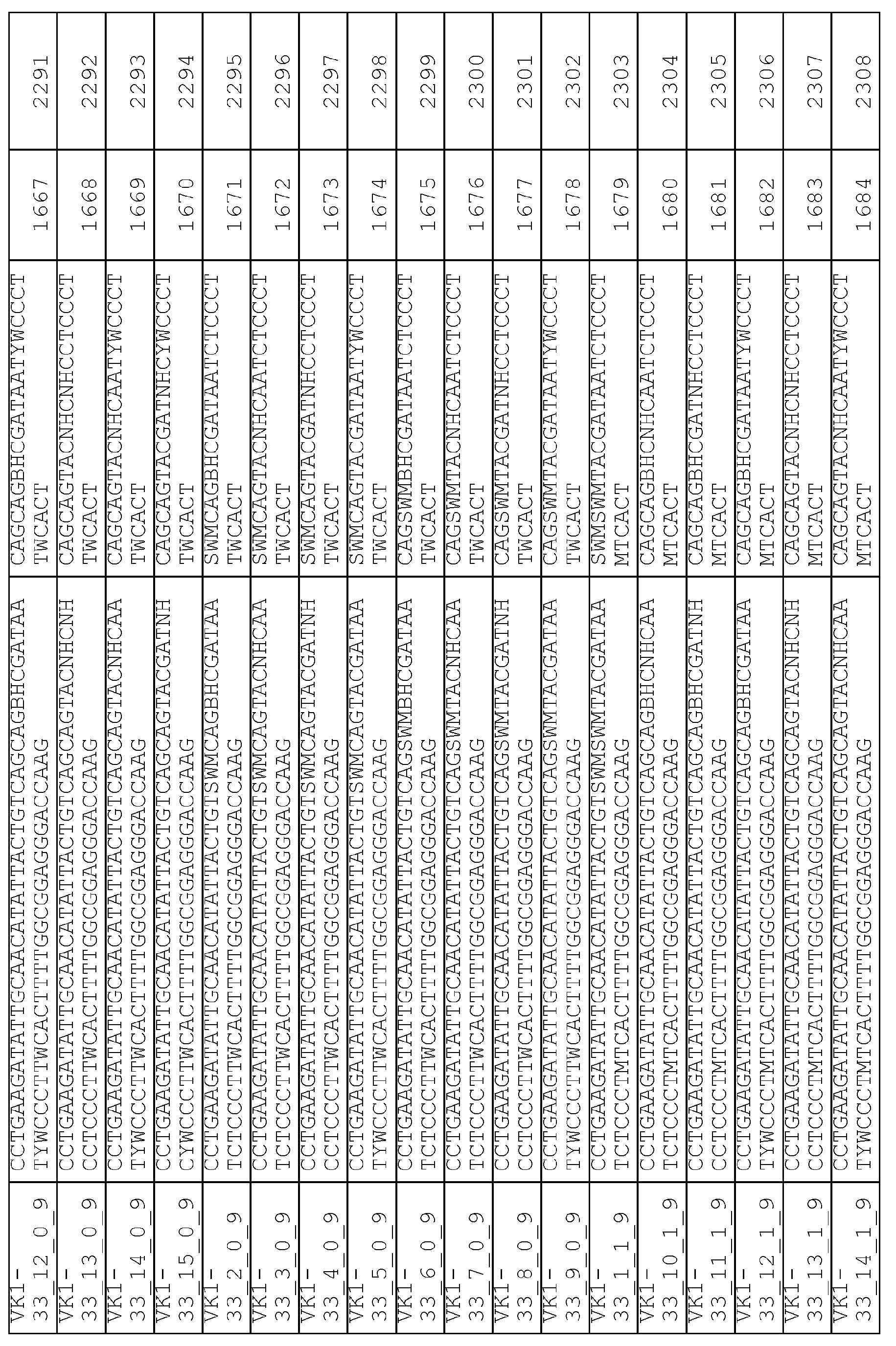

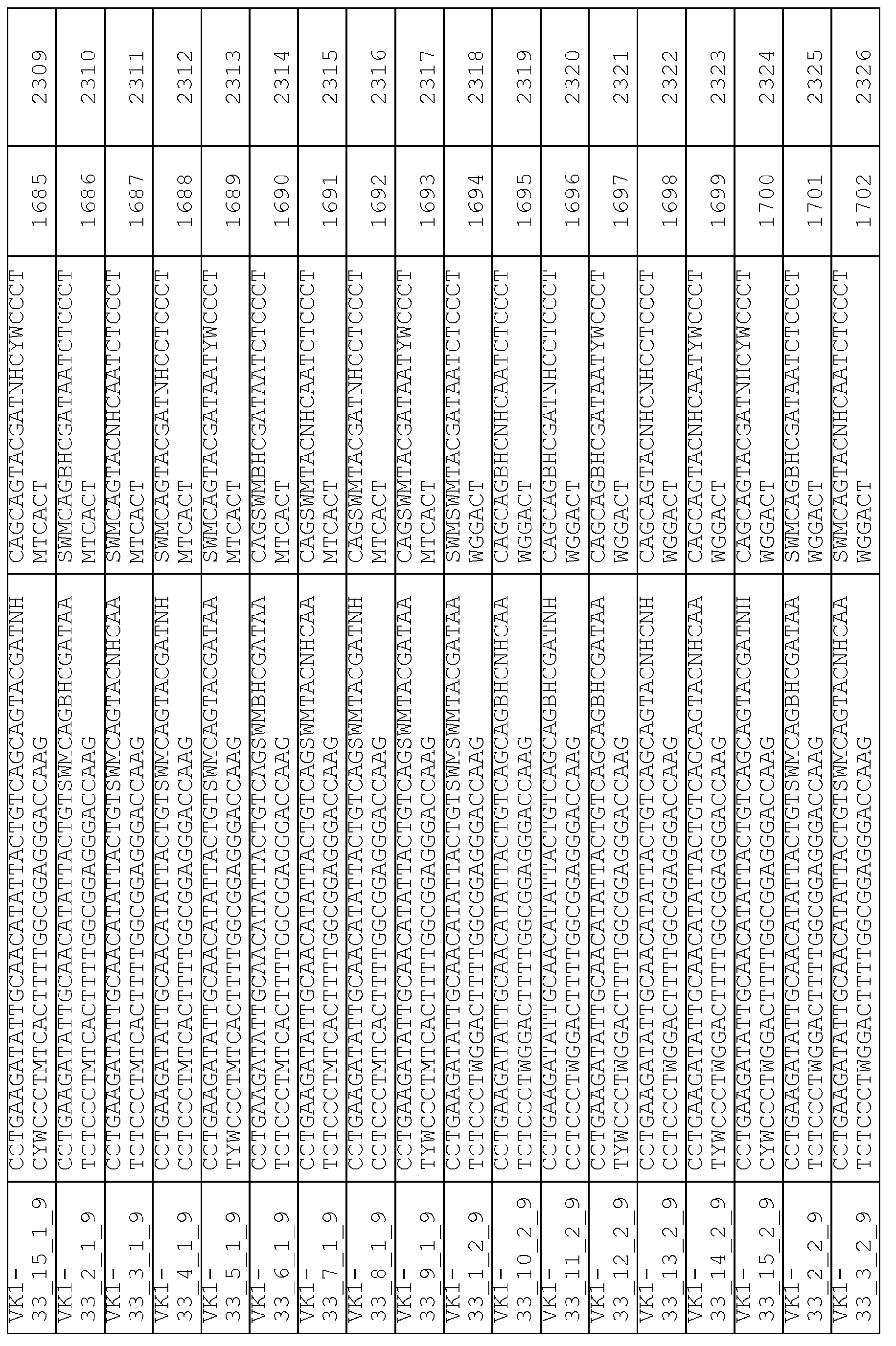

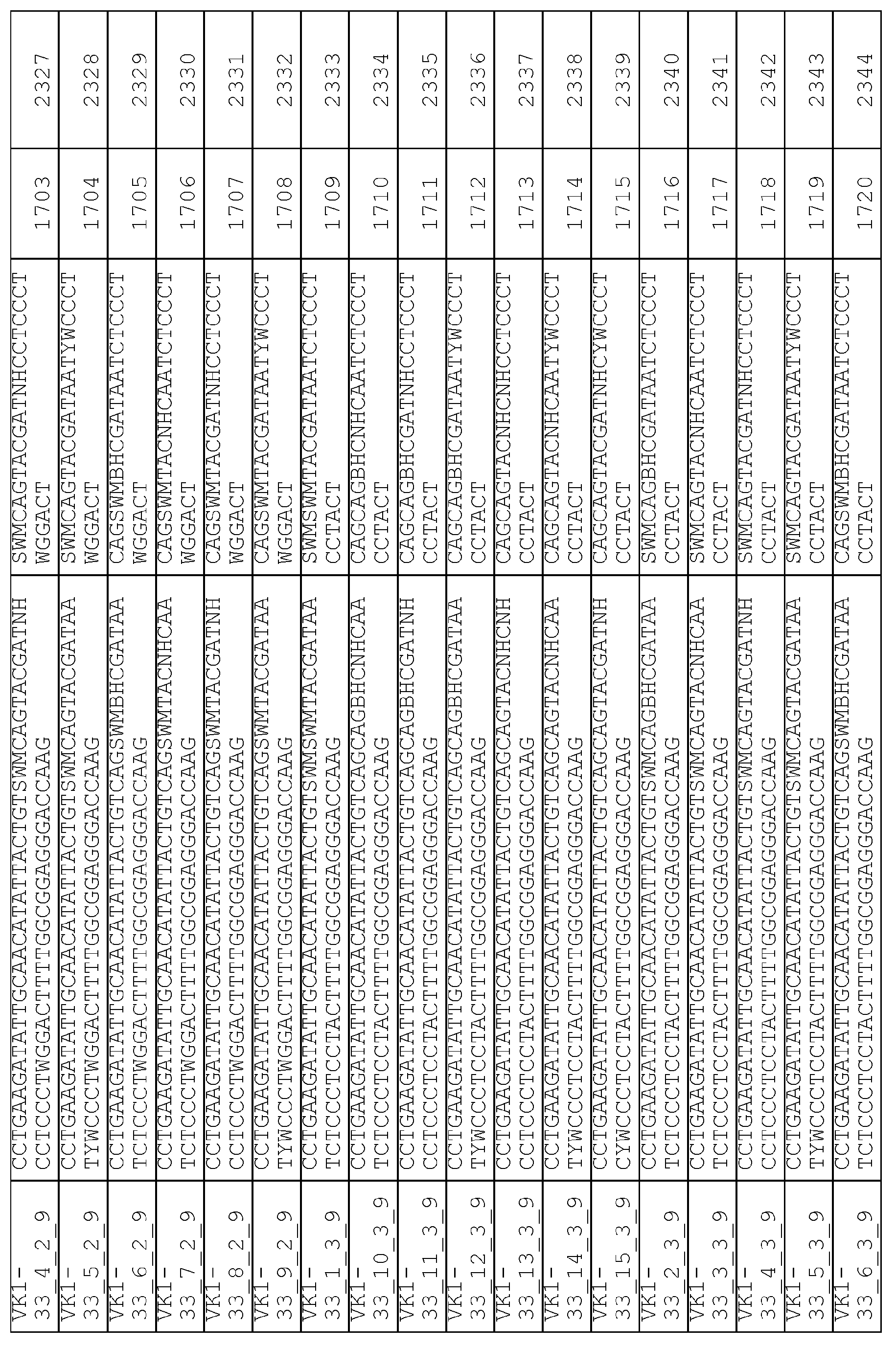

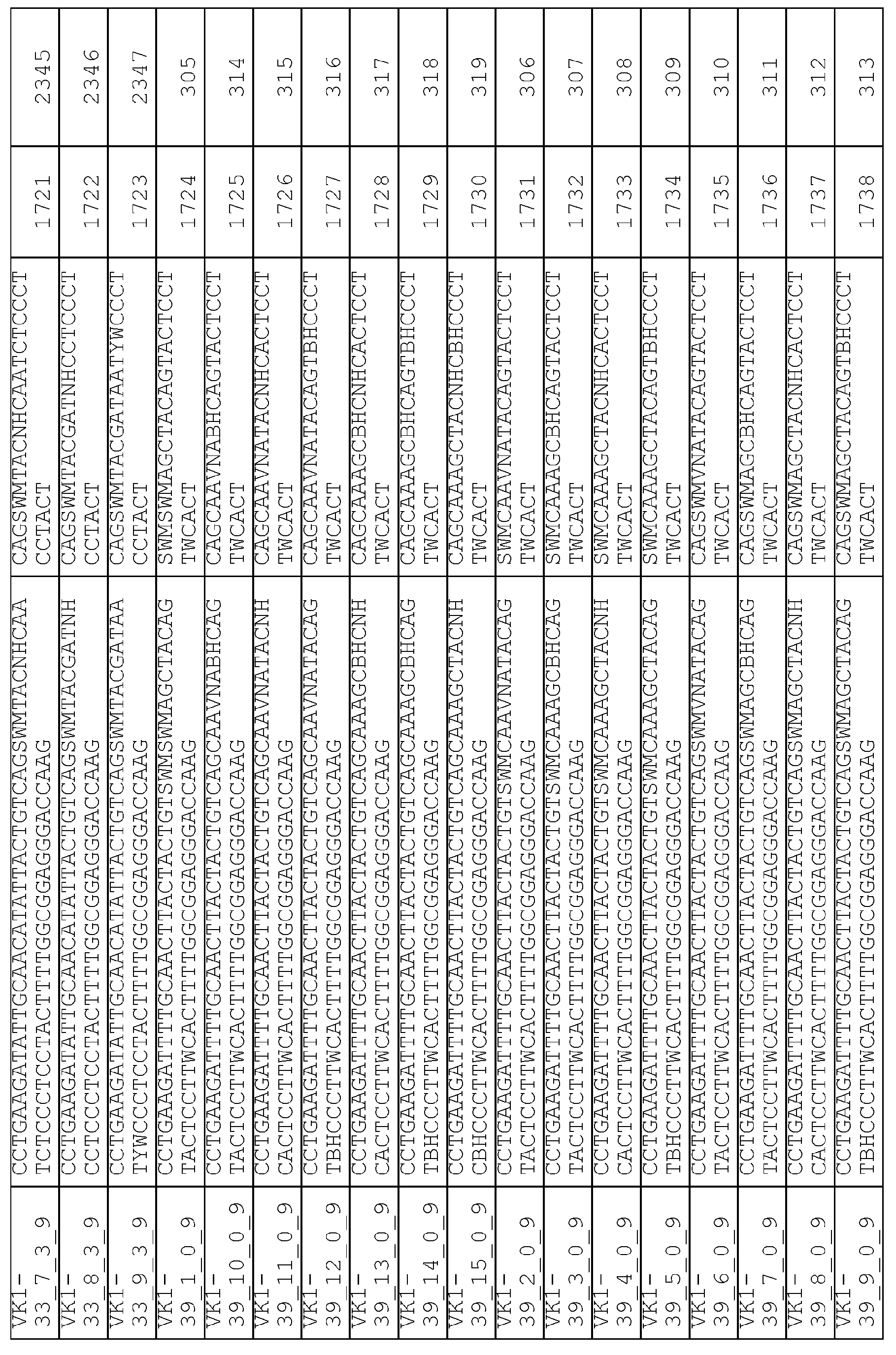

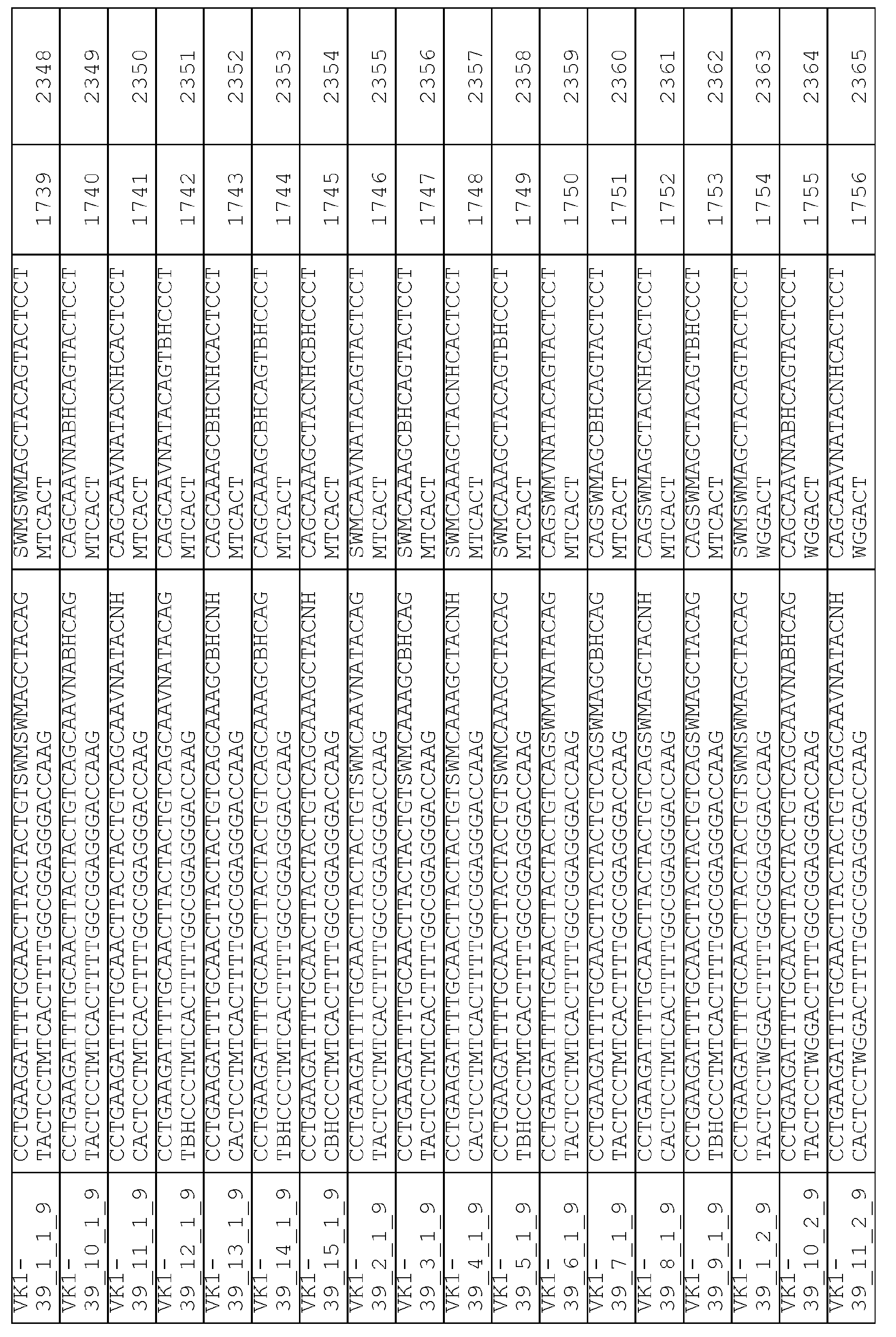

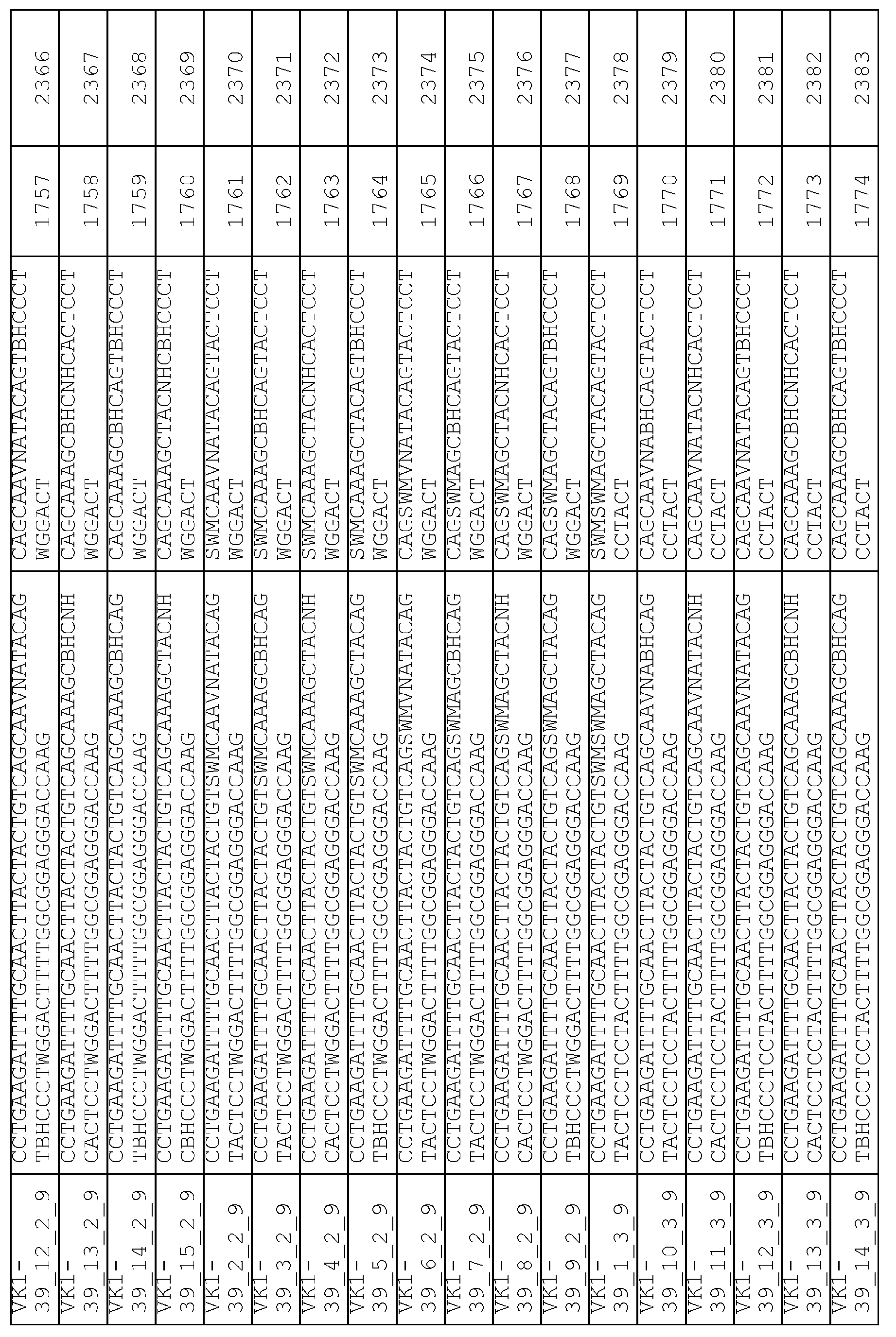

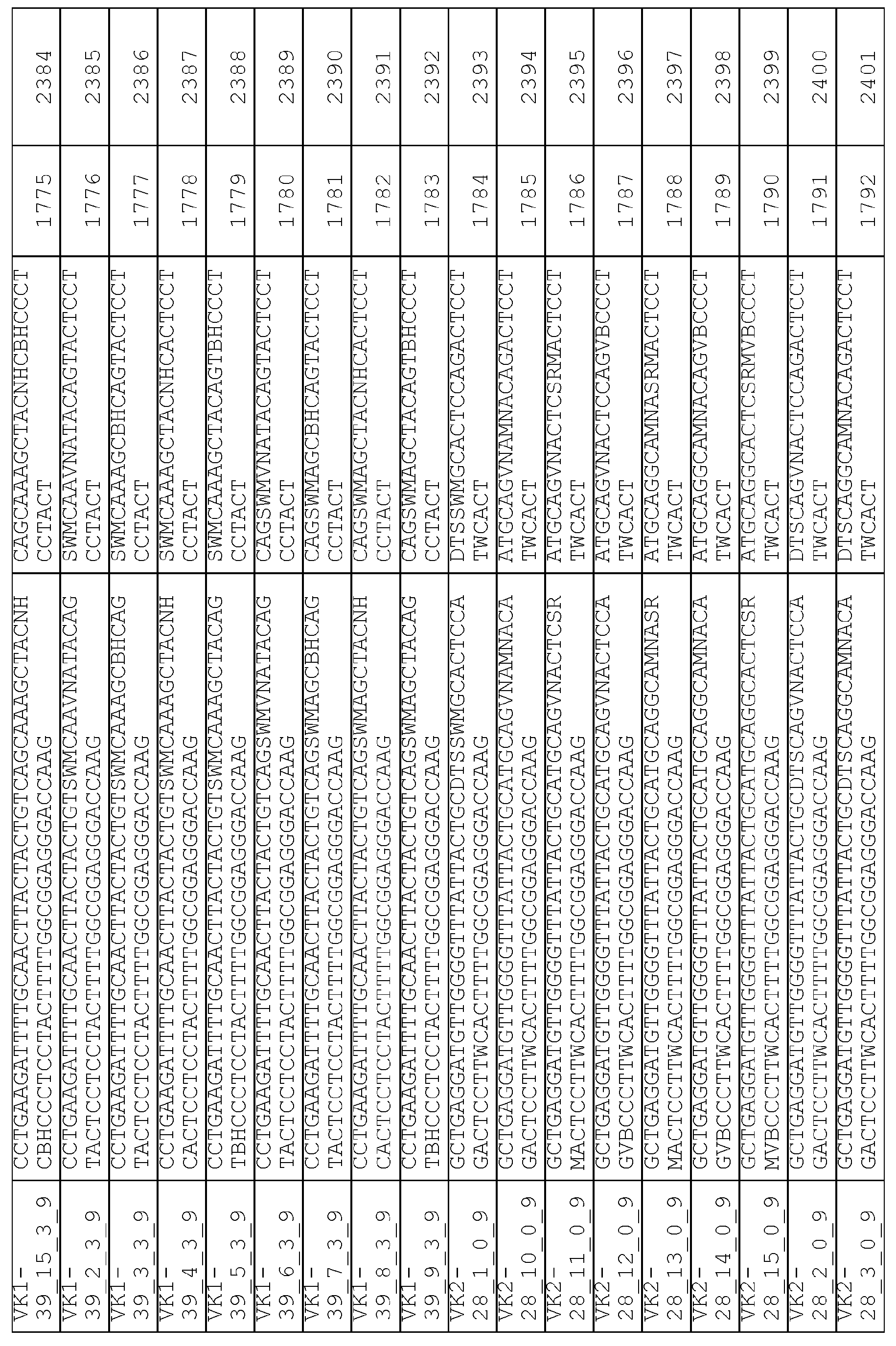

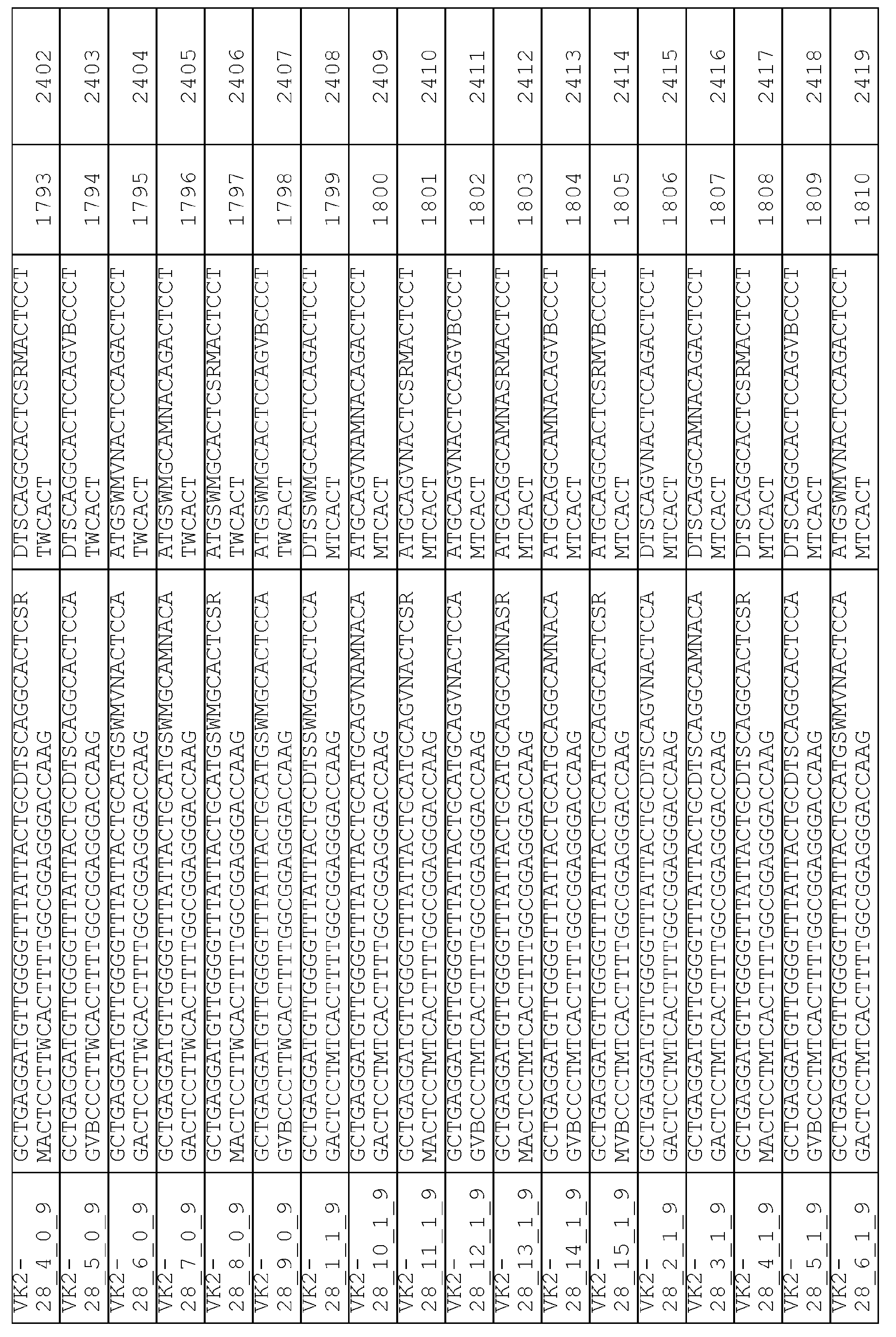

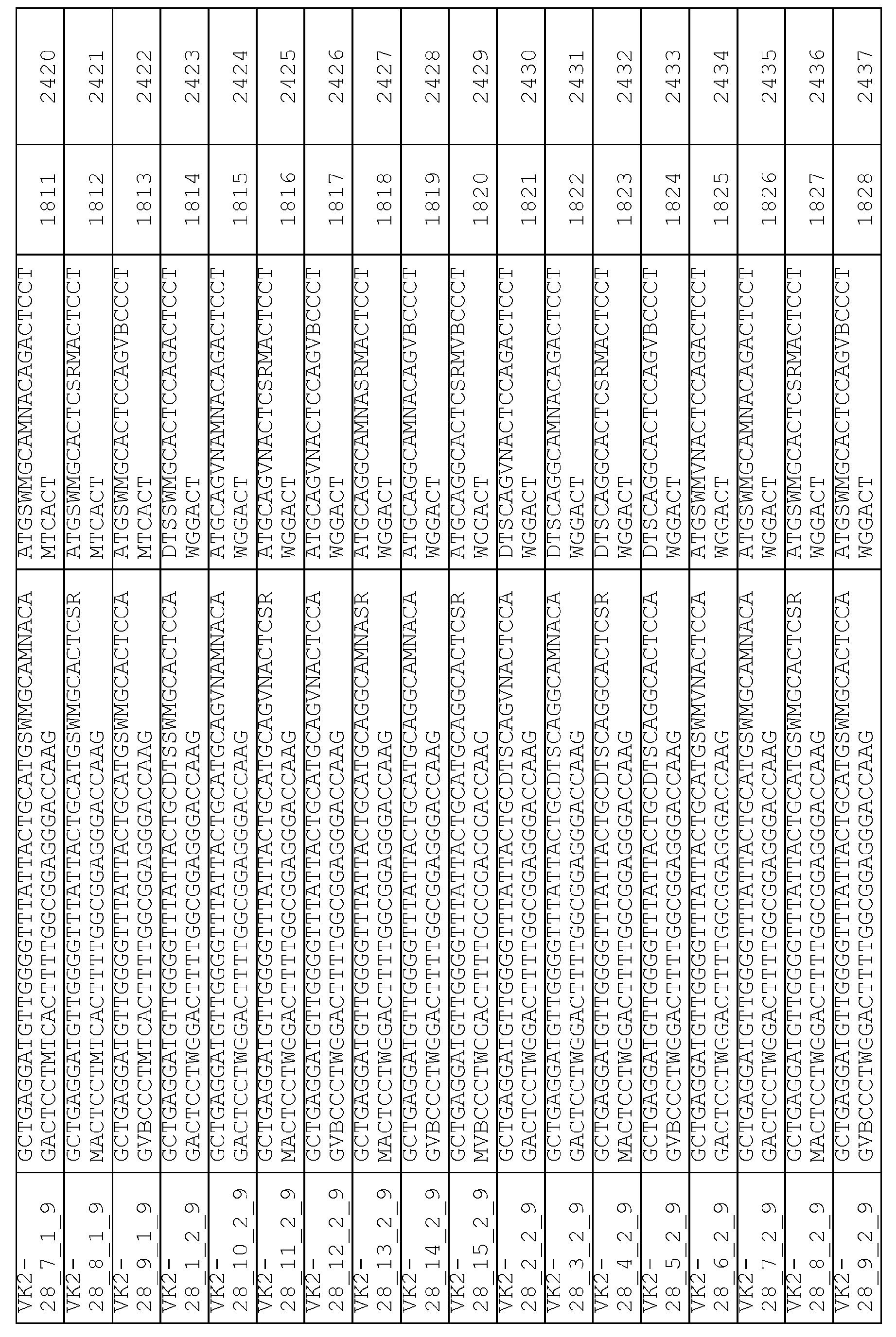

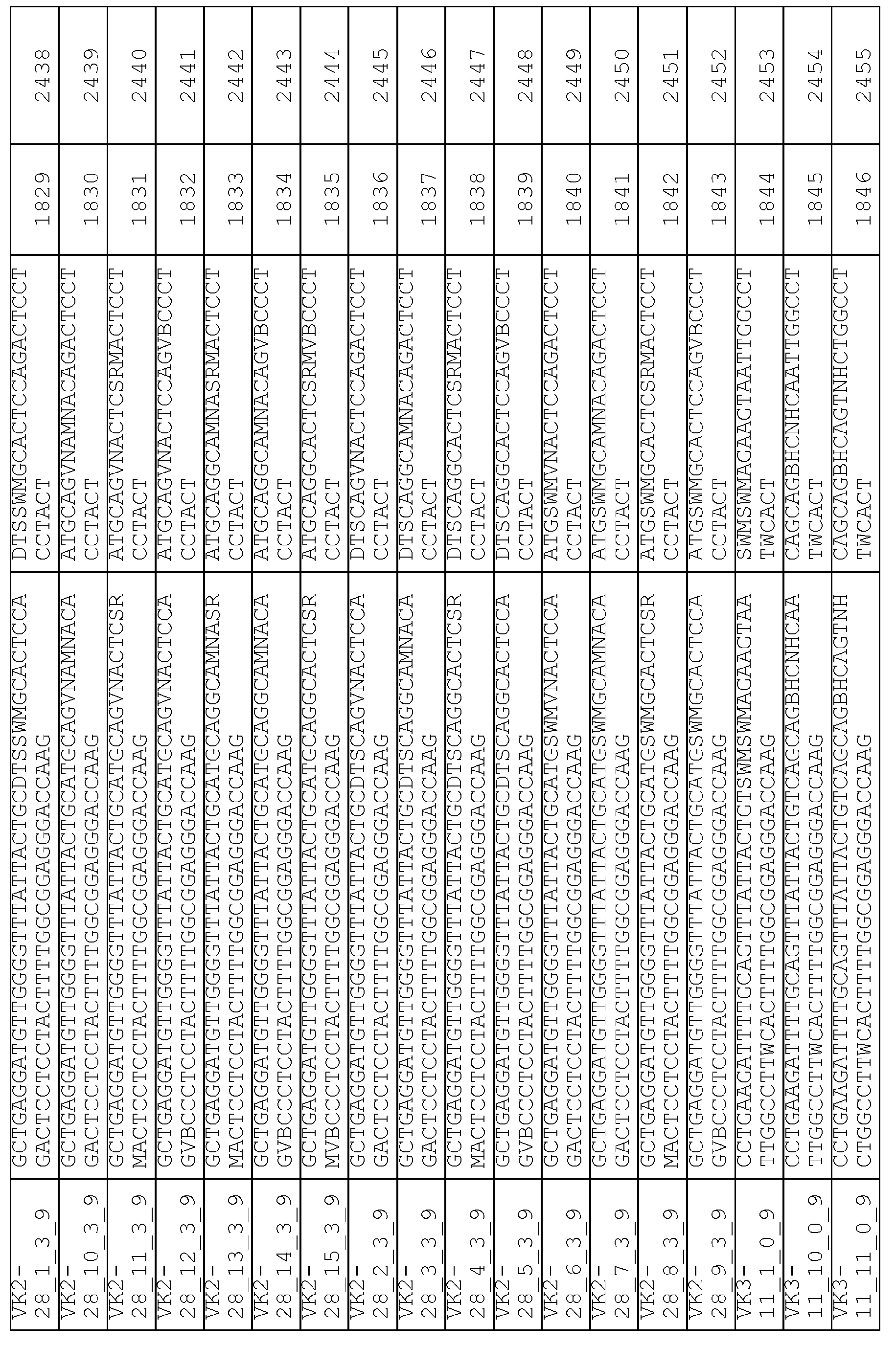

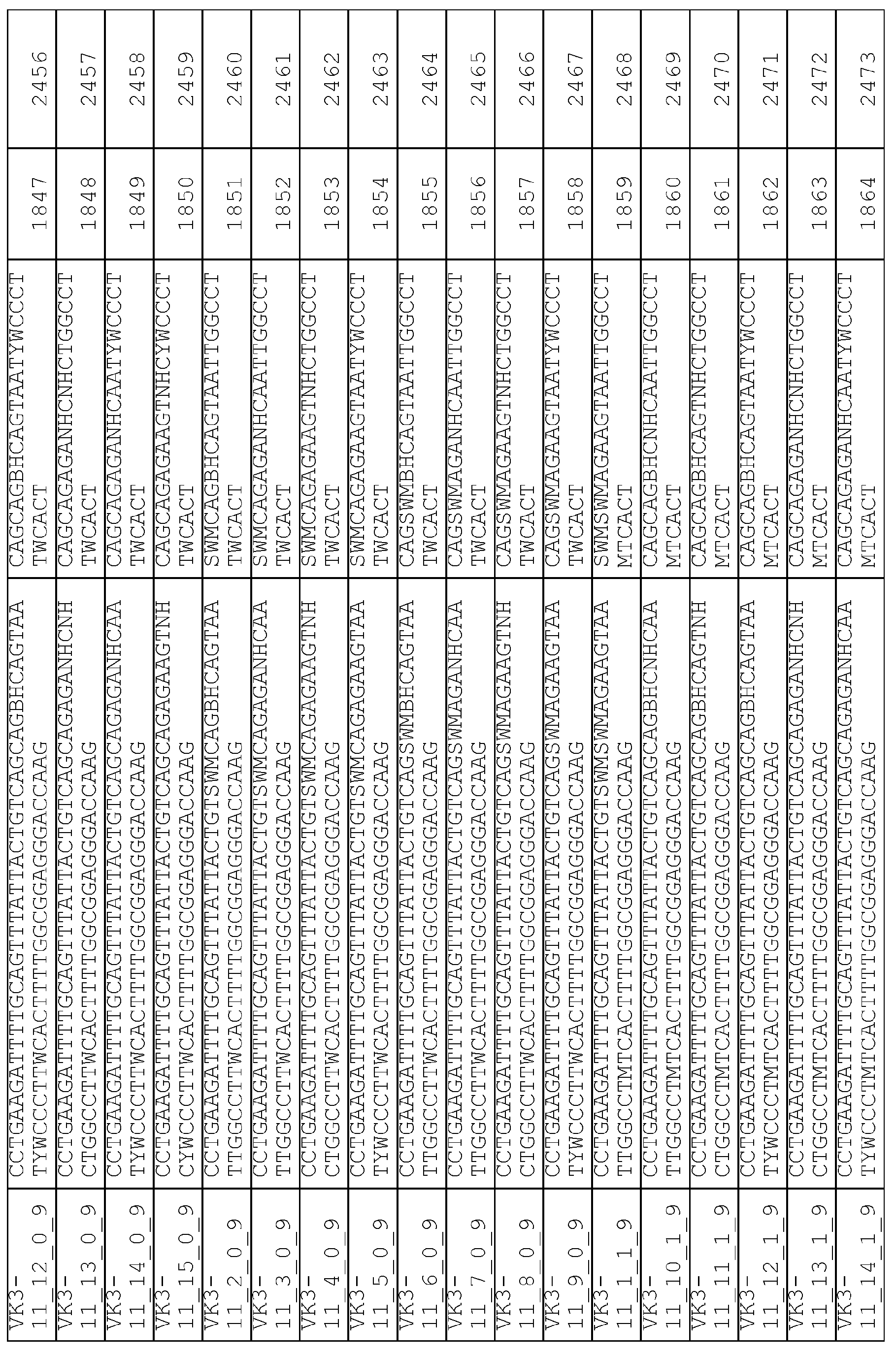

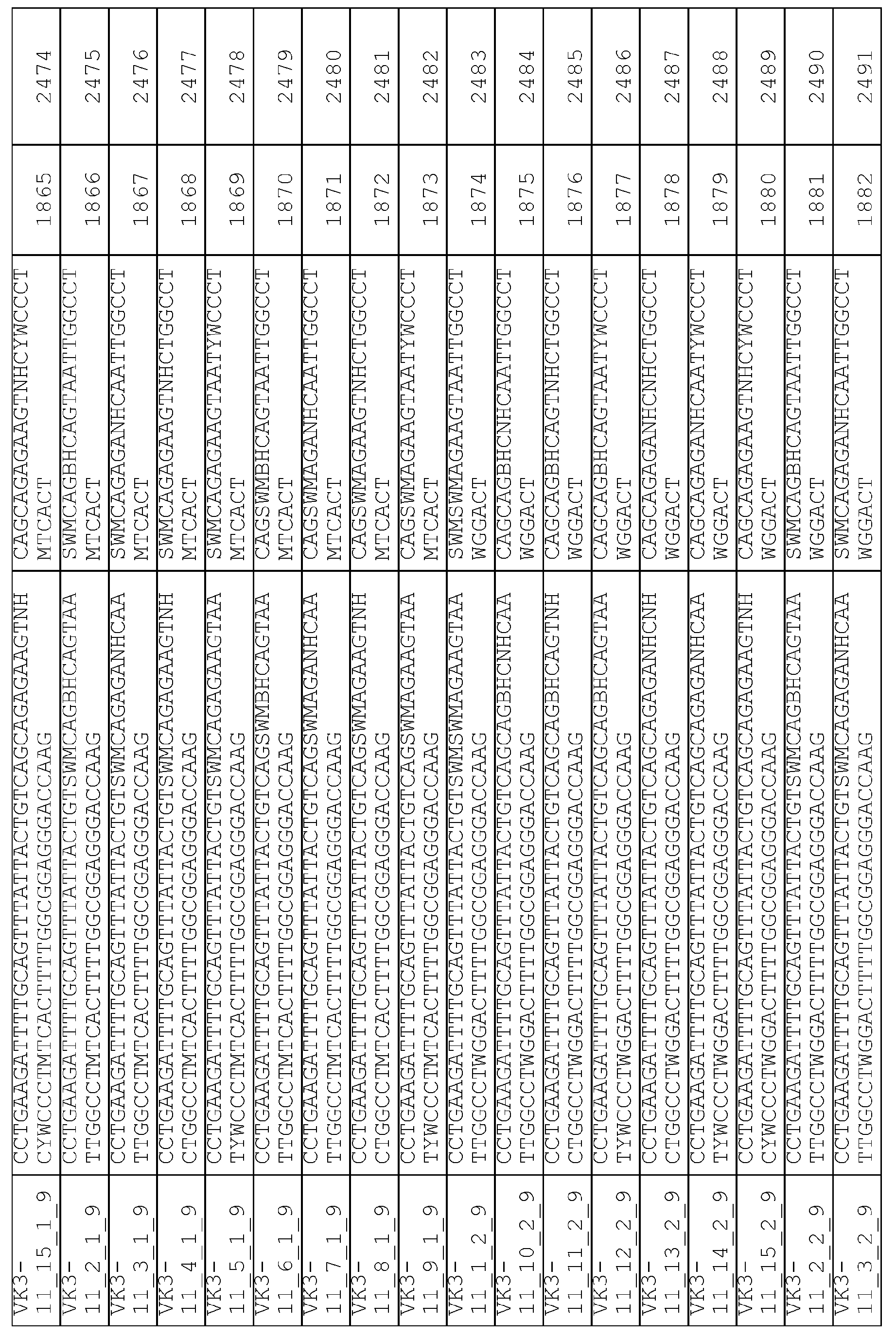

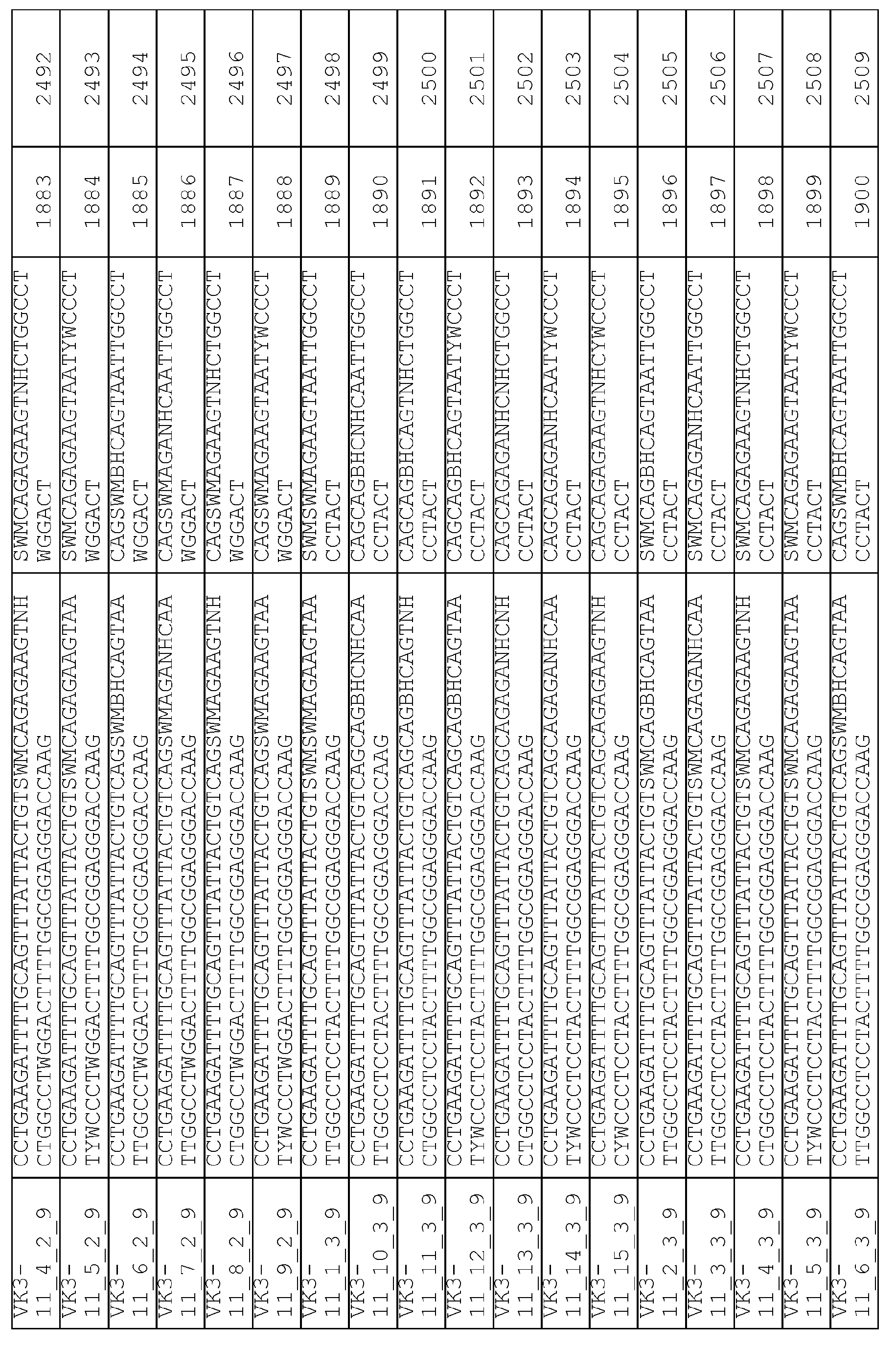

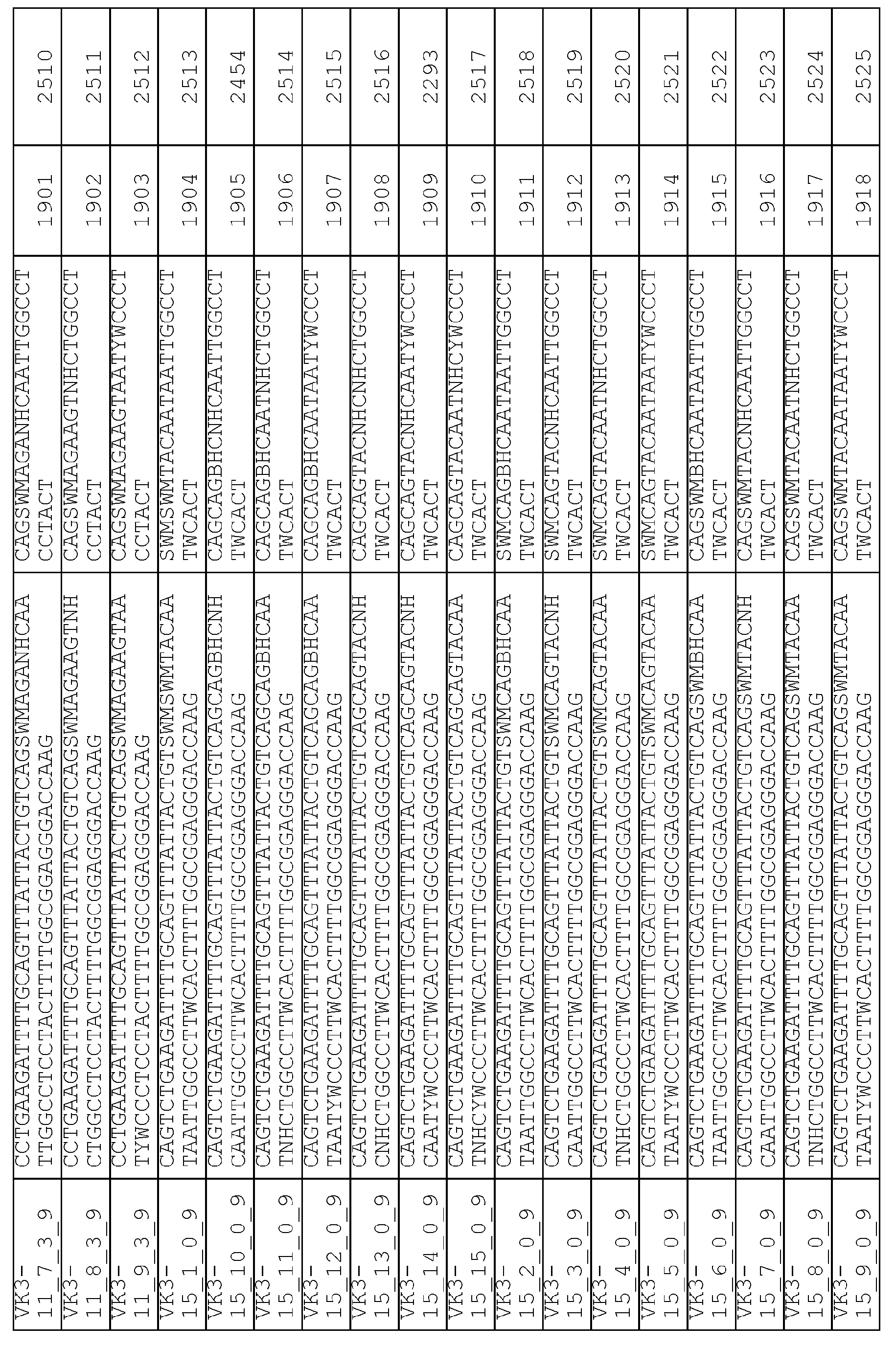

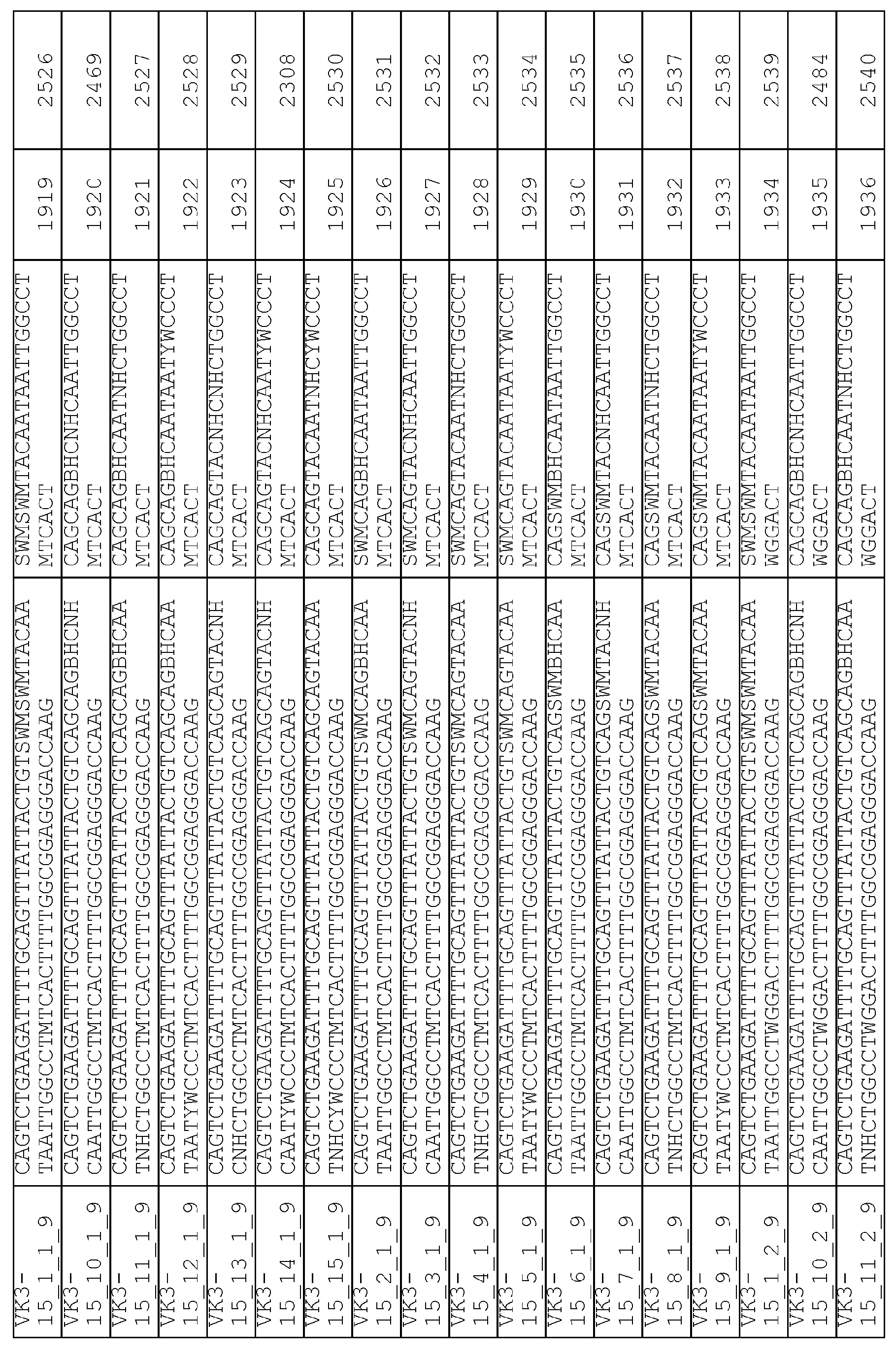

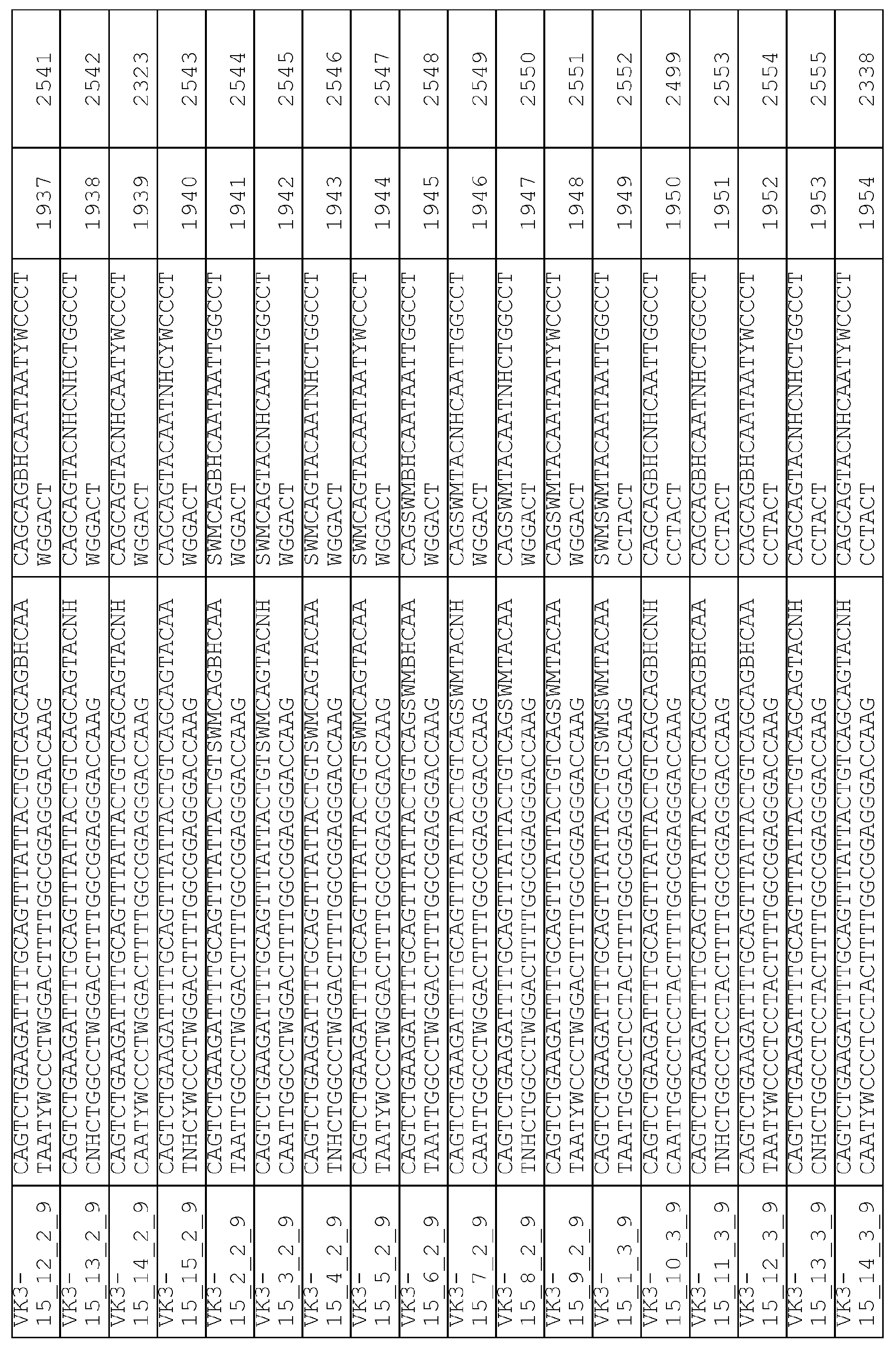

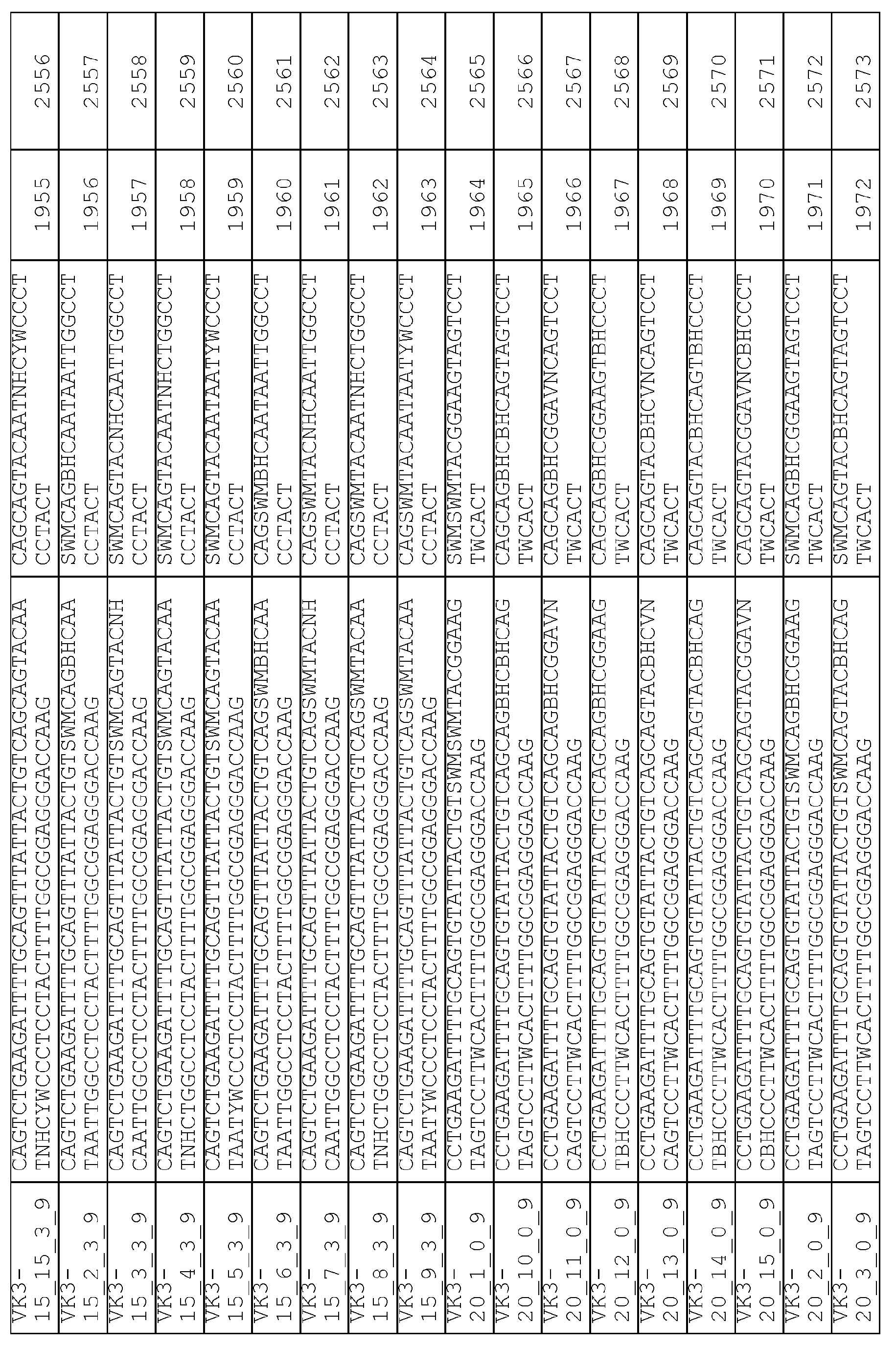

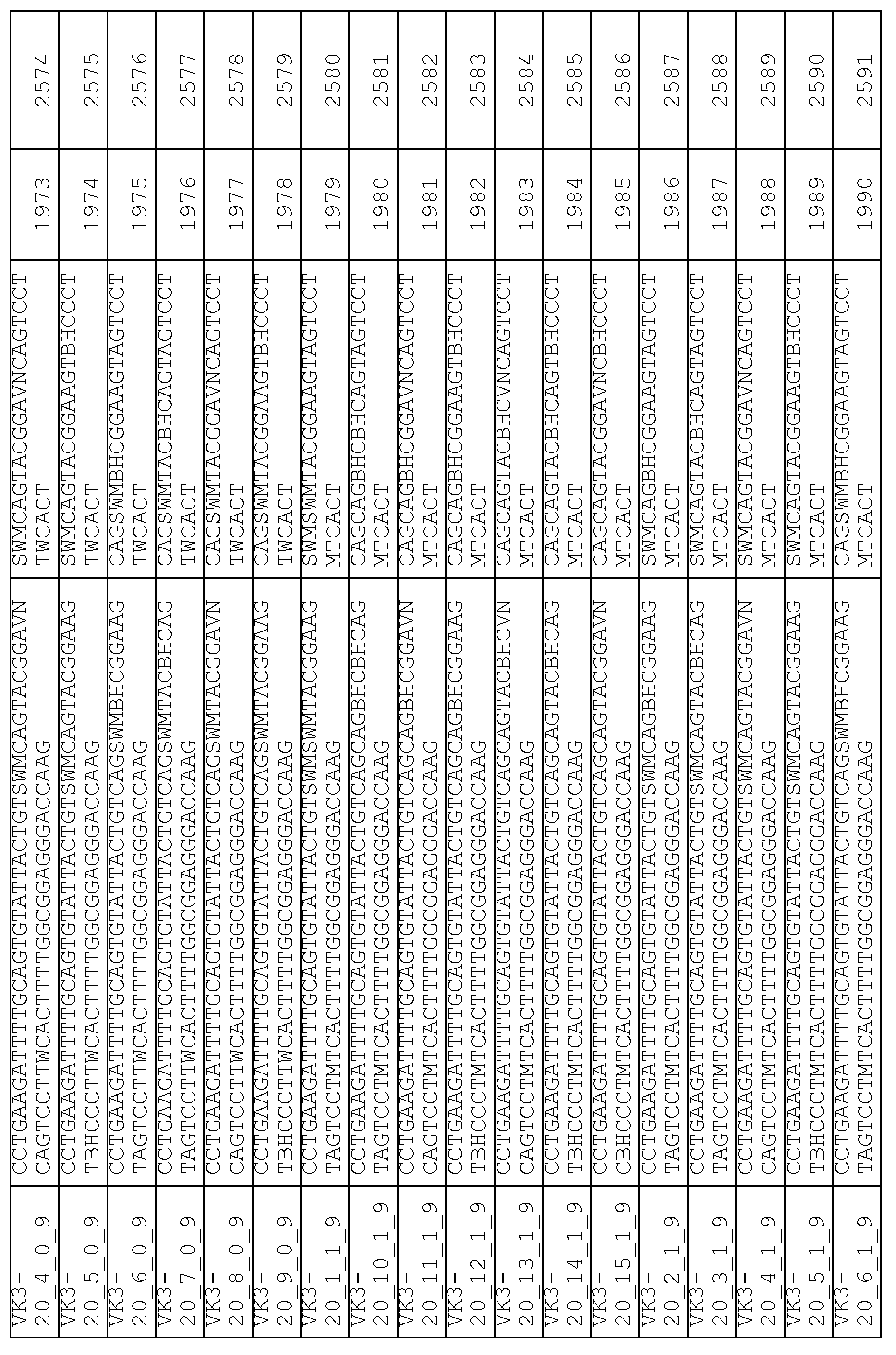

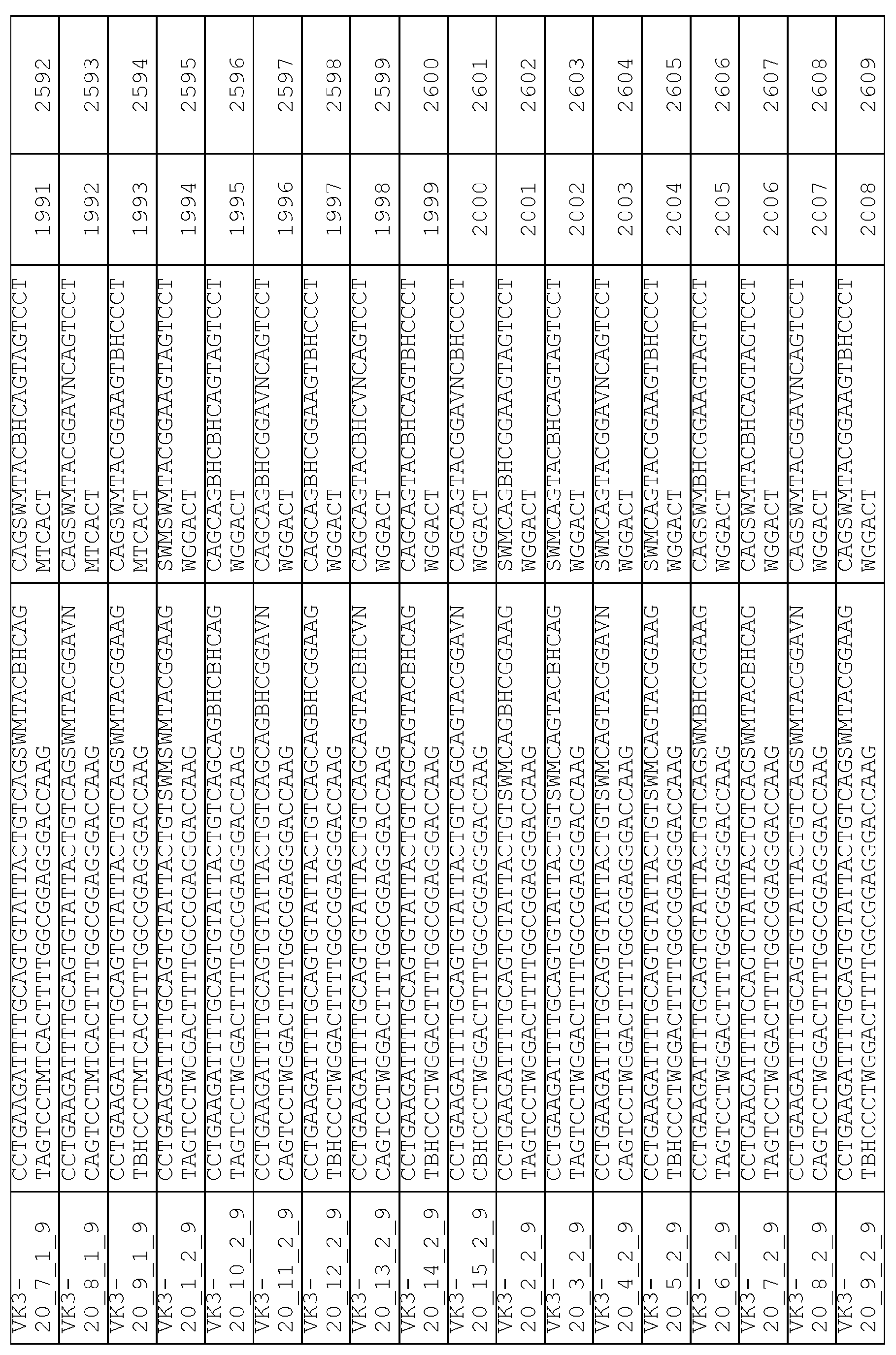

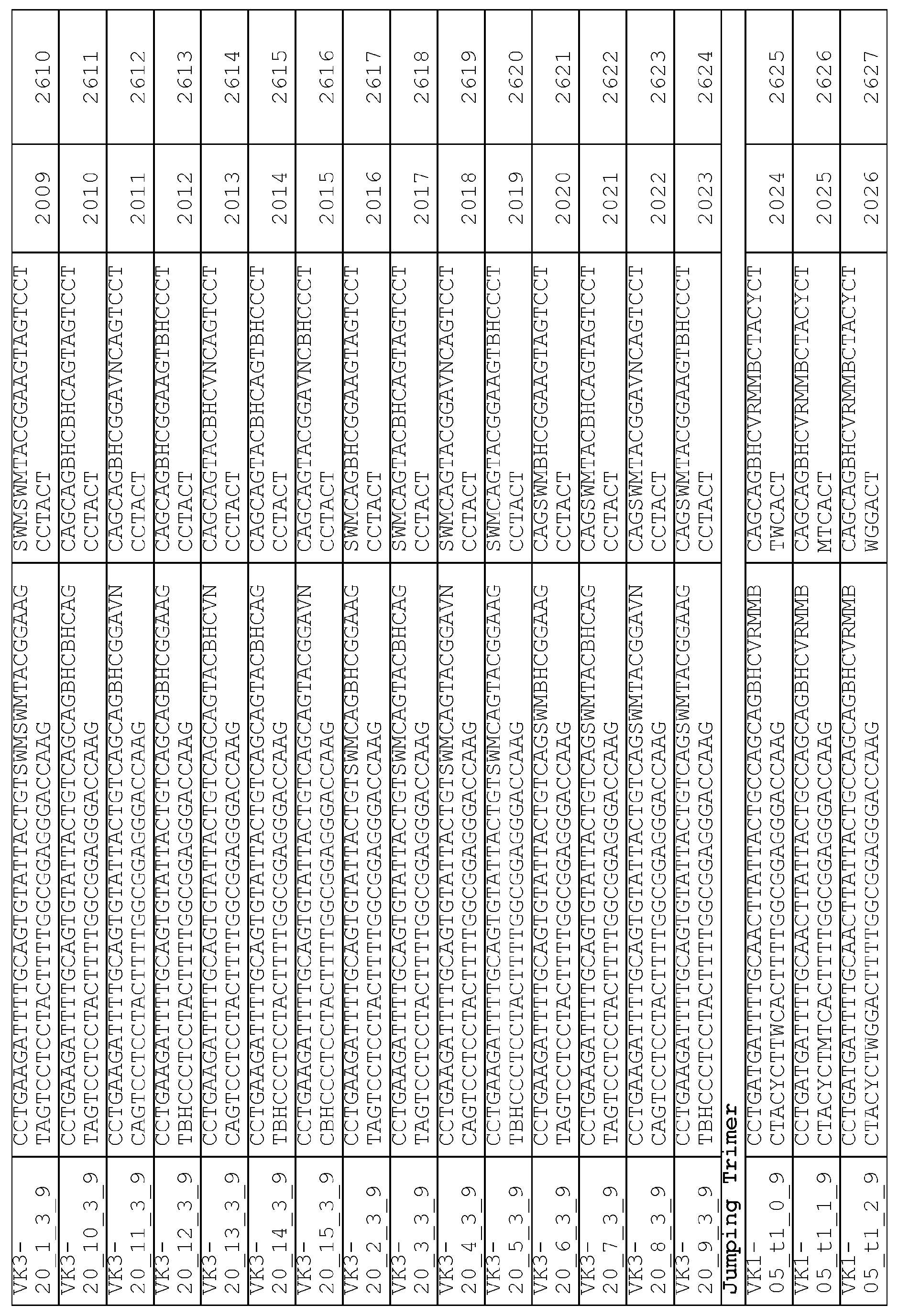

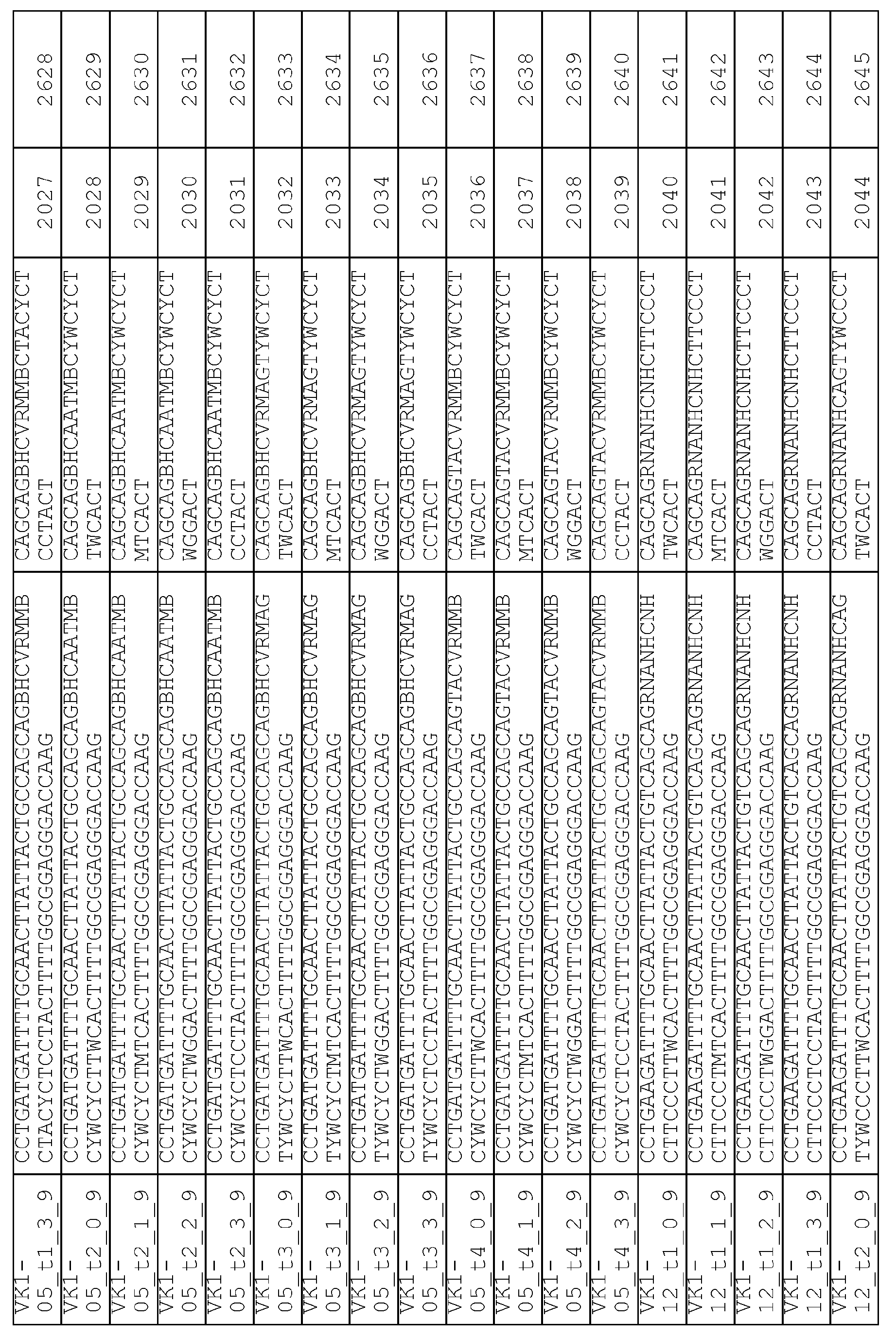

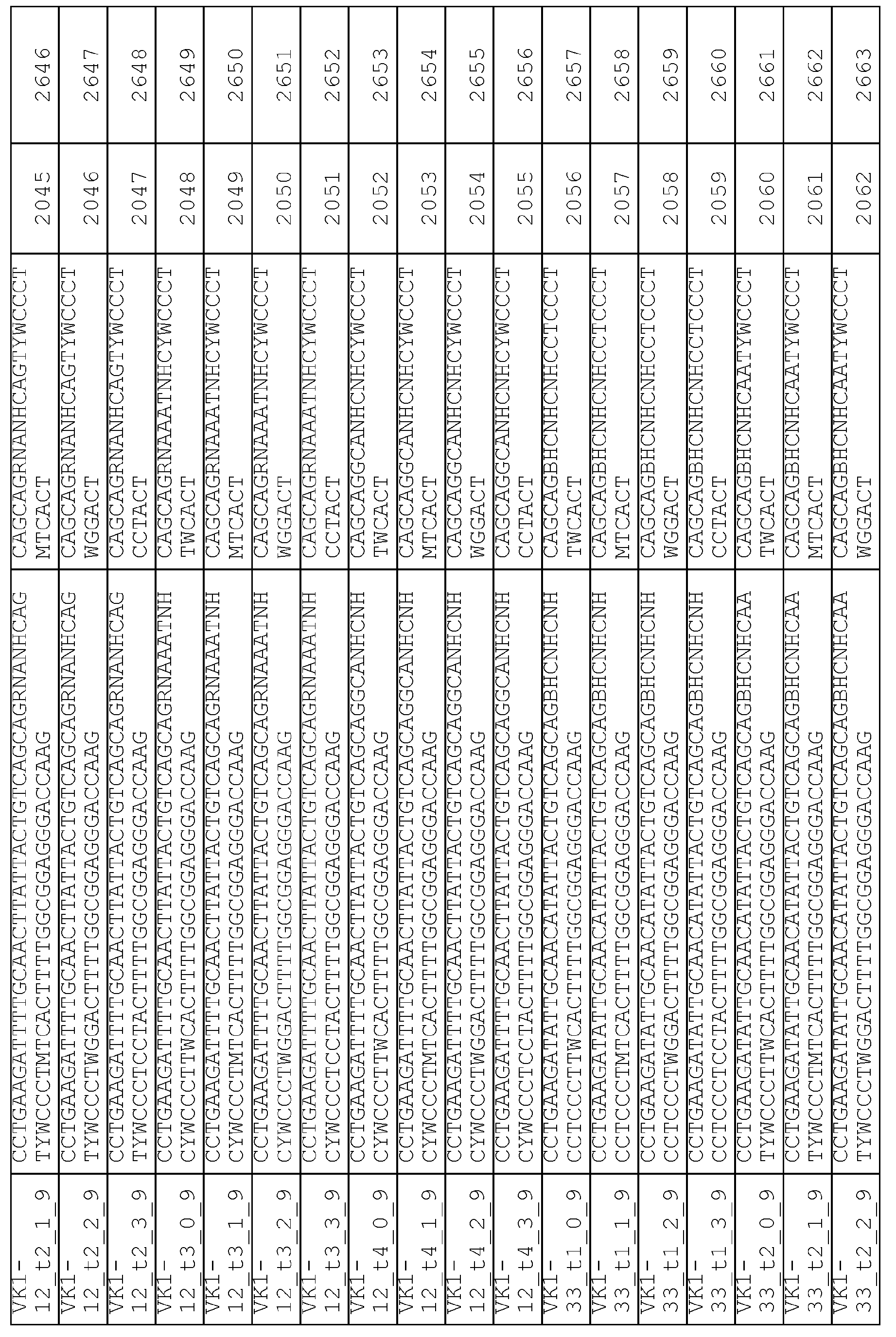

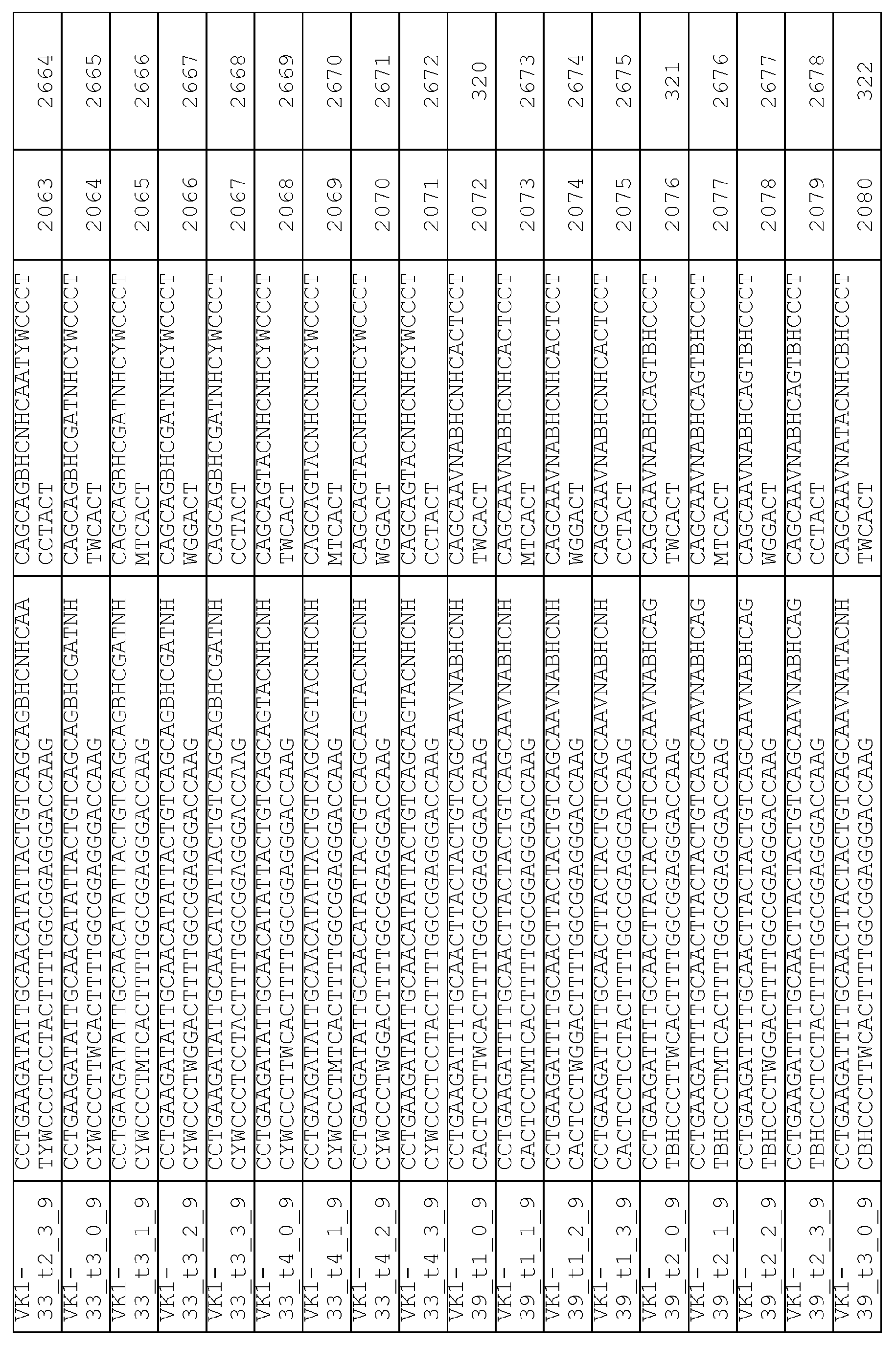

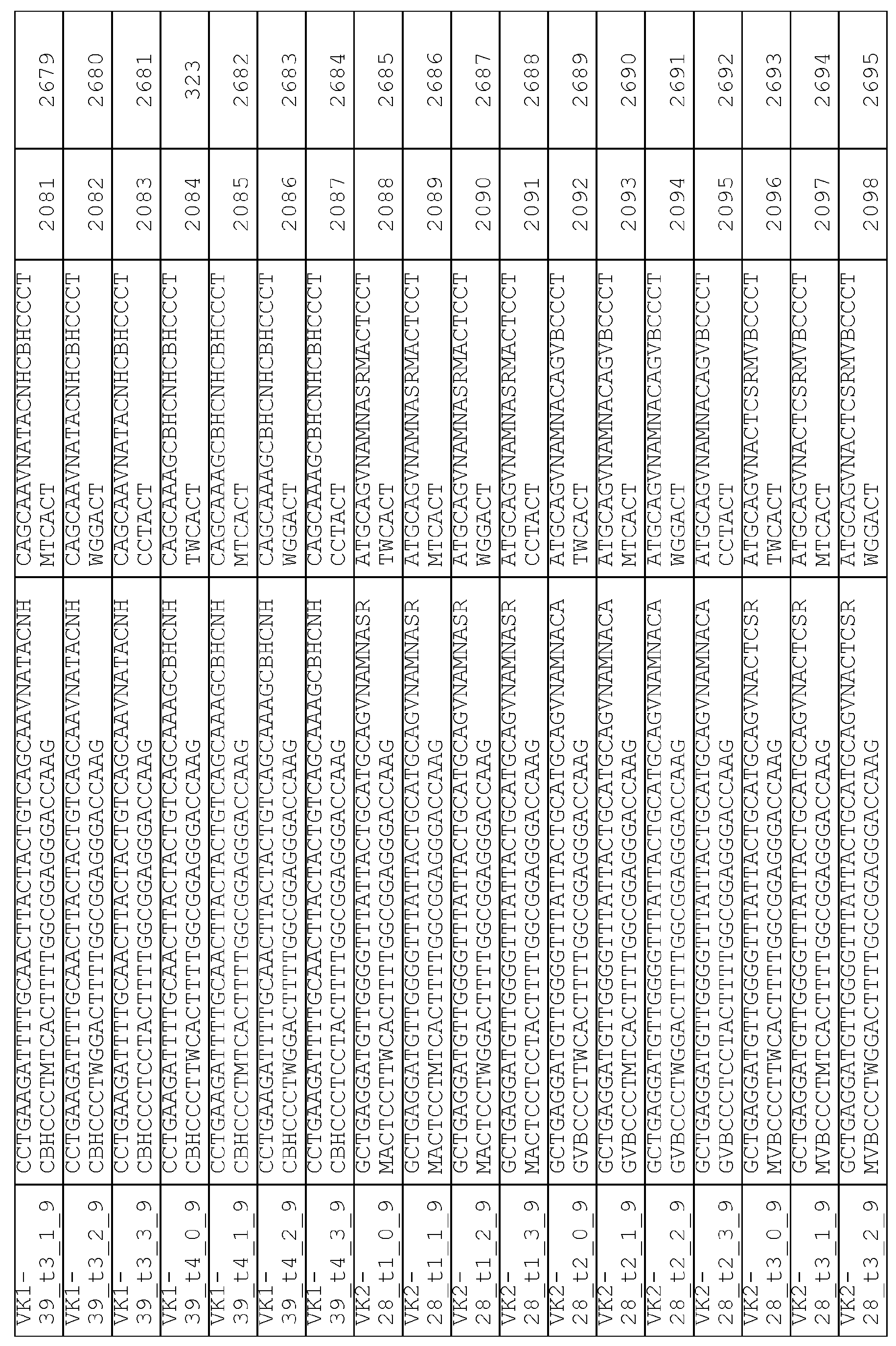

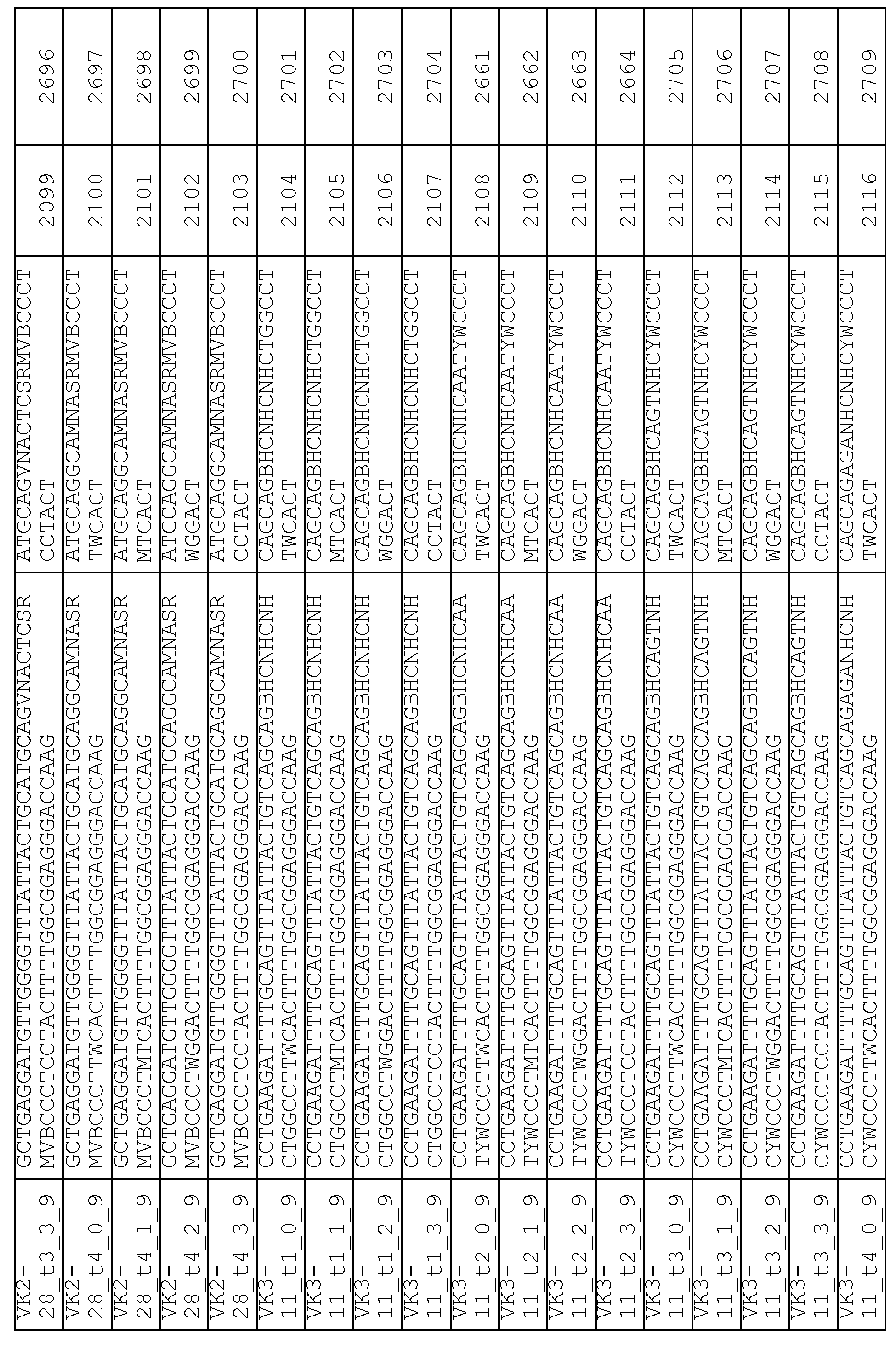

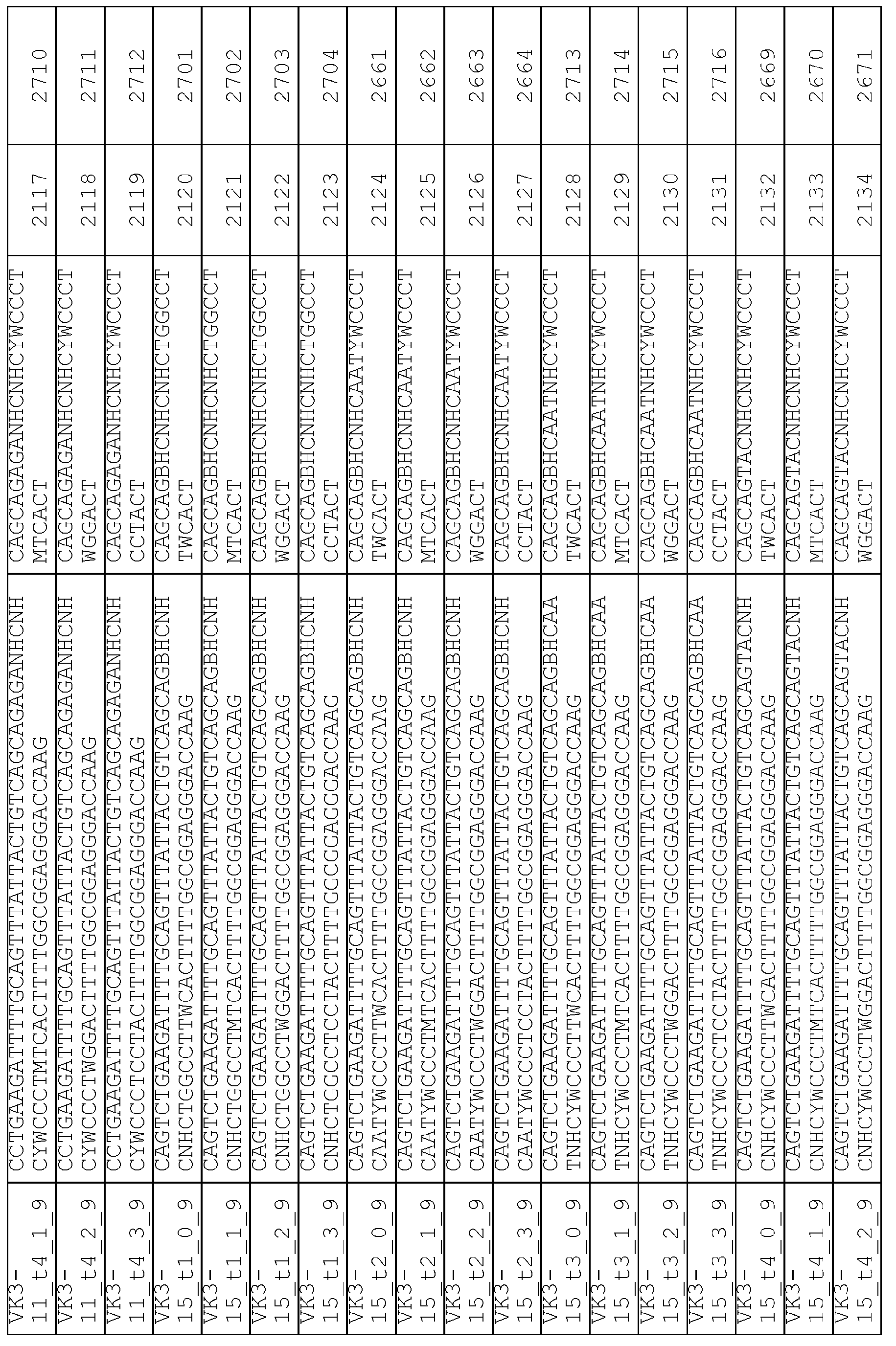

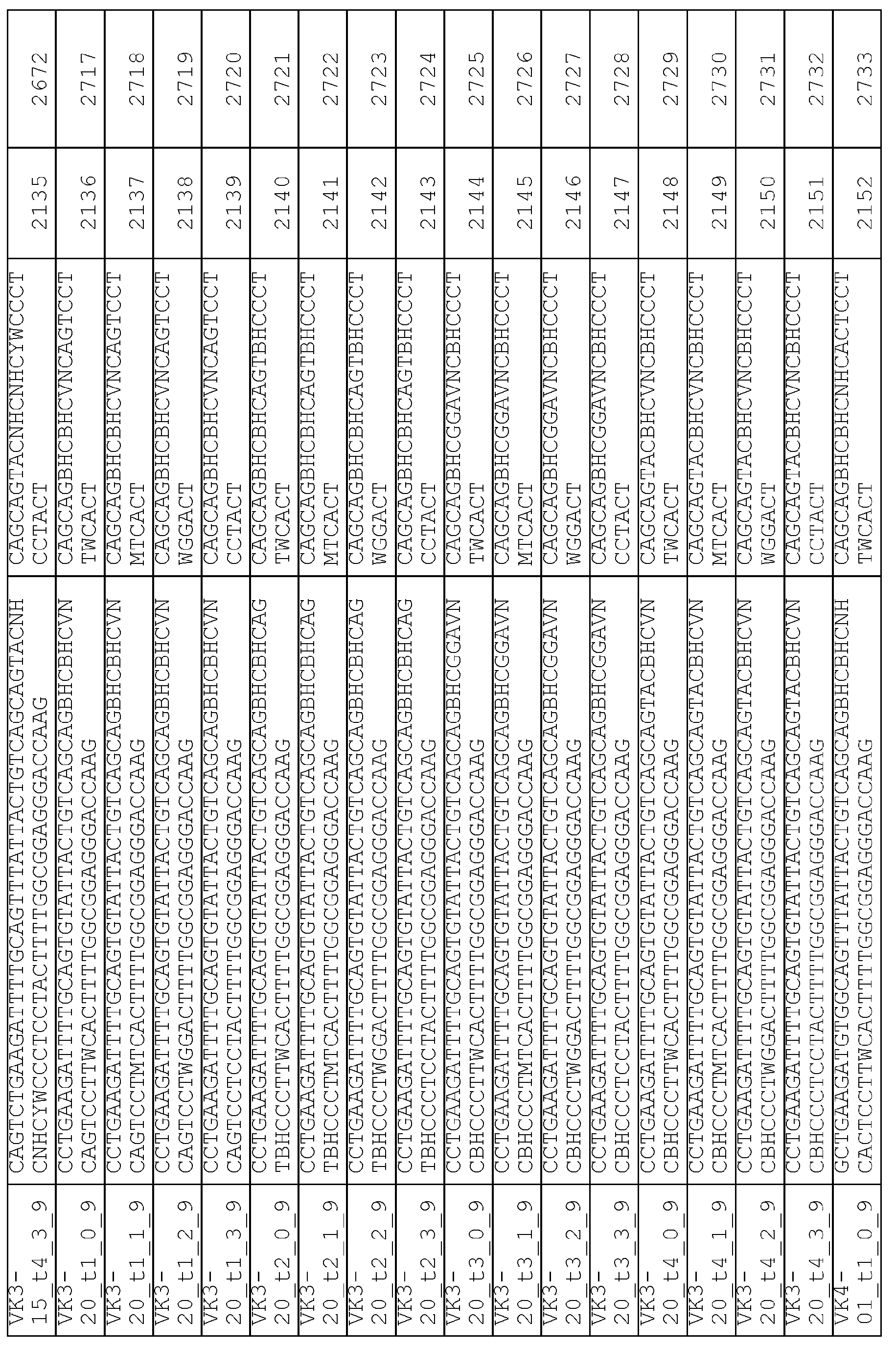

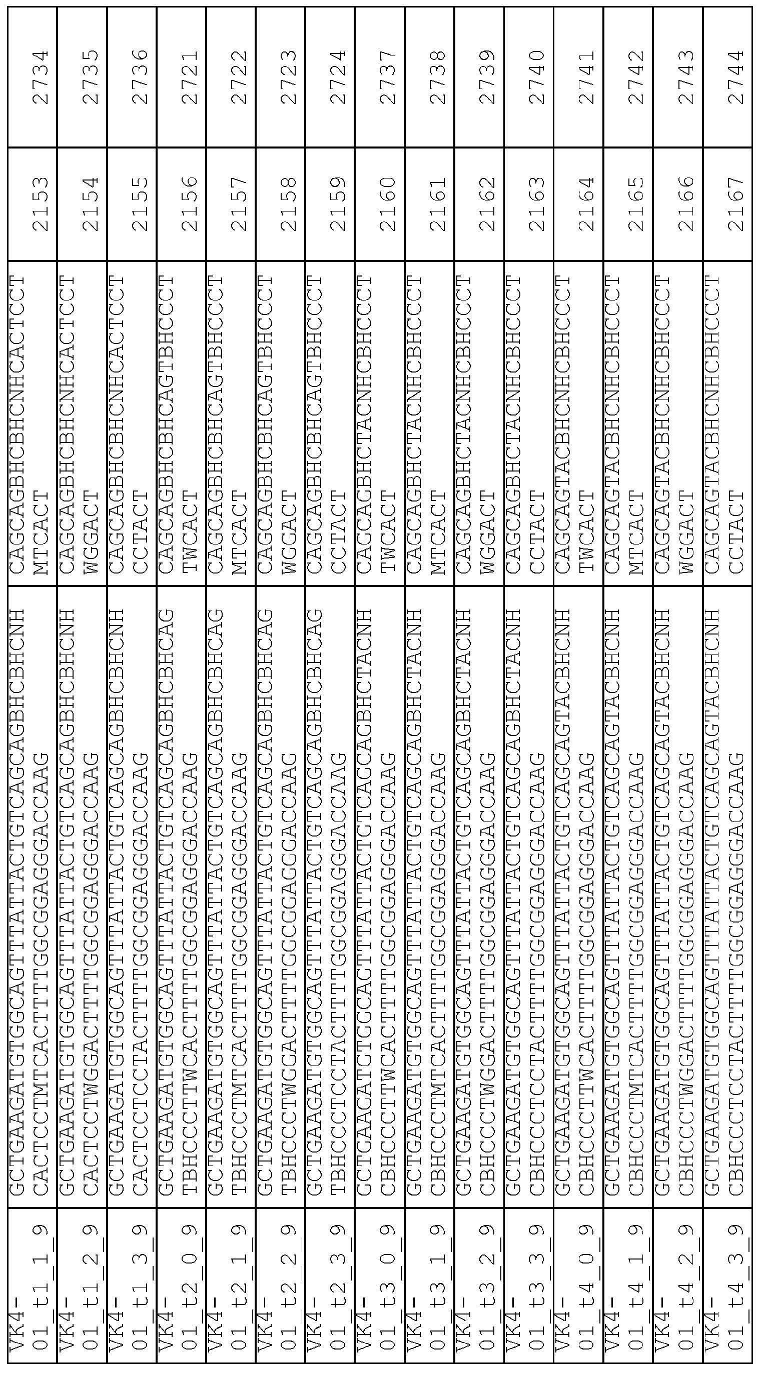

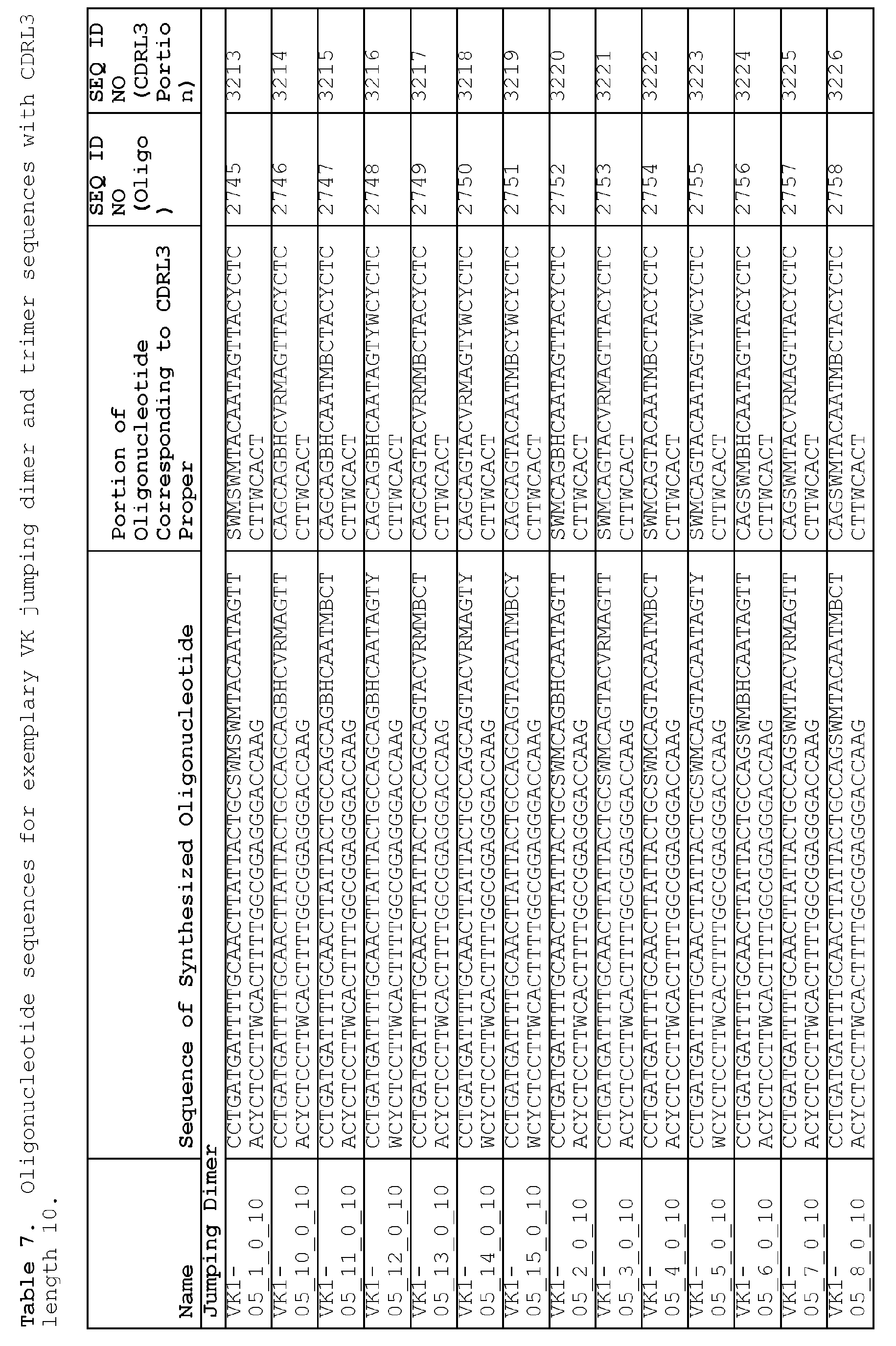

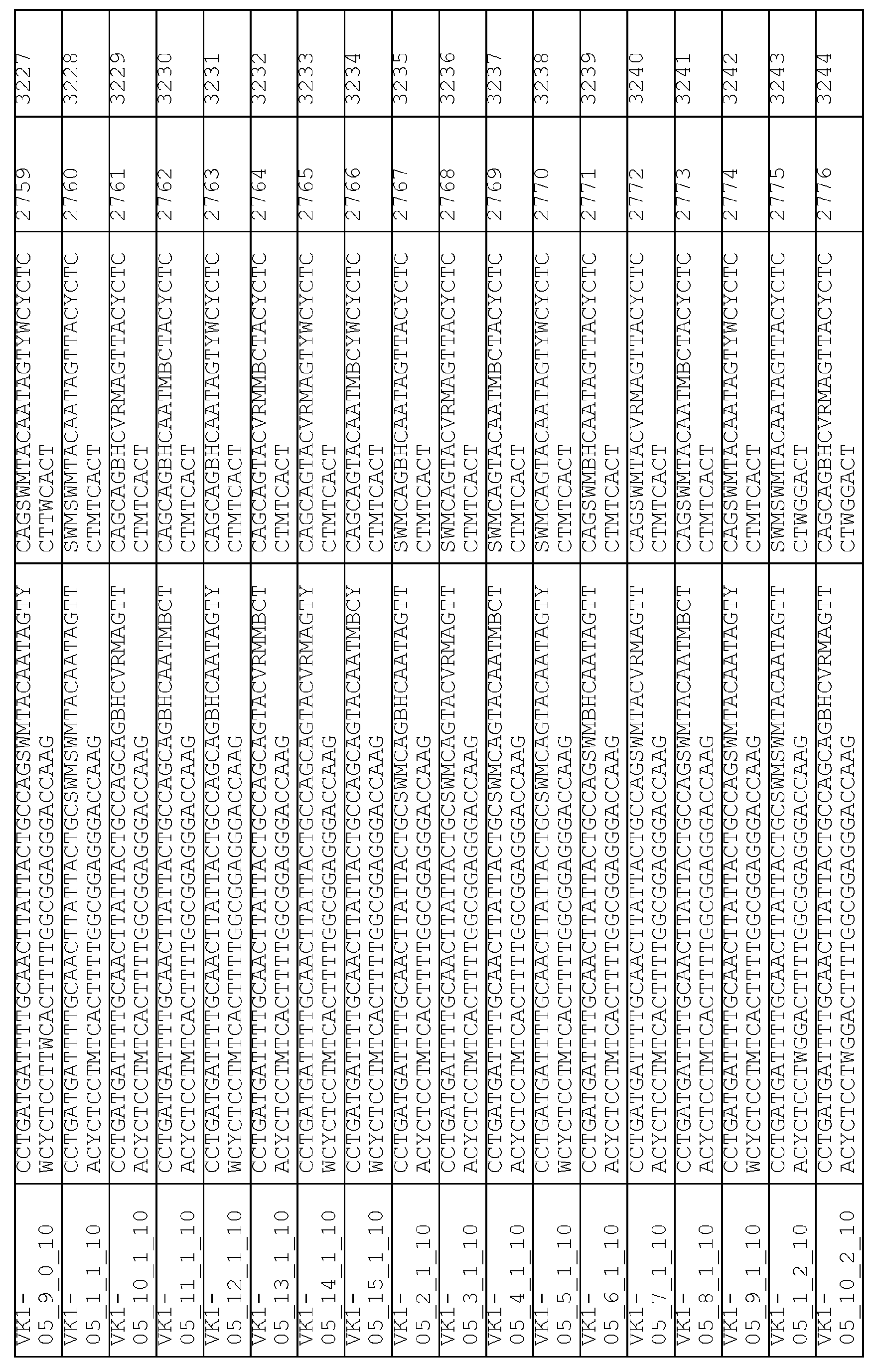

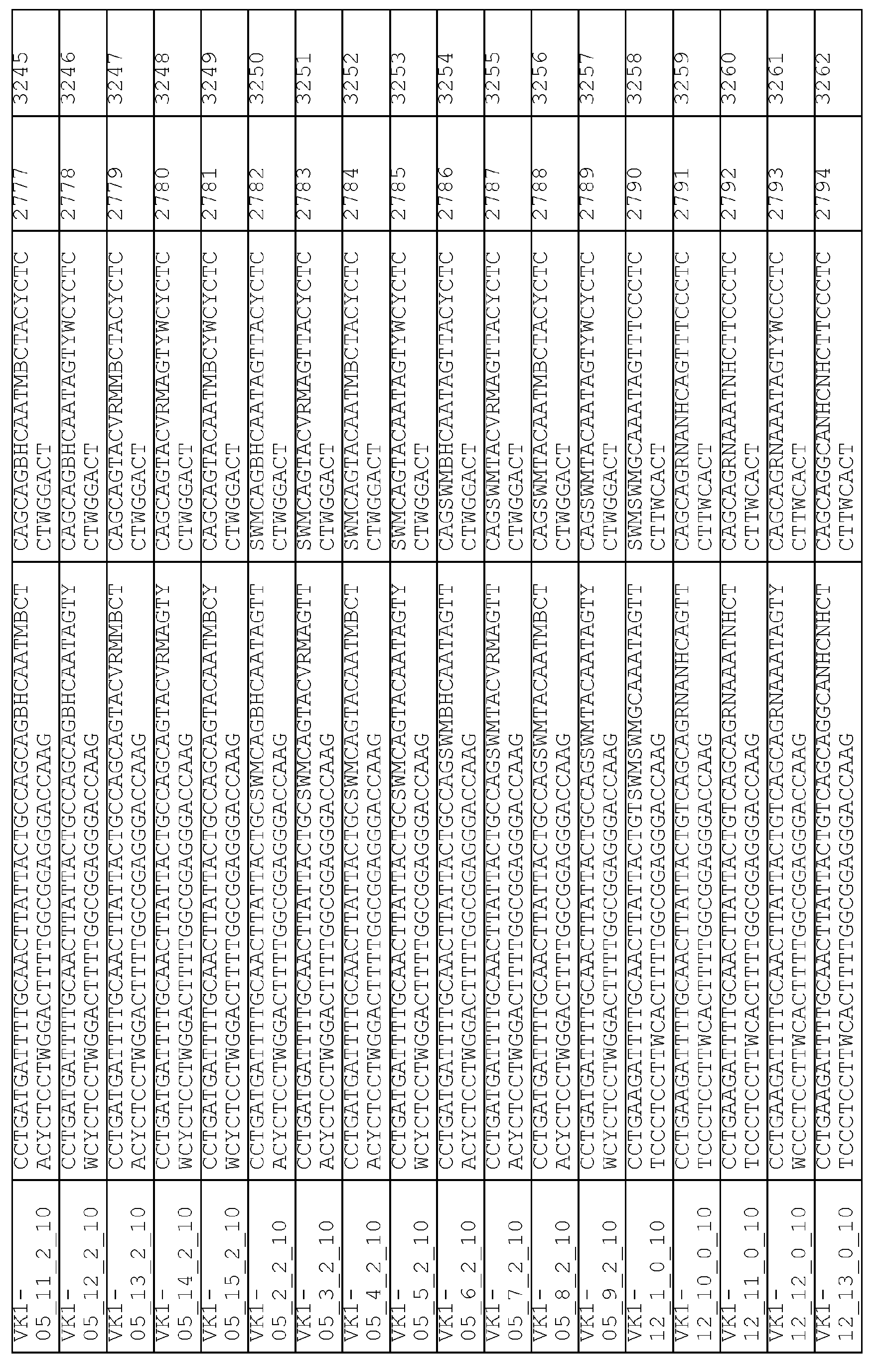

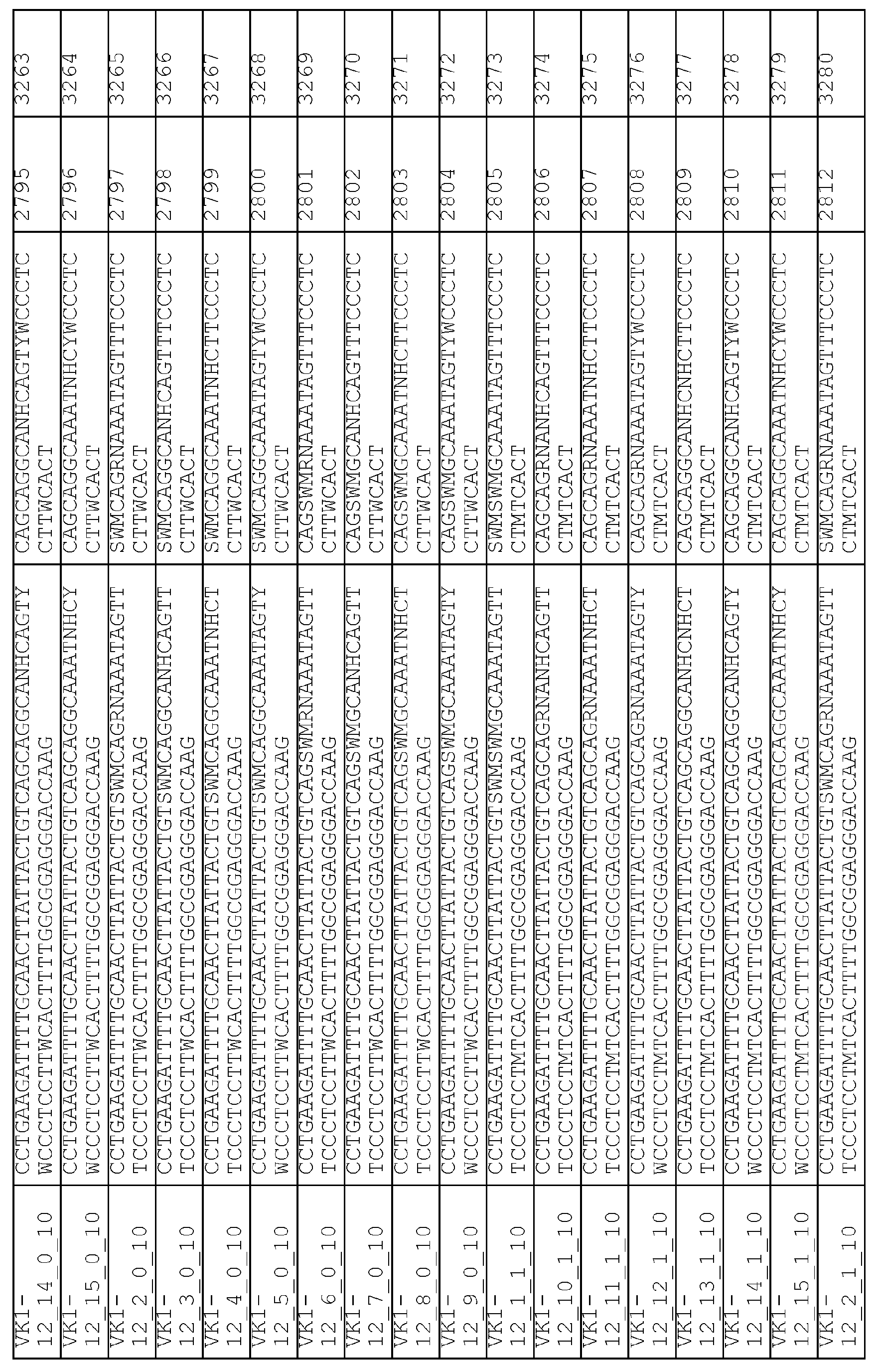

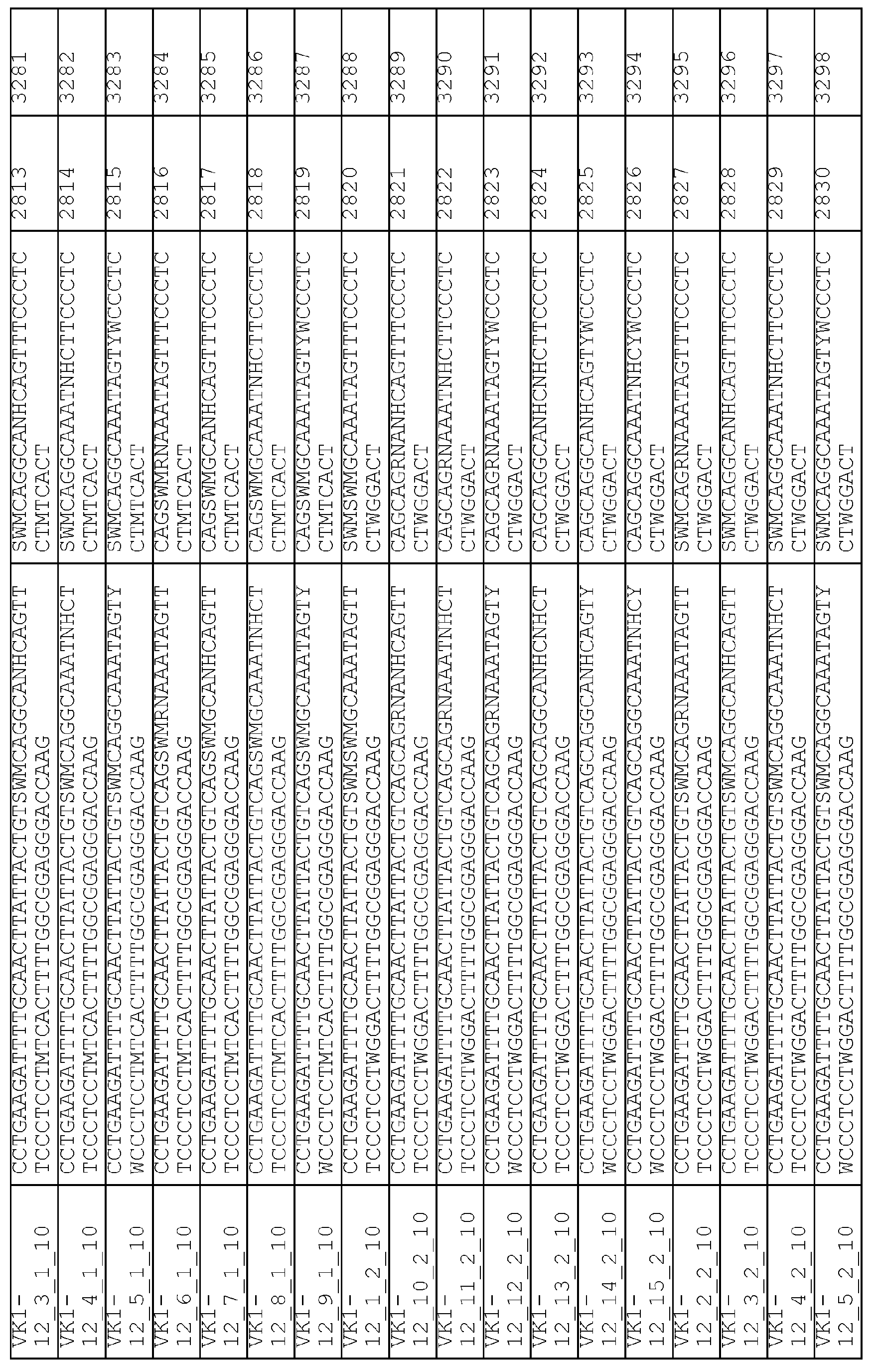

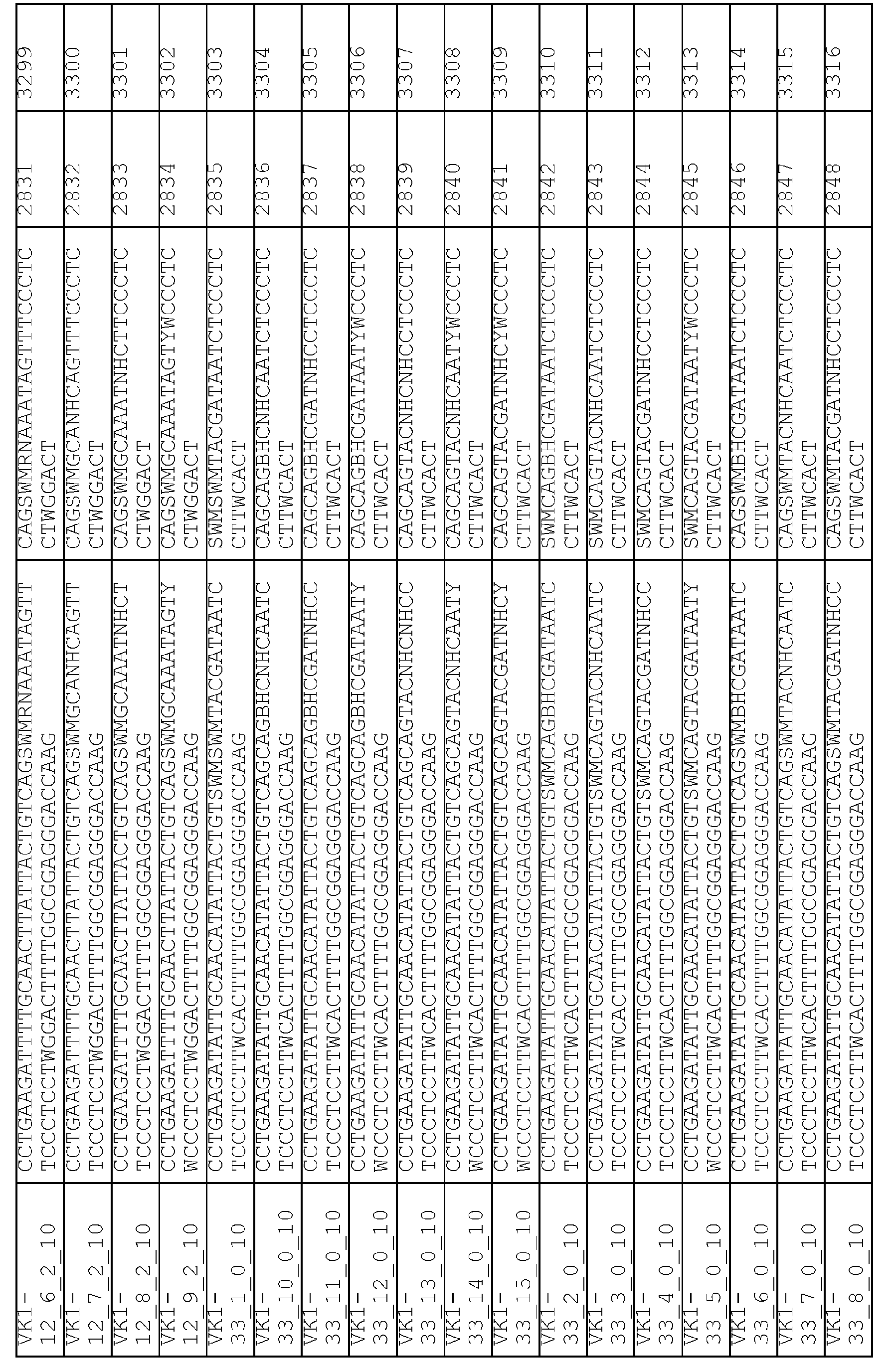

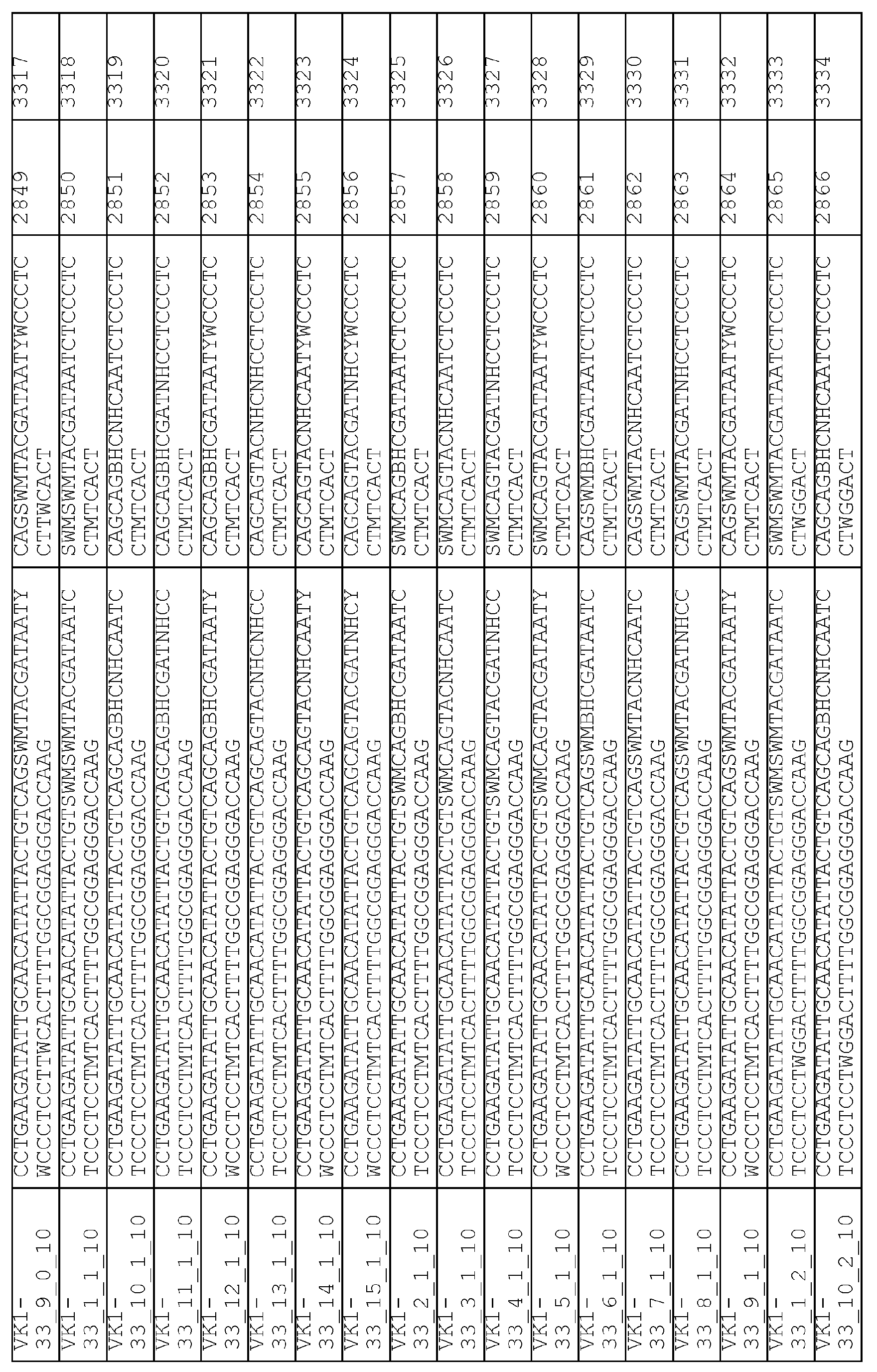

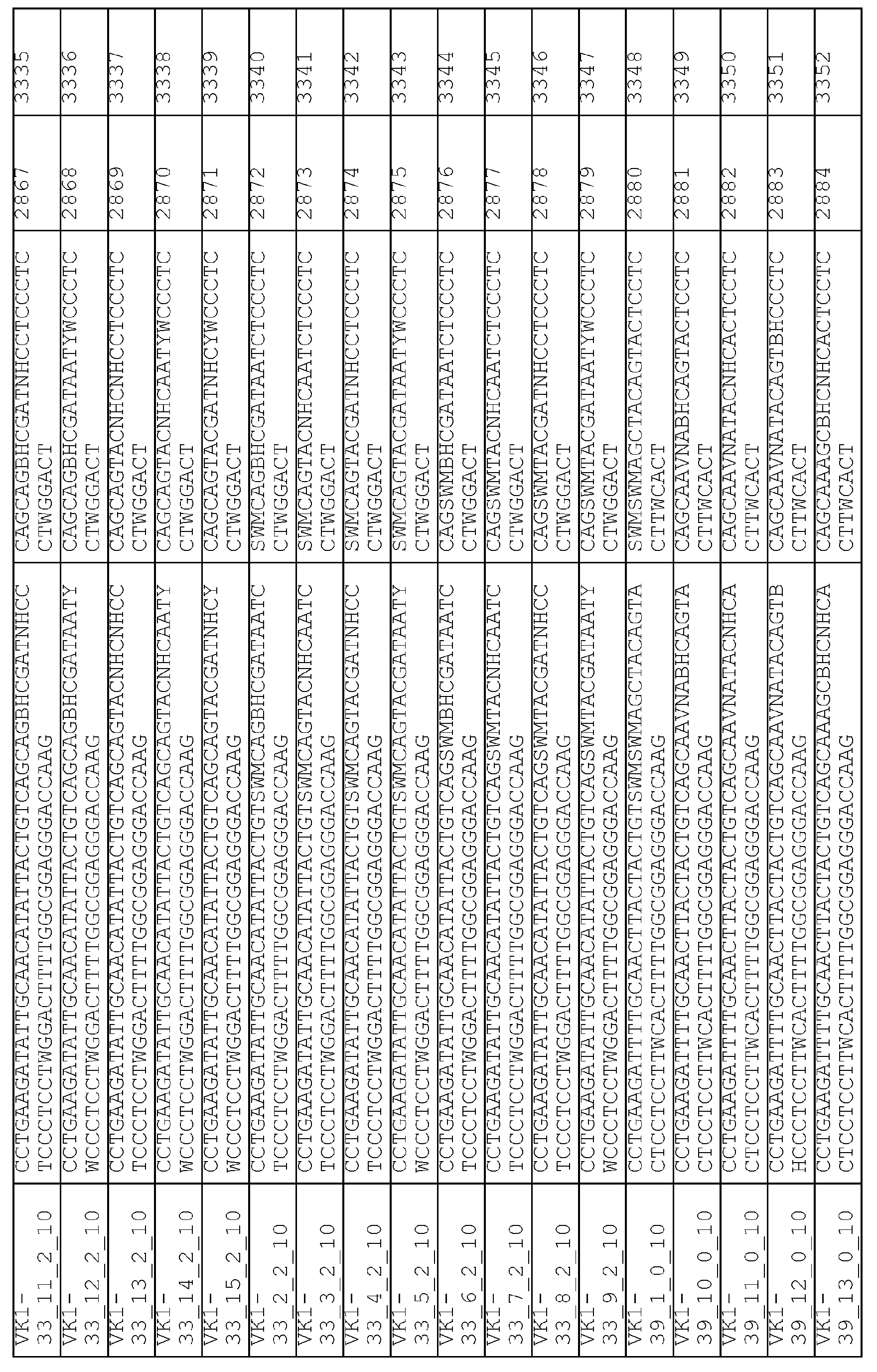

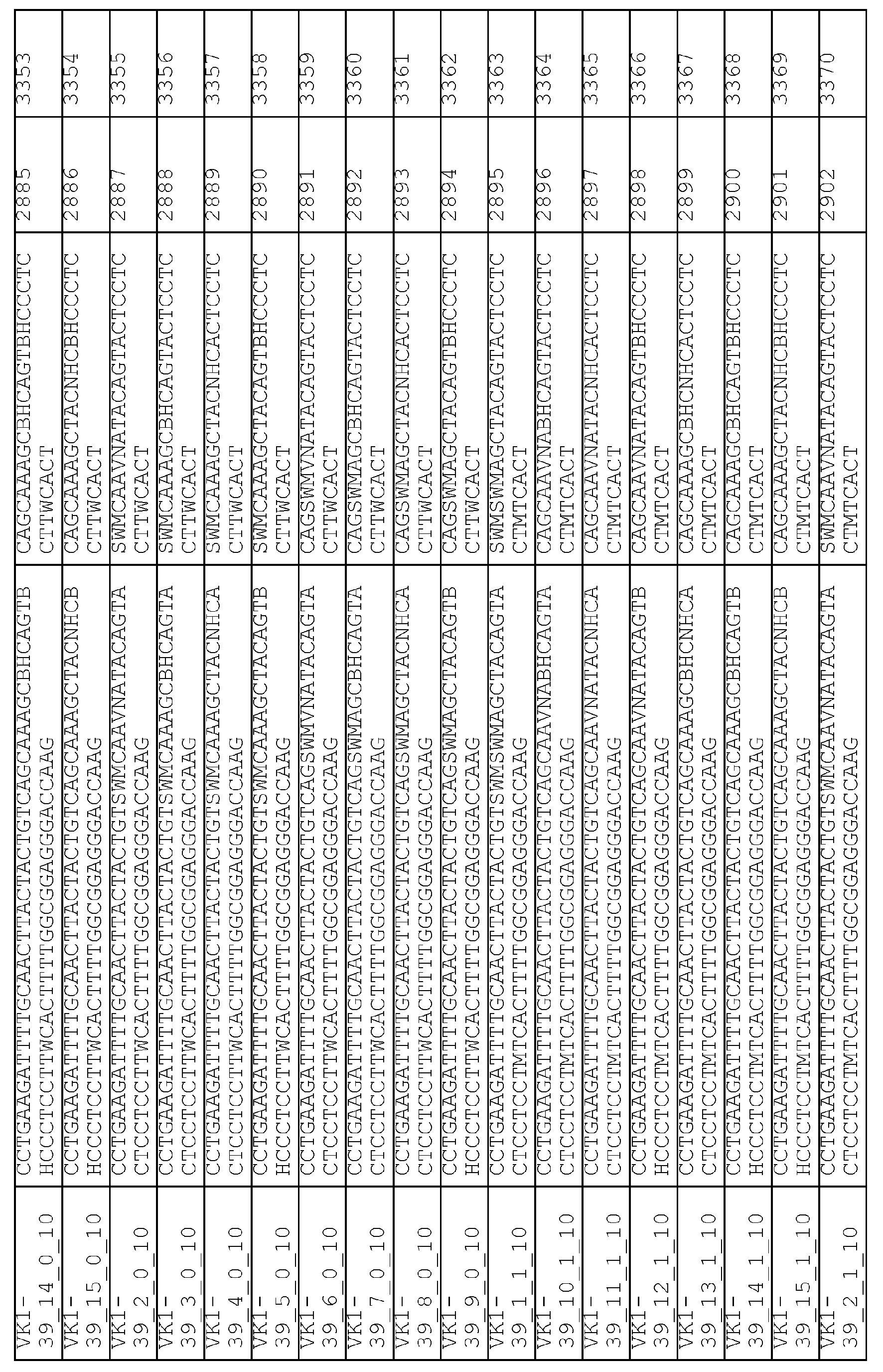

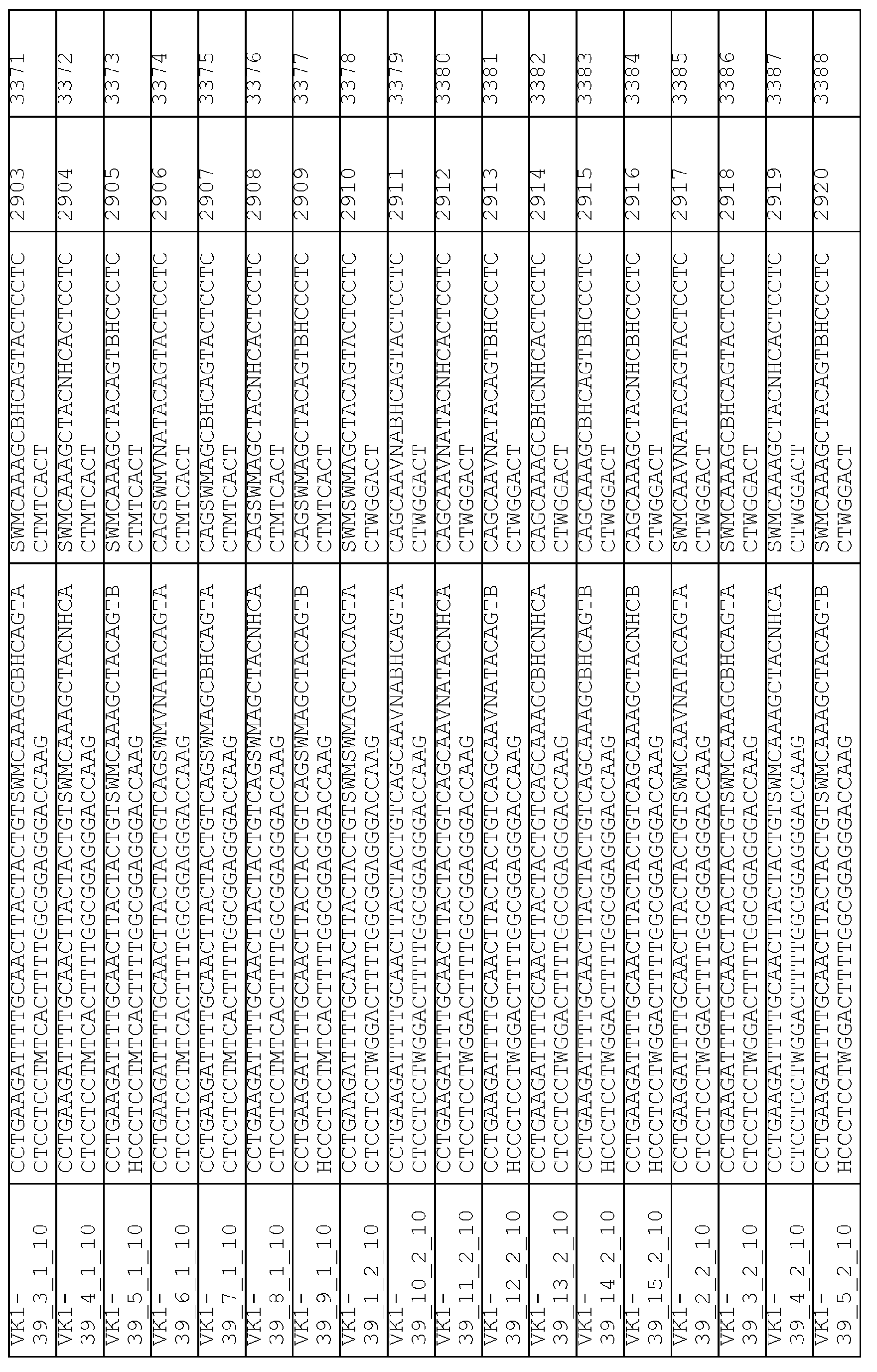

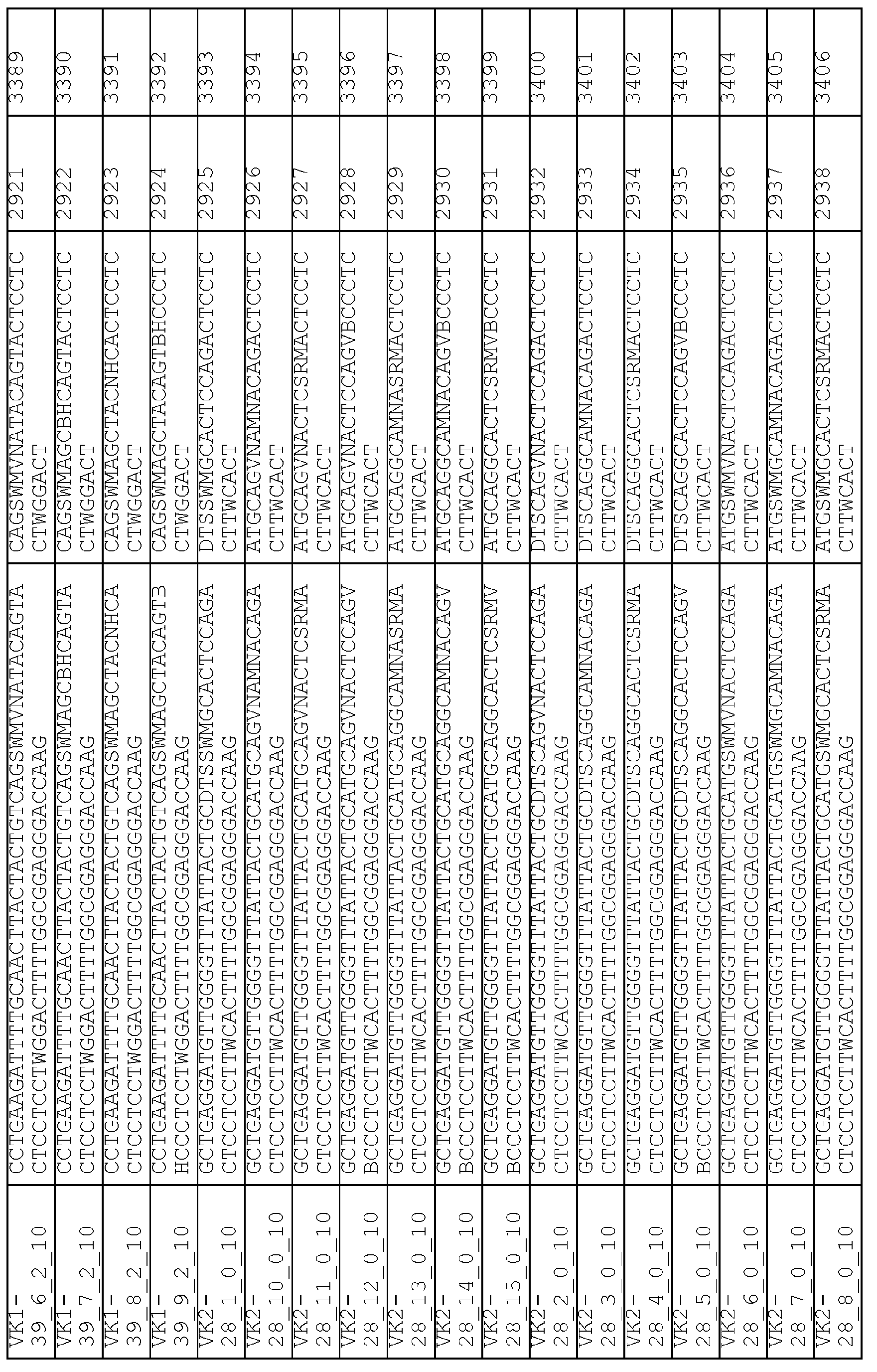

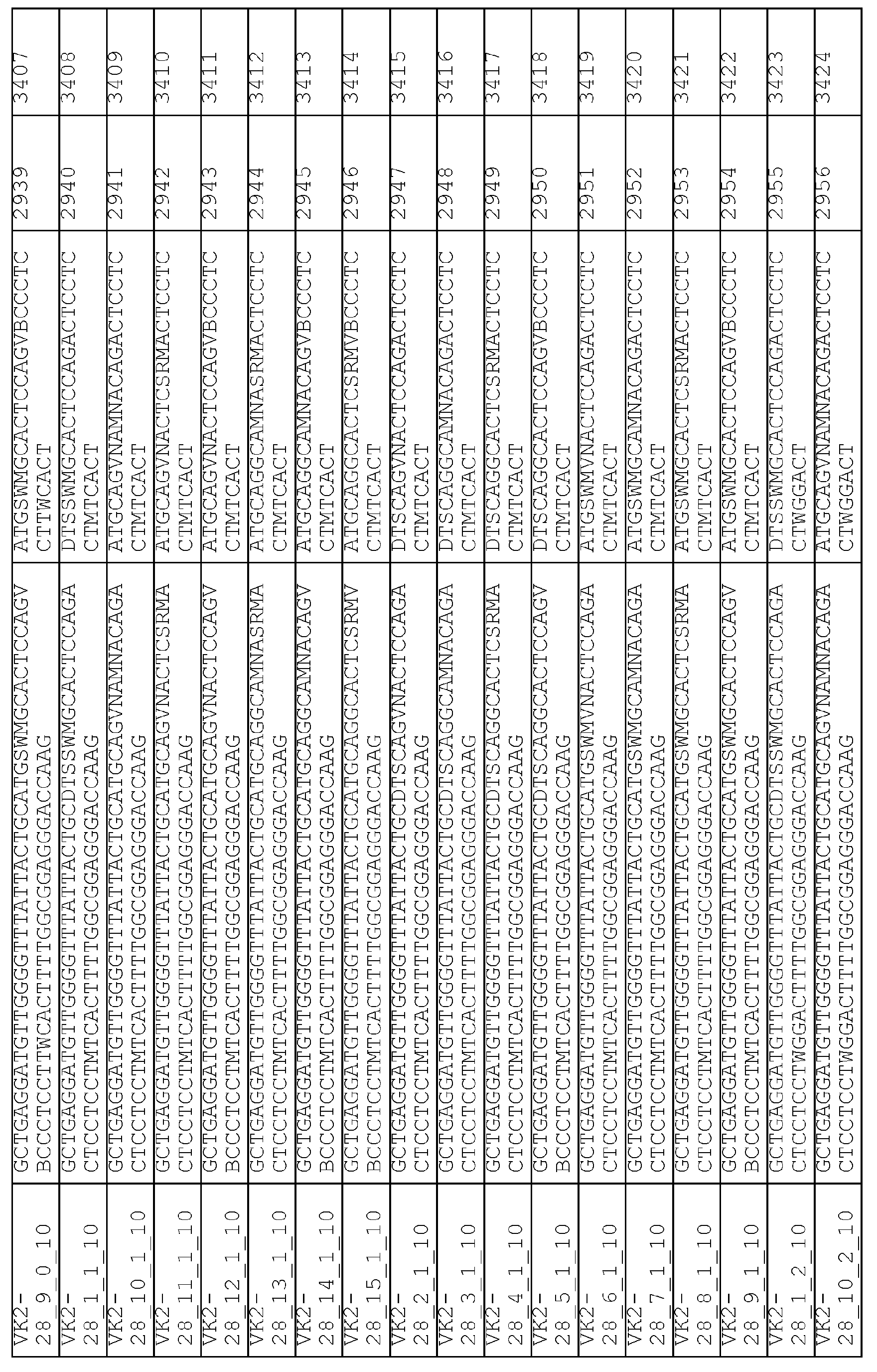

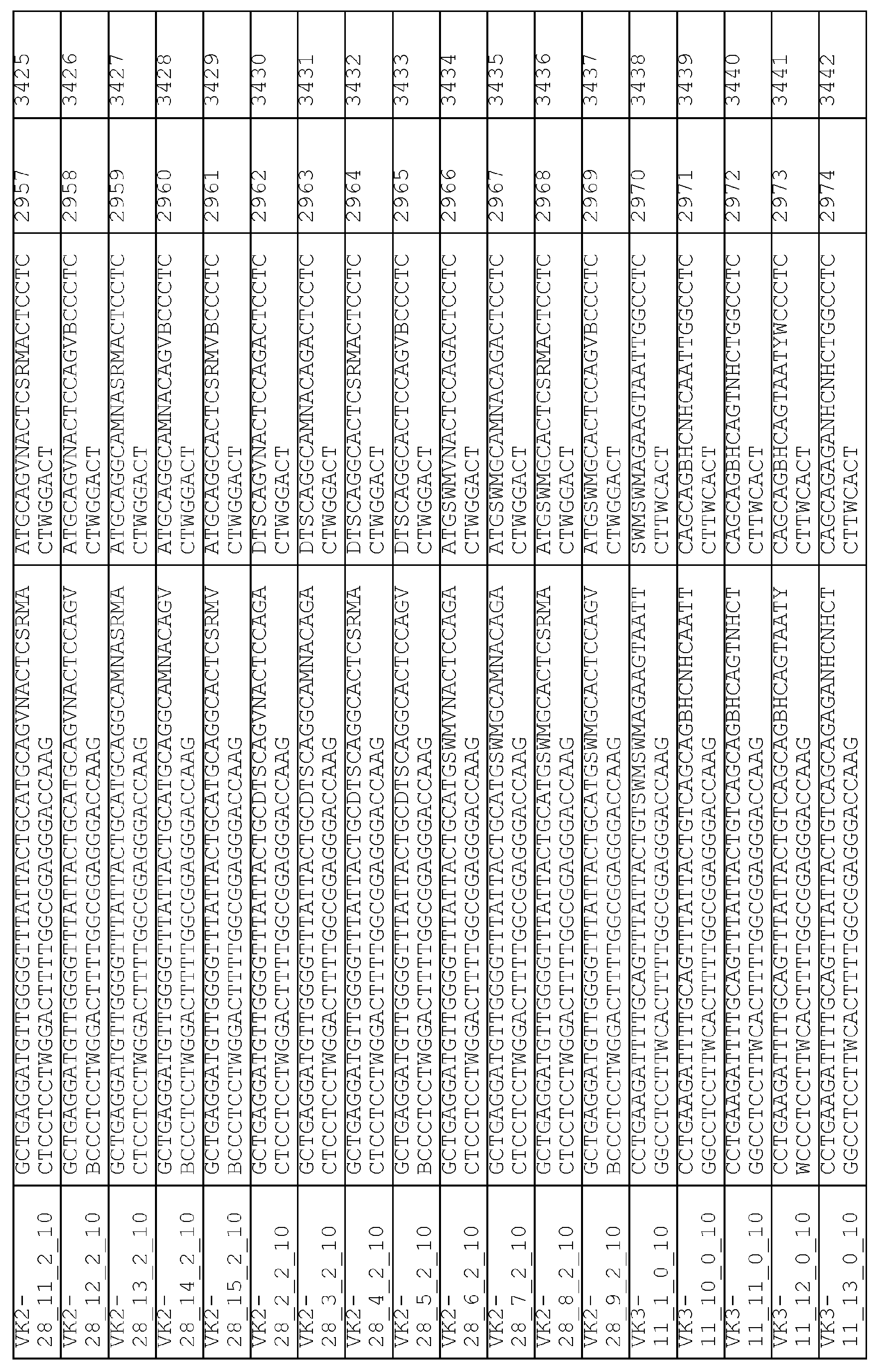

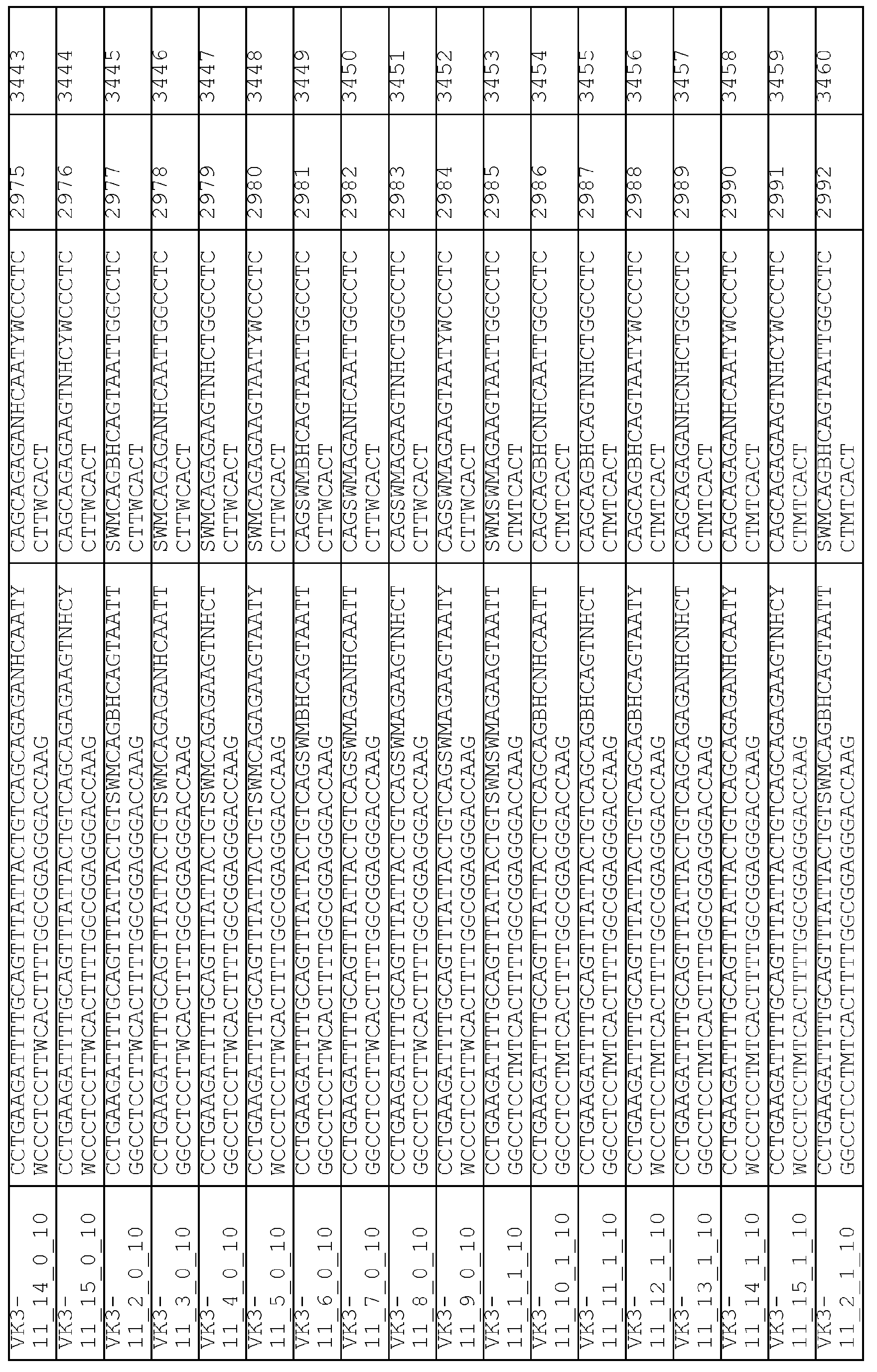

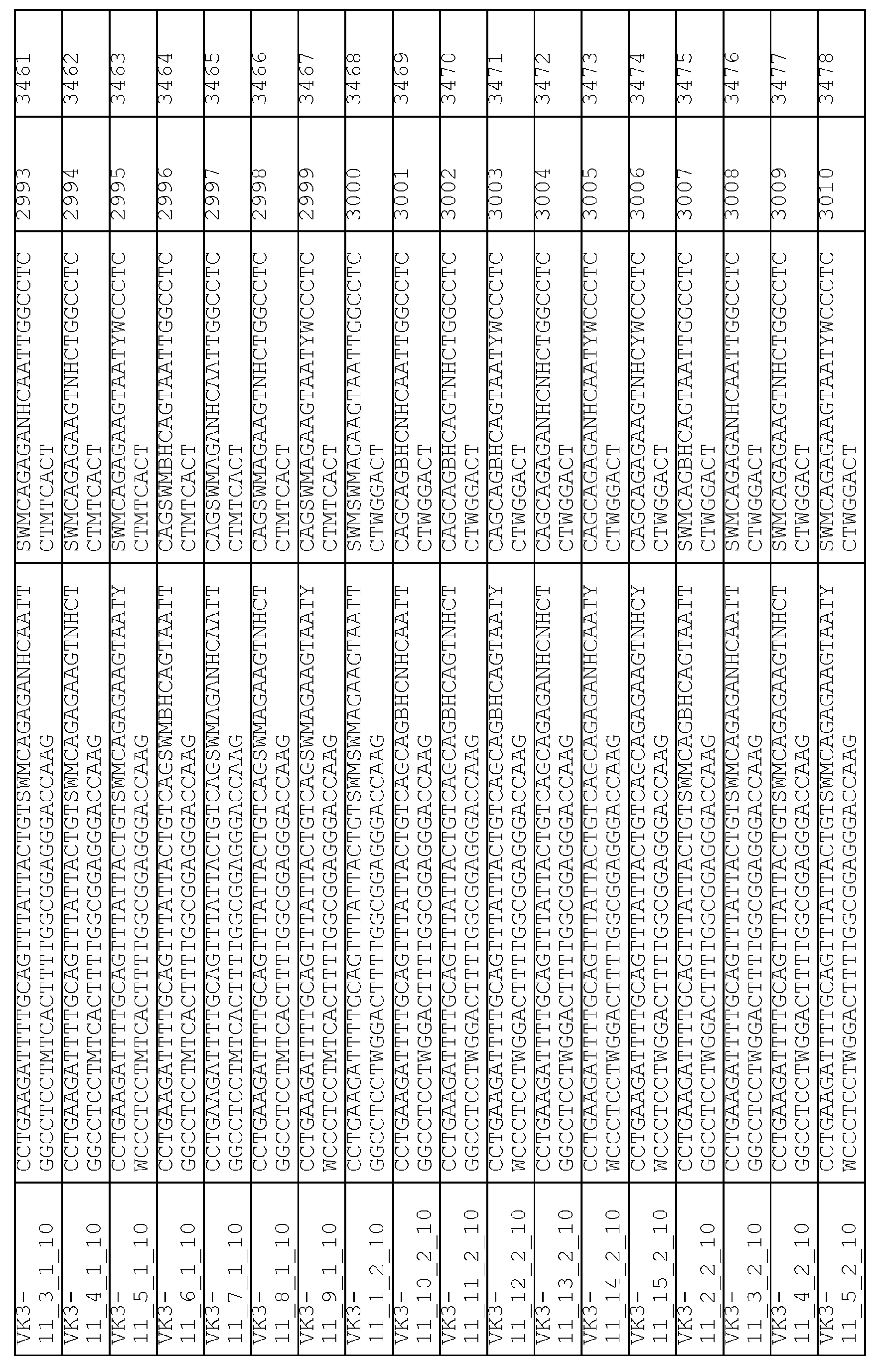

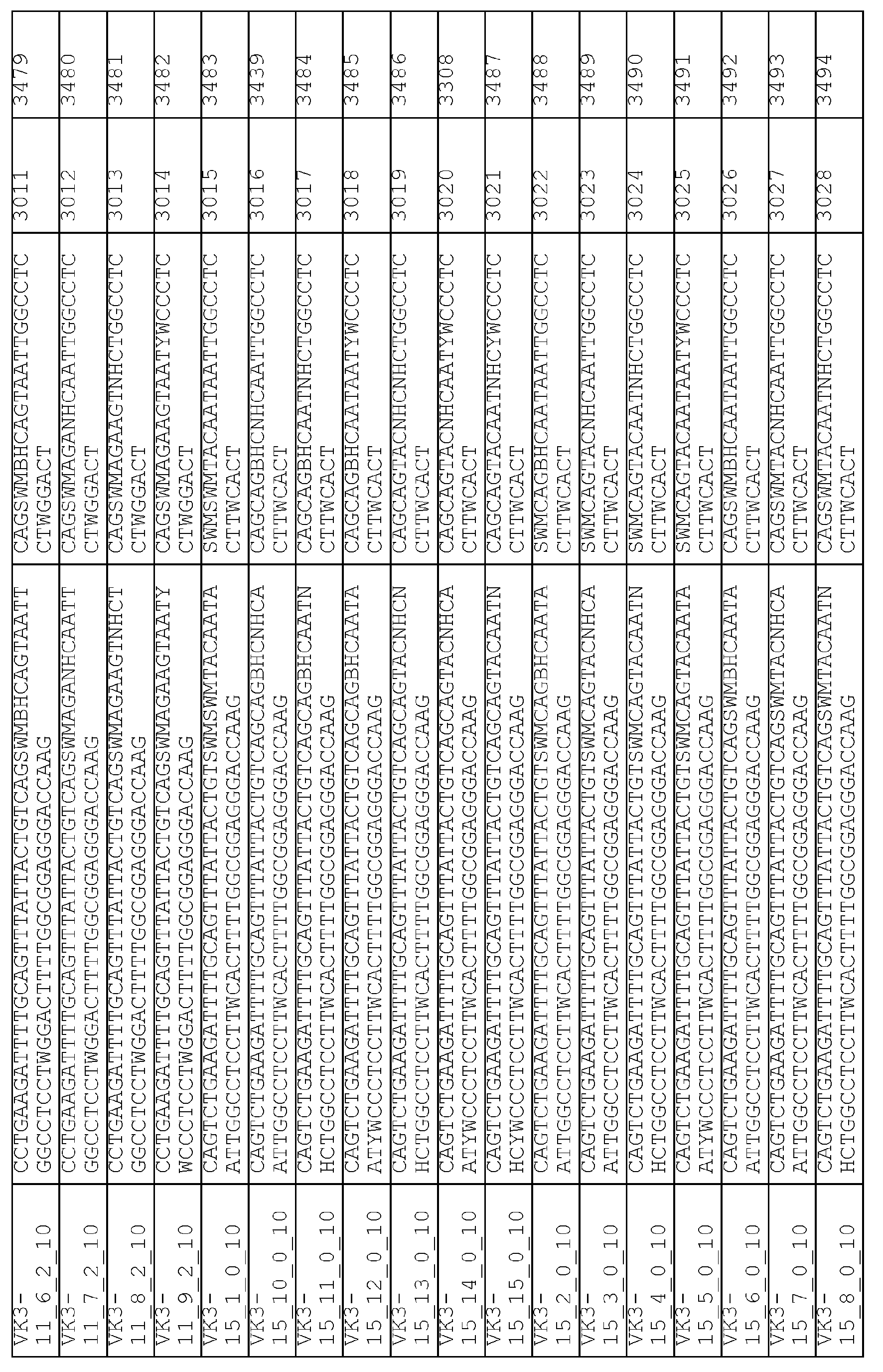

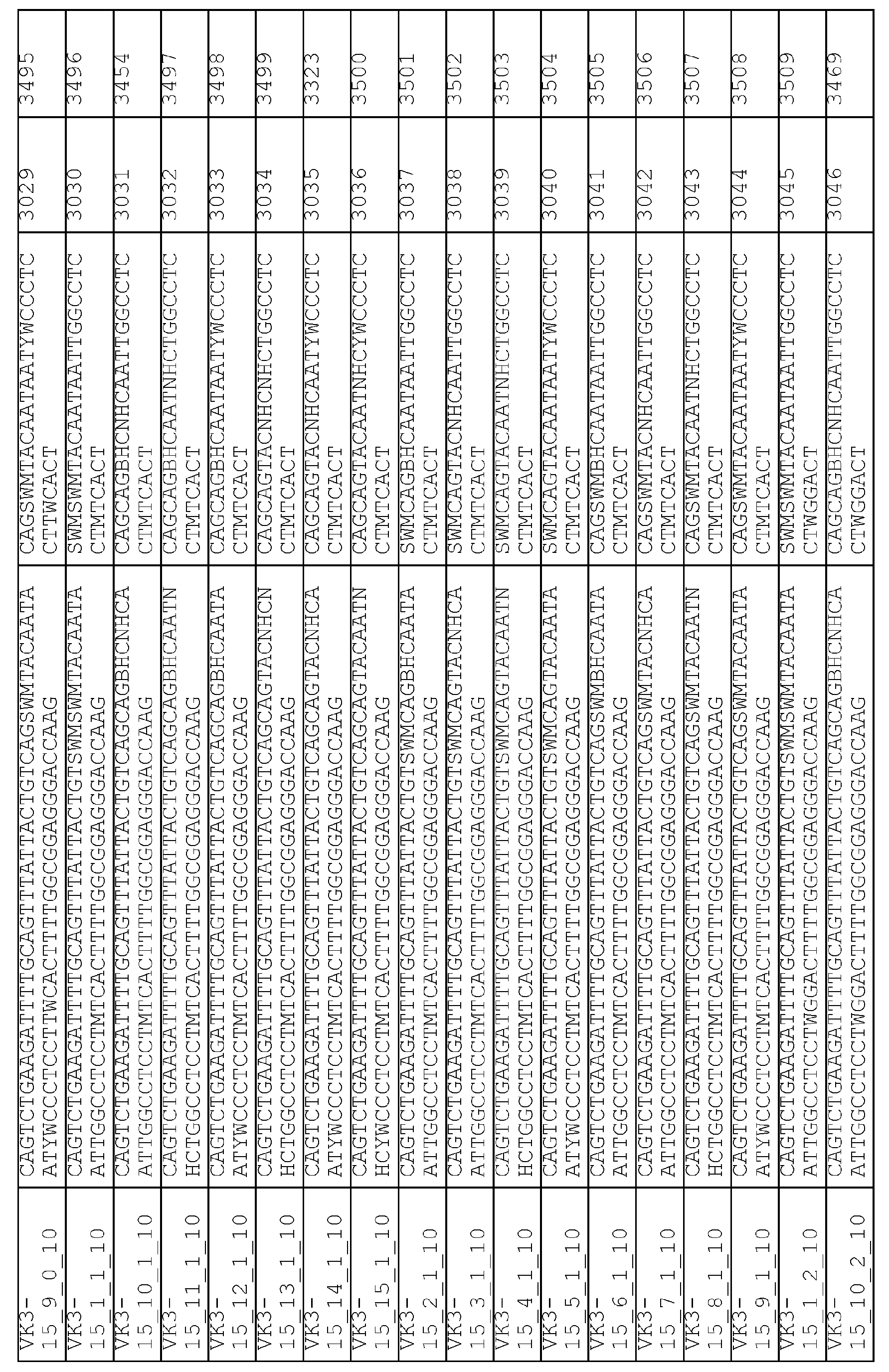

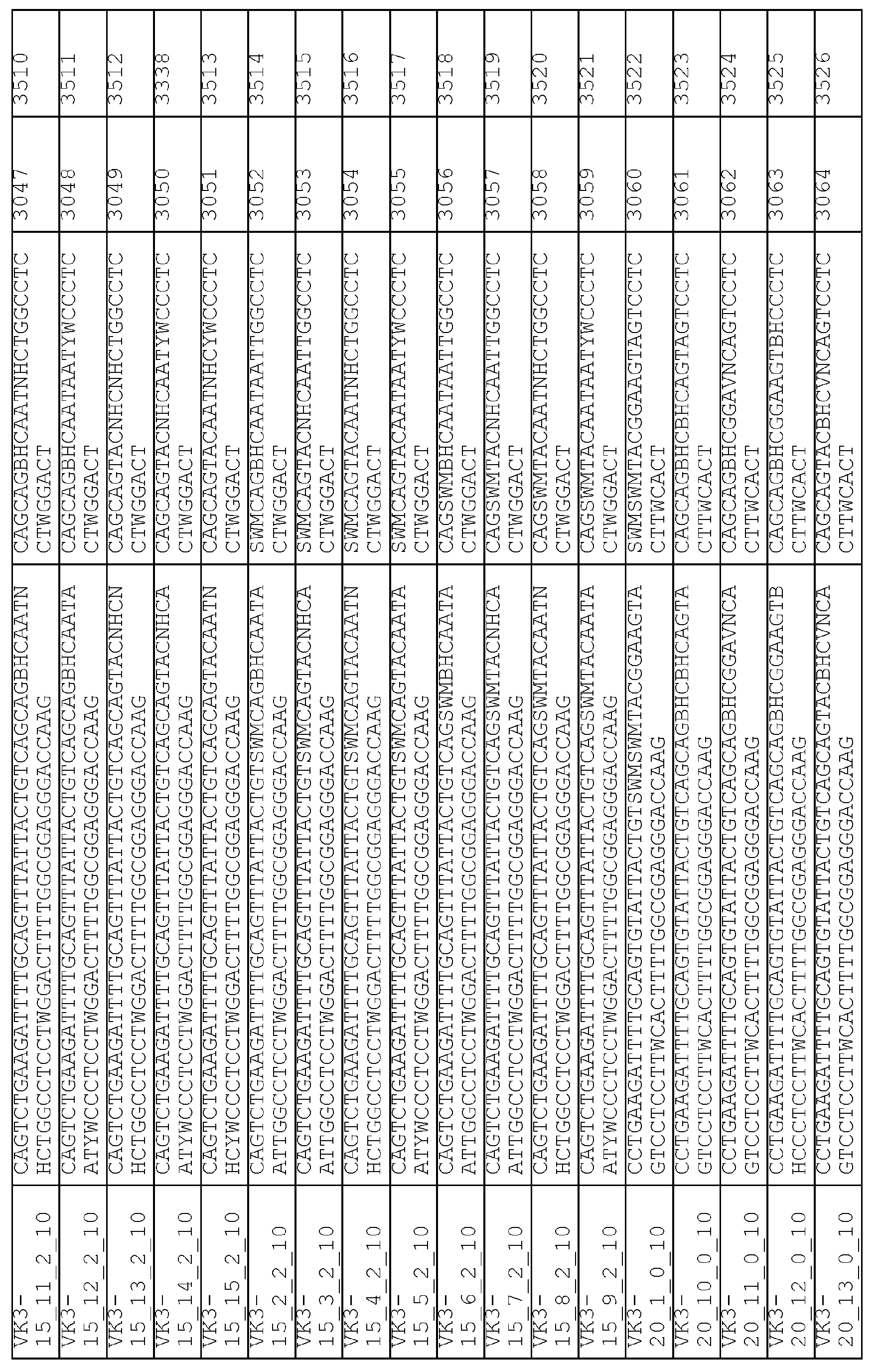

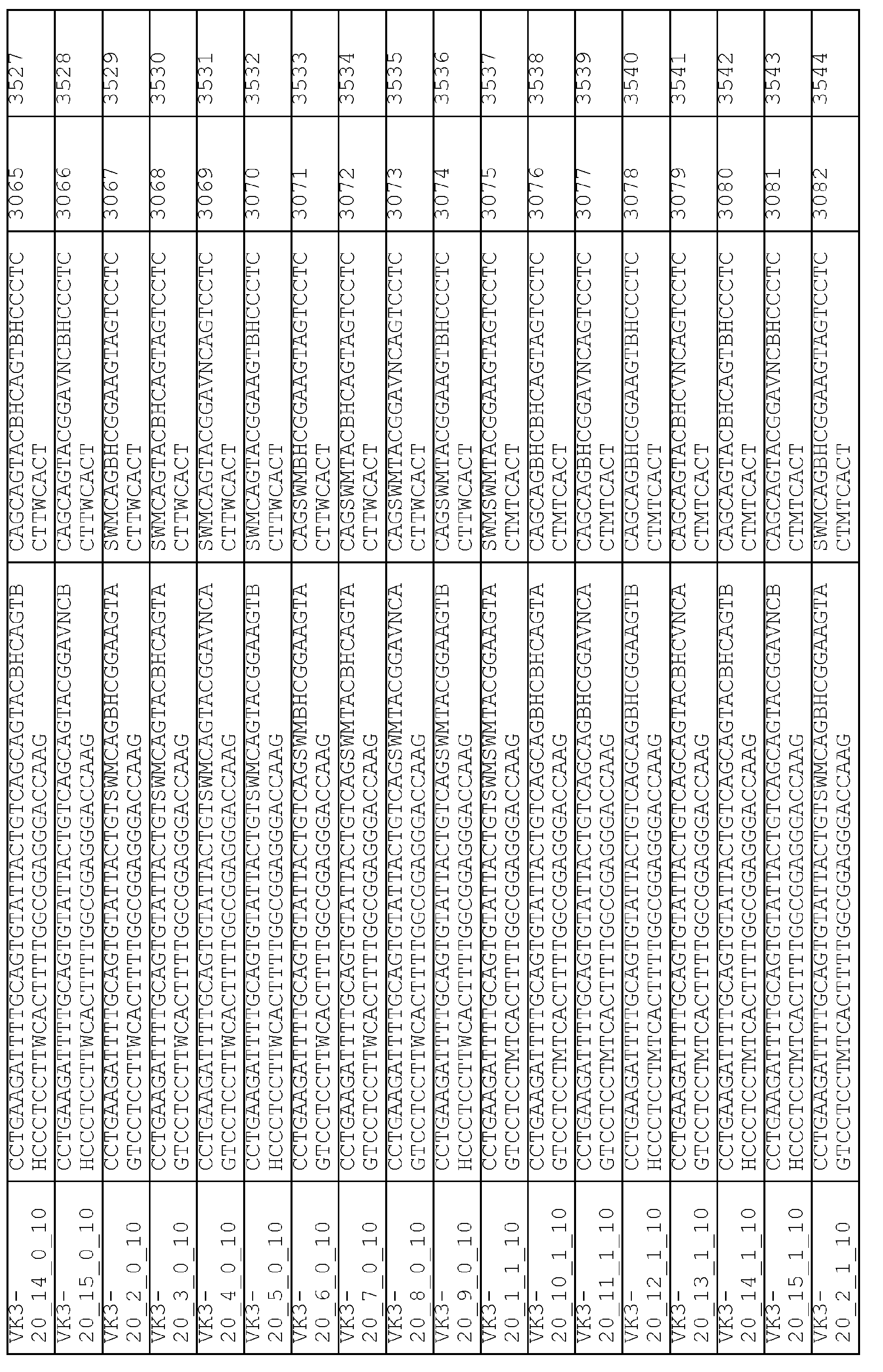

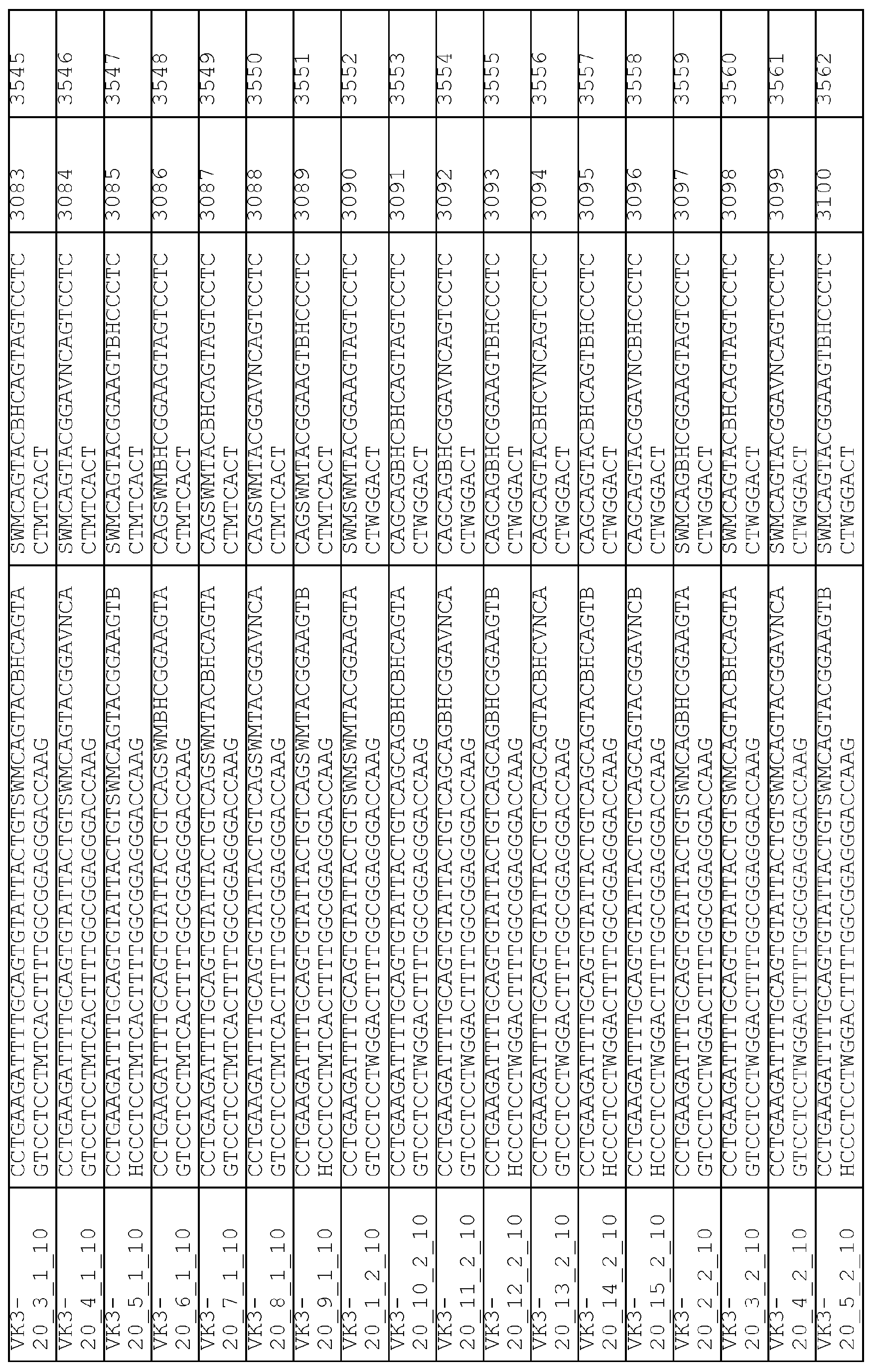

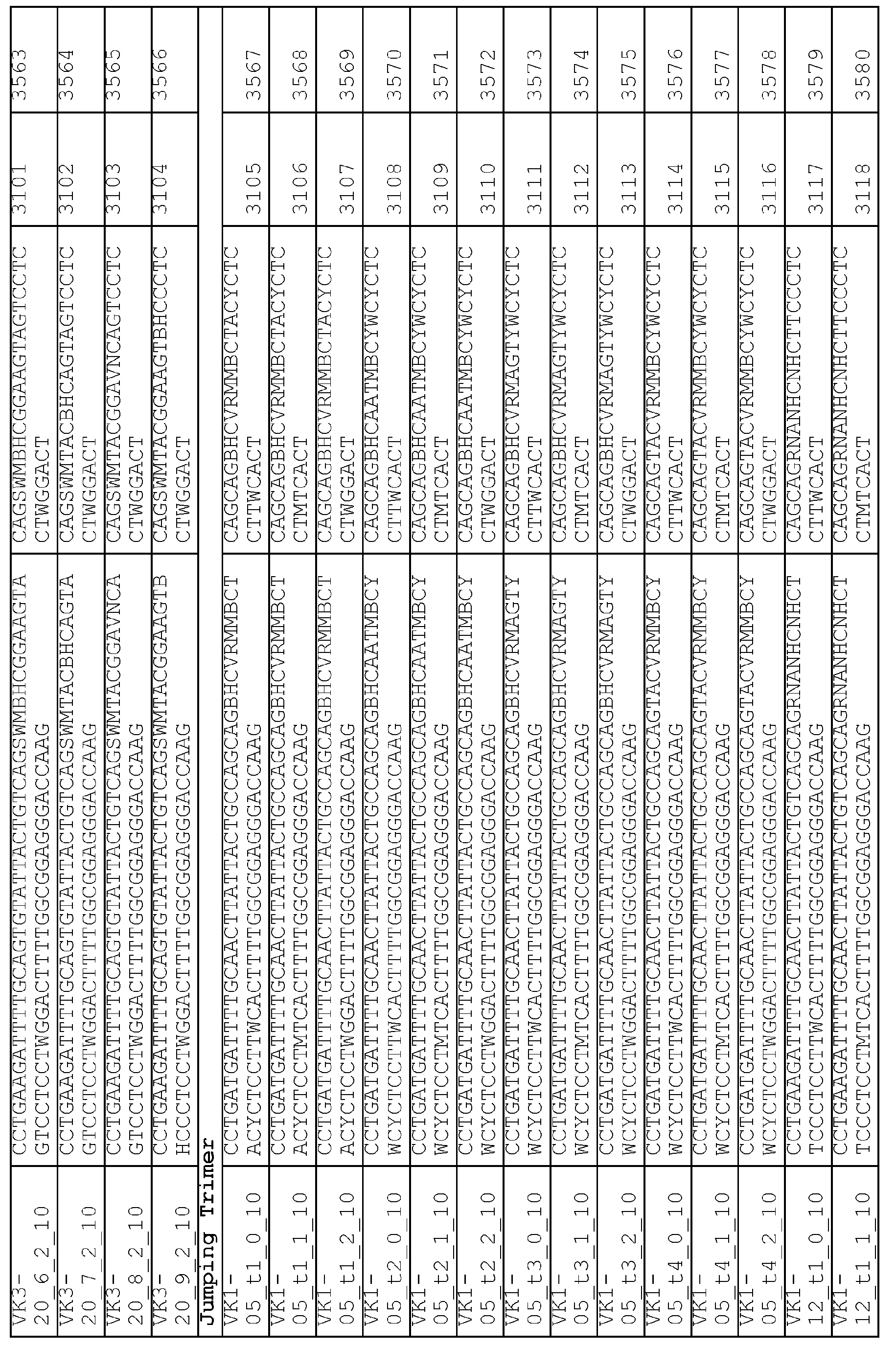

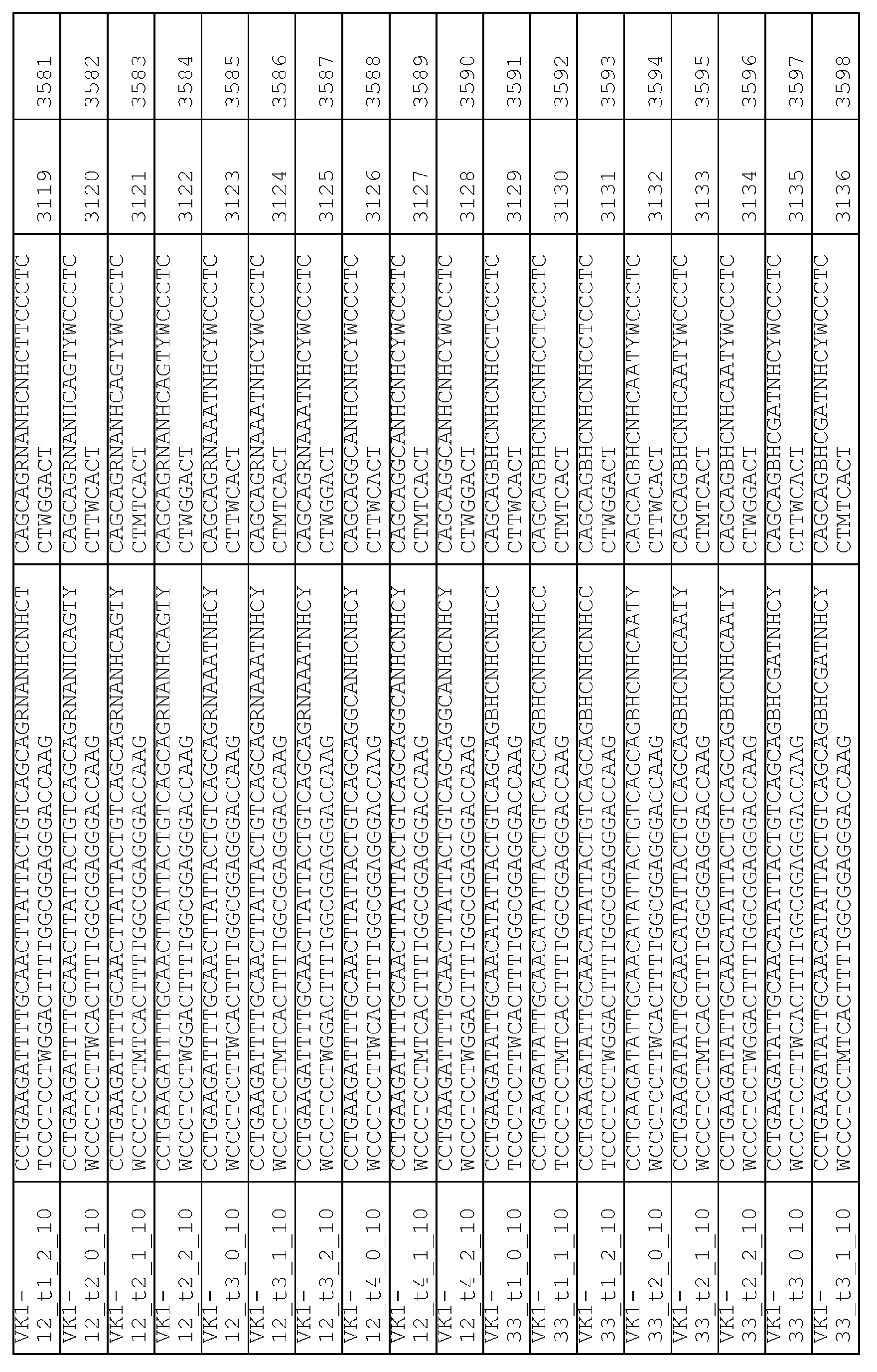

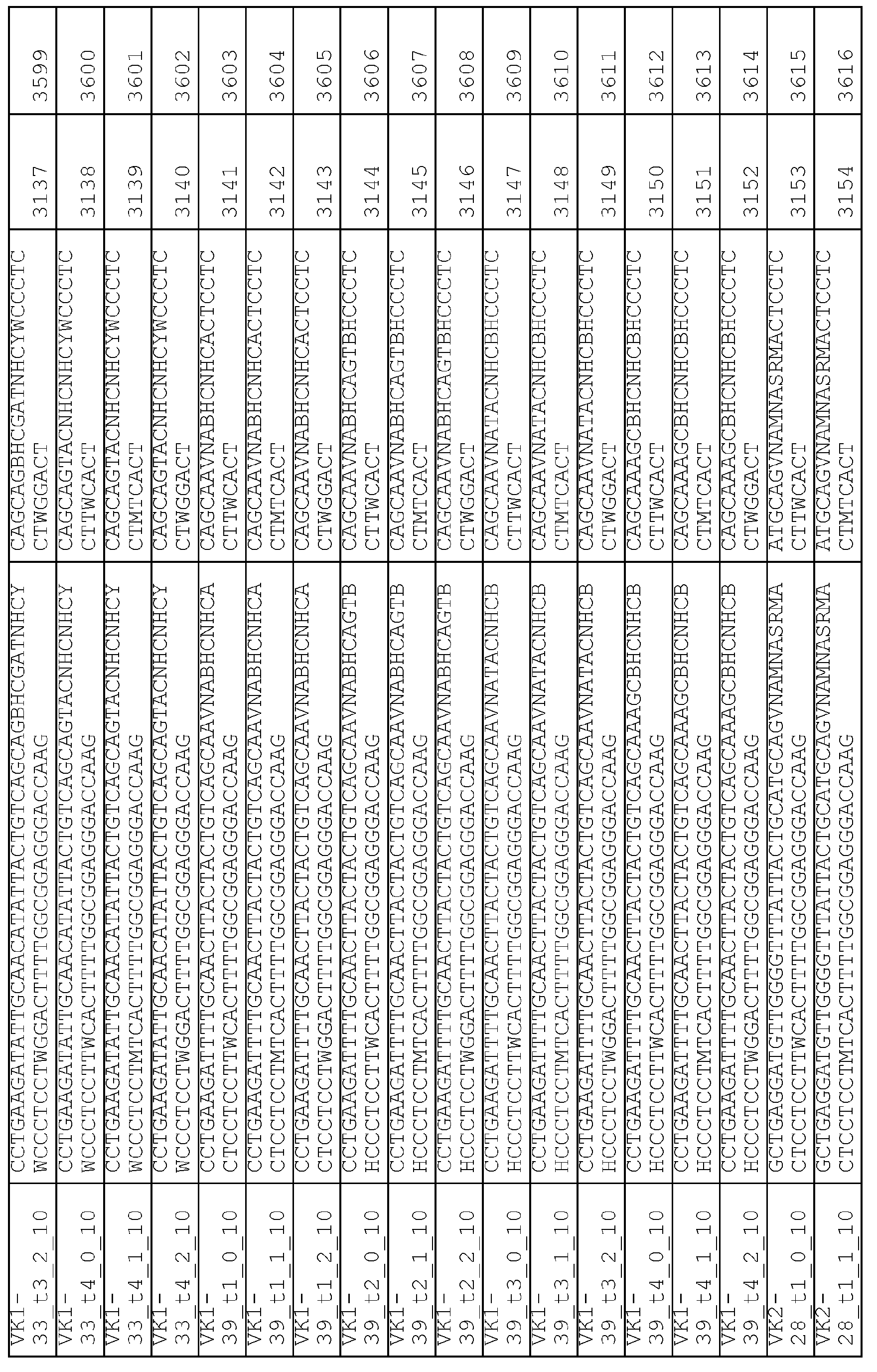

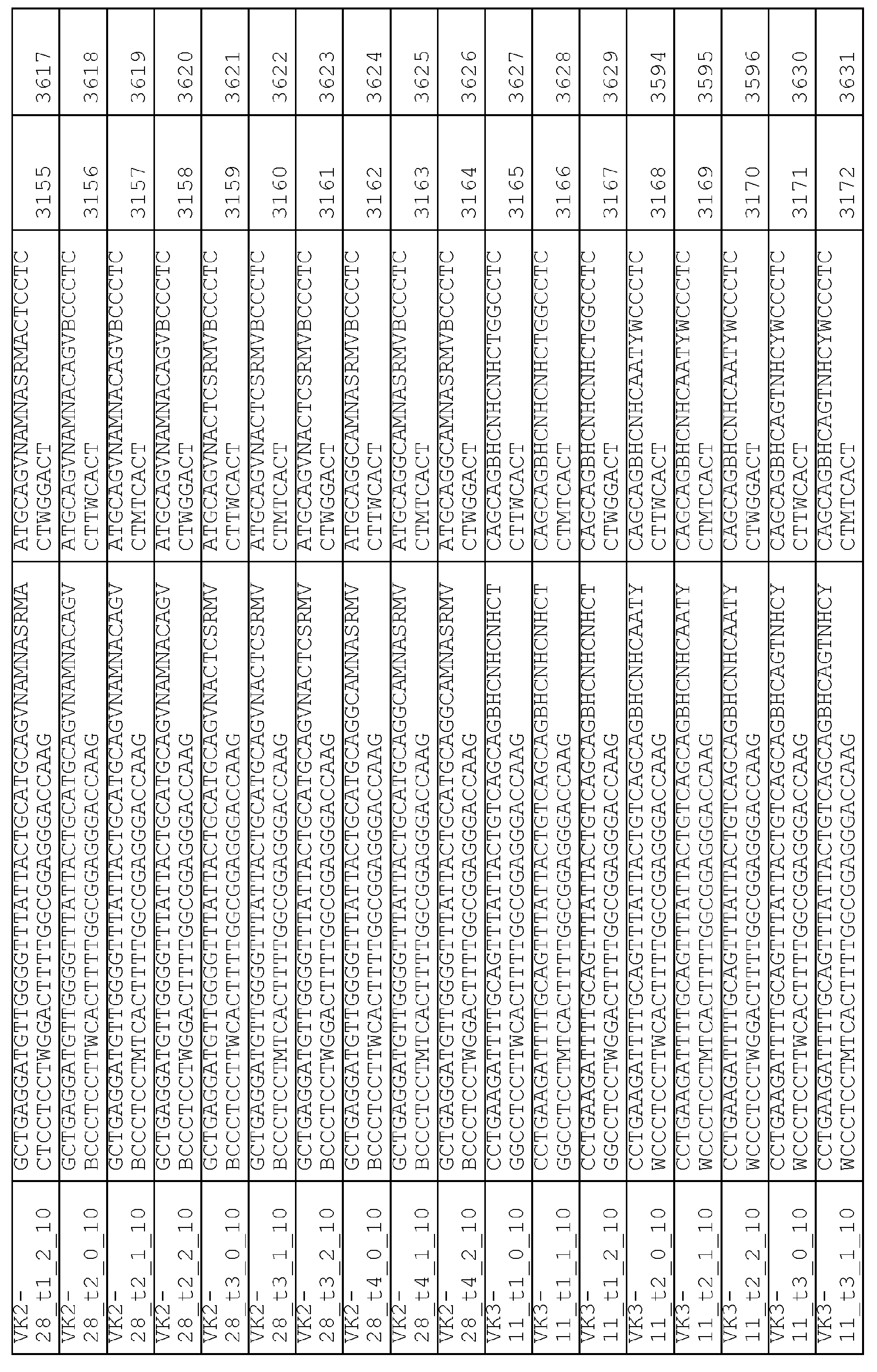

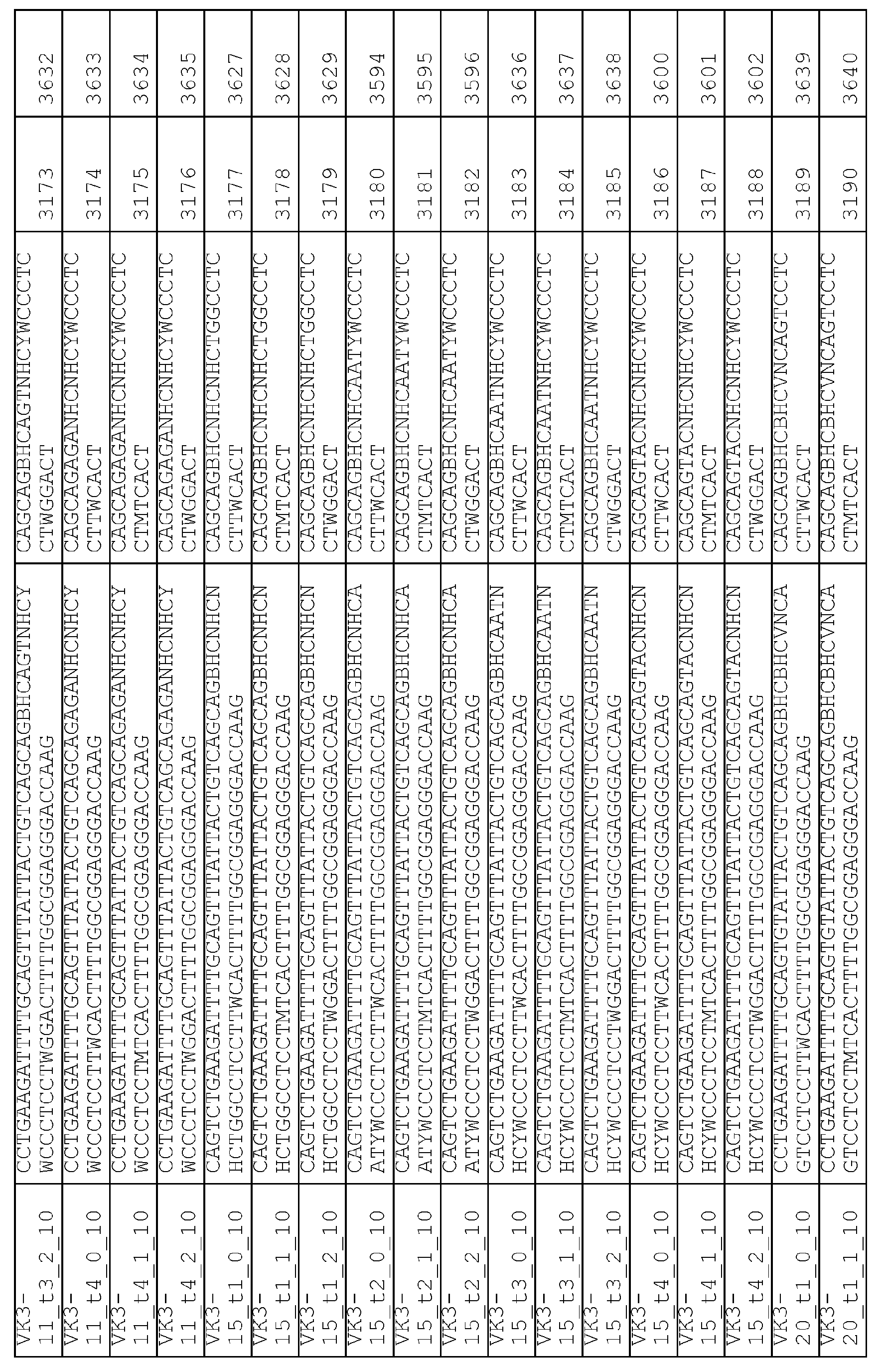

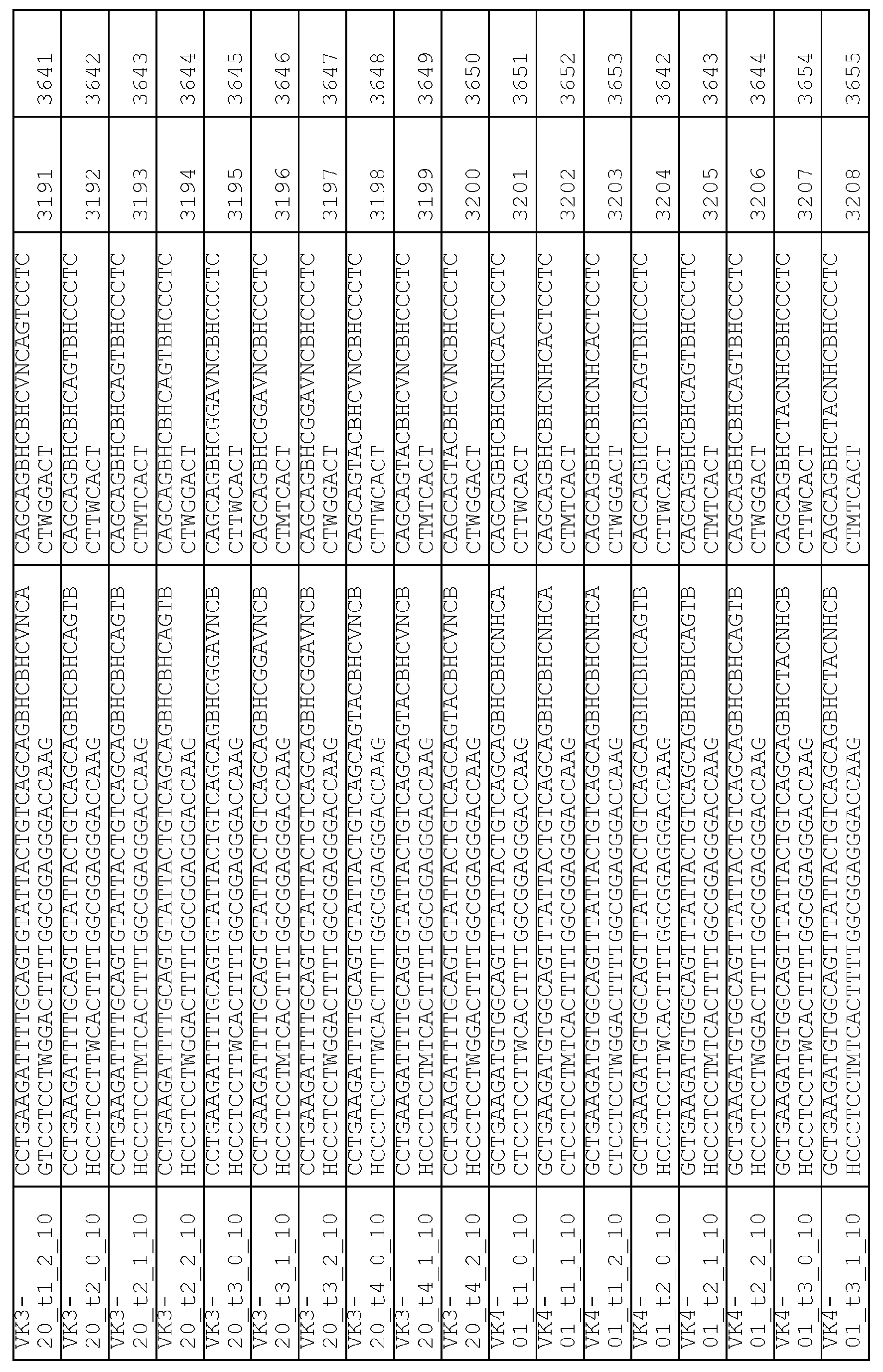

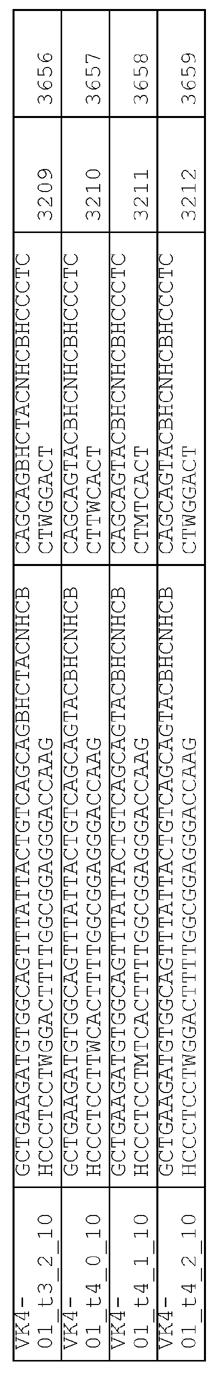

**Table 8. Number of unique CDRL3 amino acid sequences in exemplary jumping dimer ("JD") and jumping trimer ("JT") VK libraries and comparison to VK-v1.0.**

| | **L = 8 aa** | | **L = 9 aa** | | **L = 10 aa** | |
|---|---|---|---|---|---|---|
| **Jumping Dimer** | | | | | | |
| **Germline** | **JD** | **VK-v1.0** | **JD** | **VK-v1.0** | **JD** | **VK-v1.0** |
| **VK1-05** | 3549 | 3072 | 7098 | 6144 | 6084 | 13824 |
| **VK1-12** | 5250 | 2016 | 5250 | 2016 | 4500 | 5184 |
| **VK1-33** | 5502 | 4032 | 5502 | 4032 | 4716 | 10368 |
| **VK1-39** | 7224 | 3024 | 7224 | 3024 | 6192 | 7776 |
| **VK2-28** | 4396 | 2016 | 4396 | 2016 | 3768 | 5184 |
| **VK3-11** | 6048 | 2352 | 6048 | 2016 | 5184 | 6048 |
| **VK3-16** | 5789 | 2016 | 5789 | 2352 | 4962 | 5184 |
| **VK3-20** | 6405 | 2016 | 6671 | 2016 | 5490 | 5184 |
| **VK4-01** | NPE* | 2016 | 6405 | 2592 | NPE* | 5184 |
| | | | | | | |
| **Total** | **4.42*10⁴** | **2.26*10⁴** | **5.44*10⁴** | **2.62*10⁴** | **4.09*10⁴** | **6.39*10⁴** |
| | | | | | | |

| **Jumping Trimer** | | | | | | |
|---|---|---|---|---|---|---|
| **Germline** | **JT** | **VK-v1.0** | **JT** | **VK-v1.0** | **JT** | **VK-v1.0** |
| **VK1-05** | 7872 | 3072 | 13776 | 6144 | 11808 | 13824 |
| **VK1-12** | 14469 | 2016 | 14469 | 2016 | 12402 | 5184 |
| **VK1-33** | 15960 | 4032 | 15960 | 4032 | 13680 | 10368 |
| **VK1-39** | 28980 | 3024 | 28980 | 3024 | 24840 | 7776 |
| **VK2-28** | 12306 | 2016 | 12306 | 2016 | 10548 | 5184 |
| **VK3-11** | 18900 | 2352 | 18900 | 2016 | 16200 | 6048 |
| **VK3-15** | 18256 | 2016 | 18256 | 2352 | 15648 | 5184 |
| **VK3-20** | 23688 | 2016 | 23688 | 2016 | 20304 | 5184 |
| **VK4-01** | 23688 | 2016 | 23688 | 2592 | 20304 | 5184 |
| | | | | | | |
| **Total** | **1.64*10⁵** | **2.26*10⁴** | **1.70*10⁵** | **2.62*10⁴** | **1.46*10⁵** | **6.39*10⁴** |

| | | | | | | |
|---|---|---|---|---|---|---|
| * Not Presently Exemplified. However, given the teachings of the specification, a person of ordinary skill in the art could readily produce a library of such lengths, and these lengths are included within the scope of the invention. | | | | | | |

**Table 9. Matching output for exemplary CDRH3 sequences from the HPS and TSP1. Amino acid mismatches in the theoretical design are indicated in bold.**

| **Test Case** | **CDRH3 Sequence from HPS** | **SEQ ID NO** | **Mismatches** | **TN1** | **SEQ ID NO** | **DH** | **SEQ ID NO** | **N2** | **H3-JH** | **SEQ ID NO** |
|---|---|---|---|---|---|---|---|---|---|---|
| 1 | RTAHHFDY | 3660 | 0 | R | | TA | | H | HFDY | 4582 |
| 2.1 | VGIVGAASY | 3661 | 0 | V | | GIVGA | 3751 | AS | Y | |
| 2.2 | VGIVGAASY | 3661 | 0 | VG | | IVGA | 3755 | AS | Y | |
| 3.1 | DRYSGHDLGY | 3662 | 1 | DR | | YSGYD | 4389 | LG | Y | |
| 4.1 | GIAAADSNWLDP | 3663 | 1 | - | | GIAAA | 4448 | D | SNWFDP | 4600 |
| 4.2 | GIAAADSNWLDP | 3663 | 1 | - | | IAAA | 4452 | D | SNWFDP | 4600 |
| 5.1 | ERTINWGWGGVYAFDI | 3664 | 3 | EGTG | 3707 | NWG | | GGV | YAFDI | 4540 |
| 5.2 | ERTINWGWGGVYAFDI | 3664 | 3 | EGTG | 3707 | NWG | | WGT | YAFDI | 4540 |
| 5.3 | ERTINWGWGGVYAFDI | 3664 | 3 | ERGG | 3719 | NWG | | GGV | YAFDI | 4540 |
| 5.4 | ERTINWGWGGVYAFDI | 3664 | 3 | ERGG | 3719 | NWG | | WGT | YAFDI | 4540 |

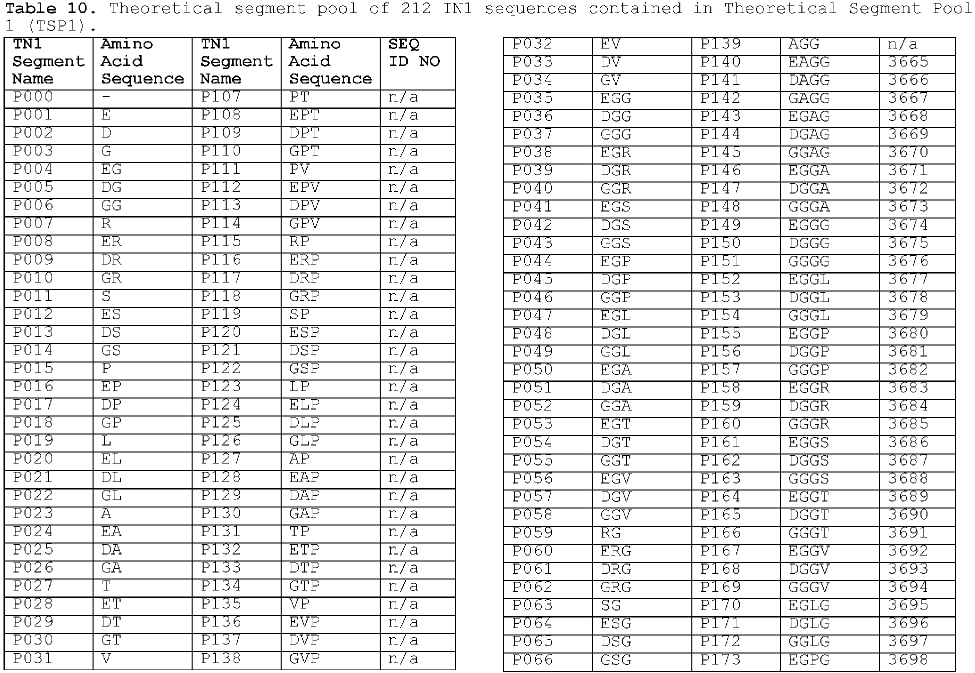

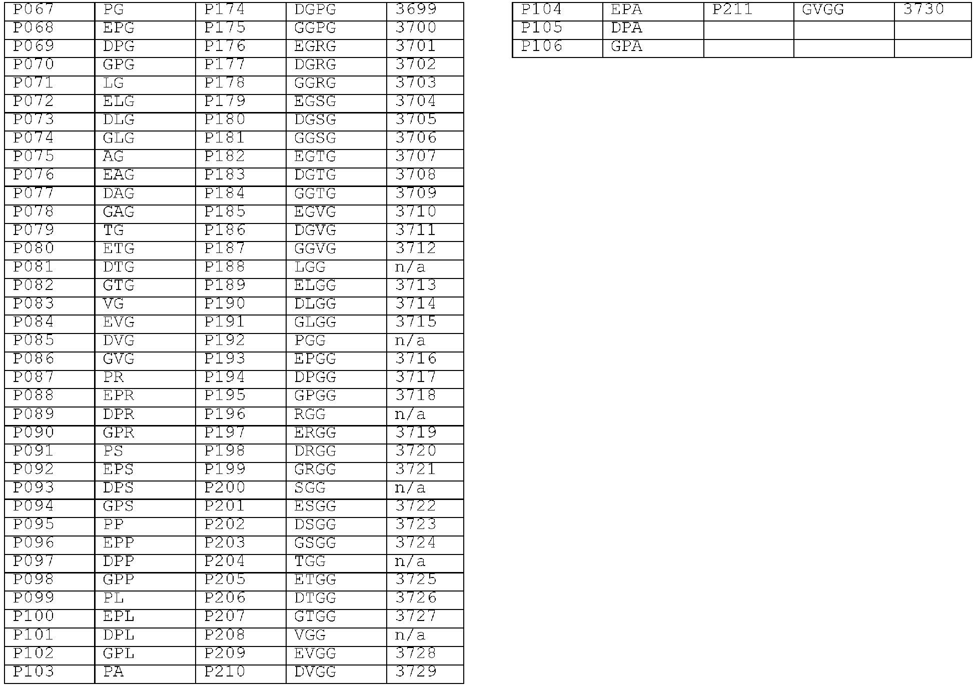

**Table 11. 1K DH Theoretical Segment Pool sequences (1,111 DH segments).**

| **DH Segment Name** | **Amino Acid Sequence** | **SEQ ID NO** | **DH Segment Name** | **Amino Acid Sequence** | **SEQ ID NO** |
|---|---|---|---|---|---|
| DHUNIV_001 | GTTGTT | 3731 | DHUNIV_557 | YDYV | 4107 |
| DHUNIV_002 | GTTGT | 3732 | DHUNIV_558 | DYVW | 4108 |
| DHUNIV_003 | TTGTT | 3733 | DHUNIV_559 | YVWG | 4109 |
| DHUNIV_004 | GTTG | 3734 | DHUNIV_560 | VWGS | 4110 |
| DHUNIV_005 | TTGT | 3735 | DHUNIV_561 | WGSY | 4111 |
| DHUNIV_006 | TGTT | 3736 | DHUNIV_562 | GSYA | 4112 |
| DHUNIV_007 | GTT | n/a | DHUNIV_563 | SYAY | 4113 |
| DHUNIV_008 | TTG | n/a | DHUNIV_564 | YAYT | 4114 |
| DHUNIV_009 | TGT | n/a | DHUNIV_565 | YYD | n/a |
| DHUNIV_010 | GT | n/a | DHUNIV_566 | YDY | n/a |
| DHUNIV_011 | TT | n/a | DHUNIV_567 | DYV | n/a |
| DHUNIV_012 | TG | n/a | DHUNIV_568 | YVW | n/a |
| DHUNIV_013 | VQLER | 3737 | DHUNIV_569 | VWG | n/a |
| DHUNIV_014 | VQLE | 3738 | DHUNIV_570 | WGS | n/a |
| DHUNIV_015 | QLER | 3739 | DHUNIV_571 | SYA | n/a |
| DHUNIV_016 | VQL | n/a | DHUNIV_572 | YAY | n/a |
| DHUNIV_017 | QLE | n/a | DHUNIV_573 | AYT | n/a |
| DHUNIV_018 | LER | n/a | DHUNIV_574 | YD | n/a |
| DHUNIV_019 | VQ | n/a | DHUNIV_575 | DY | n/a |
| DHUNIV_020 | QL | n/a | DHUNIV_576 | YV | n/a |
| DHUNIV_021 | LE | n/a | DHUNIV_577 | VW | n/a |
| DHUNIV_022 | ER | n/a | DHUNIV_578 | WG | n/a |
| DHUNIV_023 | YNWND | 3740 | DHUNIV_579 | IMITFGGVMLIP | 4115 |
| DHUNIV_024 | YNWN | 3741 | DHUNIV_580 | IMITFGGVMLI | 4116 |
| DHUNIV_025 | NWND | 3742 | DHUNIV_581 | MITFGGVMLIP | 4117 |
| DHUNIV_026 | YNW | n/a | DHUNIV_582 | IMITFGGVML | 4118 |
| DHUNIV_027 | NWN | n/a | DHUNIV_583 | MITFGGVMLI | 4119 |
| DHUNIV_028 | WND | n/a | DHUNIV_584 | ITFGGVMLIP | 4120 |
| DHUNIV_029 | YN | n/a | DHUNIV_585 | IMITFGGVM | 4121 |
| DHUNIV_030 | NW | n/a | DHUNIV_586 | MITFGGVML | 4122 |
| DHUNIV_031 | WN | n/a | DHUNIV_587 | ITFGGVMLI | 4123 |
| DHUNIV_032 | ND | n/a | DHUNIV_588 | TFGGVMLIP | 4124 |
| DHUNIV_033 | GITGTT | 3743 | DHUNIV_589 | IMITFGGV | 4125 |
| DHUNIV_034 | GITGT | 3744 | DHUNIV_590 | MITFGGVM | 4126 |
| DHUNIV_035 | ITGTT | 3745 | DHUNIV_591 | ITFGGVML | 4127 |
| DHUNIV_036 | GITG | 3746 | DHUNIV_592 | TFGGVMLI | 4128 |
| DHUNIV_037 | ITGT | 3747 | DHUNIV_593 | FGGVMLIP | 4129 |
| DHUNIV_038 | GIT | n/a | DHUNIV_594 | IMITFGG | 4130 |
| DHUNIV_039 | ITG | n/a | DHUNIV_595 | MITFGGV | 4131 |
| DHUNIV_040 | GI | n/a | DHUNIV_596 | ITFGGVM | 4132 |
| DHUNIV_041 | IT | n/a | DHUNIV_597 | TFGGVML | 4133 |
| DHUNIV_042 | GIVGATT | 3748 | DHUNIV_598 | FGGVMLI | 4134 |
| DHUNIV_043 | GIVGAT | 3749 | DHUNIV_599 | GGVMLIP | 4135 |
| DHUNIV_044 | IVGATT | 3750 | DHUNIV_600 | IMITFG | 4136 |
| DHUNIV_045 | GIVGA | 3751 | DHUNIV_601 | MITFGG | 4137 |
| DHUNIV_046 | IVGAT | 3752 | DHUNIV_602 | ITFGGV | 4138 |
| DHUNIV_047 | VGATT | 3753 | DHUNIV_603 | TFGGVM | 4139 |
| DHUNIV_048 | GIVG | 3754 | DHUNIV_604 | FGGVML | 4140 |
| DHUNIV_049 | IVGA | 3755 | DHUNIV_605 | GGVMLI | 4141 |
| DHUNIV_050 | VGAT | 3756 | DHUNIV_606 | GVMLIP | 4142 |
| DHUNIV_051 | GATT | 3757 | DHUNIV_607 | IMITF | 4143 |
| DHUNIV_052 | GIV | n/a | DHUNIV_608 | MITFG | 4144 |
| DHUNIV_053 | IVG | n/a | DHUNIV_609 | ITFGG | 4145 |
| DHUNIV_054 | VGA | n/a | DHUNIV_610 | TFGGV | 4146 |
| DHUNIV_055 | GAT | n/a | DHUNIV_611 | FGGVM | 4147 |
| DHUNIV_056 | ATT | n/a | DHUNIV_612 | GGVML | 4148 |
| DHUNIV_057 | IV | n/a | DHUNIV_613 | GVMLI | 4149 |
| DHUNIV_058 | VG | n/a | DHUNIV_614 | VMLIP | 4150 |
| DHUNIV_059 | GA | n/a | DHUNIV_615 | IMIT | 4151 |
| DHUNIV_060 | AT | n/a | DHUNIV_616 | MITF | 4152 |
| DHUNIV_061 | WELL | 3758 | DHUNIV_617 | ITFG | 4153 |
| DHUNIV_062 | WEL | n/a | DHUNIV_618 | TFGG | 4154 |
| DHUNIV_063 | ELL | n/a | DHUNIV_619 | FGGV | 4155 |
| DHUNIV_064 | WE | n/a | DHUNIV_620 | GGVM | 4156 |
| DHUNIV_065 | EL | n/a | DHUNIV_621 | GVML | 4157 |
| DHUNIV_066 | LL | n/a | DHUNIV_622 | VMLI | 4158 |
| DHUNIV_067 | YSGSYY | 3759 | DHUNIV_623 | MLIP | 4159 |
| DHUNIV_068 | YSGSY | 3760 | DHUNIV_624 | IMI | n/a |
| DHUNIV_069 | SGSYY | 7361 | DHUNIV_625 | MIT | n/a |
| DHUNIV_070 | YSGS | 3762 | DHUNIV_626 | ITF | n/a |
| DHUNIV_071 | SGSY | 3763 | DHUNIV_627 | TFG | n/a |
| DHUNIV_072 | GSYY | 3764 | DHUNIV_628 | FGG | n/a |
| DHUNIV_073 | YSG | n/a | DHUNIV_629 | GGV | n/a |
| DHUNIV_074 | SGS | n/a | DHUNIV_630 | GVM | n/a |
| DHUNIV_075 | GSY | n/a | DHUNIV_631 | VML | n/a |
| DHUNIV_076 | SYY | n/a | DHUNIV_632 | MLI | n/a |
| DHUNIV_077 | YS | n/a | DHUNIV_633 | LIP | n/a |
| DHUNIV_078 | SG | n/a | DHUNIV_634 | IM | n/a |
| DHUNIV_079 | GS | n/a | DHUNIV_635 | MI | n/a |
| DHUNIV_080 | SY | n/a | DHUNIV_636 | TF | n/a |
| DHUNIV_081 | YY | n/a | DHUNIV_637 | VM | n/a |
| DHUNIV_082 | LEL | n/a | DHUNIV_638 | LI | n/a |
| DHUNIV_083 | YNWNY | 3765 | DHUNIV_639 | WLLL | 4160 |
| DHUNIV_084 | NWNY | 3766 | DHUNIV_640 | WLL | n/a |
| DHUNIV_085 | WNY | n/a | DHUNIV_641 | WL | n/a |
| DHUNIV_086 | NY | n/a | DHUNIV_642 | YYYDSSGYYY | 4161 |
| DHUNIV_087 | RIL | n/a | DHUNIV_643 | YYYDSSGYY | 4162 |
| DHUNIV_088 | LLL | n/a | DHUNIV_644 | YYDSSGYYY | 4163 |
| DHUNIV_089 | RI | n/a | DHUNIV_645 | YYYDSSGY | 4164 |
| DHUNIV_090 | IL | n/a | DHUNIV_646 | YYDSSGYY | 4165 |
| DHUNIV_091 | WW | n/a | DHUNIV_647 | YDSSGYYY | 4166 |
| DHUNIV_092 | GYCSGGSCYS | 3767 | DHUNIV_648 | YYYDSSG | 4167 |
| DHUNIV_093 | GYCSGGSCY | 3768 | DHUNIV_649 | YYDSSGY | 4168 |
| DHUNIV_094 | YCSGGSCYS | 3769 | DHUNIV_650 | YDSSGYY | 4169 |
| DHUNIV_095 | GYCSGGSC | 3770 | DHUNIV_651 | DSSGYYY | 4170 |
| DHUNIV_096 | YCSGGSCY | 3771 | DHUNIV_652 | YYYDSS | 4171 |
| DHUNIV_097 | CSGGSCYS | 3772 | DHUNIV_653 | YYDSSG | 4172 |
| DHUNIV_098 | YCSGGSC | 3773 | DHUNIV_654 | YDSSGY | 4173 |
| DHUNIV_099 | CSGGSCY | 3774 | DHUNIV_655 | DSSGYY | 4174 |
| DHUNIV_100 | CSGGSC | 3775 | DHUNIV_656 | SSGYYY | 4175 |
| DHUNIV_101 | SGGS | 3776 | DHUNIV_657 | YYYDS | 4176 |
| DHUNIV_102 | SGG | n/a | DHUNIV_658 | YYDSS | 4177 |
| DHUNIV_103 | GGS | n/a | DHUNIV_659 | YDSSG | 4178 |
| DHUNIV_104 | GY | n/a | DHUNIV_660 | DSSGY | 4179 |
| DHUNIV_105 | GG | n/a | DHUNIV_661 | SSGYY | 4180 |
| DHUNIV_106 | DIVVVVAATP | 3777 | DHUNIV_662 | SGYYY | 4181 |
| DHUNIV_107 | DIVVVVAAT | 3778 | DHUNIV_663 | YYYD | 4182 |
| DHUNIV_108 | IVVVVAATP | 3779 | DHUNIV_664 | YYDS | 4183 |
| DHUNIV_109 | DIVVVVAA | 3780 | DHUNIV_665 | YDSS | 4184 |
| DHUNIV_110 | IVVVVAAT | 3781 | DHUNIV_666 | DSSG | 418b |
| DHUNIV_111 | VVVVAATP | 3782 | DHUNIV_667 | SSGY | 4186 |
| DHUNIV_112 | DIVVVVA | 3783 | DHUNIV_668 | SGYY | 4187 |
| DHUNIV_113 | IVVVVAA | 3784 | DHUNIV_669 | GYYY | 4188 |
| DHUNIV_114 | VVVVAAT | 3785 | DHUNIV_670 | YDS | n/a |
| DHUNIV_115 | VVVAATP | 3786 | DHUNIV_671 | DSS | n/a |
| DHUNIV_116 | DIVVVV | 3787 | DHUNIV_672 | SSG | n/a |
| DHUNIV_117 | IVVVVA | 3788 | DHUNIV_673 | SGY | n/a |
| DHUNIV_118 | VVVVAA | 3789 | DHUNIV_674 | GYY | n/a |
| DHUNIV_119 | VVVAAT | 3790 | DHUNIV_675 | DS | n/a |
| DHUNIV_120 | VVAATP | 3791 | DHUNIV_676 | ITMIVVVITT | 4189 |
| DHUNIV_121 | DIVVV | 3792 | DHUNIV_677 | ITMIVVVIT | 4190 |
| DHUNIV_122 | IVVVV | 3793 | DHUNIV_678 | TMIVVVITT | 4191 |
| DHUNIV_123 | VVVVA | 3794 | DHUNIV_679 | ITMIVVVI | 4192 |
| DHUNIV_124 | VVVAA | 3795 | DHUNIV_680 | TMIVVVIT | 4193 |
| DHUNIV_125 | VVAAT | 3796 | DHUNIV_681 | MIVVVITT | 4194 |
| DHUNIV_126 | VAATP | 3797 | DHUNIV_682 | ITMIVVV | 4195 |
| DHUNIV_127 | DIVV | 3798 | DHUNIV_683 | TMIVVVI | 4196 |
| DHUNIV_128 | IVVV | 3799 | DHUNIV_684 | MIVVVIT | 4197 |
| DHUNIV_129 | VVVV | 3800 | DHUNIV_685 | IVVVITT | 4198 |
| DHUNIV_130 | VVVA | 3801 | DHUNIV_686 | ITMIVV | 4199 |
| DHUNIV_131 | VVAA | 3802 | DHUNIV_687 | TMIVVV | 4200 |
| DHUNIV_132 | VAAT | 3803 | DHUNIV_688 | MIVVVI | 4201 |
| DHUNIV_133 | AATP | 3804 | DHUNIV_689 | IVVVIT | 4202 |
| DHUNIV_134 | DIV | n/a | DHUNIV_690 | VVVITT | 4203 |
| DHUNIV_135 | IVV | n/a | DHUNIV_691 | ITMIV | 4204 |
| DHUNIV_136 | VVV | n/a | DHUNIV_692 | TMIVV | 4205 |
| DHUNIV_137 | VVA | n/a | DHUNIV_693 | MIVVV | 4206 |
| DHUNIV_138 | VAA | n/a | DHUNIV_694 | VVVIT | 4207 |
| DHUNIV_139 | AAT | n/a | DHUNIV_695 | VVITT | 4208 |
| DHUNIV_140 | ATP | n/a | DHUNIV_696 | ITMI | 4209 |
| DHUNIV_141 | DI | n/a | DHUNIV_697 | TMIV | 4210 |
| DHUNIV_142 | VV | n/a | DHUNIV_698 | MIVV | 4211 |
| DHUNIV_143 | VA | n/a | DHUNIV_699 | VVIT | 4212 |
| DHUNIV_144 | AA | n/a | DHUNIV_700 | VITT | 4213 |
| DHUNIV_145 | TP | n/a | DHUNIV_701 | TMI | n/a |
| DHUNIV_14b | YQLL | 3805 | DHUNIV_702 | MIV | n/a |
| DHUNIV_147 | YQL | n/a | DHUNIV_703 | VIT | n/a |
| DHUNIV_148 | QLL | n/a | DHUNIV_704 | ITT | n/a |
| DHUNIV_149 | YQ | n/a | DHUNIV_705 | VLRFLEWLLY | 4214 |
| DHUNIV-150 | GYCSSTSCYA | 3806 | DHUNIV_706 | VLRFLEWLL | 4215 |
| DHUNIV_151 | GYCSSTSCY | 3807 | DHUNIV_707 | LRFLEWLLY | 4216 |
| DHUNIV_152 | YCSSTSCYA | 3808 | DHUNIV_708 | VLRFLEWL | 4217 |
| DHUNIV_153 | GYCSSTSC | 3809 | DHUNIV_709 | LRFLEWLL | 4218 |
| DHUNIV_154 | YCSSTSCY | 3810 | DHUNIV_710 | RFLEWLLY | 4219 |
| DHUNIV_155 | CSSTSCYA | 3811 | DHUNIV_711 | VLRFLEW | 4220 |
| DHUNIV_156 | YCSSTSC | 3812 | DHUNIV_712 | LRFLEWL | 4221 |
| DHUNIV_157 | CSSTSCY | 3813 | DHUNIV_713 | RFLEWLL | 4222 |
| DHUNIV_158 | CSSTSC | 3814 | DHUNIV_714 | FLEWLLY | 4223 |
| DHUNIV_159 | SSTS | 3815 | DHUNIV_715 | VLRFLE | 4224 |
| DHUNIV_160 | SST | n/a | DHUNIV_716 | LRFLEW | 4225 |
| DHUNIV_161 | STS | n/a | DHUNIV_717 | RFLEWL | 4226 |
| DHUNIV_162 | SS | n/a | DHUNIV_718 | FLEWLL | 4227 |
| DHUNIV_163 | ST | n/a | DHUNIV_719 | LEWLLY | 4228 |
| DHUNIV_164 | TS | n/a | DHUNIV_720 | VLRFL | 4229 |
| DHUNIV_165 | YA | n/a | DHUNIV_721 | LRFLE | 4230 |
| DHUNIV_166 | DIVVVPAAMP | 3816 | DHUNIV_722 | RFLEW | 4231 |
| DHUNIV_167 | DIVVVPAAM | 3817 | DHUNIV_723 | FLEWL | 4232 |
| DHUNIV_168 | IVVVPAAMP | 3818 | DHUNIV_724 | LEWLL | 4233 |
| DHUNIV_169 | DIVVVPAA | 3819 | DHUNIV_725 | EWLLY | 4234 |
| DHUNIV_170 | IVVVPAAM | 3820 | DHUNIV_726 | VLRF | 4235 |
| DHUNIV_171 | VVVPAAMP | 3821 | DHUNIV_727 | LRFL | 4236 |
| DHUNIV_172 | DIVVVPA | 3822 | DHUNIV_728 | RFLE | 4237 |
| DHUNIV_173 | IVVVPAA | 3823 | DHUNIV_729 | FLEW | 4238 |
| DHUNIV_174 | VVVPAAM | 3824 | DHUNIV_730 | LEWL | 4239 |
| DHUNIV_175 | VVPAAMP | 3825 | DHUNIV_731 | EWLL | 4240 |
| DHUNIV_176 | DIVVVP | 3826 | DHUNIV_732 | WLLY | 4241 |
| DHUNIV_177 | IVVVPA | 3827 | DHUNIV_733 | VLR | n/a |
| DHUNIV_178 | VVVPAA | 3828 | DHUNIV_734 | LRF | n/a |
| DHUNIV_179 | VVPAAM | 3829 | DHUNIV_735 | RFL | n/a |
| DHUNIV_180 | VPAAMP | 3830 | DHUNIV_736 | FLE | n/a |
| DHUNIV_181 | IVVVP | 3831 | DHUNIV_737 | LEW | n/a |
| DHUNIV_182 | VVVPA | 3832 | DHUNIV_738 | EWL | n/a |
| DHUNIV_183 | VVPAA | 3833 | DHUNIV_739 | RF | n/a |
| DHUNIV_184 | VPAAM | 3834 | DHUNIV_740 | FL | n/a |
| DHUNIV_185 | PAAMP | 3835 | DHUNIV_741 | EW | n/a |
| DHUNIV_186 | VVVP | 3836 | DHUNIV_742 | YYDFWSGYYT | 4242 |
| DHUNIV_187 | VVPA | 3837 | DHUNIV_743 | YYDFWSGYY | 4243 |
| DHUNIV_188 | VPAA | 3838 | DHUNIV_744 | YDFWSGYYT | 4244 |
| DHUNIV_189 | PAAM | 3839 | DHUNIV_745 | YYDFWSGY | 4245 |
| DHUNIV_190 | AAMP | 3840 | DHUNIV_746 | YDFWSGYY | 4246 |
| DHUNIV_191 | VVP | n/a | DHUNIV_747 | DFWSGYYT | 4247 |
| DHUNIV_192 | VPA | n/a | DHUNIV_748 | YYDFWSG | 4248 |
| DHUNIV_193 | PAA | n/a | DHUNIV_749 | YDFWSGY | 4249 |
| DHUNIV_194 | AAM | n/a | DHUNIV_750 | DFWSGYY | 4250 |
| DHUNIV_195 | AMP | n/a | DHUNIV_751 | FWSGYYT | 4251 |
| DHUNIV_196 | VP | n/a | DHUNIV_752 | YYDFWS | 4252 |
| DHUNIV_197 | PA | n/a | DHUNIV_753 | YDFWSG | 4253 |
| DHUNIV_198 | AM | n/a | DHUNIV_754 | DFWSGY | 4254 |
| DHUNIV_199 | MP | n/a | DHUNIV_755 | FWSGYY | 4255 |
| DHUNIV_200 | YQLLY | 3841 | DHUNIV_756 | WSGYYT | 4256 |
| DHUNIV_201 | QLLY | 3842 | DHUNIV_757 | YYDFW | 4257 |
| DHUNIV_202 | LLY | n/a | DHUNIV_758 | YDFWS | 4258 |
| DHUNIV_203 | LY | n/a | DHUNIV_759 | DFWSG | 4259 |
| DHUNIV_204 | GYCSSTSCYT | 3843 | DHUNIV_760 | FWSGY | 4260 |
| DHUNIV_205 | YCSSTSCYT | 3844 | DHUNIV_761 | WSGYY | 4261 |
| DHUNIV_206 | CSSTSCYT | 3845 | DHUNIV_762 | SGYYT | 4262 |
| DHUNIV_207 | YT | n/a | DHUNIV_763 | YYDF | 4263 |
| DHUNIV_208 | DIVVVPAAIP | 3846 | DHUNIV_764 | YDFW | 4264 |
| DHUNIV_209 | DIVVVPAAI | 3847 | DHUNIV_765 | DFWS | 4265 |
| DHUNIV_210 | IVVVPAAIP | 3848 | DHUNIV_766 | FWSG | 4266 |
| DHUNIV_211 | IVVVPAAI | 3849 | DHUNIV_767 | WSGY | 4267 |
| DHUNIV_212 | VVVPAAIP | 3850 | DHUNIV_768 | GYYT | 4268 |
| DHUNIV_213 | VVVPAAI | 3851 | DHUNIV_769 | YDF | n/a |
| DHUNIV 214 | VVPAAIP | 3852 | DHUNIV_770 | DFW | n/a |
| DHUNIV_215 | VVPAAI | 3853 | DHUNIV_771 | FWS | n/a |
| DHUNIV_216 | VPAAIP | 3854 | DHUNIV_772 | WSG | n/a |
| DHUNIV_217 | VPAAI | 3855 | DHUNIV_773 | YYT | n/a |
| DHUNIV_218 | PAAIP | 3856 | DHUNIV_774 | DF | n/a |
| DHUNIV_219 | PAAI | 3857 | DHUNIV_775 | FW | n/a |
| DHUNIV_220 | AAIP | 3858 | DHUNIV_776 | WS | n/a |
| DHUNIV_221 | AAI | n/a | DHUNIV_777 | ITIFGVVIIP | 4269 |
| DHUNIV_222 | AIP | n/a | DHUNIV_778 | ITIFGVVII | 4270 |
| DHUNIV_223 | AI | n/a | DHUNIV_779 | TIFGVVIIP | 4271 |
| DHUNIV_224 | IP | n/a | DHUNIV_780 | ITIFGVVI | 4272 |
| DHUNIV_225 | WIL | n/a | DHUNIV_781 | TIFGVVII | 4273 |
| DHUNIV_226 | WI | n/a | DHUNIV_782 | IFGVVIIP | 4274 |
| DHUNIV_227 | SILWW | 3859 | DHUNIV_783 | ITIFGVV | 4275 |
| DHUNIV_228 | SILW | 3860 | DHUNIV_784 | TIFGVVI | 4276 |
| DHUNIV_229 | ILWW | 3861 | DHUNIV_785 | IFGVVII | 4277 |
| DHUNIV_230 | SIL | n/a | DHUNIV_786 | FGVVIIP | 4278 |
| DHUNIV_231 | ILW | n/a | DHUNIV_787 | ITIFGV | 4279 |
| DHUNIV_232 | LWW | n/a | DHUNIV_788 | TIFGVV | 4280 |
| DHUNIV_233 | LLF | n/a | DHUNIV_789 | IFGVVI | 4281 |
| DHUNIV_234 | SI | n/a | DHUNIV_790 | FGVVII | 4282 |
| DHUNIV_235 | LW | n/a | DHUNIV_791 | GVVIIP | 4283 |
| DHUNIV_236 | LF | n/a | DHUNIV_792 | ITIFG | 4284 |
| DHUNIV_237 | AYCGGDCYS | 3862 | DHUNIV_793 | TIFGV | 4285 |
| DHUNIV_238 | AYCGGDCY | 3863 | DHUNIV_794 | IFGVV | 4286 |
| DHUNIV_239 | YCGGDCYS | 3864 | DHUNIV_795 | FGVVI | 4287 |
| DHUNIV_240 | AYCGGDC | 3865 | DHUNIV_796 | GVVII | 4288 |
| DHUNIV_241 | YCGGDCY | 3866 | DHUNIV_797 | VVIIP | 4289 |
| DHUNIV_242 | CGGDCYS | 3867 | DHUNIV_798 | ITIF | 4290 |
| DHUNIV_243 | YCGGDC | 3868 | DHUNIV_799 | TIFG | 4291 |
| DHUNIV_244 | CGGDCY | 3869 | DHUNIV_800 | IFGV | 4292 |
| DHUNIV_245 | CGGDC | 3870 | DHUNIV_801 | FGVV | 4293 |
| DHUNIV_246 | GGD | n/a | DHUNIV_802 | GVVI | 4294 |
| DHUNIV_247 | AY | n/a | DHUNIV_803 | VVII | 4295 |
| DHUNIV_248 | GD | n/a | DHUNIV_804 | VIIP | 4296 |
| DHUNIV_249 | HIVVVIAIP | 3871 | DHUNIV_805 | ITI | n/a |
| DHUNIV_250 | HIVVVIAI | 3872 | DHUNIV_806 | TIF | n/a |
| DHUNIV_251 | IVVVIAIP | 3873 | DHUNIV_807 | IFG | n/a |
| DHUNIV_252 | HIVVVIA | 3874 | DHUNIV_808 | FGV | n/a |
| DHUNIV_253 | IVVVIAI | 3875 | DHUNIV_809 | GVV | n/a |
| DHUNIV_254 | VVVIAIP | 3876 | DHUNIV_810 | IIP | n/a |
| DHUNIV_255 | HIVVVI | 3877 | DHUNIV_811 | TI | n/a |
| DHUNIV_256 | IVVVIA | 3878 | DHUNIV_812 | IF | n/a |
| DHUNIV_257 | VVVIAI | 3879 | DHUNIV_813 | VLRYFDWLL | 4297 |
| DHUNIV_258 | VVIAIP | 3880 | DHUNIV_814 | VLRYFDWL | 4298 |
| DHUNIV_259 | HIVVV | 3881 | DHUNIV_815 | LRYFDWLL | 4299 |
| DHUNIV_260 | IVVVI | 3882 | DHUNIV_816 | VLRYFDW | 4300 |
| DHUNIV_261 | VVVIA | 3883 | DHUNIV_817 | LRYFDWL | 4301 |
| DHUNIV_262 | VVIAI | 3884 | DHUNIV_818 | RYFDWLL | 4302 |
| DHUNIV_263 | VIAIP | 3885 | DHUNIV_819 | VLRYFD | 4303 |
| DHUNIV_264 | HIVV | 3886 | DHUNIV_820 | LRYFDW | 4304 |
| DHUNIV_265 | VVVI | 3887 | DHUNIV_821 | RYFDWL | 4305 |
| DHUNIV_266 | VVIA | 3888 | DHUNIV_822 | YFDWLL | 4306 |
| DHUNIV_267 | VIAI | 3889 | DHUNIV_823 | VLRYF | 4307 |
| DHUNIV_268 | IAIP | 3890 | DHUNIV_824 | LRYFD | 4308 |
| DHUNIV_269 | HIV | n/a | DHUNIV_825 | RYFDW | 4309 |
| DHUNIV_270 | VVI | n/a | DHUNIV_826 | YFDWL | 4310 |
| DHUNIV_271 | VIA | n/a | DHUNIV_827 | FDWLL | 4311 |
| DHUNIV_272 | IAI | n/a | DHUNIV_828 | VLRY | 4312 |
| DHUNIV_273 | HI | n/a | DHUNIV_829 | LRYF | 4313 |
| DHUNIV_274 | VI | n/a | DHUNIV_830 | RYFD | 4314 |
| DHUNIV_275 | IA | n/a | DHUNIV_831 | YFDW | 4315 |
| DHUNIV_276 | HIVVVTAIP | 3891 | DHUNIV_832 | FDWL | 4316 |
| DHUNIV_277 | HIVVVTAI | 3892 | DHUNIV_833 | DWLL | 4317 |
| DHUNIV_278 | IVVVTAIP | 3893 | DHUNIV_834 | LRY | n/a |
| DHUNIV_279 | HIVVVTA | 3894 | DHUNIV_835 | RYF | n/a |
| DHUNIV_280 | IVVVTAI | 3895 | DHUNIV_836 | YFD | n/a |
| DHUNIV_281 | VVVTAIP | 3896 | DHUNIV_837 | FDW | n/a |
| DHUNIV_282 | HIVVVT | 3897 | DHUNIV_838 | DWL | n/a |
| DHUNIV_283 | IVVVTA | 3898 | DHUNIV_839 | RY | n/a |
| DHUNIV_284 | VVVTAI | 3899 | DHUNIV_840 | YF | n/a |
| DHUNIV_285 | VVTAIP | 3900 | DHUNIV_841 | FD | n/a |
| DHUNIV_286 | IVVVT | 3901 | DHUNIV_842 | DW | n/a |
| DHUNIV_287 | VVVTA | 3902 | DHUNIV_843 | YYDILTGYYN | 4318 |
| DHUNIV_288 | VVTAI | 3903 | DHUNIV_844 | YYDILTGYY | 4319 |
| DHUNIV_289 | VTAIP | 3904 | DHUNIV_845 | YDILTGYYN | 4320 |
| DHUNIV_290 | VVVT | 3905 | DHUNIV_846 | YYDILTGY | 4321 |
| DHUNIV_291 | VVTA | 3906 | DHUNIV_847 | YDILTGYY | 4322 |
| DHUNIV_292 | VTAI | 3907 | DHUNIV_848 | DILTGYYN | 4323 |
| DHUNIV_293 | TAIP | 3908 | DHUNIV_849 | YYDILTG | 4324 |
| DHUNIV_294 | VVT | n/a | DHUNIV_850 | YDILTGY | 4325 |
| DHUNIV_295 | VTA | n/a | DHUNIV_851 | DILTGYY | 4326 |
| DHUNIV_296 | TAI | n/a | DHUNIV_852 | ILTGYYN | 4327 |
| DHUNIV_297 | VT | n/a | DHUNIV_853 | YYDILT | 4328 |
| DHUNIV_298 | TA | n/a | DHUNIV_854 | YDILTG | 4329 |
| DHUNIV_299 | RILY | 3909 | DHUNIV_855 | DILTGY | 4330 |
| DHUNIV_300 | ILY | n/a | DHUNIV_856 | ILTGYY | 4331 |
| DHUNIV_301 | MLY | n/a | DHUNIV_857 | LTGYYN | 4332 |
| DHUNIV_302 | ML | n/a | DHUNIV_858 | YYDIL | 4333 |
| DHUNIV_303 | GYCTNGVCYT | 3910 | DHUNIV_859 | YDILT | 4334 |
| DHUNIV_304 | GYCTNGVCY | 3911 | DHUNIV_860 | DILTG | 433b |
| DHUNIV_305 | YCTNGVCYT | 3912 | DHUNIV_861 | ILTGY | 4336 |
| DHUNIV_306 | GYCTNGVC | 3913 | DHUNIV_862 | LTGYY | 4337 |
| DHUNIV_307 | YCTNGVCY | 3914 | DHUNIV_863 | TGYYN | 4338 |
| DHUNIV_308 | CTNGVCYT | 3915 | DHUNIV_864 | YYDI | 4339 |
| DHUNIV_309 | YCTNGVC | 3916 | DHUNIV_865 | YDIL | 4340 |
| DHUNIV_310 | CTNGVCY | 3917 | DHUNIV_866 | DILT | 4341 |
| DHUNIV_311 | CTNGVC | 3918 | DHUNIV_867 | ILTG | 4342 |
| DHUNIV_312 | TNGV | 3919 | DHUNIV_868 | LTGY | 4343 |
| DHUNIV_313 | TNG | n/a | DHUNIV_869 | TGYY | 4344 |
| DHUNIV_314 | NGV | n/a | DHUNIV_870 | GYYN | 4345 |
| DHUNIV_315 | TN | n/a | DHUNIV_871 | YDI | n/a |
| DHUNIV_316 | NG | n/a | DHUNIV_872 | DIL | n/a |
| DHUNIV_317 | GV | n/a | DHUNIV_873 | ILT | n/a |
| DHUNIV_318 | DIVLMVYAIP | 3920 | DHUNIV_874 | LTG | n/a |
| DHUNIV_319 | DIVLMVYAI | 3921 | DHUNIV_875 | TGY | n/a |
| DHUNIV_320 | IVLMVYAIP | 3922 | DHUNIV_876 | LT | n/a |
| DHUNIV_321 | DIVLMVYA | 3923 | DHUNIV_877 | LVIIT | 4346 |
| DHUNIV_322 | IVLMVYAI | 3924 | DHUNIV_878 | LVII | 4347 |
| DHUNIV_323 | VLMVYAIP | 3925 | DHUNIV_879 | LVI | n/a |
| DHUNIV_324 | DIVLMVY | 3926 | DHUNIV_880 | LV | n/a |
| DHUNIV_325 | IVLMVYA | 3927 | DHUNIV_881 | DYGDY | 4348 |
| DHUNIV_326 | VLMVYAI | 3928 | DHUNIV_882 | DYGD | 4349 |
| DHUNIV_327 | LMVYAIP | 3929 | DHUNIV_883 | YGDY | 4350 |
| DHUNIV_328 | DIVLMV | 3930 | DHUNIV_884 | DYG | n/a |
| DHUNIV_329 | IVLMVY | 3931 | DHUNIV_885 | YGD | n/a |
| DHUNIV_330 | VLMVYA | 3932 | DHUNIV_886 | GDY | n/a |
| DHUNIV_331 | LMVYAI | 3933 | DHUNIV_887 | TTVTT | 4351 |
| DHUNIV_332 | MVYAIP | 3934 | DHUNIV_888 | TTVT | 4352 |
| DHUNIV_333 | DIVLM | 393b | DHUNIV_889 | TVTT | 4353 |
| DHUNIV_334 | IVLMV | 3936 | DHUNIV_890 | TTV | n/a |
| DHUNIV_335 | VLMVY | 3937 | DHUNIV_891 | TVT | n/a |
| DHUNIV_336 | LMVYA | 3938 | DHUNIV_892 | VTT | n/a |
| DHUNIV_337 | MVYAI | 3939 | DHUNIV_893 | TV | n/a |
| DHUNIV_338 | VYAIP | 3940 | DHUNIV_894 | LRW | n/a |
| DHUNIV_339 | DIVL | 3941 | DHUNIV_895 | RW | n/a |
| DHUNIV_340 | IVLM | 3942 | DHUNIV_896 | DYGGNS | 4354 |
| DHUNIV_341 | VLMV | 3943 | DHUNIV_897 | DYGGN | 4355 |
| DHUNIV_342 | LMVY | 3944 | DHUNIV_898 | YGGNS | 4356 |
| DHUNIV_343 | MVYA | 3945 | DHUNIV_899 | DYGG | 4357 |
| DHUNIV_344 | VYAI | 3946 | DHUNIV_900 | YGGN | 4358 |
| DHUNIV_345 | YAIP | 3947 | DHUNIV_901 | GGNS | 4359 |
| DHUNIV_346 | IVL | n/a | DHUNIV_902 | YGG | n/a |
| DHUNIV_347 | VLM | n/a | DHUNIV_903 | GGN | n/a |
| DHUNIV_348 | LMV | n/a | DHUNIV_904 | GNS | n/a |
| DHUNIV_349 | MVY | n/a | DHUNIV_905 | GN | n/a |
| DHUNIV_350 | VYA | n/a | DHUNIV_906 | NS | n/a |
| DHUNIV_351 | YAI | n/a | DHUNIV_907 | TTVVTP | 4360 |
| DHUNIV_352 | VL | n/a | DHUNIV_908 | TTVVT | 4361 |
| DHUNIV_353 | LM | n/a | DHUNIV_909 | TVVTP | 4362 |
| DHUNIV_354 | MV | n/a | DHUNIV_910 | TTVV | 4363 |
| DHUNIV_355 | VY | n/a | DHUNIV_911 | TVVT | 4364 |
| DHUNIV_356 | VLLWFGELL | 3948 | DHUNIV_912 | VVTP | 4365 |
| DHUNIV_357 | VLLWFGEL | 3949 | DHUNIV_913 | TVV | n/a |
| DHUNIV_358 | LLWFGELL | 3950 | DHUNIV_914 | VTP | n/a |
| DHUNIV_359 | VLLWFGE | 3951 | DHUNIV_915 | LQ | n/a |
| DHUNIV_360 | LLWFGEL | 3952 | DHUNIV_916 | DYSNY | 4366 |
| DHUNIV_361 | LWFGELL | 3953 | DHUNIV_917 | DYSN | 4367 |
| DHUNIV_362 | VLLWFG | 3954 | DHUNIV_918 | YSNY | 4368 |
| DHUNIV_363 | LLWFGE | 3955 | DHUNIV_919 | DYS | n/a |
| DHUNIV_364 | LWFGEL | 3956 | DHUNIV_920 | YSN | n/a |
| DHUNIV_365 | WFGELL | 3957 | DHUNIV_921 | SNY | n/a |
| DHUNIV_366 | VLLWF | 3958 | DHUNIV_922 | SN | n/a |
| DHUNIV_367 | LLWFG | 3959 | DHUNIV_923 | VDIVATIT | 4369 |
| DHUNIV_368 | LWFGE | 3960 | DHUNIV_924 | VDIVATI | 4370 |
| DHUNIV_369 | WFGEL | 3961 | DHUNIV_925 | DIVATIT | 4371 |
| DHUNIV_370 | FGELL | 3962 | DHUNIV_926 | VDIVAT | 4372 |
| DHUNIV_371 | VLLW | 3963 | DHUNIV_927 | DIVATI | 4373 |
| DHUNIV_372 | LLWF | 3964 | DHUNIV_928 | IVATIT | 4374 |
| DHUNIV_373 | LWFG | 3965 | DHUNIV_929 | VDIVA | 4375 |
| DHUNIV_374 | WFGE | 3966 | DHUNIV_930 | DIVAT | 4376 |
| DHUNIV_375 | FGEL | 3967 | DHUNIV_931 | IVATI | 4377 |
| DHUNIV_376 | GELL | 3968 | DHUNIV_932 | VATIT | 4378 |
| DHUNIV_377 | VLL | n/a | DHUNIV_933 | VDIV | 4379 |
| DHUNIV_378 | LLW | n/a | DHUNIV_934 | DIVA | 4380 |
| DHUNIV_379 | LWF | n/a | DHUNIV_935 | IVAT | 4381 |
| DHUNIV_380 | WFG | n/a | DHUNIV_936 | VATI | 4382 |
| DHUNIV_381 | FGE | n/a | DHUNIV_937 | ATIT | 4383 |
| DHUNIV_382 | GEL | n/a | DHUNIV_938 | VDI | n/a |
| DHUNIV_383 | WF | n/a | DHUNIV_939 | IVA | n/a |
| DHUNIV_384 | FG | n/a | DHUNIV_940 | VAT | n/a |
| DHUNIV_385 | GE | n/a | DHUNIV_941 | ATI | n/a |
| DHUNIV_386 | YYYGSGSYYN | 3969 | DHUNIV_942 | TIT | n/a |
| DHUNIV_387 | YYYGSGSYY | 3970 | DHUNIV_943 | VD | n/a |
| DHUNIV_388 | YYGSGSYYN | 3971 | DHUNIV_944 | WLRL | 4384 |
| DHUNIV_389 | YYYGSGSY | 3972 | DHUNIV_945 | WLR | n/a |
| DHUNIV_390 | YYGSGSYY | 3973 | DHUNIV_946 | GYSGYDY | 438b |
| DHUNIV_391 | YGSGSYYN | 3974 | DHUNIV_947 | GYSGYD | 4386 |
| DHUNIV_392 | YYYGSGS | 3975 | DHUNIV_948 | YSGYDY | 4387 |
| DHUNIV_393 | YYGSGSY | 3976 | DHUNIV_949 | GYSGY | 4388 |
| DHUNIV_394 | YGSGSYY | 3977 | DHUNIV_950 | YSGYD | 4389 |
| DHUNIV_395 | GSGSYYN | 3978 | DHUNIV_951 | SGYDY | 4390 |
| DHUNIV_396 | YYYGSG | 3979 | DHUNIV_952 | GYSG | 4391 |
| DHUNIV_397 | YYGSGS | 3980 | DHUNIV_953 | YSGY | 4392 |
| DHUNIV_398 | YGSGSY | 3981 | DHUNIV_954 | SGYD | 4393 |
| DHUNIV_399 | GSGSYY | 3982 | DHUNIV_955 | GYDY | 4394 |
| DHUNIV_400 | SGSYYN | 3983 | DHUNIV_956 | GYS | n/a |
| DHUNIV_401 | YYYGS | 3984 | DHUNIV_957 | GYD | n/a |
| DHUNIV_402 | YYGSG | 398b | DHUNIV_958 | VEMATIT | 4395 |
| DHUNIV_403 | YGSGS | 3986 | DHUNIV_959 | VEMATI | 4396 |
| DHUNIV_404 | GSGSY | 3987 | DHUNIV_960 | EMATIT | 4397 |
| DHUNIV_405 | GSYYN | 3988 | DHUNIV_961 | VEMAT | 4398 |
| DHUNIV_406 | YYYG | 3989 | DHUNIV_962 | EMATI | 4399 |
| DHUNIV_407 | YYGS | 3990 | DHUNIV_963 | MATIT | 4400 |
| DHUNIV_408 | YGSG | 3991 | DHUNIV_964 | VEMA | 4401 |
| DHUNIV_409 | GSGS | 3992 | DHUNIV_965 | EMAT | 4402 |
| DHUNIV_410 | SYYN | 3993 | DHUNIV_966 | MATI | 4403 |
| DHUNIV_411 | YYY | n/a | DHUNIV_967 | VEM | n/a |
| DHUNIV_412 | YYG | n/a | DHUNIV_968 | EMA | n/a |
| DHUNIV_413 | YGS | n/a | DHUNIV_969 | MAT | n/a |
| DHUNIV_414 | GSG | n/a | DHUNIV_970 | VE | n/a |
| DHUNIV_415 | YYN | n/a | DHUNIV_971 | EM | n/a |
| DHUNIV_416 | YG | n/a | DHUNIV_972 | MA | n/a |
| DHUNIV_417 | ITMVRGVIIT | 3994 | DHUNIV_973 | RWLQL | 4404 |
| DHUNIV_418 | ITMVRGVII | 3995 | DHUNIV_974 | RWLQ | 4405 |
| DHUNIV_419 | TMVRGVIIT | 3996 | DHUNIV_975 | WLQL | 4406 |
| DHUNIV_420 | ITMVRGVI | 3997 | DHUNIV_976 | RWL | n/a |
| DHUNIV_421 | TMVRGVII | 3998 | DHUNIV_977 | WLQ | n/a |
| DHUNIV_422 | MVRGVIIT | 3999 | DHUNIV_978 | LQL | n/a |
| DHUNIV_423 | ITMVRGV | 4000 | DHUNIV_979 | RDGYNY | 4407 |
| DHUNIV_424 | TMVRGVI | 4001 | DHUNIV_980 | RDGYN | 4408 |
| DHUNIV_425 | MVRGVII | 4002 | DHUNIV_981 | DGYNY | 4409 |
| DHUNIV_426 | VRGVIIT | 4003 | DHUNIV_982 | RDGY | 4410 |
| DHUNIV_427 | ITMVRG | 4004 | DHUNIV_983 | DGYN | 4411 |
| DHUNIV_428 | TMVRGV | 4005 | DHUNIV_984 | GYNY | 4412 |
| DHUNIV_429 | MVRGVI | 4006 | DHUNIV_985 | RDG | n/a |
| DHUNIV_430 | VRGVII | 4007 | DHUNIV_986 | DGY | n/a |
| DHUNIV_431 | RGVIIT | 4008 | DHUNIV_987 | GYN | n/a |
| DHUNIV_432 | ITMVR | 4009 | DHUNIV_988 | YNY | n/a |
| DHUNIV_433 | TMVRG | 4010 | DHUNIV_989 | RD | n/a |
| DHUNIV_434 | MVRGV | 4011 | DHUNIV_990 | DG | n/a |
| DHUNIV_435 | VRGVI | 4012 | DHUNIV_991 | VDTAMVT | 4413 |
| DHUNIV_436 | RGVII | 4013 | DHUNIV_992 | VDTAMV | 4414 |
| DHUNIV_437 | GVIIT | 4014 | DHUNIV_993 | DTAMVT | 4415 |
| DHUNIV_438 | ITMV | 4015 | DHUNIV_994 | VDTAM | 4416 |
| DHUNIV_439 | TMVR | 4016 | DHUNIV_995 | DTAMV | 4417 |
| DHUNIV_440 | MVRG | 4017 | DHUNIV_996 | TAMVT | 4418 |
| DHUNIV_441 | VRGV | 4018 | DHUNIV_997 | VDTA | 4419 |
| DHUNIV_442 | RGVI | 4019 | DHUNIV_998 | DTAM | 4420 |
| DHUNIV_443 | GVII | 4020 | DHUNIV_999 | TAMV | 4421 |
| DHUNIV_444 | VIIT | 4021 | DHUNIV_1000 | AMVT | 4422 |
| DHUNIV_445 | ITM | n/a | DHUNIV_1001 | VDT | n/a |
| DHUNIV_446 | TMV | n/a | DHUNIV_1002 | DTA | n/a |
| DHUNIV_447 | MVR | n/a | DHUNIV_1003 | TAM | n/a |
| DHUNIV_448 | VRG | n/a | DHUNIV_1004 | AMV | n/a |
| DHUNIV_449 | RGV | n/a | DHUNIV_1005 | MVT | n/a |
| DHUNIV_450 | GVI | n/a | DHUNIV_1006 | DT | n/a |
| DHUNIV_451 | VII | n/a | DHUNIV_1007 | WIQLWL | 4423 |
| DHUNIV_452 | IIT | n/a | DHUNIV_1008 | WIQLW | 4424 |
| DHUNIV_453 | TM | n/a | DHUNIV_1009 | IQLWL | 4425 |
| DHUNIV_454 | VR | n/a | DHUNIV_1010 | WIQL | 4426 |
| DHUNIV_455 | RG | n/a | DHUNIV_1011 | IQLW | 4427 |
| DHUNIV_456 | II | n/a | DHUNIV_1012 | QLWL | 4428 |
| DHUNIV_457 | VLLWFRELL | 4022 | DHUNIV_1013 | WIQ | n/a |
| DHUNIV_458 | VLLWFREL | 4023 | DHUNIV_1014 | IQL | n/a |
| DHUNIV_459 | LLWFRELL | 4024 | DHUNIV_1015 | QLW | n/a |
| DHUNIV_460 | VLLWFRE | 4025 | DHUNIV_1016 | LWL | n/a |
| DHUNIV_461 | LLWFREL | 4026 | DHUNIV_1017 | IQ | n/a |
| DHUNIV_462 | LWFRELL | 4027 | DHUNIV_1018 | GYSYGY | 4429 |
| DHUNIV_463 | VLLWFR | 4028 | DHUNIV_1019 | GYSYG | 4430 |
| DHUNIV_464 | LLWFRE | 4029 | DHUNIV_1020 | YSYGY | 4431 |
| DHUNIV_465 | LWFREL | 4030 | DHUNIV_1021 | GYSY | 4432 |
| DHUNIV_466 | WFRELL | 4031 | DHUNIV_1022 | YSYG | 4433 |
| DHUNIV_467 | LLWFR | 4032 | DHUNIV_1023 | SYGY | 4434 |
| DHUNIV_468 | LWFRE | 4033 | DHUNIV_1024 | YSY | n/a |
| DHUNIV_469 | WFREL | 4034 | DHUNIV_1025 | SYG | n/a |
| DHUNIV_470 | FRELL | 4035 | DHUNIV_1026 | YGY | n/a |
| DHUNIV_471 | LWFR | 4036 | DHUNIV_1027 | GYSSSWY | 4435 |
| DHUNIV_472 | WFRE | 4037 | DHUNIV_1028 | GYSSSW | 4436 |
| DHUNIV_473 | FREL | 4038 | DHUNIV_1029 | YSSSWY | 4437 |
| DHUNIV_474 | RELL | 4039 | DHUNIV_1030 | GYSSS | 4438 |
| DHUNIV_475 | WFR | n/a | DHUNIV_1031 | YSSSW | 4439 |
| DHUNIV_476 | FRE | n/a | DHUNIV_1032 | SSSWY | 4440 |
| DHUNIV_477 | REL | n/a | DHUNIV_1033 | GYSS | 4441 |
| DHUNIV_478 | FR | n/a | DHUNIV_1034 | YSSS | 4442 |
| DHUNIV_479 | RE | n/a | DHUNIV_1035 | SSSW | 4443 |
| DHUNIV_480 | ITMVQGVIIT | 4040 | DHUNIV_1036 | SSWY | 4444 |
| DHUNIV_481 | ITMVQGVII | 4041 | DHUNIV_1037 | YSS | n/a |
| DHUNIV_482 | TMVQGVIIT | 4042 | DHUNIV_1038 | SSS | n/a |
| DHUNIV_483 | ITMVQGVI | 4043 | DHUNIV_1039 | SSW | n/a |
| DHUNIV_484 | TMVQGVII | 4044 | DHUNIV_1040 | SWY | n/a |
| DHUNIV_485 | MVQGVIIT | 4045 | DHUNIV_1041 | SW | n/a |
| DHUNIV_486 | ITMVQGV | 4046 | DHUNIV_1042 | WY | n/a |
| DHUNIV_487 | TMVQGVI | 4047 | DHUNIV_1043 | GIAAAGT | 4445 |
| DHUNIV_488 | MVQGVII | 4048 | DHUNIV_1044 | GIAAAG | 4446 |
| DHUNIV_489 | VQGVIIT | 4049 | DHUNIV_1045 | IAAAGT | 4447 |
| DHUNIV_490 | ITMVQG | 4050 | DHUNIV_1046 | GIAAA | 4448 |
| DHUNIV_491 | TMVQGV | 4051 | DHUNIV_1047 | IAAAG | 4449 |
| DHUNIV_492 | MVQGVI | 4052 | DHUNIV_1048 | AAAGT | 4450 |
| DHUNIV_493 | VQGVII | 4053 | DHUNIV_1049 | GIAA | 4451 |
| DHUNIV_494 | QGVIIT | 4054 | DHUNIV_1050 | IAAA | 4452 |
| DHUNIV_495 | ITMVQ | 4055 | DHUNIV_1051 | AAAG | 4453 |
| DHUNIV_496 | TMVQG | 4056 | DHUNIV_1052 | AAGT | 4454 |
| DHUNIV_497 | MVQGV | 4057 | DHUNIV_1053 | GIA | n/a |
| DHUNIV_498 | VQGVI | 4058 | DHUNIV_1054 | IAA | n/a |
| DHUNIV_499 | QGVII | 4059 | DHUNIV_1055 | AAA | n/a |
| DHUNIV_500 | TMVQ | 4060 | DHUNIV_1056 | AAG | n/a |
| DHUNIV_501 | MVQG | 4061 | DHUNIV_1057 | AGT | n/a |
| DHUNIV_502 | VQGV | 4062 | DHUNIV_1058 | AG | n/a |
| DHUNIV_503 | QGVI | 4063 | DHUNIV_1059 | QQLV | 4455 |
| DHUNIV_504 | MVQ | n/a | DHUNIV_1060 | QQL | n/a |
| DHUNIV_505 | VQG | n/a | DHUNIV_1061 | QLV | n/a |
| DHUNIV_506 | QGV | n/a | DHUNIV_1062 | QQ | n/a |
| DHUNIV_507 | QG | n/a | DHUNIV_1063 | GYSSGWY | 4456 |
| DHUNIV_508 | LRLGEL | 4064 | DHUNIV_1064 | GYSSGW | 4457 |
| DHUNIV_509 | LRLGE | 4065 | DHUNIV_1065 | YSSGWY | 4458 |
| DHUNIV_510 | RLGEL | 4066 | DHUNIV_1066 | GYSSG | 4459 |
| DHUNIV_511 | LRLG | 4067 | DHUNIV_1067 | YSSGW | 4460 |
| DHUNIV_512 | RLGE | 4068 | DHUNIV_1068 | SSGWY | 4461 |
| DHUNIV_513 | LGEL | 4069 | DHUNIV_1069 | YSSG | 4462 |
| DHUNIV_514 | LRL | n/a | DHUNIV_1070 | SSGW | 4463 |
| DHUNIV_515 | RLG | n/a | DHUNIV_1071 | SGWY | 4464 |
| DHUNIV_516 | LGE | n/a | DHUNIV_1072 | SGW | n/a |
| DHUNIV_517 | LR | n/a | DHUNIV_1073 | GWY | n/a |
| DHUNIV_518 | RL | n/a | DHUNIV_1074 | GW | n/a |
| DHUNIV_519 | LG | n/a | DHUNIV_1075 | GIAVAGT | 4465 |
| DHUNIV_520 | YYDYVWGSYAYT | 4070 | DHUNIV_1076 | GIAVAG | 4466 |
| DHUNIV_521 | YYDYVWGSYAY | 4071 | DHUNIV_1077 | IAVAGT | 4467 |
| DHUNlV_522 | YDYVWGSYAYT | 4072 | DHUNIV_1078 | GIAVA | 4468 |
| DHUNIV_523 | YYDYVWGSYA | 4073 | DHUNIV_1079 | IAVAG | 4469 |
| DHUNIV_524 | YDYVWGSYAY | 4074 | DHUNIV_1080 | AVAGT | 4470 |
| DHUNIV_525 | DYVWGSYAYT | 4075 | DHUNIV_1081 | GIAV | 4471 |
| DHUNIV_526 | YYDYVWGSY | 4076 | DHUNIV_1082 | IAVA | 4472 |
| DHUNIV_527 | YDYVWGSYA | 4077 | DHUNIV_1083 | AVAG | 4473 |
| DHUNIV_528 | DYVWGSYAY | 4078 | DHUNIV_1084 | VAGT | 4474 |
| DHUNIV_529 | YVWGSYAYT | 4079 | DHUNIV_1085 | IAV | n/a |
| DHUNIV_530 | YYDYVWGS | 4080 | DHUNIV_1086 | AVA | n/a |
| DHUNIV_531 | YDYVWGSY | 4081 | DHUNIV_1087 | VAG | n/a |
| DHUNIV_532 | DYVWGSYA | 4082 | DHUNIV_1088 | AV | n/a |
| DHUNIV_533 | YVWGSYAY | 4083 | DHUNIV_1089 | QWLV | 4475 |
| DHUNIV_534 | VWGSYAYT | 4084 | DHUNIV_1090 | QWL | n/a |
| DHUNIV_535 | YYDYVWG | 4085 | DHUNIV_1091 | WLV | n/a |
| DHUNIV_536 | YDYVWGS | 4086 | DHUNIV_1092 | QW | n/a |
| DHUNIV_537 | DYVWGSY | 4087 | DHUNIV_1093 | EYSSSS | 4476 |
| DHUNIV_538 | YVWGSYA | 4088 | DHUNIV_1094 | EYSSS | 4477 |
| DHUNIV_539 | VWGSYAY | 4089 | DHUNIV_1095 | YSSSS | 4478 |
| DHUNIV_540 | WGSYAYT | 4090 | DHUNIV_1096 | EYSS | 4479 |
| DHUNIV_541 | YYDYVW | 4091 | DHUNIV_1097 | SSSS | 4480 |
| DHUNIV_542 | YDYVWG | 4092 | DHUNIV_1098 | EYS | n/a |
| DHUNIV_543 | DYVWGS | 4093 | DHUNIV_1099 | EY | n/a |
| DHUNIV_544 | YVWGSY | 4094 | DHUNIV_1100 | SIAARP | 4481 |
| DHUNIV_545 | VWGSYA | 4095 | DHUNIV_1101 | SIAAR | 4482 |
| DHUNIV_546 | WGSYAY | 4096 | DHUNIV_1102 | IAARP | 4483 |
| DHUNIV_547 | GSYAYT | 4097 | DHUNIV_1103 | SIAA | 4484 |
| DHUNIV_548 | YYDYV | 4098 | DHUNIV_1104 | IAAR | 448b |
| DHUNIV_549 | YDYVW | 4099 | DHUNIV_1105 | AARP | 4486 |
| DHUNIV_550 | DYVWG | 4100 | DHUNIV_1106 | SIA | n/a |
| DHUNIV_551 | YVWGS | 4101 | DHUNIV_1107 | AAR | n/a |
| DHUNIV_552 | VWGSY | 4102 | DHUNIV_1108 | ARP | n/a |
| DHUNIV_553 | WGSYA | 4103 | DHUNIV_1109 | AR | n/a |
| DHUNIV_554 | GSYAY | 4104 | DHUNIV_1110 | RP | n/a |
| DHUNIV_555 | SYAYT | 4105 | DHUNIV_1111 | NWG | n/a |
| DHUNIV_556 | YYDY | 4106 | | | |

**Table 12. Theoretical segment pool of 141 N2 segments in Theoretical Segment Pool 1 (TSP1).**

| **Segment Type** | **Sequences** | **Number** |
|---|---|---|
| "Zero" | (no addition) V segment joins directly to D segment | 1 |
| Monomers | G, P, R, A, S, L, T, V, D, E, F, H, I, K, M, Q, W, Y | 18 |
| Dimers | GG, GP, GR, GA, GS, GL, GT, GV, PG, RG, AG, SG, LG, TG, VG, PP, PR, PA, PS, PL, PT, PV, RP, AP, SP, LP, TP, VP, AR, AS, AT, AY, DL, DT, EA, EK, FH, FS, HL, HW, IS, KV, LD, LE, LR, LS, LT, NR, NT, QE, QL, QT, RA, RD, RE, RF, RH, RL, RR, RS, RV, SA, SD, SE, SF, SI, SK, SL, SQ, SR, SS, ST, SV, TA, TR, TS, TT, TW, VD, VS, WS, YS | 82 |
| Trimers | GGG, GPG, GRG, GAG, GSG, GLG, GTG, GVG, PGG, RGG, AGG, SGG, LGG, TGG, VGG, GGP, GGR, GGA, GGS, GGL, GGT, GGV , AAE, AYH, DTL, EKR, ISR, NTP, PKS, PRP, PTA, PTQ, REL, RPL, SAA, SAL, SGL, SSE, TGL, WGT | 40 |

**Table 13. Theoretical segment pool of 285 H3-JH segments.**

| **H3-JH Segment Name** | **Amino Acid Sequence** | **SEQ ID NO** | **H3-JH Segment Name** | **Amino Acid Sequence** | **SEQ ID NO** |
|---|---|---|---|---|---|
| JHUNIV_001 | TEYFQH | 4487 | JHUNIV_144 | ADY | n/a |
| JHUNIV_002 | EYFQH | 4488 | JHUNIV_145 | YDY | n/a |
| JHUNIV_003 | YFQH | 4489 | JHUNIV_146 | NYFDY | 4593 |
| JHUNIV_004 | FQH | n/a | JHUNIV_147 | DNWFDP | 4594 |
| JHUNIV_005 | QH | n/a | JHUNIV_148 | NWFDP | 4595 |
| JHUNIV_006 | H | n/a | JHUNIV_149 | WFDP | 4596 |
| JHUNIV_007 | | n/a | JHUNIV_150 | FDP | n/a |
| JHUNIV_008 | SEYFQH | 4490 | JHUNIV_151 | DP | n/a |
| JHUNIV_009 | PEYFQH | 4491 | JHUNIV_152 | P | n/a |
| JHUNIV_010 | FEYFQH | 4492 | JHUNIV_153 | YNWFDP | 4597 |
| JHUNIV_011 | HEYFQH | 4493 | JHUNIV_154 | HNWFDP | 4598 |
| JHUNIV_012 | REYFQH | 4494 | JHUNIV_155 | FNWFDP | 4599 |
| JHUNIV_013 | LEYFQH | 4495 | JHUNIV_156 | SNWFDP | 4600 |
| JHUNIV_014 | NEYFQH | 4496 | JHUNIV_157 | RNWFDP | 4601 |
| JHUNIV_015 | IEYFQH | 4497 | JHUNIV_158 | LNWFDP | 4602 |
| JHUNIV_016 | DEYFQH | 4498 | JHUNIV_159 | PNWFDP | 4603 |
| JHUNIV_017 | GEYFQH | 4499 | JHUNIV_160 | INWFDP | 4604 |
| JHUNIV_018 | VEYFQH | 4500 | JHUNIV_161 | TNWFDP | 4605 |
| JHUNIV_019 | YEYFQH | 4501 | JHUNIV_162 | GNWFDP | 4606 |
| JHUNIV_020 | KYFQH | 4502 | JHUNIV_163 | VNWFDP | 4607 |
| JHUNIV_021 | QYFQH | 4503 | JHUNIV_164 | ANWFDP | 4608 |
| JHUNIV_022 | LYFQH | 4504 | JHUNIV_165 | DWFDP | 4609 |
| JHUNIV_023 | SYFQH | 4505 | JHUNIV_166 | YWFDP | 4610 |
| JHUNIV_024 | RYFQH | 4506 | JHUNIV_167 | HWFDP | 4611 |
| JHUNIV_025 | PYFQH | 4507 | JHUNIV_168 | FWFDP | 4612 |
| JHUNIV_026 | IYFQH | 4508 | JHUNIV_169 | SWFDP | 4613 |
| JHUNIV_027 | TYFQH | 4509 | JHUNIV_170 | RWFDP | 4614 |
| JHUNIV_028 | GYFQH | 4510 | JHUNIV_171 | LWFDP | 4615 |
| JHUNIV_029 | VYFQH | 4511 | JHUNIV_172 | PWFDP | 4616 |
| JHUNIV_030 | AYFQH | 4512 | JHUNIV_173 | IWFDP | 4617 |
| JHUNIV_031 | NFQH | 4513 | JHUNIV_174 | TWFDP | 4618 |
| JHUNIV_032 | DFQH | 4514 | JHUNIV_175 | GWFDP | 4619 |
| JHUNIV_033 | HFQH | 4515 | JHUNIV 176 | VWFDP | 4620 |
| JHUNIV_034 | FFQH | 4516 | JHUNIV_177 | AWFDP | 4621 |
| JHUNIV_035 | SFQH | 4517 | JHUNIV_178 | RFDP | 4622 |
| JHUNIV_036 | RFQH | 4518 | JHUNIV_179 | GFDP | 4623 |
| JHUNIV_037 | LFQH | 4519 | JHUNIV_180 | LFDP | 4624 |
| JHUNIV_038 | PFQH | 4520 | JHUNIV_181 | SFDP | 4625 |
| JHUNIV_039 | IFQH | 4521 | JHUNIV_182 | QFDP | 4626 |
| JHUNIV_040 | TFQH | 4522 | JHUNIV_183 | PFDP | 4627 |
| JHUNIV_041 | GFQH | 4523 | JHUNIV_184 | KFDP | 4628 |
| JHUNIV_042 | VFQH | 4524 | JHUNIV_185 | MFDP | 4629 |
| JHUNIV_043 | AFQH | 4525 | JHUNIV_186 | TFDP | 4630 |
| JHUNIV_044 | AEYFQH | 4526 | JHUNIV_187 | EFDP | 4631 |
| JHUNIV_045 | YWYFDL | 4527 | JHUNIV_188 | VFDP | 4632 |
| JHUNIV_046 | WYFDL | 4528 | JHUNIV_189 | AFDP | 4633 |
| JHUNIV_047 | YFDL | 4529 | JHUNIV_190 | NNWFDP | 4634 |
| JHUNIV_048 | FDL | n/a | JHUNIV_191 | DYYYYYGMDV | 4635 |
| JHUNIV_049 | DL | n/a | JHUNIV_192 | YYYYYGMDV | 4636 |
| JHUNIV_050 | L | n/a | JHUNIV_193 | YYYYGMDV | 4637 |
| JHUNIV_051 | DWYFDL | 4530 | JHUNIV_194 | YYYGMDV | 4638 |
| JHUNIV_052 | HWYFDL | 4531 | JHUNIV_195 | YYGMDV | 4639 |
| JHUNIV_053 | NWYFDL | 4532 | JHUNIV_196 | YGMDV | 4640 |
| JHUNIV_054 | GYFDL | 4533 | JHUNIV_197 | GMDV | 4641 |
| JHUNIV_055 | RYFDL | 4534 | JHUNIV_198 | MDV | n/a |
| JHUNIV_056 | HFDL | 4535 | JHUNIV_199 | DV | n/a |
| JHUNIV_057 | NFDL | 4536 | JHUNIV_200 | V | n/a |
| JHUNIV_058 | DFDL | 4537 | JHUNIV_201 | YYYYYYGMDV | 4642 |
| JHUNIV_059 | DAFDI | 4538 | JHUNIV_202 | HYYYYYGMDV | 4643 |
| JHUNIV_060 | AFDI | 4539 | JHUNIV_203 | FYYYYYGMDV | 4644 |
| JHUNIV_061 | FDI | n/a | JHUNIV_204 | SYYYYYGMDV | 4645 |
| JHUNIV_062 | DI | n/a | JHUNIV_205 | RYYYYYGMDV | 4646 |
| JHUNIV_063 | I | n/a | JHUNIV_206 | LYYYYYGMDV | 4647 |
| JHUNIV_064 | YAFDI | 4540 | JHUNIV_207 | PYYYYYGMDV | 4648 |
| JHUNIV_065 | HAFDI | 4541 | JHUNIV_208 | IYYYYYGMDV | 4649 |
| JHUNIV_066 | FAFDI | 4542 | JHUNIV_209 | TYYYYYGMDV | 4650 |
| JHUNIV_067 | SAFDI | 4543 | JHUNIV_210 | GYYYYYGMDV | 4651 |
| JHUNIV_068 | RAFDI | 4544 | JHUNIV_211 | VYYYYYGMDV | 4652 |
| JHUNIV_069 | LAFDI | 4545 | JHUNIV_212 | AYYYYYGMDV | 4653 |
| JHUNIV_070 | PAFDI | 4546 | JHUNIV_213 | NYYYYGMDV | 4654 |
| JHUNIV_071 | IAFDI | 4547 | JHUNIV_214 | DYYYYGMDV | 4655 |
| JHUNIV_072 | TAFDI | 4548 | JHUNIV_215 | HYYYYGMDV | 4656 |
| JHUNIV_073 | GAFDI | 4549 | JHUNIV_216 | FYYYYGMDV | 4657 |
| JHUNIV_074 | VAFDI | 4550 | JHUNIV_217 | SYYYYGMDV | 4658 |
| JHUNIV_075 | AAFDI | 4551 | JHUNIV_218 | RYYYYGMDV | 4659 |
| JHUNIV_076 | TFDI | 4552 | JHUNIV_219 | LYYYYGMDV | 4660 |
| JHUNIV_077 | SFDI | 4553 | JHUNIV_220 | PYYYYGMDV | 4661 |
| JHUNIV_078 | PFDI | 4554 | JHUNIV_221 | IYYYYGMDV | 4662 |
| JHUNIV_079 | FFDI | 4555 | JHUNIV_222 | TYYYYGMDV | 4663 |
| JHUNIV_080 | HFDI | 4556 | JHUNIV_223 | GYYYYGMDV | 4664 |
| JHUNIV_081 | RFDI | 4557 | JHUNIV_224 | VYYYYGMDV | 4665 |
| JHUNIV_082 | LFDI | 4558 | JHUNIV_225 | AYYYYGMDV | 4666 |
| JHUNIV_083 | NFDI | 4559 | JHUNIV_226 | NYYYGMDV | 4667 |
| JHUNIV_084 | IFDI | 4560 | JHUNIV_227 | DYYYGMDV | 4668 |
| JHUNIV_085 | DFDI | 4561 | JHUNIV_228 | HYYYGMDV | 4669 |
| JHUNIV_086 | GFDI | 4562 | JHUNIV_229 | FYYYGMDV | 4670 |
| JHUNIV_087 | VFDI | 4563 | JHUNIV_230 | SYYYGMDV | 4671 |
| JHUNIV_088 | YFDI | 4564 | JHUNIV_231 | RYYYGMDV | 4672 |
| JHUNIV_089 | IDI | n/a | JHUNIV_232 | LYYYGMDV | 4673 |
| JHUNIV_090 | VDI | n/a | JHUNIV_233 | PYYYGMDV | 4674 |
| JHUNIV_091 | LDI | n/a | JHUNIV_234 | IYYYGMDV | 4675 |
| JHUNIV_092 | SDI | n/a | JHUNIV_235 | TYYYGMDV | 4676 |
| JHUNIV_093 | HDI | n/a | JHUNIV_236 | GYYYGMDV | 4677 |
| JHUNIV_094 | RDI | n/a | JHUNIV_237 | VYYYGMDV | 4678 |
| JHUNIV_095 | PDI | n/a | JHUNIV_238 | AYYYGMDV | 4679 |
| JHUNIV_096 | NDI | n/a | JHUNIV_239 | NYYYYYGMDV | 4680 |
| JHUNIV_097 | TDI | n/a | JHUNIV_240 | DYYYYYYMDV | 4681 |
| JHUNIV_098 | DDI | n/a | JHUNIV_241 | YYYYYYMDV | 4682 |
| JHUNIV_099 | GDI | n/a | JHUNIV_242 | YYYYYMDV | 4683 |
| JHUNIV_100 | ADI | n/a | JHUNIV_243 | YYYYMDV | 4684 |
| JHUNIV_101 | YDI | n/a | JHUNIV_244 | YYYMDV | 4685 |
| JHUNIV_102 | NAFDI | 4565 | JHUNIV_245 | YYMDV | 4686 |
| JHUNIV_103 | DYFDY | 4566 | JHUNIV_246 | YMDV | 4687 |
| JHUNIV_104 | YFDY | 4567 | JHUNIV_247 | YYYYYYYMDV | 4688 |
| JHUNIV_105 | FDY | n/a | JHUNIV_248 | HYYYYYYMDV | 4689 |
| JHUNIV_106 | DY | n/a | JHUNIV_249 | FYYYYYYMDV | 4690 |
| JHUNIV_107 | Y | n/a | JHUNIV_250 | SYYYYYYMDV | 4691 |
| JHUNIV_108 | YYFDY | 4568 | JHUNIV_251 | RYYYYYYMDV | 4692 |
| JHUNIV_109 | HYFDY | 4569 | JHUNIV_252 | LYYYYYYMDV | 4693 |
| JHUNIV_110 | FYFDY | 4570 | JHUNIV_253 | PYYYYYYMDV | 4694 |
| JHUNIV_111 | SYFDY | 4571 | JHUNIV_254 | IYYYYYYMDV | 4695 |
| JHUNIV_112 | RYFDY | 4572 | JHUNIV_255 | TYYYYYYMDV | 4696 |
| JHUNIV_113 | LYFDY | 4573 | JHUNIV_256 | GYYYYYYMDV | 4697 |
| JHUNIV_114 | PYFDY | 4574 | JHUNIV_257 | VYYYYYYMDV | 4698 |
| JHUNIV_115 | IYFDY | 4575 | JHUNIV_258 | AYYYYYYMDV | 4699 |
| JHUNIV_116 | TYFDY | 4576 | JHUNIV_259 | NYYYYYMDV | 4700 |
| JHUNIV_117 | GYFDY | 4577 | JHUNIV_260 | DYYYYYMDV | 4701 |
| JHUNIV_118 | VYFDY | 4578 | JHUNIV_261 | HYYYYYMDV | 4702 |
| JHUNIV_119 | AYFDY | 4579 | JHUNIV_262 | FYYYYYMDV | 4703 |
| JHUNIV_120 | NFDY | 4580 | JHUNIV_263 | SYYYYYMDV | 4704 |
| JHUNIV_121 | DFDY | 4581 | JHUNIV_264 | RYYYYYMDV | 4705 |
| JHUNIV_122 | HFDY | 4582 | JHUNIV_265 | LYYYYYMDV | 4706 |
| JHUNIV_123 | FFDY | 4583 | JHUNIV_266 | PYYYYYMDV | 4707 |
| JHUNIV_124 | SFDY | 4584 | JHUNIV_267 | IYYYYYMDV | 4708 |
| JHUNIV_125 | RFDY | 4585 | JHUNIV_268 | TYYYYYMDV | 4709 |
| JHUNIV_126 | LFDY | 4586 | JHUNIV_269 | GYYYYYMDV | 4710 |
| JHUNIV_127 | PFDY | 4587 | JHUNIV_270 | VYYYYYMDV | 4711 |
| JHUNIV_128 | IFDY | 4588 | JHUNIV_271 | AYYYYYMDV | 4712 |
| JHUNIV_129 | TFDY | 4589 | JHUNIV_272 | NYYYYMDV | 4713 |
| JHUNIV_130 | GFDY | 4590 | JHUNIV_273 | DYYYYMDV | 4714 |
| JHUNIV_131 | VFDY | 4591 | JHUNIV_274 | HYYYYMDV | 4715 |
| JHUNIV_132 | AFDY | 4592 | JHUNIV_275 | FYYYYMDV | 4716 |
| JHUNIV_133 | IDY | n/a | JHUNIV_276 | SYYYYMDV | 4717 |
| JHUNIV_134 | VDY | n/a | JHUNIV_277 | RYYYYMDV | 4718 |
| JHUNIV_135 | LDY | n/a | JHUNIV_278 | LYYYYMDV | 4719 |
| JHUNIV_136 | SDY | n/a | JHUNIV_279 | PYYYYMDV | 4720 |
| JHUNIV_137 | HDY | n/a | JHUNIV_280 | IYYYYMDV | 4721 |
| JHUNIV_138 | RDY | n/a | JHUNIV_281 | TYYYYMDV | 4722 |
| JHUNIV_139 | PDY | n/a | JHUNIV_282 | GYYYYMDV | 4723 |
| JHUNIV_140 | NDY | n/a | JHUNIV_283 | VYYYYMDV | 4724 |
| JHUNIV_141 | TDY | n/a | JHUNIV_284 | AYYYYMDV | 4725 |
| JHUNIV_142 | DDY | n/a | JHUNIV_285 | NYYYYYYMDV | 4726 |
| JHUNIV_143 | GDY | n/a | | | |

**Table 14. Twelve germline IGHJ genes and alleles.**

| **IGHJ Gene** | **DNA Sequence** | **SEQ ID NO** |
|---|---|---|
| IGHJ1-01 | GCTGAATACTTCCAGCACTGGGGCCAGGGCACCCTGGTCACCGTCTCCTCAG | 4727 |
| IGHJ2-01 | CTACTGGTACTTCGATCTCTGGGGCCGTGGCACCCTGGTCACTGTCTCCTCAG | 4728 |
| IGHJ3-01 | ATGCTTTTGATGTCTGGGGCCAAGGGACAATGGTCACCGTCTCTTCAG | 4729 |
| IGHJ3-02 | ATGCTTTTGATATCTGGGGCCAAGGGACAATGGTCACCGTCTCTTCAG | 4730 |
| IGHJ4-01 | ACTACTTTGACTACTGGGGCCAAGGAACCCTGGTCACCGTCTCCTCAG | 4731 |
| IGHJ4-02 | ACTACTTTGACTACTGGGGCCAGGGAACCCTGGTCACCGTCTCCTCAG | 4732 |
| IGHJ4-03 | GCTACTTTGACTACTGGGGCCAAGGGACCCTGGTCACCGTCTCCTCAG | 4733 |
| IGHJ5-01 | ACAACTGGTTCGACTCCTGGGGCCAAGGAACCCTGGTCACCGTCTCCTCAG | 4734 |
| IGHJ5-02 | ACAACTGGTTCGACCCCTGGGGCCAGGGAACCCTGGTCACCGTCTCCTCAG | 4735 |
| IGHJ6-01 | ATTACTACTACTACTACGGTATGGACGTCTGGGGGCAAGGGACCACGGTCACCGTCTCCTCAG | 4736 |
| IGHJ6-02 | ATTACTACTACTACTACGGTATGGACGTCTGGGGCCAAGGGACCACGGTCACCGTCTCCTCAG | 4737 |
| IGHJ6-03 | ATTACTACTACTACTACTACATGGACGTCTGGGGCAAAGGGACCACGGTCACCGTCTCCTCAG | 4738 |

**Table 15. Theoretical segment pool of 248 parent H3-JH segments.**

| **H3-JH Parent Segment Name** | **Amino Acid Sequence** | **SEQ ID NO** | **H3-JH Parent Segment Name** | **Amino Acid Sequence** | **SEQ ID NO** |
|---|---|---|---|---|---|
| JHparent001 | ADI | n/a | JHparent125 | TAFDI | 4548 |
| JHparent002 | ADY | n/a | JHparent126 | TWFDP | 4618 |
| JHparent003 | DDI | n/a | JHparent127 | TYFDY | 4576 |
| JHparent004 | DDY | n/a | JHparent128 | TYFQH | 4509 |
| JHparent005 | GDI | n/a | JHparent129 | VAFDI | 4550 |
| JHparent006 | GDY | n/a | JHparent130 | VWFDP | 4620 |
| JHparent007 | HDI | n/a | JHparent131 | VYFDY | 4578 |
| JHparent008 | HDY | n/a | JHparent132 | VYFQH | 4511 |
| JHparent009 | IDI | n/a | JHparent133 | WYFDL | 4528 |
| JHparent010 | IDY | n/a | JHparent134 | YAFDI | 4540 |
| JHparent011 | LDI | n/a | JHparent135 | YWFDP | 4610 |
| JHparent012 | LDY | n/a | JHparent136 | YYFDY | 4568 |
| JHparent013 | NDI | n/a | JHparent137 | AEYFQH | 4526 |
| JHparent014 | NDY | n/a | JHparent138 | ANWFDP | 4608 |
| JHparent015 | PDI | n/a | JHparent139 | DEYFQH | 4498 |
| JHparent016 | PDY | n/a | JHparent140 | DNWFDP | 4594 |
| JHparent017 | RDI | n/a | JHparent141 | DWYFDL | 4530 |
| JHparent018 | RDY | n/a | JHparent142 | FEYFQH | 4492 |
| JHparent019 | SDI | n/a | JHparent143 | FNWFDP | 4599 |
| JHparent020 | SDY | n/a | JHparent144 | GEYFQH | 4499 |
| JHparent021 | TDI | n/a | JHparent145 | GNWFDP | 4606 |
| JHparent022 | TDY | n/a | JHparent146 | HEYFQH | 4493 |
| JHparent023 | VDI | n/a | JHparent147 | HNWFDP | 4598 |
| JHparent024 | VDY | n/a | JHparent148 | HWYFDL | 4531 |
| JHparent025 | YDI | n/a | JHparent149 | IEYFQH | 4497 |
| JHparent026 | YDY | n/a | JHparent150 | INWFDP | 4604 |
| JHparent027 | AFDP | 4633 | JHparent151 | LEYFQH | 4495 |
| JHparent028 | AFDY | 4592 | JHparent152 | LNWFDP | 4602 |
| JHparent029 | AFQH | 4525 | JHparent153 | NEYFQH | 4496 |
| JHparent030 | DFDI | 4561 | JHparent154 | NNWFDP | 4634 |
| JHparent031 | DFDL | 4537 | JHparent155 | NWYFDL | 4532 |
| JHparent032 | DFDY | 4581 | JHparent156 | PEYFQH | 4491 |
| JHparent033 | DFQH | 4514 | JHparent157 | PNWFDP | 4603 |
| JHparent034 | EFDP | 4631 | JHparent158 | REYFQH | 4494 |
| JHparent035 | FFDI | 4555 | JHparent159 | RNWFDP | 4601 |
| JHparent036 | FFDY | 4583 | JHparent160 | SEYFQH | 4490 |
| JHparent037 | FFQH | 4516 | JHparent161 | SNWFDP | 4600 |
| JHparent038 | GFDI | 4562 | JHparent162 | TEYFQH | 4487 |
| JHparent039 | GFDP | 4623 | JHparent163 | TNWFDP | 4605 |
| JHparent040 | GFDY | 4590 | JHparent164 | VEYFQH | 4500 |
| JHparent041 | GFQH | 4523 | JHparent165 | VNWFDP | 4607 |
| JHparent042 | HFDI | 4556 | JHparent166 | YEYFQH | 4501 |
| JHparent043 | HFDL | 4535 | JHparent167 | YNWFDP | 4597 |
| JHparent044 | HFDY | 4582 | JHparent16H | YWYFDL | 4527 |
| JHparent045 | HFQH | 4515 | JHparent169 | AYYYGMDV | 4679 |
| JHparent046 | IFDI | 4560 | JHparent170 | AYYYYMDV | 4725 |
| JHparent047 | IFDY | 4588 | JHparent171 | DYYYGMDV | 4668 |
| JHparent048 | IFQH | 4521 | JHparent172 | DYYYYMDV | 4714 |
| JHparent049 | KFDP | 4628 | JHparent173 | FYYYGMDV | 4670 |
| JHparent050 | LFDI | 4558 | JHparent174 | FYYYYMDV | 4716 |
| JHparent051 | LFDP | 4624 | JHparent175 | GYYYGMDV | 4677 |
| JHparent052 | LFDY | 4586 | JHparent176 | GYYYYMDV | 4723 |
| JHparent053 | LFQH | 4519 | JHparent177 | HYYYGMDV | 4669 |
| JHparent054 | MFDP | 4629 | JHparent178 | HYYYYMDV | 4715 |
| JHparent055 | NFDI | 4559 | JHparent179 | IYYYGMDV | 4675 |
| JHparent056 | NFDL | 4536 | JHparent180 | IYYYYMDV | 4721 |
| JHparent057 | NFDY | 4580 | JHparent181 | LYYYGMDV | 4673 |
| JHparent058 | NFQH | 4513 | JHparent182 | LYYYYMDV | 4719 |
| JHparent059 | PFDI | 4554 | JHparent183 | NYYYGMDV | 4667 |
| JHparent060 | PFDP | 4627 | JHparent184 | NYYYYMDV | 4713 |
| JHparent061 | PFDY | 4587 | JHparent185 | PYYYGMDV | 4674 |
| JHparent062 | PFQH | 4520 | JHparent186 | PYYYYMDV | 4720 |
| JHparent063 | QFDP | 4626 | JHparent187 | RYYYGMDV | 4672 |
| JHparent064 | RFDI | 4557 | JHparent18 | RYYYYMDV | 4718 |
| JHparent065 | RFDP | 4622 | JHparent189 | SYYYGMDV | 4671 |
| JHparent066 | RFDY | 4585 | JHparent190 | SYYYYMDV | 4717 |
| JHparent067 | RFQH | 4518 | JHparent191 | TYYYGMDV | 4676 |
| JHparent068 | SFDI | 4553 | JHparent192 | TYYYYMDV | 4722 |
| JHparent069 | SFDP | 4625 | JHparent193 | VYYYGMDV | 4678 |
| JHparent070 | SFDY | 4584 | JHparent194 | VYYYYMDV | 4724 |
| JHparent071 | SFQH | 4517 | JHparent195 | AYYYYGMDV | 4666 |
| JHparent072 | TFDI | 4552 | JHparent196 | AYYYYYMDV | 4712 |
| JHparent073 | TFDP | 4630 | JHparent197 | DYYYYGMDV | 4655 |
| JHparent074 | TFDY | 4589 | JHparent198 | DYYYYYMDV | 4701 |
| JHparent075 | TFQH | 4522 | JHparent199 | FYYYYGMDV | 4657 |
| JHparent076 | VFDI | 4563 | JHparent200 | FYYYYYMDV | 4703 |
| JHparent077 | VFDP | 4632 | JHparent201 | GYYYYGMDV | 4664 |
| JHparent078 | VFDY | 4591 | JHparent202 | GYYYYYMDV | 4710 |
| JHparent079 | VFQH | 4524 | JHparent203 | HYYYYGMDV | 4656 |
| JHparent080 | YFDI | 4564 | JHparent204 | HYYYYYMDV | 4702 |
| JHparent081 | YFDL | 4529 | JHparent205 | IYYYYGMDV | 4662 |
| JHparent082 | AAFDI | 4551 | JHparent206 | IYYYYYMDV | 4708 |
| JHparent083 | AWFDP | 4621 | JHparent207 | LYYYYGMDV | 4660 |
| JHparent084 | AYFDY | 4579 | JHparent208 | LYYYYYMDV | 4706 |
| JHparent085 | AYFQH | 4512 | JHparent209 | NYYYYGMDV | 4654 |
| JHparent086 | DAFDI | 4538 | JHparent210 | NYYYYYMDV | 4700 |
| JHparent087 | DWFDP | 4609 | JHparent211 | PYYYYGMDV | 4661 |
| JHparent088 | DYFDY | 4566 | JHparent212 | PYYYYYMDV | 4707 |
| JHparent089 | FAFDI | 4542 | JHparent213 | RYYYYGMDV | 4659 |
| JHparent090 | FWFDP | 4612 | JHparent214 | RYYYYYMDV | 4705 |
| JHparent091 | FYFDY | 4570 | JHparent215 | SYYYYGMDV | 4658 |
| JHparent092 | GAFDI | 4549 | JHparent216 | SYYYYYMDV | 4704 |
| JHparent093 | GWFDP | 4619 | JHparent217 | TYYYYGMDV | 4663 |
| JHparent094 | GYFDL | 4533 | JHparent218 | TYYYYYMDV | 4709 |
| JHparent095 | GYFDY | 4577 | JHparent219 | VYYYYGMDV | 4665 |
| JHparent096 | GYFQH | 4510 | JHparent220 | VYYYYYMDV | 4711 |
| JHparent097 | HAFDI | 4541 | JHparent221 | AYYYYYGMDV | 4653 |
| JHparent098 | HWFDP | 4611 | JHparent222 | AYYYYYYMDV | 4699 |
| JHparent099 | HYFDY | 4569 | JHparent223 | DYYYYYGMDV | 4635 |
| JHparent100 | IAFDI | 4547 | JHparent224 | DYYYYYYMDV | 4681 |
| JHparent101 | IWFDP | 4617 | JHparent225 | FYYYYYGMDV | 4644 |
| JHparent102 | IYFDY | 4575 | JHparent226 | FYYYYYYMDV | 4690 |
| JHparent103 | IYFQH | 4508 | JHparent227 | GYYYYYGMDV | 4651 |
| JHparent104 | KYFQH | 4502 | JHparent228 | GYYYYYYMDV | 4697 |
| JHparent105 | LAFDI | 4545 | JHparent229 | HYYYYYGMDV | 4643 |
| JHparent106 | LWFDP | 4615 | JHparent230 | HYYYYYYMDV | 4689 |
| JHparent107 | LYFDY | 4573 | JHparent231 | IYYYYYGMDV | 4649 |
| JHparent108 | LYFQH | 4504 | JHparent232 | IYYYYYYMDV | 4695 |
| JHparent109 | NAFDI | 4565 | JHparent233 | LYYYYYGMDV | 4647 |
| JHparent110 | NYFDY | 4593 | JHparent234 | LYYYYYYMDV | 4693 |
| JHparent111 | PAFDI | 4546 | JHparent235 | NYYYYYGMDV | 4680 |
| JHparent112 | PWFDP | 4616 | JHparent236 | NYYYYYYMDV | 4726 |
| JHparent113 | PYFDY | 4574 | JHparent237 | PYYYYYGMDV | 4648 |
| JHparent114 | PYFQH | 4507 | JHparent238 | PYYYYYYMDV | 4694 |
| JHparent115 | QYFQH | 4503 | JHparent239 | RYYYYYGMDV | 4646 |
| JHparent116 | RAFDI | 4544 | JHparent240 | RYYYYYYMDV | 4692 |
| JHparent117 | RWFDP | 4614 | JHparent241 | SYYYYYGMDV | 4645 |
| JHparent118 | RYFDL | 4534 | JHparent242 | SYYYYYYMDV | 4691 |
| JHparent119 | RYFDY | 4572 | JHparent243 | TYYYYYGMDV | 4650 |
| JHparent120 | RYFQH | 4506 | JHparent244 | TYYYYYYMDV | 4696 |
| JHparent121 | SAFDI | 4543 | JHparent245 | VYYYYYGMDV | 4652 |
| JHparent122 | SWFDP | 4613 | JHparent246 | VYYYYYYMDV | 4698 |
| JHparent123 | SYFDY | 4571 | JHparent247 | YYYYYYGMDV | 4642 |
| JHparent124 | SYFQH | 4505 | JHparent248 | YYYYYYYMDV | 4688 |

**Table 16. Polynucleotide sequences of 27 human IGHD genes and alleles.**

| **IGHD Gene** | **Polynucleotide Sequence** | **SEQ ID NO** |
|---|---|---|
| IGHD1-(1)-01 | GGTACAACTGGAACGAC | 4739 |
| IGHD1-20 | GGTATAACTGGAACGAC | 4740 |
| IGHD1-26 | GGTATAGTGGGAGCTACTAC | 4741 |
| IGHD1-7 | GGTATAACTGGAACTAC | 4742 |
| IGHD2-15-01 | AGGATATTGTAGTGGTGGTAGCTGCTACTCC | 4743 |
| IGHD2-2-x | AGGATATTGTAGTAGTACCAGCTGCTATGCC | 4744 |
| IGHD2-2-y | AGGATATTGTAGTAGTACCAGCTGCTATACC | 4745 |
| IGHD2-2-z | TGGATATTGTAGTAGTACCAGCTGCTATGCC | 4746 |
| IGHD2-21-01 | AGCATATTGTGGTGGTGATTGCTATTCC | 4747 |
| IGHD2-21-02 | AGCATATTGTGGTGGTGACTGCTATTCC | 4748 |
| IGHD2-8-01 | AGGATATTGTACTAATGGTGTATGCTATACC | 4749 |
| IGHD3-10-01 | GTATTACTATGGTTCGGGGAGTTATTATAAC | 4750 |
| IGHD3-10-03 | GTATTACTATGGTTCAGGGAGTTATTATAAC | 4751 |
| IGHD3-16-02 | GTATTATGATTACGTTTGGGGGAGTTATGCTTATACC | 4752 |
| IGHD3-22-01 | GTATTACTATGATAGTAGTGGTTATTACTAC | 4753 |
| IGHD3-3-01 | GTATTACGATTTTTGGAGTGGTTATTATACC | 4754 |
| IGHD3-9-01 | GTATTACGATATTTTGACTGGTTATTATAAC | 4755 |
| IGHD4-17 | TGACTACGGTGACTAC | 4756 |
| IGHD4-23-01 | TGACTACGGTGGTAACTCC | 4757 |
| IGHD4-4/11-01 | TGACTACAGTAACTAC | 4758 |
| IGHD5-12-01 | GTGGATATAGTGGCTACGATTAC | 4759 |
| IGHD5-24-01 | GTAGAGATGGCTACAATTAC | 4760 |
| IGHD5-5/18-01 | GTGGATACAGCTATGGTTAC | 4761 |
| IGHD6-13-01 | GGGTATAGCAGCAGCTGGTAC | 4762 |
| IGHD6-19-01 | GGGTATAGCAGTGGCTGGTAC | 4763 |
| IGHD6-6-01 | GAGTATAGCAGCTCGTCC | 4764 |
| IGHD7-27-01 | CTAACTGGGGA | 4765 |

**Table 17. Theoretical segment pool of 73 DH parent Segments. "Z" represents a stop codon.**

| **DH Parent Segment Name** | **Amino Acid Sequence** | **SEQ ID NO** | **DH Parent Segment Name** | **Amino Acid Sequence** | **SEQ ID NO** |
|---|---|---|---|---|---|
| DHparent001 | LTG | n/a | DHparent038 | WIZWLRL | 4776 |
| DHparent002 | NWG | n/a | DHparent039 | VDIVATIT | 4369 |
| DHparent003 | ZLG | n/a | DHparent040 | AYCGGDCYS | 3862 |
| DHparent004 | DYGDY | 4348 | DHparent041 | HIVVVIAIP | 3871 |
| DHparent005 | DYSNY | 4366 | DHparent042 | HIVVVTAIP | 3891 |
| DHparent006 | TTVTT | 4351 | DHparent043 | SILWWZLLF | 4777 |
| DHparent007 | VQLER | 3737 | DHparent044 | DIVLMVYAIP | 3920 |
| DHparent008 | VZLEL | 4766 | DHparent045 | DIVVVPAAIP | 3846 |
| DHparent009 | VZLER | 4767 | DHparent046 | DIVVVPAAMP | 3816 |
| DHparent010 | VZQLV | 4768 | DHparent047 | DIVVVVAATP | 3777 |
| DHparent011 | YNWND | 3740 | DHparent048 | GYCSGGSCYS | 3767 |
| DHparent012 | YNWNY | 3765 | DHparent049 | GYCSSTSCYA | 3806 |
| DHparent013 | ZLQZL | 4769 | DHparent050 | GYCSSTSCYT | 3843 |
| DHparent014 | ZLRZL | 4770 | DHparent051 | GYCTNGVCYT | 3910 |
| DHparent015 | DYGGNS | 4354 | DHparent052 | ITIFGVVIIP | 4269 |
| DHparent016 | EYSSSS | 4476 | DHparent053 | ITIFZLVIIT | 4778 & 8746 |
| DHparent017 | GITGTT | 3743 | DHparent054 | ITMIVVVITT | 4189 |
| DHparent018 | GTTGTT | 3731 | DHparent055 | ITMVQGVIIT | 4040 |
| DHparent019 | GYSYGY | 4429 | DHparent056 | ITMVRGVIIT | 3994 |
| DHparent020 | RDGYNY | 4407 | DHparent057 | RILYZWCMLY | 4779 & 8747 |
| DHparent021 | SIAARP | 4481 | DHparent058 | RILZWWZLLL | 4780 |
| DHparent022 | TTVVTP | 4360 | DHparent059 | RILZZYQLLC | 4781 |
| DHparent023 | VZQQLV | 4771 | DHparent060 | RILZZYQLLY | 4782 |
| DHparent024 | VZQWLV | 4772 | DHparent061 | VLLWFGELLZ | 4783 |
| DHparent025 | VZWELL | 4773 | DHparent062 | VLLWFRELLZ | 4784 |
| DHparent026 | WIQLWL | 4423 | DHparent063 | VLLZZZWLLL | 4785 |
| DHparent027 | YSGSYY | 3759 | DHparent064 | VLRFLEWLLY | 4214 |
| DHparent028 | ZLRWZL | 4774 | DHparent065 | VLRYFDWLLZ | 4786 |
| DHparent029 | ZRWLQL | 4775 | DHparent066 | WILZZYQLLC | 4787 |
| DHparent030 | GIAAAGT | 4445 | DHparent067 | YYDFWSGYYT | 4242 |
| DHparent031 | GIAVAGT | 4465 | DHparent068 | YYDILTGYYN | 4318 |
| DHparent032 | GIVGATT | 3748 | DHparent069 | YYYDSSGYYY | 4161 |
| DHparent033 | GYSGYDY | 4385 | DHparent070 | YYYGSGSYYN | 3969 |
| DHparent034 | GYSSGWY | 4456 | DHparent071 | IMITFGGVMLIP | 4115 |
| DHparent035 | GYSSSWY | 4435 | DHparent072 | VLZLRLGELCLY | 4788 |
| DHparent036 | VDTAMVT | 4413 | DHparent073 | YYDYVWGSYAYT | 4070 |
| DHparent037 | VEMATIT | 4395 | | | |

**Table 18. Application of Equation 1 to Test Case 1.**

| **Type** | **Segment** | **Weight** |
|---|---|---|
| **TN1** | R | 1.0 |
| **DH** | TA | 1.0 |
| **N2** | H | 1.0 |
| **H3-JH** | HFDY (SEQ ID NO: 4582) | 1.0 |

**Table 19. Application of Equation 1 to Test Cases 2.1 and 2.1.**

| **Type** | **Segment** | **Weight** |
|---|---|---|
| **TN1** | V and VG | 0.5 each |
| **DH** | GIVGA (SEQ ID NO: 3751) and IVGA (SEQ ID NO: 3755) | 0.5 each |
| **N2** | AS | 1.0 |
| **H3-JH** | Y | 1.0 |

**Table 20. Application of Equation 1 to Test Case 3.1.**

| **Type** | **Segment** | **Weight** |
|---|---|---|
| **TN1** | DR | 1.0 |
| **DH** | YSGYD (SEQ ID NO: 4389) | 0.8 |
| **N2** | LG | 1.0 |
| **H3-JH** | Y | 1.0 |

**Table 21. Application of Equation 1 to Test Cases 4.1 and 4.2.**

| **Type** | **Segment** | **Weight** |
|---|---|---|
| **TN1** | "-" and G | 0.5 each |
| **DH** | GIAAA (SEQ ID NO: 4448) and IAAA (SEQ ID NO: 4452) | 0.5 each |
| **N2** | D | 1.0 |
| **H3**-**JH** | SNWFDP (SEQ ID NO: 4600) | 0.83 |

**Table 22. Application of Equation 1 to all Test Cases.**

| **Type** | **Segments** | **Weight** |
|---|---|---|
| **TN1** | DR, R, VG, V, G and "-" | 0.25, 0.25, 0.125, 0.125, 0.125 and 0.125 respectively |
| **DH** | TA, YSGYD (SEQ ID NO: 4389), IAAA (SEQ ID NO: 4452), GIAAA (SEQ ID NO: 4448), IVGA (SEQ ID NO: 3755) and GIVGA (SEQ ID NO: 3751) | 0.25, 0.20, 0.125, 0.125, 0.125 and 0.125 respectively |
| **N2** | AS, H, D and LG | 0.25 each |
| **H3**-**JH** | Y, HFDY (SEQ ID NO: 4582) and SNWFDP (SEQ ID NO: 4600) | 0.50, 0.25 and 0.209 |

**Table 23. Segments used in Exemplary Library Design 1 (ELD-1). The sequences collectively form a theoretical segment pool that comprises individual theoretical segment pools of TN1, DH, N2, and H3-JH segments.**

| **Segment No.** | **TN1** | **SEQ ID NO** | **DH** | **SEQ ID NO** | **N2** | **H3**-**JH** | **SEQ ID NO** |
|---|---|---|---|---|---|---|---|
| 1 | - | n/a | YYYDSSGYY | 4162 | - | DAFDI | 4538 |
| 2 | G | n/a | YGDY | 4350 | Y | YYFDY | 4568 |
| 3 | D | n/a | DYGDY | 4348 | G | Y | n/a |
| 4 | A | n/a | YYYDSSGY | 4164 | D | FDY | n/a |
| 5 | V | n/a | YCSSTSCY | 3810 | S | DY | n/a |
| 6 | DR | n/a | YYDSSGY | 4168 | P | YGMDV | 4640 |
| 7 | S | n/a | YCSGGSCY | 3771 | F | - | n/a |
| 8 | L | n/a | YCSSTSC | 3812 | L | LDY | n/a |
| 9 | DL | n/a | GG | n/a | A | GAFDI | 4549 |
| 10 | R | n/a | RG | n/a | E | YFDY | 4567 |
| 11 | GR | n/a | SGSY | 3763 | V | YYGMDV | 4639 |
| 12 | T | n/a | YYDSSGYY | 4165 | H | AFDI | 4539 |
| 13 | GG | n/a | SS | n/a | T | PFDY | 4587 |
| 14 | E | n/a | YDFWSGY | 4249 | R | GMDV | 4641 |
| 15 | DS | n/a | GYCSSTSC | 3809 | W | GWFDP | 4619 |
| 16 | VG | n/a | DY | n/a | SG | YYYYGMDV | 4637 |
| 17 | DG | n/a | YYYDSSG | 4167 | I | IDY | n/a |
| 18 | AP | n/a | CSSTSCY | 3813 | RG | GYFDY | 4577 |
| 19 | GL | n/a | YYDFWSGY | 4245 | K | GFDY | 4590 |
| 20 | GS | n/a | SSGWY | 4461 | LG | MDV | n/a |
| 21 | DRG | n/a | AG | n/a | Q | VDY | n/a |
| 22 | DLG | n/a | DSSGY | 4179 | GP | YYYGMDV | 4638 |
| 23 | VP | n/a | SSSW | 4443 | PG | NWFDP | 4595 |
| 24 | DP | n/a | VGAT | 3756 | LP | PDY | n/a |
| 25 | P | n/a | SY | n/a | AG | WFDP | 4596 |
| 26 | EG | n/a | DTAM | 4420 | GS | NFDY | 4580 |
| 27 | GA | n/a | IAAAG | 4449 | TS | YWYFDL | 4527 |
| 28 | AG | n/a | YSSSW | 4439 | SS | NAFDI | 4565 |
| 29 | GV | n/a | GS | n/a | GG | HFDY | 4582 |
| 30 | GP | n/a | YYDSSG | 4172 | YS | SFDY | 4584 |
| 31 | ER | n/a | VG | n/a | M | YYYYYGMDV | 4636 |
| 32 | DV | n/a | YSSSWY | 4437 | SL | DYYYGMDV | 4668 |
| 33 | VGG | n/a | YCSGGSC | 3773 | SP | DFDY | 4581 |
| 34 | SG | n/a | YDSSGYY | 4169 | SD | YNWFDP | 4597 |
| 35 | GRG | n/a | GI | n/a | AP | DYYYYGMDV | 4655 |
| 36 | DA | n/a | GYCSGGSCY | 3768 | GR | YYYMDV | 4685 |
| 37 | DRP | n/a | YSSS | 4442 | TG | LFDY | 4586 |
| 38 | DSG | n/a | SSGW | 4463 | SR | DYFDY | 4566 |
| 39 | GPR | n/a | TA | n/a | LD | NYYYYGMDV | 4654 |
| 40 | DT | n/a | DSSGYY | 4174 | LS | GDY | n/a |
| 41 | GGG | n/a | GYCSSTSCY | 3807 | GA | YDY | n/a |
| 42 | DRGG | 3720 | TTVT | 4352 | VG | SYFDY | 4571 |
| 43 | PL | n/a | YSSGWY | 4458 | PP | YYMDV | 4686 |
| 44 | DPS | n/a | GW | n/a | RR | TFDY | 4589 |
| 45 | LP | n/a | LG | n/a | GSG | YYYYYYGMDV | 4642 |
| 46 | RG | n/a | DYGD | 4349 | GT | FFDY | 4583 |
| 47 | GT | n/a | TVTT | 4353 | TP | SYYYYGMDV | 4658 |
| 48 | LG | n/a | AAA | n/a | RP | VFDY | 4591 |
| 49 | DLP | n/a | YSSGW | 4460 | RD | YAFDI | 4540 |
| 50 | DGR | n/a | LV | n/a | QL | SDY | n/a |
| 51 | ERG | n/a | YYDFWSGYY | 4243 | TT | WYFDL | 4528 |
| 52 | DGS | n/a | YYDSSGYYY | 4163 | PL | DWFDP | 4609 |
| 53 | ES | n/a | YYDFWSG | 4248 | RS | AFDY | 4592 |
| 54 | PS | n/a | YGD | n/a | WS | PYYYYGMDV | 4661 |
| 55 | GGS | n/a | YG | n/a | RV | HYFDY | 4569 |
| b6 | DPR | n/a | GT | n/a | RF | DV | n/a |
| 57 | EA | n/a | YSGSY | 3760 | SF | RFDY | 4585 |
| 58 | GGR | n/a | YYYGSGSY | 3972 | PT | NYFDY | 4593 |
| 59 | DGG | n/a | LR | n/a | PS | IFDY | 4588 |
| 60 | SP | n/a | SSS | n/a | AT | ADY | n/a |
| 61 | DPG | n/a | GD | n/a | RL | HYYYGMDV | 4669 |
| 62 | DSGG | 3723 | CSGGSCY | 3774 | SV | GYYYYYGMDV | 4651 |
| 63 | DPL | n/a | GY | n/a | RA | DWYFDL | 4530 |
| 64 | TP | n/a | YCGGDCY | 3866 | GRG | GYYYGMDV | 4677 |
| 65 | AGG | n/a | QWLV | 4475 | VP | YYYYMDV | 4684 |
| 66 | PR | n/a | IAAA | 4452 | SQ | V | n/a |
| 67 | DGT | n/a | QG | n/a | AS | FDP | n/a |
| 68 | GLG | n/a | YCSSTSCYT | 3844 | PR | DDY | n/a |
| 69 | DSP | n/a | SG | n/a | VD | GYYYYGMDV | 4664 |
| 70 | GGV | n/a | TTVTT | 4351 | GV | YMDV | 468 |
| 71 | GPS | n/a | QQL | n/a | LT | NWYFDL | 4532 |
| 72 | GVG | n/a | IAVA | 4472 | TR | PYFDY | 4574 |
| 73 | GGA | n/a | GDY | n/a | VS | FDI | n/a |
| 74 | RP | n/a | YYYDSSGYYY | 4161 | SGL | NDY | n/a |
| 75 | DPP | n/a | DGYN | 4411 | RGG | HYYYYYGMDV | 4643 |
| 76 | DPT | n/a | CSSTSC | 3814 | LR | VYYYGMDV | 4678 |
| 77 | EV | n/a | IVVVPAAI | 3849 | SGG | VYFDY | 4578 |
| 78 | GAP | n/a | AVAG | 4473 | SA | SNWFDP | 4600 |
| 79 | DGRG | 3702 | AA | n/a | AY | NYYYGMDV | 4667 |
| 80 | GPP | n/a | DSSG | 4185 | LE | LYYYYGMDV | 4660 |
| 81 | DLGG | 3714 | VR | n/a | TGG | DYYYYYGMDV | 4635 |
| 82 | ET | n/a | YDFWSG | 4253 | GL | AAFDI | 4551 |
| 83 | DGGP | 3681 | GYSSSWY | 4435 | GAG | SWFDP | 4613 |
| 84 | DGGR | 3684 | SGW | n/a | TA | SYYYYYGMDV | 4645 |
| 85 | DGSG | 3705 | WG | n/a | IS | SYYYGMDV | 4671 |
| 86 | GTG | n/a | SGSYY | 3761 | ST | P | n/a |
| 87 | DPGG | 3717 | RY | n/a | SSE | YYYYYMDV | 4683 |
| 88 | DGGS | 3687 | DS | n/a | GLG | RAFDI | 4544 |
| 89 | GGT | n/a | TT | n/a | PRP | RYFDY | 4572 |
| 90 | GGRG | 3703 | YCSGGSCYS | 3769 | RPL | FYYYYGMDV | 4657 |
| 91 | EGR | n/a | PA | n/a | EA | GNWFDP | 4606 |
| 92 | DGA | n/a | IVVVPAA | 3823 | LGG | RWFDP | 4614 |
| 93 | DGL | n/a | IAAAGT | 4447 | FS | GFDP | 4623 |
| 94 | EL | n/a | AAAG | 4453 | DL | DYYYYMDV | 4714 |
| 95 | EGG | n/a | GYCSGGSC | 3770 | GGG | PYYYGMDV | 4674 |
| 96 | LGG | n/a | SGWY | 4464 | AR | DP | n/a |
| 97 | DPA | n/a | DYGGN | 4355 | RE | HAFDI | 4541 |
| 98 | EGV | n/a | YYYDS | 4176 | PA | DNWFDP | 4594 |
| 91 | GSG | n/a | YSSG | 4462 | PTQ | LNWFDP | 4602 |
| 100 | GGP | n/a | YDSSGY | 4173 | AGG | AYYYYGMDV | 4666 |
| 101 | | | VAG | n/a | GGV | | |
| 102 | | | SSW | n/a | SI | | |
| 103 | | | GSGSY | 3987 | SAA | | |
| 104 | | | SSSS | 4480 | GGS | | |
| 105 | | | NW | n/a | GGA | | |
| 106 | | | DFWSGY | 4254 | GPG | | |
| 107 | | | QQLV | 4455 | PGG | | |
| 108 | | | YGGN | 4358 | AAE | | |
| 109 | | | YDSSG | 4178 | GGR | | |
| 110 | | | GYSYG | 4430 | TW | | |
| 111 | | | TV | n/a | GGP | | |
| 112 | | | NG | n/a | GGL | | |
| 113 | | | IVGAT | 3752 | VGG | | |
| 114 | | | IVGA | 3755 | GTG | | |
| 115 | | | YGSGSY | 9881 | NR | | |
| 116 | | | SSWY | 4444 | NTP | | |
| 117 | | | ST | n/a | PV | | |
| 118 | | | DFWSGYY | 4250 | EK | | |
| 119 | | | GSY | n/a | GVG | | |
| 120 | | | YYDSS | 4177 | KV | | |
| 121 | | | VGA | n/a | EKR | | |
| 122 | | | AT | n/a | QT | | |
| 123 | | | RP | n/a | SE | | |
| 124 | | | YYYGSGS | 3975 | SAL | | |
| 125 | | | GIAAAG | 4446 | FH | | |
| 126 | | | SGY | n/a | RH | | |
| 127 | | | TG | n/a | PTA | | |
| 128 | | | LT | n/a | HL | | |
| 129 | | | RD | n/a | WGT | | |
| 130 | | | WEL | n/a | REL | | |
| 131 | | | YSYG | 4433 | NT | | |
| 132 | | | TVT | n/a | HW | | |
| 133 | | | GYCSGGSCYS | 3767 | PKS | | |
| 134 | | | AR | n/a | DT | | |
| 135 | | | YYGSGSY | 3976 | DTL | | |
| 136 | | | RW | n/a | SK | | |
| 137 | | | DIVVVPA | 3822 | TG | | |
| 138 | | | YSGS | 3762 | AYH | | |
| 139 | | | GYSSSW | 4436 | ISR | | |
| 140 | | | YSSSS | 4478 | GGT | | |
| 141 | | | YYYDSS | 4171 | QE | | |
| 142 | | | QL | n/a | | | |
| 143 | | | GYSGYD | 4386 | | | |
| 144 | | | GE | n/a | | | |
| 145 | | | MA | n/a | | | |
| 146 | | | DSS | n/a | | | |
| 147 | | | RF | n/a | | | |
| 148 | | | DTAMV | 4417 | | | |
| 149 | | | YYGSGSYY | 3973 | | | |
| 150 | | | VDTAMV | 4414 | | | |
| 151 | | | FGVV | 4293 | | | |
| 152 | | | EYSSS | 4477 | | | |
| 153 | | | TTV | n/a | | | |
| 154 | | | SWY | n/a | | | |
| 155 | | | IAARP | 4483 | | | |
| 156 | | | VE | n/a | | | |
| 157 | | | SIAA | 4484 | | | |
| 158 | | | YSGYD | 4389 | | | |
| 159 | | | DIVVVPAA | 3819 | | | |
| 160 | | | CSGGSC | 3775 | | | |
| 161 | | | DW | n/a | | | |
| 162 | | | TS | n/a | | | |
| 163 | | | RL | n/a | | | |
| 164 | | | YSS | n/a | | | |
| 165 | | | GN | n/a | | | |
| 166 | | | SN | n/a | | | |
| 167 | | | GYSY | 4432 | | | |
| 168 | | | YYDS | 4183 | | | |
| 169 | | | VDTAM | 4416 | | | |
| 170 | | | LE | n/a | | | |
| 171 | | | AVAGT | 4470 | | | |
| 172 | | | YSY | n/a | | | |
| 173 | | | SW | n/a | | | |
| 174 | | | SSG | n/a | | | |
| 175 | | | FGV | n/a | | | |
| 176 | | | VP | n/a | | | |
| 177 | | | VA | n/a | | | |
| 178 | | | SYY | n/a | | | |
| 179 | | | QWL | n/a | | | |
| 180 | | | GSG | n/a | | | |
| 181 | | | TIFGVV | 4280 | | | |
| 182 | | | AVA | n/a | | | |
| 183 | | | FWSGY | 4260 | | | |
| 184 | | | YSGSYY | 3759 | | | |
| 185 | | | IAVAG | 4469 | | | |
| 186 | | | YS | n/a | | | |
| 187 | | | YQL | n/a | | | |
| 188 | | | SIAAR | 4482 | | | |
| 189 | | | YCGGDC | 3868 | | | |
| 190 | | | NWNY | 3766 | | | |
| 191 | | | SSSWY | 4440 | | | |
| 192 | | | GIAVA | 4468 | | | |
| 193 | | | YSYGY | 4431 | | | |
| 194 | | | GIAAA | 4448 | | | |
| 195 | | | YYG | n/a | | | |
| 196 | | | AAG | n/a | | | |
| 197 | | | AV | n/a | | | |
| 198 | | | AYCGGDCY | 3863 | | | |
| 199 | | | YYGSGS | 3980 | | | |
| 200 | | | EY | n/a | | | |

**Table 24. Segments used in Exemplary Library Design 2 (ELD-2). The sequences collectively form a theoretical segment pool that comprises individual theoretical segment pools of TN1, DH, N2, and H3-JH segments.**

| **Seg men t No.** | **TN1** | **SEQ ID NO** | **DH** | **SEQ ID NO** | **N2** | **SEQ ID NO** | **H3-JH** | **SEQ ID NO** |
|---|---|---|---|---|---|---|---|---|
| 1 | - | n/a | YYYDSSGYY | 4162 | - | n/a | DAFDI | 4538 |
| 2 | G | n/a | YGDY | 4350 | G | n/a | YYFDY | 4568 |
| 3 | D | n/a | DYGDY | 4348 | D | n/a | Y | n/a |
| 4 | A | n/a | YYYDSSGY | 4164 | A | n/a | FDY | n/a |
| 5 | V | n/a | YCSSTSCY | 3810 | V | n/a | DY | n/a |
| 6 | S | n/a | YYDSSGY | 4168 | V | n/a | YGMDV | 4640 |
| 7 | DR | n/a | YCSGGSCY | 3771 | DR | n/a | - | n/a |
| 8 | L | n/a | YCSSTSC | 3812 | L | n/a | LDY | n/a |
| 9 | R | n/a | GG | n/a | R | n/a | GAFDI | 4549 |
| 10 | DL | n/a | RG | n/a | DL | n/a | YFDY | 4567 |
| 11 | T | n/a | SGSY | 3763 | T | n/a | YYGMDV | 4639 |
| 12 | E | n/a | YYDSSGYY | 4165 | E | n/a | AFDI | 4539 |
| 13 | GR | n/a | SS | n/a | GR | n/a | PFDY | 4587 |
| 14 | GG | n/a | YDFWSGY | 4249 | GG | n/a | GMDV | 4641 |
| 15 | DG | n/a | GYCSSTSC | 3809 | DG | n/a | GWFDP | 4619 |
| 16 | DS | n/a | DY | n/a | DS | n/a | YYYYGMDV | 4637 |
| 17 | VG | n/a | YYYDSSG | 4167 | VG | n/a | IDY | n/a |
| 18 | EG | n/a | CSSTSCY | 3813 | EG | n/a | GYFDY | 4577 |
| 19 | P | n/a | YYDFWSGY | 4245 | P | n/a | GFDY | 4590 |
| 20 | GL | n/a | SSGWY | 4461 | GL | n/a | MDV | n/a |
| 21 | GS | n/a | AG | n/a | GS | n/a | VDY | n/a |
| 22 | DP | n/a | DSSGY | 4179 | DP | n/a | YYYGMDV | 4638 |
| 23 | GP | n/a | SSSW | 4443 | GP | n/a | NWFDP | 4595 |
| 24 | GA | n/a | VGAT | 3756 | GA | n/a | PDY | n/a |
| 25 | GV | n/a | SY | n/a | GV | n/a | WFDP | 4596 |
| 26 | H | n/a | DTAM | 4420 | H | n/a | NFDY | 4580 |
| 27 | DRG | n/a | IAAAG | 4449 | DRG | n/a | YWYFDL | 4527 |
| 28 | DQ | n/a | YSSSW | 4439 | DQ | n/a | NAFDI | 4565 |
| 29 | AG | n/a | GS | n/a | AG | n/a | HFDY | 4582 |
| 30 | DLG | n/a | YYDSSG | 4172 | DLG | n/a | SFDY | 4584 |
| 31 | DV | n/a | VG | n/a | DV | n/a | YYYYYGMDV | 4636 |
| 32 | Q | n/a | YSSSWY | 4437 | Q | n/a | DYYYGMDV | 4668 |
| 33 | N | n/a | YCSGGSC | 3773 | N | n/a | DFDY | 4581 |
| 34 | AP | n/a | YDSSGYY | 4169 | AP | n/a | YNWFDP | 4597 |
| 35 | GGG | n/a | GI | n/a | GGG | n/a | DYYYYGMDV | 4655 |
| 36 | DH | n/a | GYCSGGSCY | 3768 | DH | n/a | YYYMDV | 4685 |
| 37 | VP | n/a | YSSS | 4442 | VP | n/a | LFDY | 4586 |
| 38 | SG | n/a | SSGW | 4463 | SG | n/a | DYFDY | 4566 |
| 39 | GRG | n/a | TA | n/a | GRG | n/a | NYYYYGMDV | 4654 |
| 40 | AR | n/a | DSSGYY | 4174 | AR | n/a | GDY | n/a |
| 41 | RG | n/a | GYCSSTSCY | 3807 | RG | n/a | YDY | n/a |
| 42 | ER | n/a | TTVT | 4352 | ER | n/a | SYFDY | 4571 |
| 43 | DA | n/a | YSSGWY | 4458 | DA | n/a | YYMDV | 4686 |
| 44 | AS | n/a | GW | n/a | AS | n/a | TFDY | 4589 |
| 45 | PL | n/a | LG | n/a | PL | n/a | YYYYYYGMDV | 4642 |
| 46 | DQG | n/a | DYGD | 4349 | DQG | n/a | FFDY | 4583 |
| 47 | VL | n/a | TVTT | 4353 | VL | n/a | SYYYYGMDV | 4658 |
| 48 | GT | n/a | AAA | n/a | GT | n/a | VFDY | 4591 |
| 49 | DGG | n/a | YSSGW | 4460 | DGG | n/a | YAFDI | 4540 |
| 50 | DSG | n/a | LV | n/a | DSG | n/a | SDY | n/a |
| 51 | VGG | n/a | YYDFWSGYY | 4243 | VGG | n/a | WYFDL | 4528 |
| 52 | F | n/a | YYDSSGYYY | 4163 | F | n/a | DWFDP | 4609 |
| 53 | AL | n/a | YYDFWSG | 4248 | AL | n/a | AFDY | 4592 |
| 54 | PS | n/a | YGD | n/a | PS | n/a | PYYYYGMDV | 4661 |
| 55 | ES | n/a | YG | n/a | ES | n/a | HYFDY | 4569 |
| 56 | ERG | n/a | GT | n/a | ERG | n/a | DV | n/a |
| 57 | GGV | n/a | YSGSY | 3760 | GGV | n/a | RFDY | 4585 |
| 58 | DRP | n/a | YYYGSGSY | 3972 | DRP | n/a | NYFDY | 4593 |
| 59 | EA | n/a | LR | n/a | EA | n/a | IFDY | 4588 |
| 60 | TP | n/a | SSS | n/a | TP | n/a | ADY | n/a |
| 61 | GPR | n/a | GD | n/a | GPR | n/a | HYYYGMDV | 4669 |
| 62 | LH | n/a | CSGGSCY | 3774 | LH | n/a | GYYYYYGMDV | 4651 |
| 63 | SR | n/a | GY | n/a | SR | n/a | DWYFDL | 4530 |
| 64 | LP | n/a | YCGGDCY | 3866 | LP | n/a | GYYYGMDV | 4677 |
| 65 | LG | n/a | QWLV | 4475 | LG | n/a | YYYYMDV | 4684 |
| 66 | DT | n/a | IAAA | 44b2 | DT | n/a | V | n/a |
| 67 | VA | n/a | QG | n/a | VA | n/a | FDP | n/a |
| 68 | SL | n/a | YCSSTSCYT | 3844 | SL | n/a | DDY | n/a |
| 69 | EGG | n/a | SG | n/a | EGG | n/a | GYYYYGMDV | 4664 |
| 70 | DRS | n/a | TTVTT | 4351 | DRS | n/a | YMDV | 4687 |
| 71 | K | n/a | QQL | n/a | K | n/a | NWYFDL | 4532 |
| 72 | DPG | n/a | IAVA | 4472 | DPG | n/a | PYFDY | 4574 |
| 73 | I | n/a | GDY | n/a | I | n/a | FDI | n/a |
| 74 | GD | n/a | YYYDSSGYYY | 4161 | GD | n/a | NDY | n/a |
| 75 | DGT | n/a | DGYN | 4411 | DGT | n/a | HYYYYYGMDV | 4643 |
| 76 | GPP | n/a | CSSTSC | 3814 | GPP | n/a | VYYYGMDV | 4678 |
| 77 | DPP | n/a | IVVVPAAI | 3849 | DPP | n/a | VYFDY | 4578 |
| 78 | RR | n/a | AVAG | 4473 | RR | n/a | SNWFDP | 4600 |
| 79 | EGV | n/a | AA | n/a | EGV | n/a | NYYYGMDV | 4667 |
| 80 | GF | n/a | DSSG | 4185 | GF | n/a | LYYYYGMDV | 4660 |
| 81 | GVG | n/a | VR | n/a | GVG | n/a | DYYYYYGMDV | 4635 |
| 82 | DPS | n/a | YDFWSG | 4253 | DPS | n/a | AAFDI | 4551 |
| 83 | VD | n/a | GYSSSWY | 4435 | VD | n/a | SWFDP | 4613 |
| 84 | GGT | n/a | SGW | n/a | GGT | n/a | SYYYYYGMDV | 4645 |
| 85 | DK | n/a | WG | n/a | DK | n/a | SYYYGMDV | 4671 |
| 86 | GTG | n/a | SGSYY | 3761 | GTG | n/a | P | n/a |
| 87 | DF | n/a | RY | n/a | DF | n/a | YYYYYMDV | 4683 |
| 88 | GQ | n/a | DS | n/a | GQ | n/a | RAFDI | 4544 |
| 89 | SP | n/a | TT | n/a | SP | n/a | RYFDY | 4572 |
| 90 | QG | n/a | YCSGGSCYS | 3769 | QG | n/a | FYYYYGMDV | 4657 |
| 91 | DLT | n/a | PA | n/a | DLT | n/a | GNWFDP | 4606 |
| 92 | AK | n/a | IVVVPAA | 3823 | AK | n/a | RWFDP | 4614 |
| 93 | GPS | n/a | IAAAGT | 4447 | GPS | n/a | GFDP | 4623 |
| 94 | QR | n/a | AAAG | 4453 | QR | n/a | DYYYYMDV | 4714 |
| 95 | VR | n/a | GYCSGGSC | 3770 | VR | n/a | PYYYGMDV | 4674 |
| 96 | DSP | n/a | SGWY | 4464 | DSP | n/a | DP | n/a |
| 97 | DPL | n/a | DYGGN | 4355 | DPL | n/a | HAFDI | 4541 |
| 98 | EGR | n/a | YYYDS | 4176 | EGR | n/a | DNWFDP | 4594 |
| 91 | GRRG | 4789 | YSSG | 4462 | GRRG | 4789 | LNWFDP | 4602 |
| 100 | EV | n/a | YDSSGY | 4173 | EV | n/a | AYYYYGMDV | 4666 |
| 101 | | | VAG | n/a | RP | n/a | | |
| 102 | | | SSW | n/a | GH | n/a | | |
| 103 | | | GSGSY | 3987 | DGR | n/a | | |
| 104 | | | SSSS | 4480 | AA | n/a | | |
| 105 | | | NW | n/a | DD | n/a | | |
| 106 | | | DFWSGY | 4254 | W | n/a | | |
| 107 | | | QQLV | 4455 | GGS | n/a | | |
| 108 | | | YGGN | 4358 | DIS | n/a | | |
| 109 | | | YDSSG | 4178 | GGA | n/a | | |
| 110 | | | GYSYG | 4430 | GK | n/a | | |
| 111 | | | TV | n/a | DGP | n/a | | |
| 112 | | | NG | n/a | DLK | n/a | | |
| 113 | | | IVGAT | 3752 | ET | n/a | | |
| 114 | | | IVGA | 3755 | TT | n/a | | |
| 115 | | | YGSGSY | 3981 | VH | n/a | | |
| 116 | | | SSWY | 4444 | AE | n/a | | |
| 117 | | | ST | n/a | VS | n/a | | |
| 118 | | | DFWSGYY | 4250 | LGG | n/a | | |
| 119 | | | GSY | n/a | C | n/a | | |
| 120 | | | YYDSS | 4177 | DKG | n/a | | |
| 121 | | | VGA | n/a | HA | n/a | | |
| 122 | | | AT | n/a | VI | n/a | | |
| 123 | | | RP | n/a | HP | n/a | | |
| 124 | | | YYYGSGS | 3975 | GGE | n/a | | |
| 125 | | | GIAAAG | 4446 | EP | n/a | | |
| 126 | | | SGY | n/a | EF | n/a | | |
| 127 | | | TG | n/a | DRN | n/a | | |
| 128 | | | LT | n/a | DWG | n/a | | |
| 129 | | | RD | n/a | GE | n/a | | |
| 130 | | | WEL | n/a | DRA | n/a | | |
| 131 | | | YSYG | 4433 | VN | n/a | | |
| 132 | | | TVT | n/a | DRE | n/a | | |
| 133 | | | GYCSGGSCYS | 3767 | DLA | n/a | | |
| 134 | | | AR | n/a | EN | n/a | | |
| 135 | | | YYGSGSY | 3976 | VT | n/a | | |
| 136 | | | RW | n/a | HG | n/a | | |
| 137 | | | DIVVVPA | 3822 | RA | n/a | | |
| 138 | | | YSGS | 3762 | M | n/a | | |
| 139 | | | GYSSSW | 4436 | DVP | n/a | | |
| 140 | | | YSSSS | 4478 | GAP | n/a | | |
| 141 | | | YYYDSS | 4171 | GLG | n/a | | |
| 142 | | | QL | n/a | GPG | n/a | | |
| 143 | | | GYSGYD | 4386 | PG | n/a | | |
| 144 | | | GE | n/a | DSS | n/a | | |
| 145 | | | MA | n/a | SS | n/a | | |
| 146 | | | DSS | n/a | AGG | n/a | | |
| 147 | | | RF | n/a | GGR | n/a | | |
| 148 | | | DTAMV | 4417 | GPN | n/a | | |
| 149 | | | YYGSGSYY | 3973 | DRL | n/a | | |
| 150 | | | VDTAMV | 4414 | GRR | n/a | | |
| 151 | | | FGVV | 4293 | DSGG | 3723 | | |
| 152 | | | EYSSS | 4477 | TR | n/a | | |
| 153 | | | TTV | n/a | DLS | n/a | | |
| 154 | | | SWY | n/a | RGG | n/a | | |
| 155 | | | IAARP | 4483 | Y | n/a | | |
| 156 | | | VE | n/a | EVR | n/a | | |
| 157 | | | SIAA | 4484 | LI | n/a | | |
| 158 | | | YSGYD | 4389 | TF | n/a | | |
| 159 | | | DIVVVPAA | 3819 | LK | n/a | | |
| 160 | | | CSGGSC | 3775 | DLE | n/a | | |
| 161 | | | DW | n/a | GY | n/a | | |
| 162 | | | TS | n/a | DGS | n/a | | |
| 163 | | | RL | n/a | GVR | n/a | | |
| 164 | | | YSS | n/a | GQR | n/a | | |
| 165 | | | GN | n/a | EGL | n/a | | |
| 166 | | | SN | n/a | VLG | n/a | | |
| 167 | | | GYSY | 4432 | QP | n/a | | |
| 168 | | | YYDS | 4183 | VM | n/a | | |
| 169 | | | VDTAM | 4416 | VE | n/a | | |
| 170 | | | LE | n/a | DQGG | 4790 | | |
| 171 | | | AVAGT | 4470 | PN | n/a | | |
| 172 | | | YSY | n/a | DGL | n/a | | |
| 173 | | | SW | n/a | PV | n/a | | |
| 174 | | | SSG | n/a | HR | n/a | | |
| 175 | | | FGV | n/a | AD | n/a | | |
| 176 | | | VP | n/a | DLF | n/a | | |
| 177 | | | VA | n/a | LD | n/a | | |
| 178 | | | SYY | n/a | GGD | n/a | | |
| 179 | | | QWL | n/a | DRR | n/a | | |
| 180 | | | GSG | n/a | DHH | n/a | | |
| 181 | | | TIFGVV | 4280 | DW | n/a | | |
| 182 | | | AVA | n/a | DAS | n/a | | |
| 183 | | | FWSGY | 4260 | GW | n/a | | |
| 184 | | | YSGSYY | 3759 | SV | n/a | | |
| 185 | | | IAVAG | 4469 | GLR | n/a | | |
| 186 | | | YS | n/a | DGA | n/a | | |
| 187 | | | YQL | n/a | LA | n/a | | |
| 188 | | | SIAAR | 4482 | EEG | n/a | | |
| 189 | | | YCGGDC | 3868 | AV | n/a | | |
| 190 | | | NWNY | 3766 | VQ | n/a | | |
| 191 | | | SSSWY | 4440 | AH | n/a | | |
| 192 | | | GIAVA | 4468 | RS | n/a | | |
| 193 | | | YSYGY | 4431 | WA | n/a | | |
| 194 | | | GIAAA | 4448 | LR | n/a | | |
| 195 | | | YYG | n/a | GSG | n/a | | |
| 196 | | | AAG | n/a | GGSG | 3706 | | |
| 197 | | | AV | n/a | DLR | n/a | | |
| 198 | | | AYCGGDCY | 3863 | VWG | n/a | | |
| 199 | | | YYGSGS | 3980 | HL | n/a | | |
| 200 | | | EY | n/a | EH | n/a | | |

**Table 25. Segments used in Exemplary Library Design 3 (ELD-3). The sequences collectively form a theoretical segment pool that comprises individual theoretical segment pools of TN1, DH, N2, and H3-JH segments.**

| **Segment No.** | **TN1 (plus AR or AK)¹** | **SEQ ID NO** | **TN1 Nucleotides (plus AR or AK)¹** | **SEQ ID NO** | **DH** | **SEQ ID NO** | **DH Nucleotides** | **SEQ ID NO** | **N 2** | **N2 Nucleotides** | **H3-JH** | **SEQ ID NO** | **H3-JH Nucleotides** | **SEQ ID NO** |
|---|---|---|---|---|---|---|---|---|---|---|---|---|---|---|
| 1 | AR | n/a | GCCAGA | n/a | GT | n/a | GGTACT | n/a | - | - | - | n/a | - | n/a |
| 2 | ARE | n/a | GCCAGAG AG | n/a | TT | n/a | ACTACT | n/a | A | GCT | Y | n/a | TAT | n/a |
| 3 | ARD | n/a | GCCAGAG AC | n/a | TG | n/a | ACAGGC | n/a | D | GAT | DI | n/a | GATATT | n/a |
| 4 | ARG | n/a | GCCAGAG GA | n/a | ER | n/a | GAGCGT | n/a | E | GAG | DL | n/a | GACTTG | n/a |
| 5 | AREG | 4791 | GCCAGAG AGGGA | 4963 | QLE | n/a | CAATTAGA G | n/a | F | TTC | DP | n/a | GATCCT | n/a |
| 6 | ARDG | 4792 | GCCAGAG ACGGA | 4964 | LER | n/a | TTAGAGCG T | n/a | G | GGC | DV | n/a | GATGTA | n/a |
| 7 | ARGG | 4793 | GCCAGAG GTGGA | 4965 | VGAT | 3756 | GTTGGCGC AACT | 5135 | H | CAT | DY | n/a | GACTAT | n/a |
| 8 | ARR | n/a | GCCAGGA GA | n/a | YSG | n/a | TATAGTGG T | n/a | I | ATC | QH | n/a | CAGCAC | n/a |
| 9 | ARER | 4794 | GCCAGAG AGAGA | 4966 | YSGS Y | 3760 | TACTCTGG CTCTTAT | 5136 | K | AAA | ADY | n/a | GCTGATT AT | n/a |
| 10 | ARDR | 4795 | GCCAGAG ACAGA | 4967 | VG | n/a | GTAGGC | n/a | L | CTG | DDY | n/a | GATGACT AT | n/a |
| 11 | ARGR | 4796 | GCCAGAG GCAGA | 4968 | AT | n/a | GCCACT | n/a | M | ATG | FDI | n/a | TTTGACA TT | n/a |
| 12 | ARS | n/a | GCCAGAT CT | n/a | WEL | n/a | TGGGAGCT T | n/a | P | CCT | FDL | n/a | TTCGACT TA | n/a |
| 13 | ARES | 4797 | GCCAGAG AATCT | 4969 | YS | n/a | TACAGC | n/a | Q | CAG | FDP | n/a | TTTGACC CT | n/a |
| 14 | ARDS | 4798 | GCCAGAG ACTCT | 4970 | SG | n/a | AGTGGT | n/a | R | AGG | FDY | n/a | TTCGACT AT | n/a |
| 15 | ARGS | 4799 | GCCAGAG GTTCT | 4971 | GS | n/a | GGTTCT | n/a | S | TCA | FQH | n/a | TTCCAGC AC | n/a |
| 16 | ARP | n/a | GCCAGAC CT | n/a | SY | n/a | AGCTAC | n/a | T | ACC | GDY | n/a | GGTGACT AC | n/a |
| 17 | ARDP | 4800 | GCCAGAG ACCCT | 4972 | CSST SC | 3814 | TGTAGTAG TACAAGTT GC | 5137 | V | GTT | IDY | n/a | ATCGACT AT | n/a |
| 18 | ARGP | 4801 | GCCAGAG GGCCT | 4973 | CSST SCY | 3813 | TGCTCATC TACATCAT GCTAT | 5138 | W | TGG | LDY | n/a | TTGGACT AT | n/a |
| 19 | ARL | n/a | GCCAGAT TG | n/a | YCSS TSC | 3812 | TATTGTTC AAGTACAT CTTGT | 5139 | Y | TAC | MDV | n/a | ATGGATG TG | n/a |
| 20 | ARDL | 4802 | GCCAGAG ACTTG | 4974 | GYCS STSC | 3809 | GGGTATTG CTCCAGTA CCTCATGT | 5140 | A D | GCTGAT | PDY | n/a | CCAGATT AT | n/a |
| 21 | ARGL | 4803 | GCCAGAG GGTTG | 4975 | YCSS TSCY | 3810 | TACTGCAG CAGCACAA GTTGTTAC | 5141 | A G | GCAGGC | SDY | n/a | TCTGATT AC | n/a |
| 22 | ARA | n/a | GCCAGAG CT | n/a | GYCS STSC Y | 3807 | GGGTATTG CAGTTCAA CTAGTTGT TAT | 5142 | A P | GCCCCA | VDY | n/a | GTTGACT AC | n/a |
| 23 | AREA | 4804 | GCCAGAG AGGCT | 4976 | YCSS TSCY T | 3844 | TACTGTTC ATCAACCT CCTGTTAT ACT | 5143 | A Q | GCTCAG | YDY | n/a | TATGATT AC | n/a |
| 24 | ARDA | 4805 | GCCAGAG ATGCT | 4977 | PAA | n/a | CCTGCCGC T | n/a | A R | GCTAGG | AFD I | 453 9 | GCCTTCG ATATC | 5251 |
| 25 | ARGA | 4806 | GCCAGAG GTGCT | 4978 | CSGG SCY | 3774 | TGCTCTGG GGGTAGCT GCTAT | 5144 | A S | GCTAGT | AFD Y | 459 2 | GCCTTCG ATTAC | 5252 |
| 26 | ART | n/a | GCCAGAA CT | n/a | YCSG GSC | 3773 | TACTGTAG CGGTGGTA GTTGC | 5145 | A T | GCTACC | DFD Y | 458 1 | GATTTCG ACTAT | 5253 |
| 27 | ARET | 4807 | GCCAGAG AGACT | 4979 | GYCS GGSC | 3770 | GGATACTG TAGTGGCG GATCCTGC | 5146 | A Y | GCCTAC | FFD Y | 458 3 | TTCTTCG ATTAC | 5254 |
| 28 | ARDT | 4808 | GCCAGAG ATACT | 4980 | YCSG GSCY | 3771 | TACTGCTC CGGAGGAA GTTGTTAT | 5147 | D A | GACGCC | GFD P | 462 3 | GGGTTTG ACCCA | 5255 |
| 29 | ARGT | 4809 | GCCAGAG GCACT | 4981 | GYCS GGSC Y | 3768 | GGTTATTG CAGTGGGG GTTCATGT TAC | 5148 | D D | GACGAT | GFD Y | 459 0 | GGGTTCG ACTAC | 5256 |
| 30 | ARV | n/a | GCCAGAG TG | n/a | YCSG GSCY S | 3769 | TACTGTTC CGGAGGTA GCTGTTAC TCT | 5149 | D E | GACGAG | GMD V | 464 1 | GGCATGG ATGTA | 5257 |
| 31 | AREV | 4810 | GCCAGAG AGGTG | 4982 | RI | n/a | AGAATC | n/a | D G | GATGGT | HFD Y | 458 2 | CACTTTG ACTAT | 5258 |
| 32 | ARDV | 4811 | GCCAGAG ATGTG | 4983 | GY | n/a | GGATAT | n/a | D L | GACTTG | IFD Y | 458 8 | ATATTCG ATTAC | 5259 |
| 33 | ARGV | 4812 | GCCAGAG GGGTG | 4984 | GG | n/a | GGCGGT | n/a | D P | GACCCT | LFD Y | 458 6 | TTATTTG ATTAT | 5260 |
| 34 | AREG G | 4813 | GCCAGAG AGGGAGG A | 4985 | ATP | n/a | GCTACCCC T | n/a | D S | GACTCC | NFD Y | 458 0 | AACTTTG ATTAC | 5261 |
| 35 | ARDG G | 4814 | GCCAGAG ATGGTGG A | 4986 | DI | n/a | GACATC | n/a | D Y | GATTAT | PFD Y | 458 7 | CCCTTCG ACTAT | 5262 |
| 36 | ARGG G | 4815 | GCCAGAG GTGGAGG A | 4987 | TP | n/a | ACTCCT | n/a | E A | GAGGCC | RFD Y | 458 5 | AGGTTTG ACTAT | 5263 |
| 37 | ARDG R | 4816 | GCCAGAG ACGGCAG A | 4988 | GD | n/a | GGAGAT | n/a | E D | GAGGAC | SFD Y | 458 4 | AGTTTCG ATTAC | 5264 |
| 38 | ARGG S | 4817 | GCCAGAG GCGGTTC T | 4989 | AYCG GDCY | 3863 | GCCTATTG CGGTGGTG ACTGCTAT | 5150 | E G | GAAGGA | TFD Y | 458 9 | ACATTTG ACTAC | 5265 |
| 39 | ARGG P | 4818 | GCCAGAG GTGGGCC T | 4990 | AYCG GDC | 3865 | GCATATTG CGGAGGGG ATTGC | 5151 | E K | GAGAAA | VFD Y | 459 1 | GTTTTCG ATTAT | 5266 |
| 40 | ARGG A | 4819 | GCCAGAG GAGGTGC T | 4991 | YCGG DCY | 3866 | TATTGTGG TGGGGACT GCTAT | 5152 | E R | GAAAGA | WFD P | 459 6 | TGGTTCG ATCCA | 5267 |
| 41 | ARDG T | 4820 | GCCAGAG ACGGTAC T | 4992 | YCGG DC | 3868 | TACTGCGG AGGCGATT GC | 5153 | E S | GAATCT | YFD L | 452 9 | TATTTCG ACTTA | 5268 |
| 42 | ARGG T | 4821 | GCCAGAG GTGGAAC T | 4993 | HI | n/a | CACATC | n/a | E T | GAAACA | YFD Y | 456 7 | TACTTCG ATTAC | 5269 |
| 43 | AREG V | 4822 | GCCAGAG AGGGAGT G | 4994 | TA | n/a | ACAGCT | n/a | F A | TTCGCT | YFQ H | 448 9 | TATTTCC AGCAC | 5270 |
| 44 | ARGG V | 4823 | GCCAGAG GTGGCGT G | 4995 | GYCS STSC YA | 3806 | GGGTACTG CTCTAGCA CTTCATGC TACGCC | 5154 | F H | TTCCAT | YMD V | 468 7 | TATATGG ATGTC | 5271 |
| 45 | ARRG | 4824 | GCCAGAA GAGGA | 4996 | SS | n/a | AGTTCT | n/a | F L | TTCTTG | DAF DI | 453 8 | GATGCCT TCGACAT A | 5272 |
| 46 | ARER G | 4825 | GCCAGAG AGCGTGG A | 4997 | ST | n/a | AGTACT | n/a | F R | TTTAGG | DWF DP | 460 9 | GACTGGT TTGACCC C | 5273 |
| 47 | ARDR G | 4826 | GCCAGAG ATCGTGG A | 4998 | TS | n/a | ACCAGC | n/a | F S | TTTAGT | DYF DY | 456 6 | GACTACT TTGATTA C | 5274 |
| 48 | ARGR G | 4827 | GCCAGAG GCAGGGG A | 4999 | PAAM P | 3835 | CCAGCAGC TATGCCT | 5155 | G A | GGAGCC | EYF QH | 448 8 | GAATACT TCCAACA C | 5275 |
| 49 | ARSG | 4828 | GCCAGAT CAGGA | 5000 | PA | n/a | CCCGCC | n/a | G D | GGTGAT | GAF DI | 454 9 | GGCGCAT TCGATAT T | 5276 |
| 50 | ARDS G | 4829 | GCCAGAG ACTCAGG A | 5001 | MP | n/a | ATGCCT | n/a | G E | GGTGAG | GWF DP | 461 9 | GGGTGGT TTGATCC A | 5277 |
| 51 | ARDP G | 4830 | GCCAGAG ATCCAGG A | 5002 | VYAI P | 3940 | GTCTATGC AATTCCT | 5156 | G G | GGAGGC | GYF DY | 457 7 | GGCTATT TTGACTA C | 5278 |
| 52 | ARLG | 4831 | GCCAGAT TGGGA | 5003 | WFGE | 3966 | TGGTTTGG GGAG | 5157 | G L | GGATTG | HAF DI | 454 1 | CATGCTT TTGATAT A | 5279 |
| 53 | ARDL G | 4832 | GCCAGAG ACTTGGG A | 5004 | FGE | n/a | TTTGGAGA G | n/a | G P | GGACCA | HYF DY | 456 9 | CATTACT TCGATTA C | 5280 |
| 54 | ARAG | 4833 | GCCAGAG CTGGA | 5005 | GEL | n/a | GGCGAGCT T | n/a | G R | GGTAGG | NAF DI | 456 5 | AACGCAT TCGATAT T | 5281 |
| 55 | ARVG | 4834 | GCCAGAG TGGGA | 5006 | WFG | n/a | TGGTTCGG T | n/a | G S | GGCAGT | NWF DP | 459 5 | AACTGGT TCGATCC A | 5282 |
| 56 | ARGV G | 4835 | GCCAGAG GCGTAGG A | 5007 | GSG | n/a | GGTTCAGG C | n/a | G T | GGAACA | NYF DY | 459 3 | AATTATT TCGACTA T | 5283 |
| 57 | ARPR | 4836 | GCCAGAC CCAGA | 5008 | SGSY | 3763 | AGTGGATC TTAT | 5158 | G V | GGAGTT | PYF DY | 457 4 | CCCTACT TTGACTA T | 5284 |
| 58 | ARGP R | 4837 | GCCAGAG GACCAAG A | 5009 | YYGS | 3990 | TATTATGG CAGT | 5159 | G W | GGATGG | RAF DI | 454 4 | AGAGCCT TTGATAT C | 5285 |
| 59 | ARPS | 4838 | GCCAGAC CATCT | 5010 | YYYG | 3989 | TACTACTA TGGC | 5160 | G Y | GGATAT | RYF DY | 457 2 | AGGTACT TCGATTA C | 5286 |
| 60 | ARDP S | 4839 | GCCAGAG ATCCCTC T | 5011 | GSGS Y | 3987 | GGCAGCGG TTCCTAC | 5161 | H E | CATGAG | SWF DP | 461 3 | TCATGGT TCGACCC C | 5287 |
| 61 | ARGP S | 4840 | GCAAGAG GACCTTC T | 5012 | SGSY Y | 3761 | AGTGGATC CTATTAC | 5162 | H L | CATTTG | SYF DY | 457 1 | AGTTACT TTGACTA T | 5288 |
| 62 | ARDP P | 4841 | GCCAGAG ACCCACC T | 5013 | YYYG SG | 3979 | TATTACTA CGGGTCTG GC | 5163 | H P | CATCCT | TYF DY | 457 6 | ACTTATT TCGACTA C | 5289 |
| 63 | ARGP P | 4842 | GCCAGAG GACCGCC T | 5014 | SGS | n/a | AGCGGCAG T | n/a | H S | CACTCC | VAF DI | 455 0 | GTGGCCT TCGACAT T | 5290 |
| 64 | ARPL | 4843 | GCCAGAC CGTTG | 5015 | YYYG SGS | 3975 | TATTACTA CGGATCTG GCTCT | 5164 | I F | ATCTTC | VYF DY | 457 8 | GTCTATT TTGATTA T | 5291 |
| 65 | ARDP L | 4844 | GCCAGAG ATCCTTT G | 5016 | YYYG SGSY | 3972 | TATTACTA TGGCTCTG GTAGCTAC | 5165 | I G | ATCGGC | WYF DL | 452 8 | TGGTATT TCGATTT G | 5292 |
| 66 | ARRP | 4845 | GCCAGAA GGCCT | 5017 | YGS | n/a | TATGGCTC C | n/a | I R | ATAAGG | YAF DI | 454 0 | TACGCAT TTGACAT C | 5293 |
| 67 | ARDR P | 4846 | GCCAGAG ACCGTCC T | 5018 | YYG | n/a | TACTATGG T | n/a | I S | ATCAGT | YGM DV | 464 0 | TACGGCA TGGACGT G | 5294 |
| 68 | ARSP | 4847 | GCCAGAT CACCT | 5019 | YYY | n/a | TATTATTA T | n/a | K G | AAAGGA | YYF DY | 456 8 | TATTATT TTGATTA C | 5295 |
| 69 | ARLP | 4848 | GCCAGAC TTCCT | 5020 | MVRG | 4017 | ATGGTAAG AGGT | 5166 | K R | AAGAGA | YYM DV | 468 6 | TATTATA TGGACGT C | 5296 |
| 70 | ARAP | 4849 | GCCAGAG CCCCT | 5021 | TMVR G | 4010 | ACCATGGT GAGGGGT | 5167 | K V | AAAGTG | AEY FQH | 452 6 | GCAGAGT ACTTCCA GCAC | 5297 |
| 71 | ARTP | 4850 | GCCAGAA CTCCT | 5022 | RGV | n/a | AGAGGAGT T | n/a | L D | TTGGAT | DNW FDP | 459 4 | GACAATT GGTTTGA TCCC | 5298 |
| 72 | ARVP | 4851 | GCCAGAG TCCCT | 5023 | VRG | n/a | GTCAGAGG C | n/a | L E | TTAGAG | DWY FDL | 453 0 | GATTGGT ACTTCGA CTTG | 5299 |
| 73 | ARVG G | 4852 | GCCAGAG TTGGAGG A | 5024 | FG | n/a | TTCGGC | n/a | L G | TTAGGT | GNW FDP | 460 6 | GGGAATT GGTTTGA TCCT | 5300 |
| 74 | ARQ | n/a | GCAAGAC AG | n/a | GE | n/a | GGCGAG | n/a | L H | TTACAT | NWY FDL | 453 2 | AACTGGT ATTTCGA CTTA | 5301 |
| 75 | ARH | n/a | GCCAGAC AC | n/a | YG | n/a | TACGGC | n/a | L L | TTATTG | PNW FDP | 460 3 | CCCAATT GGTTTGA TCCA | 5302 |
| 76 | ARDQ | 4853 | GCCAGGG ACCAG | 5025 | VR | n/a | GTGCGT | n/a | L P | TTACCA | SNW FDP | 460 0 | AGTAATT GGTTTGA CCCC | 5303 |
| 77 | ARDH | 4854 | GCAAGAG ACCAC | 5026 | RG | n/a | AGAGGT | n/a | L R | TTGAGG | YNW FDP | 459 7 | TATAATT GGTTTGA TCCT | 5304 |
| 78 | ARAR | 4855 | GCAAGGG CTAGA | 5027 | FRE | n/a | TTCAGGGA G | n/a | L S | TTAAGC | YWY FDL | 452 7 | TATTGGT ATTTTGA TTTG | 5305 |
| 79 | ARAS | 4856 | GCTAGGG CATCT | 5028 | RE | n/a | AGAGAG | n/a | L T | TTGACA | YYG MDV | 463 9 | TACTATG GGATGGA CGTG | 5306 |
| 80 | ARDQ G | 4857 | GCTAGGG ATCAGGG A | 5029 | QG | n/a | CAAGGT | n/a | L V | TTGGTA | YYY MDV | 468 5 | TACTATT ACATGGA CGTT | 5307 |
| 81 | ARSR | 4858 | GCTAGAT CAAGA | 5030 | LR | n/a | TTACGT | n/a | L W | TTGTGG | YYY GMD V | 463 8 | TATTATT ACGGTAT GGACGTC | 5308 |
| 82 | ARDR S | 4859 | GCCAGGG ACAGGTC T | 5031 | YYDY VWGS YAYT | 4070 | TACTATGA TTACGTCT GGGGGTCT TATGCTTA CACT | 5168 | L Y | TTGTAC | YYY YMD V | 468 4 | TACTATT ATTACAT GGATGTC | 5309 |
| 83 | ARSL | 4860 | GCTAGAT CTTTG | 5032 | YYDY VWGS YAY | 4071 | TACTACGA CTATGTAT GGGGCTCA TATGCTTA C | 5169 | M G | ATGGGC | DYY YGM DV | 466 8 | GACTATT ATTACGG TATGGAT GTT | 5310 |
| 84 | ARLH | 4861 | GCTAGGT TGCAC | 5033 | YYDY VWGS YA | 4073 | TACTACGA TTACGTAT GGGGAAGC TACGCT | 5170 | M T | ATGACC | GYY YGM DV | 467 7 | GGCTACT ATTATGG TATGGAC GTC | 5311 |
| 85 | ARDL T | 4862 | GCCAGGG ATTTGAC T | 5034 | YDYV WGSY AY | 4074 | TATGATTA TGTGTGGG GGTCATAC GCATAC | 5171 | P A | CCTGCT | HYY YGM DV | 466 9 | CATTACT ACTATGG GATGGAT GTA | 5312 |
| 86 | ARK | n/a | GCCAGAA AG | n/a | DY | n/a | GATTAC | n/a | P D | CCTGAT | NYY YYM DV | 471 3 | AACTATT ATTATTA TATGGAT GTC | 5313 |
| 87 | ARAE | 4863 | GCAAGAG CCGAG | 5035 | WG | n/a | TGGGGC | n/a | P E | CCTGAG | PYY YYM DV | 472 0 | CCCTACT ACTACTA TATGGAT GTG | 5314 |
| 88 | ARDL S | 4864 | GCAAGGG ATTTGTC T | 5036 | DYVW GSYA YT | 4075 | GATTATGT GTGGGGGT CTTACGCC TACACC | 5172 | P F | CCTTTC | RYY YYM DV | 471 8 | AGGTATT ACTACTA CATGGAC GTC | 5315 |
| 89 | ARGD | 4865 | GCTAGAG GGGAC | 5037 | YDYV WGSY A | 4077 | TACGACTA TGTGTGGG GTTCCTAT GCT | 5173 | P G | CCTGGT | YYY YGM DV | 463 7 | TACTATT ATTATGG GATGGAT GTA | 5316 |
| 90 | ARRR | 4866 | GCTAGGA GGAGA | 5038 | YYDS | 4183 | TACTACGA TTCC | 5174 | P H | CCACAT | DYY YYG MDV | 465 5 | GATTACT ATTATTA CGGAATG GATGTT | 5317 |
| 91 | ARDK | 4867 | GCTAGAG ATAAG | 5039 | YYYD | 4182 | TATTATTA TGAC | 5175 | P L | CCATTA | GYY YYG MDV | 466 4 | GGGTATT ACTACTA CGGCATG GACGTA | 5318 |
| 92 | ARVS | 4868 | GCTAGAG TATCT | 5040 | DSSG Y | 4179 | GACAGTTC CGGGTAC | 5176 | P P | CCTCCA | NYY YYG MDV | 465 4 | AATTACT ATTACTA TGGCATG GATGTG | 5319 |
| 93 | ARDR L | 4869 | GCCAGAG ACAGGTT G | 5041 | YDSS G | 4178 | TATGATAG CTCAGGT | 5177 | P Q | CCTCAG | PYY YYG MDV | 466 1 | CCATATT ACTATTA CGGCATG GATGTC | 5320 |
| 94 | ARGQ | 4870 | GCTAGGG GCCAG | 5042 | YYDS S | 4177 | TACTATGA CTCATCC | 5178 | P R | CCAAGG | SYY YYG MDV | 465 8 | AGCTACT ACTACTA CGGAATG GACGTC | 5321 |
| 95 | ARVR | 4871 | GCCAGGG TCAGA | 5043 | YYYD S | 4176 | TATTATTA CGATAGT | 5179 | P S | CCTTCT | YYY YYG MDV | 463 6 | TACTACT ACTATTA CGGTATG GACGTA | 5322 |
| 96 | ARAK | 4872 | GCTAGGG CTAAG | 5044 | GYY | n/a | GGATATTA C | n/a | P T | CCTACA | DYY YYY YMD V | 468 1 | GATTATT ATTACTA CTACTAC ATGGATG TA | 5323 |
| 97 | ARGK | 4873 | GCCAGGG GTAAG | 5045 | DSSG YY | 4174 | GATTCTTC CGGGTACT AC | 5180 | P V | CCTGTT | GYY YYY GMD V | 465 1 | GGTTATT ATTACTA CTATGGG ATGGATG TA | 5324 |
| 98 | ARDI S | 4874 | GCAAGGG ATATTTC T | 5046 | YDSS GY | 4173 | TATGATTC CAGCGGAT AC | 5181 | Q G | CAGGGC | HYY YYY GMD V | 464 3 | CACTACT ATTATTA TTACGGG ATGGATG TA | 5325 |
| 99 | ARDF T | 4875 | GCTAGGG ATTTCAC T | 5047 | YYDS SG | 4172 | TACTACGA TAGCTCCG GT | 5182 | Q L | CAATTA | RYY YYY YMD V | 469 2 | AGATACT ACTACTA TTATTAC ATGGATG TA | 5326 |
| 100 | ARQG | 4876 | GCCAGGC AGGGA | 5048 | YYYD SS | 4171 | TATTATTA CGACTCTT CC | 5183 | Q P | CAGCCA | YYY YYY GMD V | 464 2 | TATTACT ACTATTA CTATGGT ATGGACG TT | 5327 |
| 101 | AK | n/a | GCCAAG | n/a | YDSS GYY | 4169 | TACGACTC TTCTGGTT ATTAC | 5184 | Q S | CAGTCA | | | | |
| 102 | AKE | n/a | GCCAAGG AG | n/a | YYDS SGY | 4168 | TATTATGA CAGCAGCG GGTAT | 5185 | Q T | CAGACT | | | | |
| 103 | AKD | n/a | GCCAAGG AC | n/a | YYYD SSG | 4167 | TACTACTA CGATTCCA GCGGT | 5186 | R A | AGGGCT | | | | |
| 104 | AKG | n/a | GCCAAGG GA | n/a | YDSS GYYY | 4166 | TACGACAG TTCCGGAT ATTATTAC | 5187 | R D | AGGGAC | | | | |
| 105 | AKEG | 4877 | GCCAAGG AAGGA | 5049 | SGY | n/a | AGCGGATA T | n/a | R E | AGGGAG | | | | |
| 106 | AKDG | 4878 | GCCAAGG ACGGA | 5050 | YYDS SGYY | 4165 | TACTATGA TAGTAGTG GGTACTAT | 5188 | R F | AGATTC | | | | |
| 107 | AKGG | 4879 | GCCAAGG GCGGA | 5051 | YYYD SSGY | 4164 | TACTACTA TGACAGCT CAGGGTAT | 5189 | R G | AGGGGA | | | | |
| 108 | AKR | n/a | GCCAAGA GA | n/a | YYDS SGYY Y | 4163 | TATTACGA CAGCAGTG GCTACTAC TAT | 5190 | R H | AGGCAT | | | | |
| 109 | AKER | 4880 | GCCAAGG AAAGA | 5052 | YYYD SSGY Y | 4162 | TACTACTA CGATAGCT CTGGATAC TAT | 5191 | R L | AGATTA | | | | |
| 110 | AKDR | 4881 | GCCAAGG ACAGA | 5053 | YYYD SSGY YY | 4161 | TATTATTA CGATTCCA GTGGTTAT TATTAT | 5192 | R M | AGGATG | | | | |
| 111 | AKGR | 4882 | GCCAAGG GCAGA | 5054 | YDS | n/a | TACGACTC C | n/a | R P | AGGCCA | | | | |
| 112 | AKS | n/a | GCCAAGT CT | n/a | YYD | n/a | TACTATGA C | n/a | R R | CGTAGA | | | | |
| 113 | AKES | 4883 | GCCAAGG AATCT | 5055 | DSSG | 4185 | GACTCATC CGGT | 5193 | R S | AGAAGT | | | | |
| 114 | AKDS | 4884 | GCCAAGG ATTCT | 5056 | GYYY | 4188 | GGTTACTA TTAC | 5194 | R V | AGAGTG | | | | |
| 115 | AKGS | 4885 | GCCAAGG GATCT | 5057 | SGYY | 4187 | AGCGGCTA CTAT | 5195 | R Y | AGATAT | | | | |
| 116 | AKP | n/a | GCCAAGC CT | n/a | DS | n/a | GACTCT | n/a | S A | TCAGCC | | | | |
| 117 | AKDP | 4886 | GCCAAGG ATCCT | 5058 | RFLE W | 4231 | AGATTTTT GGAGTGG | 5196 | S D | TCAGAC | | | | |
| 118 | AKGP | 4887 | GCCAAGG GTCCT | 5059 | EWL | n/a | GAATGGCT T | n/a | S E | TCCGAG | | | | |
| 119 | AKL | n/a | GCCAAGT TG | n/a | RF | n/a | AGATTC | n/a | S F | TCATTC | | | | |
| 120 | AKDL | 4888 | GCCAAGG ACTTG | 5060 | YYDF WSGY YT | 4242 | TACTATGA TTTTTGGA GTGGATAT TATACC | 5197 | S G | AGCGGA | | | | |
| 121 | AKGL | 4889 | GCCAAGG GGTTG | 5061 | YDFW SG | 4253 | TATGATTT TTGGTCTG GT | 5198 | S H | AGTCAC | | | | |
| 122 | AKA | n/a | GCCAAGG CT | n/a | DFWS GY | 4254 | GATTTTTG GAGCGGCT AT | 5199 | S I | TCTATC | | | | |
| 123 | AKEA | 4890 | GCCAAGG AAGCT | 5062 | FWSG Y | 4260 | TTTTGGAG CGGGTAT | 5200 | S K | AGTAAA | | | | |
| 124 | AKDA | 4891 | GCCAAGG ACGCT | 5063 | YYDF WSGY Y | 4243 | TACTACGA CTTCTGGA GCGGGTAT TAC | 5201 | S L | TCCTTG | | | | |
| 125 | AKGA | 4892 | GCCAAGG GCGCT | 5064 | YYDF WSGY | 4245 | TACTACGA TTTTTGGT CTGGATAT | 5202 | S P | AGCCCA | | | | |
| 126 | AKT | n/a | GCCAAGA CT | n/a | YDFW SGYY | 4246 | TATGACTT TTGGAGTG GTTACTAC | 5203 | S Q | AGCCAA | | | | |
| 127 | AKET | 4893 | GCCAAGG AAACT | 5065 | YYDF WSG | 4248 | TACTACGA TTTCTGGT CAGGC | 5204 | S R | TCAAGA | | | | |
| 128 | AKDT | 4894 | GCCAAGG ATACT | 5066 | YDFW SGY | 4249 | TATGACTT CTGGAGTG GTTAC | 5205 | S S | TCCTCA | | | | |
| 129 | AKGT | 4895 | GCCAAGG GAACT | 5067 | DFWS GYY | 4250 | GACTTCTG GTCAGGAT ACTAC | 5206 | S T | AGTACA | | | | |
| 130 | AKV | n/a | GCCAAGG TG | n/a | VLRY F | 4307 | GTGTTGAG GTACTTC | 5207 | S V | TCAGTA | | | | |
| 131 | AKEV | 4896 | GCCAAGG AAGTG | 5068 | LRYF D | 4308 | TTAAGATA CTTTGAT | 5208 | S W | TCATGG | | | | |
| 132 | AKDV | 4897 | GCCAAGG ACGTG | 5069 | RYFD W | 4309 | AGATACTT TGATTGG | 5209 | S Y | TCTTAC | | | | |
| 133 | AKGV | 4898 | GCCAAGG GCGTG | 5070 | VLRY | 4312 | GTGTTGAG GTAT | 5210 | T A | ACCGCC | | | | |
| 134 | AKEG G | 4899 | GCCAAGG AGGGAGG A | 5071 | LRYF | 4313 | TTGAGATA TTTC | 5211 | T G | ACTGGC | | | | |
| 135 | AKDG G | 4900 | GCCAAGG ACGGTGG A | 5072 | RYFD | 4314 | AGATACTT TGAT | 5212 | T P | ACACCA | | | | |
| 136 | AKGG G | 4901 | GCCAAGG GAGGAGG A | 5073 | VLRY FDWL | 4298 | GTCTTAAG GTACTTCG ATTGGCTT | 5213 | T R | ACAAGA | | | | |
| 137 | AKDG R | 4902 | GCCAAGG ACGGTAG A | 5074 | LRY | n/a | TTAAGATA C | n/a | T S | ACATCT | | | | |
| 138 | AKGG S | 4903 | GCCAAGG GAGGTTC T | 5075 | RYF | n/a | AGATACTT C | n/a | T T | ACTACT | | | | |
| 139 | AKGG P | 4904 | GCCAAGG GAGGTCC T | 5076 | RY | n/a | AGATAT | n/a | T V | ACAGTT | | | | |
| 140 | AKGG A | 4905 | GCCAAGG GAGGCGC T | 5077 | FD | n/a | TTCGAT | n/a | T W | ACTTGG | | | | |
| 141 | AKDG T | 4906 | GCCAAGG ATGGCAC T | 5078 | DW | n/a | GATTGG | n/a | T Y | ACTTAT | | | | |
| 142 | AKGG T | 4907 | GCCAAGG GAGGCAC T | 5079 | VLRY FDW | 4300 | GTCTTAAG ATACTTTG ATTGG | 5214 | V A | GTAGCC | | | | |
| 143 | AKEG V | 4908 | GCCAAGG AAGGAGT G | 5080 | VLRY FD | 4303 | GTGTTGAG GTACTTTG AC | 5215 | V D | GTCGAC | | | | |
| 144 | AKGG V | 4909 | GCCAAGG GCGGTGT G | 5081 | LRYF DW | 4304 | TTAAGATA CTTCGATT GG | 5216 | V G | GTTGGA | | | | |
| 145 | AKRG | 4910 | GCCAAGA GAGGA | 5082 | RYFD WL | 4305 | AGATATTT CGACTGGC TT | 5217 | V L | GTCTTG | | | | |
| 146 | AKER G | 4911 | GCCAAGG AGAGAGG A | 5083 | YDIL TGYY | 4322 | TATGACAT ATTGACTG GCTACTAC | 5218 | V P | GTTCCT | | | | |
| 147 | AKDR G | 4912 | GCCAAGG ATAGGGG A | 5084 | YDIL TGY | 4325 | TATGATAT ATTAACTG GGTAC | 5219 | V R | GTGAGA | | | | |
| 148 | AKGR G | 4913 | GCCAAGG GTAGGGG A | 5085 | DILT GYY | 4326 | GATATCTT AACCGGGT ATTAT | 5220 | V S | GTTTCA | | | | |
| 149 | AKSG | 4914 | GCCAAGT CTGGA | 5086 | DYG | n/a | GATTATGG T | n/a | V T | GTTACC | | | | |
| 150 | AKDS G | 4915 | GCCAAGG ATAGTGG A | 5087 | GDY | n/a | GGGGACTA C | n/a | V V | GTAGTA | | | | |
| 151 | AKDP G | 4916 | GCCAAGG ACCCCGG A | 5088 | YGD | n/a | TACGGTGA C | n/a | W G | TGGGGT | | | | |
| 152 | AKLG | 4917 | GCCAAGT TAGGA | 5089 | DYGD | 4349 | GATTACGG CGAT | 5221 | W S | TGGTCA | | | | |
| 153 | AKDL G | 4918 | GCCAAGG ATCTTGG A | 5090 | YGDY | 4350 | TACGGCGA TTAT | 5222 | Y A | TACGCT | | | | |
| 154 | AKAG | 4919 | GCCAAGG CTGGA | 5091 | DYGD Y | 4348 | GACTACGG AGATTAT | 5223 | Y D | TATGAC | | | | |
| 155 | AKVG | 4920 | GCCAAGG TAGGA | 5092 | TTVT T | 4351 | ACCACAGT AACCACC | 5224 | Y E | TATGAG | | | | |
| 156 | AKGV G | 4921 | GCCAAGG GTGTCGG A | 5093 | TTVT | 4352 | ACAACTGT GACT | 5225 | Y S | TATTCT | | | | |
| 157 | AKPR | 4922 | GCCAAGC CTAGA | 5094 | TVTT | 4353 | ACAGTAAC TACT | 5226 | A A A | GCTGCC GCT | | | | |
| 158 | AKGP R | 4923 | GCCAAGG GCCCCAG A | 5095 | RW | n/a | AGATGG | n/a | A G M | GCAGGC ATG | | | | |
| 159 | AKPS | 4924 | GCCAAGC CTTCT | 5096 | VTP | n/a | GTAACTCC T | n/a | D G G | GATGGG GGT | | | | |
| 160 | AKDP S | 4925 | GCCAAGG ATCCCTC T | 5097 | VD | n/a | GTTGAC | n/a | D G V | GATGGT GTA | | | | |
| 161 | AKGP S | 4926 | GCCAAAG GGCCATC T | 5098 | GYSG YD | 4386 | GGCTACTC AGGATACG AC | 5227 | F G G | TTCGGG GGC | | | | |
| 162 | AKDP P | 4927 | GCCAAGG ATCCACC T | 5099 | YSGY D | 4389 | TATAGCGG ATATGAC | 5228 | G A G | GGAGCT GGA | | | | |
| 163 | AKGP P | 4928 | GCCAAGG GCCCTCC T | 5100 | RD | n/a | AGAGAT | n/a | G G A | GGAGGG GCC | | | | |
| 164 | AKPL | 4929 | GCCAAGC CGTTG | 5101 | RDGY | 4410 | AGAGATGG TTAC | 5229 | G G G | GGAGGC GGT | | | | |
| 165 | AKDP L | 4930 | GCCAAGG ACCCTTT G | 5102 | RDG | n/a | AGAGATGG T | n/a | G G L | GGAGGC TTA | | | | |
| 166 | AKRP | 4931 | GCCAAGA GGCCT | 5103 | DTAM | 4420 | GATACTGC TATG | 5230 | G G R | GGTGGT AGA | | | | |
| 167 | AKDR P | 4932 | GCCAAGG ACCGTCC T | 5104 | YGY | n/a | TACGGCTA C | n/a | G G S | GGCGGG AGC | | | | |
| 168 | AKSP | 4933 | GCCAAGA GTCCT | 5105 | YSY | n/a | TATTCTTA C | n/a | G G V | GGTGGG GTA | | | | |
| 169 | AKLP | 4934 | GCCAAGC TACCT | 5106 | YSYG | 4433 | TATTCATA TGGT | 5231 | G L G | GGATTA GGC | | | | |
| 170 | AKAP | 4935 | GCCAAGG CTCCT | 5107 | GYSY G | 4430 | GGATATAG TTATGGC | 5232 | G P G | GGCCCC GGC | | | | |
| 171 | AKTP | 4936 | GCCAAGA CGCCT | 5108 | SSS | n/a | AGTTCAAG C | n/a | G P P | GGACCA CCT | | | | |
| 172 | AKVP | 4937 | GCCAAGG TACCT | 5109 | YSSS WY | 4437 | TACAGTAG CTCTTGGT AC | 5233 | G R G | GGAAGG GGC | | | | |
| 173 | AKVG G | 4938 | GCCAAGG TGGGTGG A | 5110 | GYSS SWY | 4435 | GGCTACAG TTCAAGCT GGTAT | 5234 | G S G | GGATCT GGC | | | | |
| 174 | AKQ | n/a | GCAAAAC AG | n/a | SSW | n/a | AGTTCCTG G | n/a | G T G | GGAACT GGC | | | | |
| 175 | AKH | n/a | GCCAAAC AC | n/a | SWY | n/a | AGCTGGTA C | n/a | G V G | GGAGTA GGT | | | | |
| 176 | AKDQ | 4939 | GCCAAGG ATCAG | 5111 | SSSW | 4443 | AGTAGCTC TTGG | 5235 | L G G | TTGGGA GGC | | | | |
| 177 | AKDH | 4940 | GCTAAAG ACCAC | 5112 | YSSS | 4442 | TACAGCAG CTCC | 5236 | L G H | TTGGGC CAC | | | | |
| 178 | AKAR | 4941 | GCCAAGG CAAGA | 5113 | YSSS W | 4439 | TACTCTTC CTCATGG | 5237 | P G G | CCTGGC GGC | | | | |
| 179 | AKAS | 4942 | GCCAAGG CATCT | 5114 | SW | n/a | AGCTGG | n/a | P K Q | CCAAAG CAG | | | | |
| 180 | AKDQ G | 4943 | GCAAAGG ATCAGGG A | 5115 | AG | n/a | GCTGGT | n/a | P K R | CCTAAA AGG | | | | |
| 181 | AKSR | 4944 | GCCAAGA GTAGA | 5116 | QQLV | 4455 | CAGCAATT GGTT | 5238 | P T Q | CCAACT CAG | | | | |
| 182 | AKDR S | 4945 | GCAAAAG ACAGGTC T | 5117 | QQL | n/a | CAGCAACT T | n/a | R F E | AGGTTT GAG | | | | |
| 183 | AKSL | 4946 | GCAAAGA GCTTG | 5118 | GWY | n/a | GGTTGGTA C | n/a | R G G | AGGGGA GGT | | | | |
| 184 | AKLH | 4947 | GCTAAAT TGCAC | 5119 | SSGW Y | 4461 | AGCTCTGG ATGGTAC | 5239 | R G L | AGGGGA TTG | | | | |
| 185 | AKDL T | 4948 | GCCAAGG ACTTGAC T | 5120 | YSSG W | 4460 | TATAGTAG CGGATGG | 5240 | R G S | AGGGGT AGT | | | | |
| 186 | AKK | n/a | GCTAAAA AG | n/a | YSSG WY | 4458 | TATAGCAG CGGTTGGT AC | 5241 | R P L | AGGCCA TTA | | | | |
| 187 | AKAE | 4949 | GCTAAAG CAGAG | 5121 | GYSS GWY | 4456 | GGTTATTC ATCAGGTT GGTAT | 5242 | R P Y | AGGCCA TAT | | | | |
| 188 | AKDL S | 4950 | GCAAAAG ACTTGTC T | 5122 | SGW | n/a | AGTGGTTG G | n/a | S A A | TCTGCC GCC | | | | |
| 189 | AKGD | 4951 | GCAAAAG GGGAC | 5123 | YSS | n/a | TACAGTTC C | n/a | S G E | AGCGGG GAG | | | | |
| 190 | AKRR | 4952 | GCAAAAA GGAGA | 5124 | SGWY | 4464 | AGTGGTTG GTAT | 5243 | S G G | TCTGGC GGC | | | | |
| 191 | AKDK | 4953 | GCAAAAG ACAAG | 5125 | SSGW | 4463 | AGTTCCGG TTGG | 5244 | S G L | TCAGGG TTA | | | | |
| 192 | AKVS | 4954 | GCCAAAG TATCT | 5126 | YSSG | 4462 | TACTCAAG TGGT | 5245 | S G W | AGCGGT TGG | | | | |
| 193 | AKDR L | 4955 | GCAAAAG ACAGGTT G | 5127 | GW | n/a | GGTTGG | n/a | S G Y | AGCGGC TAC | | | | |
| 194 | AKGQ | 4956 | GCCAAAG GACAG | 5128 | QWLV | 4475 | CAGTGGTT AGTT | 5246 | S R G | TCAAGA GGT | | | | |
| 195 | AKVR | 4957 | GCAAAAG TCAGA | 5129 | EYSS | 4479 | GAGTACTC ATCC | 5247 | S S E | TCATCA GAG | | | | |
| 196 | AKAK | 4958 | GCAAAGG CAAAG | 5130 | SIAA RP | 4481 | AGCATAGC AGCAAGGC CT | 5248 | S S W | AGCTCA TGG | | | | |
| 197 | AKGK | 4959 | GCAAAGG GCAAG | 5131 | RP | n/a | CGTCCT | n/a | T G G | ACCGGT GGC | | | | |
| 198 | AKDI S | 4960 | GCAAAGG ACATTTC T | 5132 | IAAR P | 4483 | ATAGCAGC AAGGCCT | 5249 | V G R | GTTGGC AGA | | | | |
| 199 | AKDF T | 4961 | GCTAAAG ATTTCAC T | 5133 | AARP | 4486 | GCCGCAAG ACCT | 5250 | V Q G | GTGCAA GGA | | | | |
| 200 | AKQG | 4962 | GCCAAGC AAGGA | 5134 | ARP | n/a | GCTAGACC T | n/a | V T A | GTCACA GCT | | | | |

| | | | | | | | | | | | | | | |
|---|---|---|---|---|---|---|---|---|---|---|---|---|---|---|
| ¹ "AR" and "AK" refer to the last two C-terminal amino acids of the heavy chain chassis used in the current example. They are not part of the TN1 segment. | | | | | | | | | | | | | | |

**Table 26. Theoretical segment pool of 300 TN1 segments (plus AR/AK; which is not part of TN1) used in the library of Example 12.**

| **Peptide (plus AR or AK)¹** | **SEQ ID NO** | **Nucleotide (plus AR or AK)¹** | **SEQ ID NO** | **In ELD-3?** |
|---|---|---|---|---|
| AR | n/a | GCCAGA | n/a | YES |
| AK | n/a | GCCAAG | n/a | YES |
| ARE | n/a | GCCAGAGAG | n/a | YES |
| AKE | n/a | GCCAAGGAG | n/a | YES |
| ARD | n/a | GCCAGAGAC | n/a | YES |
| AKD | n/a | GCCAAGGAC | n/a | YES |
| ARG | n/a | GCCAGAGGA | n/a | YES |
| AKG | n/a | GCCAAGGGA | n/a | YES |
| AREG | 4791 | GCCAGAGAGGGA | 4963 | YES |
| AKEG | 4877 | GCCAAGGAAGGA | 5049 | YES |
| ARDG | 4792 | GCCAGAGACGGA | 4964 | YES |
| AKDG | 4878 | GCCAAGGACGGA | 5050 | YES |
| ARGG | 4793 | GCCAGAGGTGGA | 4965 | YES |
| AKGG | 4879 | GCCAAGGGCGGA | 5051 | YES |
| ARR | n/a | GCCAGGAGA | n/a | YES |
| AKR | n/a | GCCAAGAGA | n/a | YES |
| ARER | 4794 | GCCAGAGAGAGA | 4966 | YES |
| AKER | 4880 | GCCAAGGAAAGA | 5052 | YES |
| ARDR | 4795 | GCCAGAGACAGA | 4967 | YES |
| AKDR | 4881 | GCCAAGGACAGA | 5053 | YES |
| ARGR | 4796 | GCCAGAGGCAGA | 4968 | YES |
| AKGR | 4882 | GCCAAGGGCAGA | 5054 | YES |
| ARS | n/a | GCCAGATCT | n/a | YES |
| AKS | n/a | GCCAAGTCT | n/a | YES |
| ARES | 4797 | GCCAGAGAATCT | 4969 | YES |
| AKES | 4883 | GCCAAGGAATCT | 5055 | YES |
| ARDS | 4798 | GCCAGAGACTCT | 4970 | YES |
| AKDS | 4884 | GCCAAGGATTCT | 5056 | YES |
| ARGS | 4799 | GCCAGAGGTTCT | 4971 | YES |
| AKGS | 4885 | GCCAAGGGATCT | 5057 | YES |
| ARP | n/a | GCCAGACCT | n/a | YES |
| AKP | n/a | GCCAAGCCT | n/a | YES |
| AREP | 5328 | GCCAGAGAGCCT | 5428 | NO |
| AKEP | 5329 | GCCAAGGAGCCT | 5429 | NO |
| ARDP | 4800 | GCCAGAGACCCT | 4972 | YES |
| AKDP | 4886 | GCCAAGGATCCT | 5058 | YES |
| ARGP | 4801 | GCCAGAGGGCCT | 4973 | YES |
| AKGP | 4887 | GCCAAGGGTCCT | 5059 | YES |
| ARL | n/a | GCCAGATTG | n/a | YES |
| AKL | n/a | GCCAAGTTG | n/a | YES |
| AREL | 5330 | GCCAGAGAGTTG | 5430 | NO |
| AKEL | 5331 | GCCAAGGAATTG | 5431 | NO |
| ARDL | 4802 | GCCAGAGACTTG | 4974 | YES |
| AKDL | 4888 | GCCAAGGACTTG | 5060 | YES |
| ARGL | 4803 | GCCAGAGGGTTG | 4975 | YES |
| AKGL | 4889 | GCCAAGGGGTTG | 5061 | YES |
| ARA | n/a | GCCAGAGCT | n/a | YES |
| AKA | n/a | GCCAAGGCT | n/a | YES |
| AREA | 4804 | GCCAGAGAGGCT | 4976 | YES |
| AKEA | 4890 | GCCAAGGAAGCT | 5062 | YES |
| ARDA | 4805 | GCCAGAGATGCT | 4977 | YES |
| AKDA | 4891 | GCCAAGGACGCT | 5063 | YES |
| ARGA | 4806 | GCCAGAGGTGCT | 4978 | YES |
| AKGA | 4892 | GCCAAGGGCGCT | 5064 | YES |
| ART | n/a | GCCAGAACT | n/a | YES |
| AKT | n/a | GCCAAGACT | n/a | YES |
| ARET | 4807 | GCCAGAGAGACT | 4979 | YES |
| AKET | 4893 | GCCAAGGAAACT | 5065 | YES |
| ARDT | 4808 | GCCAGAGATACT | 4980 | YES |
| AKDT | 4894 | GCCAAGGATACT | 5066 | YES |
| ARGT | 4809 | GCCAGAGGCACT | 4981 | YES |
| AKGT | 4895 | GCCAAGGGAACT | 5067 | YES |
| ARV | n/a | GCCAGAGTG | n/a | YES |
| AKV | n/a | GCCAAGGTG | n/a | YES |
| AREV | 4810 | GCCAGAGAGGTG | 4982 | YES |
| AKEV | 4896 | GCCAAGGAAGTG | 5068 | YES |
| ARDV | 4811 | GCCAGAGATGTG | 4983 | YES |
| AKDV | 4897 | GCCAAGGACGTG | 5069 | YES |
| ARGV | 4812 | GCCAGAGGGGTG | 4984 | YES |
| AKGV | 4898 | GCCAAGGGCGTG | 5070 | YES |
| AREGG | 4813 | GCCAGAGAGGGAGGA | 4985 | YES |
| AKEGG | 4899 | GCCAAGGAGGGAGGA | 5071 | YES |
| ARDGG | 4814 | GCCAGAGATGGTGGA | 4986 | YES |
| AKDGG | 4900 | GCCAAGGACGGTGGA | 5072 | YES |
| ARGGG | 4815 | GCCAGAGGTGGAGGA | 4987 | YES |
| AKGGG | 4901 | GCCAAGGGAGGAGGA | 5073 | YES |
| AREGR | 5332 | GCCAGAGAAGGGAGA | 5432 | NO |
| AKEGR | 5333 | GCCAAGGAAGGCAGA | 5433 | NO |
| ARDGR | 4816 | GCCAGAGACGGCAGA | 4988 | YES |
| AKDGR | 4902 | GCCAAGGACGGTAGA | 5074 | YES |
| ARGGR | 5334 | GCCAGAGGAGGTAGA | 5434 | NO |
| AKGGR | 5335 | GCCAAGGGAGGTAGA | 5435 | NO |
| AREGS | 5336 | GCCAGAGAAGGATCT | 5436 | NO |
| AKEGS | 5337 | GCCAAGGAAGGATCT | 5437 | NO |
| ARDGS | 5338 | GCCAGAGACGGATCT | 5438 | NO |
| AKDGS | 5339 | GCCAAGGATGGTTCT | 5439 | NO |
| ARGGS | 4817 | GCCAGAGGCGGTTCT | 4989 | YES |
| AKGGS | 4903 | GCCAAGGGAGGTTCT | 5075 | YES |
| AREGP | 5340 | GCCAGAGAAGGTCCT | 5440 | NO |
| AKEGP | 5341 | GCCAAGGAGGGGCCT | 5441 | NO |
| ARDGP | 5342 | GCCAGAGACGGTCCT | 5442 | NO |
| AKDGP | 5343 | GCCAAGGACGGTCCT | 5443 | NO |
| ARGGP | 4818 | GCCAGAGGTGGGCCT | 4990 | YES |
| AKGGP | 4904 | GCCAAGGGAGGTCCT | 5076 | YES |
| AREGL | 5344 | GCCAGAGAGGGCTTG | 5444 | NO |
| AKEGL | 5345 | GCCAAGGAAGGGTTG | 5445 | NO |
| ARDGL | 5346 | GCCAGAGATGGGTTG | 5446 | NO |
| AKDGL | 5347 | GCCAAGGACGGTTTG | 5447 | NO |
| ARGGL | 5348 | GCCAGAGGTGGATTG | 5448 | NO |
| AKGGL | 5349 | GCCAAGGGAGGGTTG | 5449 | NO |
| AREGA | 5350 | GCCAGAGAAGGAGCT | 5450 | NO |
| AKEGA | 5351 | GCCAAGGAGGGAGCT | 5451 | NO |
| ARDGA | 5352 | GCCAGAGATGGCGCT | 5452 | NO |
| AKDGA | 5353 | GCCAAGGATGGAGCT | 5453 | NO |
| ARGGA | 4819 | GCCAGAGGAGGTGCT | 4991 | YES |
| AKGGA | 4905 | GCCAAGGGAGGCGCT | 5077 | YES |
| ARDGT | 4820 | GCCAGAGACGGTACT | 4992 | YES |
| AKDGT | 4906 | GCCAAGGATGGCACT | 5078 | YES |
| ARGGT | 4821 | GCCAGAGGTGGAACT | 4993 | YES |
| AKGGT | 4907 | GCCAAGGGAGGCACT | 5079 | YES |
| AREGV | 4822 | GCCAGAGAGGGAGTG | 4994 | YES |
| AKEGV | 4908 | GCCAAGGAAGGAGTG | 5080 | YES |
| ARDGV | 5354 | GCCAGAGATGGTGTG | 5454 | NO |
| AKDGV | 5355 | GCCAAGGATGGTGTG | 5455 | NO |
| ARGGV | 4823 | GCCAGAGGTGGCGTG | 4995 | YES |
| AKGGV | 4909 | GCCAAGGGCGGTGTG | 5081 | YES |
| ARRG | 4824 | GCCAGAAGAGGA | 4996 | YES |
| AKRG | 4910 | GCCAAGAGAGGA | 5082 | YES |
| ARERG | 4825 | GCCAGAGAGCGTGGA | 4997 | YES |
| AKERG | 4911 | GCCAAGGAGAGAGGA | 5083 | YES |
| ARDRG | 4826 | GCCAGAGATCGTGGA | 4998 | YES |
| AKDRG | 4912 | GCCAAGGATAGGGGA | 5084 | YES |
| ARGRG | 4827 | GCCAGAGGCAGGGGA | 4999 | YES |
| AKGRG | 4913 | GCCAAGGGTAGGGGA | 5085 | YES |
| ARSG | 4828 | GCCAGATCAGGA | 5000 | YES |
| AKSG | 4914 | GCCAAGTCTGGA | 5086 | YES |
| ARESG | 5356 | GCCAGAGAGTCTGGA | 5456 | NO |
| AKESG | 5357 | GCCAAGGAAAGTGGA | 5457 | NO |
| ARDSG | 4829 | GCCAGAGACTCAGGA | 5001 | YES |
| AKDSG | 4915 | GCCAAGGATAGTGGA | 5087 | YES |
| ARGSG | 5358 | GCCAGAGGCTCTGGA | 5458 | NO |
| AKGSG | 5359 | GCCAAGGGGTCTGGA | 5459 | NO |
| ARPG | 5360 | GCCAGACCAGGA | 5460 | NO |
| AKPG | 5361 | GCCAAGCCCGGA | 5461 | NO |
| ARDPG | 4830 | GCCAGAGATCCAGGA | 5002 | YES |
| AKDPG | 4916 | GCCAAGGACCCCGGA | 5088 | YES |
| ARGPG | 5362 | GCCAGAGGACCTGGA | 5462 | NO |
| AKGPG | 5363 | GCCAAGGGGCCTGGA | 5463 | NO |
| ARLG | 4831 | GCCAGATTGGGA | 5003 | YES |
| AKLG | 4917 | GCCAAGTTAGGA | 5089 | YES |
| ARDLG | 4832 | GCCAGAGACTTGGGA | 5004 | YES |
| AKDLG | 4918 | GCCAAGGATCTTGGA | 5090 | YES |
| ARGLG | 5364 | GCCAGAGGACTAGGA | 5464 | NO |
| AKGLG | 5365 | GCCAAGGGTTTGGGA | 5465 | NO |
| ARAG | 4833 | GCCAGAGCTGGA | 5005 | YES |
| AKAG | 4919 | GCCAAGGCTGGA | 5091 | YES |
| AREAG | 5366 | GCCAGAGAAGCCGGA | 5466 | NO |
| AKEAG | 5367 | GCCAAGGAGGCTGGA | 5467 | NO |
| ARDAG | 5368 | GCCAGAGACGCAGGA | 5468 | NO |
| AKDAG | 5369 | GCCAAGGATGCCGGA | 5469 | NO |
| ARGAG | 5370 | GCCAGAGGTGCCGGA | 5470 | NO |
| AKGAG | 5371 | GCCAAGGGAGCAGGA | 5471 | NO |
| ARTG | 5372 | GCCAGAACTGGA | 5472 | NO |
| AKTG | 5373 | GCCAAGACCGGA | 5473 | NO |
| ARDTG | 5374 | GCCAGAGACACGGGA | 5474 | NO |
| AKDTG | 5375 | GCCAAGGATACGGGA | 5475 | NO |
| ARVG | 4834 | GCCAGAGTGGGA | 5006 | YES |
| AKVG | 4920 | GCCAAGGTAGGA | 5092 | YES |
| AREVG | 5376 | GCCAGAGAAGTCGGA | 5476 | NO |
| AKEVG | 5377 | GCCAAGGAGGTAGGA | 5477 | NO |
| ARDVG | 5378 | GCCAGAGATGTAGGA | 5478 | NO |
| AKDVG | 5379 | GCCAAGGACGTAGGA | 5479 | NO |
| ARGVG | 4835 | GCCAGAGGCGTAGGA | 5007 | YES |
| AKGVG | 4921 | GCCAAGGGTGTCGGA | 5093 | YES |
| ARPR | 4836 | GCCAGACCCAGA | 5008 | YES |
| AKPR | 4922 | GCCAAGCCTAGA | 5094 | YES |
| ARDPR | 5380 | GCCAGAGATCCAAGA | 5480 | NO |
| AKDPR | 5381 | GCCAAGGATCCTAGA | 5481 | NO |
| ARGPR | 4837 | GCCAGAGGACCAAGA | 5009 | YES |
| AKGPR | 4923 | GCCAAGGGCCCCAGA | 5095 | YES |
| ARPS | 4838 | GCCAGACCATCT | 5010 | YES |
| AKPS | 4924 | GCCAAGCCTTCT | 5096 | YES |
| ARDPS | 4839 | GCCAGAGATCCCTCT | 5011 | YES |
| AKDPS | 4925 | GCCAAGGATCCCTCT | 5097 | YES |
| ARGPS | 4840 | GCAAGAGGACCTTCT | 5012 | YES |
| AKGPS | 4926 | GCCAAAGGGCCATCT | 5098 | YES |
| ARPP | 5382 | GCCAGACCACCT | 5482 | NO |
| AKPP | 5383 | GCCAAGCCACCT | 5483 | NO |
| ARDPP | 4841 | GCCAGAGACCCACCT | 5013 | YES |
| AKDPP | 4927 | GCCAAGGATCCACCT | 5099 | YES |
| ARGPP | 4842 | GCCAGAGGACCGCCT | 5014 | YES |
| AKGPP | 4928 | GCCAAGGGCCCTCCT | 5100 | YES |
| ARPL | 4843 | GCCAGACCGTTG | 5015 | YES |
| AKPL | 4929 | GCCAAGCCGTTG | 5101 | YES |
| ARDPL | 4844 | GCCAGAGATCCTTTG | 5016 | YES |
| AKDPL | 4930 | GCCAAGGACCCTTTG | 5102 | YES |
| ARGPL | 5384 | GCCAGAGGTCCCTTG | 5484 | NO |
| AKGPL | 5385 | GCCAAGGGGCCGTTG | 5485 | NO |
| ARPA | 5386 | GCCAGACCAGCT | 5486 | NO |
| AKPA | 5387 | GCCAAGCCGGCT | 5487 | NO |
| ARDPA | 5388 | GCCAGAGATCCCGCT | 5488 | NO |
| AKDPA | 5389 | GCCAAGGACCCCGCT | 5489 | NO |
| ARPT | 5390 | GCCAGACCTACT | 5490 | NO |
| AKPT | 5391 | GCCAAGCCTACT | 5491 | NO |
| ARDPT | 5392 | GCCAGAGATCCGACT | 5492 | NO |
| AKDPT | 5393 | GCCAAGGACCCTACT | 5493 | NO |
| ARGPT | 5394 | GCCAGAGGACCCACT | 5494 | NO |
| AKGPT | 5395 | GCCAAGGGGCCCACT | 5495 | NO |
| ARPV | 5396 | GCCAGACCGGTG | 5496 | NO |
| AKPV | 5397 | GCCAAGCCAGTG | 5497 | NO |
| ARDPV | 5398 | GCCAGAGATCCGGTG | 5498 | NO |
| AKDPV | 5399 | GCCAAGGACCCTGTG | 5499 | NO |
| ARRP | 4845 | GCCAGAAGGCCT | 5017 | YES |
| AKRP | 4931 | GCCAAGAGGCCT | 5103 | YES |
| ARDRP | 4846 | GCCAGAGACCGTCCT | 5018 | YES |
| AKDRP | 4932 | GCCAAGGACCGTCCT | 5104 | YES |
| ARGRP | 5400 | GCCAGAGGAAGGCCT | 5500 | NO |
| AKGRP | 5401 | GCCAAGGGCCGTCCT | 5501 | NO |
| ARSP | 4847 | GCCAGATCACCT | 5019 | YES |
| AKSP | 4933 | GCCAAGAGTCCT | 5105 | YES |
| ARDSP | 5402 | GCCAGAGACTCTCCT | 5502 | NO |
| AKDSP | 5403 | GCCAAGGACTCCCCT | 5503 | NO |
| ARGSP | 5404 | GCCAGAGGTTCCCCT | 5504 | NO |
| AKGSP | 5405 | GCCAAGGGTTCACCT | 5505 | NO |
| ARLP | 4848 | GCCAGACTTCCT | 5020 | YES |
| AKLP | 4934 | GCCAAGCTACCT | 5106 | YES |
| ARDLP | 5406 | GCCAGAGATCTTCCT | 5506 | NO |
| AKDLP | 5407 | GCCAAGGATCTACCT | 5507 | NO |
| ARAP | 4849 | GCCAGAGCCCCT | 5021 | YES |
| AKAP | 4935 | GCCAAGGCTCCT | 5107 | YES |
| ARDAP | 5408 | GCCAGAGATGCTCCT | 5508 | NO |
| AKDAP | 5409 | GCCAAGGATGCTCCT | 5509 | NO |
| ARGAP | 5410 | GCCAGAGGGGCCCCT | 5510 | NO |
| AKGAP | 5411 | GCCAAGGGTGCCCCT | 5511 | NO |
| ARTP | 4850 | GCCAGAACTCCT | 5022 | YES |
| AKTP | 4936 | GCCAAGACGCCT | 5108 | YES |
| ARDTP | 5412 | GCCAGAGATACCCCT | 5512 | NO |
| AKDTP | 5413 | GCCAAGGACACGCCT | 5513 | NO |
| ARVP | 4851 | GCCAGAGTCCCT | 5023 | YES |
| AKVP | 4937 | GCCAAGGTACCT | 5109 | YES |
| ARAGG | 5414 | GCCAGAGCTGGCGGA | 5514 | NO |
| AKAGG | 5415 | GCCAAGGCCGGTGGA | 5515 | NO |
| ARDGGG | 5416 | GCCAGAGATGGTGGCGGA | 5516 | NO |
| AKDGGG | 5417 | GCCAAGGACGGCGGTGGA | 5517 | NO |
| ARLGG | 5418 | GCCAGATTGGGCGGA | 5518 | NO |
| AKLGG | 5419 | GCCAAGCTAGGCGGA | 5519 | NO |
| ARDLGG | 5420 | GCCAGAGATTTGGGTGGA | 5520 | NO |
| AKDLGG | 5421 | GCCAAGGATTTGGGTGGA | 5521 | NO |
| ARRGG | 5422 | GCCAGAAGAGGTGGA | 5522 | NO |
| AKRGG | 5423 | GCCAAGAGAGGAGGA | 5523 | NO |
| ARDRGG | 5424 | GCCAGAGACCGTGGCGGA | 5524 | NO |
| AKDRGG | 5425 | GCCAAGGACAGAGGTGGA | 5525 | NO |
| ARSGG | 5426 | GCCAGATCAGGCGGA | 5526 | NO |
| AKSGG | 5427 | GCCAAGTCCGGTGGA | 5527 | NO |
| ARVGG | 4852 | GCCAGAGTTGGAGGA | 5024 | YES |
| AKVGG | 4938 | GCCAAGGTGGGTGGA | 5110 | YES |
| ARQ | n/a | GCAAGACAG | n/a | YES |
| AKQ | n/a | GCAAAACAG | n/a | YES |
| ARH | n/a | GCCAGACAC | n/a | YES |
| AKH | n/a | GCCAAACAC | n/a | YES |
| ARDQ | 4853 | GCCAGGGACCAG | 5025 | YES |
| AKDQ | 4939 | GCCAAGGATCAG | 5111 | YES |
| ARDH | 4854 | GCAAGAGACCAC | 5026 | YES |
| AKDH | 4940 | GCTAAAGACCAC | 5112 | YES |
| ARAR | 4855 | GCAAGGGCTAGA | 5027 | YES |
| AKAR | 4941 | GCCAAGGCAAGA | 5113 | YES |
| ARAS | 4856 | GCTAGGGCATCT | 5028 | YES |
| AKAS | 4942 | GCCAAGGCATCT | 5114 | YES |
| ARDQG | 4857 | GCTAGGGATCAGGGA | 5029 | YES |
| AKDQG | 4943 | GCAAAGGATCAGGGA | 5115 | YES |
| ARSR | 4858 | GCTAGATCAAGA | 5030 | YES |
| AKSR | 4944 | GCCAAGAGTAGA | 5116 | YES |
| ARDRS | 4859 | GCCAGGGACAGGTCT | 5031 | YES |
| AKDRS | 4945 | GCAAAAGACAGGTCT | 5117 | YES |
| ARSL | 4860 | GCTAGATCTTTG | 5032 | YES |
| AKSL | 4946 | GCAAAGAGCTTG | 5118 | YES |
| ARLH | 4861 | GCTAGGTTGCAC | 5033 | YES |
| AKLH | 4947 | GCTAAATTGCAC | 5119 | YES |
| ARDLT | 4862 | GCCAGGGATTTGACT | 5034 | YES |
| AKDLT | 4948 | GCCAAGGACTTGACT | 5120 | YES |
| ARK | n/a | GCCAGAAAG | n/a | YES |
| AKK | n/a | GCTAAAAAG | n/a | YES |
| ARAE | 4863 | GCAAGAGCCGAG | 5035 | YES |
| AKAE | 4949 | GCTAAAGCAGAG | 5121 | YES |
| ARDLS | 4864 | GCAAGGGATTTGTCT | 5036 | YES |
| AKDLS | 4950 | GCAAAAGACTTGTCT | 5122 | YES |
| ARGD | 4865 | GCTAGAGGGGAC | 5037 | YES |
| AKGD | 4951 | GCAAAAGGGGAC | 5123 | YES |
| ARRR | 4866 | GCTAGGAGGAGA | 5038 | YES |
| AKRR | 4952 | GCAAAAAGGAGA | 5124 | YES |
| ARDK | 4867 | GCTAGAGATAAG | 5039 | YES |
| AKDK | 4953 | GCAAAAGACAAG | 5125 | YES |
| ARVS | 4868 | GCTAGAGTATCT | 5040 | YES |
| AKVS | 4954 | GCCAAAGTATCT | 5126 | YES |
| ARDRL | 4869 | GCCAGAGACAGGTTG | 5041 | YES |
| AKDRL | 4955 | GCAAAAGACAGGTTG | 5127 | YES |
| ARGQ | 4870 | GCTAGGGGCCAG | 5042 | YES |
| AKGQ | 4956 | GCCAAAGGACAG | 5128 | YES |
| ARVR | 4871 | GCCAGGGTCAGA | 5043 | YES |
| AKVR | 4957 | GCAAAAGTCAGA | 5129 | YES |
| ARAK | 4872 | GCTAGGGCTAAG | 5044 | YES |
| AKAK | 4958 | GCAAAGGCAAAG | 5130 | YES |
| ARGK | 4873 | GCCAGGGGTAAG | 5045 | YES |
| AKGK | 4959 | GCAAAGGGCAAG | 5131 | YES |
| ARDIS | 4874 | GCAAGGGATATTTCT | 5046 | YES |
| AKDIS | 4960 | GCAAAGGACATTTCT | 5132 | YES |
| ARDFT | 4875 | GCTAGGGATTTCACT | 5047 | YES |
| AKDFT | 4961 | GCTAAAGATTTCACT | 5133 | YES |
| ARQG | 4876 | GCCAGGCAGGGA | 5048 | YES |
| AKQG | 4862 | GCCAAGCAAGGA | 5134 | YES |

| | | | | |
|---|---|---|---|---|
| ¹ "AR" and "AK" refer to the last two C-terminal amino acids of the heavy chain chassis used in the current example. They are not part of the TN1 segment. | | | | |

**Table 27. Theoretical segment pool of degenerate oligonucleotide sequences encoding DH segments of Example 13.**

| **Name** | **Degenerate Oligo** | **Peptide Length** | **SEQ ID NO** |
|---|---|---|---|
| DH_001 | KHTGAK | 2 | n/a |
| DH_002 | KHTKGG | 2 | n/a |
| DH_003 | KHTCMT | 2 | n/a |
| DH_004 | KHTMCT | 2 | n/a |
| DH_005 | GVCWSG | 2 | n/a |
| DH_006 | SVCYAT | 2 | n/a |
| DH_007 | BYCSAG | 2 | n/a |
| DH_008 | SBAMAG | 2 | n/a |
| DH_009 | VSCMAA | 2 | n/a |
| DH_010 | GRABYT | 2 | n/a |
| DH_011 | GRAKBG | 2 | n/a |
| DH_012 | RDAGAK | 2 | n/a |
| DH_013 | RDAGRT | 2 | n/a |
| DH_014 | YHTSAC | 2 | n/a |
| DH_015 | YHTKAC | 2 | n/a |
| DH_016 | YHTMCG | 2 | n/a |
| DH_017 | MHAGAW | 2 | n/a |
| DH_018 | MHAGRT | 2 | n/a |
| DH_019 | MHAMCT | 2 | n/a |
| DH_020 | MBCYAT | 2 | n/a |
| DH_021 | CVACNG | 2 | n/a |
| DH_022 | MSCAHG | 2 | n/a |
| DH_023 | CRGKBG | 2 | n/a |
| DH_024 | WSGHCT | 2 | n/a |
| DH_025 | WGGKHT | 2 | n/a |
| DH_026 | BGGSAK | 2 | n/a |
| DH_027 | BWCAMA | 2 | n/a |
| DH_028 | BHCTGG | 2 | n/a |
| DH_029 | TGGVBT | 2 | n/a |
| DH_030 | BHCAGT | 2 | n/a |
| DH_031 | SRTATT | 2 | n/a |
| DH_032 | ACABHT | 2 | n/a |
| DH_033 | SVCGCT | 2 | n/a |
| DH_034 | ATGSVT | 2 | n/a |
| DH_035 | SWGAGG | 2 | n/a |
| DH_036 | GTAGCAVBT | 3 | n/a |
| DH_037 | DBGSWACTT | 3 | n/a |
| DH_038 | VNCBCAGGT | 3 | n/a |
| DH_039 | VNCDCATAT | 3 | n/a |
| DH_040 | VHAKKGTTG | 3 | n/a |
| DH_041 | CCAGCABHT | 3 | n/a |
| DH_042 | VHASRACTT | 3 | n/a |
| DH_043 | BHCAGCRST | 3 | n/a |
| DH_044 | BHCGGAKMT | 3 | n/a |
| DH_045 | BHCGGAGDT | 3 | n/a |
| DH_046 | BHCAGCKMT | 3 | n/a |
| DH_047 | NHCCRACTT | 3 | n/a |
| DH_048 | NHCAGCKGG | 3 | n/a |
| DH_049 | BHCGGAKSG | 3 | n/a |
| DH_050 | VBCGGAGNT | 3 | n/a |
| DH_051 | NHCAGCGVT | 3 | n/a |
| DH_052 | NHCTACGVT | 3 | n/a |
| DH_053 | NHCAGCGVG | 3 | n/a |
| DH_054 | VHATGGSYG | 3 | n/a |
| DH_055 | VNCGHCTAT | 3 | n/a |
| DH_056 | GGTRNACTT | 3 | n/a |
| DH_057 | NHCABAGGT | 3 | n/a |
| DH_058 | VHAGCAGNT | 3 | n/a |
| DH_059 | DBGKYCGGT | 3 | n/a |
| DH_060 | BHCGGARKT | 3 | n/a |
| DH_061 | NHCGTAGVT | 3 | n/a |
| DH_062 | VNTTHCTAT | 3 | n/a |
| DH_063 | GTAGTABHT | 3 | n/a |
| DH_064 | VBCGNCCTT | 3 | n/a |
| DH_065 | BHCGGAGNG | 3 | n/a |
| DH_066 | VNCGHCGGT | 3 | n/a |
| DH_067 | AGGBHCGGT | 3 | n/a |
| DH_068 | VNCTBGTAT | 3 | n/a |
| DH_069 | VNCTBGCTT | 3 | n/a |
| DH_070 | NHCKACTAT | 3 | n/a |
| DH_071 | CTARNACTT | 3 | n/a |
| DH_072 | NHCBCAGGT | 3 | n/a |
| DH_073 | NHCTACBAT | 3 | n/a |
| DH_074 | BHCACAGCCAKS | 4 | 5528 |
| DH_075 | VHGGBAGCAACT | 4 | 5529 |
| DH_076 | DBGTTCGGAGNG | 4 | 5530 |
| DH_077 | BHCGGAKMCTAT | 4 | 5531 |
| DH_078 | TACAGCAGCVBT | 4 | 5532 |
| DH_079 | VHGGTARSAGGT | 4 | 5533 |
| DH_080 | VBCGACGGATHT | 4 | 5534 |
| DH_081 | NHCTACGGAGVT | 4 | 5535 |
| DH_082 | VHGRYGGCAACT | 4 | 5536 |
| DH_083 | VHATACAGCRST | 4 | 5537 |
| DH_084 | CAGTGGCTABHT | 4 | 5538 |
| DH_085 | VHAGTAGCAGNT | 4 | 5539 |
| DH_086 | VBCACAGTARMG | 4 | 5540 |
| DH_087 | AGCAGCAGCDBG | 4 | 5541 |
| DH_088 | NHCTMCTACGGT | 4 | 5542 |
| DH_089 | BHCAGCTGGTHT | 4 | 5543 |
| DH_090 | VHACAACTAGNT | 4 | 5544 |
| DH_091 | BHCGGAAGCKMT | 4 | 5545 |
| DH_092 | BHCGGATSGTAT | 4 | 5546 |
| DH_093 | NHCAGCGGABGG | 4 | 5547 |
| DH_094 | BHCGGATACKMT | 4 | 5548 |
| DH_095 | VHAGTAACARMG | 4 | 5549 |
| DH_096 | GCAGCAGCAVBT | 4 | 5550 |
| DH_097 | VBCAYATTCGGT | 4 | 5551 |
| DH_098 | GTAGCAGCAVHA | 4 | 5552 |
| DH_099 | NHCTACTACGVT | 4 | 5553 |
| DH_100 | VBCKMCGGATAT | 4 | 5554 |
| DH_101 | VHACAACTAKKG | 4 | 5555 |
| DH_102 | VHGGGARKCGCT | 4 | 5556 |
| DH_103 | VBTBTCGGAGAG | 4 | 5557 |
| DH_104 | BHCTACAGCKMT | 4 | 5558 |
| DH_105 | VHAGTASSAGCT | 4 | 5559 |
| DH_106 | VBTCDAGGAGTT | 4 | 5560 |
| DH_107 | GACAGCAGCDBG | 4 | 5561 |
| DH_108 | VBCGVCTACAGT | 4 | 5562 |
| DH_109 | NHCTACGGAKCT | 4 | 5563 |
| DH_110 | NHCTACTACTHT | 4 | 5564 |
| DH_111 | BHCVGCTACAGT | 4 | 5565 |
| DH_112 | VBCTGGTTCGGT | 4 | 5566 |
| DH_113 | VNCTACTACTHT | 4 | 5567 |
| DH_114 | VHABTCGGAGGT | 4 | 5568 |
| DH_115 | NHCATGGTAAGAGVT | 5 | 5569 |
| DH_116 | NHCTACGGAGACTHT | 5 | 5570 |
| DH_117 | VBCTACAGCTACGNT | b | 5571 |
| DH_118 | VNCAGCGGAAGCTHT | 5 | 5572 |
| DH_119 | VBCTTTCTAGAATBG | 5 | 5573 |
| DH_120 | BHCGGAAGCTACKMT | 5 | 5574 |
| DH_121 | NHCAGCGGAAGCTHT | 5 | 5575 |
| DH_122 | BHCAGCAGCAGCTBG | 5 | 5576 |
| DH_123 | BHCAGCAGCGGATBG | 5 | 5577 |
| DH_124 | VBCACAGTAACAANA | 5 | 5578 |
| DH_125 | BHCAGCAGCAGCTHT | 5 | 5579 |
| DH_126 | NHCAGCGGATGGTHT | 5 | 5580 |
| DH_127 | NHCAGCGGATACGVT | 5 | 5581 |
| DH_128 | NHCTACTACGACABT | 5 | 5582 |
| DH_129 | BHCAGCAGCGGATHT | b | 5583 |
| DH_130 | VHAGCAGCAAGACNT | 5 | 5584 |
| DH_131 | VHGTACTACTACGVT | 5 | 5585 |
| DH_132 | VBCACAATGGTACRG | 5 | 5586 |
| DH_133 | NHCATGGTACRAGGT | 5 | 5587 |
| DH_134 | NHCTSGGGAAGCTAT | 5 | 5588 |
| DH_135 | BHCAGCAGCAGCTGGTHT | 6 | 5589 |
| DH_136 | NHCTACTACGACAGCABT | 6 | 5590 |
| DH_137 | VBCTACAGCGGATACGNT | 6 | 5591 |
| DH_138 | BHCAGCAGCGGATGGTHT | 6 | 5592 |
| DH_139 | NHCTACTACGGAAGCGVT | 6 | 5593 |
| DH_140 | BHCAGCAGCGGATACTHT | 6 | 5594 |
| DH_141 | NHCTACGACAGCAGCGVT | 6 | 5595 |
| DH_142 | NHCGACTTCTGGAGCGVT | 6 | 5596 |
| DH_143 | BHCGACAGCAGCGGATHT | 6 | 5597 |
| DH_144 | SNATACTTCGACTGGYYT | 6 | 5598 |
| DH_145 | TGTRGCRGCACAAGCTGT | 6 | 5599 |
| DH_146 | NHCTACTACGGAAGCGVG | 6 | 5600 |
| DH_147 | TGTRGCRGCGGAAGCTGT | 6 | 5601 |
| DH_148 | NHCTTTTGGAGCGGATHT | 6 | 5602 |
| DH_149 | NHCTACTACGACAGCAGCGVT | 7 | 5603 |
| DH_150 | NHCGACATACTAACAGGATHT | 7 | 5604 |
| DH_151 | NHCTACGACTTCTGGAGCGVT | 7 | 5605 |
| DH_152 | BHCTGTAGCAGCACAAGCTGT | 7 | 5606 |
| DH_153 | DBGTACAGCAGCAGCTGGTHT | 7 | 5607 |
| DH_154 | NHCTACGACAGCAGCGGATHT | 7 | 5608 |
| DH_155 | NHCTACTACGGAAGCGGAABT | 7 | 5609 |
| DH_156 | DBGTACAGCAGCGGATGGTHT | 7 | 5610 |
| DH_157 | BHCTGTAGCGGAGGAAGCTGT | 7 | 5611 |
| DH_158 | TGTAGCGGAGGAAGCTGTYHT | 7 | 5612 |
| DH_159 | TGTAGCAGCACAAGCTGTYHT | 7 | 5613 |
| DH_160 | NHCTGTGGAGGAGACTGTTHT | 7 | 5614 |
| DH_161 | NHCGACTTCTGGAGCGGATHT | 7 | 5615 |
| DH_162 | BHCGACAGCAGCGGATACTHT | 7 | 5616 |
| DH_163 | VHATACTGTGGAGGAGACTGT | 7 | 5617 |
| DH_164 | NHCTACTACGACAGCAGCGGATHT | 8 | 5618 |
| DH_165 | NHCTACTACGGAAGCGGAAGCTHT | 8 | 5619 |
| DH_166 | VBCTACTGTAGCAGCACAAGCTGT | 8 | 5620 |
| DH_167 | VBCTACTGTAGCGGAGGAAGCTGT | 8 | 5621 |
| DH_168 | NHCTACGACTTCTGGAGCGGATHT | 8 | 5622 |
| DH_169 | NHCGACATACTAACAGGATACTHT | 8 | 5623 |
| DH_170 | BHCTGTAGCGGAGGAAGCTGTTHT | 8 | 5624 |
| DH_171 | BHCTGTAGCAGCACAAGCTGTTHT | 8 | 5625 |
| DH_172 | VHATACTGTGGAGGAGACTGTTHT | 8 | 5626 |
| DH_173 | NHCTACGACAGCAGCGGATACTHT | 8 | 5627 |
| DH_174 | VNCTACTACGGAAGCGGAAGCTMT | 8 | 5628 |
| DH_175 | NHCGACTTCTGGAGCGGATACTHT | 8 | 5629 |
| DH_176 | VHACTAAGATACTTCGACTGGYWT | 8 | 5630 |
| DH_177 | NHCTACTACGACAGCAGCGGATACTHT | 9 | 5631 |
| DH_178 | VBCTACTGTAGCGGAGGAAGCTGTTHT | 9 | 5632 |
| DH_179 | VBCTACTGTAGCAGCACAAGCTGTTHT | 9 | 5633 |
| DH_180 | NHCTACGACTTCTGGAGCGGATACTHT | 9 | 5634 |
| DH_181 | BHCTGTAGCGGAGGAAGCTGTTACTHT | 9 | 5635 |
| DH_182 | NHCTACTACGACAGCAGCGGATACTACTHT | 10 | 5636 |
| DH_183 | NHCTACGACTACGTATGGGGAAGCTACGCATHT | 11 | 5637 |
| DH_184 | NHCTACGACTACGTATGGGGAAGCTACGCATACAHA | 12 | 5638 |

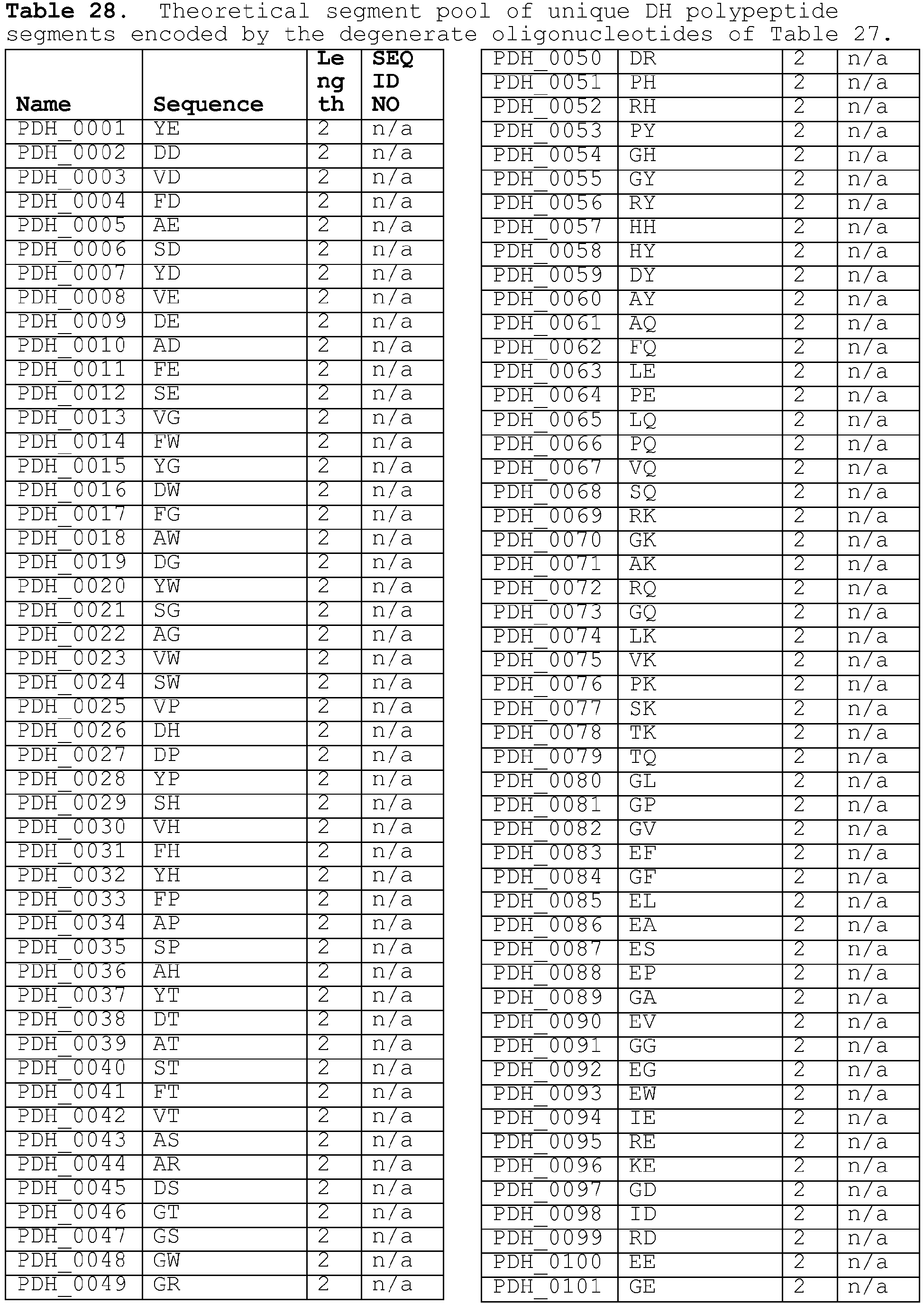

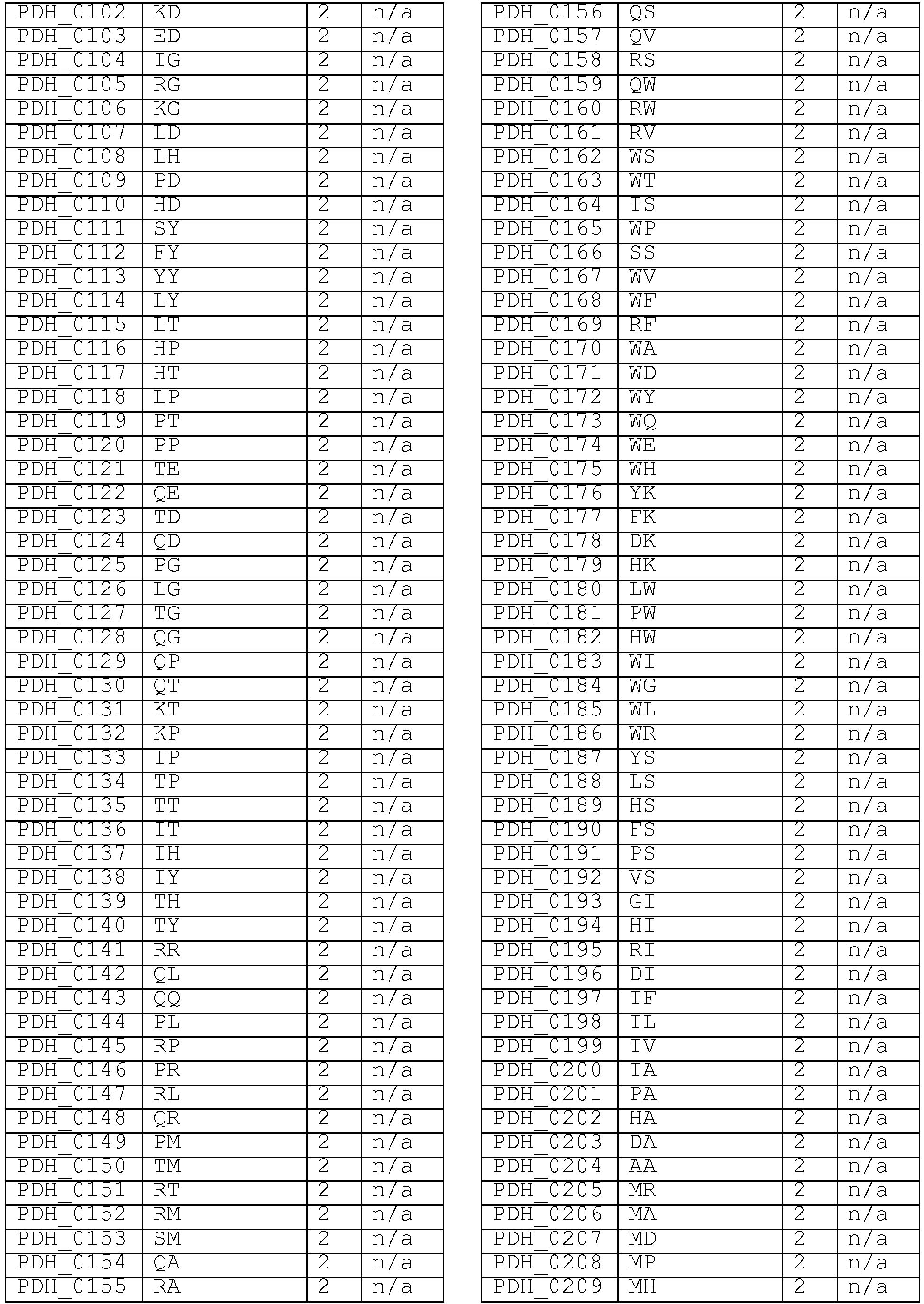

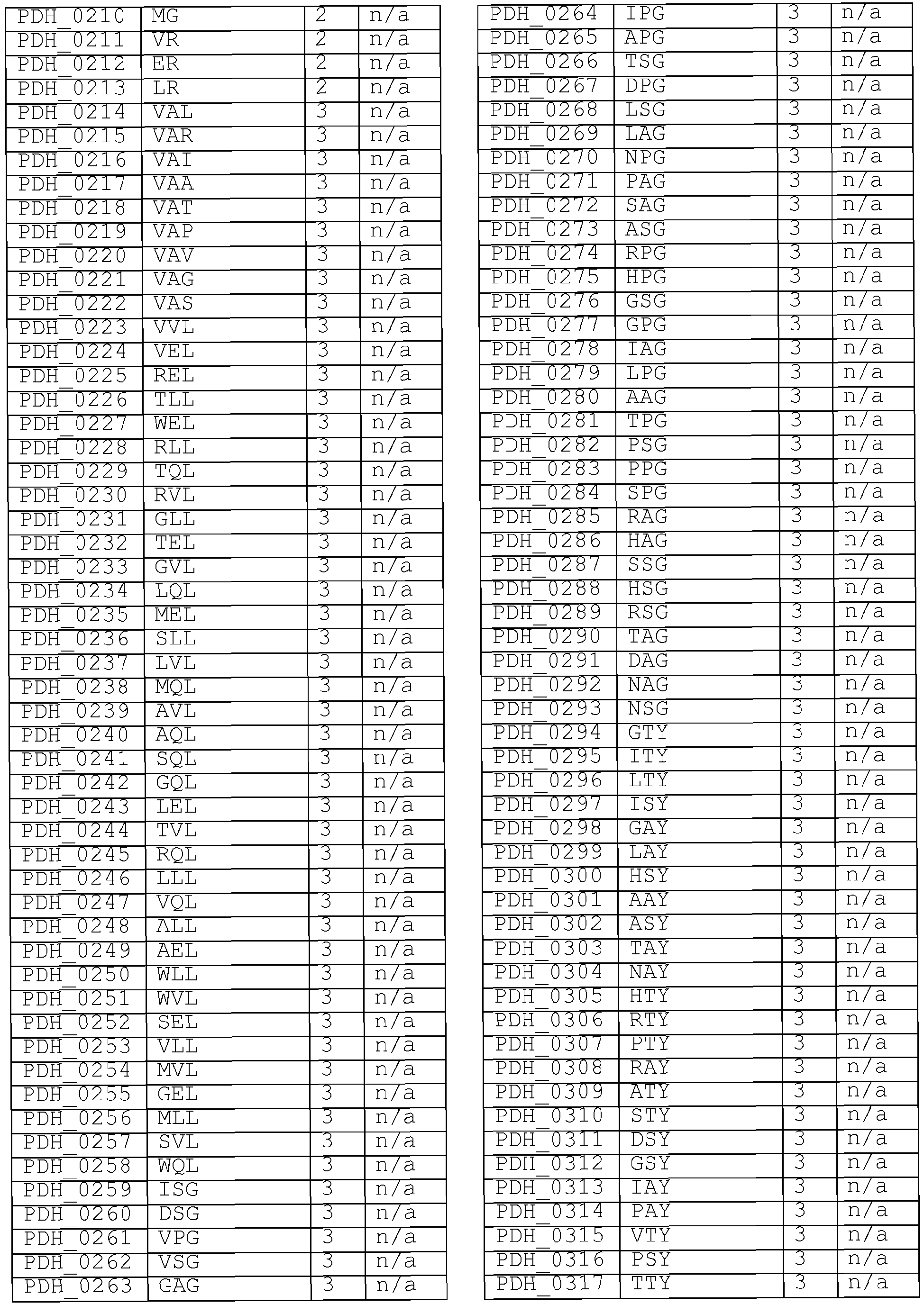

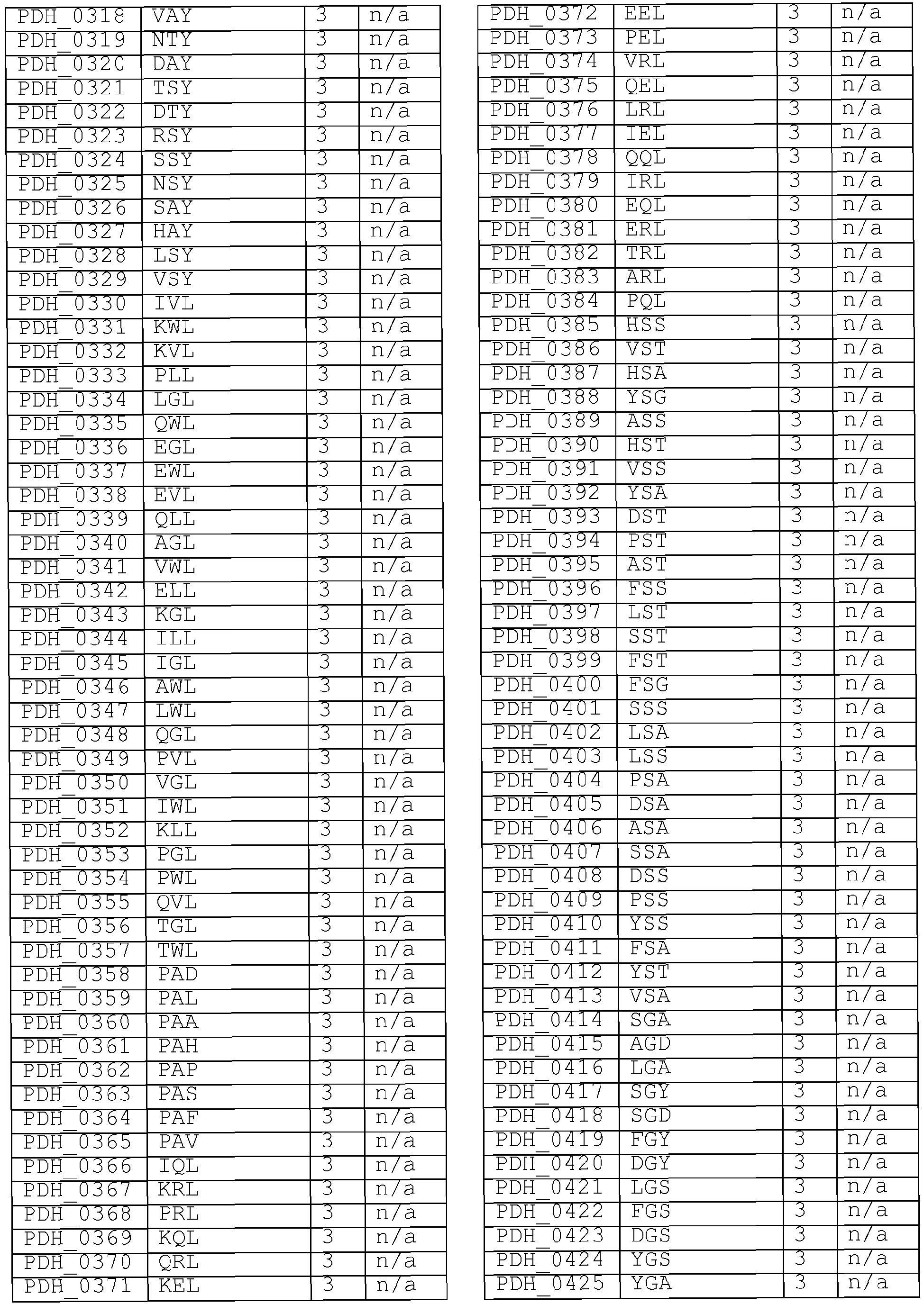

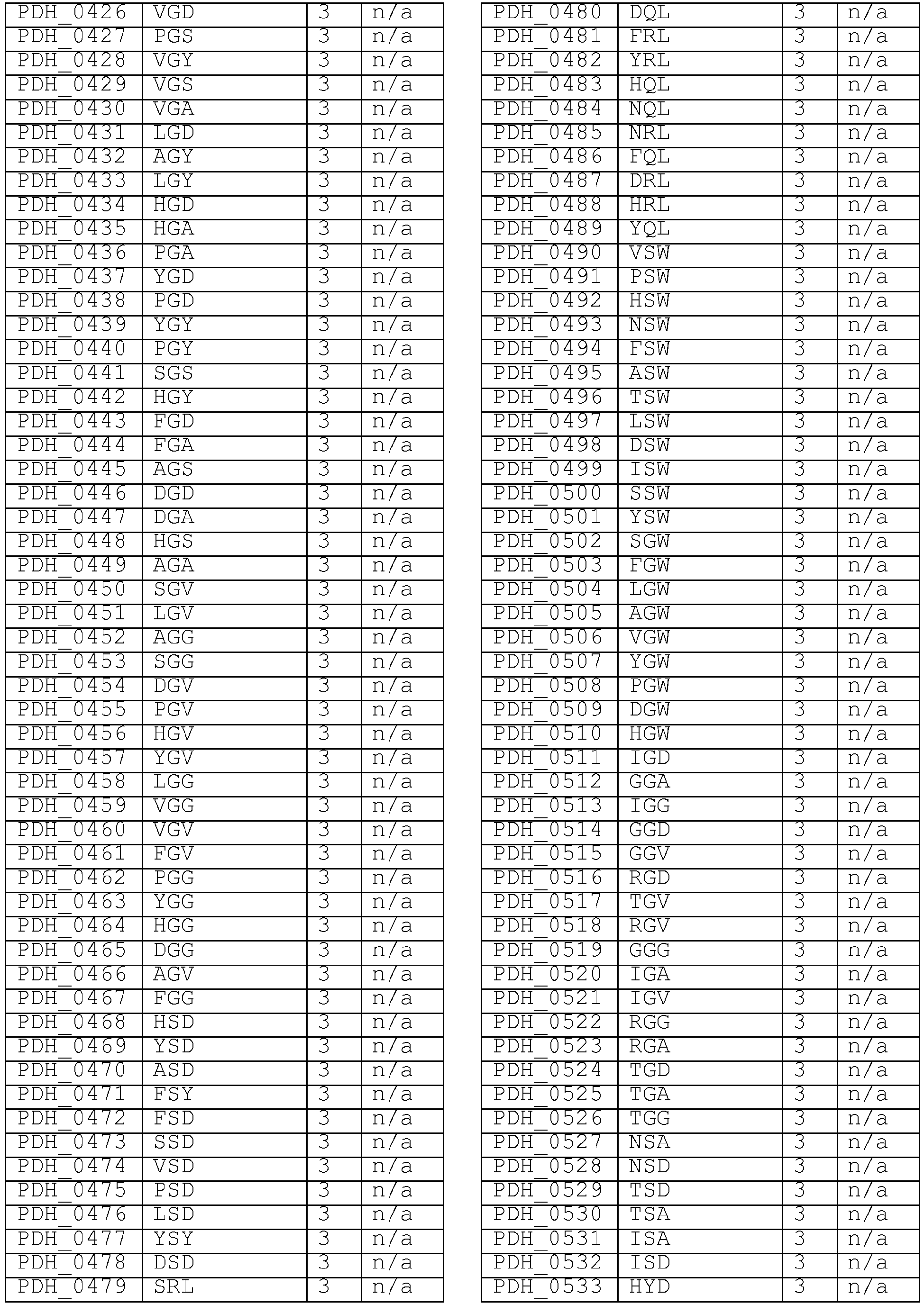

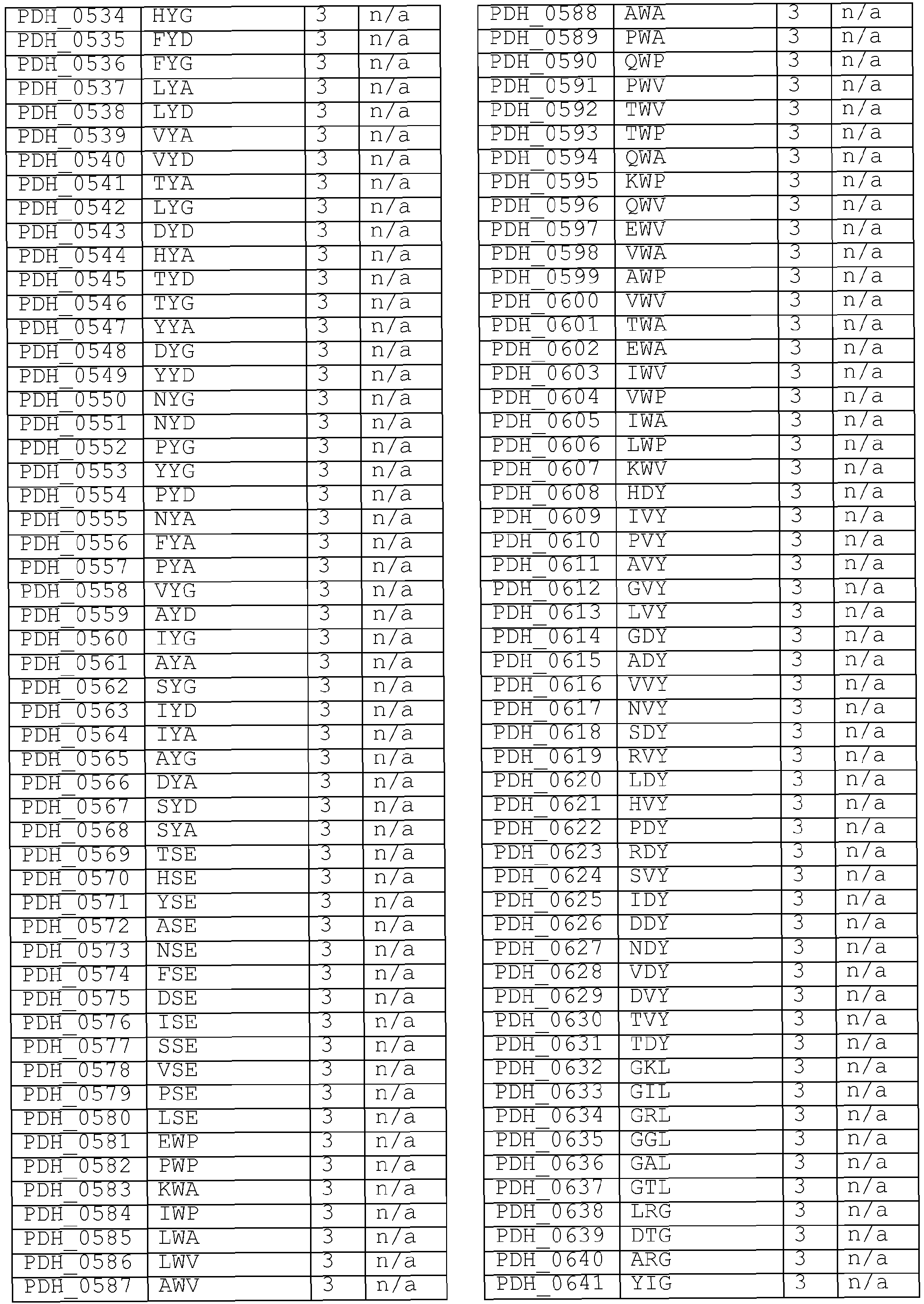

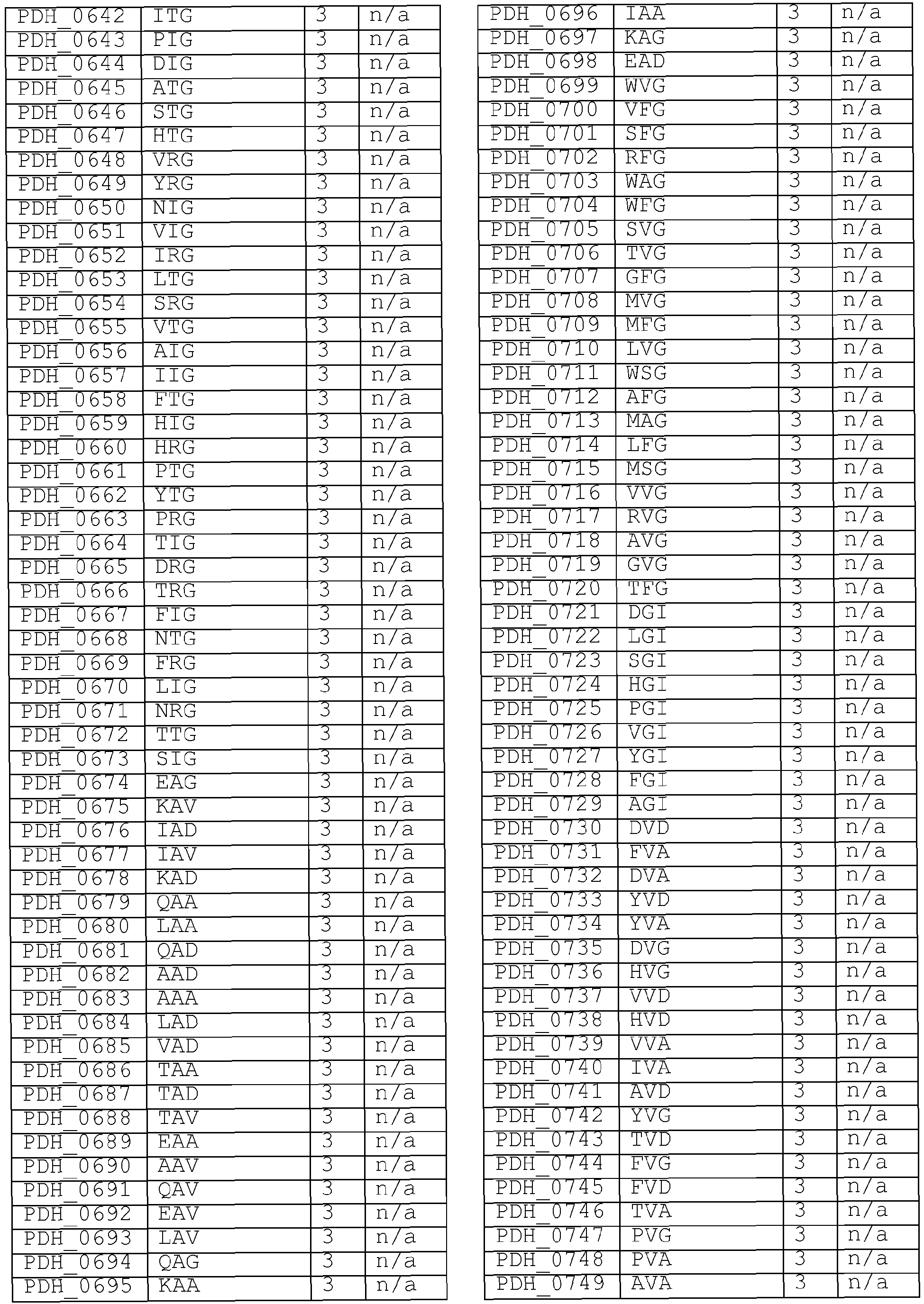

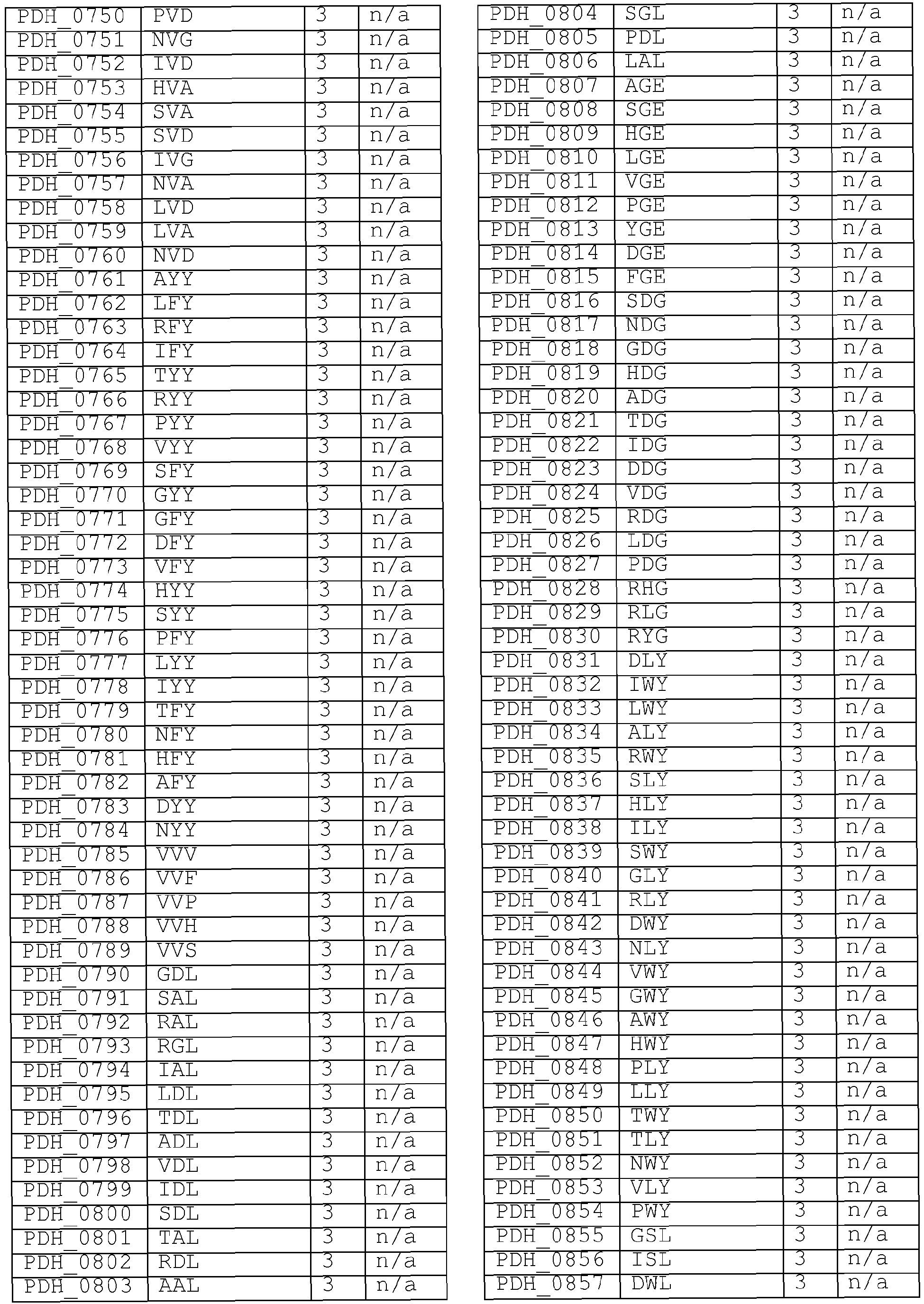

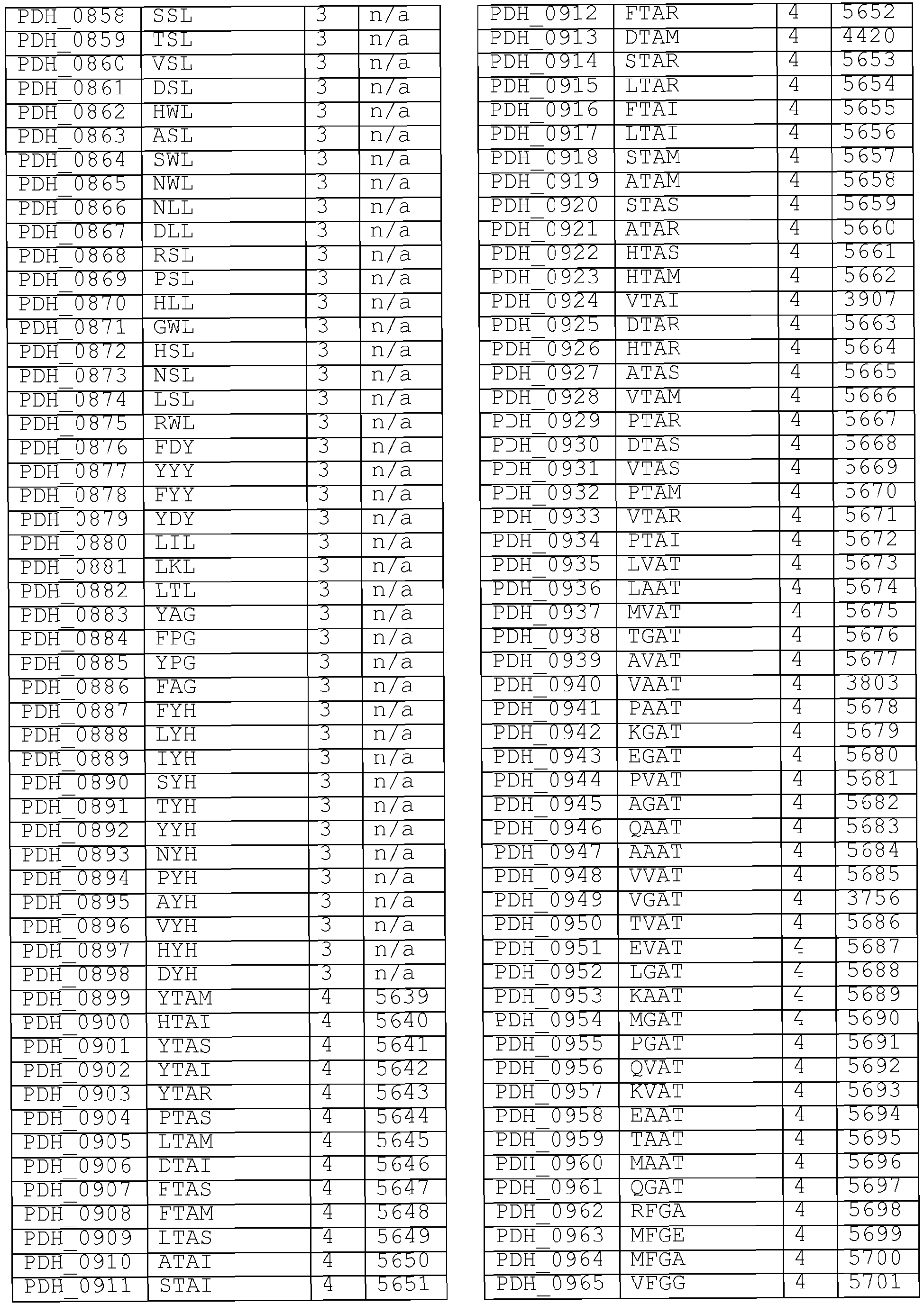

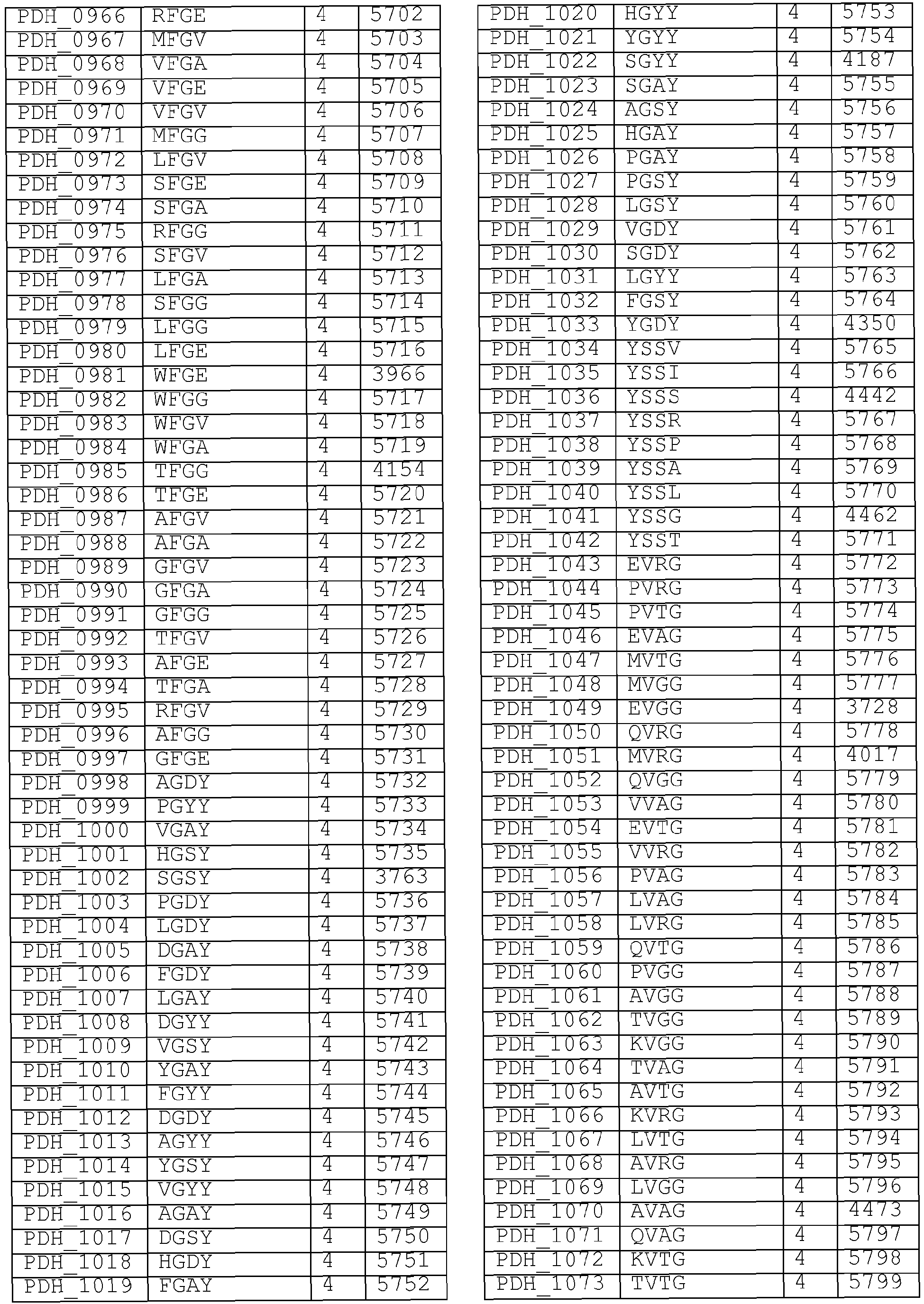

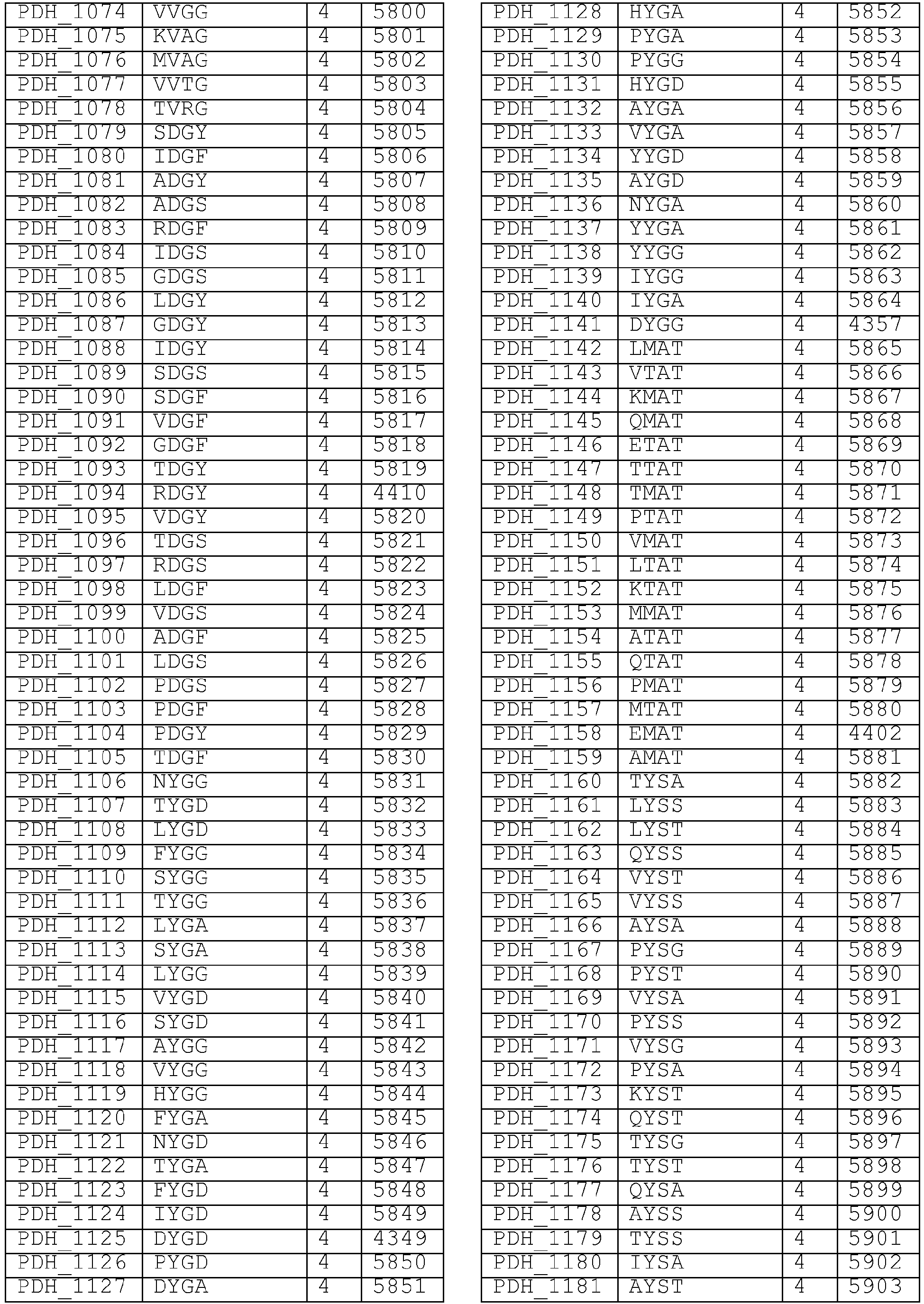

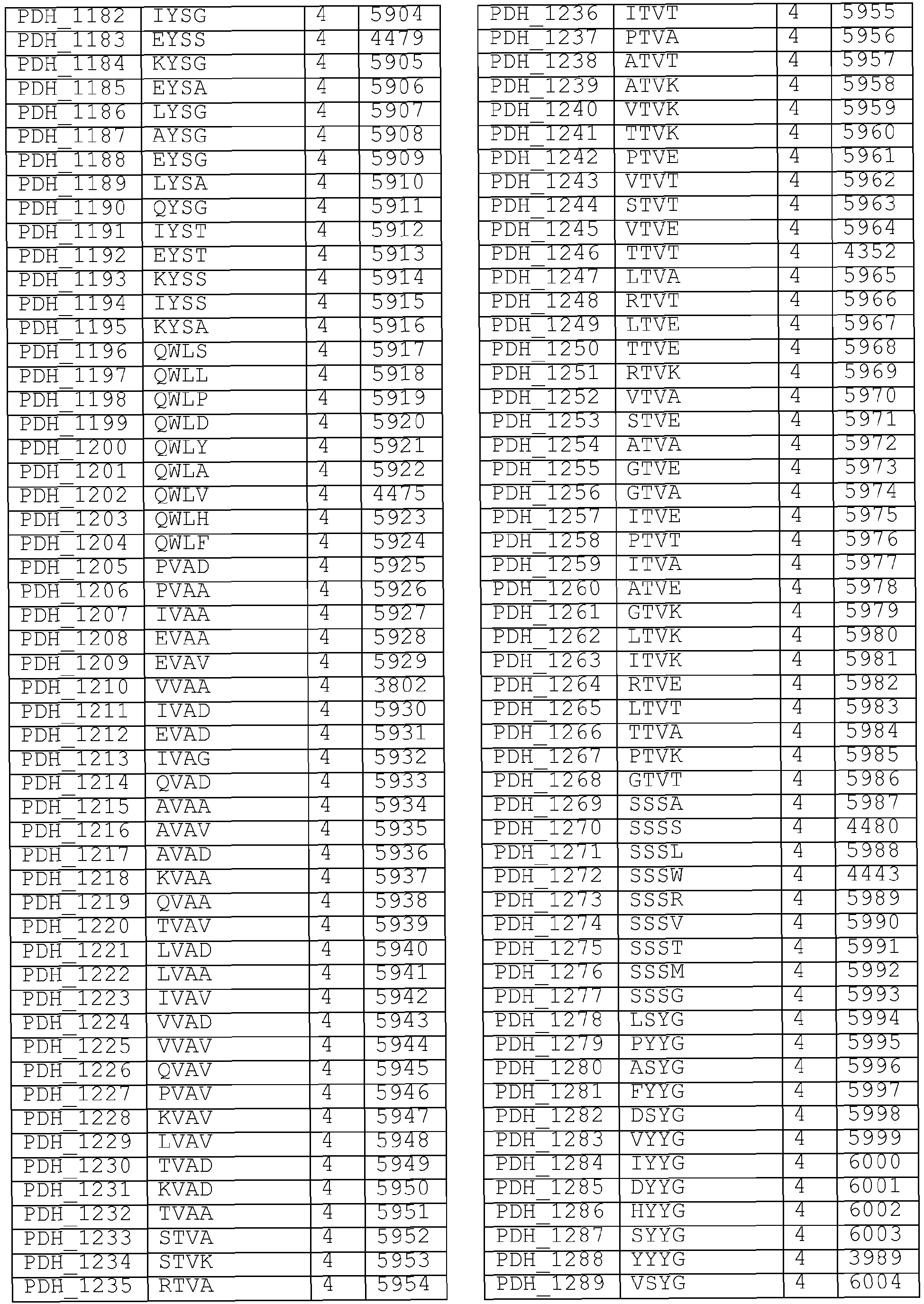

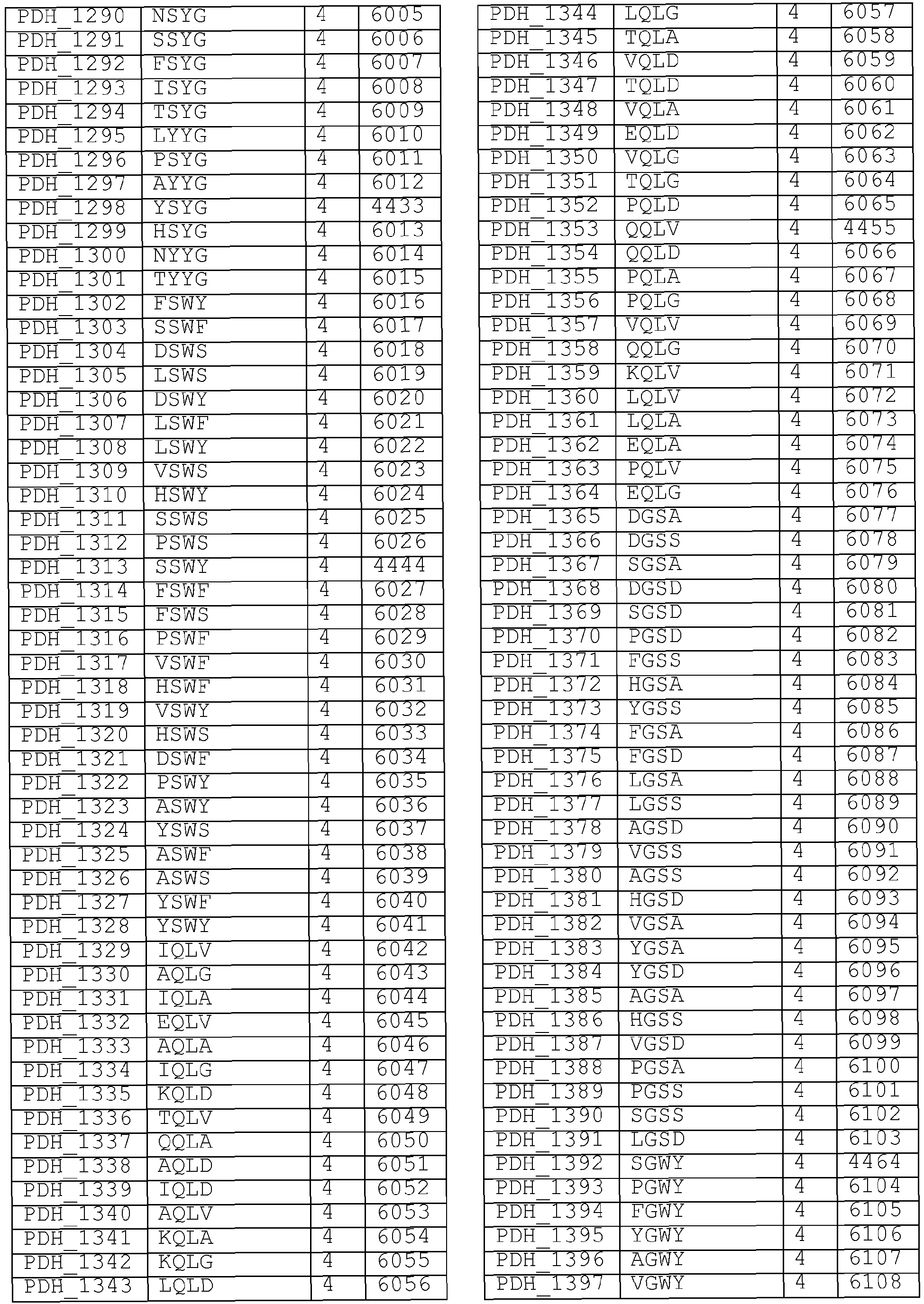

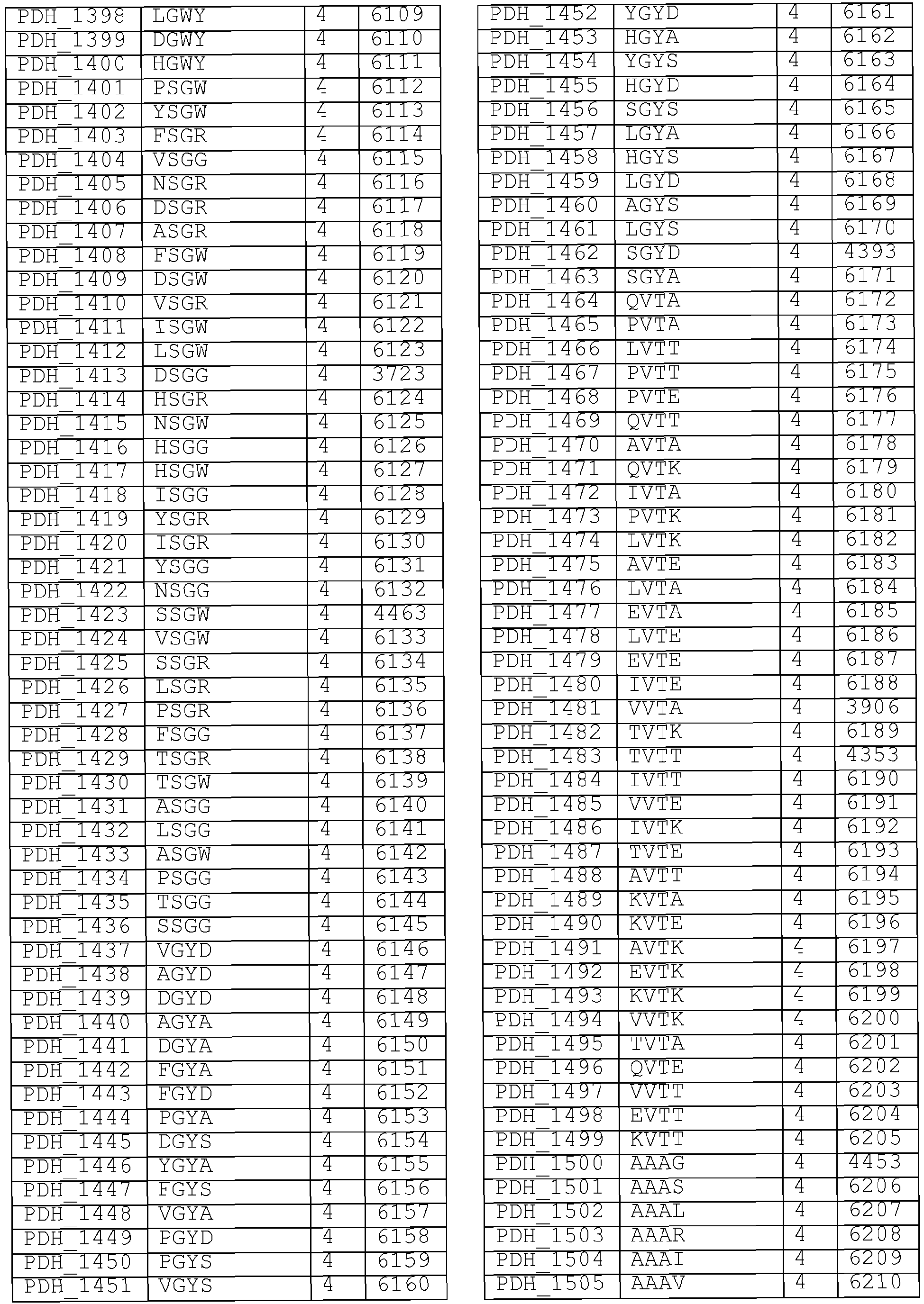

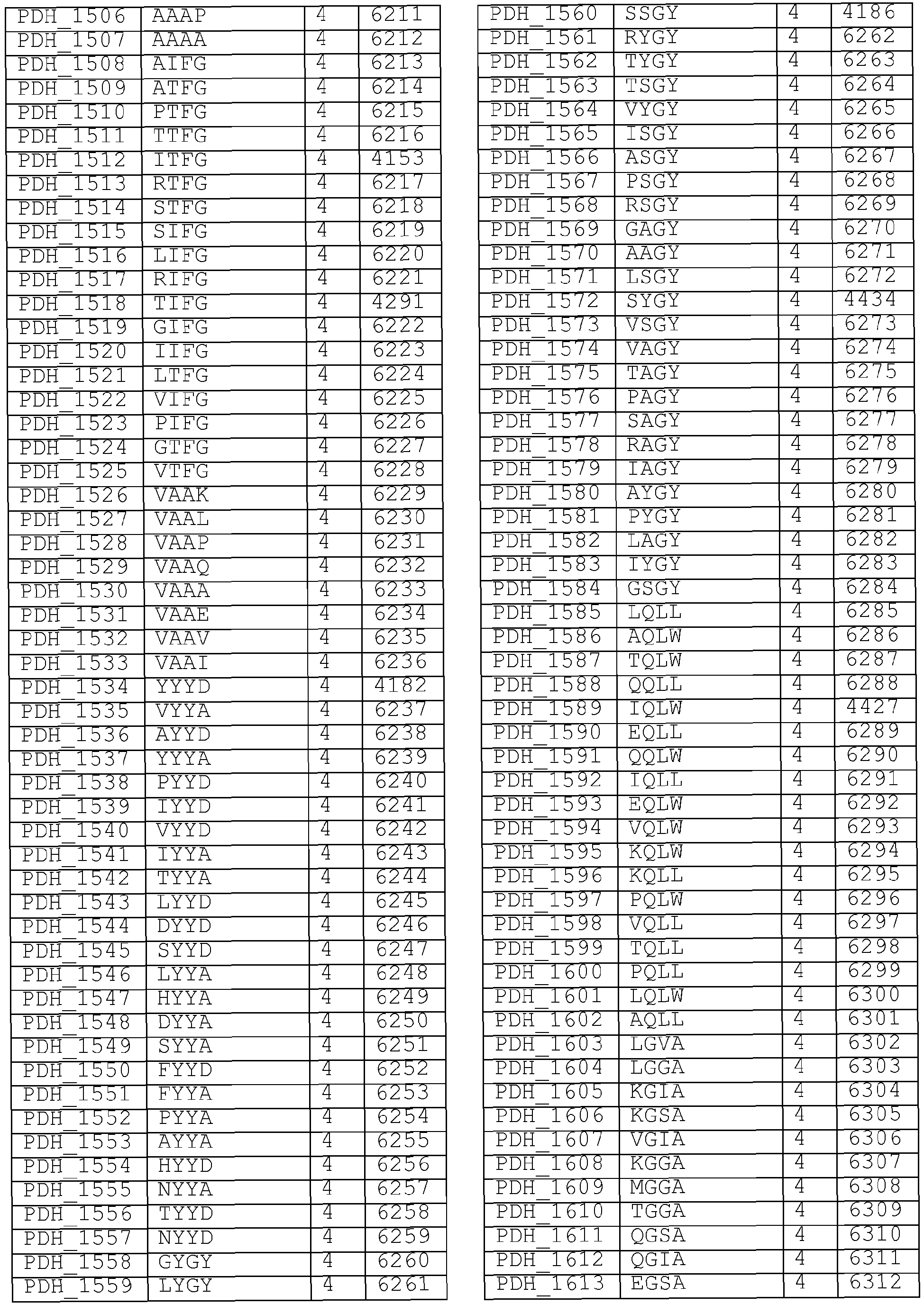

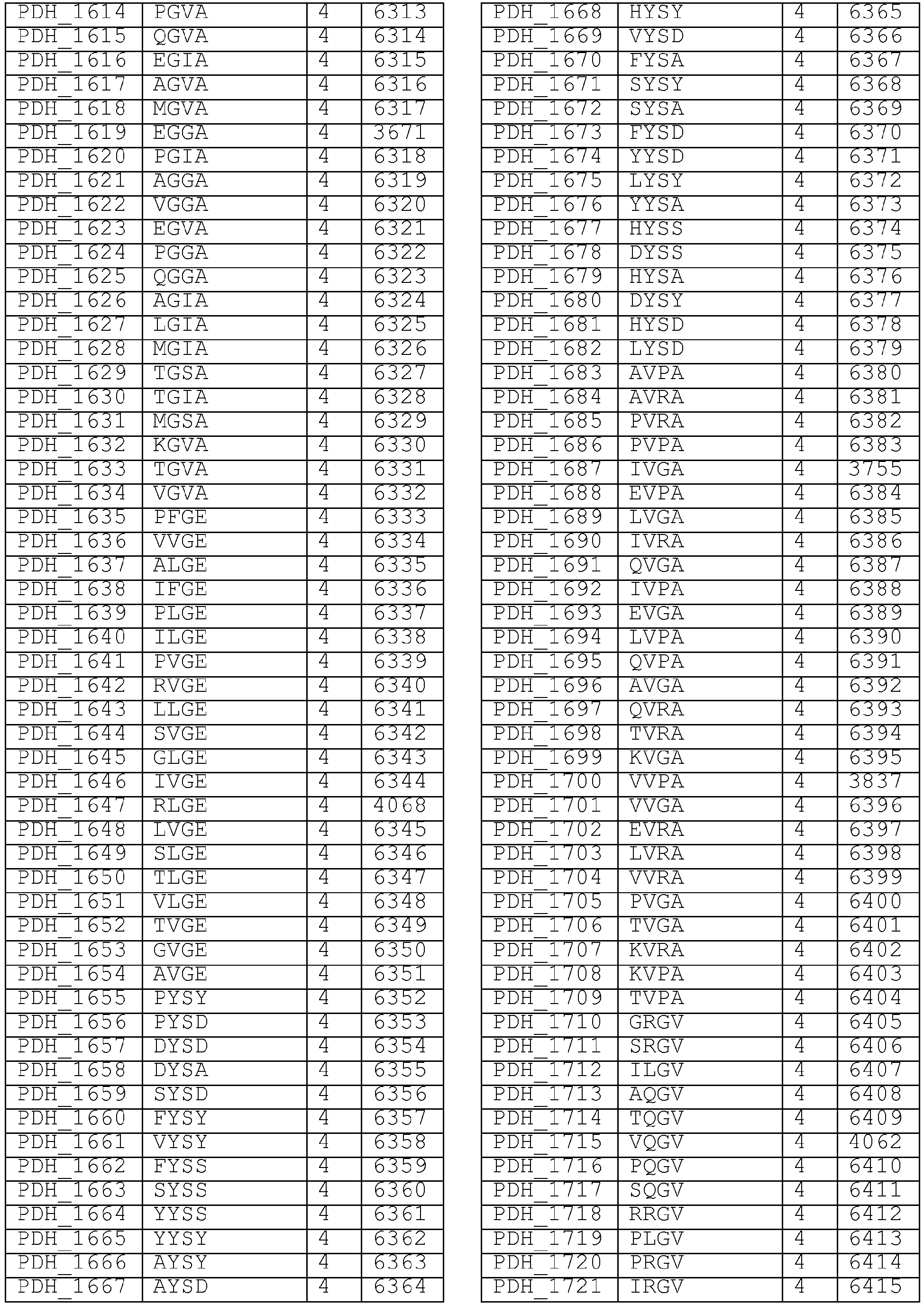

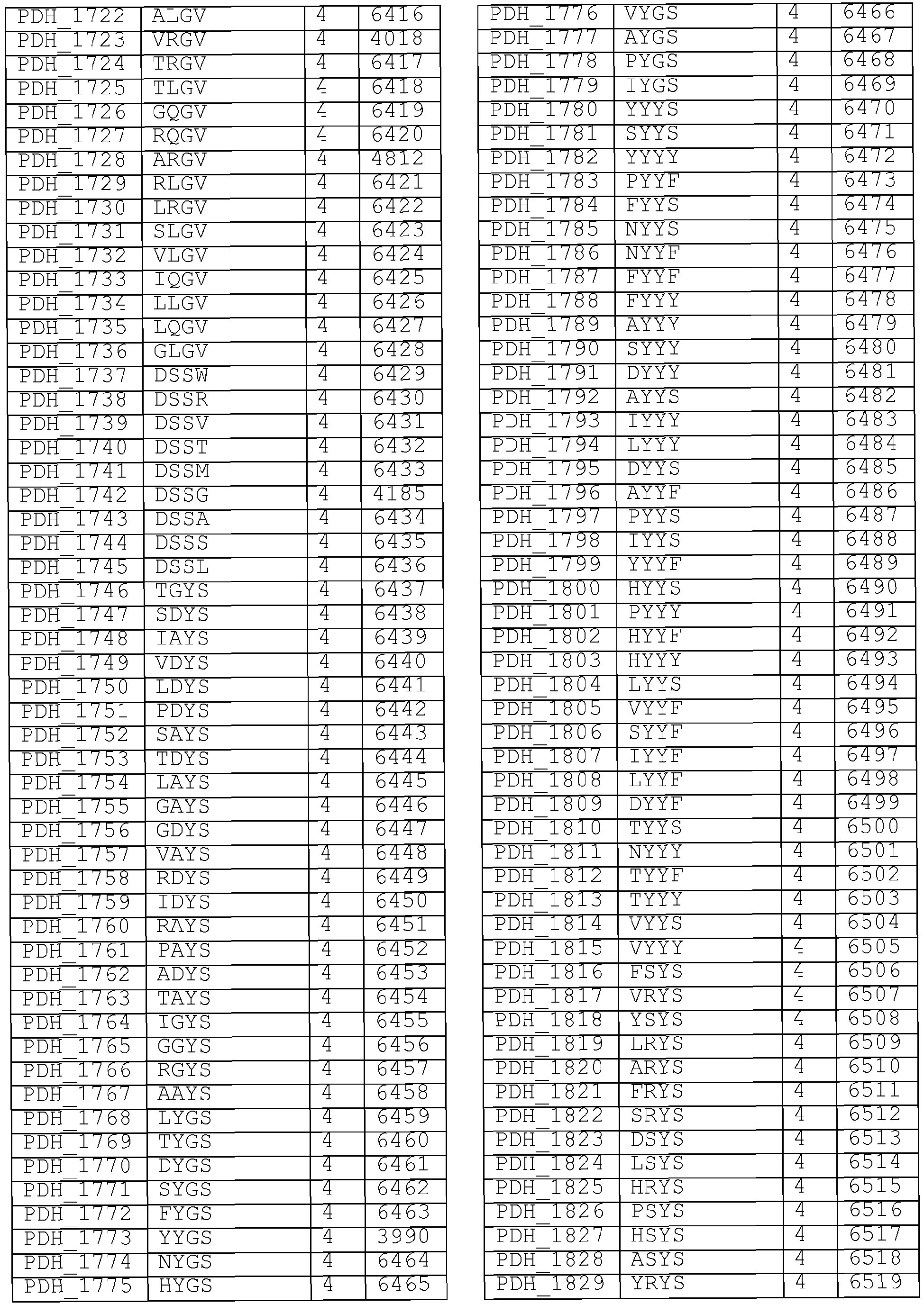

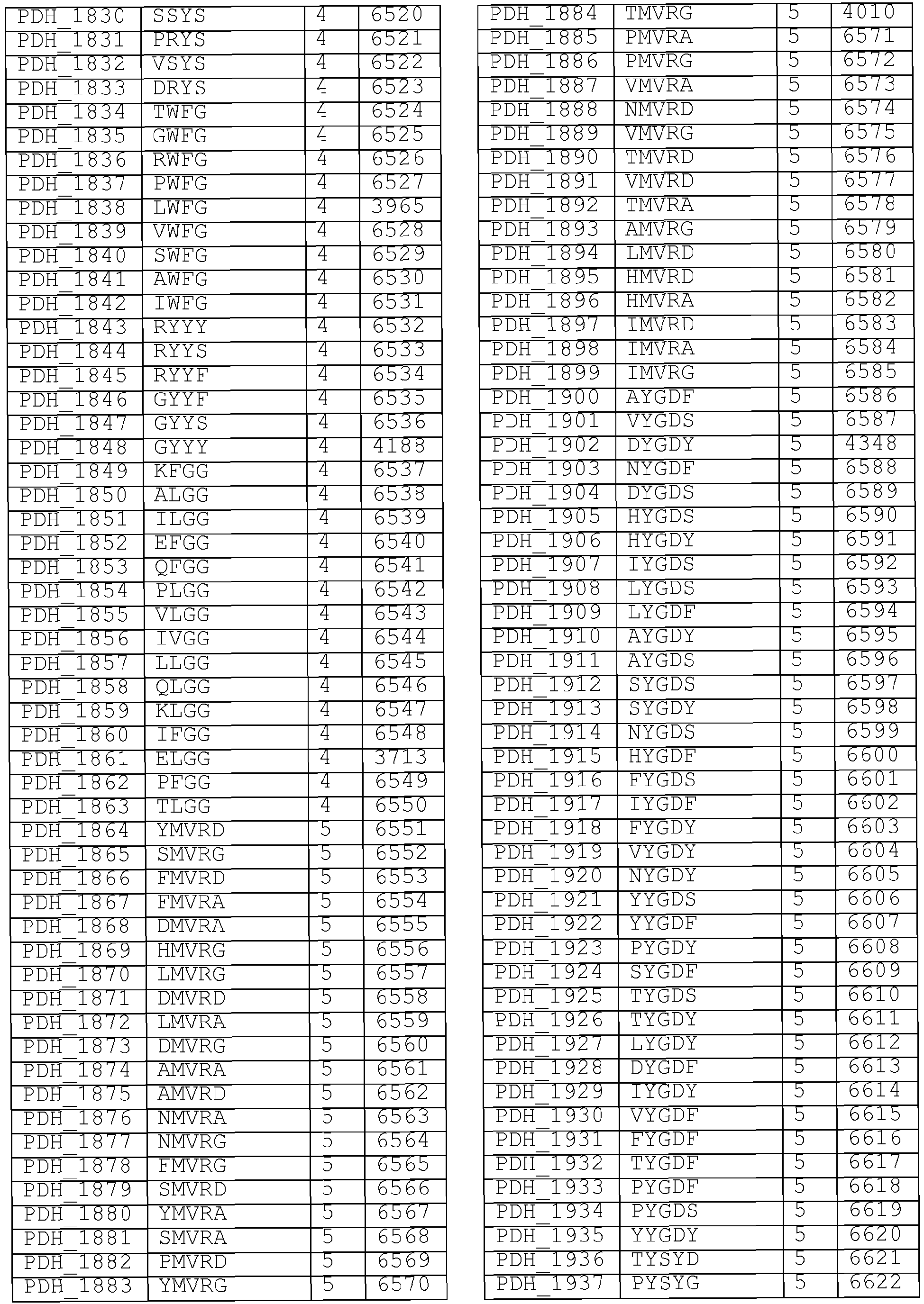

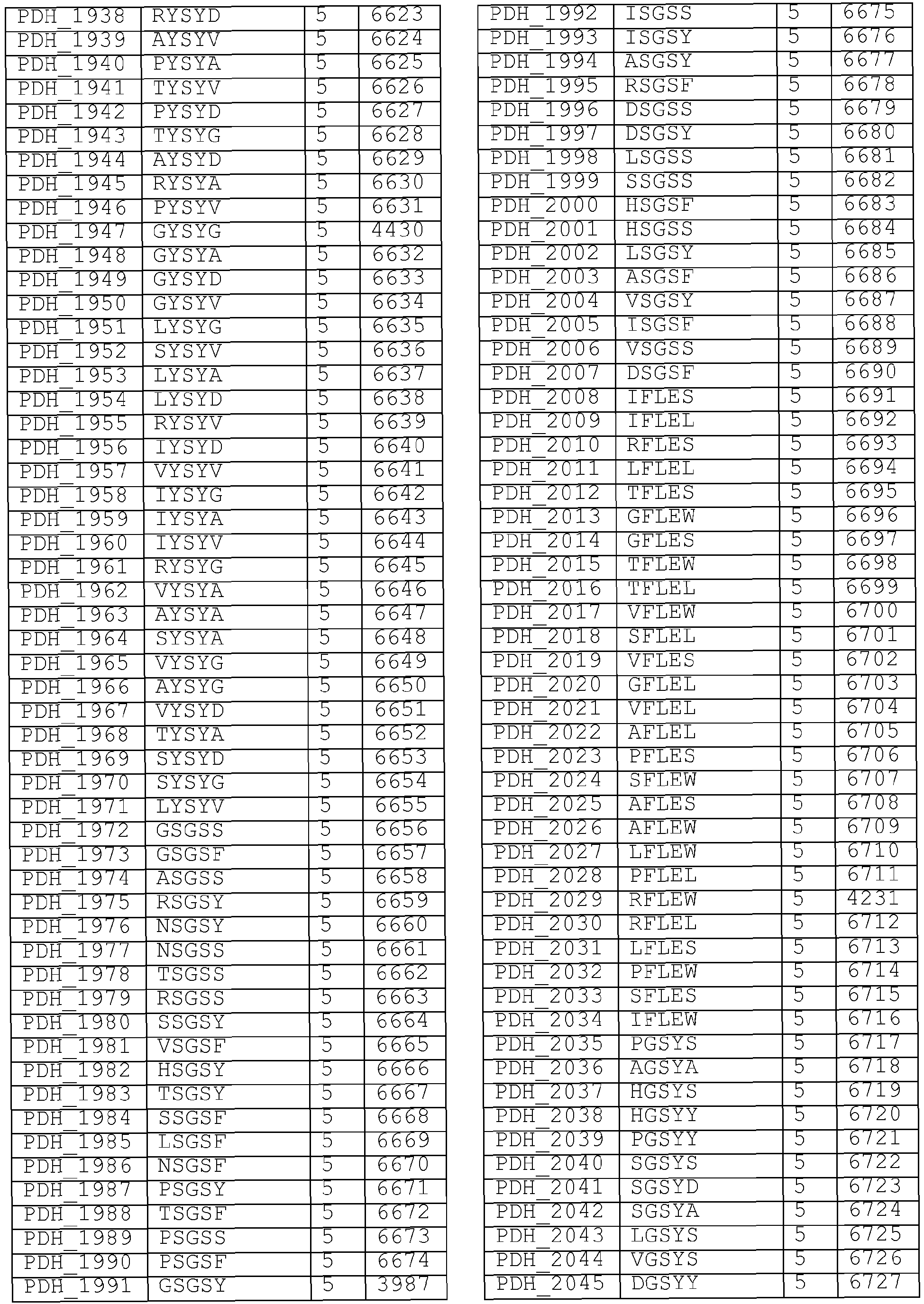

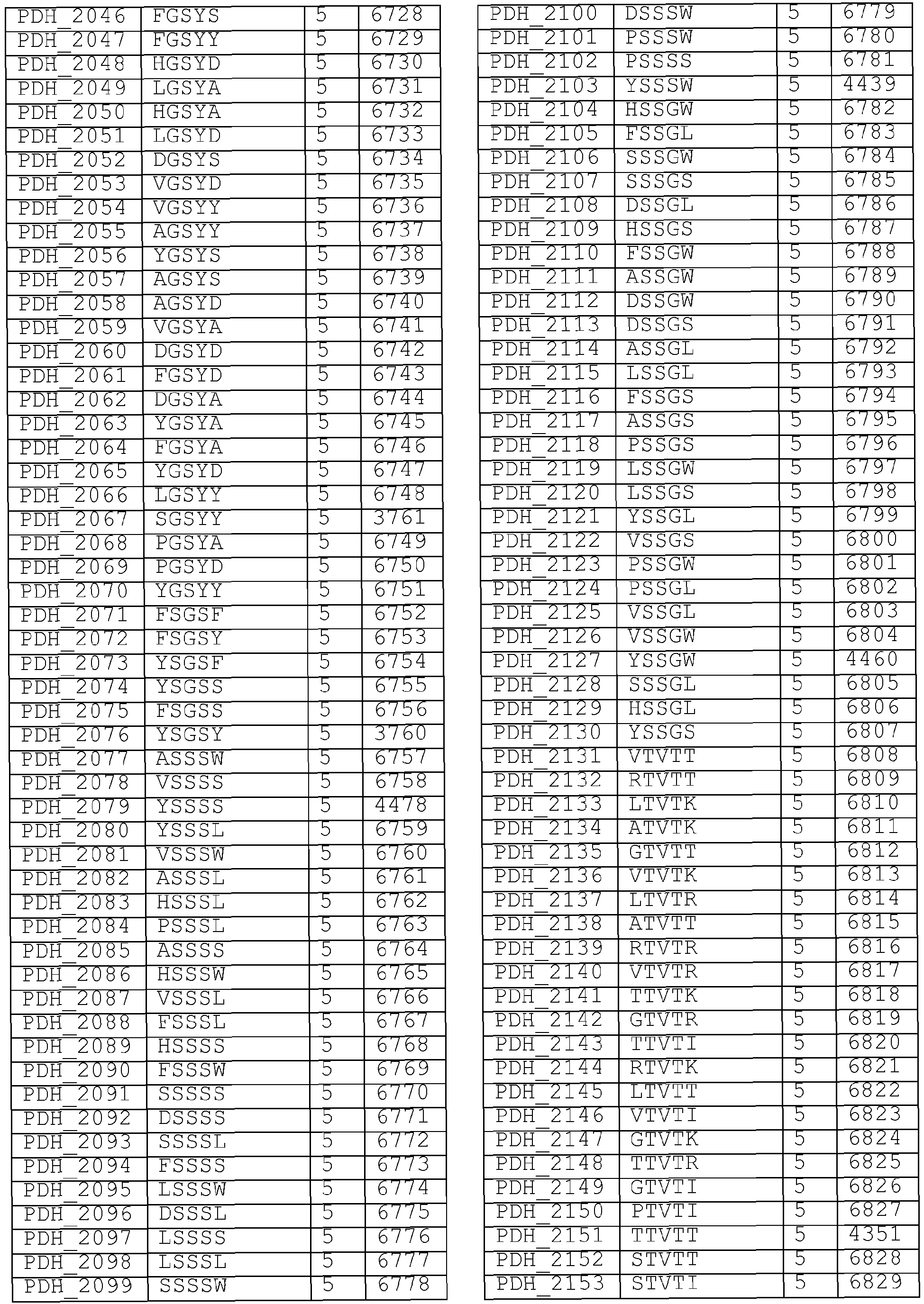

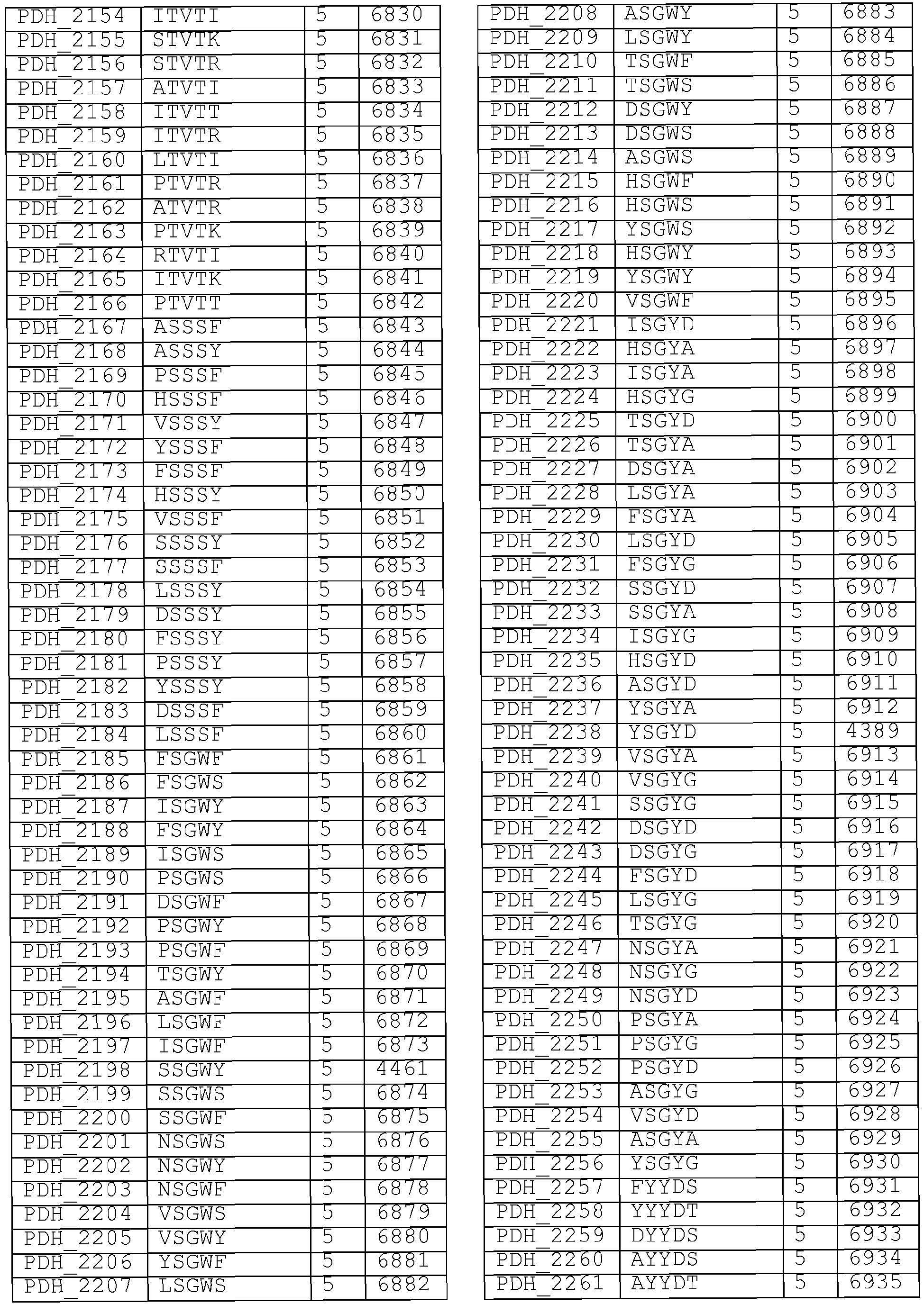

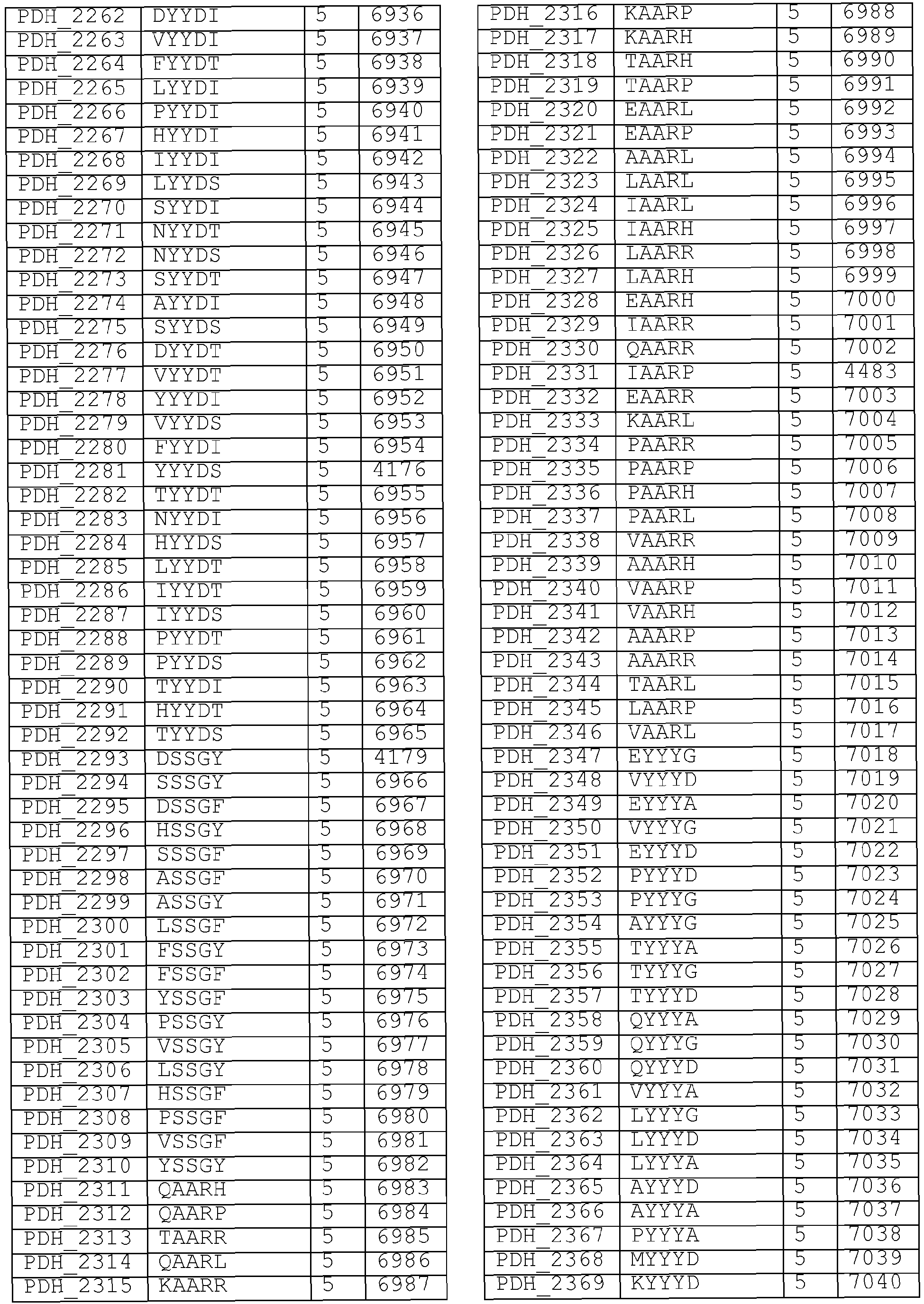

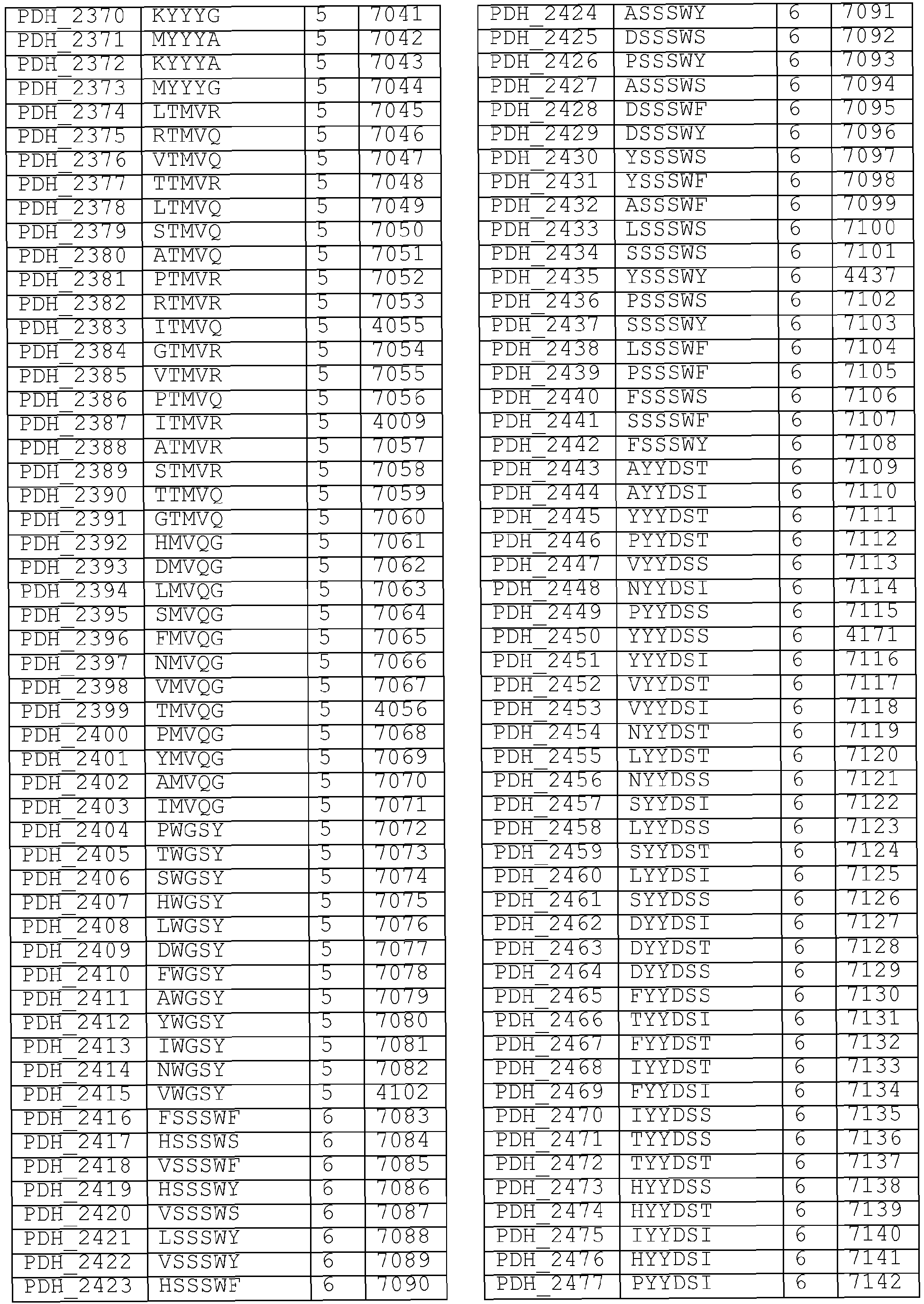

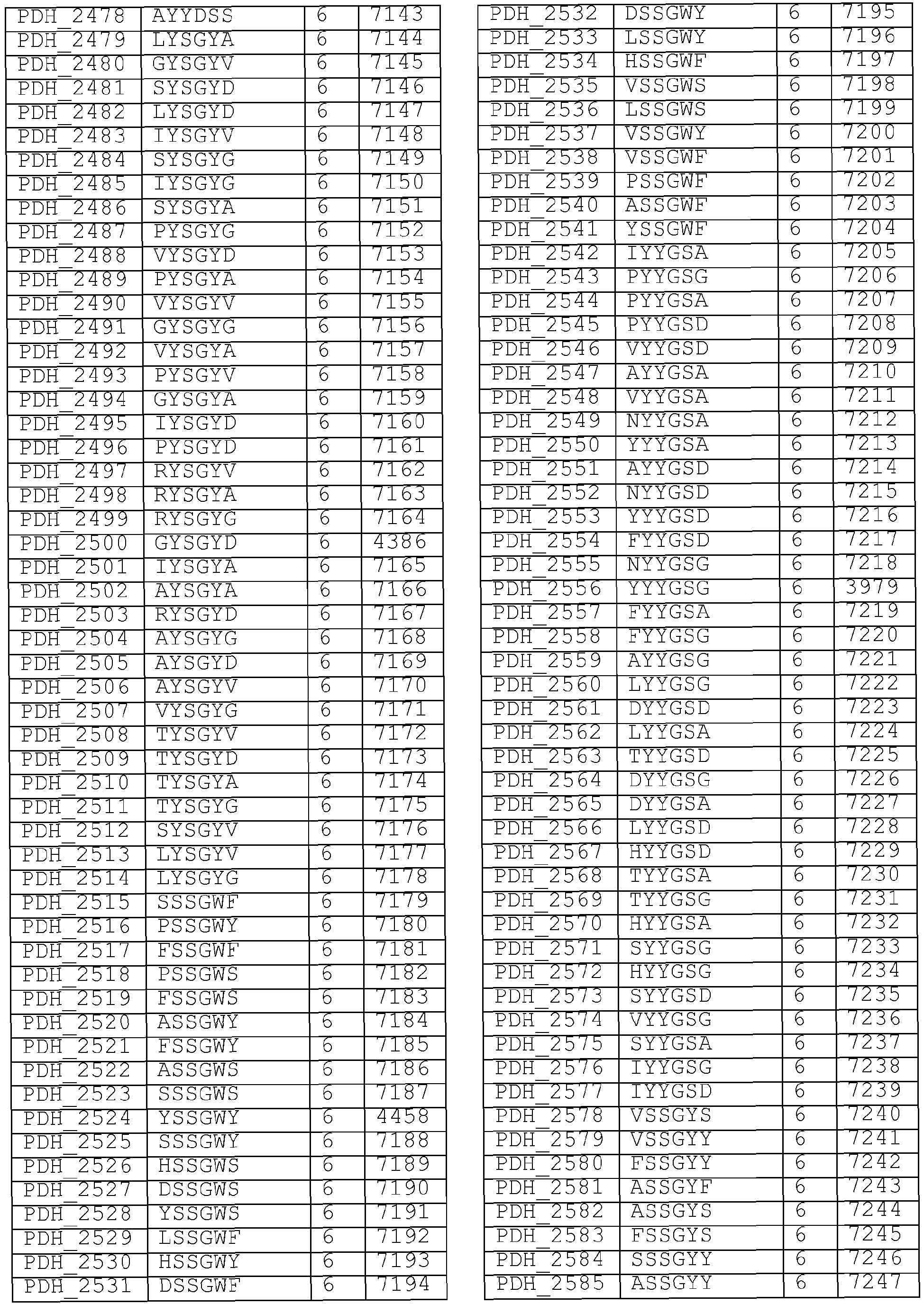

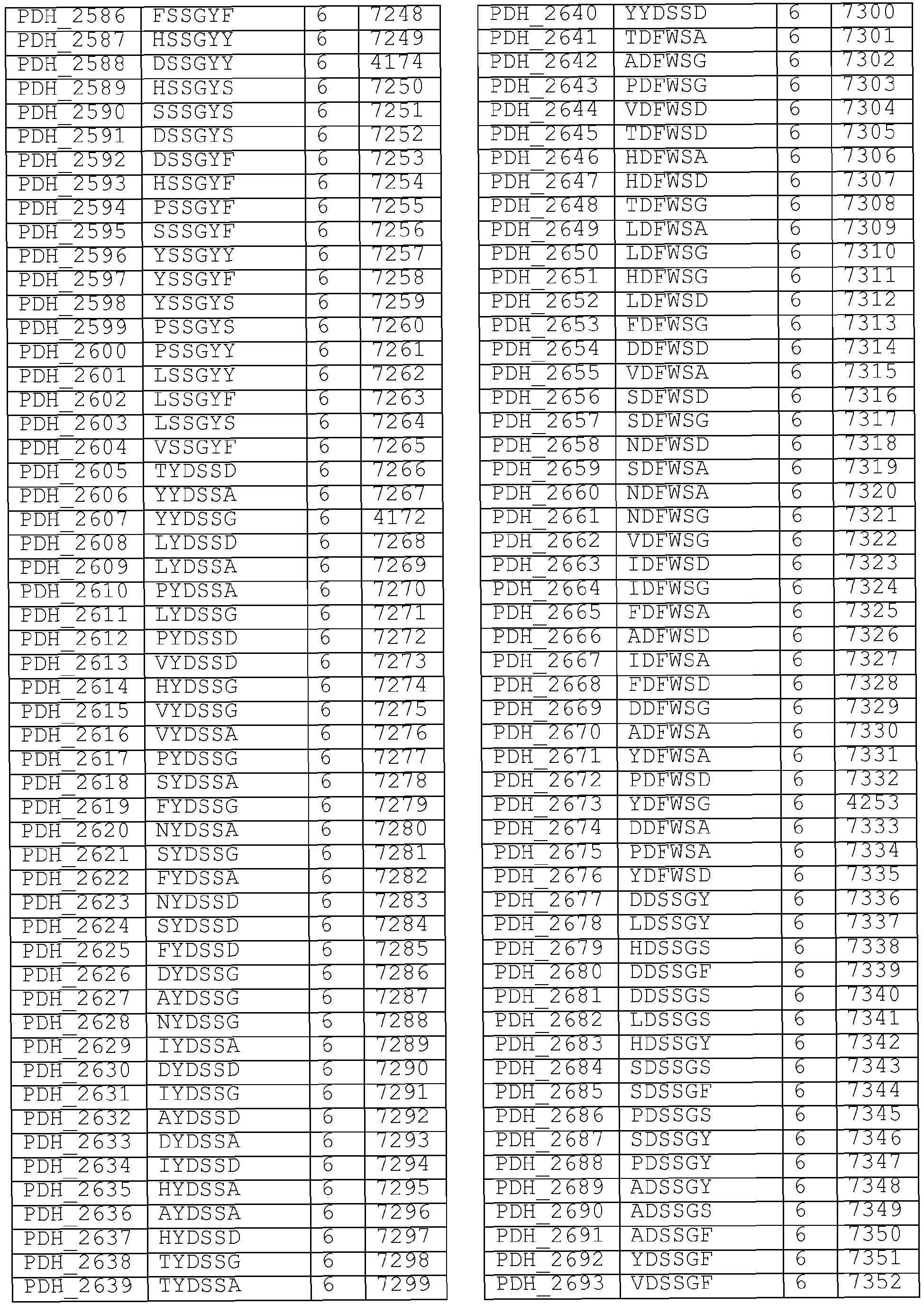

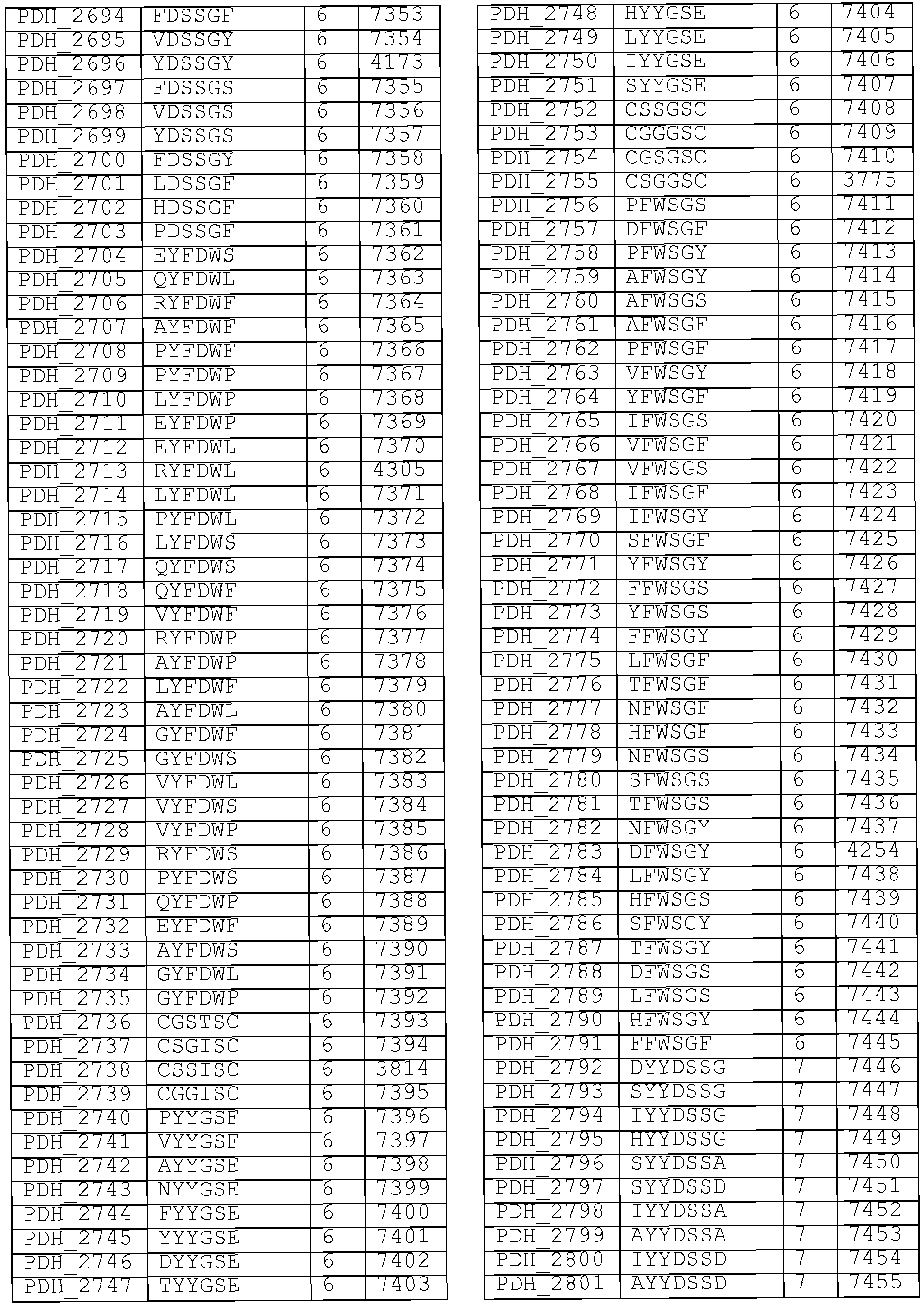

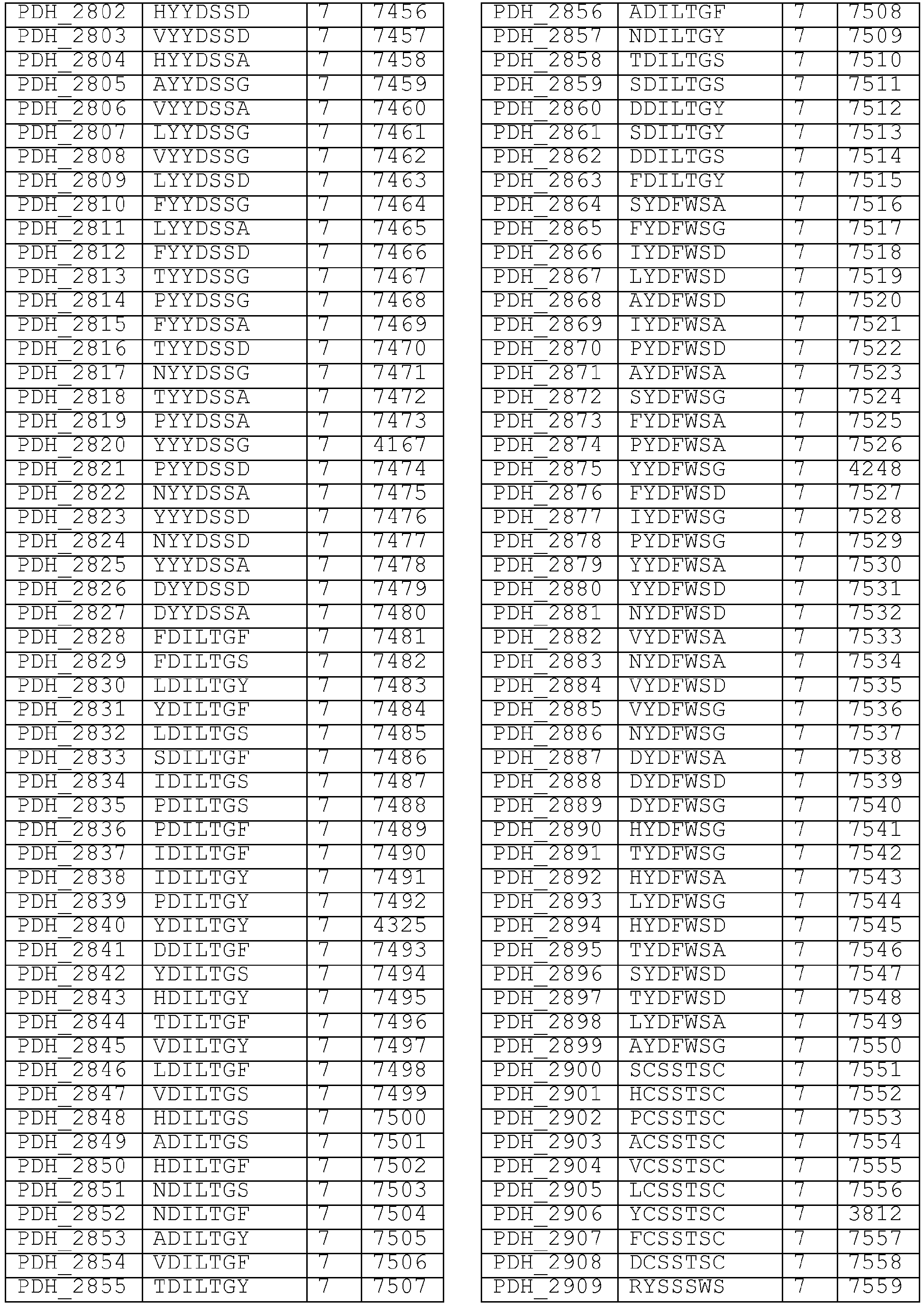

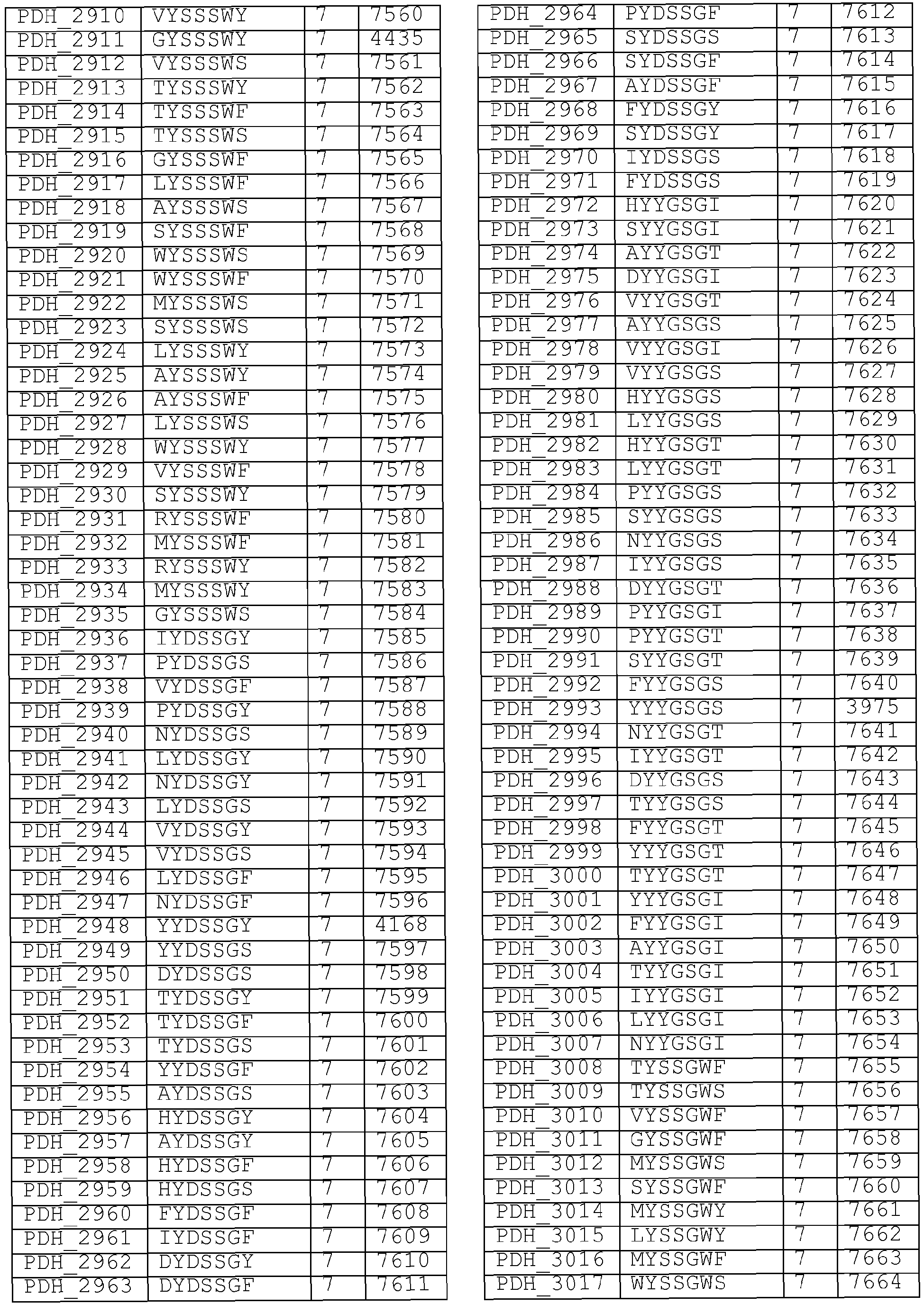

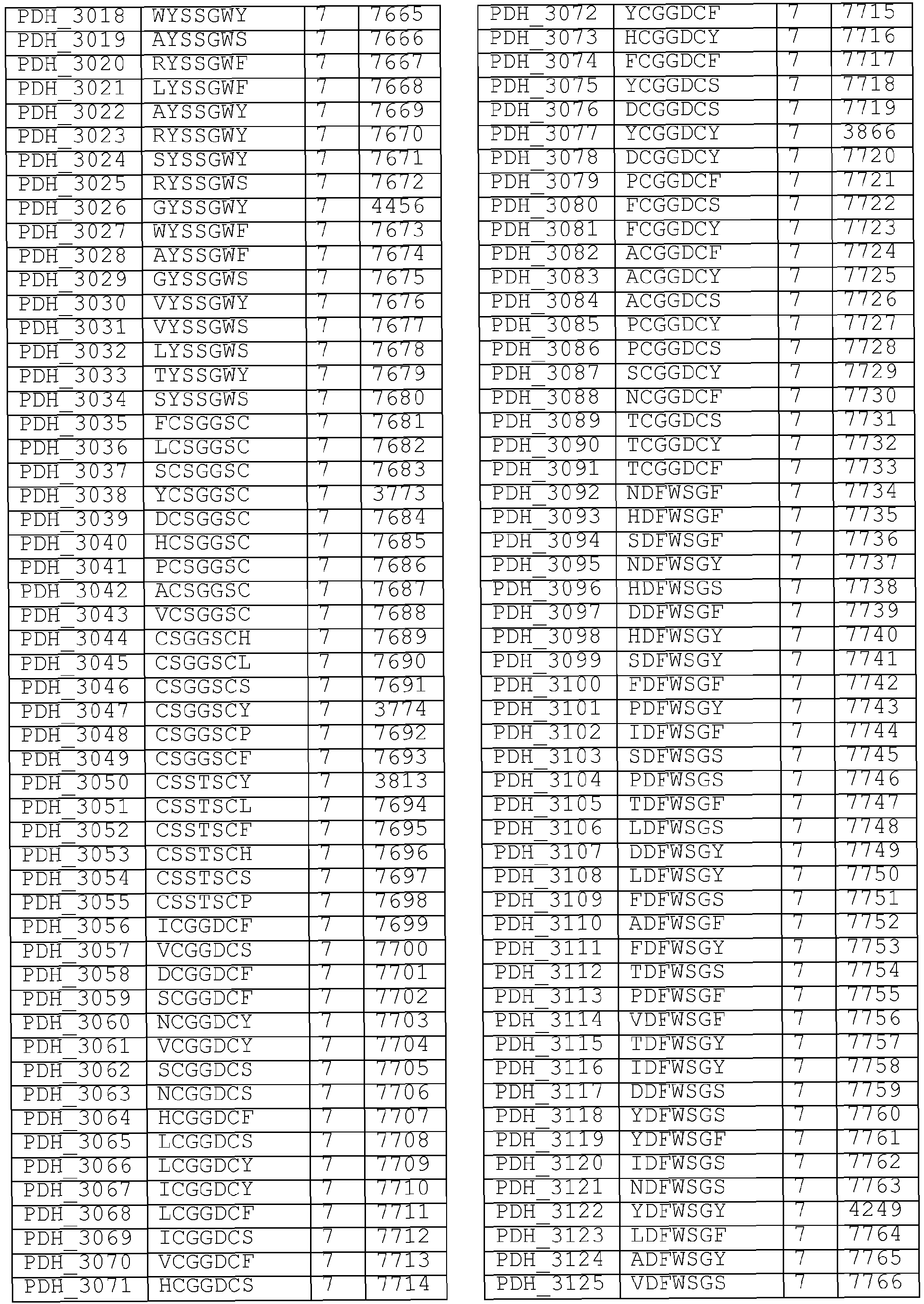

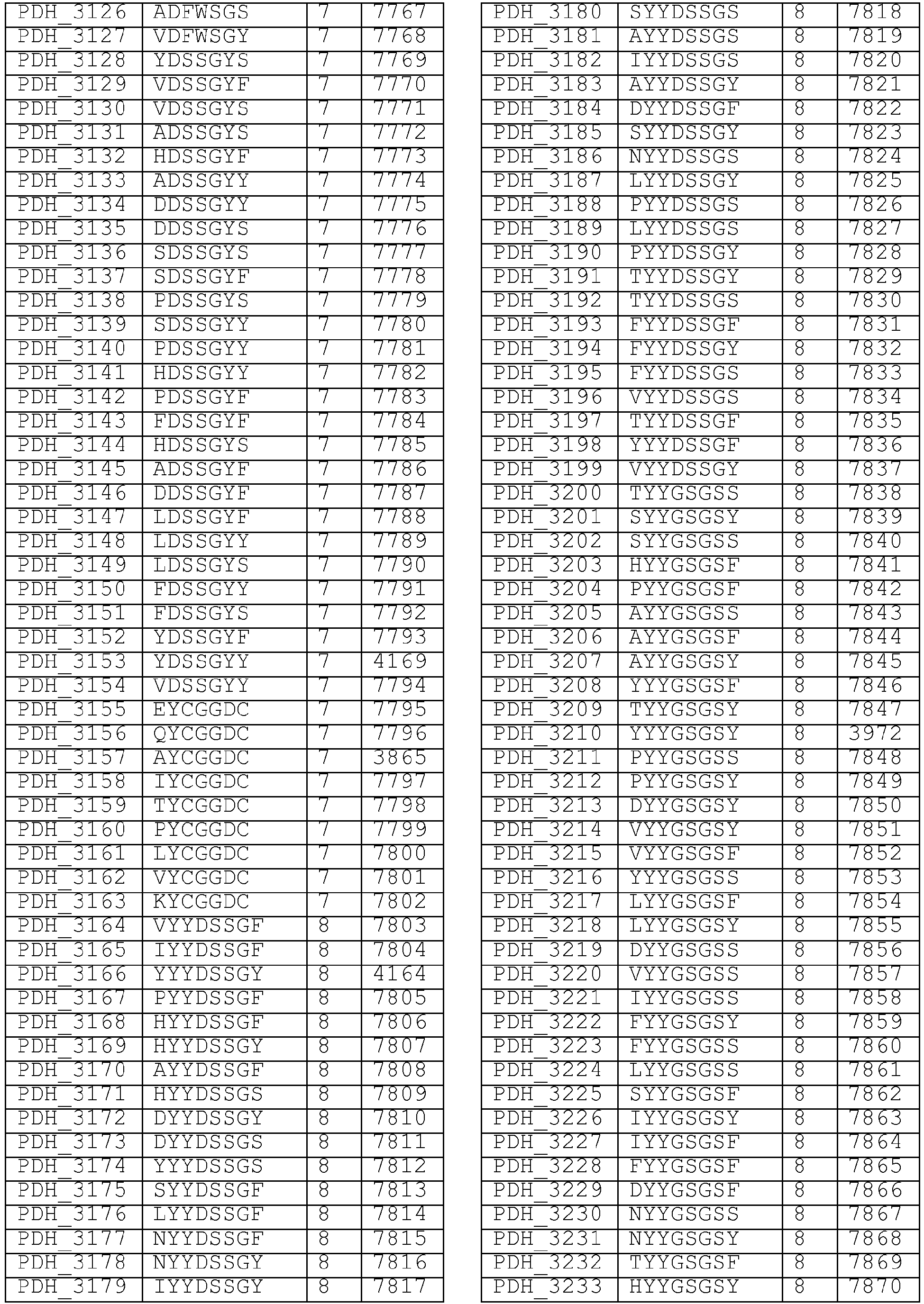

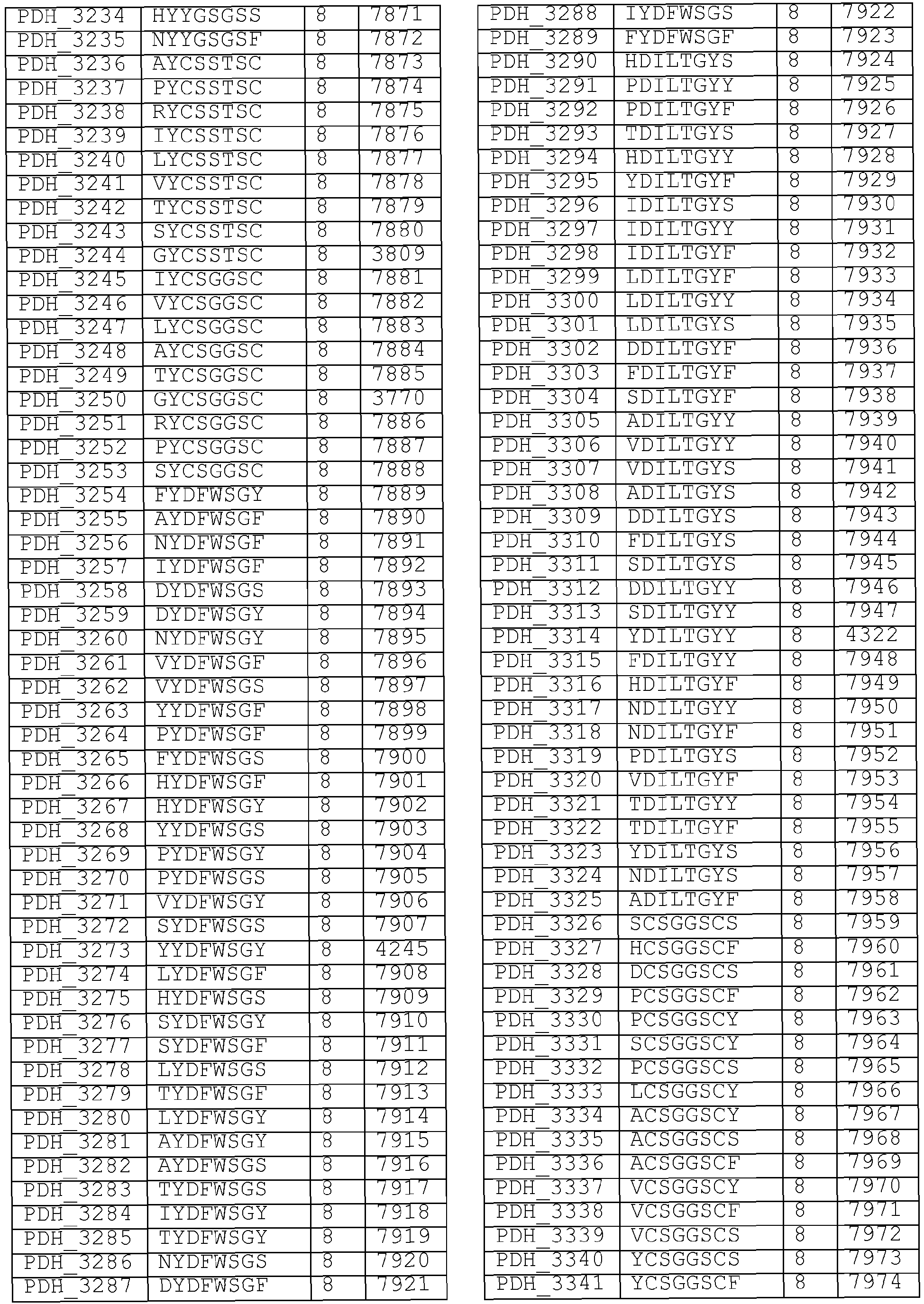

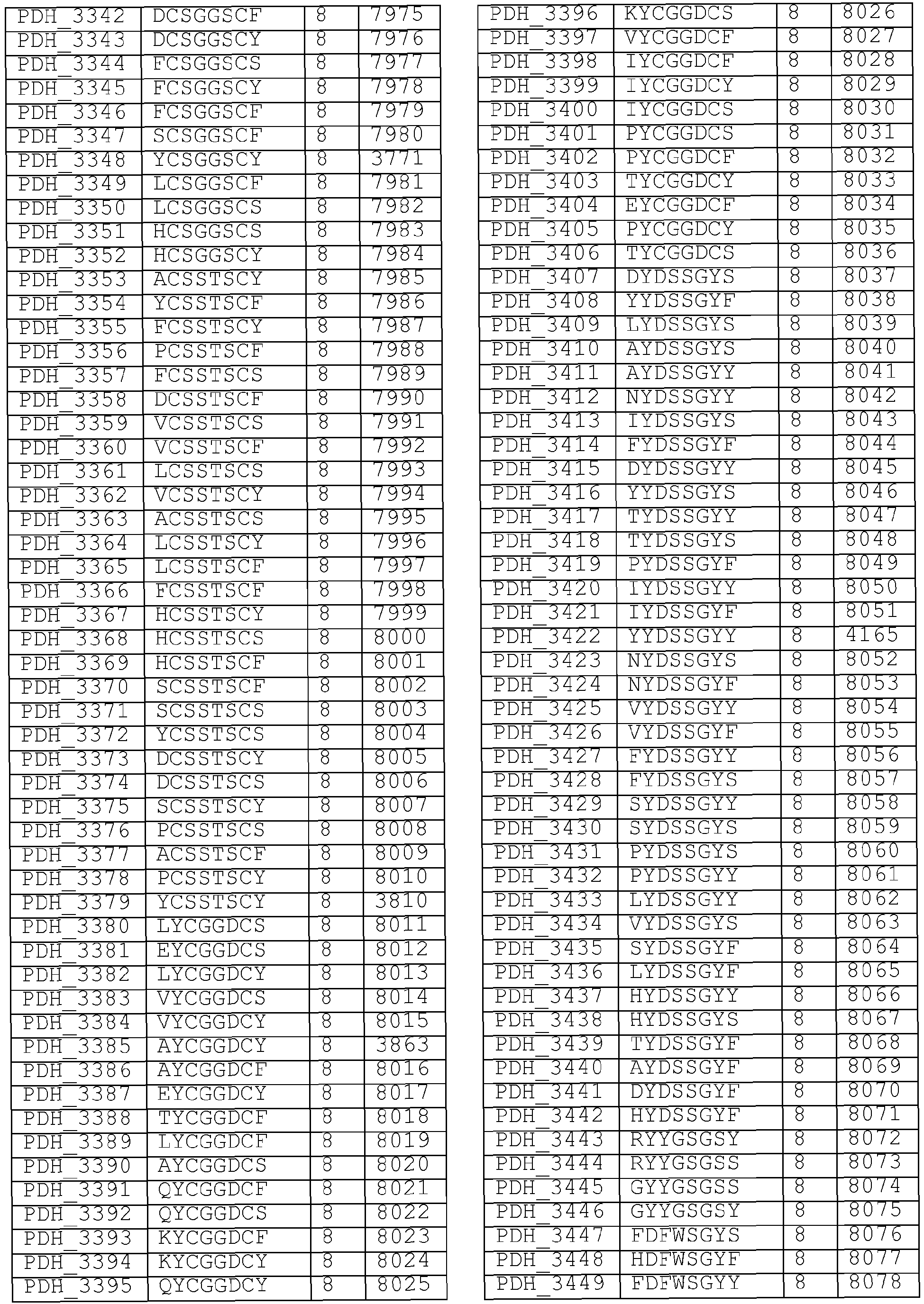

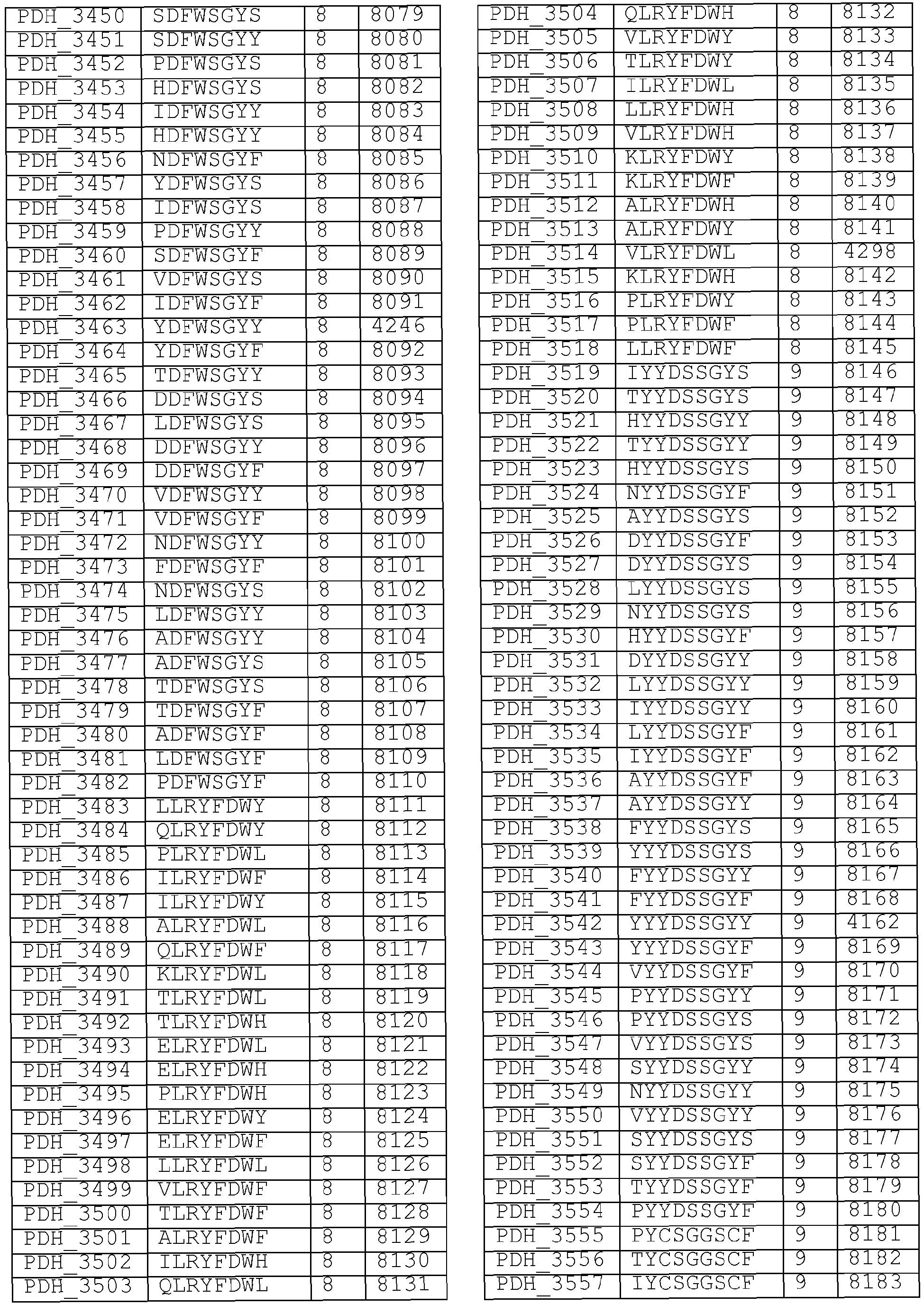

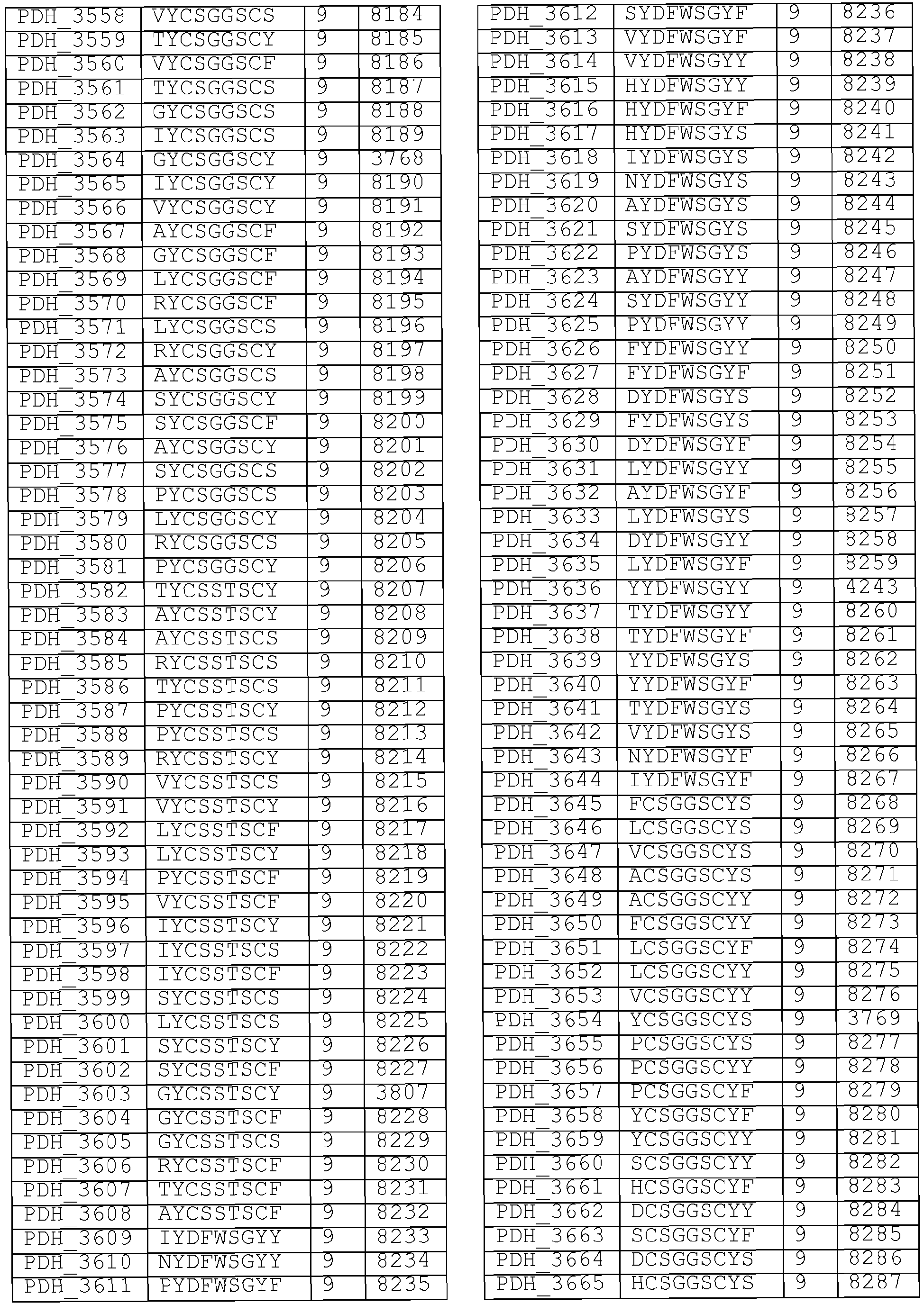

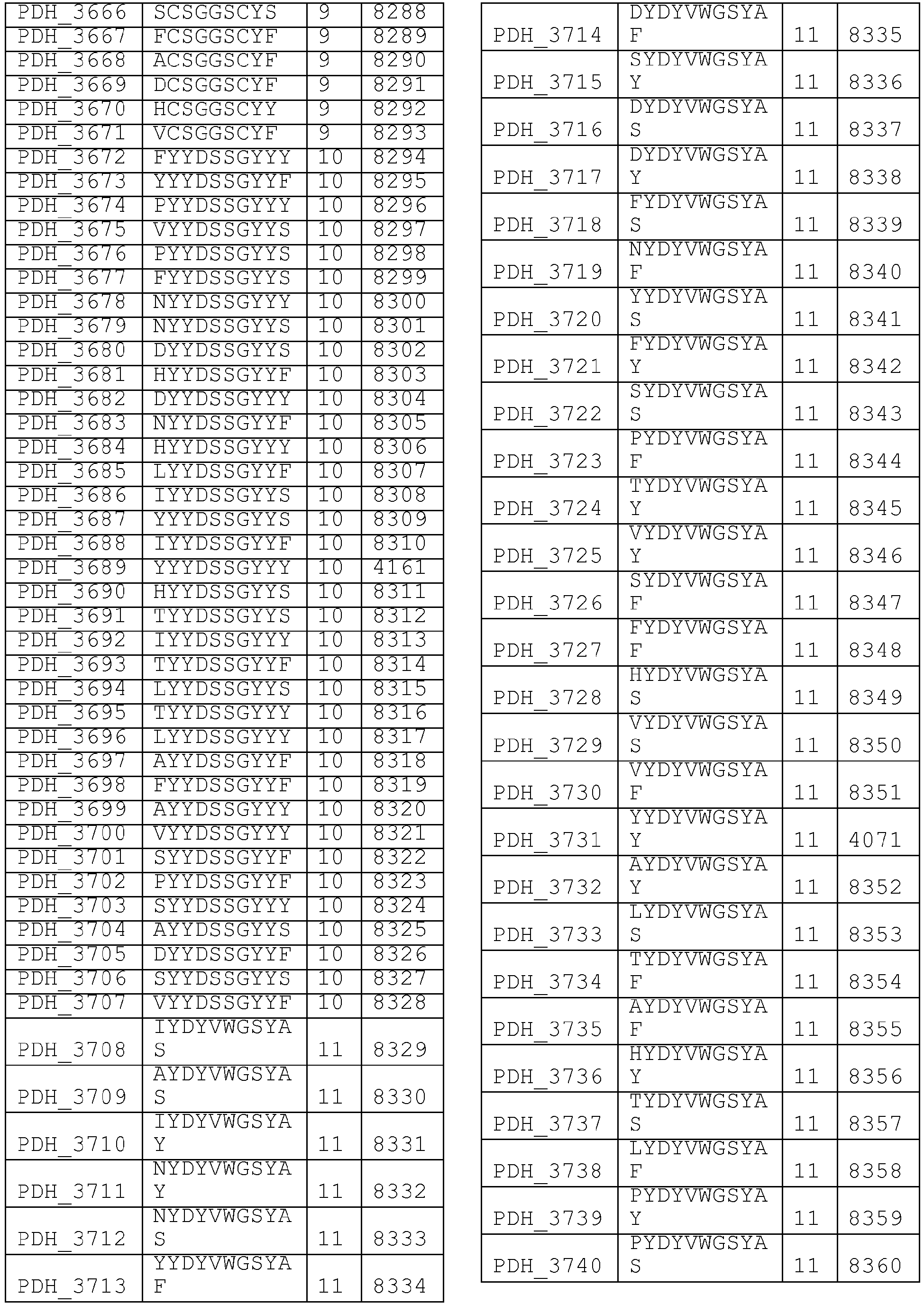

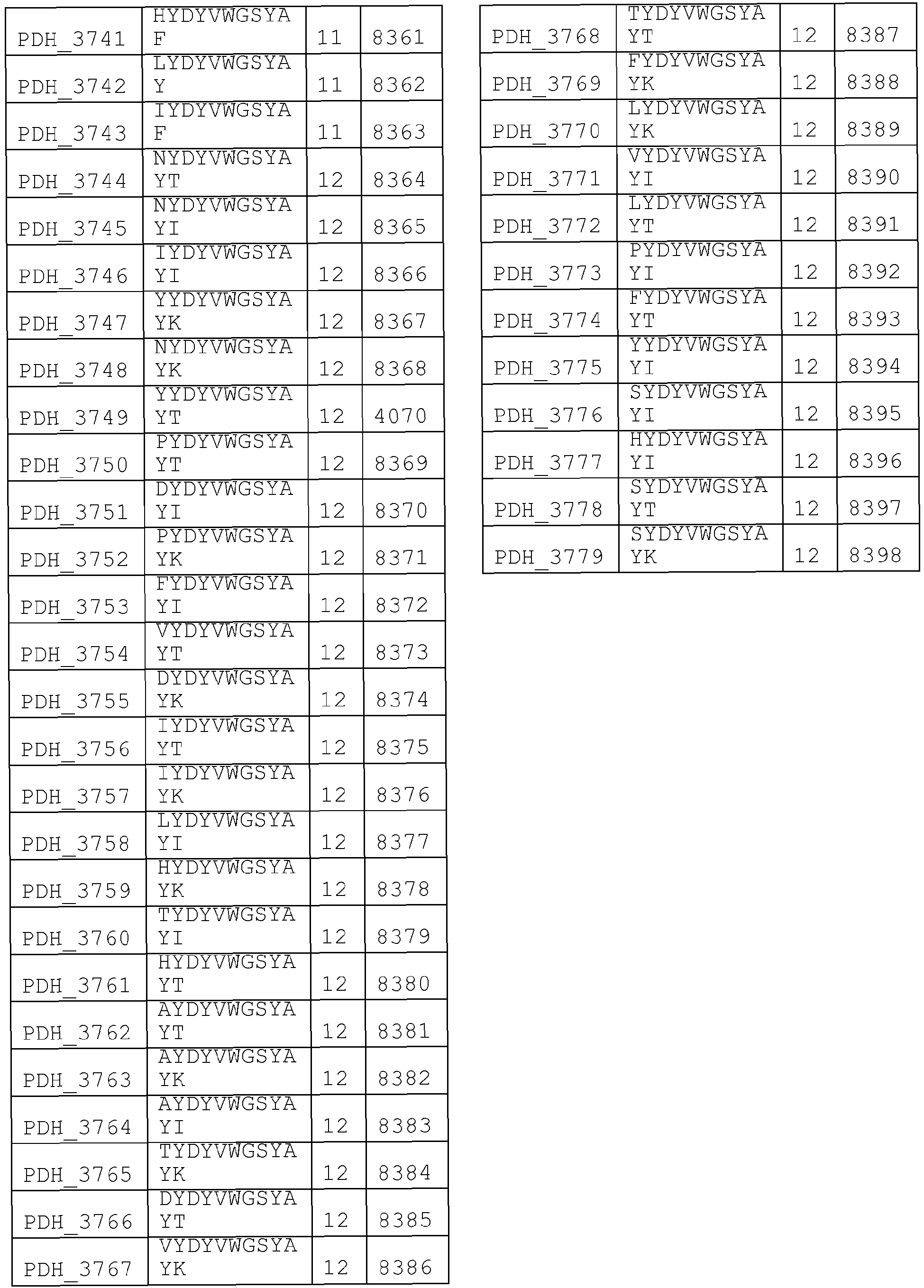

**Table 29. Theoretical segment pool of oligonucleotide sequences encoding N2 segments of Example 14.**

| **Name** | **Degenerate Oligo** | **Peptide Length** | | **SEQ ID NO** |
|---|---|---|---|---|
| N2_000 | | 0 | Not degenerate | n/a |
| N2_001 | GCT | 1 | Not degenerate | n/a |
| N2_002 | GAT | 1 | Not degenerate | n/a |
| N2_003 | GAG | 1 | Not degenerate | n/a |
| N2_004 | TTT | 1 | Not degenerate | n/a |
| N2_005 | GGC | 1 | Not degenerate | n/a |
| N2_006 | CAT | 1 | Not degenerate | n/a |
| N2_007 | ATC | 1 | Not degenerate | n/a |
| N2_008 | AAA | 1 | Not degenerate | n/a |
| N2_009 | TTG | 1 | Not degenerate | n/a |
| N2_010 | ATG | 1 | Not degenerate | n/a |
| N2_011 | CCT | 1 | Not degenerate | n/a |
| N2_012 | CAA | 1 | Not degenerate | n/a |
| N2_013 | AGG | 1 | Not degenerate | n/a |
| N2_014 | TCA | 1 | Not degenerate | n/a |
| N2_015 | ACC | 1 | Not degenerate | n/a |
| N2_016 | GTT | 1 | Not degenerate | n/a |
| N2_017 | TGG | 1 | Not degenerate | n/a |
| N2_018 | TAC | 1 | Not degenerate | n/a |
| N2_019 | GMCKHT | 2 | | n/a |
| N2_020 | GMCSVT | 2 | | n/a |
| N2_021 | GMCSHT | 2 | | n/a |
| N2_022 | GMCSVG | 2 | | n/a |
| N2_023 | GMTDYT | 2 | | n/a |
| N2_024 | KHCGAS | 2 | | n/a |
| N2_025 | KHCGRC | 2 | | n/a |
| N2_026 | KHCGWG | 2 | | n/a |
| N2_027 | KHTTTM | 2 | | n/a |
| N2_028 | KHTTYC | 2 | | n/a |
| N2_029 | KHTTWC | 2 | | n/a |
| N2_030 | KHCRGA | 2 | | n/a |
| N2_031 | KHCKGG | 2 | | n/a |
| N2_032 | KHCCWC | 2 | | n/a |
| N2_033 | KHCCMT | 2 | | n/a |
| N2_034 | KHCMCA | 2 | | n/a |
| N2_035 | GVCSWG | 2 | | n/a |
| N2_036 | GVCMKC | 2 | | n/a |
| N2_037 | GVCWSG | 2 | | n/a |
| N2_038 | SVCYAC | 2 | | n/a |
| N2_039 | GNAAHA | 2 | | n/a |
| N2_040 | BYCSAG | 2 | | n/a |
| N2_041 | RBAAWA | 2 | | n/a |
| N2_042 | RBAAYA | 2 | | n/a |
| N2_043 | SBAMAA | 2 | | n/a |
| N2_044 | VSCMAA | 2 | | n/a |
| N2_045 | GRARVG | 2 | | n/a |
| N2_046 | GRADYT | 2 | | n/a |
| N2_047 | GRABYT | 2 | | n/a |
| N2_048 | GRAKBG | 2 | | n/a |
| N2_046 | RDAGMT | 2 | | n/a |
| N2_050 | RDAGAK | 2 | | n/a |
| N2_051 | RDAGRT | 2 | | n/a |
| N2_052 | RDAGWG | 2 | | n/a |
| N2_053 | RDARGG | 2 | | n/a |
| N2_054 | RDACYA | 2 | | n/a |
| N2_055 | SDACSA | 2 | | n/a |
| N2_056 | VWACYA | 2 | | n/a |
| N2_057 | VWATYA | 2 | | n/a |
| N2_058 | VWAASA | 2 | | n/a |
| N2_059 | YHCGMC | 2 | | n/a |
| N2_060 | YHCGMG | 2 | | n/a |
| N2_061 | YHCGST | 2 | | n/a |
| N2_062 | YHCSCG | 2 | | n/a |
| N2_063 | YHCKCG | 2 | | n/a |
| N2_064 | YHCSAC | 2 | | n/a |
| N2_065 | YHCKAC | 2 | | n/a |
| N2_066 | YHCRGA | 2 | | n/a |
| N2_067 | YHCCWC | 2 | | n/a |
| N2_068 | YHCMCA | 2 | | n/a |
| N2_069 | WTCYHT | 2 | | n/a |
| N2_070 | HYCGWG | 2 | | n/a |
| N2_071 | HYCTTM | 2 | | n/a |
| N2_072 | HYCAGM | 2 | | n/a |
| N2_073 | HYCTMC | 2 | | n/a |
| N2_074 | VKCTWT | 2 | | n/a |
| N2_075 | CNCVGC | 2 | | n/a |
| N2_076 | MHAGAK | 2 | | n/a |
| N2_077 | MHAGRC | 2 | | n/a |
| N2_078 | MHAGWG | 2 | | n/a |
| N2_079 | MHAMCA | 2 | | n/a |
| N2_080 | ANAGBT | 2 | | n/a |
| N2_081 | MBCYAC | 2 | | n/a |
| N2_082 | MBCAWA | 2 | | n/a |
| N2_083 | MHGGKA | 2 | | n/a |
| N2_084 | CNABTT | 2 | | n/a |
| N2_085 | CVACNA | 2 | | n/a |
| N2_086 | CVAYSG | 2 | | n/a |
| N2_087 | MSCAHG | 2 | | n/a |
| N2_088 | CRAKBG | 2 | | n/a |
| N2_089 | WSGHCA | 2 | | n/a |
| N2_090 | WGGKHC | 2 | | n/a |
| N2_091 | MBCATR | 2 | | n/a |
| N2_092 | AYABSG | 2 | | n/a |
| N2_093 | VYCAWG | 2 | | n/a |
| N2_094 | BGGSAK | 2 | | n/a |
| N2_095 | AHGRYT | 2 | | n/a |
| N2_096 | BWCAMA | 2 | | n/a |
| N2_097 | BHCTGG | 2 | | n/a |
| N2_098 | TGGBHC | 2 | | n/a |
| N2_099 | TGGVBT | 2 | | n/a |
| N2_100 | NHCGCAGCC | 3 | | n/a |
| N2_101 | BHCGGAATG | 3 | | n/a |
| N2_102 | BHCGGAGGA | 3 | | n/a |
| N2_103 | BHCGGAGTA | 3 | | n/a |
| N2_104 | VNCGCAGGA | 3 | | n/a |
| N2_105 | VBCGGAGCC | 3 | | n/a |
| N2_106 | VBCGGAGGA | 3 | | n/a |
| N2_107 | VBCGGACTA | 3 | | n/a |
| N2_108 | VBCGGAAGG | 3 | | n/a |
| N2_109 | VBCGGAAGC | 3 | | n/a |
| N2_110 | VBCGGAGTA | 3 | | n/a |
| N2_111 | VNCCTTGGA | 3 | | n/a |
| N2_112 | VNCCCAGGA | 3 | | n/a |
| N2_113 | VNCCCACCA | 3 | | n/a |
| N2_114 | VNCAGAGGA | 3 | | n/a |
| N2_115 | VNCAGCGGA | 3 | | n/a |
| N2_116 | VBCACAGGA | 3 | | n/a |
| N2_117 | VNCGTAGGA | 3 | | n/a |
| N2_118 | BHCGGACAC | 3 | | n/a |
| N2_119 | NHCAAACAA | 3 | | n/a |
| N2_120 | NHCAAAAGA | 3 | | n/a |
| N2_121 | BHCACACAA | 3 | | n/a |
| N2_122 | VNCTTTGAG | 3 | | n/a |
| N2_123 | VNCCCACTA | 3 | | n/a |
| N2_124 | VNCCCATAC | 3 | | n/a |
| N2_125 | BHCGGAGAG | 3 | | n/a |
| N2_126 | BHCGGACTA | 3 | | n/a |
| N2_127 | BHCGGATGG | 3 | | n/a |
| N2_128 | BHCGGATAC | 3 | | n/a |
| N2_129 | NHCAGAGGA | 3 | | n/a |
| N2_130 | NHCAGCGAG | 3 | | n/a |
| N2_131 | NHCAGCTGG | 3 | | n/a |
| N2_132 | VHAGGAGGA | 3 | | n/a |
| N2_133 | BHCGGAAGG | 3 | | n/a |
| N2_134 | NHCCAAGGA | 3 | | n/a |
| N2_135 | BHCACAGCT | 3 | | n/a |
| N2_136 | GGABHCGGATAC | 4 | | 8399 |
| N2_137 | AGABHCGGATAC | 4 | | 8400 |
| N2_138 | AGCBHCGGATAC | 4 | | 8401 |
| N2_139 | CCABHCGGATAC | 4 | | 8402 |
| N2_140 | GGTAGAVHGTAC | 4 | | 8403 |
| N2_141 | AGGAGAVHGTAC | 4 | | 8404 |
| N2_142 | GGABHCGGATGG | 4 | | 8405 |
| N2_143 | GGABHCGGACTA | 4 | | 8406 |
| N2_144 | GGABHCACAGCT | 4 | | 8407 |
| N2_145 | GGABHCACACAA | 4 | | 8408 |

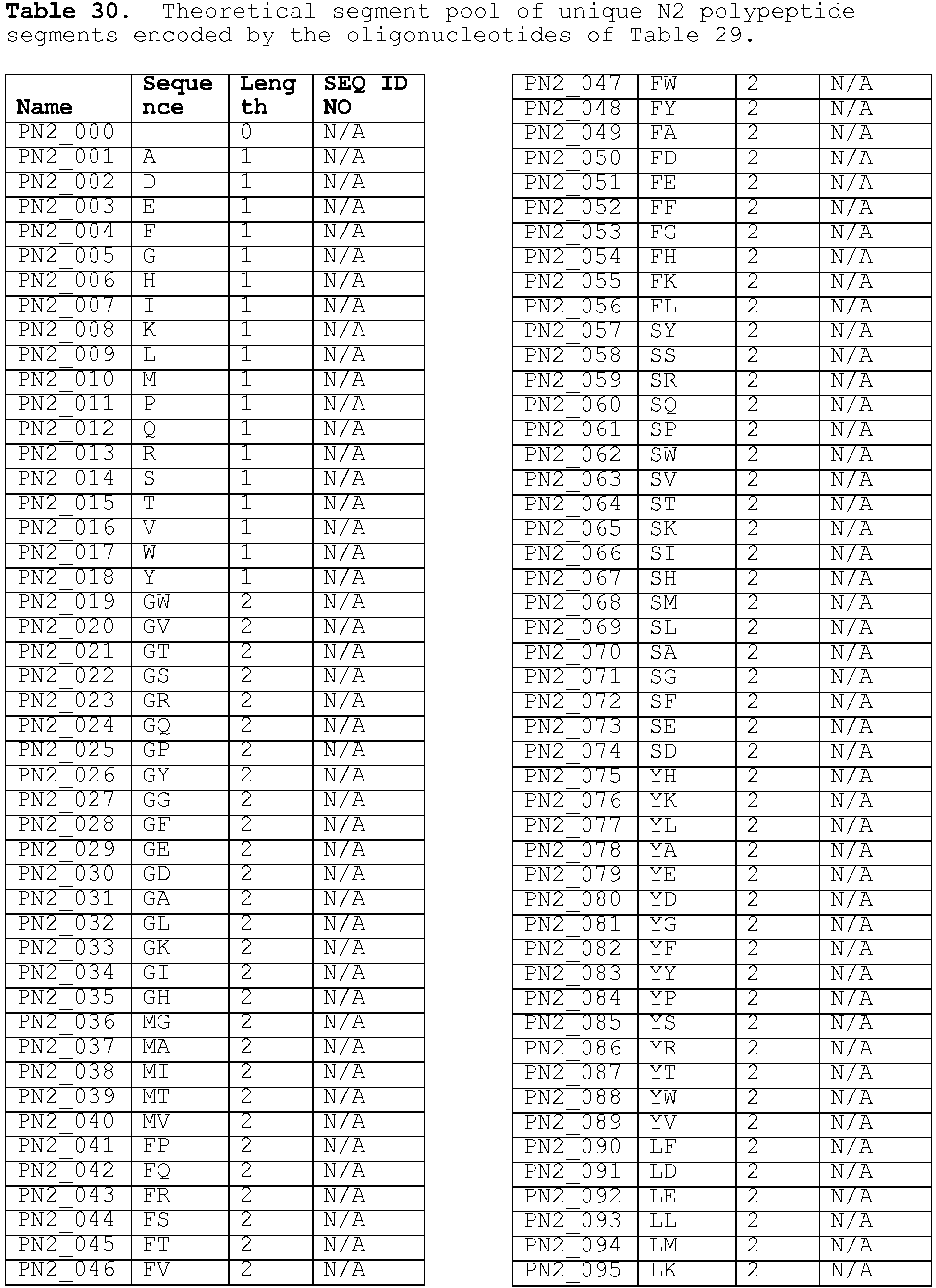

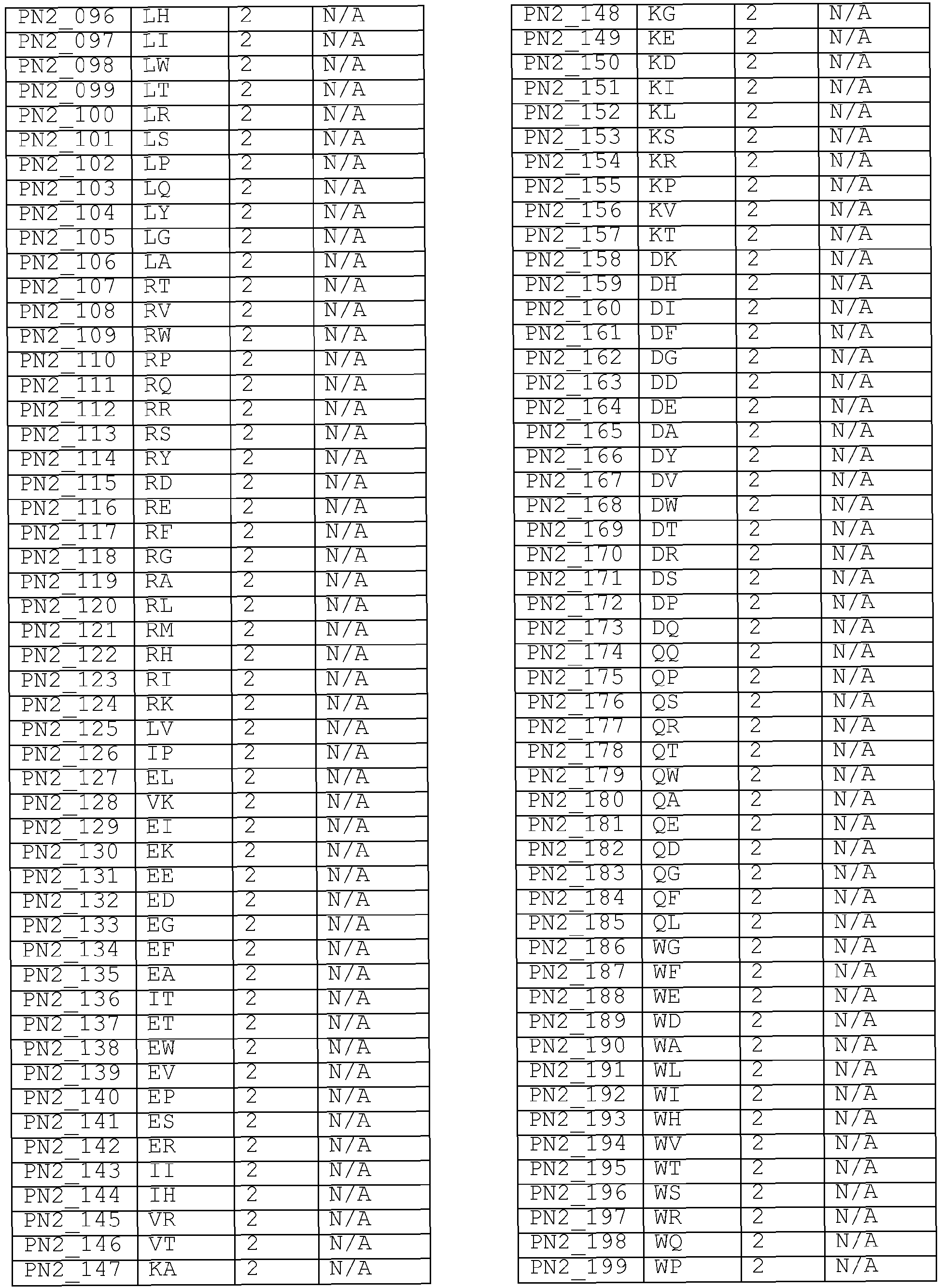

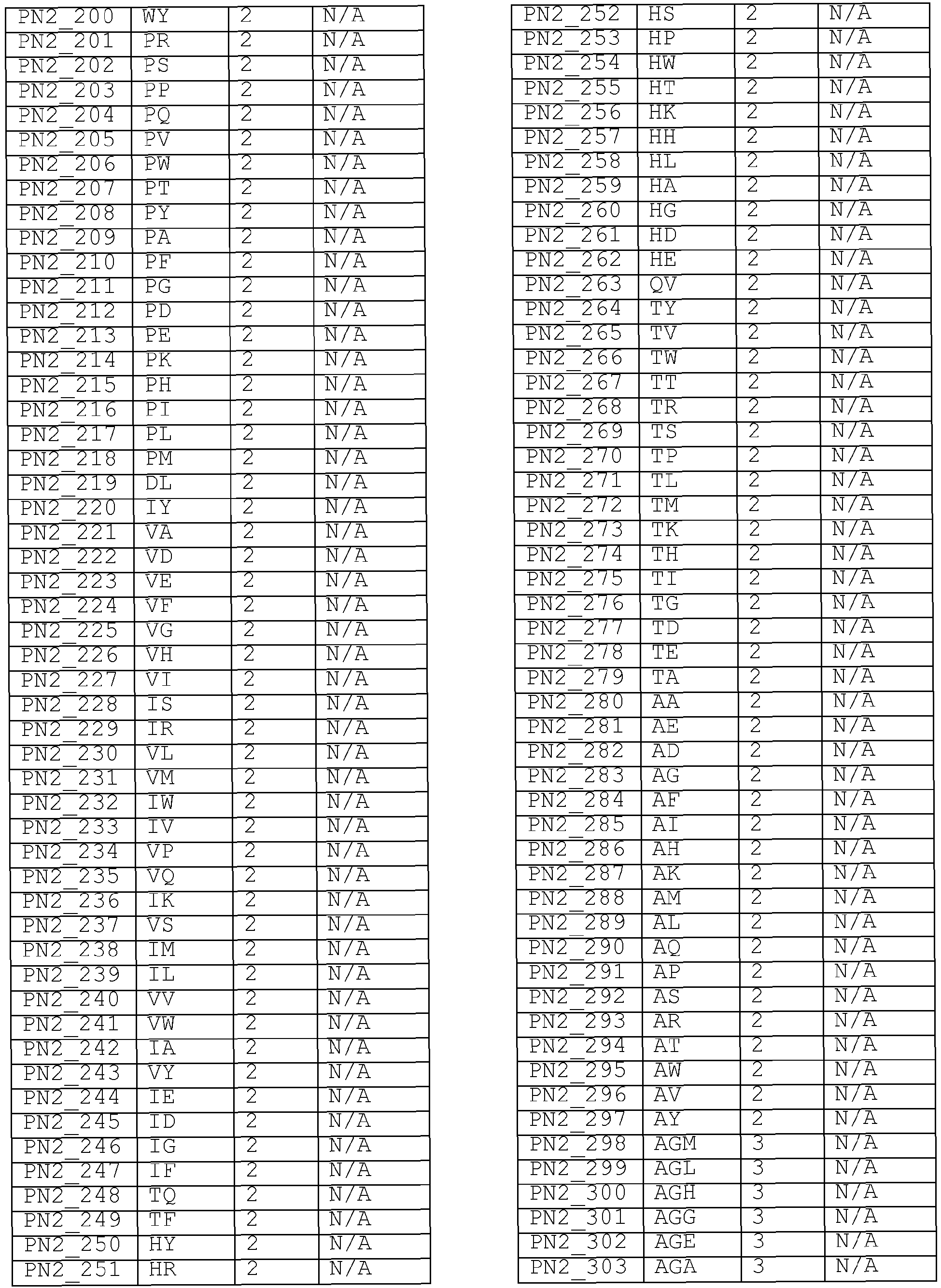

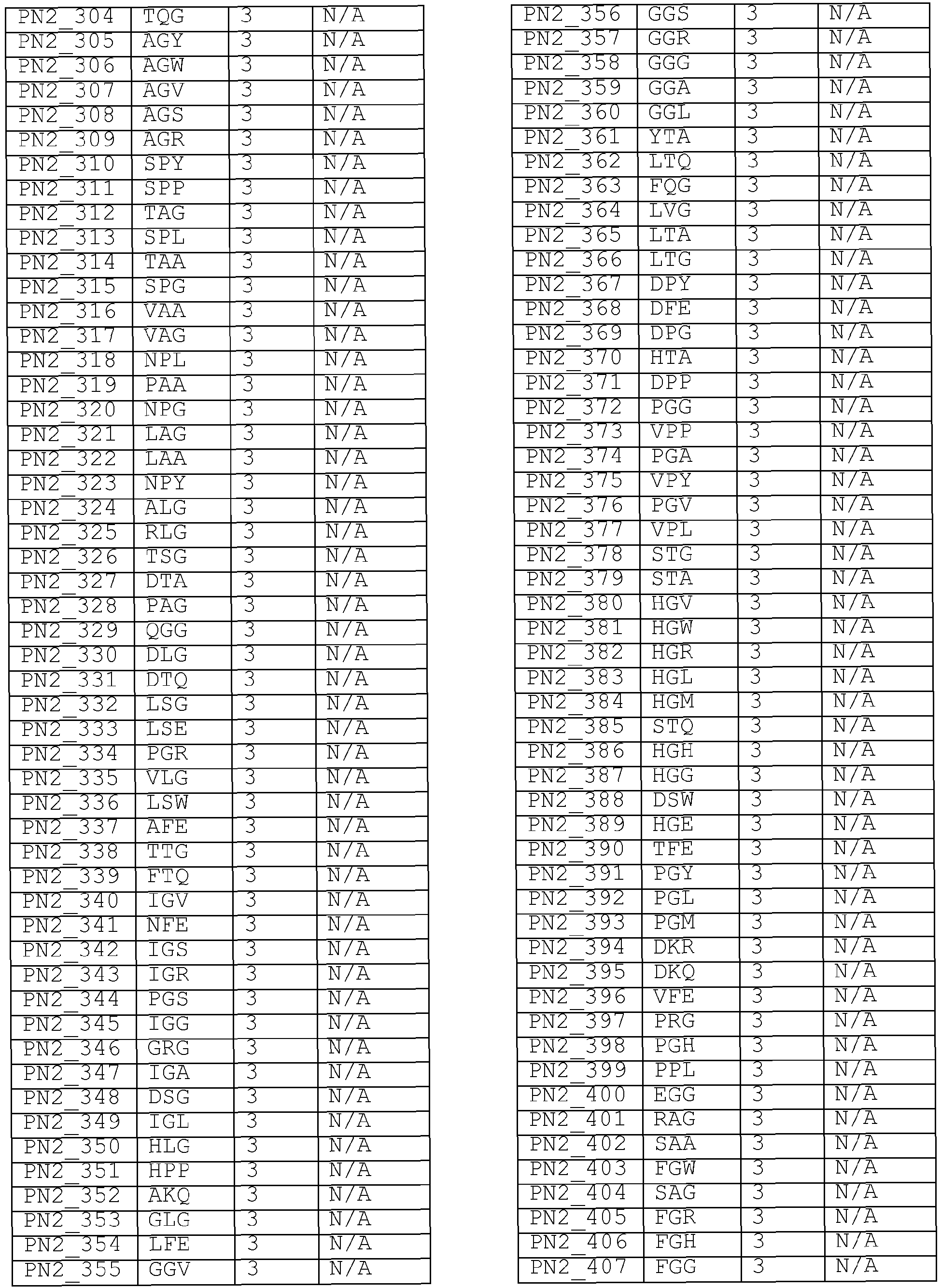

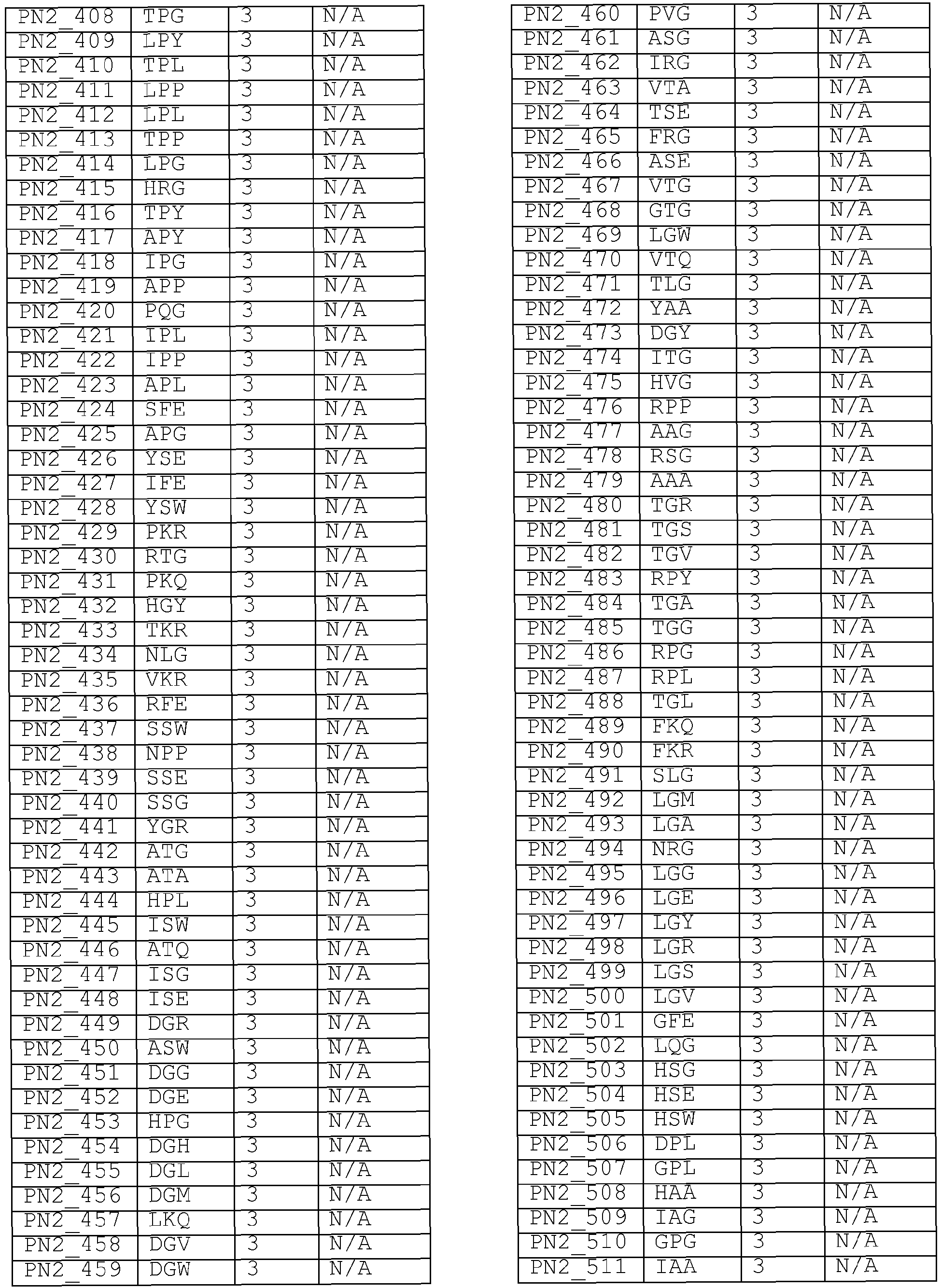

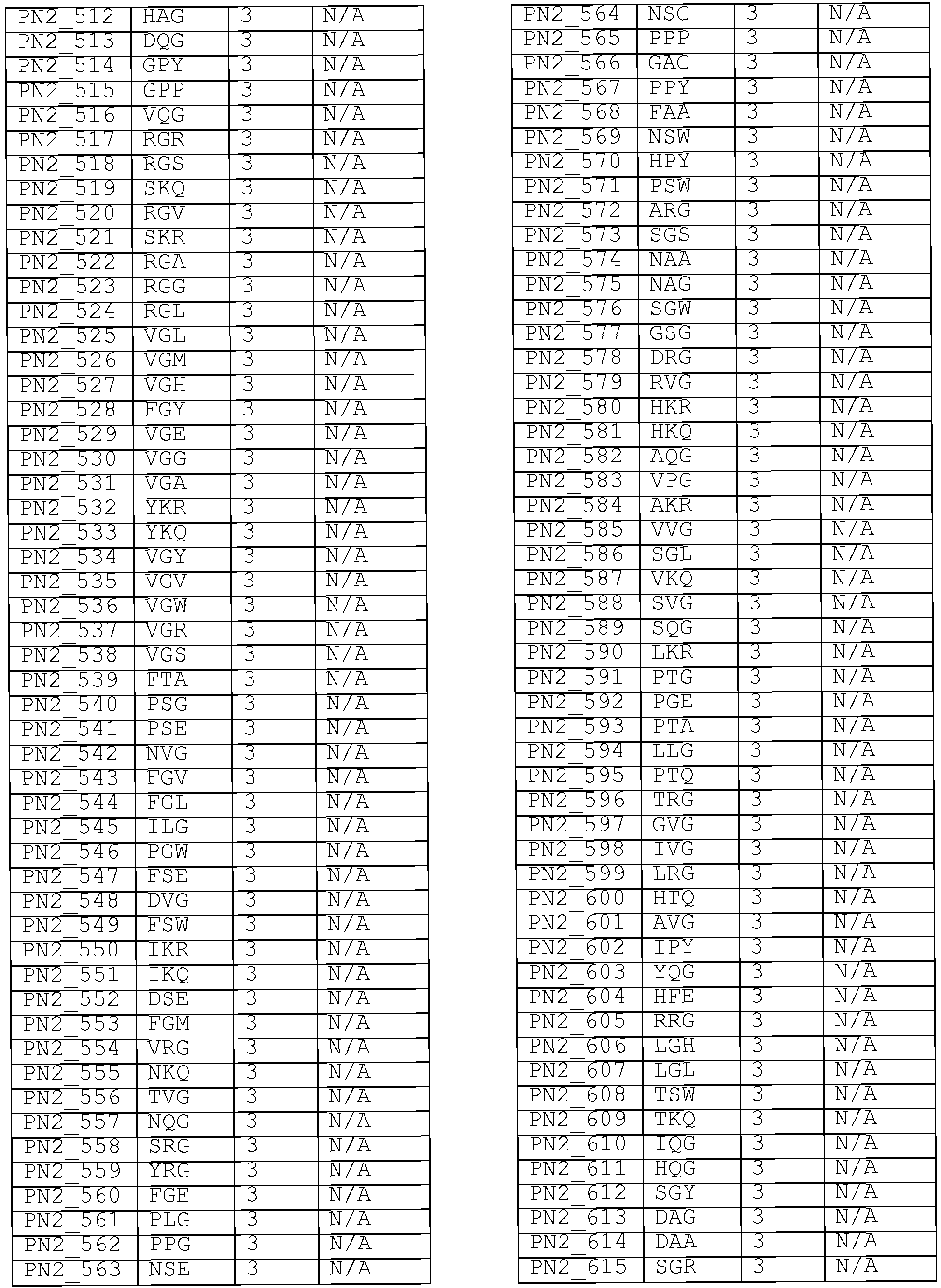

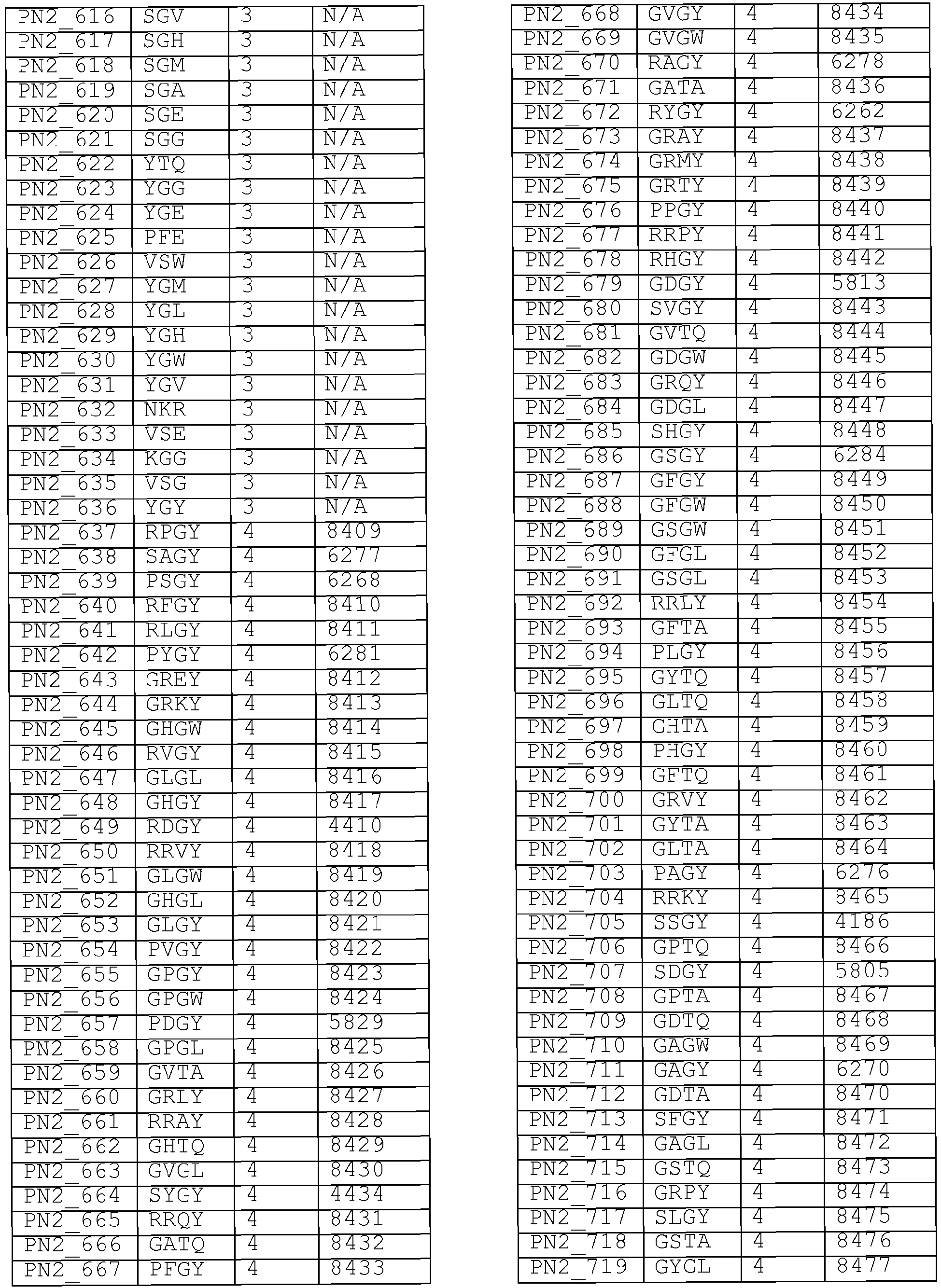

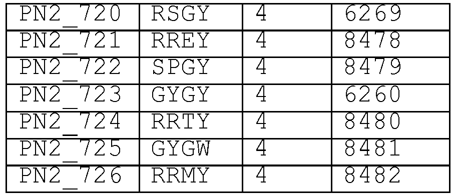

**Table 31. Theoretical segment pool of oligonucleotides encoding JH segments of Example 15.**

| **Name** | **Degenerate Oligo** | **Pepti e Length** | **Degenerate** | **SEQ ID NO** |
|---|---|---|---|---|
| JH4_001 | TGGGGACAGGGTACATTGGTCACCGTCTCCTCA | 0 | | 8483 |
| JH1_002 | CATTGGGGACAGGGTACATTGGTCACCGTCTCCTCA | 1 | | 8484 |
| JH1_003 | ATTTGGGGACAGGGTACATTGGTCACCGTCTCCTCA | 1 | | 8485 |
| JH1_004 | TACTGGGGACAGGGTACATTGGTCACCGTCTCCTCA | 1 | | 8486 |
| JH1_005 | CCATGGGGACAGGGTACATTGGTCACCGTCTCCTCA | 1 | | 8487 |
| JH1_006 | GTCTGGGGACAGGGTACATTGGTCACCGTCTCCTCA | 1 | | 8488 |
| JH1_007 | GATTGGGGACAGGGTACATTGGTCACCGTCTCCTCA | 1 | | 8489 |
| JH1_008 | TTCTGGGGACAGGGTACATTGGTCACCGTCTCCTCA | 1 | | 8490 |
| JH1_009 | AATTGGGGACAGGGTACATTGGTCACCGTCTCCTCA | 1 | | 8491 |
| JH1_010 | AGTTGGGGACAGGGTACATTGGTCACCGTCTCCTCA | 1 | | 8492 |
| JH1_011 | ACTTGGGGACAGGGTACATTGGTCACCGTCTCCTCA | 1 | | 8493 |
| JH1_200 | GCATGGGGACAGGGTACATTGGTCACCGTCTCCTCA | 1 | | 8494 |
| JH4_013 | GATTACTGGGGACAGGGTACATTGGTCACCGTCTCCTCA | 2 | | 8495 |
| JH4_016 | GCTTACTGGGGACAGGGTACATTGGTCACCGTCTCCTCA | 2 | | 8496 |
| JH4_017 | TTCTACTGGGGACAGGGTACATTGGTCACCGTCTCCTCA | 2 | | 8497 |
| JH4_018 | GGCTATTGGGGACAGGGTACATTGGTCACCGTCTCCTCA | 2 | | 8498 |
| JH4_019 | CATTACTGGGGACAGGGTACATTGGTCACCGTCTCCTCA | 2 | | 8499 |
| JH4_023 | AGTTACTGGGGACAGGGTACATTGGTCACCGTCTCCTCA | 2 | | 8500 |
| JH4_024 | GTTTATTGGGGACAGGGTACATTGGTCACCGTCTCCTCA | 2 | | 8501 |
| JH4_025 | TACTATTGGGGACAGGGTACATTGGTCACCGTCTCCTCA | 2 | | 8502 |
| JH4_022 | CSATACTGGGGACAGGGTACATTGGTCACCGTCTCCTCA | 2 | YES | 8503 |
| JH3_012 | RACATATGGGGTCAGGGTACAATGGTCACCGTCTCCTCA | 2 | YES | 8504 |
| JH5_014 | RACCCATGGGGACAGGGTACATTGGTCACCGTCTCCTCA | 2 | YES | 8505 |
| JH3_015 | RACGTATGGGGTCAGGGTACAATGGTCACCGTCTCCTCA | 2 | YES | 8506 |
| JH4_021 | AMCTACTGGGGACAGGGTACATTGGTCACCGTCTCCTCA | 2 | YES | 8507 |
| JH4_020 | MTATACTGGGGACAGGGTACATTGGTCACCGTCTCCTCA | 2 | YES | 8508 |
| JH4_029 | TTCGATTATTGGGGACAGGGTACATTGGTCACCGTCTCCTCA | 3 | | 8509 |
| JH4_030 | ATTGACTACTGGGGACAGGGTACATTGGTCACCGTCTCCTCA | 3 | | 8510 |
| JH4_031 | GTGGACTACTGGGGACAGGGTACATTGGTCACCGTCTCCTCA | 3 | | 8511 |
| JH4_032 | TTAGATTACTGGGGACAGGGTACATTGGTCACCGTCTCCTCA | 3 | | 8512 |
| JH4_033 | TCCGATTATTGGGGACAGGGTACATTGGTCACCGTCTCCTCA | 3 | | 8513 |
| JH4_034 | CACGATTACTGGGGACAGGGTACATTGGTCACCGTCTCCTCA | 3 | | 8514 |
| JH4_035 | AGAGACTACTGGGGACAGGGTACATTGGTCACCGTCTCCTCA | 3 | | 8515 |
| JH4_036 | CCAGATTACTGGGGACAGGGTACATTGGTCACCGTCTCCTCA | 3 | | 8516 |
| JH4_037 | AACGATTACTGGGGACAGGGTACATTGGTCACCGTCTCCTCA | 3 | | 8517 |
| JH4_038 | ACTGATTACTGGGGACAGGGTACATTGGTCACCGTCTCCTCA | 3 | | 8518 |
| JH4_039 | GATGATTATTGGGGACAGGGTACATTGGTCACCGTCTCCTCA | 3 | | 8519 |
| JH4_040 | GGTGATTATTGGGGACAGGGTACATTGGTCACCGTCTCCTCA | 3 | | 8520 |
| JH4_041 | GCAGACTACTGGGGACAGGGTACATTGGTCACCGTCTCCTCA | 3 | | 8521 |
| JH4_042 | TACGATTATTGGGGACAGGGTACATTGGTCACCGTCTCCTCA | 3 | | 8522 |
| JH5_043 | TTCGATCCCTGGGGACAGGGTACATTGGTCACCGTCTCCTCA | 3 | | 8b23 |
| JH6_044 | ATGGATGTGTGGGGCCAGGGAACAACTGTCACCGTCTCCTCA | 3 | | 8524 |
| JH1_026 | TTMCAACACTGGGGACAGGGTACATTGGTCACCGTCTCCTCA | 3 | YES | 8525 |
| JH3_046 | STAGACGTATGGGGTCAGGGTACAATGGTCACCGTCTCCTCA | 3 | YES | 8526 |
| JH3_028 | TTMGACATATGGGGTCAGGGTACAATGGTCACCGTCTCCTCA | 3 | YES | 8527 |
| JH2_027 | TTMGACCTATGGGGGAGAGGTACCTTGGTCACCGTCTCCTCA | 3 | YES | 8528 |
| JH5_045 | TYAGACCCATGGGGACAGGGTACATTGGTCACCGTCTCCTCA | 3 | YES | 8529 |
| JH3_049 | GCCTTTGATATTTGGGGTCAGGGTACAATGGTCACCGTCTCCTCA | 4 | | 8530 |
| JH4_051 | TACTTTGACTACTGGGGACAGGGTACATTGGTCACCGTCTCCTCA | 4 | | 8531 |
| JH4_052 | AATTTCGACTACTGGGGACAGGGTACATTGGTCACCGTCTCCTCA | 4 | | 8532 |
| JH4_053 | GACTTCGACTATTGGGGACAGGGTACATTGGTCACCGTCTCCTCA | 4 | | 8533 |
| JH4_054 | CATTTCGATTACTGGGGACAGGGTACATTGGTCACCGTCTCCTCA | 4 | | 8534 |
| JH4_055 | TTCTTTGATTATTGGGGACAGGGTACATTGGTCACCGTCTCCTCA | 4 | | 8535 |
| JH4_056 | TCTTTTGATTATTGGGGACAGGGTACATTGGTCACCGTCTCCTCA | 4 | | 8536 |
| JH4_057 | AGATTCGACTACTGGGGACAGGGTACATTGGTCACCGTCTCCTCA | 4 | | 8537 |
| JH4_058 | TTGTTCGACTACTGGGGACAGGGTACATTGGTCACCGTCTCCTCA | 4 | | 8538 |
| JH4_059 | CCCTTCGACTATTGGGGACAGGGTACATTGGTCACCGTCTCCTCA | 4 | | 8539 |
| JH4_060 | ATTTTCGATTATTGGGGACAGGGTACATTGGTCACCGTCTCCTCA | 4 | | 8540 |
| JH4_061 | ACCTTTGATTACTGGGGACAGGGTACATTGGTCACCGTCTCCTCA | 4 | | 8541 |
| JH4_062 | GGATTCGATTACTGGGGACAGGGTACATTGGTCACCGTCTCCTCA | 4 | | 8542 |
| JH4_063 | GTTTTCGATTACTGGGGACAGGGTACATTGGTCACCGTCTCCTCA | 4 | | 8543 |
| JH4_064 | GCTTTTGACTACTGGGGACAGGGTACATTGGTCACCGTCTCCTCA | 4 | | 8544 |
| JH5_065 | TGGTTTGATCCATGGGGACAGGGTACATTGGTCACCGTCTCCTCA | 4 | | 8545 |
| JH6_068 | GGAATGGACGTATGGGGCCAGGGAACAACTGTCACCGTCTCCTCA | 4 | | 8546 |
| JH6_070 | YCAATGGACGTATGGGGCCAGGGAACAACTGTCACCGTCTCCTCA | 4 | YES | 8547 |
| JH6_069 | KACATGGACGTATGGGGCAAGGGTACAACTGTCACCGTCTCCTCA | 4 | YES | 8548 |
| JH3_198 | STATTCGACATATGGGGTCAGGGTACAATGGTCACCGTCTCCTCA | 4 | YES | 8549 |
| JH2_048 | KACTTCGACCTATGGGGGAGAGGTACCTTGGTCACCGTCTCCTCA | 4 | YES | 8550 |
| JH1_047 | KACTTCCAACACTGGGGACAGGGTACATTGGTCACCGTCTCCTCA | 4 | YES | 8551 |
| JH5_067 | GSATTCGACCCATGGGGACAGGGTACATTGGTCACCGTCTCCTCA | 4 | YES | 8552 |
| JH3_050 | YCATTCGACATATGGGGTCAGGGTACAATGGTCACCGTCTCCTCA | 4 | YES | 8553 |
| JH5_066 | AGMTTCGACCCATGGGGACAGGGTACATTGGTCACCGTCTCCTCA | 4 | YES | 8554 |
| JH2_072 | TGGTACTTCGACTTATGGGGGAGAGGTACCTTGGTCACCGTCTCCTCA | 5 | | 8555 |
| JH3_075 | GACGCATTTGATATATGGGGTCAGGGTACAATGGTCACCGTCTCCTCA | 5 | | 8556 |
| JH3_076 | TACGCATTTGATATTTGGGGTCAGGGTACAATGGTCACCGTCTCCTCA | 5 | | 8557 |
| JH3_077 | CACGCATTCGACATCTGGGGTCAGGGTACAATGGTCACCGTCTCCTCA | 5 | | 8558 |
| JH3_078 | TTCGCATTCGATATCTGGGGTCAGGGTACAATGGTCACCGTCTCCTCA | 5 | | 8559 |
| JH3_079 | TCAGCTTTCGACATATGGGGTCAGGGTACAATGGTCACCGTCTCCTCA | 5 | | 8560 |
| JH3_080 | AGAGCCTTCGATATATGGGGTCAGGGTACAATGGTCACCGTCTCCTCA | 5 | | 8561 |
| JH3_081 | TTAGCCTTCGATATCTGGGGTCAGGGTACAATGGTCACCGTCTCCTCA | 5 | | 8562 |
| JH3_082 | GGAGCCTTCGACATATGGGGTCAGGGTACAATGGTCACCGTCTCCTCA | 5 | | 8563 |
| JH4_086 | GACTATTTTGATTACTGGGGACAGGGTACATTGGTCACCGTCTCCTCA | 5 | | 8564 |
| JH4_087 | TATTACTTTGATTACTGGGGACAGGGTACATTGGTCACCGTCTCCTCA | 5 | | 8565 |
| JH4_088 | CACTATTTCGACTATTGGGGACAGGGTACATTGGTCACCGTCTCCTCA | 5 | | 8566 |
| JH4_089 | TTCTATTTTGATTACTGGGGACAGGGTACATTGGTCACCGTCTCCTCA | 5 | | 8567 |
| JH4_090 | AGTTATTTTGACTATTGGGGACAGGGTACATTGGTCACCGTCTCCTCA | 5 | | 8568 |
| JH4_091 | AGATACTTTGATTACTGGGGACAGGGTACATTGGTCACCGTCTCCTCA | 5 | | 8569 |
| JH4_092 | TTATATTTCGACTATTGGGGACAGGGTACATTGGTCACCGTCTCCTCA | 5 | | 8570 |
| JH4_093 | CCCTACTTTGACTATTGGGGACAGGGTACATTGGTCACCGTCTCCTCA | 5 | | 8571 |
| JH4_095 | GGATATTTCGATTATTGGGGACAGGGTACATTGGTCACCGTCTCCTCA | 5 | | 8572 |
| JH4_096 | GTTTACTTTGATTATTGGGGACAGGGTACATTGGTCACCGTCTCCTCA | 5 | | 8573 |
| JH4_097 | GCTTACTTTGATTATTGGGGACAGGGTACATTGGTCACCGTCTCCTCA | 5 | | 8574 |
| JH4_098 | AACTACTTCGATTACTGGGGACAGGGTACATTGGTCACCGTCTCCTCA | 5 | | 8575 |
| JH5_099 | AATTGGTTCGATCCTTGGGGACAGGGTACATTGGTCACCGTCTCCTCA | 5 | | 8576 |
| JH5_100 | GATTGGTTTGATCCATGGGGACAGGGTACATTGGTCACCGTCTCCTCA | 5 | | 8577 |
| JH5_101 | TATTGGTTTGATCCATGGGGACAGGGTACATTGGTCACCGTCTCCTCA | 5 | | 8578 |
| JH5_102 | CACTGGTTCGATCCATGGGGACAGGGTACATTGGTCACCGTCTCCTCA | 5 | | 8579 |
| JH5_103 | TTCTGGTTTGACCCCTGGGGACAGGGTACATTGGTCACCGTCTCCTCA | 5 | | 8580 |
| JH5_104 | TCTTGGTTTGATCCCTGGGGACAGGGTACATTGGTCACCGTCTCCTCA | 5 | | 8581 |
| JH5_105 | AGATGGTTTGATCCTTGGGGACAGGGTACATTGGTCACCGTCTCCTCA | 5 | | 8582 |
| JH5_107 | GGTTGGTTCGATCCCTGGGGACAGGGTACATTGGTCACCGTCTCCTCA | 5 | | 8583 |
| JH5_109 | GCTTGGTTTGATCCATGGGGACAGGGTACATTGGTCACCGTCTCCTCA | 5 | | 8584 |
| JH6_110 | TACGGTATGGACGTTTGGGGCCAGGGAACAACTGTCACCGTCTCCTCA | 5 | | 8585 |
| JH6_112 | GATGGGATGGATGTTTGGGGCCAGGGAACAACTGTCACCGTCTCCTCA | 5 | | 8586 |
| JH3_084 | SCAGCATTCGACATATGGGGTCAGGGTACAATGGTCACCGTCTCCTCA | 5 | YES | 8587 |
| JH5_106 | MCATGGTTCGACCCATGGGGACAGGGTACATTGGTCACCGTCTCCTCA | 5 | YES | 8588 |
| JH2_073 | RGCTACTTCGACCTATGGGGGAGAGGTACCTTGGTCACCGTCTCCTCA | 5 | YES | 8589 |
| JH4_094 | AYATACTTCGACTACTGGGGACAGGGTACATTGGTCACCGTCTCCTCA | 5 | YES | 8590 |
| JH5_108 | RTATGGTTCGACCCATGGGGACAGGGTACATTGGTCACCGTCTCCTCA | 5 | YES | 8591 |
| JH6_113 | CWCGGAATGGACGTATGGGGCCAGGGAACAACTGTCACCGTCTCCTCA | 5 | YES | 8592 |
| JH6_114 | KCAGGAATGGACGTATGGGGCCAGGGAACAACTGTCACCGTCTCCTCA | 5 | YES | 8593 |
| JH3_083 | RTAGCATTCGACATATGGGGTCAGGGTACAATGGTCACCGTCTCCTCA | 5 | YES | 8594 |
| JH1_071 | RAGTACTTCCAACACTGGGGACAGGGTACATTGGTCACCGTCTCCTCA | 5 | YES | 8595 |
| JH3_085 | AMCGCATTCGACATATGGGGTCAGGGTACAATGGTCACCGTCTCCTCA | 5 | YES | 8596 |
| JH2_074 | CKATACTTCGACCTATGGGGGAGAGGTACCTTGGTCACCGTCTCCTCA | 5 | YES | 8597 |
| JH6_111 | KACTACATGGACGTATGGGGCAAGGGTACAACTGTCACCGTCTCCTCA | 5 | YES | 8598 |
| JH2_116 | TACTGGTACTTCGATTTGTGGGGGAGAGGTACCTTGGTCACCGTCTCCTCA | 6 | | 8599 |
| JH2_117 | GATTGGTACTTCGATTTATGGGGGAGAGGTACCTTGGTCACCGTCTCCTCA | 6 | | 8600 |
| JH5_120 | GATAATTGGTTCGATCCATGGGGACAGGGTACATTGGTCACCGTCTCCTCA | 6 | | 8601 |
| JH5_121 | TATAACTGGTTCGATCCCTGGGGACAGGGTACATTGGTCACCGTCTCCTCA | 6 | | 8602 |
| JH5_122 | CACAATTGGTTCGACCCCTGGGGACAGGGTACATTGGTCACCGTCTCCTCA | 6 | | 8603 |
| JH5_123 | TTCAATTGGTTTGATCCCTGGGGACAGGGTACATTGGTCACCGTCTCCTCA | 6 | | 8604 |
| JH5_124 | AGCAACTGGTTCGACCCATGGGGACAGGGTACATTGGTCACCGTCTCCTCA | 6 | | 8605 |
| JH5_125 | AGAAACTGGTTTGATCCATGGGGACAGGGTACATTGGTCACCGTCTCCTCA | 6 | | 8606 |
| JH5_126 | TTAAATTGGTTCGACCCTTGGGGACAGGGTACATTGGTCACCGTCTCCTCA | 6 | | 8607 |
| JH5_127 | CCCAATTGGTTTGATCCATGGGGACAGGGTACATTGGTCACCGTCTCCTCA | 6 | | 8608 |
| JH5_128 | ATAAATTGGTTCGACCCTTGGGGACAGGGTACATTGGTCACCGTCTCCTCA | 6 | | 8609 |
| JH5_129 | ACTAACTGGTTTGACCCATGGGGACAGGGTACATTGGTCACCGTCTCCTCA | 6 | | 8610 |
| JH5_130 | GGTAACTGGTTTGACCCATGGGGACAGGGTACATTGGTCACCGTCTCCTCA | 6 | | 8611 |
| JH5_131 | GTGAACTGGTTTGATCCCTGGGGACAGGGTACATTGGTCACCGTCTCCTCA | 6 | | 8612 |
| JH5_132 | GCCAACTGGTTCGATCCCTGGGGACAGGGTACATTGGTCACCGTCTCCTCA | 6 | | 8613 |
| JH5_133 | AACAATTGGTTCGACCCATGGGGACAGGGTACATTGGTCACCGTCTCCTCA | 6 | | 8614 |
| JH6_134 | TACTACGGCATGGATGTATGGGGCCAGGGAACAACTGTCACCGTCTCCTCA | 6 | | 8615 |
| JH6_136 | GATTATGGAATGGACGTATGGGGCCAGGGAACAACTGTCACCGTCTCCTCA | 6 | | 8616 |
| JH6_137 | TTCTACGGTATGGATGTCTGGGGCCAGGGAACAACTGTCACCGTCTCCTCA | 6 | | 8617 |
| JH6_138 | CATTACGGAATGGACGTATGGGGCCAGGGAACAACTGTCACCGTCTCCTCA | 6 | | 8618 |
| JH6_139 | TTGTACGGAATGGACGTTTGGGGCCAGGGAACAACTGTCACCGTCTCCTCA | 6 | | 8619 |
| JH6_140 | AACTATGGCATGGATGTGTGGGGCCAGGGAACAACTGTCACCGTCTCCTCA | 6 | | 8620 |
| JH2_118 | CWCTGGTACTTCGACCTATGGGGGAGAGGTACCTTGGTCACCGTCTCCTCA | 6 | YES | 8621 |
| JH6_141 | SCATACGGAATGGACGTATGGGGCCAGGGAACAACTGTCACCGTCTCCTCA | 6 | YES | 8622 |
| JH1_115 | SCAGAATACTTCCAACACTGGGGACAGGGTACATTGGTCACCGTCTCCTCA | 6 | YES | 8623 |
| JH6_135 | KACTACTACATGGACGTATGGGGCAAGGGTACAACTGTCACCGTCTCCTCA | 6 | YES | 8624 |
| JH6_199 | GKATACGGAATGGACGTATGGGGCCAGGGAACAACTGTCACCGTCTCCTCA | 6 | YES | 8625 |
| JH6_142 | AKCTACGGAATGGACGTATGGGGCCAGGGAACAACTGTCACCGTCTCCTCA | 6 | YES | 8626 |
| JH2_119 | ARCTGGTACTTCGACCTATGGGGGAGAGGTACCTTGGTCACCGTCTCCTCA | 6 | YES | 8627 |
| JH6_143 | ASATACGGAATGGACGTATGGGGCCAGGGAACAACTGTCACCGTCTCCTCA | 6 | YES | 8628 |
| JH6_144 | TATTACTATGGTATGGATGTATGGGGCCAGGGAACAACTGTCACCGTCTCCTCA | 7 | | 8629 |
| JH6_145 | TATTACTATTATATGGATGTTTGGGGCAAGGGTACAACTGTCACCGTCTCCTCA | 7 | | 8630 |
| JH6_146 | GATTACTACGGCATGGATGTTTGGGGCCAGGGAACAACTGTCACCGTCTCCTCA | 7 | | 8631 |
| JH26_148 | AACTACTACGGCATGGACGTATGGGGCCAGGGAACAACTGTCACCGTCTCCTCA | 7 | | 8632 |
| JH6_147 | CWCTACTACGGAATGGACGTATGGGGCCAGGGAACAACTGTCACCGTCTCCTCA | 7 | YES | 8633 |
| JH6_150 | RACTACTACTACATGGACGTATGGGGCAAGGGTACAACTGTCACCGTCTCCTCA | 7 | YES | 8634 |
| JH6_149 | YCATACTACGGAATGGACGTATGGGGCCAGGGAACAACTGTCACCGTCTCCTCA | 7 | YES | 8635 |
| JH6_151 | TATTACTACTACGGAATGGACGTTTGGGGCCAGGGAACAACTGTCACCGTCTCCTCA | 8 | | 8636 |
| JH6-152 | AATTATTATTACGGCATGGACGTCTGGGGCCAGGGAACAACTGTCACCGTCTCCTCA | 8 | | 8637 |
| JH6_153 | GATTACTATTACGGTATGGATGTCTGGGGCCAGGGAACAACTGTCACCGTCTCCTCA | 8 | | 8638 |
| JH6_154 | CACTATTACTACGGCATGGACGTATGGGGCCAGGGAACAACTGTCACCGTCTCCTCA | 8 | | 8639 |
| JH6_155 | TTCTATTATTATGGTATGGATGTTTGGGGCCAGGGAACAACTGTCACCGTCTCCTCA | 8 | | 8640 |
| JH6_156 | TCTTACTACTATGGGATGGACGTGTGGGGCCAGGGAACAACTGTCACCGTCTCCTCA | 8 | | 8641 |
| JH2_157 | AGATATTACTACGGCATGGATGTTTGGGGCCAGGGAACAACTGTCACCGTCTCCTCA | 8 | | 8652 |
| JH6_158 | TTATACTACTATGGGATGGATGTATGGGGCCAGGGAACAACTGTCACCGTCTCCTCA | 8 | | 8643 |
| JH6_159 | CCTTACTACTATGGCATGGACGTCTGGGGCCAGGGAACAACTGTCACCGTCTCCTCA | 8 | | 8644 |
| JH6_160 | ACCTATTACTATGGTATGGATGTGTGGGGCCAGGGAACAACTGTCACCGTCTCCTCA | 8 | | 8645 |
| JH6_161 | GGATACTACTATGGGATGGACGTCTGGGGCCAGGGAACAACTGTCACCGTCTCCTCA | 8 | | 8646 |
| JH6_163 | GCCTACTATTATGGCATGGACGTCTGGGGCCAGGGAACAACTGTCACCGTCTCCTCA | 8 | | 8647 |
| JH6_164 | TACTACTATTATTATATGGACGTTTGGGGCAAGGGTACAACTGTCACCGTCTCCTCA | 8 | | 8648 |
| JH6_165 | AACTACTACTACTATATGGATGTTTGGGGCAAGGGTACAACTGTCACCGTCTCCTCA | 8 | | 8649 |
| JH6_166 | GATTATTATTACTATATGGACGTCTGGGGCAAGGGTACAACTGTCACCGTCTCCTCA | 8 | | 8650 |
| JH6_162 | RTATACTACTACGGAATGGACGTATGGGGCCAGGGAACAACTGTCACCGTCTCCTCA | 8 | YES | 8651 |
| JH6_168 | RGCTACTACTACTACATGGACGTATGGGGCAAGGGTACAACTGTCACCGTCTCCTCA | 8 | YES | 8652 |
| JH6_167 | CMCTACTACTACTACATGGACGTATGGGGCAAGGGTACAACTGTCACCGTCTCCTCA | 8 | YES | 8653 |
| JH6_169 | TATTACTATTATTACGGGATGGACGTATGGGGCCAGGGAACAACTGTCACCGTCTCCTCA | 9 | | 8654 |
| JH6_170 | AATTATTATTATTATGGGATGGACGTATGGGGCCAGGGAACAACTGTCACCGTCTCCTCA | 9 | | 8655 |
| JH6_171 | GACTATTACTATTATGGAATGGATGTATGGGGCCAGGGAACAACTGTCACCGTCTCCTCA | 9 | | 8656 |
| JH6_172 | CATTATTATTATTACGGAATGGACGTTTGGGGCCAGGGAACAACTGTCACCGTCTCCTCA | 9 | | 8657 |
| JH6_173 | TTCTATTACTATTATGGCATGGATGTGTGGGGCCAGGGAACAACTGTCACCGTCTCCTCA | 9 | | 8658 |
| JH6_174 | AGCTACTACTATTATGGTATGGACGTGTGGGGCCAGGGAACAACTGTCACCGTCTCCTCA | 9 | | 8659 |
| JH6_175 | AGATATTACTACTATGGCATGGATGTCTGGGGCCAGGGAACAACTGTCACCGTCTCCTCA | 9 | | 8660 |
| JH6_176 | TTATACTACTATTACGGCATGGATGTCTGGGGCCAGGGAACAACTGTCACCGTCTCCTCA | 9 | | 8661 |
| JH6_177 | CCCTATTATTACTACGGAATGGACGTTTGGGGCCAGGGAACAACTGTCACCGTCTCCTCA | 9 | | 8662 |
| JH6_178 | ATCTATTACTATTATGGCATGGATGTATGGGGCCAGGGAACAACTGTCACCGTCTCCTCA | 9 | | 8663 |
| JH6_179 | ACCTATTACTACTATGGCATGGACGTTTGGGGCCAGGGAACAACTGTCACCGTCTCCTCA | 9 | | 8664 |
| JH6_180 | GGCTACTATTACTATGGGATGGACGTCTGGGGCCAGGGAACAACTGTCACCGTCTCCTCA | 9 | | 8665 |
| JH6_181 | GTCTACTATTATTATGGCATGGACGTATGGGGCCAGGGAACAACTGTCACCGTCTCCTCA | 9 | | 8666 |
| JH6_182 | GCTTACTATTACTACGGCATGGACGTATGGGGCCAGGGAACAACTGTCACCGTCTCCTCA | 9 | | 8667 |
| JH6_183 | GATTATTATTATTACTATGGTATGGATGTGTGGGGCCAGGGAACAACTGTCACCGTCTCCTCA | 10 | | 8668 |
| JH6_184 | TACTATTACTACTATTATGGCATGGACGTCTGGGGCCAGGGAACAACTGTCACCGTCTCCTCA | 10 | | 8669 |
| JH6_185 | CACTACTACTATTATTATGGGATGGACGTATGGGGCCAGGGAACAACTGTCACCGTCTCCTCA | 10 | | 8670 |
| JH6_186 | TTCTATTATTATTATTACGGAATGGACGTCTGGGGCCAGGGAACAACTGTCACCGTCTCCTCA | 10 | | 8671 |
| JH6_187 | AGCTACTATTACTACTATGGGATGGATGTGTGGGGCCAGGGAACAACTGTCACCGTCTCCTCA | 10 | | 8672 |
| JH6_185 | AGATATTACTATTACTATGGTATGGACGTTTGGGGCCAGGGAACAACTGTCACCGTCTCCTCA | 10 | | 8673 |
| JH6_189 | TTGTACTATTACTATTATGGAATGGACGTTTGGGGCCAGGGAACAACTGTCACCGTCTCCTCA | 10 | | 8674 |
| JH6_190 | CCTTACTATTATTATTATGGGATGGATGTCTGGGGCCAGGGAACAACTGTCACCGTCTCCTCA | 10 | | 8675 |
| JH6_191 | ATATATTACTATTACTACGGGATGGATGTTTGGGGCCAGGGAACAACTGTCACCGTCTCCTCA | 10 | | 8676 |
| JH6_192 | ACCTACTATTATTATTACGGGATGGACGTTTGGGGCCAGGGAACAACTGTCACCGTCTCCTCA | 10 | | 8677 |
| JH6_193 | GGTTACTATTATTACTACGGGATGGACGTGTGGGGCCAGGGAACAACTGTCACCGTCTCCTCA | 10 | | 8678 |
| JH6_194 | GTGTATTACTATTACTACGGGATGGACGTGTGGGGCCAGGGAACAACTGTCACCGTCTCCTCA | 10 | | 8679 |
| JH6_195 | GCCTATTACTACTACTATGGGATGGATGTGTGGGGCCAGGGAACAACTGTCACCGTCTCCTCA | 10 | | 8680 |
| JH6_196 | AATTATTATTACTATTACGGTATGGACGTGTGGGGCCAGGGAACAACTGTCACCGTCTCCTCA | 10 | | 8681 |
| JH6_197 | KACTACTACTACTACTACTACATGGACGTATGGGGCAAGGGTACAACTGTCACCGTCTCCTCA | 10 | YES | 8682 |

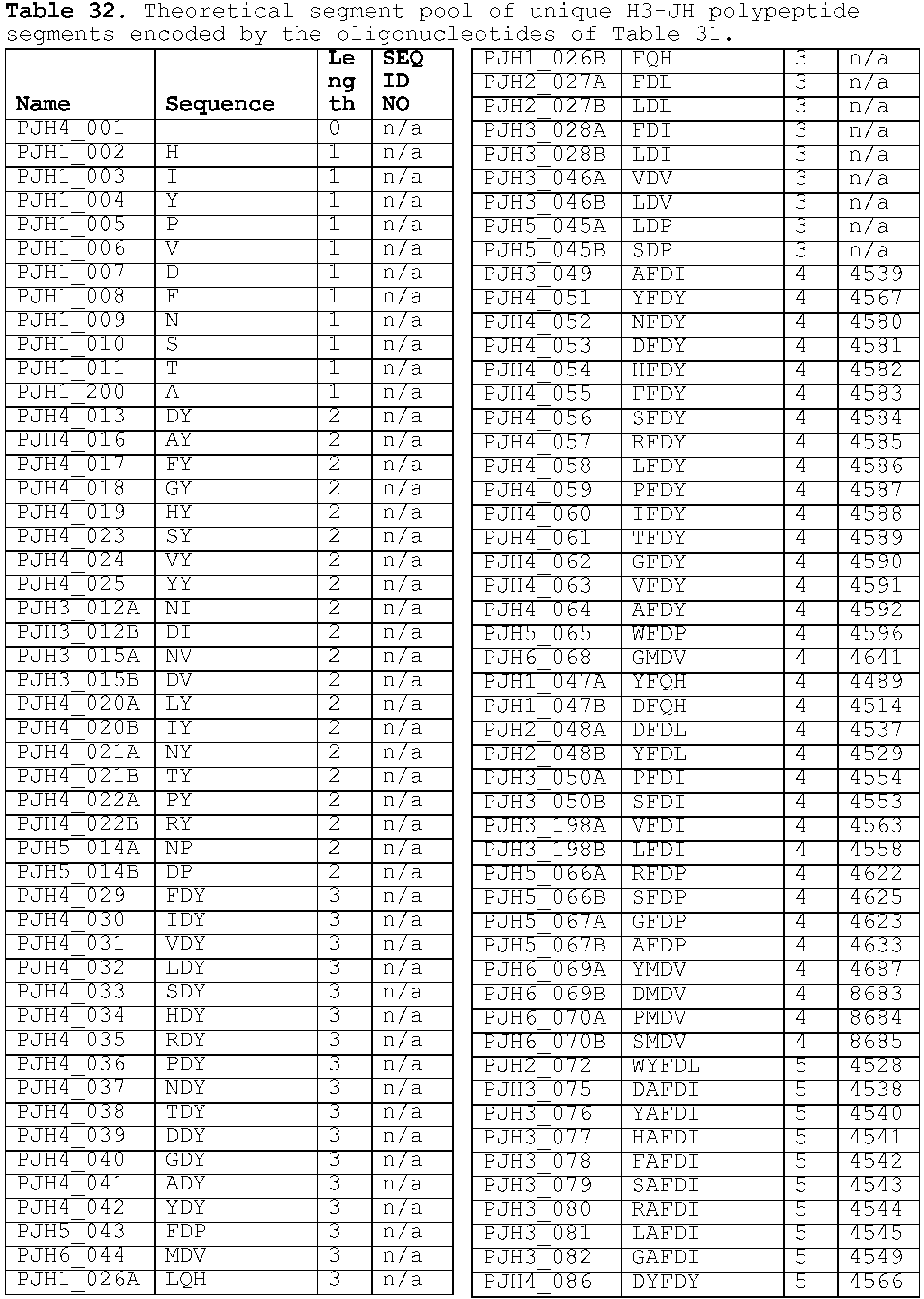

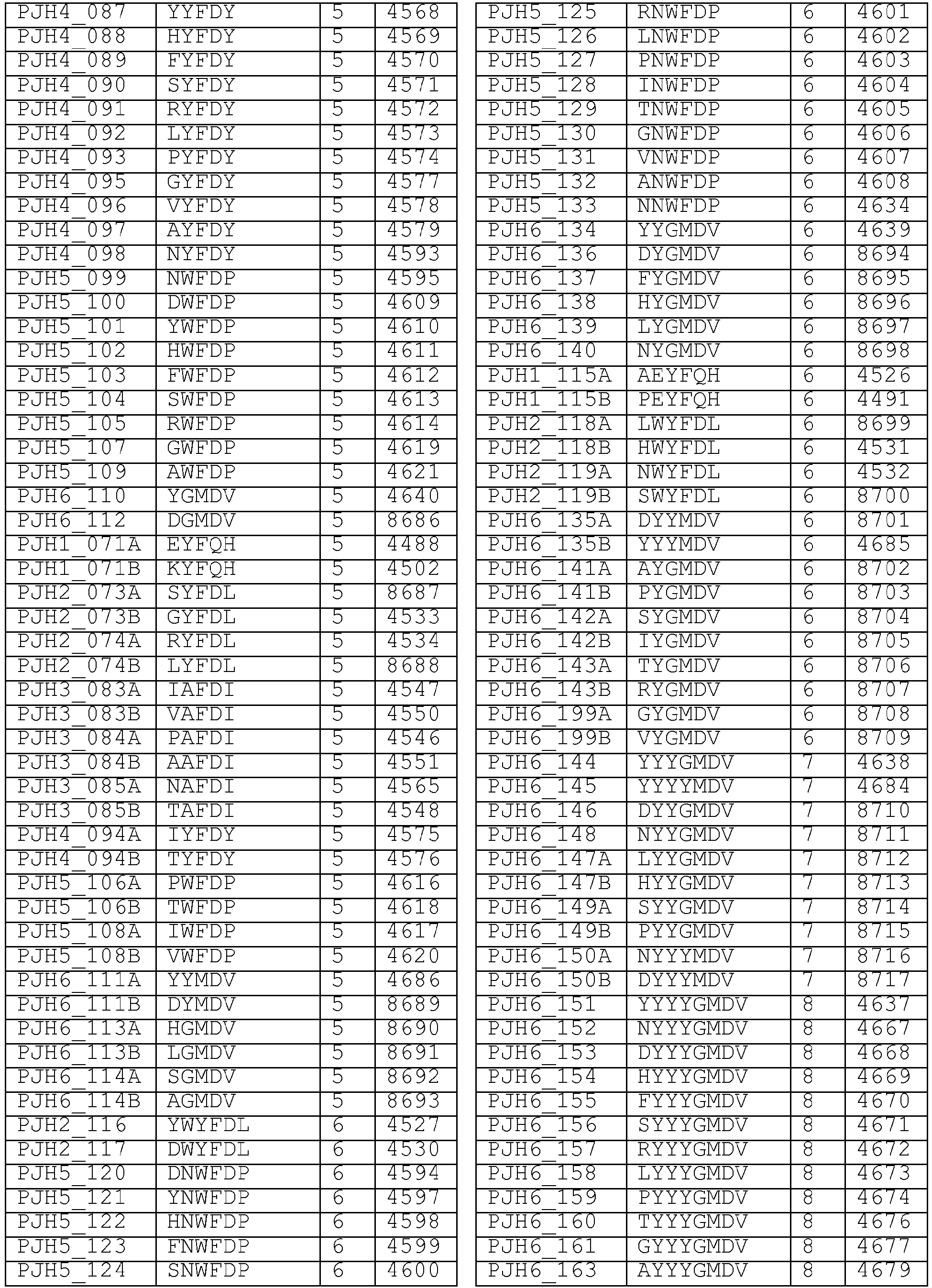

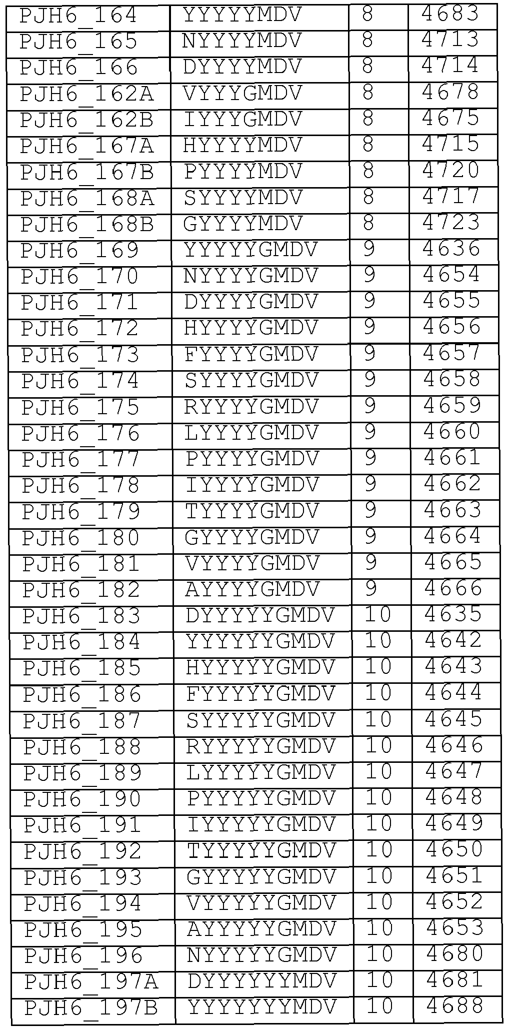

### APPENDIX A

**GI NUMBERS OF 3,571 SEQUENCES IN THE HEALTHY PREIMMUNE SET (HPS)**

| | | | | | |
|---|---|---|---|---|---|
| 33628 | 587317 | 1154839 | 1770747 | 1770837 | 1770918 |
| 37745 | 1052611 | 1154840 | 1770751 | 1770839 | 1770922 |
| 37747 | 1052620 | 1154841 | 1770755 | 1770840 | 1770932 |
| 37749 | 1052622 | 1154843 | 1770756 | 1770843 | 1770936 |
| 37751 | 1052626 | 1154844 | 1770758 | 1770844 | 1770937 |
| 37753 | 1052627 | 1154845 | 1770759 | 1770845 | 1770950 |
| 37755 | 1052634 | 1154847 | 1770761 | 1770846 | 1770952 |
| 37757 | 1052637 | 1154848 | 1770763 | 1770847 | 1770954 |
| 37759 | 1052639 | 1197299 | 1770765 | 1770848 | 1770958 |
| 37761 | 1052640 | 1197300 | 1770766 | 1770851 | 1770961 |
| 37763 | 1052642 | 1197304 | 1770770 | 1770852 | 1770962 |
| 37765 | 1052644 | 1197307 | 1770771 | 1770853 | 1770963 |
| 37767 | 1052655 | 1197308 | 1770772 | 1770854 | 1770964 |
| 37769 | 1052656 | 1197309 | 1770775 | 1770855 | 1770967 |
| 37773 | 1052657 | 1197312 | 1770776 | 1770860 | 1770969 |
| 37777 | 1052658 | 1197313 | 1770777 | 1770861 | 1770971 |
| 38383 | 1052659 | 1197314 | 1770779 | 1770865 | 1770972 |
| 38391 | 1052662 | 1197315 | 1770780 | 1770866 | 1770974 |
| 38393 | 1052668 | 1197316 | 1770783 | 1770867 | 1770976 |
| 38397 | 1052669 | 1197318 | 1770784 | 1770869 | 1770979 |
| 38401 | 1052671 | 1197319 | 1770785 | 1770870 | 1770981 |
| 185292 | 1052674 | 1197321 | 1770789 | 1770872 | 1770982 |
| 264183 | 1052676 | 1197322 | 1770791 | 1770874 | 1770983 |
| 297147 | 1052683 | 1197323 | 1770792 | 1770875 | 1770989 |
| 306949 | 1052685 | 1197324 | 1770793 | 1770876 | 1770992 |
| 306951 | 1052691 | 1197325 | 1770794 | 1770877 | 1770994 |
| 306953 | 1052692 | 1197326 | 1770795 | 1770878 | 1770995 |
| 483332 | 1052693 | 1197327 | 1770796 | 1770879 | 1770997 |
| 483333 | 1052695 | 1197328 | 1770797 | 1770880 | 1770998 |
| 483335 | 1154682 | 1495508 | 1770799 | 1770881 | 1771002 |
| 483336 | 1154691 | 1495511 | 1770800 | 1770882 | 1771004 |
| 483338 | 1154698 | 1495512 | 1770801 | 1770883 | 1771008 |
| 483339 | 1154699 | 1495516 | 1770805 | 1770884 | 1771010 |
| 483348 | 1154706 | 1495518 | 1770806 | 1770885 | 1771014 |
| 483350 | 1154710 | 1592729 | 1770807 | 1770887 | 1771016 |
| 510999 | 1154713 | 1685210 | 1770808 | 1770888 | 1771017 |
| 547164 | 1154715 | 1685220 | 1770809 | 1770891 | 1771018 |
| 587252 | 1154724 | 1685222 | 1770810 | 1770892 | 1771022 |
| 587254 | 1154754 | 1685228 | 1770811 | 1770893 | 1771026 |
| 587266 | 1154769 | 1685234 | 1770812 | 1770894 | 1771027 |
| 587276 | 1154770 | 1685238 | 1770813 | 1770895 | 1771029 |
| 587278 | 1154805 | 1685240 | 1770814 | 1770896 | 1771033 |
| 587280 | 1154807 | 1685242 | 1770815 | 1770898 | 1771034 |
| 587286 | 1154808 | 1685246 | 1770816 | 1770902 | 1771035 |
| 587288 | 1154809 | 1685248 | 1770817 | 1770904 | 1771036 |
| 587291 | 1154810 | 1685250 | 1770818 | 1770905 | 1771038 |
| 587293 | 1154811 | 1685252 | 1770820 | 1770906 | 1771039 |
| 587295 | 1154813 | 1685254 | 1770822 | 1770908 | 1771042 |
| 587299 | 1154818 | 1685256 | 1770824 | 1770909 | 1771044 |
| 587301 | 1154820 | 1685258 | 1770826 | 1770910 | 1771045 |
| 587304 | 1154822 | 1685260 | 1770829 | 1770911 | 1771055 |
| 587306 | 1154824 | 1685264 | 1770830 | 1770912 | 1771057 |
| 587308 | 1154825 | 1685266 | 1770831 | 1770913 | 1771058 |
| 587311 | 1154834 | 1685268 | 1770833 | 1770914 | 1771059 |
| 587313 | 1154837 | 1770744 | 1770835 | 1770915 | 1771060 |
| 587315 | 1154838 | 1770746 | 1770836 | 1770916 | 1771061 |
| 1771063 | 1791190 | 3170726 | 3170884 | 4995317 | 4995484 |
| 1791008 | 1791194 | 3170728 | 3170890 | 4995319 | 4995486 |
| 1791010 | 1791196 | 3170730 | 3170894 | 4995321 | 4995488 |
| 1791012 | 1791200 | 3170734 | 3170898 | 4995323 | 4995490 |
| 1791018 | 1791204 | 3170736 | 3170902 | 4995325 | 4995492 |
| 1791020 | 1791206 | 3170738 | 3170908 | 4995327 | 4995494 |
| 1791026 | 1869905 | 3170740 | 3170910 | 4995329 | 4995496 |
| 1791028 | 1869907 | 3170748 | 3170916 | 4995331 | 4995498 |
| 1791030 | 1869912 | 3170752 | 3170918 | 4995333 | 4995500 |
| 1791032 | 1869913 | 3170754 | 3170922 | 4995335 | 4995502 |
| 1791034 | 1869915 | 3170756 | 3170924 | 4995337 | 4995504 |
| 1791036 | 1869918 | 3170758 | 3170926 | 4995339 | 4995506 |
| 1791040 | 1869919 | 3170760 | 3170930 | 4995341 | 4995508 |
| 1791042 | 1934921 | 3170762 | 3170932 | 4995343 | 4995510 |
| 1791046 | 2367538 | 3170764 | 3170934 | 4995345 | 4995512 |
| 1791050 | 2388836 | 3170766 | 3170936 | 4995347 | 4995514 |
| 1791052 | 2388837 | 3170768 | 3170938 | 4995349 | 4995516 |
| 1791054 | 2388839 | 3170772 | 3170944 | 4995351 | 4995520 |
| 1791058 | 2388840 | 3170774 | 3170946 | 4995353 | 4995524 |
| 1791060 | 2388841 | 3170778 | 3170954 | 4995355 | 4995530 |
| 1791062 | 2388842 | 3170782 | 3170958 | 4995357 | 4995535 |
| 1791064 | 2388843 | 3170784 | 3170960 | 4995359 | 4995537 |
| 1791072 | 2388846 | 3170786 | 3170964 | 4995361 | 4995539 |
| 1791074 | 2388847 | 3170788 | 3170966 | 4995365 | 4995549 |
| 1791076 | 2388848 | 3170794 | 3170968 | 4995367 | 4995555 |
| 1791078 | 2388851 | 3170796 | 3170970 | 4995375 | 4995557 |
| 1791080 | 2388852 | 3170802 | 3170974 | 4995383 | 4995563 |
| 1791082 | 2388853 | 3170808 | 3170978 | 4995385 | 4995569 |
| 1791084 | 2388856 | 3170810 | 3170980 | 4995389 | 4995575 |
| 1791086 | 2388859 | 3170812 | 3170984 | 4995391 | 4995581 |
| 1791088 | 2388861 | 3170816 | 3170986 | 4995393 | 4995589 |
| 1791090 | 2388862 | 3170820 | 3170988 | 4995397 | 4995591 |
| 1791096 | 2388863 | 3170822 | 3170990 | 4995399 | 5833973 |
| 1791098 | 2388864 | 3170824 | 3170992 | 4995400 | 5833980 |
| 1791100 | 2388865 | 3170826 | 3171006 | 4995404 | 5833984 |
| 1791104 | 2388868 | 3170830 | 3171008 | 4995406 | 5833986 |
| 1791106 | 2388871 | 3170832 | 3171010 | 4995408 | 5834003 |
| 1791108 | 2388873 | 3170834 | 3171016 | 4995410 | 5834011 |
| 1791110 | 2388875 | 3170836 | 3171018 | 4995418 | 5834015 |
| 1791112 | 2388876 | 3170840 | 3171020 | 4995422 | 5834019 |
| 1791114 | 2388878 | 3170842 | 3171022 | 4995426 | 5834031 |
| 1791116 | 2773082 | 3170844 | 3171024 | 4995428 | 5834035 |
| 1791118 | 3170658 | 3170846 | 3171026 | 4995430 | 5834037 |
| 1791122 | 3170662 | 3170848 | 3171028 | 4995432 | 5834039 |
| 1791124 | 3170664 | 3170852 | 3171030 | 4995434 | 5834041 |
| 1791130 | 3170668 | 3170854 | 3171038 | 4995436 | 5834043 |
| 1791132 | 3170670 | 3170856 | 3171040 | 4995438 | 5834047 |
| 1791134 | 3170686 | 3170858 | 3171042 | 4995440 | 5834049 |
| 1791136 | 3170688 | 3170862 | 3171044 | 4995442 | 5834051 |
| 1791142 | 3170692 | 3170864 | 3171242 | 4995446 | 5834053 |
| 1791144 | 3170694 | 3170866 | 3608440 | 4995456 | 5834055 |
| 1791152 | 3170696 | 3170868 | 3608462 | 4995462 | 5834057 |
| 1791154 | 3170702 | 3170870 | 3954953 | 4995466 | 5834059 |
| 1791160 | 3170704 | 3170872 | 3954955 | 4995470 | 5834065 |
| 1791164 | 3170712 | 3170874 | 4530538 | 4995474 | 5834069 |
| 1791176 | 3170714 | 3170876 | 4530544 | 4995476 | 5834071 |
| 1791182 | 3170716 | 3170878 | 4753741 | 4995478 | 5834073 |
| 1791184 | 3170720 | 3170880 | 4959477 | 4995480 | 5834075 |
| 1791186 | 3170722 | 3170882 | 4995315 | 4995482 | 5834077 |
| 5834079 | 6013043 | 8249622 | 11137253 | 11137428 | 13172003 |
| 5834081 | 6013045 | 8249632 | 11137261 | 11137430 | 13172005 |
| 5834083 | 6531445 | 8249650 | 11137262 | 11137432 | 13172007 |
| 5834089 | 6531457 | 8249652 | 11137274 | 11137439 | 13172009 |
| 5834091 | 6531461 | 8249654 | 11137276 | 11137441 | 13172013 |
| 5834093 | 6531465 | 8249656 | 11137279 | 11137445 | 13172019 |
| 5834095 | 6531481 | 8249662 | 11137281 | 11137448 | 13172021 |
| 5834097 | 6531489 | 8249674 | 11137283 | 11137450 | 13172025 |
| 5834099 | 6531493 | 8249682 | 11137285 | 11137452 | 13172027 |
| 5834101 | 6531495 | 8249698 | 11137289 | 11137454 | 13172033 |
| 5834103 | 6531507 | 8249712 | 11137290 | 11137460 | 13172037 |
| 5834105 | 6531509 | 8249716 | 11137293 | 11137462 | 13172043 |
| 5834107 | 6531511 | 8249718 | 11137295 | 11137467 | 13172045 |
| 5834109 | 6531513 | 8249730 | 11137298 | 11137470 | 13172053 |
| 5834113 | 6531517 | 8249738 | 11137301 | 11137474 | 13172061 |
| 5834115 | 6531521 | 8249740 | 11137303 | 11137476 | 13172065 |
| 5834119 | 6531525 | 8249744 | 11137305 | 11137480 | 13172069 |
| 5834121 | 6531533 | 8249754 | 11137307 | 11137482 | 13172073 |
| 5834123 | 6531537 | 8249756 | 11137309 | 11137487 | 13172083 |
| 5834125 | 6531539 | 8249760 | 11137313 | 11137494 | 13172091 |
| 5834127 | 6531554 | 8249772 | 11137315 | 11137500 | 13172093 |
| 5834129 | 6531600 | 8249778 | 11137317 | 11137502 | 13172099 |
| 5834131 | 6723523 | 8249784 | 11137319 | 11137507 | 13172117 |
| 5834133 | 6723525 | 8249786 | 11137322 | 11137509 | 13172125 |
| 5834135 | 6723527 | 8249788 | 11137326 | 13171905 | 13172129 |
| 5834137 | 6723531 | 8249790 | 11137329 | 13171909 | 13172133 |
| 5834139 | 6723535 | 8249812 | 11137333 | 13171911 | 13172135 |
| 5834141 | 6723537 | 8249816 | 11137335 | 13171913 | 13172137 |
| 5834143 | 6723543 | 8249826 | 11137339 | 13171915 | 13172141 |
| 5834145 | 6723545 | 8249828 | 11137343 | 13171917 | 13172143 |
| 5834147 | 6723549 | 8249838 | 11137348 | 13171921 | 13172147 |
| 5834149 | 6723551 | 8250248 | 11137350 | 13171923 | 13172149 |
| 5834151 | 6723558 | 8250255 | 11137352 | 13171925 | 13172151 |
| 5834153 | 6723565 | 8489274 | 11137354 | 13171927 | 13172155 |
| 5834155 | 6723581 | 8489276 | 11137359 | 13171929 | 13172157 |
| 5834159 | 6723583 | 8489278 | 11137361 | 13171931 | 13172159 |
| 5834161 | 6723595 | 8489280 | 11137363 | 13171935 | 13172163 |
| 5834163 | 6723597 | 8489282 | 11137365 | 13171937 | 13172169 |
| 5834165 | 6723599 | 8489284 | 11137367 | 13171939 | 13172177 |
| 5834169 | 7161042 | 8489286 | 11137369 | 13171941 | 13623574 |
| 5834175 | 7161061 | 8489289 | 11137371 | 13171945 | 14289029 |
| 5834177 | 7161129 | 8489291 | 11137373 | 13171947 | 14289035 |
| 5834179 | 7161136 | 11137164 | 11137375 | 13171949 | 14289037 |
| 5834183 | 7161164 | 11137170 | 11137377 | 13171951 | 14289049 |
| 5834185 | 8249510 | 11137172 | 11137379 | 13171953 | 14289057 |
| 5834187 | 8249514 | 11137174 | 11137382 | 13171955 | 14289061 |
| 5834191 | 8249518 | 11137178 | 11137386 | 13171957 | 14289065 |
| 5834193 | 8249524 | 11137183 | 11137388 | 13171959 | 14289067 |
| 5834195 | 8249528 | 11137186 | 11137392 | 13171961 | 14289071 |
| 5834197 | 8249538 | 11137188 | 11137399 | 13171965 | 14289073 |
| 5834199 | 8249546 | 11137196 | 11137403 | 13171967 | 14289079 |
| 5834201 | 8249552 | 11137200 | 11137407 | 13171969 | 14289097 |
| 5834203 | 8249554 | 11137205 | 11137411 | 13171971 | 14289099 |
| 5834205 | 8249558 | 11137215 | 11137413 | 13171973 | 14289109 |
| 5834207 | 8249560 | 11137219 | 11137415 | 13171975 | 14289111 |
| 5834209 | 8249562 | 11137229 | 11137418 | 13171977 | 16075408 |
| 5834213 | 8249566 | 11137231 | 11137420 | 13171981 | 16075410 |
| 5834215 | 8249568 | 11137242 | 11137422 | 13171987 | 16075412 |
| 6013039 | 8249608 | 11137251 | 11137426 | 13171999 | 16075414 |
| 16075416 | 37987904 | 47846534 | 47846768 | 54779192 | 54779324 |
| 16075418 | 37987932 | 47846538 | 47846778 | 54779194 | 54779328 |
| 16075420 | 37987938 | 47846540 | 47846782 | 54779196 | 54779330 |
| 16075422 | 37987960 | 47846542 | 47846784 | 54779198 | 54779332 |
| 16075424 | 37987970 | 47846544 | 47846786 | 54779200 | 54779334 |
| 16075426 | 39644659 | 47846546 | 49256420 | 54779204 | 54779336 |
| 16075428 | 39645530 | 47846548 | 49256426 | 54779206 | 54779338 |
| 16075430 | 47846366 | 47846550 | 49258105 | 54779208 | 54779340 |
| 16075432 | 47846370 | 47846558 | 49523831 | 54779210 | 54779342 |
| 16075434 | 47846372 | 47846562 | 49523833 | 54779212 | 54779344 |
| 16075436 | 47846376 | 47846564 | 49523835 | 54779214 | 54779350 |
| 16075438 | 47846378 | 47846566 | 49523837 | 54779218 | 54779354 |
| 16075440 | 47846380 | 47846570 | 49523841 | 54779220 | 54779356 |
| 16075442 | 47846386 | 47846572 | 49523843 | 54779222 | 54779358 |
| 16075444 | 47846388 | 47846574 | 49523849 | 54779224 | 54779360 |
| 16075448 | 47846394 | 47846578 | 49523851 | 54779226 | 54779362 |
| 16075450 | 47846398 | 47846580 | 49523853 | 54779228 | 54779364 |
| 16075452 | 47846416 | 47846582 | 49523855 | 54779230 | 54780155 |
| 16075454 | 47846418 | 47846586 | 49523861 | 54779232 | 54780163 |
| 16075456 | 47846420 | 47846588 | 49523865 | 54779234 | 54780167 |
| 16075458 | 47846422 | 47846590 | 49523871 | 54779236 | 54780171 |
| 16075460 | 47846426 | 47846594 | 49523873 | 54779238 | 54780177 |
| 16075464 | 47846428 | 47846596 | 49523879 | 54779240 | 54780179 |
| 16075466 | 47846430 | 47846598 | 49523881 | 54779242 | 54780185 |
| 16076270 | 47846432 | 47846600 | 49523887 | 54779244 | 54780187 |
| 16076286 | 47846434 | 47846602 | 49523895 | 54779248 | 54780191 |
| 17511791 | 47846438 | 47846604 | 49523905 | 54779250 | 54780193 |
| 18044958 | 47846442 | 47846606 | 49523919 | 54779252 | 54780209 |
| 19171939 | 47846446 | 47846612 | 49523921 | 54779256 | 54780211 |
| 19550754 | 47846448 | 47846614 | 49523923 | 54779258 | 54780213 |
| 19848531 | 47846450 | 47846618 | 49523927 | 54779260 | 54780227 |
| 19848533 | 47846456 | 47846620 | 49523929 | 54779262 | 54780229 |
| 19848543 | 47846458 | 47846626 | 49523931 | 54779264 | 54780235 |
| 19848545 | 47846466 | 47846632 | 49523946 | 54779266 | 54780237 |
| 21702275 | 47846468 | 47846644 | 49523950 | 54779268 | 54780239 |
| 21702277 | 47846472 | 47846646 | 54779136 | 54779270 | 54780243 |
| 21702281 | 47846476 | 47846658 | 54779140 | 54779272 | 54780247 |
| 21702282 | 47846478 | 47846660 | 54779142 | 54779274 | 54780251 |
| 21702287 | 47846482 | 47846664 | 54779144 | 54779276 | 54780253 |
| 21702289 | 47846484 | 47846666 | 54779146 | 54779278 | 54780259 |
| 21702291 | 47846486 | 47846674 | 54779148 | 54779280 | 54780709 |
| 21702293 | 47846488 | 47846678 | 54779150 | 54779282 | 54780711 |
| 21702295 | 47846490 | 47846680 | 54779152 | 54779284 | 54780713 |
| 21702297 | 47846492 | 47846684 | 54779156 | 54779286 | 54780715 |
| 21702299 | 47846494 | 47846690 | 54779158 | 54779288 | 54780717 |
| 21702301 | 47846498 | 47846692 | 54779160 | 54779290 | 54780719 |
| 21702303 | 47846506 | 47846696 | 54779162 | 54779292 | 54780721 |
| 21702305 | 47846508 | 47846698 | 54779166 | 54779296 | 54780723 |
| 21702307 | 47846510 | 47846716 | 54779168 | 54779298 | 54780731 |
| 21702309 | 47846512 | 47846718 | 54779170 | 54779300 | 54780733 |
| 21702311 | 47846514 | 47846724 | 54779172 | 54779302 | 54780735 |
| 21702313 | 47846516 | 47846728 | 54779174 | 54779306 | 54780741 |
| 21702314 | 47846518 | 47846730 | 54779178 | 54779308 | 54780745 |
| 21702315 | 47846520 | 47846734 | 54779180 | 54779310 | 54780753 |
| 23337033 | 47846524 | 47846750 | 54779182 | 54779314 | 54780757 |
| 27370812 | 47846526 | 47846752 | 54779184 | 54779316 | 54780759 |
| 31076438 | 47846528 | 47846756 | 54779186 | 54779318 | 54780761 |
| 33873883 | 47846530 | 47846762 | 54779188 | 54779320 | 54780763 |
| 33989177 | 47846532 | 47846764 | 54779190 | 54779322 | 54780765 |
| 54780767 | 111918251 | 145911177 | 145911421 | 145911959 | 145912700 |
| 54780771 | 111918262 | 145911180 | 145911427 | 145911968 | 145912707 |
| 54780775 | 111918647 | 145911183 | 145911436 | 145911983 | 145912717 |
| 54780777 | 121488404 | 145911186 | 145911442 | 145911992 | 145912725 |
| 54780779 | 145910925 | 145911190 | 145911451 | 145912001 | 145912735 |
| 54780781 | 145910934 | 145911193 | 145911457 | 145912009 | 145912744 |
| 54780783 | 145910938 | 145911199 | 145911466 | 145912023 | 145912753 |
| 54780785 | 145910942 | 145911202 | 145911482 | 145912037 | 145912760 |
| 54780787 | 145910945 | 145911205 | 145911491 | 145912044 | 145912780 |
| 54780791 | 145910949 | 145911214 | 145911498 | 145912059 | 145912790 |
| 54780793 | 145910952 | 145911217 | 145911502 | 145912100 | 145912799 |
| 54780795 | 145910955 | 145911220 | 145911510 | 145912107 | 145912814 |
| 54780801 | 145910958 | 145911223 | 145911517 | 145912114 | 145912824 |
| 54780803 | 145910966 | 145911226 | 145911523 | 145912123 | 145912844 |
| 54780805 | 145910969 | 145911235 | 145911536 | 145912132 | 145912853 |
| 54780807 | 145910972 | 145911238 | 145911544 | 145912152 | 145912861 |
| 54780809 | 145910975 | 145911248 | 145911553 | 145912167 | 145912868 |
| 54780815 | 145910983 | 145911257 | 145911561 | 145912176 | 145912879 |
| 54780817 | 145910986 | 145911287 | 145911568 | 145912186 | 145912888 |
| 54780821 | 145910989 | 145911291 | 145911576 | 145912211 | 145912898 |
| 54780825 | 145910992 | 145911294 | 145911585 | 145912220 | 145912909 |
| 54780827 | 145910995 | 145911298 | 145911597 | 145912229 | 145912919 |
| 54780831 | 145910998 | 145911301 | 145911604 | 145912238 | 145912930 |
| 54780833 | 145911001 | 145911305 | 145911611 | 145912249 | 145912940 |
| 54780835 | 145911004 | 145911308 | 145911618 | 145912260 | 145912949 |
| 54780837 | 145911013 | 145911311 | 145911621 | 145912278 | 145912958 |
| 54780839 | 145911017 | 145911314 | 145911655 | 145912308 | 145912978 |
| 54780841 | 145911020 | 145911317 | 145911663 | 145912353 | 145912996 |
| 54780843 | 145911023 | 145911320 | 145911679 | 145912361 | 145913026 |
| 54780845 | 145911026 | 145911323 | 145911687 | 145912371 | 145913035 |
| 54780847 | 145911029 | 145911326 | 145911695 | 145912381 | 145913042 |
| 54780853 | 145911032 | 145911329 | 145911703 | 145912399 | 145913066 |
| 54780857 | 145911038 | 145911332 | 145911713 | 145912409 | 145913107 |
| 54780859 | 145911041 | 145911335 | 145911722 | 145912418 | 145913138 |
| 54780861 | 145911044 | 145911338 | 145911746 | 145912436 | 145913155 |
| 54780863 | 145911047 | 145911341 | 145911748 | 145912446 | 145913181 |
| 55228577 | 145911050 | 145911344 | 145911750 | 145912456 | 145913209 |
| 55228579 | 145911053 | 145911347 | 145911752 | 145912466 | 145913219 |
| 55228584 | 145911061 | 145911350 | 145911754 | 145912470 | 145913232 |
| 55228638 | 145911064 | 145911353 | 145911768 | 145912479 | 145913236 |
| 55228640 | 145911072 | 145911356 | 145911786 | 145912495 | 145913241 |
| 55228646 | 145911075 | 145911359 | 145911795 | 145912504 | 145913248 |
| 55228650 | 145911081 | 145911362 | 145911823 | 145912508 | 145913257 |
| 55228651 | 145911086 | 145911365 | 145911832 | 145912528 | 145913268 |
| 55228652 | 145911090 | 145911368 | 145911840 | 145912547 | 145913278 |
| 60688113 | 145911092 | 145911371 | 145911849 | 145912566 | 145913288 |
| 74095346 | 145911096 | 145911374 | 145911857 | 145912575 | 145913297 |
| 74095348 | 145911102 | 145911377 | 145911883 | 145912587 | 145913308 |
| 74095350 | 145911105 | 145911384 | 145911892 | 145912589 | 145913344 |
| 74095355 | 145911108 | 145911388 | 145911914 | 145912591 | 145913354 |
| 74095358 | 145911111 | 145911391 | 145911936 | 145912595 | 145913377 |
| 91979763 | 145911133 | 145911394 | 145911938 | 145912598 | 145913386 |
| 91979789 | 145911150 | 145911397 | 145911940 | 145912614 | 145913394 |
| 91979839 | 145911156 | 145911400 | 145911942 | 145912624 | 145913404 |
| 91979849 | 145911159 | 145911403 | 145911944 | 145912635 | 145913415 |
| 111918091 | 145911162 | 145911407 | 145911946 | 145912647 | 145913425 |
| 111918116 | 145911165 | 145911410 | 145911948 | 145912674 | 145913433 |
| 111918127 | 145911171 | 145911413 | 145911950 | 145912682 | 145913470 |
| 111918184 | 145911174 | 145911416 | 145911953 | 145912691 | 145913480 |
| 145913489 | 145913988 | 145938755 | 145939449 | 145940052 | 145940636 |
| 145913518 | 145913991 | 145938771 | 145939459 | 145940070 | 145940645 |
| 145913528 | 145913995 | 145938808 | 145939470 | 145940091 | 145940650 |
| 145913539 | 145914000 | 145938830 | 145939475 | 145940115 | 145940656 |
| 145913549 | 145914004 | 145938837 | 145939484 | 145940124 | 145940662 |
| 145913569 | 145914011 | 145938865 | 145939501 | 145940133 | 145940675 |
| 145913578 | 145914017 | 145938874 | 145939514 | 145940152 | 145940681 |
| 145913588 | 145914020 | 145938892 | 145939566 | 145940167 | 145940700 |
| 145913596 | 145914023 | 145938899 | 145939578 | 145940173 | 145940706 |
| 145913608 | 145914026 | 145938906 | 145939586 | 145940190 | 145940711 |
| 145913620 | 145914038 | 145938916 | 145939593 | 145940218 | 145940727 |
| 145913640 | 145914045 | 145938926 | 145939602 | 145940226 | 145940735 |
| 145913650 | 145914049 | 145938944 | 145939609 | 145940239 | 145940742 |
| 145913660 | 145914056 | 145938952 | 145939634 | 145940269 | 145940748 |
| 145913670 | 145914060 | 145938969 | 145939643 | 145940316 | 145940756 |
| 145913682 | 145914063 | 145938986 | 145939651 | 145940325 | 145940762 |
| 145913687 | 145938277 | 145938995 | 145939657 | 145940332 | 145940774 |
| 145913722 | 145938293 | 145939005 | 145939670 | 145940340 | 145940783 |
| 145913730 | 145938315 | 145939023 | 145939678 | 145940354 | 145940789 |
| 145913746 | 145938332 | 145939030 | 145939686 | 145940362 | 145940797 |
| 145913752 | 145938348 | 145939044 | 145939694 | 145940370 | 145940804 |
| 145913757 | 145938356 | 145939053 | 145939699 | 145940379 | 145940818 |
| 145913766 | 145938362 | 145939061 | 145939704 | 145940387 | 145940825 |
| 145913772 | 145938375 | 145939069 | 145939707 | 145940399 | 145940832 |
| 145913777 | 145938384 | 145939083 | 145939711 | 145940404 | 145940838 |
| 145913782 | 145938391 | 145939085 | 145939718 | 145940411 | 145940846 |
| 145913787 | 145938403 | 145939087 | 145939724 | 145940416 | 145940853 |
| 145913792 | 145938411 | 145939093 | 145939730 | 145940428 | 145940858 |
| 145913797 | 145938421 | 145939095 | 145939738 | 145940439 | 145940865 |
| 145913803 | 145938426 | 145939097 | 145939747 | 145940441 | 145940877 |
| 145913808 | 145938430 | 145939106 | 145939753 | 145940445 | 145940884 |
| 145913813 | 145938438 | 145939132 | 145939760 | 145940448 | 145940891 |
| 145913840 | 145938446 | 145939147 | 145939766 | 145940450 | 145940902 |
| 145913852 | 145938454 | 145939155 | 145939768 | 145940452 | 145940907 |
| 145913856 | 145938462 | 145939161 | 145939770 | 145940456 | 145940912 |
| 145913867 | 145938470 | 145939169 | 145939776 | 145940461 | 145940917 |
| 145913875 | 145938490 | 145939181 | 145939778 | 145940468 | 145940921 |
| 145913879 | 145938504 | 145939189 | 145939782 | 145940482 | 145940926 |
| 145913883 | 145938513 | 145939197 | 145939788 | 145940489 | 145940940 |
| 145913888 | 145938531 | 145939206 | 145939805 | 145940494 | 145941075 |
| 145913893 | 145938537 | 145939215 | 145939817 | 145940498 | 145941079 |
| 145913898 | 145938553 | 145939231 | 145939824 | 145940508 | 145941083 |
| 145913902 | 145938562 | 145939237 | 145939834 | 145940510 | 145941090 |
| 145913915 | 145938570 | 145939252 | 145939844 | 145940515 | 145941097 |
| 145913919 | 145938577 | 145939271 | 145939858 | 145940520 | 145941111 |
| 145913921 | 145938596 | 145939285 | 145939865 | 145940530 | 145941118 |
| 145913923 | 145938621 | 145939302 | 145939872 | 145940535 | 145941131 |
| 145913927 | 145938629 | 145939309 | 145939879 | 145940541 | 145941134 |
| 145913929 | 145938639 | 145939317 | 145939900 | 145940547 | 145941137 |
| 145913932 | 145938647 | 145939331 | 145939910 | 145940552 | 145941143 |
| 145913943 | 145938674 | 145939338 | 145939921 | 145940557 | 145941151 |
| 145913955 | 145938680 | 145939346 | 145939940 | 145940567 | 145941158 |
| 145913961 | 145938689 | 145939356 | 145939949 | 145940573 | 145941167 |
| 145913965 | 145938698 | 145939367 | 145939970 | 145940583 | 145941176 |
| 145913969 | 145938706 | 145939384 | 145939986 | 145940593 | 145941194 |
| 145913973 | 145938713 | 145939392 | 145940002 | 145940597 | 145941226 |
| 145913977 | 145938721 | 145939407 | 145940029 | 145940602 | 145941231 |
| 145913980 | 145938730 | 145939416 | 145940036 | 145940613 | 145941239 |
| 145913984 | 145938737 | 145939432 | 145940043 | 145940631 | 145941247 |
| 145941255 | 145941854 | 159034223 | 159034307 | 159034392 | 159034476 |
| 145941262 | 145941863 | 159034224 | 159034308 | 159034393 | 159034479 |
| 145941276 | 145941877 | 159034225 | 159034309 | 159034395 | 159034481 |
| 145941296 | 145941886 | 159034226 | 159034310 | 159034396 | 159034482 |
| 145941328 | 145941908 | 159034227 | 159034311 | 159034397 | 159034484 |
| 145941336 | 145941915 | 159034228 | 159034313 | 159034398 | 159034485 |
| 145941349 | 145941933 | 159034230 | 159034315 | 159034399 | 159034486 |
| 145941358 | 145942086 | 159034231 | 159034316 | 159034400 | 159034490 |
| 145941365 | 145942146 | 159034233 | 159034318 | 159034402 | 159034492 |
| 145941373 | 145942158 | 159034235 | 159034320 | 159034403 | 159034493 |
| 145941380 | 145942175 | 159034236 | 159034323 | 159034404 | 159034494 |
| 145941388 | 145942206 | 159034238 | 159034324 | 159034405 | 159034495 |
| 145941393 | 145942223 | 159034239 | 159034325 | 159034408 | 159034497 |
| 145941399 | 145942261 | 159034240 | 159034328 | 159034410 | 159034499 |
| 145941425 | 145942265 | 159034241 | 159034329 | 159034414 | 159034500 |
| 145941459 | 145942309 | 159034242 | 159034330 | 159034415 | 159034501 |
| 145941466 | 145942383 | 159034243 | 159034331 | 159034417 | 159034502 |
| 145941474 | 145942405 | 159034244 | 159034335 | 159034419 | 159034503 |
| 145941483 | 145942487 | 159034245 | 159034337 | 159034420 | 159034504 |
| 145941488 | 145942497 | 159034249 | 159034339 | 159034421 | 159034511 |
| 145941499 | 145942506 | 159034250 | 159034340 | 159034422 | 159034512 |
| 145941505 | 145942509 | 159034252 | 159034341 | 159034423 | 159034515 |
| 145941512 | 145942544 | 159034253 | 159034342 | 159034424 | 159034516 |
| 145941518 | 145942565 | 159034254 | 159034343 | 159034425 | 159034518 |
| 145941539 | 145942606 | 159034258 | 159034344 | 159034426 | 159034521 |
| 145941544 | 148717962 | 159034259 | 159034345 | 159034429 | 159034522 |
| 145941550 | 148717964 | 159034260 | 159034346 | 159034430 | 159034523 |
| 145941558 | 148717966 | 159034262 | 159034347 | 159034431 | 159034524 |
| 145941571 | 148910865 | 159034266 | 159034348 | 159034433 | 159034526 |
| 145941577 | 159034187 | 159034267 | 159034349 | 159034434 | 159034527 |
| 145941588 | 159034188 | 159034268 | 159034350 | 159034435 | 159034529 |
| 145941597 | 159034189 | 159034273 | 159034354 | 159034436 | 159034530 |
| 145941605 | 159034190 | 159034274 | 159034355 | 159034438 | 159034531 |
| 145941618 | 159034191 | 159034276 | 159034356 | 159034439 | 159034532 |
| 145941634 | 159034192 | 159034277 | 159034358 | 159034440 | 159034534 |
| 145941639 | 159034193 | 159034278 | 159034359 | 159034441 | 159034535 |
| 145941644 | 159034194 | 159034279 | 159034362 | 159034443 | 159034536 |
| 145941650 | 159034195 | 159034280 | 159034364 | 159034444 | 159034537 |
| 145941657 | 159034196 | 159034282 | 159034365 | 159034445 | 159034538 |
| 145941669 | 159034197 | 159034283 | 159034366 | 159034446 | 159034539 |
| 145941674 | 159034198 | 159034284 | 159034367 | 159034447 | 159034540 |
| 145941680 | 159034200 | 159034285 | 159034368 | 159034448 | 159034541 |
| 145941685 | 159034202 | 159034286 | 159034369 | 159034449 | 159034542 |
| 145941698 | 159034203 | 159034287 | 159034370 | 159034450 | 159034543 |
| 145941704 | 159034204 | 159034288 | 159034372 | 159034451 | 159034545 |
| 145941717 | 159034205 | 159034290 | 159034373 | 159034453 | 159034546 |
| 145941724 | 159034207 | 159034291 | 159034375 | 159034455 | 159034547 |
| 145941731 | 159034208 | 159034293 | 159034376 | 159034460 | 159034549 |
| 145941745 | 159034209 | 159034296 | 159034378 | 159034461 | 159034550 |
| 145941752 | 159034211 | 159034297 | 159034379 | 159034462 | 159034552 |
| 145941758 | 159034212 | 159034298 | 159034381 | 159034463 | 159034554 |
| 145941764 | 159034213 | 159034299 | 159034383 | 159034464 | 159034556 |
| 145941791 | 159034214 | 159034300 | 159034384 | 159034465 | 159034559 |
| 145941806 | 159034215 | 159034301 | 159034385 | 159034466 | 159034562 |
| 145941819 | 159034216 | 159034302 | 159034386 | 159034467 | 159034563 |
| 145941822 | 159034217 | 159034303 | 159034387 | 159034468 | 159034564 |
| 145941824 | 159034218 | 159034304 | 159034388 | 159034471 | 159034565 |
| 145941828 | 159034219 | 159034305 | 159034389 | 159034472 | 159034566 |
| 145941837 | 159034222 | 159034306 | 159034390 | 159034474 | 159034568 |
| 159034570 | 159034664 | 159034747 | 159034827 | 159034905 | 159034988 |
| 159034571 | 159034665 | 159034748 | 159034828 | 159034906 | 159034989 |
| 159034572 | 159034668 | 159034749 | 159034829 | 159034907 | 159034991 |
| 159034573 | 159034669 | 159034750 | 159034831 | 159034908 | 159034992 |
| 159034575 | 159034672 | 159034751 | 159034832 | 159034909 | 159034993 |
| 159034576 | 159034673 | 159034752 | 159034833 | 159034910 | 159034995 |
| 159034578 | 159034676 | 159034754 | 159034834 | 159034911 | 159034996 |
| 159034580 | 159034677 | 159034756 | 159034835 | 159034912 | 159034997 |
| 159034581 | 159034678 | 159034758 | 159034837 | 159034913 | 159034998 |
| 159034582 | 159034679 | 159034759 | 159034838 | 159034914 | 159035002 |
| 159034584 | 159034680 | 159034760 | 159034839 | 159034917 | 159035003 |
| 159034587 | 159034681 | 159034762 | 159034840 | 159034918 | 159035005 |
| 159034588 | 159034683 | 159034763 | 159034842 | 159034919 | 159035007 |
| 159034589 | 159034686 | 159034764 | 159034843 | 159034920 | 159035008 |
| 159034591 | 159034687 | 159034765 | 159034844 | 159034923 | 159035009 |
| 159034596 | 159034688 | 159034766 | 159034845 | 159034925 | 159035010 |
| 159034599 | 159034689 | 159034767 | 159034846 | 159034926 | 159035013 |
| 159034600 | 159034690 | 159034768 | 159034847 | 159034928 | 159035014 |
| 159034601 | 159034692 | 159034771 | 159034848 | 159034929 | 159035015 |
| 159034602 | 159034693 | 159034773 | 159034849 | 159034931 | 159035016 |
| 159034604 | 159034695 | 159034774 | 159034852 | 159034934 | 159035017 |
| 159034607 | 159034696 | 159034778 | 159034853 | 159034935 | 159035018 |
| 159034609 | 159034697 | 159034779 | 159034856 | 159034936 | 159035021 |
| 159034611 | 159034698 | 159034780 | 159034858 | 159034937 | 159035022 |
| 159034612 | 159034699 | 159034781 | 159034859 | 159034938 | 159035023 |
| 159034613 | 159034700 | 159034782 | 159034860 | 159034939 | 159035024 |
| 159034617 | 159034701 | 159034783 | 159034861 | 159034940 | 159035025 |
| 159034619 | 159034704 | 159034784 | 159034862 | 159034942 | 159035026 |
| 159034620 | 159034705 | 159034786 | 159034863 | 159034945 | 159035027 |
| 159034621 | 159034706 | 159034787 | 159034864 | 159034946 | 159035028 |
| 159034622 | 159034708 | 159034788 | 159034866 | 159034947 | 159035031 |
| 159034625 | 159034709 | 159034789 | 159034869 | 159034948 | 159035033 |
| 159034626 | 159034710 | 159034790 | 159034871 | 159034950 | 159035034 |
| 159034628 | 159034711 | 159034791 | 159034872 | 159034952 | 159035035 |
| 159034629 | 159034712 | 159034793 | 159034874 | 159034953 | 159035036 |
| 159034631 | 159034713 | 159034794 | 159034876 | 159034954 | 159035038 |
| 159034632 | 159034714 | 159034795 | 159034877 | 159034955 | 159035039 |
| 159034634 | 159034717 | 159034796 | 159034879 | 159034957 | 159035040 |
| 159034635 | 159034718 | 159034798 | 159034880 | 159034959 | 159035041 |
| 159034636 | 159034720 | 159034799 | 159034882 | 159034961 | 159035042 |
| 159034637 | 159034721 | 159034801 | 159034883 | 159034962 | 159035043 |
| 159034638 | 159034722 | 159034802 | 159034885 | 159034963 | 159035044 |
| 159034640 | 159034725 | 159034804 | 159034886 | 159034964 | 159035045 |
| 159034641 | 159034726 | 159034805 | 159034887 | 159034965 | 159035046 |
| 159034642 | 159034728 | 159034809 | 159034888 | 159034967 | 159035047 |
| 159034643 | 159034729 | 159034811 | 159034889 | 159034970 | 159035049 |
| 159034647 | 159034730 | 159034812 | 159034890 | 159034971 | 159035050 |
| 159034648 | 159034731 | 159034813 | 159034892 | 159034973 | 159035051 |
| 159034649 | 159034732 | 159034815 | 159034893 | 159034974 | 159035052 |
| 159034650 | 159034733 | 159034816 | 159034894 | 159034975 | 159035053 |
| 159034651 | 159034734 | 159034817 | 159034895 | 159034976 | 159035054 |
| 159034652 | 159034736 | 159034818 | 159034897 | 159034978 | 159035055 |
| 159034653 | 159034737 | 159034819 | 159034898 | 159034980 | 159035056 |
| 159034654 | 159034738 | 159034820 | 159034899 | 159034981 | 159035057 |
| 159034657 | 159034741 | 159034821 | 159034900 | 159034982 | 159035058 |
| 159034658 | 159034742 | 159034822 | 159034901 | 159034983 | 159035060 |
| 159034659 | 159034743 | 159034823 | 159034902 | 159034984 | 159035061 |
| 159034660 | 159034744 | 159034824 | 159034903 | 159034985 | 159035063 |
| 159034661 | 159034745 | 159034825 | 159034904 | 159034987 | 159035064 |
| 159035065 | 159035149 | 159035242 | 159035338 | 159035445 | 159035538 |
| 159035066 | 159035150 | 159035245 | 159035342 | 159035446 | 159035541 |
| 159035067 | 159035151 | 159035247 | 159035343 | 159035447 | 159035542 |
| 159035072 | 159035152 | 159035250 | 159035344 | 159035449 | 159035546 |
| 159035073 | 159035155 | 159035253 | 159035346 | 159035450 | 159035548 |
| 159035074 | 159035158 | 159035256 | 159035348 | 159035451 | 159035549 |
| 159035075 | 159035159 | 159035257 | 159035349 | 159035452 | 159035550 |
| 159035076 | 159035160 | 159035260 | 159035350 | 159035453 | 159035552 |
| 159035079 | 159035162 | 159035261 | 159035351 | 159035454 | 159035553 |
| 159035080 | 159035163 | 159035262 | 159035353 | 159035456 | 159035554 |
| 159035081 | 159035164 | 159035263 | 159035360 | 159035457 | 159035556 |
| 159035083 | 159035166 | 159035264 | 159035361 | 159035458 | 159035557 |
| 159035084 | 159035170 | 159035265 | 159035362 | 159035459 | 159035559 |
| 159035085 | 159035172 | 159035267 | 159035363 | 159035461 | 159035560 |
| 159035086 | 159035173 | 159035268 | 159035364 | 159035463 | 159035562 |
| 159035087 | 159035174 | 159035269 | 159035366 | 159035464 | 159035563 |
| 159035089 | 159035175 | 159035270 | 159035368 | 159035466 | 159035564 |
| 159035090 | 159035176 | 159035272 | 159035369 | 159035468 | 159035565 |
| 159035092 | 159035177 | 159035273 | 159035370 | 159035470 | 159035566 |
| 159035097 | 159035179 | 159035274 | 159035372 | 159035472 | 159035567 |
| 159035100 | 159035180 | 159035275 | 159035373 | 159035473 | 159035568 |
| 159035101 | 159035181 | 159035277 | 159035374 | 159035475 | 159035569 |
| 159035102 | 159035182 | 159035279 | 159035378 | 159035476 | 159035570 |
| 159035103 | 159035183 | 159035280 | 159035380 | 159035477 | 159035571 |
| 159035104 | 159035185 | 159035282 | 159035382 | 159035479 | 159035573 |
| 159035106 | 159035186 | 159035284 | 159035384 | 159035483 | 159035574 |
| 159035108 | 159035187 | 159035285 | 159035385 | 159035484 | 159035575 |
| 159035109 | 159035188 | 159035287 | 159035386 | 159035490 | 159035576 |
| 159035110 | 159035191 | 159035288 | 159035390 | 159035492 | 159035577 |
| 159035111 | 159035192 | 159035289 | 159035391 | 159035493 | 159035581 |
| 159035112 | 159035193 | 159035290 | 159035393 | 159035496 | 159035582 |
| 159035113 | 159035194 | 159035292 | 159035394 | 159035497 | 159035583 |
| 159035114 | 159035195 | 159035293 | 159035395 | 159035498 | 159035584 |
| 159035115 | 159035199 | 159035294 | 159035400 | 159035499 | 159035585 |
| 159035116 | 159035201 | 159035296 | 159035401 | 159035501 | 159035586 |
| 159035117 | 159035204 | 159035299 | 159035402 | 159035502 | 159035589 |
| 159035118 | 159035205 | 159035302 | 159035403 | 159035503 | 159035590 |
| 159035120 | 159035206 | 159035303 | 159035404 | 159035507 | 159035591 |
| 159035122 | 159035207 | 159035305 | 159035405 | 159035508 | 159035592 |
| 159035123 | 159035208 | 159035307 | 159035406 | 159035512 | 159035593 |
| 159035124 | 159035209 | 159035308 | 159035407 | 159035513 | 159035594 |
| 159035125 | 159035210 | 159035309 | 159035408 | 159035514 | 159035595 |
| 159035126 | 159035211 | 159035310 | 159035413 | 159035515 | 159035596 |
| 159035128 | 159035212 | 159035312 | 159035418 | 159035516 | 159035597 |
| 159035129 | 159035213 | 159035313 | 159035420 | 159035519 | 159035598 |
| 159035130 | 159035215 | 159035314 | 159035422 | 159035520 | 159035602 |
| 159035131 | 159035218 | 159035315 | 159035423 | 159035523 | 159035603 |
| 159035133 | 159035221 | 159035316 | 159035424 | 159035524 | 159035604 |
| 159035134 | 159035224 | 159035318 | 159035425 | 159035525 | 159035605 |
| 159035135 | 159035226 | 159035319 | 159035426 | 159035526 | 159035609 |
| 159035136 | 159035227 | 159035320 | 159035427 | 159035527 | 159035610 |
| 159035137 | 159035230 | 159035321 | 159035430 | 159035528 | 159035611 |
| 159035138 | 159035232 | 159035323 | 159035432 | 159035529 | 159035612 |
| 159035139 | 159035234 | 159035327 | 159035433 | 159035531 | 159035614 |
| 159035140 | 159035235 | 159035329 | 159035435 | 159035532 | 159035618 |
| 159035143 | 159035238 | 159035330 | 159035436 | 159035533 | 159035619 |
| 159035145 | 159035239 | 159035331 | 159035437 | 159035535 | 159035624 |
| 159035146 | 159035240 | 159035334 | 159035439 | 159035536 | 159035626 |
| 159035147 | 159035241 | 159035335 | 159035441 | 159035537 | 159035627 |
| 159035628 | 162950138 | 162950294 | 162950375 | 162950464 | 162950564 |
| 159035630 | 162950139 | 162950295 | 162950377 | 162950465 | 162950565 |
| 159035631 | 162950140 | 162950296 | 162950381 | 162950466 | 162950566 |
| 159035632 | 162950141 | 162950297 | 162950382 | 162950467 | 162950568 |
| 159035633 | 162950143 | 162950298 | 162950384 | 162950469 | 162950570 |
| 159035634 | 162950146 | 162950299 | 162950385 | 162950470 | 162950571 |
| 159035636 | 162950147 | 162950300 | 162950386 | 162950471 | 162950572 |
| 159035637 | 162950148 | 162950301 | 162950387 | 162950474 | 162950573 |
| 159035638 | 162950152 | 162950302 | 162950388 | 162950476 | 162950574 |
| 159035640 | 162950188 | 162950303 | 162950390 | 162950481 | 162950575 |
| 159035641 | 162950189 | 162950305 | 162950391 | 162950483 | 162950577 |
| 159035642 | 162950191 | 162950306 | 162950392 | 162950484 | 162950578 |
| 159035644 | 162950199 | 162950308 | 162950393 | 162950485 | 162950579 |
| 159035646 | 162950202 | 162950309 | 162950394 | 162950487 | 162950580 |
| 159035649 | 162950204 | 162950310 | 162950396 | 162950489 | 162950581 |
| 159035650 | 162950205 | 162950311 | 162950397 | 162950490 | 162950582 |
| 159035653 | 162950210 | 162950312 | 162950398 | 162950491 | 162950583 |
| 159035655 | 162950211 | 162950313 | 162950399 | 162950492 | 162950584 |
| 159035656 | 162950213 | 162950314 | 162950400 | 162950493 | 162950585 |
| 159035658 | 162950227 | 162950315 | 162950401 | 162950494 | 162950586 |
| 159035659 | 162950241 | 162950316 | 162950404 | 162950496 | 162950587 |
| 159035661 | 162950243 | 162950317 | 162950405 | 162950498 | 162950589 |
| 159035662 | 162950244 | 162950318 | 162950406 | 162950500 | 162950590 |
| 159035663 | 162950245 | 162950319 | 162950408 | 162950503 | 162950591 |
| 159035664 | 162950248 | 162950321 | 162950410 | 162950504 | 162950592 |
| 162950025 | 162950249 | 162950323 | 162950411 | 162950514 | 162950593 |
| 162950028 | 162950250 | 162950325 | 162950416 | 162950515 | 162950594 |
| 162950034 | 162950251 | 162950326 | 162950418 | 162950516 | 162950596 |
| 162950035 | 162950252 | 162950327 | 162950419 | 162950517 | 162950597 |
| 162950039 | 162950253 | 162950328 | 162950420 | 162950518 | 162950598 |
| 162950043 | 162950254 | 162950329 | 162950421 | 162950519 | 162950599 |
| 162950050 | 162950255 | 162950330 | 162950423 | 162950520 | 162950600 |
| 162950051 | 162950257 | 162950333 | 162950425 | 162950522 | 162950602 |
| 162950052 | 162950258 | 162950334 | 162950426 | 162950525 | 162950604 |
| 162950054 | 162950260 | 162950335 | 162950428 | 162950526 | 162950605 |
| 162950055 | 162950261 | 162950337 | 162950430 | 162950527 | 162950606 |
| 162950057 | 162950263 | 162950338 | 162950431 | 162950528 | 162950609 |
| 162950065 | 162950265 | 162950339 | 162950432 | 162950529 | 162950610 |
| 162950067 | 162950266 | 162950341 | 162950433 | 162950530 | 162950611 |
| 162950077 | 162950267 | 162950342 | 162950434 | 162950531 | 162950613 |
| 162950097 | 162950269 | 162950343 | 162950435 | 162950532 | 162950614 |
| 162950098 | 162950270 | 162950344 | 162950437 | 162950534 | 162950615 |
| 162950100 | 162950272 | 162950345 | 162950438 | 162950535 | 162950617 |
| 162950111 | 162950273 | 162950347 | 162950439 | 162950536 | 162950618 |
| 162950114 | 162950275 | 162950348 | 162950440 | 162950537 | 162950619 |
| 162950115 | 162950277 | 162950350 | 162950441 | 162950539 | 162950620 |
| 162950120 | 162950278 | 162950352 | 162950442 | 162950540 | 162950621 |
| 162950121 | 162950279 | 162950353 | 162950444 | 162950542 | 162950622 |
| 162950123 | 162950282 | 162950355 | 162950445 | 162950543 | 162950626 |
| 162950125 | 162950284 | 162950359 | 162950446 | 162950546 | 162950627 |
| 162950126 | 162950285 | 162950360 | 162950447 | 162950547 | 162950628 |
| 162950127 | 162950286 | 162950361 | 162950449 | 162950551 | 162950629 |
| 162950128 | 162950287 | 162950363 | 162950450 | 162950552 | 162950631 |
| 162950129 | 162950288 | 162950365 | 162950451 | 162950555 | 162950632 |
| 162950130 | 162950289 | 162950367 | 162950453 | 162950556 | 162950633 |
| 162950132 | 162950290 | 162950369 | 162950454 | 162950557 | 162950634 |
| 162950133 | 162950291 | 162950372 | 162950456 | 162950558 | 162950635 |
| 162950134 | 162950292 | 162950373 | 162950461 | 162950559 | 162950640 |
| 162950137 | 162950293 | 162950374 | 162950463 | 162950562 | 162950641 |
| 162950642 | | | | | |
| 162950644 | | | | | |
| 162950645 | | | | | |
| 162950646 | | | | | |
| 162950647 | | | | | |
| 162950649 | | | | | |
| 162950650 | | | | | |
| 162950651 | | | | | |
| 162950652 | | | | | |
| 162950654 | | | | | |
| 162950655 | | | | | |
| 162950656 | | | | | |
| 162950659 | | | | | |
| 162950660 | | | | | |
| 162950661 | | | | | |
| 162950662 | | | | | |
| 162950665 | | | | | |
| 162950666 | | | | | |
| 162950671 | | | | | |
| 162950673 | | | | | |
| 162950674 | | | | | |
| 162950675 | | | | | |
| 162950676 | | | | | |
| 162950685 | | | | | |
| 162950686 | | | | | |
| 162950688 | | | | | |
| 162950689 | | | | | |
| 162950691 | | | | | |
| 162950692 | | | | | |
| 162950693 | | | | | |
| 162950694 | | | | | |
| 162950695 | | | | | |
| 162950696 | | | | | |
| 162950697 | | | | | |
| 162950698 | | | | | |
| 162950710 | | | | | |
| 162950714 | | | | | |
| 162950716 | | | | | |
| 162950720 | | | | | |
| 162950724 | | | | | |
| 162950725 | | | | | |
| 162950726 | | | | | |
| 162950728 | | | | | |
| 162950729 | | | | | |
| 194719560 | | | | | |
| 194719575 | | | | | |
| 218454113 | | | | | |
| 218454117 | | | | | |
| 219937557 | | | | | |

| | | | |
|---|---|---|---|
| PDH 2047 | FGSYY | 5 | |
| PDH 2048 | HGSYD | 5 | |
| PDH 2049 | LGSYA | 5 | |
| PDH 2050 | HGSYA | 5 | |
| PDH 2051 | LGSYD | 5 | |
| PDH 2052 | DGSYS | 5 | |
| PDH 2053 | VGSYD | 5 | |
| PDH 2054 | VGSYY | 5 | |
| PDH 2055 | AGSYY | 5 | |
| PDH 2056 | YGSYS | 5 | |
| PDH 2057 | AGSYS | 5 | |
| PDH 2058 | AGSYD | 5 | |
| PDH 2059 | VGSYA | 5 | |
| PDH 2060 | DGSYD | 5 | |
| PDH 2061 | FGSYD | 5 | |
| PDH 2062 | DGSYA | 5 | |
| PDH 2063 | YGSYA | 5 | |
| PDH 2064 | FGSYA | 5 | |
| PDH 2065 | YGSYD | 5 | |
| PDH 2066 | LGSYY | 5 | |
| PDH 2067 | SGSYY | 5 | |
| PDH 2068 | PGSYA | 5 | |
| PDH 2069 | PGSYD | 5 | |
| PDH 2070 | YGSYY | 5 | |
| PDH 2071 | FSGSF | 5 | |
| PDH 2072 | FSGSY | 5 | |
| PDH 2073 | YSGSF | 5 | |
| PDH 2074 | YSGSS | 5 | |
| PDH 2075 | FSGSS | 5 | |
| PDH 2076 | YSGSY | 5 | |
| PDH 2077 | ASSSW | 5 | |
| PDH 2078 | VSSSS | 5 | |
| PDH 2079 | YSSSS | 5 | |
| PDH 2080 | YSSSL | 5 | |
| PDH 2081 | VSSSW | 5 | |
| PDH 2082 | ASSSL | 5 | |
| PDH 2083 | HSSSL | 5 | |
| PDH 2084 | PSSSL | 5 | |
| PDH 2085 | ASSSS | 5 | |
| PDH 2086 | HSSSW | 5 | |
| PDH 2087 | VSSSL | 5 | |
| PDH 2088 | FSSSL | 5 | |
| PDH 2089 | HSSSS | 5 | |
| PDH 2090 | FSSSW | 5 | |
| PDH 2091 | SSSSS | 5 | |
| PDH 2092 | DSSSS | 5 | |
| PDH 2093 | SSSSL | 5 | |
| PDH 2094 | FSSSS | 5 | |
| PDH 2095 | LSSSW | 5 | |
| PDH 2096 | DSSSL | 5 | |
| PDH 2097 | LSSSS | 5 | |
| PDH 2098 | LSSSL | 5 | |
| PDH 2099 | SSSSW | 5 | |
| PDH 2100 | DSSSW | 5 | |
| PDH 2101 | PSSSW | 5 | |
| PDH 2102 | PSSSS | 5 | |
| PDH 2103 | YSSSW | 5 | |
| PDH 2104 | HSSGW | 5 | |
| PDH 2105 | FSSGL | 5 | |
| PDH 2106 | SSSGW | 5 | |
| PDH 2107 | SSSGS | 5 | |
| PDH 2108 | DSSGL | 5 | |
| PDH 2109 | HSSGS | 5 | |
| PDH 2110 | FSSGW | 5 | |
| PDH 2111 | ASSGW | 5 | |
| PDH 2112 | DSSGW | 5 | |
| PDH 2113 | DSSGS | 5 | |
| PDH 2114 | ASSGL | 5 | |
| PDH 2115 | LSSGL | 5 | |
| PDH 2116 | FSSGS | 5 | |
| PDH 2117 | ASSGS | 5 | |
| PDH 2118 | PSSGS | 5 | |
| PDH 2119 | LSSGW | 5 | |
| PDH 2120 | LSSGS | 5 | |
| PDH 2121 | YSSGL | 5 | |
| PDH 2122 | VSSGS | 5 | |
| PDH 2123 | PSSGW | 5 | |
| PDH 2124 | PSSGL | 5 | |
| PDH 2125 | VSSGL | 5 | |
| PDH 2126 | VSSGW | 5 | |
| PDH 2127 | YSSGW | 5 | |
| PDH 2128 | SSSGL | 5 | |
| PDH 2129 | HSSGL | 5 | |
| PDH 2130 | YSSGS | 5 | |
| PDH 2131 | VTVTT | 5 | |
| PDH 2132 | RTVTT | 5 | |
| PDH 2133 | LTVTK | 5 | |
| PDH 2134 | ATVTK | 5 | |
| PDH 2135 | GTVTT | 5 | |
| PDH 2136 | VTVTK | 5 | |
| PDH 2137 | LTVTR | 5 | |
| PDH 2138 | ATVTT | 5 | |
| PDH 2139 | RTVTR | 5 | |
| PDH 2140 | VTVTR | 5 | |
| PDH 2141 | TTVTK | 5 | |
| PDH 2142 | GTVTR | 5 | |
| PDH 2143 | TTVTI | 5 | |
| PDH 2144 | RTVTK | 5 | |
| PDH 2145 | LTVTT | 5 | |
| PDH 2146 | VTVTI | 5 | |
| PDH 2147 | GTVTK | 5 | |
| PDH 2148 | TTVTR | 5 | |
| PDH 2149 | GTVTI | 5 | |
| PDH 2150 | PTVTI | 5 | |
| PDH 2151 | TTVTT | 5 | |
| PDH 2152 | STVTT | 5 | |
| PDH 2153 | STVTI | 5 | |
| PDH 2154 | ITVTI | 5 | |
| PDH 2155 | STVTK | 5 | |
| PDH 2156 | STVTR | 5 | |
| PDH 2157 | ATVTI | 5 | |
| PDH 2158 | ITVTT | 5 | |
| PDH 2159 | ITVTR | 5 | |
| PDH 2160 | LTVTI | 5 | |
| PDH 2161 | PTVTR | 5 | |
| PDH 2162 | ATVTR | 5 | |
| PDH 2163 | PTVTK | 5 | |
| PDH 2164 | RTVTI | 5 | |
| PDH 2165 | ITVTK | 5 | |
| PDH 2166 | PTVTT | 5 | |
| PDH 2167 | ASSSF | 5 | |
| PDH 2168 | ASSSY | 5 | |
| PDH 2169 | PSSSF | 5 | |
| PDH 2170 | HSSSF | 5 | |
| PDH 2171 | VSSSY | 5 | |
| PDH 2172 | YSSSF | 5 | |
| PDH 2173 | FSSSF | 5 | |
| PDH 2174 | HSSSY | 5 | |
| PDH 2175 | VSSSF | 5 | |
| PDH 2176 | SSSSY | 5 | |
| PDH 2177 | SSSSF | 5 | |
| PDH 2178 | LSSSY | 5 | |
| PDH 2179 | DSSSY | 5 | |
| PDH 2180 | FSSSY | 5 | |
| PDH 2181 | PSSSY | 5 | |
| PDH 2182 | YSSSY | 5 | |
| PDH 2183 | DSSSF | 5 | |
| PDH 2184 | LSSSF | 5 | |
| PDH 2185 | FSGWF | 5 | |
| PDH 2186 | FSGWS | 5 | |
| PDH 2187 | ISGWY | 5 | |
| PDH 2188 | FSGWY | 5 | |
| PDH 2189 | ISGWS | 5 | |
| PDH 2190 | PSGWS | 5 | |
| PDH 2191 | DSGWF | 5 | |
| PDH 2192 | PSGWY | 5 | |
| PDH 2193 | PSGWF | 5 | |
| PDH 2194 | TSGWY | 5 | |
| PDH 2195 | ASGWF | 5 | |
| PDH 2196 | LSGWF | 5 | |
| PDH 2197 | ISGWF | 5 | |
| PDH 2198 | SSGWY | 5 | |
| PDH 2199 | SSGWS | 5 | |
| PDH 2200 | SSGWF | 5 | |
| PDH 2201 | NSGWS | 5 | |
| PDH 2202 | NSGWY | 5 | |
| PDH 2203 | NSGWF | 5 | |
| PDH 2204 | VSGWS | 5 | |
| PDH 2205 | VSGWY | 5 | |
| PDH 2206 | YSGWF | 5 | |
| PDH 2207 | LSGWS | 5 | |
| PDH 2208 | ASGWY | 5 | |
| PDH 2209 | LSGWY | 5 | |
| PDH 2210 | TSGWF | 5 | |
| PDH 2211 | TSGWS | 5 | |
| PDH 2212 | DSGWY | 5 | |
| PDH 2213 | DSGWS | 5 | |
| PDH 2214 | ASGWS | 5 | |
| PDH 2215 | HSGWF | 5 | |
| PDH 2216 | HSGWS | 5 | |
| PDH 2217 | YSGWS | 5 | |
| PDH 2218 | HSGWY | 5 | |
| PDH 2219 | YSGWY | 5 | |
| PDH 2220 | VSGWF | 5 | |
| PDH 2221 | ISGYD | 5 | |
| PDH 2222 | HSGYA | 5 | |
| PDH 2223 | ISGYA | 5 | |
| PDH 2224 | HSGYG | 5 | |
| PDH 2225 | TSGYD | 5 | |
| PDH 2226 | TSGYA | 5 | |
| PDH 2227 | DSGYA | 5 | |
| PDH 2228 | LSGYA | 5 | |
| PDH 2229 | FSGYA | 5 | |
| PDH 2230 | LSGYD | 5 | |
| PDH 2231 | FSGYG | 5 | |
| PDH 2232 | SSGYD | 5 | |
| PDH 2233 | SSGYA | 5 | |
| PDH 2234 | ISGYG | 5 | |
| PDH 2235 | HSGYD | 5 | |
| PDH 2236 | ASGYD | 5 | |
| PDH 2237 | YSGYA | 5 | |
| PDH 2238 | YSGYD | 5 | |
| PDH 2239 | VSGYA | 5 | |
| PDH 2240 | VSGYG | 5 | |
| PDH 2241 | SSGYG | 5 | |
| PDH 2242 | DSGYD | 5 | |
| PDH 2243 | DSGYG | 5 | |
| PDH 2244 | FSGYD | 5 | |
| PDH 2245 | LSGYG | 5 | |
| PDH 2246 | TSGYG | 5 | |
| PDH 2247 | NSGYA | 5 | |
| PDH 2248 | NSGYG | 5 | |
| PDH 2249 | NSGYD | 5 | |
| PDH 2250 | PSGYA | 5 | |
| PDH 2251 | PSGYG | 5 | |
| PDH 2252 | PSGYD | 5 | |
| PDH 2253 | ASGYG | 5 | |
| PDH 2254 | VSGYD | 5 | |
| PDH 2255 | ASGYA | 5 | |
| PDH 2256 | YSGYG | 5 | |
| PDH 2257 | FYYDS | 5 | |
| PDH 2258 | YYYDT | 5 | |
| PDH 2259 | DYYDS | 5 | |
| PDH 2260 | AYYDS | 5 | |
| PDH 2261 | AYYDT | 5 | |
| PDH 2262 | DYYDI | 5 | |
| PDH 2263 | VYYDI | 5 | |
| PDH 2264 | FYYDT | 5 | |
| PDH 2265 | LYYDI | 5 | |
| PDH 2266 | PYYDI | 5 | |
| PDH 2267 | HYYDI | 5 | |
| PDH 2268 | IYYDI | 5 | |
| PDH 2269 | LYYDS | 5 | |
| PDH 2270 | SYYDI | 5 | |
| PDH 2271 | NYYDT | 5 | |
| PDH 2272 | NYYDS | 5 | |
| PDH 2273 | SYYDT | 5 | |
| PDH 2274 | AYYDI | 5 | |
| PDH 2275 | SYYDS | 5 | |
| PDH 2276 | DYYDT | 5 | |
| PDH 2277 | VYYDT | 5 | |
| PDH 2278 | YYYDI | 5 | |
| PDH 2279 | VYYDS | 5 | |
| PDH 2280 | FYYDI | 5 | |
| PDH 2281 | YYYDS | 5 | |
| PDH 2282 | TYYDT | 5 | |
| PDH 2283 | NYYDI | 5 | |
| PDH 2284 | HYYDS | 5 | |
| PDH 2285 | LYYDT | 5 | |
| PDH 2286 | IYYDT | 5 | |
| PDH 2287 | IYYDS | 5 | |
| PDH 2288 | PYYDT | 5 | |
| PDH 2289 | PYYDS | 5 | |
| PDH 2290 | TYYDI | 5 | |
| PDH 2291 | HYYDT | 5 | |
| PDH 2292 | TYYDS | 5 | |
| PDH 2293 | DSSGY | 5 | |
| PDH 2294 | SSSGY | 5 | |
| PDH 2295 | DSSGF | 5 | |
| PDH 2296 | HSSGY | 5 | |
| PDH 2297 | SSSGF | 5 | |
| PDH 2298 | ASSGF | 5 | |
| PDH 2299 | ASSGY | 5 | |
| PDH 2300 | LSSGF | 5 | |
| PDH 2301 | FSSGY | 5 | |
| PDH 2302 | FSSGF | 5 | |
| PDH 2303 | YSSGF | 5 | |
| PDH 2304 | PSSGY | 5 | |
| PDH 2305 | VSSGY | 5 | |
| PDH 2306 | LSSGY | 5 | |
| PDH 2307 | HSSGF | 5 | |
| PDH 2308 | PSSGF | 5 | |
| PDH 2309 | VSSGF | 5 | |
| PDH 2310 | YSSGY | 5 | |
| PDH 2311 | QAARH | 5 | |
| PDH 2312 | QAARP | 5 | |
| PDH 2313 | TAARR | 5 | |
| PDH 2314 | QAARL | 5 | |
| PDH 2315 | KAARR | 5 | |
| PDH 2316 | KAARP | 5 | |
| PDH 2317 | KAARH | 5 | |
| PDH 2318 | TAARH | 5 | |
| PDH 2319 | TAARP | 5 | |
| PDH 2320 | EAARL | 5 | |
| PDH 2321 | EAARP | 5 | |
| PDH 2322 | AAARL | 5 | |
| PDH 2323 | LAARL | 5 | |
| PDH 2324 | IAARL | 5 | |
| PDH 2325 | IAARH | 5 | |
| PDH 2326 | LAARR | 5 | |
| PDH 2327 | LAARH | 5 | |
| PDH 2328 | EAARH | 5 | |
| PDH 2329 | IAARR | 5 | |
| PDH 2330 | QAARR | 5 | |
| PDH 2331 | IAARP | 5 | |
| PDH 2332 | EAARR | 5 | |
| PDH 2333 | KAARL | 5 | |
| PDH 2334 | PAARR | 5 | |
| PDH 2335 | PAARP | 5 | |
| PDH 2336 | PAARH | 5 | |
| PDH 2337 | PAARL | 5 | |
| PDH 2338 | VAARR | 5 | |
| PDH 2339 | AAARH | 5 | |
| PDH 2340 | VAARP | 5 | |
| PDH 2341 | VAARH | 5 | |
| PDH 2342 | AAARP | 5 | |
| PDH 2343 | AAARR | 5 | |
| PDH 2344 | TAARL | 5 | |
| PDH 2345 | LAARP | 5 | |
| PDH 2346 | VAARL | 5 | |
| PDH 2347 | EYYYG | 5 | |
| PDH 2348 | VYYYD | 5 | |
| PDH 2349 | EYYYA | 5 | |
| PDH 2350 | VYYYG | 5 | |
| PDH 2351 | EYYYD | 5 | |
| PDH 2352 | PYYYD | 5 | |
| PDH 2353 | PYYYG | 5 | |
| PDH 2354 | AYYYG | 5 | |
| PDH 2355 | TYYYA | 5 | |
| PDH 2356 | TYYYG | 5 | |
| PDH 2357 | TYYYD | 5 | |
| PDH 2358 | QYYYA | 5 | |
| PDH 2359 | QYYYG | 5 | |
| PDH 2360 | QYYYD | 5 | |
| PDH 2361 | VYYYA | 5 | |
| PDH 2362 | LYYYG | 5 | |
| PDH 2363 | LYYYD | 5 | |
| PDH 2364 | LYYYA | 5 | |
| PDH 2365 | AYYYD | 5 | |
| PDH 2366 | AYYYA | 5 | |
| PDH 2367 | PYYYA | 5 | |
| PDH 2368 | MYYYD | 5 | |
| PDH 2369 | KYYYD | 5 | |
| PDH 2370 | KYYYG | 5 | |
| PDH 2371 | MYYYA | 5 | |
| PDH 2372 | KYYYA | 5 | |
| PDH 2373 | MYYYG | 5 | |
| PDH 2374 | LTMVR | 5 | |
| PDH 2375 | RTMVQ | 5 | |
| PDH 2376 | VTMVQ | 5 | |
| PDH 2377 | TTMVR | 5 | |
| PDH 2378 | LTMVQ | 5 | |
| PDH 2379 | STMVQ | 5 | |
| PDH 2380 | ATMVQ | 5 | |
| PDH 2381 | PTMVR | 5 | |
| PDH 2382 | RTMVR | 5 | |
| PDH 2383 | ITMVQ | 5 | |
| PDH 2384 | GTMVR | 5 | |
| PDH 2385 | VTMVR | 5 | |
| PDH 2386 | PTMVQ | 5 | |
| PDH 2387 | ITMVR | 5 | |
| PDH 2388 | ATMVR | 5 | |
| PDH 2389 | STMVR | 5 | |
| PDH 2390 | TTMVQ | 5 | |
| PDH 2391 | GTMVQ | 5 | |
| PDH 2392 | HMVQG | 5 | |
| PDH 2393 | DMVQG | 5 | |
| PDH 2394 | LMVQG | 5 | |
| PDH 2395 | SMVQG | 5 | |
| PDH 2396 | FMVQG | 5 | |
| PDH 2397 | NMVQG | 5 | |
| PDH 2398 | VMVQG | 5 | |
| PDH 2399 | TMVQG | 5 | |
| PDH 2400 | PMVQG | 5 | |
| PDH 2401 | YMVQG | 5 | |
| PDH 2402 | AMVQG | 5 | |
| PDH 2403 | IMVQG | 5 | |
| PDH 2404 | PWGSY | 5 | |
| PDH 2405 | TWGSY | 5 | |
| PDH 2406 | SWGSY | 5 | |
| PDH 2407 | HWGSY | 5 | |
| PDH 2408 | LWGSY | 5 | |
| PDH 2409 | DWGSY | 5 | |
| PDH 2410 | FWGSY | 5 | |
| PDH 2411 | AWGSY | 5 | |
| PDH 2412 | YWGSY | 5 | |
| PDH 2413 | IWGSY | 5 | |
| PDH 2414 | NWGSY | 5 | |
| PDH 2415 | VWGSY | 5 | |
| PDH 2416 | FSSSWF | 6 | |
| PDH 2417 | HSSSWS | 6 | |
| PDH 2418 | VSSSWF | 6 | |
| PDH 2419 | HSSSWY | 6 | |
| PDH 2420 | VSSSWS | 6 | |
| PDH 2421 | LSSSWY | 6 | |
| PDH 2422 | VSSSWY | 6 | |
| PDH 2423 | HSSSWF | 6 | |
| PDH 2424 | ASSSWY | 6 | |
| PDH 2425 | DSSSWS | 6 | |
| PDH 2426 | PSSSWY | 6 | |
| PDH 2427 | ASSSWS | 6 | |
| PDH 2428 | DSSSWF | 6 | |
| PDH 2429 | DSSSWY | 6 | |
| PDH 2430 | YSSSWS | 6 | |
| PDH 2431 | YSSSWF | 6 | |
| PDH 2432 | ASSSWF | 6 | |
| PDH 2433 | LSSSWS | 6 | |
| PDH 2434 | SSSSWS | 6 | |
| PDH 2435 | YSSSWY | 6 | |
| PDH 2436 | PSSSWS | 6 | |
| PDH 2437 | SSSSWY | 6 | |
| PDH 2438 | LSSSWF | 6 | |
| PDH 2439 | PSSSWF | 6 | |
| PDH 2440 | FSSSWS | 6 | |
| PDH 2441 | SSSSWF | 6 | |
| PDH 2442 | FSSSWY | 6 | |
| PDH 2443 | AYYDST | 6 | |
| PDH 2444 | AYYDSI | 6 | |
| PDH 2445 | YYYDST | 6 | |
| PDH 2446 | PYYDST | 6 | |
| PDH 2447 | VYYDSS | 6 | |
| PDH 2448 | NYYDSI | 6 | |
| PDH 2449 | PYYDSS | 6 | |
| PDH 2450 | YYYDSS | 6 | |
| PDH 2451 | YYYDSI | 6 | |
| PDH 2452 | VYYDST | 6 | |
| PDH 2453 | VYYDSI | 6 | |
| PDH 2454 | NYYDST | 6 | |
| PDH 2455 | LYYDST | 6 | |
| PDH 2456 | NYYDSS | 6 | |
| PDH 2457 | SYYDSI | 6 | |
| PDH 2458 | LYYDSS | 6 | |
| PDH 2459 | SYYDST | 6 | |
| PDH 2460 | LYYDSI | 6 | |
| PDH 2461 | SYYDSS | 6 | |
| PDH 2462 | DYYDSI | 6 | |
| PDH 2463 | DYYDST | 6 | |
| PDH 2464 | DYYDSS | 6 | |
| PDH 2465 | FYYDSS | 6 | |
| PDH 2466 | TYYDSI | 6 | |
| PDH 2467 | FYYDST | 6 | |
| PDH 2468 | IYYDST | 6 | |
| PDH 2469 | FYYDSI | 6 | |
| PDH 2470 | IYYDSS | 6 | |
| PDH 2471 | TYYDSS | 6 | |
| PDH 2472 | TYYDST | 6 | |
| PDH 2473 | HYYDSS | 6 | |
| PDH 2474 | HYYDST | 6 | |
| PDH 2475 | IYYDSI | 6 | |
| PDH 2476 | HYYDSI | 6 | |
| PDH 2477 | PYYDSI | 6 | |
| PDH 2478 | AYYDSS | 6 | |
| PDH 2479 | LYSGYA | 6 | |
| PDH 2480 | GYSGYV | 6 | |
| PDH 2481 | SYSGYD | 6 | |
| PDH 2482 | LYSGYD | 6 | |
| PDH 2483 | IYSGYV | 6 | |
| PDH 2484 | SYSGYG | 6 | |
| PDH 2485 | IYSGYG | 6 | |
| PDH 2486 | SYSGYA | 6 | |
| PDH 2487 | PYSGYG | 6 | |
| PDH 2488 | VYSGYD | 6 | |
| PDH 2489 | PYSGYA | 6 | |
| PDH 2490 | VYSGYV | 6 | |
| PDH 2491 | GYSGYG | 6 | |
| PDH 2492 | VYSGYA | 6 | |
| PDH 2493 | PYSGYV | 6 | |
| PDH 2494 | GYSGYA | 6 | |
| PDH 2495 | IYSGYD | 6 | |
| PDH 2496 | PYSGYD | 6 | |
| PDH 2497 | RYSGYV | 6 | |
| PDH 2498 | RYSGYA | 6 | |
| PDH 2499 | RYSGYG | 6 | |
| PDH 2500 | GYSGYD | 6 | |
| PDH 2501 | IYSGYA | 6 | |
| PDH 2502 | AYSGYA | 6 | |
| PDH 2503 | RYSGYD | 6 | |
| PDH 2504 | AYSGYG | 6 | |
| PDH 2505 | AYSGYD | 6 | |
| PDH 2506 | AYSGYV | 6 | |
| PDH 2507 | VYSGYG | 6 | |
| PDH 2508 | TYSGYV | 6 | |
| PDH 2509 | TYSGYD | 6 | |
| PDH 2510 | TYSGYA | 6 | |
| PDH 2511 | TYSGYG | 6 | |
| PDH 2512 | SYSGYV | 6 | |
| PDH 2513 | LYSGYV | 6 | |
| PDH 2514 | LYSGYG | 6 | |
| PDH 2515 | SSSGWF | 6 | |
| PDH 2516 | PSSGWY | 6 | |
| PDH 2517 | FSSGWF | 6 | |
| PDH 2518 | PSSGWS | 6 | |
| PDH 2519 | FSSGWS | 6 | |
| PDH 2520 | ASSGWY | 6 | |
| PDH 2521 | FSSGWY | 6 | |
| PDH 2522 | ASSGWS | 6 | |
| PDH 2523 | SSSGWS | 6 | |
| PDH 2524 | YSSGWY | 6 | |
| PDH 2525 | SSSGWY | 6 | |
| PDH 2526 | HSSGWS | 6 | |
| PDH 2527 | DSSGWS | 6 | |
| PDH 2528 | YSSGWS | 6 | |
| PDH 2529 | LSSGWF | 6 | |
| PDH 2530 | HSSGWY | 6 | |
| PDH 2531 | DSSGWF | 6 | |
| PDH 2532 | DSSGWY | 6 | |
| PDH 2533 | LSSGWY | 6 | |
| PDH 2534 | HSSGWF | 6 | |
| PDH 2535 | VSSGWS | 6 | |
| PDH 2536 | LSSGWS | 6 | |
| PDH 2537 | VSSGWY | 6 | |
| PDH 2538 | VSSGWF | 6 | |
| PDH 2539 | PSSGWF | 6 | |
| PDH 2540 | ASSGWF | 6 | |
| PDH 2541 | YSSGWF | 6 | |
| PDH 2542 | IYYGSA | 6 | |
| PDH 2543 | PYYGSG | 6 | |
| PDH 2544 | PYYGSA | 6 | |
| PDH 2545 | PYYGSD | 6 | |
| PDH 2546 | VYYGSD | 6 | |
| PDH 2547 | AYYGSA | 6 | |
| PDH 2548 | VYYGSA | 6 | |
| PDH 2549 | NYYGSA | 6 | |
| PDH 2550 | YYYGSA | 6 | |
| PDH 2551 | AYYGSD | 6 | |
| PDH 2552 | NYYGSD | 6 | |
| PDH 2553 | YYYGSD | 6 | |
| PDH 2554 | FYYGSD | 6 | |
| PDH 2555 | NYYGSG | 6 | |
| PDH 2556 | YYYGSG | 6 | |
| PDH 2557 | FYYGSA | 6 | |
| PDH 2558 | FYYGSG | 6 | |
| PDH 2559 | AYYGSG | 6 | |
| PDH 2560 | LYYGSG | 6 | |
| PDH 2561 | DYYGSD | 6 | |
| PDH 2562 | LYYGSA | 6 | |
| PDH 2563 | TYYGSD | 6 | |
| PDH 2564 | DYYGSG | 6 | |
| PDH 2565 | DYYGSA | 6 | |
| PDH 2566 | LYYGSD | 6 | |
| PDH 2567 | HYYGSD | 6 | |
| PDH 2568 | TYYGSA | 6 | |
| PDH 2569 | TYYGSG | 6 | |
| PDH 2570 | HYYGSA | 6 | |
| PDH 2571 | SYYGSG | 6 | |
| PDH 2572 | HYYGSG | 6 | |
| PDH 2573 | SYYGSD | 6 | |
| PDH 2574 | VYYGSG | 6 | |
| PDH 2575 | SYYGSA | 6 | |
| PDH 2576 | IYYGSG | 6 | |
| PDH 2577 | IYYGSD | 6 | |
| PDH 2578 | VSSGYS | 6 | |
| PDH 2579 | VSSGYY | 6 | |
| PDH 2580 | FSSGYY | 6 | |
| PDH 2581 | ASSGYF | 6 | |
| PDH 2582 | ASSGYS | 6 | |
| PDH 2583 | FSSGYS | 6 | |
| PDH 2584 | SSSGYY | 6 | |
| PDH 2585 | ASSGYY | 6 | |
| PDH 2586 | FSSGYF | 6 | |
| PDH 2587 | HSSGYY | 6 | |
| PDH 2588 | DSSGYY | 6 | |
| PDH 2589 | HSSGYS | 6 | |
| PDH 2590 | SSSGYS | 6 | |
| PDH 2591 | DSSGYS | 6 | |
| PDH 2592 | DSSGYF | 6 | |
| PDH 2593 | HSSGYF | 6 | |
| PDH 2594 | PSSGYF | 6 | |
| PDH 2595 | SSSGYF | 6 | |
| PDH 2596 | YSSGYY | 6 | |
| PDH 2597 | YSSGYF | 6 | |
| PDH 2598 | YSSGYS | 6 | |
| PDH 2599 | PSSGYS | 6 | |
| PDH 2600 | PSSGYY | 6 | |
| PDH 2601 | LSSGYY | 6 | |
| PDH 2602 | LSSGYF | 6 | |
| PDH 2603 | LSSGYS | 6 | |
| PDH 2604 | VSSGYF | 6 | |
| PDH 2605 | TYDSSD | 6 | |
| PDH 2606 | YYDSSA | 6 | |
| PDH 2607 | YYDSSG | 6 | |
| PDH 2608 | LYDSSD | 6 | |
| PDH 2609 | LYDSSA | 6 | |
| PDH 2610 | PYDSSA | 6 | |
| PDH 2611 | LYDSSG | 6 | |
| PDH 2612 | PYDSSD | 6 | |
| PDH 2613 | VYDSSD | 6 | |
| PDH 2614 | HYDSSG | 6 | |
| PDH 2615 | VYDSSG | 6 | |
| PDH 2616 | VYDSSA | 6 | |
| PDH 2617 | PYDSSG | 6 | |
| PDH 2618 | SYDSSA | 6 | |
| PDH 2619 | FYDSSG | 6 | |
| PDH 2620 | NYDSSA | 6 | |
| PDH 2621 | SYDSSG | 6 | |
| PDH 2622 | FYDSSA | 6 | |
| PDH 2623 | NYDSSD | 6 | |
| PDH 2624 | SYDSSD | 6 | |
| PDH 2625 | FYDSSD | 6 | |
| PDH 2626 | DYDSSG | 6 | |
| PDH 2627 | AYDSSG | 6 | |
| PDH 2628 | NYDSSG | 6 | |
| PDH 2629 | IYDSSA | 6 | |
| PDH 2630 | DYDSSD | 6 | |
| PDH 2631 | IYDSSG | 6 | |
| PDH 2632 | AYDSSD | 6 | |
| PDH 2633 | DYDSSA | 6 | |
| PDH 2634 | IYDSSD | 6 | |
| PDH 2635 | HYDSSA | 6 | |
| PDH 2636 | AYDSSA | 6 | |
| PDH 2637 | HYDSSD | 6 | |
| PDH 2638 | TYDSSG | 6 | |
| PDH 2639 | TYDSSA | 6 | |
| PDH 2640 | YYDSSD | 6 | |
| PDH 2641 | TDFWSA | 6 | |
| PDH 2642 | ADFWSG | 6 | |
| PDH 2643 | PDFWSG | 6 | |
| PDH 2644 | VDFWSD | 6 | |
| PDH 2645 | TDFWSD | 6 | |
| PDH 2646 | HDFWSA | 6 | |
| PDH 2647 | HDFWSD | 6 | |
| PDH 2648 | TDFWSG | 6 | |
| PDH 2649 | LDFWSA | 6 | |
| PDH 2650 | LDFWSG | 6 | |
| PDH 2651 | HDFWSG | 6 | |
| PDH 2652 | LDFWSD | 6 | |
| PDH 2653 | FDFWSG | 6 | |
| PDH 2654 | DDFWSD | 6 | |
| PDH 2655 | VDFWSA | 6 | |
| PDH 2656 | SDFWSD | 6 | |
| PDH 2657 | SDFWSG | 6 | |
| PDH 2658 | NDFWSD | 6 | |
| PDH 2659 | SDFWSA | 6 | |
| PDH 2660 | NDFWSA | 6 | |
| PDH 2661 | NDFWSG | 6 | |
| PDH 2662 | VDFWSG | 6 | |
| PDH 2663 | IDFWSD | 6 | |
| PDH 2664 | IDFWSG | 6 | |
| PDH 2665 | FDFWSA | 6 | |
| PDH 2666 | ADFWSD | 6 | |
| PDH 2667 | IDFWSA | 6 | |
| PDH 2668 | FDFWSD | 6 | |
| PDH 2669 | DDFWSG | 6 | |
| PDH 2670 | ADFWSA | 6 | |
| PDH 2671 | YDFWSA | 6 | |
| PDH 2672 | PDFWSD | 6 | |
| PDH 2673 | YDFWSG | 6 | |
| PDH 2674 | DDFWSA | 6 | |
| PDH 2675 | PDFWSA | 6 | |
| PDH 2676 | YDFWSD | 6 | |
| PDH 2677 | DDSSGY | 6 | |
| PDH 2678 | LDSSGY | 6 | |
| PDH 2679 | HDSSGS | 6 | |
| PDH 2680 | DDSSGF | 6 | |
| PDH 2681 | DDSSGS | 6 | |
| PDH 2682 | LDSSGS | 6 | |
| PDH 2683 | HDSSGY | 6 | |
| PDH 2684 | SDSSGS | 6 | |
| PDH 2685 | SDSSGF | 6 | |
| PDH 2686 | PDSSGS | 6 | |
| PDH 2687 | SDSSGY | 6 | |
| PDH 2688 | PDSSGY | 6 | |
| PDH 2689 | ADSSGY | 6 | |
| PDH 2690 | ADSSGS | 6 | |
| PDH 2691 | ADSSGF | 6 | |
| PDH 2692 | YDSSGF | 6 | |
| PDH 2693 | VDSSGF | 6 | |
| PDH 2694 | FDSSGF | 6 | |
| PDH 2695 | VDSSGY | 6 | |
| PDH 2696 | YDSSGY | 6 | |
| PDH 2697 | FDSSGS | 6 | |
| PDH 2698 | VDSSGS | 6 | |
| PDH 2699 | YDSSGS | 6 | |
| PDH 2700 | FDSSGY | 6 | |
| PDH 2701 | LDSSGF | 6 | |
| PDH 2702 | HDSSGF | 6 | |
| PDH 2703 | PDSSGF | 6 | |
| PDH 2704 | EYFDWS | 6 | |
| PDH 2705 | QYFDWL | 6 | |
| PDH 2706 | RYFDWF | 6 | |
| PDH 2707 | AYFDWF | 6 | |
| PDH 2708 | PYFDWF | 6 | |
| PDH 2709 | PYFDWP | 6 | |
| PDH 2710 | LYFDWP | 6 | |
| PDH 2711 | EYFDWP | 6 | |
| PDH 2712 | EYFDWL | 6 | |
| PDH 2713 | RYFDWL | 6 | |
| PDH 2714 | LYFDWL | 6 | |
| PDH 2715 | PYFDWL | 6 | |
| PDH 2716 | LYFDWS | 6 | |
| PDH 2717 | QYFDWS | 6 | |
| PDH 2718 | QYFDWF | 6 | |
| PDH 2719 | VYFDWF | 6 | |
| PDH 2720 | RYFDWP | 6 | |
| PDH 2721 | AYFDWP | 6 | |
| PDH 2722 | LYFDWF | 6 | |
| PDH 2723 | AYFDWL | 6 | |
| PDH 2724 | GYFDWF | 6 | |
| PDH 2725 | GYFDWS | 6 | |
| PDH 2726 | VYFDWL | 6 | |
| PDH 2727 | VYFDWS | 6 | |
| PDH 2728 | VYFDWP | 6 | |
| PDH 2729 | RYFDWS | 6 | |
| PDH 2730 | PYFDWS | 6 | |
| PDH 2731 | QYFDWP | 6 | |
| PDH 2732 | EYFDWF | 6 | |
| PDH 2733 | AYFDWS | 6 | |
| PDH 2734 | GYFDWL | 6 | |
| PDH 2735 | GYFDWP | 6 | |
| PDH 2736 | CGSTSC | 6 | |
| PDH 2737 | CSGTSC | 6 | |
| PDH 2738 | CSSTSC | 6 | |
| PDH 2739 | CGGTSC | 6 | |
| PDH 2740 | PYYGSE | 6 | |
| PDH 2741 | VYYGSE | 6 | |
| PDH 2742 | AYYGSE | 6 | |
| PDH 2743 | NYYGSE | 6 | |
| PDH 2744 | FYYGSE | 6 | |
| PDH 2745 | YYYGSE | 6 | |
| PDH 2746 | DYYGSE | 6 | |
| PDH 2747 | TYYGSE | 6 | |
| PDH 2748 | HYYGSE | 6 | |
| PDH 2749 | LYYGSE | 6 | |
| PDH 2750 | IYYGSE | 6 | |
| PDH 2751 | SYYGSE | 6 | |
| PDH 2752 | CSSGSC | 6 | |
| PDH 2753 | CGGGSC | 6 | |
| PDH 2754 | CGSGSC | 6 | |
| PDH 2755 | CSGGSC | 6 | |
| PDH 2756 | PFWSGS | 6 | |
| PDH 2757 | DFWSGF | 6 | |
| PDH 2758 | PFWSGY | 6 | |
| PDH 2759 | AFWSGY | 6 | |
| PDH 2760 | AFWSGS | 6 | |
| PDH 2761 | AFWSGF | 6 | |
| PDH 2762 | PFWSGF | 6 | |
| PDH 2763 | VFWSGY | 6 | |
| PDH 2764 | YFWSGF | 6 | |
| PDH 2765 | IFWSGS | 6 | |
| PDH 2766 | VFWSGF | 6 | |
| PDH 2767 | VFWSGS | 6 | |
| PDH 2768 | IFWSGF | 6 | |
| PDH 2769 | IFWSGY | 6 | |
| PDH 2770 | SFWSGF | 6 | |
| PDH 2771 | YFWSGY | 6 | |
| PDH 2772 | FFWSGS | 6 | |
| PDH 2773 | YFWSGS | 6 | |
| PDH 2774 | FFWSGY | 6 | |
| PDH 2775 | LFWSGF | 6 | |
| PDH 2776 | TFWSGF | 6 | |
| PDH 2777 | NFWSGF | 6 | |
| PDH 2778 | HFWSGF | 6 | |
| PDH 2779 | NFWSGS | 6 | |
| PDH 2780 | SFWSGS | 6 | |
| PDH 2781 | TFWSGS | 6 | |
| PDH 2782 | NFWSGY | 6 | |
| PDH 2783 | DFWSGY | 6 | |
| PDH 2784 | LFWSGY | 6 | |
| PDH 2785 | HFWSGS | 6 | |
| PDH 2786 | SFWSGY | 6 | |
| PDH 2787 | TFWSGY | 6 | |
| PDH 2788 | DFWSGS | 6 | |
| PDH 2789 | LFWSGS | 6 | |
| PDH 2790 | HFWSGY | 6 | |
| PDH 2791 | FFWSGF | 6 | |
| PDH 2792 | DYYDSSG | 7 | |
| PDH 2793 | SYYDSSG | 7 | |
| PDH 2794 | IYYDSSG | 7 | |
| PDH 2795 | HYYDSSG | 7 | |
| PDH 2796 | SYYDSSA | 7 | |
| PDH 2797 | SYYDSSD | 7 | |
| PDH 2798 | IYYDSSA | 7 | |
| PDH 2799 | AYYDSSA | 7 | |
| PDH 2800 | IYYDSSD | 7 | |
| PDH 2801 | AYYDSSD | 7 | |
| PDH 2802 | HYYDSSD | 7 | |
| PDH 2803 | VYYDSSD | 7 | |
| PDH 2804 | HYYDSSA | 7 | |
| PDH 2805 | AYYDSSG | 7 | |
| PDH 2806 | VYYDSSA | 7 | |
| PDH 2807 | LYYDSSG | 7 | |
| PDH 2808 | VYYDSSG | 7 | |
| PDH 2809 | LYYDSSD | 7 | |
| PDH 2810 | FYYDSSG | 7 | |
| PDH 2811 | LYYDSSA | 7 | |
| PDH 2812 | FYYDSSD | 7 | |
| PDH 2813 | TYYDSSG | 7 | |
| PDH 2814 | PYYDSSG | 7 | |
| PDH 2815 | FYYDSSA | 7 | |
| PDH 2816 | TYYDSSD | 7 | |
| PDH 2817 | NYYDSSG | 7 | |
| PDH 2818 | TYYDSSA | 7 | |
| PDH 2819 | PYYDSSA | 7 | |
| PDH 2820 | YYYDSSG | 7 | |
| PDH 2821 | PYYDSSD | 7 | |
| PDH 2822 | NYYDSSA | 7 | |
| PDH 2823 | YYYDSSD | 7 | |
| PDH 2824 | NYYDSSD | 7 | |
| PDH 2825 | YYYDSSA | 7 | |
| PDH 2826 | DYYDSSD | 7 | |
| PDH 2827 | DYYDSSA | 7 | |
| PDH 2828 | FDILTGF | 7 | |
| PDH 2829 | FDILTGS | 7 | |
| PDH 2830 | LDILTGY | 7 | |
| PDH 2831 | YDILTGF | 7 | |
| PDH 2832 | LDILTGS | 7 | |
| PDH 2833 | SDILTGF | 7 | |
| PDH 2834 | IDILTGS | 7 | |
| PDH 2835 | PDILTGS | 7 | |
| PDH 2836 | PDILTGF | 7 | |
| PDH 2837 | IDILTGF | 7 | |
| PDH 2838 | IDILTGY | 7 | |
| PDH 2839 | PDILTGY | 7 | |
| PDH 2840 | YDILTGY | 7 | |
| PDH 2841 | DDILTGF | 7 | |
| PDH 2842 | YDILTGS | 7 | |
| PDH 2843 | HDILTGY | 7 | |
| PDH 2844 | TDILTGF | 7 | |
| PDH 2845 | VDILTGY | 7 | |
| PDH 2846 | LDILTGF | 7 | |
| PDH 2847 | VDILTGS | 7 | |
| PDH 2848 | HDILTGS | 7 | |
| PDH 2849 | ADILTGS | 7 | |
| PDH 2850 | HDILTGF | 7 | |
| PDH 2851 | NDILTGS | 7 | |
| PDH 2852 | NDILTGF | 7 | |
| PDH 2853 | ADILTGY | 7 | |
| PDH 2854 | VDILTGF | 7 | |
| PDH 2855 | TDILTGY | 7 | |
| PDH 2856 | ADILTGF | 7 | |
| PDH 2857 | NDILTGY | 7 | |
| PDH 2858 | TDILTGS | 7 | |
| PDH 2859 | SDILTGS | 7 | |
| PDH 2860 | DDILTGY | 7 | |
| PDH 2861 | SDILTGY | 7 | |
| PDH 2862 | DDILTGS | 7 | |
| PDH 2863 | FDILTGY | 7 | |
| PDH 2864 | SYDFWSA | 7 | |
| PDH 2865 | FYDFWSG | 7 | |
| PDH 2866 | IYDFWSD | 7 | |
| PDH 2867 | LYDFWSD | 7 | |
| PDH 2868 | AYDFWSD | 7 | |
| PDH 2869 | IYDFWSA | 7 | |
| PDH 2870 | PYDFWSD | 7 | |
| PDH 2871 | AYDFWSA | 7 | |
| PDH 2872 | SYDFWSG | 7 | |
| PDH 2873 | FYDFWSA | 7 | |
| PDH 2874 | PYDFWSA | 7 | |
| PDH 2875 | YYDFWSG | 7 | |
| PDH 2876 | FYDFWSD | 7 | |
| PDH 2877 | IYDFWSG | 7 | |
| PDH 2878 | PYDFWSG | 7 | |
| PDH 2879 | YYDFWSA | 7 | |
| PDH 2880 | YYDFWSD | 7 | |
| PDH 2881 | NYDFWSD | 7 | |
| PDH 2882 | VYDFWSA | 7 | |
| PDH 2883 | NYDFWSA | 7 | |
| PDH 2884 | VYDFWSD | 7 | |
| PDH 2885 | VYDFWSG | 7 | |
| PDH 2886 | NYDFWSG | 7 | |
| PDH 2887 | DYDFWSA | 7 | |
| PDH 2888 | DYDFWSD | 7 | |
| PDH 2889 | DYDFWSG | 7 | |
| PDH 2890 | HYDFWSG | 7 | |
| PDH 2891 | TYDFWSG | 7 | |
| PDH 2892 | HYDFWSA | 7 | |
| PDH 2893 | LYDFWSG | 7 | |
| PDH 2894 | HYDFWSD | 7 | |
| PDH 2895 | TYDFWSA | 7 | |
| PDH 2896 | SYDFWSD | 7 | |
| PDH 2897 | TYDFWSD | 7 | |
| PDH 2898 | LYDFWSA | 7 | |
| PDH 2899 | AYDFWSG | 7 | |
| PDH 2900 | SCSSTSC | 7 | |
| PDH 2901 | HCSSTSC | 7 | |
| PDH 2902 | PCSSTSC | 7 | |
| PDH 2903 | ACSSTSC | 7 | |
| PDH 2904 | VCSSTSC | 7 | |
| PDH 2905 | LCSSTSC | 7 | |
| PDH 2906 | YCSSTSC | 7 | |
| PDH 2907 | FCSSTSC | 7 | |
| PDH 2908 | DCSSTSC | 7 | |
| PDH 2909 | RYSSSWS | 7 | |
| PDH 2910 | VYSSSWY | 7 | |
| PDH 2911 | GYSSSWY | 7 | |
| PDH 2912 | VYSSSWS | 7 | |
| PDH 2913 | TYSSSWY | 7 | |
| PDH 2914 | TYSSSWF | 7 | |
| PDH 2915 | TYSSSWS | 7 | |
| PDH 2916 | GYSSSWF | 7 | |
| PDH 2917 | LYSSSWF | 7 | |
| PDH 2918 | AYSSSWS | 7 | |
| PDH 2919 | SYSSSWF | 7 | |
| PDH 2920 | WYSSSWS | 7 | |
| PDH 2921 | WYSSSWF | 7 | |
| PDH 2922 | MYSSSWS | 7 | |
| PDH 2923 | SYSSSWS | 7 | |
| PDH 2924 | LYSSSWY | 7 | |
| PDH 2925 | AYSSSWY | 7 | |
| PDH 2926 | AYSSSWF | 7 | |
| PDH 2927 | LYSSSWS | 7 | |
| PDH 2928 | WYSSSWY | 7 | |
| PDH 2929 | VYSSSWF | 7 | |
| PDH 2930 | SYSSSWY | 7 | |
| PDH 2931 | RYSSSWF | 7 | |
| PDH 2932 | MYSSSWF | 7 | |
| PDH 2933 | RYSSSWY | 7 | |
| PDH 2934 | MYSSSWY | 7 | |
| PDH 2935 | GYSSSWS | 7 | |
| PDH 2936 | IYDSSGY | 7 | |
| PDH 2937 | PYDSSGS | 7 | |
| PDH 2938 | VYDSSGF | 7 | |
| PDH 2939 | PYDSSGY | 7 | |
| PDH 2940 | NYDSSGS | 7 | |
| PDH 2941 | LYDSSGY | 7 | |
| PDH 2942 | NYDSSGY | 7 | |
| PDH 2943 | LYDSSGS | 7 | |
| PDH 2944 | VYDSSGY | 7 | |
| PDH 2945 | VYDSSGS | 7 | |
| PDH 2946 | LYDSSGF | 7 | |
| PDH 2947 | NYDSSGF | 7 | |
| PDH 2948 | YYDSSGY | 7 | |
| PDH 2949 | YYDSSGS | 7 | |
| PDH 2950 | DYDSSGS | 7 | |
| PDH 2951 | TYDSSGY | 7 | |
| PDH 2952 | TYDSSGF | 7 | |
| PDH 2953 | TYDSSGS | 7 | |
| PDH 2954 | YYDSSGF | 7 | |
| PDH 2955 | AYDSSGS | 7 | |
| PDH 2956 | HYDSSGY | 7 | |
| PDH 2957 | AYDSSGY | 7 | |
| PDH 2958 | HYDSSGF | 7 | |
| PDH 2959 | HYDSSGS | 7 | |
| PDH 2960 | FYDSSGF | 7 | |
| PDH 2961 | IYDSSGF | 7 | |
| PDH 2962 | DYDSSGY | 7 | |
| PDH 2963 | DYDSSGF | 7 | |
| PDH 2964 | PYDSSGF | 7 | |
| PDH 2965 | SYDSSGS | 7 | |
| PDH 2966 | SYDSSGF | 7 | |
| PDH 2967 | AYDSSGF | 7 | |
| PDH 2968 | FYDSSGY | 7 | |
| PDH 2969 | SYDSSGY | 7 | |
| PDH 2970 | IYDSSGS | 7 | |
| PDH 2971 | FYDSSGS | 7 | |
| PDH 2972 | HYYGSGI | 7 | |
| PDH 2973 | SYYGSGI | 7 | |
| PDH 2974 | AYYGSGT | 7 | |
| PDH 2975 | DYYGSGI | 7 | |
| PDH 2976 | VYYGSGT | 7 | |
| PDH 2977 | AYYGSGS | 7 | |
| PDH 2978 | VYYGSGI | 7 | |
| PDH 2979 | VYYGSGS | 7 | |
| PDH 2980 | HYYGSGS | 7 | |
| PDH 2981 | LYYGSGS | 7 | |
| PDH 2982 | HYYGSGT | 7 | |
| PDH 2983 | LYYGSGT | 7 | |
| PDH 2984 | PYYGSGS | 7 | |
| PDH 2985 | SYYGSGS | 7 | |
| PDH 2986 | NYYGSGS | 7 | |
| PDH 2987 | IYYGSGS | 7 | |
| PDH 2988 | DYYGSGT | 7 | |
| PDH 2989 | PYYGSGI | 7 | |
| PDH 2990 | PYYGSGT | 7 | |
| PDH 2991 | SYYGSGT | 7 | |
| PDH 2992 | FYYGSGS | 7 | |
| PDH 2993 | YYYGSGS | 7 | |
| PDH 2994 | NYYGSGT | 7 | |
| PDH 2995 | IYYGSGT | 7 | |
| PDH 2996 | DYYGSGS | 7 | |
| PDH 2997 | TYYGSGS | 7 | |
| PDH 2998 | FYYGSGT | 7 | |
| PDH 2999 | YYYGSGT | 7 | |
| PDH 3000 | TYYGSGT | 7 | |
| PDH 3001 | YYYGSGI | 7 | |
| PDH 3002 | FYYGSGI | 7 | |
| PDH 3003 | AYYGSGI | 7 | |
| PDH 3004 | TYYGSGI | 7 | |
| PDH 3005 | IYYGSGI | 7 | |
| PDH 3006 | LYYGSGI | 7 | |
| PDH 3007 | NYYGSGI | 7 | |
| PDH 3008 | TYSSGWF | 7 | |
| PDH 3009 | TYSSGWS | 7 | |
| PDH 3010 | VYSSGWF | 7 | |
| PDH 3011 | GYSSGWF | 7 | |
| PDH 3012 | MYSSGWS | 7 | |
| PDH 3013 | SYSSGWF | 7 | |
| PDH 3014 | MYSSGWY | 7 | |
| PDH 3015 | LYSSGWY | 7 | |
| PDH 3016 | MYSSGWF | 7 | |
| PDH 3017 | WYSSGWS | 7 | |
| PDH 3018 | WYSSGWY | 7 | |
| PDH 3019 | AYSSGWS | 7 | |
| PDH 3020 | RYSSGWF | 7 | |
| PDH 3021 | LYSSGWF | 7 | |
| PDH 3022 | AYSSGWY | 7 | |
| PDH 3023 | RYSSGWY | 7 | |
| PDH 3024 | SYSSGWY | 7 | |
| PDH 3025 | RYSSGWS | 7 | |
| PDH 3026 | GYSSGWY | 7 | |
| PDH 3027 | WYSSGWF | 7 | |
| PDH 3028 | AYSSGWF | 7 | |
| PDH 3029 | GYSSGWS | 7 | |
| PDH 3030 | VYSSGWY | 7 | |
| PDH 3031 | VYSSGWS | 7 | |
| PDH 3032 | LYSSGWS | 7 | |
| PDH 3033 | TYSSGWY | 7 | |
| PDH 3034 | SYSSGWS | 7 | |
| PDH 3035 | FCSGGSC | 7 | |
| PDH 3036 | LCSGGSC | 7 | |
| PDH 3037 | SCSGGSC | 7 | |
| PDH 3038 | YCSGGSC | 7 | |
| PDH 3039 | DCSGGSC | 7 | |
| PDH 3040 | HCSGGSC | 7 | |
| PDH 3041 | PCSGGSC | 7 | |
| PDH 3042 | ACSGGSC | 7 | |
| PDH 3043 | VCSGGSC | 7 | |
| PDH 3044 | CSGGSCH | 7 | |
| PDH 3045 | CSGGSCL | 7 | |
| PDH 3046 | CSGGSCS | 7 | |
| PDH 3047 | CSGGSCY | 7 | |
| PDH 3048 | CSGGSCP | 7 | |
| PDH 3049 | CSGGSCF | 7 | |
| PDH 3050 | CSSTSCY | 7 | |
| PDH 3051 | CSSTSCL | 7 | |
| PDH 3052 | CSSTSCF | 7 | |
| PDH 3053 | CSSTSCH | 7 | |
| PDH 3054 | CSSTSCS | 7 | |
| PDH 3055 | CSSTSCP | 7 | |
| PDH 3056 | ICGGDCF | 7 | |
| PDH 3057 | VCGGDCS | 7 | |
| PDH 3058 | DCGGDCF | 7 | |
| PDH 3059 | SCGGDCF | 7 | |
| PDH 3060 | NCGGDCY | 7 | |
| PDH 3061 | VCGGDCY | 7 | |
| PDH 3062 | SCGGDCS | 7 | |
| PDH 3063 | NCGGDCS | 7 | |
| PDH 3064 | HCGGDCF | 7 | |
| PDH 3065 | LCGGDCS | 7 | |
| PDH 3066 | LCGGDCY | 7 | |
| PDH 3067 | ICGGDCY | 7 | |
| PDH 3068 | LCGGDCF | 7 | |
| PDH 3069 | ICGGDCS | 7 | |
| PDH 3070 | VCGGDCF | 7 | |
| PDH 3071 | HCGGDCS | 7 | |
| PDH 3072 | YCGGDCF | 7 | |
| PDH 3073 | HCGGDCY | 7 | |
| PDH 3074 | FCGGDCF | 7 | |
| PDH 3075 | YCGGDCS | 7 | |
| PDH 3076 | DCGGDCS | 7 | |
| PDH 3077 | YCGGDCY | 7 | |
| PDH 3078 | DCGGDCY | 7 | |
| PDH 3079 | PCGGDCF | 7 | |
| PDH 3080 | FCGGDCS | 7 | |
| PDH 3081 | FCGGDCY | 7 | |
| PDH 3082 | ACGGDCF | 7 | |
| PDH 3083 | ACGGDCY | 7 | |
| PDH 3084 | ACGGDCS | 7 | |
| PDH 3085 | PCGGDCY | 7 | |
| PDH 3086 | PCGGDCS | 7 | |
| PDH 3087 | SCGGDCY | 7 | |
| PDH 3088 | NCGGDCF | 7 | |
| PDH 3089 | TCGGDCS | 7 | |
| PDH 3090 | TCGGDCY | 7 | |
| PDH 3091 | TCGGDCF | 7 | |
| PDH 3092 | NDFWSGF | 7 | |
| PDH 3093 | HDFWSGF | 7 | |
| PDH 3094 | SDFWSGF | 7 | |
| PDH 3095 | NDFWSGY | 7 | |
| PDH 3096 | HDFWSGS | 7 | |
| PDH 3097 | DDFWSGF | 7 | |
| PDH 3098 | HDFWSGY | 7 | |
| PDH 3099 | SDFWSGY | 7 | |
| PDH 3100 | FDFWSGF | 7 | |
| PDH 3101 | PDFWSGY | 7 | |
| PDH 3102 | IDFWSGF | 7 | |
| PDH 3103 | SDFWSGS | 7 | |
| PDH 3104 | PDFWSGS | 7 | |
| PDH 3105 | TDFWSGF | 7 | |
| PDH 3106 | LDFWSGS | 7 | |
| PDH 3107 | DDFWSGY | 7 | |
| PDH 3108 | LDFWSGY | 7 | |
| PDH 3109 | FDFWSGS | 7 | |
| PDH 3110 | ADFWSGF | 7 | |
| PDH 3111 | FDFWSGY | 7 | |
| PDH 3112 | TDFWSGS | 7 | |
| PDH 3113 | PDFWSGF | 7 | |
| PDH 3114 | VDFWSGF | 7 | |
| PDH 3115 | TDFWSGY | 7 | |
| PDH 3116 | IDFWSGY | 7 | |
| PDH 3117 | DDFWSGS | 7 | |
| PDH 3118 | YDFWSGS | 7 | |
| PDH 3119 | YDFWSGF | 7 | |
| PDH 3120 | IDFWSGS | 7 | |
| PDH 3121 | NDFWSGS | 7 | |
| PDH 3122 | YDFWSGY | 7 | |
| PDH 3123 | LDFWSGF | 7 | |
| PDH 3124 | ADFWSGY | 7 | |
| PDH 3125 | VDFWSGS | 7 | |
| PDH 3126 | ADFWSGS | 7 | |
| PDH 3127 | VDFWSGY | 7 | |
| PDH 3128 | YDSSGYS | 7 | |
| PDH 3129 | VDSSGYF | 7 | |
| PDH 3130 | VDSSGYS | 7 | |
| PDH 3131 | ADSSGYS | 7 | |
| PDH 3132 | HDSSGYF | 7 | |
| PDH 3133 | ADSSGYY | 7 | |
| PDH 3134 | DDSSGYY | 7 | |
| PDH 3135 | DDSSGYS | 7 | |
| PDH 3136 | SDSSGYS | 7 | |
| PDH 3137 | SDSSGYF | 7 | |
| PDH 3138 | PDSSGYS | 7 | |
| PDH 3139 | SDSSGYY | 7 | |
| PDH 3140 | PDSSGYY | 7 | |
| PDH 3141 | HDSSGYY | 7 | |
| PDH 3142 | PDSSGYF | 7 | |
| PDH 3143 | FDSSGYF | 7 | |
| PDH 3144 | HDSSGYS | 7 | |
| PDH 3145 | ADSSGYF | 7 | |
| PDH 3146 | DDSSGYF | 7 | |
| PDH 3147 | LDSSGYF | 7 | |
| PDH 3148 | LDSSGYY | 7 | |
| PDH 3149 | LDSSGYS | 7 | |
| PDH 3150 | FDSSGYY | 7 | |
| PDH 3151 | FDSSGYS | 7 | |
| PDH 3152 | YDSSGYF | 7 | |
| PDH 3153 | YDSSGYY | 7 | |
| PDH 3154 | VDSSGYY | 7 | |
| PDH 3155 | EYCGGDC | 7 | |
| PDH 3156 | QYCGGDC | 7 | |
| PDH 3157 | AYCGGDC | 7 | |
| PDH 3158 | IYCGGDC | 7 | |
| PDH 3159 | TYCGGDC | 7 | |
| PDH 3160 | PYCGGDC | 7 | |
| PDH 3161 | LYCGGDC | 7 | |
| PDH 3162 | VYCGGDC | 7 | |
| PDH 3163 | KYCGGDC | 7 | |
| PDH 3164 | VYYDSSGF | 8 | |
| PDH 3165 | IYYDSSGF | 8 | |
| PDH 3166 | YYYDSSGY | 8 | |
| PDH 3167 | PYYDSSGF | 8 | |
| PDH 3168 | HYYDSSGF | 8 | |
| PDH 3169 | HYYDSSGY | 8 | |
| PDH 3170 | AYYDSSGF | 8 | |
| PDH 3171 | HYYDSSGS | 8 | |
| PDH 3172 | DYYDSSGY | 8 | |
| PDH 3173 | DYYDSSGS | 8 | |
| PDH 3174 | YYYDSSGS | 8 | |
| PDH 3175 | SYYDSSGF | 8 | |
| PDH 3176 | LYYDSSGF | 8 | |
| PDH 3177 | NYYDSSGF | 8 | |
| PDH 3178 | NYYDSSGY | 8 | |
| PDH 3179 | IYYDSSGY | 8 | |
| PDH 3180 | SYYDSSGS | 8 | |
| PDH 3181 | AYYDSSGS | 8 | |
| PDH 3182 | IYYDSSGS | 8 | |
| PDH 3183 | AYYDSSGY | 8 | |
| PDH 3184 | DYYDSSGF | 8 | |
| PDH 3185 | SYYDSSGY | 8 | |
| PDH 3186 | NYYDSSGS | 8 | |
| PDH 3187 | LYYDSSGY | 8 | |
| PDH 3188 | PYYDSSGS | 8 | |
| PDH 3189 | LYYDSSGS | 8 | |
| PDH 3190 | PYYDSSGY | 8 | |
| PDH 3191 | TYYDSSGY | 8 | |
| PDH 3192 | TYYDSSGS | 8 | |
| PDH 3193 | FYYDSSGF | 8 | |
| PDH 3194 | FYYDSSGY | 8 | |
| PDH 3195 | FYYDSSGS | 8 | |
| PDH 3196 | VYYDSSGS | 8 | |
| PDH 3197 | TYYDSSGF | 8 | |
| PDH 3198 | YYYDSSGF | 8 | |
| PDH 3199 | VYYDSSGY | 8 | |
| PDH 3200 | TYYGSGSS | 8 | |
| PDH 3201 | SYYGSGSY | 8 | |
| PDH 3202 | SYYGSGSS | 8 | |
| PDH 3203 | HYYGSGSF | 8 | |
| PDH 3204 | PYYGSGSF | 8 | |
| PDH 3205 | AYYGSGSS | 8 | |
| PDH 3206 | AYYGSGSF | 8 | |
| PDH 3207 | AYYGSGSY | 8 | |
| PDH 3208 | YYYGSGSF | 8 | |
| PDH 3209 | TYYGSGSY | 8 | |
| PDH 3210 | YYYGSGSY | 8 | |
| PDH 3211 | PYYGSGSS | 8 | |
| PDH 3212 | PYYGSGSY | 8 | |
| PDH 3213 | DYYGSGSY | 8 | |
| PDH 3214 | VYYGSGSY | 8 | |
| PDH 3215 | VYYGSGSF | 8 | |
| PDH 3216 | YYYGSGSS | 8 | |
| PDH 3217 | LYYGSGSF | 8 | |
| PDH 3218 | LYYGSGSY | 8 | |
| PDH 3219 | DYYGSGSS | 8 | |
| PDH 3220 | VYYGSGSS | 8 | |
| PDH 3221 | IYYGSGSS | 8 | |
| PDH 3222 | FYYGSGSY | 8 | |
| PDH 3223 | FYYGSGSS | 8 | |
| PDH 3224 | LYYGSGSS | 8 | |
| PDH 3225 | SYYGSGSF | 8 | |
| PDH 3226 | IYYGSGSY | 8 | |
| PDH 3227 | IYYGSGSF | 8 | |
| PDH 3228 | FYYGSGSF | 8 | |
| PDH 3229 | DYYGSGSF | 8 | |
| PDH 3230 | NYYGSGSS | 8 | |
| PDH 3231 | NYYGSGSY | 8 | |
| PDH 3232 | TYYGSGSF | 8 | |
| PDH 3233 | HYYGSGSY | 8 | |
| PDH 3234 | HYYGSGSS | 8 | |
| PDH 3235 | NYYGSGSF | 8 | |
| PDH 3236 | AYCSSTSC | 8 | |
| PDH 3237 | PYCSSTSC | 8 | |
| PDH 3238 | RYCSSTSC | 8 | |
| PDH 3239 | IYCSSTSC | 8 | |
| PDH 3240 | LYCSSTSC | 8 | |
| PDH 3241 | VYCSSTSC | 8 | |
| PDH 3242 | TYCSSTSC | 8 | |
| PDH 3243 | SYCSSTSC | 8 | |
| PDH 3244 | GYCSSTSC | 8 | |
| PDH 3245 | IYCSGGSC | 8 | |
| PDH 3246 | VYCSGGSC | 8 | |
| PDH 3247 | LYCSGGSC | 8 | |
| PDH 3248 | AYCSGGSC | 8 | |
| PDH 3249 | TYCSGGSC | 8 | |
| PDH 3250 | GYCSGGSC | 8 | |
| PDH 3251 | RYCSGGSC | 8 | |
| PDH 3252 | PYCSGGSC | 8 | |
| PDH 3253 | SYCSGGSC | 8 | |
| PDH 3254 | FYDFWSGY | 8 | |
| PDH 3255 | AYDFWSGF | 8 | |
| PDH 3256 | NYDFWSGF | 8 | |
| PDH 3257 | IYDFWSGF | 8 | |
| PDH 3258 | DYDFWSGS | 8 | |
| PDH 3259 | DYDFWSGY | 8 | |
| PDH 3260 | NYDFWSGY | 8 | |
| PDH 3261 | VYDFWSGF | 8 | |
| PDH 3262 | VYDFWSGS | 8 | |
| PDH 3263 | YYDFWSGF | 8 | |
| PDH 3264 | PYDFWSGF | 8 | |
| PDH 3265 | FYDFWSGS | 8 | |
| PDH 3266 | HYDFWSGF | 8 | |
| PDH 3267 | HYDFWSGY | 8 | |
| PDH 3268 | YYDFWSGS | 8 | |
| PDH 3269 | PYDFWSGY | 8 | |
| PDH 3270 | PYDFWSGS | 8 | |
| PDH 3271 | VYDFWSGY | 8 | |
| PDH 3272 | SYDFWSGS | 8 | |
| PDH 3273 | YYDFWSGY | 8 | |
| PDH 3274 | LYDFWSGF | 8 | |
| PDH 3275 | HYDFWSGS | 8 | |
| PDH 3276 | SYDFWSGY | 8 | |
| PDH 3277 | SYDFWSGF | 8 | |
| PDH 3278 | LYDFWSGS | 8 | |
| PDH 3279 | TYDFWSGF | 8 | |
| PDH 3280 | LYDFWSGY | 8 | |
| PDH 3281 | AYDFWSGY | 8 | |
| PDH 3282 | AYDFWSGS | 8 | |
| PDH 3283 | TYDFWSGS | 8 | |
| PDH 3284 | IYDFWSGY | 8 | |
| PDH 3285 | TYDFWSGY | 8 | |
| PDH 3286 | NYDFWSGS | 8 | |
| PDH 3287 | DYDFWSGF | 8 | |
| PDH 3288 | IYDFWSGS | 8 | |
| PDH 3289 | FYDFWSGF | 8 | |
| PDH 3290 | HDILTGYS | 8 | |
| PDH 3291 | PDILTGYY | 8 | |
| PDH 3292 | PDILTGYF | 8 | |
| PDH 3293 | TDILTGYS | 8 | |
| PDH 3294 | HDILTGYY | 8 | |
| PDH 3295 | YDILTGYF | 8 | |
| PDH 3296 | IDILTGYS | 8 | |
| PDH 3297 | IDILTGYY | 8 | |
| PDH 3298 | IDILTGYF | 8 | |
| PDH 3299 | LDILTGYF | 8 | |
| PDH 3300 | LDILTGYY | 8 | |
| PDH 3301 | LDILTGYS | 8 | |
| PDH 3302 | DDILTGYF | 8 | |
| PDH 3303 | FDILTGYF | 8 | |
| PDH 3304 | SDILTGYF | 8 | |
| PDH 3305 | ADILTGYY | 8 | |
| PDH 3306 | VDILTGYY | 8 | |
| PDH 3307 | VDILTGYS | 8 | |
| PDH 3308 | ADILTGYS | 8 | |
| PDH 3309 | DDILTGYS | 8 | |
| PDH 3310 | FDILTGYS | 8 | |
| PDH 3311 | SDILTGYS | 8 | |
| PDH 3312 | DDILTGYY | 8 | |
| PDH 3313 | SDILTGYY | 8 | |
| PDH 3314 | YDILTGYY | 8 | |
| PDH 3315 | FDILTGYY | 8 | |
| PDH 3316 | HDILTGYF | 8 | |
| PDH 3317 | NDILTGYY | 8 | |
| PDH 3318 | NDILTGYF | 8 | |
| PDH 3319 | PDILTGYS | 8 | |
| PDH 3320 | VDILTGYF | 8 | |
| PDH 3321 | TDILTGYY | 8 | |
| PDH 3322 | TDILTGYF | 8 | |
| PDH 3323 | YDILTGYS | 8 | |
| PDH 3324 | NDILTGYS | 8 | |
| PDH 3325 | ADILTGYF | 8 | |
| PDH 3326 | SCSGGSCS | 8 | |
| PDH 3327 | HCSGGSCF | 8 | |
| PDH 3328 | DCSGGSCS | 8 | |
| PDH 3329 | PCSGGSCF | 8 | |
| PDH 3330 | PCSGGSCY | 8 | |
| PDH 3331 | SCSGGSCY | 8 | |
| PDH 3332 | PCSGGSCS | 8 | |
| PDH 3333 | LCSGGSCY | 8 | |
| PDH 3334 | ACSGGSCY | 8 | |
| PDH 3335 | ACSGGSCS | 8 | |
| PDH 3336 | ACSGGSCF | 8 | |
| PDH 3337 | VCSGGSCY | 8 | |
| PDH 3338 | VCSGGSCF | 8 | |
| PDH 3339 | VCSGGSCS | 8 | |
| PDH 3340 | YCSGGSCS | 8 | |
| PDH 3341 | YCSGGSCF | 8 | |
| PDH 3342 | DCSGGSCF | 8 | |
| PDH 3343 | DCSGGSCY | 8 | |
| PDH 3344 | FCSGGSCS | 8 | |
| PDH 3345 | FCSGGSCY | 8 | |
| PDH 3346 | FCSGGSCF | 8 | |
| PDH 3347 | SCSGGSCF | 8 | |
| PDH 3348 | YCSGGSCY | 8 | |
| PDH 3349 | LCSGGSCF | 8 | |
| PDH 3350 | LCSGGSCS | 8 | |
| PDH 3351 | HCSGGSCS | 8 | |
| PDH 3352 | HCSGGSCY | 8 | |
| PDH 3353 | ACSSTSCY | 8 | |
| PDH 3354 | YCSSTSCF | 8 | |
| PDH 3355 | FCSSTSCY | 8 | |
| PDH 3356 | PCSSTSCF | 8 | |
| PDH 3357 | FCSSTSCS | 8 | |
| PDH 3358 | DCSSTSCF | 8 | |
| PDH 3359 | VCSSTSCS | 8 | |
| PDH 3360 | VCSSTSCF | 8 | |
| PDH 3361 | LCSSTSCS | 8 | |
| PDH 3362 | VCSSTSCY | 8 | |
| PDH 3363 | ACSSTSCS | 8 | |
| PDH 3364 | LCSSTSCY | 8 | |
| PDH 3365 | LCSSTSCF | 8 | |
| PDH 3366 | FCSSTSCF | 8 | |
| PDH 3367 | HCSSTSCY | 8 | |
| PDH 3368 | HCSSTSCS | 8 | |
| PDH 3369 | HCSSTSCF | 8 | |
| PDH 3370 | SCSSTSCF | 8 | |
| PDH 3371 | SCSSTSCS | 8 | |
| PDH 3372 | YCSSTSCS | 8 | |
| PDH 3373 | DCSSTSCY | 8 | |
| PDH 3374 | DCSSTSCS | 8 | |
| PDH 3375 | SCSSTSCY | 8 | |
| PDH 3376 | PCSSTSCS | 8 | |
| PDH 3377 | ACSSTSCF | 8 | |
| PDH 3378 | PCSSTSCY | 8 | |
| PDH 3379 | YCSSTSCY | 8 | |
| PDH 3380 | LYCGGDCS | 8 | |
| PDH 3381 | EYCGGDCS | 8 | |
| PDH 3382 | LYCGGDCY | 8 | |
| PDH 3383 | VYCGGDCS | 8 | |
| PDH 3384 | VYCGGDCY | 8 | |
| PDH 3385 | AYCGGDCY | 8 | |
| PDH 3386 | AYCGGDCF | 8 | |
| PDH 3387 | EYCGGDCY | 8 | |
| PDH 3388 | TYCGGDCF | 8 | |
| PDH 3389 | LYCGGDCF | 8 | |
| PDH 3390 | AYCGGDCS | 8 | |
| PDH 3391 | QYCGGDCF | 8 | |
| PDH 3392 | QYCGGDCS | 8 | |
| PDH 3393 | KYCGGDCF | 8 | |
| PDH 3394 | KYCGGDCY | 8 | |
| PDH 3395 | QYCGGDCY | 8 | |
| PDH 3396 | KYCGGDCS | 8 | |
| PDH 3397 | VYCGGDCF | 8 | |
| PDH 3398 | IYCGGDCF | 8 | |
| PDH 3399 | IYCGGDCY | 8 | |
| PDH 3400 | IYCGGDCS | 8 | |
| PDH 3401 | PYCGGDCS | 8 | |
| PDH 3402 | PYCGGDCF | 8 | |
| PDH 3403 | TYCGGDCY | 8 | |
| PDH 3404 | EYCGGDCF | 8 | |
| PDH 3405 | PYCGGDCY | 8 | |
| PDH 3406 | TYCGGDCS | 8 | |
| PDH 3407 | DYDSSGYS | 8 | |
| PDH 3408 | YYDSSGYF | 8 | |
| PDH 3409 | LYDSSGYS | 8 | |
| PDH 3410 | AYDSSGYS | 8 | |
| PDH 3411 | AYDSSGYY | 8 | |
| PDH 3412 | NYDSSGYY | 8 | |
| PDH 3413 | IYDSSGYS | 8 | |
| PDH 3414 | FYDSSGYF | 8 | |
| PDH 3415 | DYDSSGYY | 8 | |
| PDH 3416 | YYDSSGYS | 8 | |
| PDH 3417 | TYDSSGYY | 8 | |
| PDH 3418 | TYDSSGYS | 8 | |
| PDH 3419 | PYDSSGYF | 8 | |
| PDH 3420 | IYDSSGYY | 8 | |
| PDH 3421 | IYDSSGYF | 8 | |
| PDH 3422 | YYDSSGYY | 8 | |
| PDH 3423 | NYDSSGYS | 8 | |
| PDH 3424 | NYDSSGYF | 8 | |
| PDH 3425 | VYDSSGYY | 8 | |
| PDH 3426 | VYDSSGYF | 8 | |
| PDH 3427 | FYDSSGYY | 8 | |
| PDH 3428 | FYDSSGYS | 8 | |
| PDH 3429 | SYDSSGYY | 8 | |
| PDH 3430 | SYDSSGYS | 8 | |
| PDH 3431 | PYDSSGYS | 8 | |
| PDH 3432 | PYDSSGYY | 8 | |
| PDH 3433 | LYDSSGYY | 8 | |
| PDH 3434 | VYDSSGYS | 8 | |
| PDH 3435 | SYDSSGYF | 8 | |
| PDH 3436 | LYDSSGYF | 8 | |
| PDH 3437 | HYDSSGYY | 8 | |
| PDH 3438 | HYDSSGYS | 8 | |
| PDH 3439 | TYDSSGYF | 8 | |
| PDH 3440 | AYDSSGYF | 8 | |
| PDH 3441 | DYDSSGYF | 8 | |
| PDH 3442 | HYDSSGYF | 8 | |
| PDH 3443 | RYYGSGSY | 8 | |
| PDH 3444 | RYYGSGSS | 8 | |
| PDH 3445 | GYYGSGSS | 8 | |
| PDH 3446 | GYYGSGSY | 8 | |
| PDH 3447 | FDFWSGYS | 8 | |
| PDH 3448 | HDFWSGYF | 8 | |
| PDH 3449 | FDFWSGYY | 8 | |
| PDH 3450 | SDFWSGYS | 8 | |
| PDH 3451 | SDFWSGYY | 8 | |
| PDH 3452 | PDFWSGYS | 8 | |
| PDH 3453 | HDFWSGYS | 8 | |
| PDH 3454 | IDFWSGYY | 8 | |
| PDH 3455 | HDFWSGYY | 8 | |
| PDH 3456 | NDFWSGYF | 8 | |
| PDH 3457 | YDFWSGYS | 8 | |
| PDH 3458 | IDFWSGYS | 8 | |
| PDH 3459 | PDFWSGYY | 8 | |
| PDH 3460 | SDFWSGYF | 8 | |
| PDH 3461 | VDFWSGYS | 8 | |
| PDH 3462 | IDFWSGYF | 8 | |
| PDH 3463 | YDFWSGYY | 8 | |
| PDH 3464 | YDFWSGYF | 8 | |
| PDH 3465 | TDFWSGYY | 8 | |
| PDH 3466 | DDFWSGYS | 8 | |
| PDH 3467 | LDFWSGYS | 8 | |
| PDH 3468 | DDFWSGYY | 8 | |
| PDH 3469 | DDFWSGYF | 8 | |
| PDH 3470 | VDFWSGYY | 8 | |
| PDH 3471 | VDFWSGYF | 8 | |
| PDH 3472 | NDFWSGYY | 8 | |
| PDH 3473 | FDFWSGYF | 8 | |
| PDH 3474 | NDFWSGYS | 8 | |
| PDH 3475 | LDFWSGYY | 8 | |
| PDH 3476 | ADFWSGYY | 8 | |
| PDH 3477 | ADFWSGYS | 8 | |
| PDH 3478 | TDFWSGYS | 8 | |
| PDH 3479 | TDFWSGYF | 8 | |
| PDH 3480 | ADFWSGYF | 8 | |
| PDH 3481 | LDFWSGYF | 8 | |
| PDH 3482 | PDFWSGYF | 8 | |
| PDH 3483 | LLRYFDWY | 8 | |
| PDH 3484 | QLRYFDWY | 8 | |
| PDH 3485 | PLRYFDWL | 8 | |
| PDH 3486 | ILRYFDWF | 8 | |
| PDH 3487 | ILRYFDWY | 8 | |
| PDH 3488 | ALRYFDWL | 8 | |
| PDH 3489 | QLRYFDWF | 8 | |
| PDH 3490 | KLRYFDWL | 8 | |
| PDH 3491 | TLRYFDWL | 8 | |
| PDH 3492 | TLRYFDWH | 8 | |
| PDH 3493 | ELRYFDWL | 8 | |
| PDH 3494 | ELRYFDWH | 8 | |
| PDH 3495 | PLRYFDWH | 8 | |
| PDH 3496 | ELRYFDWY | 8 | |
| PDH 3497 | ELRYFDWF | 8 | |
| PDH 3498 | LLRYFDWL | 8 | |
| PDH 3499 | VLRYFDWF | 8 | |
| PDH 3500 | TLRYFDWF | 8 | |
| PDH 3501 | ALRYFDWF | 8 | |
| PDH 3502 | ILRYFDWH | 8 | |
| PDH 3503 | QLRYFDWL | 8 | |
| PDH 3504 | QLRYFDWH | 8 | |
| PDH 3505 | VLRYFDWY | 8 | |
| PDH 3506 | TLRYFDWY | 8 | |
| PDH 3507 | ILRYFDWL | 8 | |
| PDH 3508 | LLRYFDWH | 8 | |
| PDH 3509 | VLRYFDWH | 8 | |
| PDH 3510 | KLRYFDWY | 8 | |
| PDH 3511 | KLRYFDWF | 8 | |
| PDH 3512 | ALRYFDWH | 8 | |
| PDH 3513 | ALRYFDWY | 8 | |
| PDH 3514 | VLRYFDWL | 8 | |
| PDH 3515 | KLRYFDWH | 8 | |
| PDH 3516 | PLRYFDWY | 8 | |
| PDH 3517 | PLRYFDWF | 8 | |
| PDH 3518 | LLRYFDWF | 8 | |
| PDH 3519 | IYYDSSGYS | 9 | |
| PDH 3520 | TYYDSSGYS | 9 | |
| PDH 3521 | HYYDSSGYY | 9 | |
| PDH 3522 | TYYDSSGYY | 9 | |
| PDH 3523 | HYYDSSGYS | 9 | |
| PDH 3524 | NYYDSSGYF | 9 | |
| PDH 3525 | AYYDSSGYS | 9 | |
| PDH 3526 | DYYDSSGYF | 9 | |
| PDH 3527 | DYYDSSGYS | 9 | |
| PDH 3528 | LYYDSSGYS | 9 | |
| PDH 3529 | NYYDSSGYS | 9 | |
| PDH 3530 | HYYDSSGYF | 9 | |
| PDH 3531 | DYYDSSGYY | 9 | |
| PDH 3532 | LYYDSSGYY | 9 | |
| PDH 3533 | IYYDSSGYY | 9 | |
| PDH 3534 | LYYDSSGYF | 9 | |
| PDH 3535 | IYYDSSGYF | 9 | |
| PDH 3536 | AYYDSSGYF | 9 | |
| PDH 3537 | AYYDSSGYY | 9 | |
| PDH 3538 | FYYDSSGYS | 9 | |
| PDH 3539 | YYYDSSGYS | 9 | |
| PDH 3540 | FYYDSSGYY | 9 | |
| PDH 3541 | FYYDSSGYF | 9 | |
| PDH 3542 | YYYDSSGYY | 9 | |
| PDH 3543 | YYYDSSGYF | 9 | |
| PDH 3544 | VYYDSSGYF | 9 | |
| PDH 3545 | PYYDSSGYY | 9 | |
| PDH 3546 | PYYDSSGYS | 9 | |
| PDH 3547 | VYYDSSGYS | 9 | |
| PDH 3548 | SYYDSSGYY | 9 | |
| PDH 3549 | NYYDSSGYY | 9 | |
| PDH 3550 | VYYDSSGYY | 9 | |
| PDH 3551 | SYYDSSGYS | 9 | |
| PDH 3552 | SYYDSSGYF | 9 | |
| PDH 3553 | TYYDSSGYF | 9 | |
| PDH 3554 | PYYDSSGYF | 9 | |
| PDH 3555 | PYCSGGSCF | 9 | |
| PDH 3556 | TYCSGGSCF | 9 | |
| PDH 3557 | IYCSGGSCF | 9 | |
| PDH 3558 | VYCSGGSCS | 9 | |
| PDH 3559 | TYCSGGSCY | 9 | |
| PDH 3560 | VYCSGGSCF | 9 | |
| PDH 3561 | TYCSGGSCS | 9 | |
| PDH 3562 | GYCSGGSCS | 9 | |
| PDH 3563 | IYCSGGSCS | 9 | |
| PDH 3564 | GYCSGGSCY | 9 | |
| PDH 3565 | IYCSGGSCY | 9 | |
| PDH 3566 | VYCSGGSCY | 9 | |
| PDH 3567 | AYCSGGSCF | 9 | |
| PDH 3568 | GYCSGGSCF | 9 | |
| PDH 3569 | LYCSGGSCF | 9 | |
| PDH 3570 | RYCSGGSCF | 9 | |
| PDH 3571 | LYCSGGSCS | 9 | |
| PDH 3572 | RYCSGGSCY | 9 | |
| PDH 3573 | AYCSGGSCS | 9 | |
| PDH 3574 | SYCSGGSCY | 9 | |
| PDH 3575 | SYCSGGSCF | 9 | |
| PDH 3576 | AYCSGGSCY | 9 | |
| PDH 3577 | SYCSGGSCS | 9 | |
| PDH 3578 | PYCSGGSCS | 9 | |
| PDH 3579 | LYCSGGSCY | 9 | |
| PDH 3580 | RYCSGGSCS | 9 | |
| PDH 3581 | PYCSGGSCY | 9 | |
| PDH 3582 | TYCSSTSCY | 9 | |
| PDH 3583 | AYCSSTSCY | 9 | |
| PDH 3584 | AYCSSTSCS | 9 | |
| PDH 3585 | RYCSSTSCS | 9 | |
| PDH 3586 | TYCSSTSCS | 9 | |
| PDH 3587 | PYCSSTSCY | 9 | |
| PDH 3588 | PYCSSTSCS | 9 | |
| PDH 3589 | RYCSSTSCY | 9 | |
| PDH 3590 | VYCSSTSCS | 9 | |
| PDH 3591 | VYCSSTSCY | 9 | |
| PDH 3592 | LYCSSTSCF | 9 | |
| PDH 3593 | LYCSSTSCY | 9 | |
| PDH 3594 | PYCSSTSCF | 9 | |
| PDH 3595 | VYCSSTSCF | 9 | |
| PDH 3596 | IYCSSTSCY | 9 | |
| PDH 3597 | IYCSSTSCS | 9 | |
| PDH 3598 | IYCSSTSCF | 9 | |
| PDH 3599 | SYCSSTSCS | 9 | |
| PDH 3600 | LYCSSTSCS | 9 | |
| PDH 3601 | SYCSSTSCY | 9 | |
| PDH 3602 | SYCSSTSCF | 9 | |
| PDH 3603 | GYCSSTSCY | 9 | |
| PDH 3604 | GYCSSTSCF | 9 | |
| PDH 3605 | GYCSSTSCS | 9 | |
| PDH 3606 | RYCSSTSCF | 9 | |
| PDH 3607 | TYCSSTSCF | 9 | |
| PDH 3608 | AYCSSTSCF | 9 | |
| PDH 3609 | IYDFWSGYY | 9 | |
| PDH 3610 | NYDFWSGYY | 9 | |
| PDH 3611 | PYDFWSGYF | 9 | |
| PDH 3612 | SYDFWSGYF | 9 | |
| PDH 3613 | VYDFWSGYF | 9 | |
| PDH 3614 | VYDFWSGYY | 9 | |
| PDH 3615 | HYDFWSGYY | 9 | |
| PDH 3616 | HYDFWSGYF | 9 | |
| PDH 3617 | HYDFWSGYS | 9 | |
| PDH 3618 | IYDFWSGYS | 9 | |
| PDH 3619 | NYDFWSGYS | 9 | |
| PDH 3620 | AYDFWSGYS | 9 | |
| PDH 3621 | SYDFWSGYS | 9 | |
| PDH 3622 | PYDFWSGYS | 9 | |
| PDH 3623 | AYDFWSGYY | 9 | |
| PDH 3624 | SYDFWSGYY | 9 | |
| PDH 3625 | PYDFWSGYY | 9 | |
| PDH 3626 | FYDFWSGYY | 9 | |
| PDH 3627 | FYDFWSGYF | 9 | |
| PDH 3628 | DYDFWSGYS | 9 | |
| PDH 3629 | FYDFWSGYS | 9 | |
| PDH 3630 | DYDFWSGYF | 9 | |
| PDH 3631 | LYDFWSGYY | 9 | |
| PDH 3632 | AYDFWSGYF | 9 | |
| PDH 3633 | LYDFWSGYS | 9 | |
| PDH 3634 | DYDFWSGYY | 9 | |
| PDH 3635 | LYDFWSGYF | 9 | |
| PDH 3636 | YYDFWSGYY | 9 | |
| PDH 3637 | TYDFWSGYY | 9 | |
| PDH 3638 | TYDFWSGYF | 9 | |
| PDH 3639 | YYDFWSGYS | 9 | |
| PDH 3640 | YYDFWSGYF | 9 | |
| PDH 3641 | TYDFWSGYS | 9 | |
| PDH 3642 | VYDFWSGYS | 9 | |
| PDH 3643 | NYDFWSGYF | 9 | |
| PDH 3644 | IYDFWSGYF | 9 | |
| PDH 3645 | FCSGGSCYS | 9 | |
| PDH 3646 | LCSGGSCYS | 9 | |
| PDH 3647 | VCSGGSCYS | 9 | |
| PDH 3648 | ACSGGSCYS | 9 | |
| PDH 3649 | ACSGGSCYY | 9 | |
| PDH 3650 | FCSGGSCYY | 9 | |
| PDH 3651 | LCSGGSCYF | 9 | |
| PDH 3652 | LCSGGSCYY | 9 | |
| PDH 3653 | VCSGGSCYY | 9 | |
| PDH 3654 | YCSGGSCYS | 9 | |
| PDH 3655 | PCSGGSCYS | 9 | |
| PDH 3656 | PCSGGSCYY | 9 | |
| PDH 3657 | PCSGGSCYF | 9 | |
| PDH 3658 | YCSGGSCYF | 9 | |
| PDH 3659 | YCSGGSCYY | 9 | |
| PDH 3660 | SCSGGSCYY | 9 | |
| PDH 3661 | HCSGGSCYF | 9 | |
| PDH 3662 | DCSGGSCYY | 9 | |
| PDH 3663 | SCSGGSCYF | 9 | |
| PDH 3664 | DCSGGSCYS | 9 | |
| PDH 3665 | HCSGGSCYS | 9 | |
| PDH 3666 | SCSGGSCYS | 9 | |
| PDH 3667 | FCSGGSCYF | 9 | |
| PDH 3668 | ACSGGSCYF | 9 | |
| PDH 3669 | DCSGGSCYF | 9 | |
| PDH 3670 | HCSGGSCYY | 9 | |
| PDH 3671 | VCSGGSCYF | 9 | |
| PDH 3672 | FYYDSSGYYY | 10 | |
| PDH 3673 | YYYDSSGYYF | 10 | |
| PDH 3674 | PYYDSSGYYY | 10 | |
| PDH 3675 | VYYDSSGYYS | 10 | |
| PDH 3676 | PYYDSSGYYS | 10 | |
| PDH 3677 | FYYDSSGYYS | 10 | |
| PDH 3678 | NYYDSSGYYY | 10 | |
| PDH 3679 | NYYDSSGYYS | 10 | |
| PDH 3680 | DYYDSSGYYS | 10 | |
| PDH 3681 | HYYDSSGYYF | 10 | |
| PDH 3682 | DYYDSSGYYY | 10 | |
| PDH 3683 | NYYDSSGYYF | 10 | |
| PDH 3684 | HYYDSSGYYY | 10 | |
| PDH 3685 | LYYDSSGYYF | 10 | |
| PDH 3686 | IYYDSSGYYS | 10 | |
| PDH 3687 | YYYDSSGYYS | 10 | |
| PDH 3688 | IYYDSSGYYF | 10 | |
| PDH 3689 | YYYDSSGYYY | 10 | |
| PDH 3690 | HYYDSSGYYS | 10 | |
| PDH 3691 | TYYDSSGYYS | 10 | |
| PDH 3692 | IYYDSSGYYY | 10 | |
| PDH 3693 | TYYDSSGYYF | 10 | |
| PDH 3694 | LYYDSSGYYS | 10 | |
| PDH 3695 | TYYDSSGYYY | 10 | |
| PDH 3696 | LYYDSSGYYY | 10 | |
| PDH 3697 | AYYDSSGYYF | 10 | |
| PDH 3698 | FYYDSSGYYF | 10 | |
| PDH 3699 | AYYDSSGYYY | 10 | |
| PDH 3700 | VYYDSSGYYY | 10 | |
| PDH 3701 | SYYDSSGYYF | 10 | |
| PDH 3702 | PYYDSSGYYF | 10 | |
| PDH 3703 | SYYDSSGYYY | 10 | |
| PDH 3704 | AYYDSSGYYS | 10 | |
| PDH 3705 | DYYDSSGYYF | 10 | |
| PDH 3706 | SYYDSSGYYS | 10 | |
| PDH 3707 | VYYDSSGYYF | 10 | |
| PDH 3708 | IYDYVWGSYAS | 11 | |
| PDH 3709 | AYDYVWGSYAS | 11 | |
| PDH 3710 | IYDYVWGSYAY | 11 | |
| PDH 3711 | NYDYVWGSYAY | 11 | |
| PDH 3712 | NYDYVWGSYAS | 11 | |
| PDH 3713 | YYDYVWGSYAF | 11 | |
| PDH 3714 | DYDYVWGSYAF | 11 | |
| PDH 3715 | SYDYVWGSYAY | 11 | |
| PDH 3716 | DYDYVWGSYAS | 11 | |
| PDH 3717 | DYDYVWGSYAY | 11 | |
| PDH 3718 | FYDYVWGSYAS | 11 | |
| PDH 3719 | NYDYVWGSYAF | 11 | |
| PDH 3720 | YYDYVWGSYAS | 11 | |
| PDH 3721 | FYDYVWGSYAY | 11 | |
| PDH 3722 | SYDYVWGSYAS | 11 | |
| PDH 3723 | PYDYVWGSYAF | 11 | |
| PDH 3724 | TYDYVWGSYAY | 11 | |
| PDH 3725 | VYDYVWGSYAY | 11 | |
| PDH 3726 | SYDYVWGSYAF | 11 | |
| PDH 3727 | FYDYVWGSYAF | 11 | |
| PDH 3728 | HYDYVWGSYAS | 11 | |
| PDH 3729 | VYDYVWGSYAS | 11 | |
| PDH 3730 | VYDYVWGSYAF | 11 | |
| PDH 3731 | YYDYVWGSYAY | 11 | |
| PDH 3732 | AYDYVWGSYAY | 11 | |
| PDH 3733 | LYDYVWGSYAS | 11 | |
| PDH 3734 | TYDYVWGSYAF | 11 | |
| PDH 3735 | AYDYVWGSYAF | 11 | |
| PDH 3736 | HYDYVWGSYAY | 11 | |
| PDH 3737 | TYDYVWGSYAS | 11 | |
| PDH 3738 | LYDYVWGSYAF | 11 | |
| PDH 3739 | PYDYVWGSYAY | 11 | |
| PDH 3740 | PYDYVWGSYAS | 11 | |
| PDH 3741 | HYDYVWGSYAF | 11 | |
| PDH 3742 | LYDYVWGSYAY | 11 | |
| PDH 3743 | IYDYVWGSYAF | 11 | |
| PDH 3744 | NYDYVWGSYAYT | 12 | |
| PDH 3745 | NYDYVWGSYAYI | 12 | |
| PDH 3746 | IYDYVWGSYAYI | 12 | |
| PDH 3747 | YYDYVWGSYAYK | 12 | |
| PDH 3748 | NYDYVWGSYAYK | 12 | |
| PDH 3749 | YYDYVWGSYAYT | 12 | |
| PDH 3750 | PYDYVWGSYAYT | 12 | |
| PDH 3751 | DYDYVWGSYAYI | 12 | |
| PDH 3752 | PYDYVWGSYAYK | 12 | |
| PDH 3753 | FYDYVWGSYAYI | 12 | |
| PDH 3754 | VYDYVWGSYAYT | 12 | |
| PDH 3755 | DYDYVWGSYAYK | 12 | |
| PDH 3756 | IYDYVWGSYAYT | 12 | |
| PDH 3757 | IYDYVWGSYAYK | 12 | |
| PDH 3758 | LYDYVWGSYAYI | 12 | |
| PDH 3759 | HYDYVWGSYAYK | 12 | |
| PDH 3760 | TYDYVWGSYAYI | 12 | |
| PDH 3761 | HYDYVWGSYAYT | 12 | |
| PDH 3762 | AYDYVWGSYAYT | 12 | |
| PDH 3763 | AYDYVWGSYAYK | 12 | |
| PDH 3764 | AYDYVWGSYAYI | 12 | |
| PDH 3765 | TYDYVWGSYAYK | 12 | |
| PDH 3766 | DYDYVWGSYAYT | 12 | |
| PDH 3767 | VYDYVWGSYAYK | 12 | |
| PDH 3768 | TYDYVWGSYAYT | 12 | |
| PDH 3769 | FYDYVWGSYAYK | 12 | |
| PDH 3770 | LYDYVWGSYAYK | 12 | |
| PDH 3771 | VYDYVWGSYAYI | 12 | |
| PDH 3772 | LYDYVWGSYAYT | 12 | |
| PDH 3773 | PYDYVWGSYAYI | 12 | |
| PDH 3774 | FYDYVWGSYAYT | 12 | |
| PDH 3775 | YYDYVWGSYAYI | 12 | |
| PDH 3776 | SYDYVWGSYAYI | 12 | |
| PDH 3777 | HYDYVWGSYAYI | 12 | |
| PDH 3778 | SYDYVWGSYAYT | 12 | |
| PDH 3779 | SYDYVWGSYAYK | 12 | |

**Table 29. Theoretical segment pool of oligonucleotide sequences encoding N2 segments of Example 14.**

| **Name** | **Degenerate Oligo** | **Peptide Length** | | **SEQ ID NO** |
|---|---|---|---|---|
| N2 000 | | 0 | Not degenerate | |
| N2 001 | GCT | 1 | Not degenerate | |
| N2 002 | GAT | 1 | Not degenerate | |
| N2 003 | GAG | 1 | Not degenerate | |
| N2 004 | TTT | 1 | Not degenerate | |
| N2 005 | GGC | 1 | Not degenerate | |
| N2 006 | CAT | 1 | Not degenerate | |
| N2 007 | ATC | 1 | Not degenerate | |
| N2 008 | AAA | 1 | Not degenerate | |
| N2 009 | TTG | 1 | Not degenerate | |
| N2 010 | ATG | 1 | Not degenerate | |
| N2 011 | CCT | 1 | Not degenerate | |
| N2 012 | CAA | 1 | Not degenerate | |
| N2 013 | AGG | 1 | Not degenerate | |
| N2 014 | TCA | 1 | Not degenerate | |
| N2 015 | ACC | 1 | Not degenerate | |
| N2 016 | GTT | 1 | Not degenerate | |
| N2 017 | TGG | 1 | Not degenerate | |
| N2 018 | TAC | 1 | Not degenerate | |
| N2 019 | GMCKHT | 2 | | |
| N2 020 | GMCSVT | 2 | | |
| N2 021 | GMCSHT | 2 | | |
| N2 022 | GMCSVG | 2 | | |
| N2 023 | GMTDYT | 2 | | |
| N2 024 | KHCGAS | 2 | | |
| N2 025 | KHCGRC | 2 | | |
| N2 026 | KHCGWG | 2 | | |
| N2 027 | KHTTTM | 2 | | |
| N2 028 | KHTTYC | 2 | | |
| N2 029 | KHTTWC | 2 | | |
| N2 030 | KHCRGA | 2 | | |
| N2 031 | KHCKGG | 2 | | |
| N2 032 | KHCCWC | 2 | | |
| N2 033 | KHCCMT | 2 | | |
| N2 034 | KHCMCA | 2 | | |
| N2 035 | GVCSWG | 2 | | |
| N2 036 | GVCMKC | 2 | | |
| N2 037 | GVCWSG | 2 | | |
| N2 038 | SVCYAC | 2 | | |
| N2 039 | GNAAHA | 2 | | |
| N2 040 | BYCSAG | 2 | | |
| N2 041 | RBAAWA | 2 | | |
| N2 042 | RBAAYA | 2 | | |
| N2 043 | SBAMAA | 2 | | |
| N2 044 | VSCMAA | 2 | | |
| N2 045 | GRARVG | 2 | | |
| N2 046 | GRADYT | 2 | | |
| N2 047 | GRABYT | 2 | | |
| N2 048 | GRAKBG | 2 | | |
| N2 049 | RDAGMT | 2 | | |
| N2 050 | RDAGAK | 2 | | |
| N2 051 | RDAGRT | 2 | | |
| N2 052 | RDAGWG | 2 | | |
| N2 053 | RDARGG | 2 | | |
| N2 054 | RDACYA | 2 | | |
| N2 055 | SDACSA | 2 | | |
| N2 056 | VWACYA | 2 | | |
| N2 057 | VWATYA | 2 | | |
| N2 058 | VWAASA | 2 | | |
| N2 059 | YHCGMC | 2 | | |
| N2 060 | YHCGMG | 2 | | |
| N2 061 | YHCGST | 2 | | |
| N2 062 | YHCSCG | 2 | | |
| N2 063 | YHCKCG | 2 | | |
| N2 064 | YHCSAC | 2 | | |
| N2 065 | YHCKAC | 2 | | |
| N2 066 | YHCRGA | 2 | | |
| N2 067 | YHCCWC | 2 | | |
| N2 068 | YHCMCA | 2 | | |
| N2 069 | WTCYHT | 2 | | |
| N2 070 | HYCGWG | 2 | | |
| N2 071 | HYCTTM | 2 | | |
| N2 072 | HYCAGM | 2 | | |
| N2 073 | HYCTMC | 2 | | |
| N2 074 | VKCTWT | 2 | | |
| N2 075 | CNCVGC | 2 | | |
| N2 076 | MHAGAK | 2 | | |
| N2 077 | MHAGRC | 2 | | |
| N2 078 | MHAGWG | 2 | | |
| N2 079 | MHAMCA | 2 | | |
| N2 080 | ANAGBT | 2 | | |
| N2 081 | MBCYAC | 2 | | |
| N2 082 | MBCAWA | 2 | | |
| N2 083 | MHGGKA | 2 | | |
| N2 084 | CNABTT | 2 | | |
| N2 085 | CVACNA | 2 | | |
| N2 086 | CVAYSG | 2 | | |
| N2 087 | MSCAHG | 2 | | |
| N2 088 | CRAKBG | 2 | | |
| N2 089 | WSGHCA | 2 | | |
| N2 090 | WGGKHC | 2 | | |
| N2 091 | MBCATR | 2 | | |
| N2 092 | AYABSG | 2 | | |
| N2 093 | VYCAWG | 2 | | |
| N2 094 | BGGSAK | 2 | | |
| N2 095 | AHGRYT | 2 | | |
| N2 096 | BWCAMA | 2 | | |
| N2 097 | BHCTGG | 2 | | |
| N2 098 | TGGBHC | 2 | | |
| N2 099 | TGGVBT | 2 | | |
| N2 100 | NHCGCAGCC | 3 | | |
| N2 101 | BHCGGAATG | 3 | | |
| N2 102 | BHCGGAGGA | 3 | | |
| N2 103 | BHCGGAGTA | 3 | | |
| N2 104 | VNCGCAGGA | 3 | | |
| N2 105 | VBCGGAGCC | 3 | | |
| N2 106 | VBCGGAGGA | 3 | | |
| N2 107 | VBCGGACTA | 3 | | |
| N2 108 | VBCGGAAGG | 3 | | |
| N2 109 | VBCGGAAGC | 3 | | |
| N2 110 | VBCGGAGTA | 3 | | |
| N2 111 | VNCCTTGGA | 3 | | |
| N2 112 | VNCCCAGGA | 3 | | |
| N2 113 | VNCCCACCA | 3 | | |
| N2 114 | VNCAGAGGA | 3 | | |
| N2 115 | VNCAGCGGA | 3 | | |
| N2 116 | VBCACAGGA | 3 | | |
| N2 117 | VNCGTAGGA | 3 | | |
| N2 118 | BHCGGACAC | 3 | | |
| N2 119 | NHCAAACAA | 3 | | |
| N2 120 | NHCAAAAGA | 3 | | |
| N2 121 | BHCACACAA | 3 | | |
| N2 122 | VNCTTTGAG | 3 | | |
| N2 123 | VNCCCACTA | 3 | | |
| N2 124 | VNCCCATAC | 3 | | |
| N2 125 | BHCGGAGAG | 3 | | |
| N2 126 | BHCGGACTA | 3 | | |
| N2 127 | BHCGGATGG | 3 | | |
| N2 128 | BHCGGATAC | 3 | | |
| N2 129 | NHCAGAGGA | 3 | | |
| N2 130 | NHCAGCGAG | 3 | | |
| N2 131 | NHCAGCTGG | 3 | | |
| N2 132 | VHAGGAGGA | 3 | | |
| N2 133 | BHCGGAAGG | 3 | | |
| N2 134 | NHCCAAGGA | 3 | | |
| N2 135 | BHCACAGCT | 3 | | |
| N2 136 | GGABHCGGATAC | 4 | | |
| N2 137 | AGABHCGGATAC | 4 | | |
| N2 138 | AGCBHCGGATAC | 4 | | |
| N2 139 | CCABHCGGATAC | 4 | | |
| N2 140 | GGTAGAVHGTAC | 4 | | |
| N2 141 | AGGAGAVHGTAC | 4 | | |
| N2 142 | GGABHCGGATGG | 4 | | |
| N2 143 | GGABHCGGACTA | 4 | | |
| N2 144 | GGABHCACAGCT | 4 | | |
| N2 145 | GGABHCACACAA | 4 | | |

**Table 30. Theoretical segment pool of unique N2 polypeptide segments encoded by the oligonucleotides of Table 29.**

| **Name** | **Sequence** | **Length** | **SEQ ID NO** |
|---|---|---|---|
| PN2 000 | | 0 | |
| PN2 001 | A | 1 | |
| PN2 002 | D | 1 | |
| PN2 003 | E | 1 | |
| PN2 004 | F | 1 | |
| PN2 005 | G | 1 | |
| PN2 006 | H | 1 | |
| PN2 007 | I | 1 | |
| PN2 008 | K | 1 | |
| PN2 009 | L | 1 | |
| PN2 010 | M | 1 | |
| PN2 011 | P | 1 | |
| PN2 012 | Q | 1 | |
| PN2 013 | R | 1 | |
| PN2 014 | S | 1 | |
| PN2 015 | T | 1 | |
| PN2 016 | V | 1 | |
| PN2 017 | W | 1 | |
| PN2 018 | Y | 1 | |
| PN2 019 | GW | 2 | |
| PN2 020 | GV | 2 | |
| PN2 021 | GT | 2 | |
| PN2 022 | GS | 2 | |
| PN2 023 | GR | 2 | |
| PN2 024 | GQ | 2 | |
| PN2 025 | GP | 2 | |
| PN2 026 | GY | 2 | |
| PN2 027 | GG | 2 | |
| PN2 028 | GF | 2 | |
| PN2 029 | GE | 2 | |
| PN2 030 | GD | 2 | |
| PN2 031 | GA | 2 | |
| PN2 032 | GL | 2 | |
| PN2 033 | GK | 2 | |
| PN2 034 | GI | 2 | |
| PN2 035 | GH | 2 | |
| PN2 036 | MG | 2 | |
| PN2 037 | MA | 2 | |
| PN2 038 | MI | 2 | |
| PN2 039 | MT | 2 | |
| PN2 040 | MV | 2 | |
| PN2 041 | FP | 2 | |
| PN2 042 | FQ | 2 | |
| PN2 043 | FR | 2 | |
| PN2 044 | FS | 2 | |
| PN2 045 | FT | 2 | |
| PN2 046 | FV | 2 | |
| PN2 047 | FW | 2 | |
| PN2 048 | FY | 2 | |
| PN2 049 | FA | 2 | |
| PN2 050 | FD | 2 | |
| PN2 051 | FE | 2 | |
| PN2 052 | FF | 2 | |
| PN2 053 | FG | 2 | |
| PN2 054 | FH | 2 | |
| PN2 055 | FK | 2 | |
| PN2 056 | FL | 2 | |
| PN2 057 | SY | 2 | |
| PN2 058 | SS | 2 | |
| PN2 059 | SR | 2 | |
| PN2 060 | SQ | 2 | |
| PN2 061 | SP | 2 | |
| PN2 062 | SW | 2 | |
| PN2 063 | SV | 2 | |
| PN2 064 | ST | 2 | |
| PN2 065 | SK | 2 | |
| PN2 066 | SI | 2 | |
| PN2 067 | SH | 2 | |
| PN2 068 | SM | 2 | |
| PN2 069 | SL | 2 | |
| PN2 070 | SA | 2 | |
| PN2 071 | SG | 2 | |
| PN2 072 | SF | 2 | |
| PN2 073 | SE | 2 | |
| PN2 074 | SD | 2 | |
| PN2 075 | YH | 2 | |
| PN2 076 | YK | 2 | |
| PN2 077 | YL | 2 | |
| PN2 078 | YA | 2 | |
| PN2 079 | YE | 2 | |
| PN2 080 | YD | 2 | |
| PN2 081 | YG | 2 | |
| PN2 082 | YF | 2 | |
| PN2 083 | YY | 2 | |
| PN2 084 | YP | 2 | |
| PN2 085 | YS | 2 | |
| PN2 086 | YR | 2 | |
| PN2 087 | YT | 2 | |
| PN2 088 | YW | 2 | |
| PN2 089 | YV | 2 | |
| PN2 090 | LF | 2 | |
| PN2 091 | LD | 2 | |
| PN2 092 | LE | 2 | |
| PN2 093 | LL | 2 | |
| PN2 094 | LM | 2 | |
| PN2 095 | LK | 2 | |
| PN2 096 | LH | 2 | |
| PN2 097 | LI | 2 | |
| PN2 098 | LW | 2 | |
| PN2 099 | LT | 2 | |
| PN2 100 | LR | 2 | |
| PN2 101 | LS | 2 | |
| PN2 102 | LP | 2 | |
| PN2 103 | LQ | 2 | |
| PN2 104 | LY | 2 | |
| PN2 105 | LG | 2 | |
| PN2 106 | LA | 2 | |
| PN2 107 | RT | 2 | |
| PN2 108 | RV | 2 | |
| PN2 109 | RW | 2 | |
| PN2 110 | RP | 2 | |
| PN2 111 | RQ | 2 | |
| PN2 112 | RR | 2 | |
| PN2 113 | RS | 2 | |
| PN2 114 | RY | 2 | |
| PN2 115 | RD | 2 | |
| PN2 116 | RE | 2 | |
| PN2 117 | RF | 2 | |
| PN2 118 | RG | 2 | |
| PN2 119 | RA | 2 | |
| PN2 120 | RL | 2 | |
| PN2 121 | RM | 2 | |
| PN2 122 | RH | 2 | |
| PN2 123 | RI | 2 | |
| PN2 124 | RK | 2 | |
| PN2 125 | LV | 2 | |
| PN2 126 | IP | 2 | |
| PN2 127 | EL | 2 | |
| PN2 128 | VK | 2 | |
| PN2 129 | EI | 2 | |
| PN2 130 | EK | 2 | |
| PN2 131 | EE | 2 | |
| PN2 132 | ED | 2 | |
| PN2 133 | EG | 2 | |
| PN2 134 | EF | 2 | |
| PN2 135 | EA | 2 | |
| PN2 136 | IT | 2 | |
| PN2 137 | ET | 2 | |
| PN2 138 | EW | 2 | |
| PN2 139 | EV | 2 | |
| PN2 140 | EP | 2 | |
| PN2 141 | ES | 2 | |
| PN2 142 | ER | 2 | |
| PN2 143 | II | 2 | |
| PN2 144 | IH | 2 | |
| PN2 145 | VR | 2 | |
| PN2 146 | VT | 2 | |
| PN2 147 | KA | 2 | |
| PN2 148 | KG | 2 | |
| PN2 149 | KE | 2 | |
| PN2 150 | KD | 2 | |
| PN2 151 | KI | 2 | |
| PN2 152 | KL | 2 | |
| PN2 153 | KS | 2 | |
| PN2 154 | KR | 2 | |
| PN2 155 | KP | 2 | |
| PN2 156 | KV | 2 | |
| PN2 157 | KT | 2 | |
| PN2 158 | DK | 2 | |
| PN2 159 | DH | 2 | |
| PN2 160 | DI | 2 | |
| PN2 161 | DF | 2 | |
| PN2 162 | DG | 2 | |
| PN2 163 | DD | 2 | |
| PN2 164 | DE | 2 | |
| PN2 165 | DA | 2 | |
| PN2 166 | DY | 2 | |
| PN2 167 | DV | 2 | |
| PN2 168 | DW | 2 | |
| PN2 169 | DT | 2 | |
| PN2 170 | DR | 2 | |
| PN2 171 | DS | 2 | |
| PN2 172 | DP | 2 | |
| PN2 173 | DQ | 2 | |
| PN2 174 | QQ | 2 | |
| PN2 175 | QP | 2 | |
| PN2 176 | QS | 2 | |
| PN2 177 | QR | 2 | |
| PN2 178 | QT | 2 | |
| PN2 179 | QW | 2 | |
| PN2 180 | QA | 2 | |
| PN2 181 | QE | 2 | |
| PN2 182 | QD | 2 | |
| PN2 183 | QG | 2 | |
| PN2 184 | QF | 2 | |
| PN2 185 | QL | 2 | |
| PN2 186 | WG | 2 | |
| PN2 187 | WF | 2 | |
| PN2 188 | WE | 2 | |
| PN2 189 | WD | 2 | |
| PN2 190 | WA | 2 | |
| PN2 191 | WL | 2 | |
| PN2 192 | WI | 2 | |
| PN2 193 | WH | 2 | |
| PN2 194 | WV | 2 | |
| PN2 195 | WT | 2 | |
| PN2 196 | WS | 2 | |
| PN2 197 | WR | 2 | |
| PN2 198 | WQ | 2 | |
| PN2 199 | WP | 2 | |
| PN2 200 | WY | 2 | |
| PN2 201 | PR | 2 | |
| PN2 202 | PS | 2 | |
| PN2 203 | PP | 2 | |
| PN2 204 | PQ | 2 | |
| PN2 205 | PV | 2 | |
| PN2 206 | PW | 2 | |
| PN2 207 | PT | 2 | |
| PN2 208 | PY | 2 | |
| PN2 209 | PA | 2 | |
| PN2 210 | PF | 2 | |
| PN2 211 | PG | 2 | |
| PN2 212 | PD | 2 | |
| PN2 213 | PE | 2 | |
| PN2 214 | PK | 2 | |
| PN2 215 | PH | 2 | |
| PN2 216 | PI | 2 | |
| PN2 217 | PL | 2 | |
| PN2 218 | PM | 2 | |
| PN2 219 | DL | 2 | |
| PN2 220 | IY | 2 | |
| PN2 221 | VA | 2 | |
| PN2 222 | VD | 2 | |
| PN2 223 | VE | 2 | |
| PN2 224 | VF | 2 | |
| PN2 225 | VG | 2 | |
| PN2 226 | VH | 2 | |
| PN2 227 | VI | 2 | |
| PN2 228 | IS | 2 | |
| PN2 229 | IR | 2 | |
| PN2 230 | VL | 2 | |
| PN2 231 | VM | 2 | |
| PN2 232 | IW | 2 | |
| PN2 233 | IV | 2 | |
| PN2 234 | VP | 2 | |
| PN2 235 | VQ | 2 | |
| PN2 236 | IK | 2 | |
| PN2 237 | VS | 2 | |
| PN2 238 | IM | 2 | |
| PN2 239 | IL | 2 | |
| PN2 240 | VV | 2 | |
| PN2 241 | VW | 2 | |
| PN2 242 | IA | 2 | |
| PN2 243 | VY | 2 | |
| PN2 244 | IE | 2 | |
| PN2 245 | ID | 2 | |
| PN2 246 | IG | 2 | |
| PN2 247 | IF | 2 | |
| PN2 248 | TQ | 2 | |
| PN2 249 | TF | 2 | |
| PN2 250 | HY | 2 | |
| PN2 251 | HR | 2 | |
| PN2 252 | HS | 2 | |
| PN2 253 | HP | 2 | |
| PN2 254 | HW | 2 | |
| PN2 255 | HT | 2 | |
| PN2 256 | HK | 2 | |
| PN2 257 | HH | 2 | |
| PN2 258 | HL | 2 | |
| PN2 259 | HA | 2 | |
| PN2 260 | HG | 2 | |
| PN2 261 | HD | 2 | |
| PN2 262 | HE | 2 | |
| PN2 263 | QV | 2 | |
| PN2 264 | TY | 2 | |
| PN2 265 | TV | 2 | |
| PN2 266 | TW | 2 | |
| PN2 267 | TT | 2 | |
| PN2 268 | TR | 2 | |
| PN2 269 | TS | 2 | |
| PN2 270 | TP | 2 | |
| PN2 271 | TL | 2 | |
| PN2 272 | TM | 2 | |
| PN2 273 | TK | 2 | |
| PN2 274 | TH | 2 | |
| PN2 275 | TI | 2 | |
| PN2 276 | TG | 2 | |
| PN2 277 | TD | 2 | |
| PN2 278 | TE | 2 | |
| PN2 279 | TA | 2 | |
| PN2 280 | AA | 2 | |
| PN2 281 | AE | 2 | |
| PN2 282 | AD | 2 | |
| PN2 283 | AG | 2 | |
| PN2 284 | AF | 2 | |
| PN2 285 | AI | 2 | |
| PN2 286 | AH | 2 | |
| PN2 287 | AK | 2 | |
| PN2 288 | AM | 2 | |
| PN2 289 | AL | 2 | |
| PN2 290 | AQ | 2 | |
| PN2 291 | AP | 2 | |
| PN2 292 | AS | 2 | |
| PN2 293 | AR | 2 | |
| PN2 294 | AT | 2 | |
| PN2 295 | AW | 2 | |
| PN2 296 | AV | 2 | |
| PN2 297 | AY | 2 | |
| PN2 298 | AGM | 3 | |
| PN2 299 | AGL | 3 | |
| PN2 300 | AGH | 3 | |
| PN2 301 | AGG | 3 | |
| PN2 302 | AGE | 3 | |
| PN2 303 | AGA | 3 | |
| PN2 304 | TQG | 3 | |
| PN2 305 | AGY | 3 | |
| PN2 306 | AGW | 3 | |
| PN2 307 | AGV | 3 | |
| PN2 308 | AGS | 3 | |
| PN2 309 | AGR | 3 | |
| PN2 310 | SPY | 3 | |
| PN2 311 | SPP | 3 | |
| PN2 312 | TAG | 3 | |
| PN2 313 | SPL | 3 | |
| PN2 314 | TAA | 3 | |
| PN2 315 | SPG | 3 | |
| PN2 316 | VAA | 3 | |
| PN2 317 | VAG | 3 | |
| PN2 318 | NPL | 3 | |
| PN2 319 | PAA | 3 | |
| PN2 320 | NPG | 3 | |
| PN2 321 | LAG | 3 | |
| PN2 322 | LAA | 3 | |
| PN2 323 | NPY | 3 | |
| PN2 324 | ALG | 3 | |
| PN2 325 | RLG | 3 | |
| PN2 326 | TSG | 3 | |
| PN2 327 | DTA | 3 | |
| PN2 328 | PAG | 3 | |
| PN2 329 | QGG | 3 | |
| PN2 330 | DLG | 3 | |
| PN2 331 | DTQ | 3 | |
| PN2 332 | LSG | 3 | |
| PN2 333 | LSE | 3 | |
| PN2 334 | PGR | 3 | |
| PN2 335 | VLG | 3 | |
| PN2 336 | LSW | 3 | |
| PN2 337 | AFE | 3 | |
| PN2 338 | TTG | 3 | |
| PN2 339 | FTQ | 3 | |
| PN2 340 | IGV | 3 | |
| PN2 341 | NFE | 3 | |
| PN2 342 | IGS | 3 | |
| PN2 343 | IGR | 3 | |
| PN2 344 | PGS | 3 | |
| PN2 345 | IGG | 3 | |
| PN2 346 | GRG | 3 | |
| PN2 347 | IGA | 3 | |
| PN2 348 | DSG | 3 | |
| PN2 349 | IGL | 3 | |
| PN2 350 | HLG | 3 | |
| PN2 351 | HPP | 3 | |
| PN2 352 | AKQ | 3 | |
| PN2 353 | GLG | 3 | |
| PN2 354 | LFE | 3 | |
| PN2 355 | GGV | 3 | |
| PN2 356 | GGS | 3 | |
| PN2 357 | GGR | 3 | |
| PN2 358 | GGG | 3 | |
| PN2 359 | GGA | 3 | |
| PN2 360 | GGL | 3 | |
| PN2 361 | YTA | 3 | |
| PN2 362 | LTQ | 3 | |
| PN2 363 | FQG | 3 | |
| PN2 364 | LVG | 3 | |
| PN2 365 | LTA | 3 | |
| PN2 366 | LTG | 3 | |
| PN2 367 | DPY | 3 | |
| PN2 368 | DFE | 3 | |
| PN2 369 | DPG | 3 | |
| PN2 370 | HTA | 3 | |
| PN2 371 | DPP | 3 | |
| PN2 372 | PGG | 3 | |
| PN2 373 | VPP | 3 | |
| PN2 374 | PGA | 3 | |
| PN2 375 | VPY | 3 | |
| PN2 376 | PGV | 3 | |
| PN2 377 | VPL | 3 | |
| PN2 378 | STG | 3 | |
| PN2 379 | STA | 3 | |
| PN2 380 | HGV | 3 | |
| PN2 381 | HGW | 3 | |
| PN2 382 | HGR | 3 | |
| PN2 383 | HGL | 3 | |
| PN2 384 | HGM | 3 | |
| PN2 385 | STQ | 3 | |
| PN2 386 | HGH | 3 | |
| PN2 387 | HGG | 3 | |
| PN2 388 | DSW | 3 | |
| PN2 389 | HGE | 3 | |
| PN2 390 | TFE | 3 | |
| PN2 391 | PGY | 3 | |
| PN2 392 | PGL | 3 | |
| PN2 393 | PGM | 3 | |
| PN2 394 | DKR | 3 | |
| PN2 395 | DKQ | 3 | |
| PN2 396 | VFE | 3 | |
| PN2 397 | PRG | 3 | |
| PN2 398 | PGH | 3 | |
| PN2 399 | PPL | 3 | |
| PN2 400 | EGG | 3 | |
| PN2 401 | RAG | 3 | |
| PN2 402 | SAA | 3 | |
| PN2 403 | FGW | 3 | |
| PN2 404 | SAG | 3 | |
| PN2 405 | FGR | 3 | |
| PN2 406 | FGH | 3 | |
| PN2 407 | FGG | 3 | |
| PN2 408 | TPG | 3 | |
| PN2 409 | LPY | 3 | |
| PN2 410 | TPL | 3 | |
| PN2 411 | LPP | 3 | |
| PN2 412 | LPL | 3 | |
| PN2 413 | TPP | 3 | |
| PN2 414 | LPG | 3 | |
| PN2 415 | HRG | 3 | |
| PN2 416 | TPY | 3 | |
| PN2 417 | APY | 3 | |
| PN2 418 | IPG | 3 | |
| PN2 419 | APP | 3 | |
| PN2 420 | PQG | 3 | |
| PN2 421 | IPL | 3 | |
| PN2 422 | IPP | 3 | |
| PN2 423 | APL | 3 | |
| PN2 424 | SFE | 3 | |
| PN2 425 | APG | 3 | |
| PN2 426 | YSE | 3 | |
| PN2 427 | IFE | 3 | |
| PN2 428 | YSW | 3 | |
| PN2 429 | PKR | 3 | |
| PN2 430 | RTG | 3 | |
| PN2 431 | PKQ | 3 | |
| PN2 432 | HGY | 3 | |
| PN2 433 | TKR | 3 | |
| PN2 434 | NLG | 3 | |
| PN2 435 | VKR | 3 | |
| PN2 436 | RFE | 3 | |
| PN2 437 | SSW | 3 | |
| PN2 438 | NPP | 3 | |
| PN2 439 | SSE | 3 | |
| PN2 440 | SSG | 3 | |
| PN2 441 | YGR | 3 | |
| PN2 442 | ATG | 3 | |
| PN2 443 | ATA | 3 | |
| PN2 444 | HPL | 3 | |
| PN2 445 | ISW | 3 | |
| PN2 446 | ATQ | 3 | |
| PN2 447 | ISG | 3 | |
| PN2 448 | ISE | 3 | |
| PN2 449 | DGR | 3 | |
| PN2 450 | ASW | 3 | |
| PN2 451 | DGG | 3 | |
| PN2 452 | DGE | 3 | |
| PN2 453 | HPG | 3 | |
| PN2 454 | DGH | 3 | |
| PN2 455 | DGL | 3 | |
| PN2 456 | DGM | 3 | |
| PN2 457 | LKQ | 3 | |
| PN2 458 | DGV | 3 | |
| PN2 459 | DGW | 3 | |
| PN2 460 | PVG | 3 | |
| PN2 461 | ASG | 3 | |
| PN2 462 | IRG | 3 | |
| PN2 463 | VTA | 3 | |
| PN2 464 | TSE | 3 | |
| PN2 465 | FRG | 3 | |
| PN2 466 | ASE | 3 | |
| PN2 467 | VTG | 3 | |
| PN2 468 | GTG | 3 | |
| PN2 469 | LGW | 3 | |
| PN2 470 | VTQ | 3 | |
| PN2 471 | TLG | 3 | |
| PN2 472 | YAA | 3 | |
| PN2 473 | DGY | 3 | |
| PN2 474 | ITG | 3 | |
| PN2 475 | HVG | 3 | |
| PN2 476 | RPP | 3 | |
| PN2 477 | AAG | 3 | |
| PN2 478 | RSG | 3 | |
| PN2 479 | AAA | 3 | |
| PN2 480 | TGR | 3 | |
| PN2 481 | TGS | 3 | |
| PN2 482 | TGV | 3 | |
| PN2 483 | RPY | 3 | |
| PN2 484 | TGA | 3 | |
| PN2 485 | TGG | 3 | |
| PN2 486 | RPG | 3 | |
| PN2 487 | RPL | 3 | |
| PN2 488 | TGL | 3 | |
| PN2 489 | FKQ | 3 | |
| PN2 490 | FKR | 3 | |
| PN2 491 | SLG | 3 | |
| PN2 492 | LGM | 3 | |
| PN2 493 | LGA | 3 | |
| PN2 494 | NRG | 3 | |
| PN2 495 | LGG | 3 | |
| PN2 496 | LGE | 3 | |
| PN2 497 | LGY | 3 | |
| PN2 498 | LGR | 3 | |
| PN2 499 | LGS | 3 | |
| PN2 500 | LGV | 3 | |
| PN2 501 | GFE | 3 | |
| PN2 502 | LQG | 3 | |
| PN2 503 | HSG | 3 | |
| PN2 504 | HSE | 3 | |
| PN2 505 | HSW | 3 | |
| PN2 506 | DPL | 3 | |
| PN2 507 | GPL | 3 | |
| PN2 508 | HAA | 3 | |
| PN2 509 | IAG | 3 | |
| PN2 510 | GPG | 3 | |
| PN2 511 | IAA | 3 | |
| PN2 512 | HAG | 3 | |
| PN2 513 | DQG | 3 | |
| PN2 514 | GPY | 3 | |
| PN2 515 | GPP | 3 | |
| PN2 516 | VQG | 3 | |
| PN2 517 | RGR | 3 | |
| PN2 518 | RGS | 3 | |
| PN2 519 | SKQ | 3 | |
| PN2 520 | RGV | 3 | |
| PN2 521 | SKR | 3 | |
| PN2 522 | RGA | 3 | |
| PN2 523 | RGG | 3 | |
| PN2 524 | RGL | 3 | |
| PN2 525 | VGL | 3 | |
| PN2 526 | VGM | 3 | |
| PN2 527 | VGH | 3 | |
| PN2 528 | FGY | 3 | |
| PN2 529 | VGE | 3 | |
| PN2 530 | VGG | 3 | |
| PN2 531 | VGA | 3 | |
| PN2 532 | YKR | 3 | |
| PN2 533 | YKQ | 3 | |
| PN2 534 | VGY | 3 | |
| PN2 535 | VGV | 3 | |
| PN2 536 | VGW | 3 | |
| PN2 537 | VGR | 3 | |
| PN2 538 | VGS | 3 | |
| PN2 539 | FTA | 3 | |
| PN2 540 | PSG | 3 | |
| PN2 541 | PSE | 3 | |
| PN2 542 | NVG | 3 | |
| PN2 543 | FGV | 3 | |
| PN2 544 | FGL | 3 | |
| PN2 545 | ILG | 3 | |
| PN2 546 | PGW | 3 | |
| PN2 547 | FSE | 3 | |
| PN2 548 | DVG | 3 | |
| PN2 549 | FSW | 3 | |
| PN2 550 | IKR | 3 | |
| PN2 551 | IKQ | 3 | |
| PN2 552 | DSE | 3 | |
| PN2 553 | FGM | 3 | |
| PN2 554 | VRG | 3 | |
| PN2 555 | NKQ | 3 | |
| PN2 556 | TVG | 3 | |
| PN2 557 | NQG | 3 | |
| PN2 558 | SRG | 3 | |
| PN2 559 | YRG | 3 | |
| PN2 560 | FGE | 3 | |
| PN2 561 | PLG | 3 | |
| PN2 562 | PPG | 3 | |
| PN2 563 | NSE | 3 | |
| PN2 564 | NSG | 3 | |
| PN2 565 | PPP | 3 | |
| PN2 566 | GAG | 3 | |
| PN2 567 | PPY | 3 | |
| PN2 568 | FAA | 3 | |
| PN2 569 | NSW | 3 | |
| PN2 570 | HPY | 3 | |
| PN2 571 | PSW | 3 | |
| PN2 572 | ARG | 3 | |
| PN2 573 | SGS | 3 | |
| PN2 574 | NAA | 3 | |
| PN2 575 | NAG | 3 | |
| PN2 576 | SGW | 3 | |
| PN2 577 | GSG | 3 | |
| PN2 578 | DRG | 3 | |
| PN2 579 | RVG | 3 | |
| PN2 580 | HKR | 3 | |
| PN2 581 | HKQ | 3 | |
| PN2 582 | AQG | 3 | |
| PN2 583 | VPG | 3 | |
| PN2 584 | AKR | 3 | |
| PN2 585 | WG | 3 | |
| PN2 586 | SGL | 3 | |
| PN2 587 | VKQ | 3 | |
| PN2 588 | SVG | 3 | |
| PN2 589 | SQG | 3 | |
| PN2 590 | LKR | 3 | |
| PN2 591 | PTG | 3 | |
| PN2 592 | PGE | 3 | |
| PN2 593 | PTA | 3 | |
| PN2 594 | LLG | 3 | |
| PN2 595 | PTQ | 3 | |
| PN2 596 | TRG | 3 | |
| PN2 597 | GVG | 3 | |
| PN2 598 | IVG | 3 | |
| PN2 599 | LRG | 3 | |
| PN2 600 | HTQ | 3 | |
| PN2 601 | AVG | 3 | |
| PN2 602 | IPY | 3 | |
| PN2 603 | YQG | 3 | |
| PN2 604 | HFE | 3 | |
| PN2 605 | RRG | 3 | |
| PN2 606 | LGH | 3 | |
| PN2 607 | LGL | 3 | |
| PN2 608 | TSW | 3 | |
| PN2 609 | TKQ | 3 | |
| PN2 610 | IQG | 3 | |
| PN2 611 | HQG | 3 | |
| PN2 612 | SGY | 3 | |
| PN2 613 | DAG | 3 | |
| PN2 614 | DAA | 3 | |
| PN2 615 | SGR | 3 | |
| PN2 616 | SGV | 3 | |
| PN2 617 | SGH | 3 | |
| PN2 618 | SGM | 3 | |
| PN2 619 | SGA | 3 | |
| PN2 620 | SGE | 3 | |
| PN2 621 | SGG | 3 | |
| PN2 622 | YTQ | 3 | |
| PN2 623 | YGG | 3 | |
| PN2 624 | YGE | 3 | |
| PN2 625 | PFE | 3 | |
| PN2 626 | VSW | 3 | |
| PN2 627 | YGM | 3 | |
| PN2 628 | YGL | 3 | |
| PN2 629 | YGH | 3 | |
| PN2 630 | YGW | 3 | |
| PN2 631 | YGV | 3 | |
| PN2 632 | NKR | 3 | |
| PN2 633 | VSE | 3 | |
| PN2 634 | KGG | 3 | |
| PN2 635 | VSG | 3 | |
| PN2 636 | YGY | 3 | |
| PN2 637 | RPGY | 4 | |
| PN2 638 | SAGY | 4 | |
| PN2 639 | PSGY | 4 | |
| PN2 640 | RFGY | 4 | |
| PN2 641 | RLGY | 4 | |
| PN2 642 | PYGY | 4 | |
| PN2 643 | GREY | 4 | |
| PN2 644 | GRKY | 4 | |
| PN2 645 | GHGW | 4 | |
| PN2 646 | RVGY | 4 | |
| PN2 647 | GLGL | 4 | |
| PN2 648 | GHGY | 4 | |
| PN2 649 | RDGY | 4 | |
| PN2 650 | RRVY | 4 | |
| PN2 651 | GLGW | 4 | |
| PN2 652 | GHGL | 4 | |
| PN2 653 | GLGY | 4 | |
| PN2 654 | PVGY | 4 | |
| PN2 655 | GPGY | 4 | |
| PN2 656 | GPGW | 4 | |
| PN2 657 | PDGY | 4 | |
| PN2 658 | GPGL | 4 | |
| PN2 659 | GVTA | 4 | |
| PN2 660 | GRLY | 4 | |
| PN2 661 | RRAY | 4 | |
| PN2 662 | GHTQ | 4 | |
| PN2 663 | GVGL | 4 | |
| PN2 664 | SYGY | 4 | |
| PN2 665 | RRQY | 4 | |
| PN2 666 | GATQ | 4 | |
| PN2 667 | PFGY | 4 | |
| PN2 668 | GVGY | 4 | |
| PN2 669 | GVGW | 4 | |
| PN2 670 | RAGY | 4 | |
| PN2 671 | GATA | 4 | |
| PN2 672 | RYGY | 4 | |
| PN2 673 | GRAY | 4 | |
| PN2 674 | GRMY | 4 | |
| PN2 675 | GRTY | 4 | |
| PN2 676 | PPGY | 4 | |
| PN2 677 | RRPY | 4 | |
| PN2 678 | RHGY | 4 | |
| PN2 679 | GDGY | 4 | |
| PN2 680 | SVGY | 4 | |
| PN2 681 | GVTQ | 4 | |
| PN2 682 | GDGW | 4 | |
| PN2 683 | GRQY | 4 | |
| PN2 684 | GDGL | 4 | |
| PN2 685 | SHGY | 4 | |
| PN2 686 | GSGY | 4 | |
| PN2 687 | GFGY | 4 | |
| PN2 688 | GFGW | 4 | |
| PN2 689 | GSGW | 4 | |
| PN2 690 | GFGL | 4 | |
| PN2 691 | GSGL | 4 | |
| PN2 692 | RRLY | 4 | |
| PN2 693 | GFTA | 4 | |
| PN2 694 | PLGY | 4 | |
| PN2 695 | GYTQ | 4 | |
| PN2 696 | GLTQ | 4 | |
| PN2 697 | GHTA | 4 | |
| PN2 698 | PHGY | 4 | |
| PN2 699 | GFTQ | 4 | |
| PN2 700 | GRVY | 4 | |
| PN2 701 | GYTA | 4 | |
| PN2 702 | GLTA | 4 | |
| PN2 703 | PAGY | 4 | |
| PN2 704 | RRKY | 4 | |
| PN2 705 | SSGY | 4 | |
| PN2 706 | GPTQ | 4 | |
| PN2 707 | SDGY | 4 | |
| PN2 708 | GPTA | 4 | |
| PN2 709 | GDTQ | 4 | |
| PN2 710 | GAGW | 4 | |
| PN2 711 | GAGY | 4 | |
| PN2 712 | GDTA | 4 | |
| PN2 713 | SFGY | 4 | |
| PN2 714 | GAGL | 4 | |
| PN2 715 | GSTQ | 4 | |
| PN2 716 | GRPY | 4 | |
| PN2 717 | SLGY | 4 | |
| PN2 718 | GSTA | 4 | |
| PN2 719 | GYGL | 4 | |
| PN2 720 | RSGY | 4 | |
| PN2 721 | RREY | 4 | |
| PN2 722 | SPGY | 4 | |
| PN2 723 | GYGY | 4 | |
| PN2 724 | RRTY | 4 | |
| PN2 725 | GYGW | 4 | |
| PN2 726 | RRMY | 4 | |

**Table 31. Theoretical segment pool of oligonucleotides encoding JH segments of Example 15.**

| **Name** | **Degenerate Oligo** | **Peptide Length** | **Degenerate** | **SEQ ID NO** |
|---|---|---|---|---|
| JH4 001 | TGGGGACAGGGTACATTGGTCACCGTCTCCTCA | 0 | | |
| JH1 002 | CATTGGGGACAGGGTACATTGGTCACCGTCTCCTCA | 1 | | |
| JH1 003 | ATTTGGGGACAGGGTACATTGGTCACCGTCTCCTCA | 1 | | |
| JH1 004 | TACTGGGGACAGGGTACATTGGTCACCGTCTCCTCA | 1 | | |
| JH1 005 | CCATGGGGACAGGGTACATTGGTCACCGTCTCCTCA | 1 | | |
| JH1 006 | GTCTGGGGACAGGGTACATTGGTCACCGTCTCCTCA | 1 | | |
| JH1 007 | GATTGGGGACAGGGTACATTGGTCACCGTCTCCTCA | 1 | | |
| JH1 008 | TTCTGGGGACAGGGTACATTGGTCACCGTCTCCTCA | 1 | | |
| JH1 009 | AATTGGGGACAGGGTACATTGGTCACCGTCTCCTCA | 1 | | |
| JH1 010 | AGTTGGGGACAGGGTACATTGGTCACCGTCTCCTCA | 1 | | |
| JH1 011 | ACTTGGGGACAGGGTACATTGGTCACCGTCTCCTCA | 1 | | |
| JH1 200 | GCATGGGGACAGGGTACATTGGTCACCGTCTCCTCA | 1 | | |
| JH4 013 | GATTACTGGGGACAGGGTACATTGGTCACCGTCTCCTCA | 2 | | |
| JH4 016 | GCTTACTGGGGACAGGGTACATTGGTCACCGTCTCCTCA | 2 | | |
| JH4 017 | TTCTACTGGGGACAGGGTACATTGGTCACCGTCTCCTCA | 2 | | |
| JH4 018 | GGCTATTGGGGACAGGGTACATTGGTCACCGTCTCCTCA | 2 | | |
| JH4 019 | CATTACTGGGGACAGGGTACATTGGTCACCGTCTCCTCA | 2 | | |
| JH4 023 | AGTTACTGGGGACAGGGTACATTGGTCACCGTCTCCTCA | 2 | | |
| JH4 024 | GTTTATTGGGGACAGGGTACATTGGTCACCGTCTCCTCA | 2 | | |
| JH4 025 | TACTATTGGGGACAGGGTACATTGGTCACCGTCTCCTCA | 2 | | |
| JH4 022 | CSATACTGGGGACAGGGTACATTGGTCACCGTCTCCTCA | 2 | YES | |
| JH3 012 | RACATATGGGGTCAGGGTACAATGGTCACCGTCTCCTCA | 2 | YES | |
| JH5 014 | RACCCATGGGGACAGGGTACATTGGTCACCGTCTCCTCA | 2 | YES | |
| JH3 015 | RACGTATGGGGTCAGGGTACAATGGTCACCGTCTCCTCA | 2 | YES | |
| JH4 021 | AMCTACTGGGGACAGGGTACATTGGTCACCGTCTCCTCA | 2 | YES | |
| JH4 020 | MTATACTGGGGACAGGGTACATTGGTCACCGTCTCCTCA | 2 | YES | |
| JH4 029 | TTCGATTATTGGGGACAGGGTACATTGGTCACCGTCTCCTCA | 3 | | |
| JH4 030 | ATTGACTACTGGGGACAGGGTACATTGGTCACCGTCTCCTCA | 3 | | |
| JH4 031 | GTGGACTACTGGGGACAGGGTACATTGGTCACCGTCTCCTCA | 3 | | |
| JH4 032 | TTAGATTACTGGGGACAGGGTACATTGGTCACCGTCTCCTCA | 3 | | |
| JH4 033 | TCCGATTATTGGGGACAGGGTACATTGGTCACCGTCTCCTCA | 3 | | |
| JH4 034 | CACGATTACTGGGGACAGGGTACATTGGTCACCGTCTCCTCA | 3 | | |
| JH4 035 | AGAGACTACTGGGGACAGGGTACATTGGTCACCGTCTCCTCA | 3 | | |
| JH4 036 | CCAGATTACTGGGGACAGGGTACATTGGTCACCGTCTCCTCA | 3 | | |
| JH4 037 | AACGATTACTGGGGACAGGGTACATTGGTCACCGTCTCCTCA | 3 | | |
| JH4 038 | ACTGATTACTGGGGACAGGGTACATTGGTCACCGTCTCCTCA | 3 | | |
| JH4 039 | GATGATTATTGGGGACAGGGTACATTGGTCACCGTCTCCTCA | 3 | | |
| JH4 040 | GGTGATTATTGGGGACAGGGTACATTGGTCACCGTCTCCTCA | 3 | | |
| JH4 041 | GCAGACTACTGGGGACAGGGTACATTGGTCACCGTCTCCTCA | 3 | | |
| JH4 042 | TACGATTATTGGGGACAGGGTACATTGGTCACCGTCTCCTCA | 3 | | |
| JH5 043 | TTCGATCCCTGGGGACAGGGTACATTGGTCACCGTCTCCTCA | 3 | | |
| JH6 044 | ATGGATGTGTGGGGCCAGGGAACAACTGTCACCGTCTCCTCA | 3 | | |
| JH1 026 | TTMCAACACTGGGGACAGGGTACATTGGTCACCGTCTCCTCA | 3 | YES | |
| JH3 046 | STAGACGTATGGGGTCAGGGTACAATGGTCACCGTCTCCTCA | 3 | YES | |
| JH3 028 | TTMGACATATGGGGTCAGGGTACAATGGTCACCGTCTCCTCA | 3 | YES | |
| JH2 027 | TTMGACCTATGGGGGAGAGGTACCTTGGTCACCGTCTCCTCA | 3 | YES | |
| JH5 045 | TYAGACCCATGGGGACAGGGTACATTGGTCACCGTCTCCTCA | 3 | YES | |
| JH3 049 | GCCTTTGATATTTGGGGTCAGGGTACAATGGTCACCGTCTCCTCA | 4 | | |
| JH4 051 | TACTTTGACTACTGGGGACAGGGTACATTGGTCACCGTCTCCTCA | 4 | | |
| JH4 052 | AATTTCGACTACTGGGGACAGGGTACATTGGTCACCGTCTCCTCA | 4 | | |
| JH4 053 | GACTTCGACTATTGGGGACAGGGTACATTGGTCACCGTCTCCTCA | 4 | | |
| JH4 054 | CATTTCGATTACTGGGGACAGGGTACATTGGTCACCGTCTCCTCA | 4 | | |
| JH4 055 | TTCTTTGATTATTGGGGACAGGGTACATTGGTCACCGTCTCCTCA | 4 | | |
| JH4 056 | TCTTTTGATTATTGGGGACAGGGTACATTGGTCACCGTCTCCTCA | 4 | | |
| JH4 057 | AGATTCGACTACTGGGGACAGGGTACATTGGTCACCGTCTCCTCA | 4 | | |
| JH4 058 | TTGTTCGACTACTGGGGACAGGGTACATTGGTCACCGTCTCCTCA | 4 | | |
| JH4 059 | CCCTTCGACTATTGGGGACAGGGTACATTGGTCACCGTCTCCTCA | 4 | | |
| JH4 060 | ATTTTCGATTATTGGGGACAGGGTACATTGGTCACCGTCTCCTCA | 4 | | |
| JH4 061 | ACCTTTGATTACTGGGGACAGGGTACATTGGTCACCGTCTCCTCA | 4 | | |
| JH4 062 | GGATTCGATTACTGGGGACAGGGTACATTGGTCACCGTCTCCTCA | 4 | | |
| JH4 063 | GTTTTCGATTACTGGGGACAGGGTACATTGGTCACCGTCTCCTCA | 4 | | |
| JH4 064 | GCTTTTGACTACTGGGGACAGGGTACATTGGTCACCGTCTCCTCA | 4 | | |
| JH5 065 | TGGTTTGATCCATGGGGACAGGGTACATTGGTCACCGTCTCCTCA | 4 | | |
| JH6 068 | GGAATGGACGTATGGGGCCAGGGAACAACTGTCACCGTCTCCTCA | 4 | | |
| JH6 070 | YCAATGGACGTATGGGGCCAGGGAACAACTGTCACCGTCTCCTCA | 4 | YES | |
| JH6 069 | KACATGGACGTATGGGGCAAGGGTACAACTGTCACCGTCTCCTCA | 4 | YES | |
| JH3 198 | STATTCGACATATGGGGTCAGGGTACAATGGTCACCGTCTCCTCA | 4 | YES | |
| JH2 048 | KACTTCGACCTATGGGGGAGAGGTACCTTGGTCACCGTCTCCTCA | 4 | YES | |
| JH1 047 | KACTTCCAACACTGGGGACAGGGTACATTGGTCACCGTCTCCTCA | 4 | YES | |
| JH5 067 | GSATTCGACCCATGGGGACAGGGTACATTGGTCACCGTCTCCTCA | 4 | YES | |
| JH3 050 | YCATTCGACATATGGGGTCAGGGTACAATGGTCACCGTCTCCTCA | 4 | YES | |
| JH5 066 | AGMTTCGACCCATGGGGACAGGGTACATTGGTCACCGTCTCCTCA | 4 | YES | |
| JH2 072 | TGGTACTTCGACTTATGGGGGAGAGGTACCTTGGTCACCGTCTCCTCA | 5 | | |
| JH3 075 | GACGCATTTGATATATGGGGTCAGGGTACAATGGTCACCGTCTCCTCA | 5 | | |
| JH3 076 | TACGCATTTGATATTTGGGGTCAGGGTACAATGGTCACCGTCTCCTCA | 5 | | |
| JH3 077 | CACGCATTCGACATCTGGGGTCAGGGTACAATGGTCACCGTCTCCTCA | 5 | | |
| JH3 078 | TTCGCATTCGATATCTGGGGTCAGGGTACAATGGTCACCGTCTCCTCA | 5 | | |
| JH3 079 | TCAGCTTTCGACATATGGGGTCAGGGTACAATGGTCACCGTCTCCTCA | 5 | | |
| JH3 080 | AGAGCCTTCGATATATGGGGTCAGGGTACAATGGTCACCGTCTCCTCA | 5 | | |
| JH3 081 | TTAGCCTTCGATATCTGGGGTCAGGGTACAATGGTCACCGTCTCCTCA | 5 | | |
| JH3 082 | GGAGCCTTCGACATATGGGGTCAGGGTACAATGGTCACCGTCTCCTCA | 5 | | |
| JH4 086 | GACTATTTTGATTACTGGGGACAGGGTACATTGGTCACCGTCTCCTCA | 5 | | |
| JH4 087 | TATTACTTTGATTACTGGGGACAGGGTACATTGGTCACCGTCTCCTCA | 5 | | |
| JH4 088 | CACTATTTCGACTATTGGGGACAGGGTACATTGGTCACCGTCTCCTCA | 5 | | |
| JH4 089 | TTCTATTTTGATTACTGGGGACAGGGTACATTGGTCACCGTCTCCTCA | 5 | | |
| JH4 090 | AGTTATTTTGACTATTGGGGACAGGGTACATTGGTCACCGTCTCCTCA | 5 | | |
| JH4 091 | AGATACTTTGATTACTGGGGACAGGGTACATTGGTCACCGTCTCCTCA | 5 | | |
| JH4 092 | TTATATTTCGACTATTGGGGACAGGGTACATTGGTCACCGTCTCCTCA | 5 | | |
| JH4 093 | CCCTACTTTGACTATTGGGGACAGGGTACATTGGTCACCGTCTCCTCA | 5 | | |
| JH4 095 | GGATATTTCGATTATTGGGGACAGGGTACATTGGTCACCGTCTCCTCA | 5 | | |
| JH4 096 | GTTTACTTTGATTATTGGGGACAGGGTACATTGGTCACCGTCTCCTCA | 5 | | |
| JH4 097 | GCTTACTTTGATTATTGGGGACAGGGTACATTGGTCACCGTCTCCTCA | 5 | | |
| JH4 098 | AACTACTTCGATTACTGGGGACAGGGTACATTGGTCACCGTCTCCTCA | 5 | | |
| JH5 099 | AATTGGTTCGATCCTTGGGGACAGGGTACATTGGTCACCGTCTCCTCA | 5 | | |
| JH5 100 | GATTGGTTTGATCCATGGGGACAGGGTACATTGGTCACCGTCTCCTCA | 5 | | |
| JH5 101 | TATTGGTTTGATCCATGGGGACAGGGTACATTGGTCACCGTCTCCTCA | 5 | | |
| JH5 102 | CACTGGTTCGATCCATGGGGACAGGGTACATTGGTCACCGTCTCCTCA | 5 | | |
| JH5 103 | TTCTGGTTTGACCCCTGGGGACAGGGTACATTGGTCACCGTCTCCTCA | 5 | | |
| JH5 104 | TCTTGGTTTGATCCCTGGGGACAGGGTACATTGGTCACCGTCTCCTCA | 5 | | |
| JH5 105 | AGATGGTTTGATCCTTGGGGACAGGGTACATTGGTCACCGTCTCCTCA | 5 | | |
| JH5 107 | GGTTGGTTCGATCCCTGGGGACAGGGTACATTGGTCACCGTCTCCTCA | 5 | | |
| JH5 109 | GCTTGGTTTGATCCATGGGGACAGGGTACATTGGTCACCGTCTCCTCA | 5 | | |
| JH6 110 | TACGGTATGGACGTTTGGGGCCAGGGAACAACTGTCACCGTCTCCTCA | 5 | | |
| JH6 112 | GATGGGATGGATGTTTGGGGCCAGGGAACAACTGTCACCGTCTCCTCA | 5 | | |
| JH3 084 | SCAGCATTCGACATATGGGGTCAGGGTACAATGGTCACCGTCTCCTCA | 5 | YES | |
| JH5 106 | MCATGGTTCGACCCATGGGGACAGGGTACATTGGTCACCGTCTCCTCA | 5 | YES | |
| JH2 073 | RGCTACTTCGACCTATGGGGGAGAGGTACCTTGGTCACCGTCTCCTCA | 5 | YES | |
| JH4 094 | AYATACTTCGACTACTGGGGACAGGGTACATTGGTCACCGTCTCCTCA | 5 | YES | |
| JH5 108 | RTATGGTTCGACCCATGGGGACAGGGTACATTGGTCACCGTCTCCTCA | 5 | YES | |
| JH6 113 | CWCGGAATGGACGTATGGGGCCAGGGAACAACTGTCACCGTCTCCTCA | 5 | YES | |
| JH6 114 | KCAGGAATGGACGTATGGGGCCAGGGAACAACTGTCACCGTCTCCTCA | 5 | YES | |
| JH3 083 | RTAGCATTCGACATATGGGGTCAGGGTACAATGGTCACCGTCTCCTCA | 5 | YES | |
| JH1 071 | RAGTACTTCCAACACTGGGGACAGGGTACATTGGTCACCGTCTCCTCA | 5 | YES | |
| JH3 085 | AMCGCATTCGACATATGGGGTCAGGGTACAATGGTCACCGTCTCCTCA | 5 | YES | |
| JH2 074 | CKATACTTCGACCTATGGGGGAGAGGTACCTTGGTCACCGTCTCCTCA | 5 | YES | |
| JH6 111 | KACTACATGGACGTATGGGGCAAGGGTACAACTGTCACCGTCTCCTCA | 5 | YES | |
| JH2 116 | TACTGGTACTTCGATTTGTGGGGGAGAGGTACCTTGGTCACCGTCTCCTCA | 6 | | |
| JH2 117 | GATTGGTACTTCGATTTATGGGGGAGAGGTACCTTGGTCACCGTCTCCTCA | 6 | | |
| JH5 120 | GATAATTGGTTCGATCCATGGGGACAGGGTACATTGGTCACCGTCTCCTCA | 6 | | |
| JH5 121 | TATAACTGGTTCGATCCCTGGGGACAGGGTACATTGGTCACCGTCTCCTCA | 6 | | |
| JH5 122 | CACAATTGGTTCGACCCCTGGGGACAGGGTACATTGGTCACCGTCTCCTCA | 6 | | |
| JH5 123 | TTCAATTGGTTTGATCCCTGGGGACAGGGTACATTGGTCACCGTCTCCTCA | 6 | | |
| JH5 124 | AGCAACTGGTTCGACCCATGGGGACAGGGTACATTGGTCACCGTCTCCTCA | 6 | | |
| JH5 125 | AGAAACTGGTTTGATCCATGGGGACAGGGTACATTGGTCACCGTCTCCTCA | 6 | | |
| JH5 126 | TTAAATTGGTTCGACCCTTGGGGACAGGGTACATTGGTCACCGTCTCCTCA | 6 | | |
| JH5 127 | CCCAATTGGTTTGATCCATGGGGACAGGGTACATTGGTCACCGTCTCCTCA | 6 | | |
| JH5 128 | ATAAATTGGTTCGACCCTTGGGGACAGGGTACATTGGTCACCGTCTCCTCA | 6 | | |
| JH5 129 | ACTAACTGGTTTGACCCATGGGGACAGGGTACATTGGTCACCGTCTCCTCA | 6 | | |
| JH5 130 | GGTAACTGGTTTGACCCATGGGGACAGGGTACATTGGTCACCGTCTCCTCA | 6 | | |
| JH5 131 | GTGAACTGGTTTGATCCCTGGGGACAGGGTACATTGGTCACCGTCTCCTCA | 6 | | |
| JH5 132 | GCCAACTGGTTCGATCCCTGGGGACAGGGTACATTGGTCACCGTCTCCTCA | 6 | | |
| JH5 133 | AACAATTGGTTCGACCCATGGGGACAGGGTACATTGGTCACCGTCTCCTCA | 6 | | |
| JH6 134 | TACTACGGCATGGATGTATGGGGCCAGGGAACAACTGTCACCGTCTCCTCA | 6 | | |
| JH6 136 | GATTATGGAATGGACGTATGGGGCCAGGGAACAACTGTCACCGTCTCCTCA | 6 | | |
| JH6 137 | TTCTACGGTATGGATGTCTGGGGCCAGGGAACAACTGTCACCGTCTCCTCA | 6 | | |
| JH6 138 | CATTACGGAATGGACGTATGGGGCCAGGGAACAACTGTCACCGTCTCCTCA | 6 | | |
| JH6 139 | TTGTACGGAATGGACGTTTGGGGCCAGGGAACAACTGTCACCGTCTCCTCA | 6 | | |
| JH6 140 | AACTATGGCATGGATGTGTGGGGCCAGGGAACAACTGTCACCGTCTCCTCA | 6 | | |
| JH2 118 | CWCTGGTACTTCGACCTATGGGGGAGAGGTACCTTGGTCACCGTCTCCTCA | 6 | YES | |
| JH6 141 | SCATACGGAATGGACGTATGGGGCCAGGGAACAACTGTCACCGTCTCCTCA | 6 | YES | |
| JH1 115 | SCAGAATACTTCCAACACTGGGGACAGGGTACATTGGTCACCGTCTCCTCA | 6 | YES | |
| JH6 135 | KACTACTACATGGACGTATGGGGCAAGGGTACAACTGTCACCGTCTCCTCA | 6 | YES | |
| JH6 199 | GKATACGGAATGGACGTATGGGGCCAGGGAACAACTGTCACCGTCTCCTCA | 6 | YES | |
| JH6 142 | AKCTACGGAATGGACGTATGGGGCCAGGGAACAACTGTCACCGTCTCCTCA | 6 | YES | |
| JH2 119 | ARCTGGTACTTCGACCTATGGGGGAGAGGTACCTTGGTCACCGTCTCCTCA | 6 | YES | |
| JH6 143 | ASATACGGAATGGACGTATGGGGCCAGGGAACAACTGTCACCGTCTCCTCA | 6 | YES | |
| JH6 144 | TATTACTATGGTATGGATGTATGGGGCCAGGGAACAACTGTCACCGTCTCCTCA | 7 | | |
| JH6 145 | TATTACTATTATATGGATGTTTGGGGCAAGGGTACAACTGTCACCGTCTCCTCA | 7 | | |
| JH6 146 | GATTACTACGGCATGGATGTTTGGGGCCAGGGAACAACTGTCACCGTCTCCTCA | 7 | | |
| JH6 148 | AACTACTACGGCATGGACGTATGGGGCCAGGGAACAACTGTCACCGTCTCCTCA | 7 | | |
| JH6 147 | CWCTACTACGGAATGGACGTATGGGGCCAGGGAACAACTGTCACCGTCTCCTCA | 7 | YES | |
| JH6 150 | RACTACTACTACATGGACGTATGGGGCAAGGGTACAACTGTCACCGTCTCCTCA | 7 | YES | |
| JH6 149 | YCATACTACGGAATGGACGTATGGGGCCAGGGAACAACTGTCACCGTCTCCTCA | 7 | YES | |
| JH6 151 | TATTACTACTACGGAATGGACGTTTGGGGCCAGGGAACAACTGTCACCGTCTCCTCA | 8 | | |
| JH6 152 | AATTATTATTACGGCATGGACGTCTGGGGCCAGGGAACAACTGTCACCGTCTCCTCA | 8 | | |
| JH6 153 | GATTACTATTACGGTATGGATGTCTGGGGCCAGGGAACAACTGTCACCGTCTCCTCA | 8 | | |
| JH6 154 | CACTATTACTACGGCATGGACGTATGGGGCCAGGGAACAACTGTCACCGTCTCCTCA | 8 | | |
| JH6 155 | TTCTATTATTATGGTATGGATGTTTGGGGCCAGGGAACAACTGTCACCGTCTCCTCA | 8 | | |
| JH6 156 | TCTTACTACTATGGGATGGACGTGTGGGGCCAGGGAACAACTGTCACCGTCTCCTCA | 8 | | |
| JH6 157 | AGATATTACTACGGCATGGATGTTTGGGGCCAGGGAACAACTGTCACCGTCTCCTCA | 8 | | |
| JH6 158 | TTATACTACTATGGGATGGATGTATGGGGCCAGGGAACAACTGTCACCGTCTCCTCA | 8 | | |
| JH6 159 | CCTTACTACTATGGCATGGACGTCTGGGGCCAGGGAACAACTGTCACCGTCTCCTCA | 8 | | |
| JH6 160 | ACCTATTACTATGGTATGGATGTGTGGGGCCAGGGAACAACTGTCACCGTCTCCTCA | 8 | | |
| JH6 161 | GGATACTACTATGGGATGGACGTCTGGGGCCAGGGAACAACTGTCACCGTCTCCTCA | 8 | | |
| JH6 163 | GCCTACTATTATGGCATGGACGTCTGGGGCCAGGGAACAACTGTCACCGTCTCCTCA | 8 | | |
| JH6 164 | TACTACTATTATTATATGGACGTTTGGGGCAAGGGTACAACTGTCACCGTCTCCTCA | 8 | | |
| JH6 165 | AACTACTACTACTATATGGATGTTTGGGGCAAGGGTACAACTGTCACCGTCTCCTCA | 8 | | |
| JH6 166 | GATTATTATTACTATATGGACGTCTGGGGCAAGGGTACAACTGTCACCGTCTCCTCA | 8 | | |
| JH6 162 | RTATACTACTACGGAATGGACGTATGGGGCCAGGGAACAACTGTCACCGTCTCCTCA | 8 | YES | |
| JH6 168 | RGCTACTACTACTACATGGACGTATGGGGCAAGGGTACAACTGTCACCGTCTCCTCA | 8 | YES | |
| JH6 167 | CMCTACTACTACTACATGGACGTATGGGGCAAGGGTACAACTGTCACCGTCTCCTCA | 8 | YES | |
| JH6 169 | TATTACTATTATTACGGGATGGACGTATGGGGCCAGGGAACAACTGTCACCGTCTCCTCA | 9 | | |
| JH6 170 | AATTATTATTATTATGGGATGGACGTATGGGGCCAGGGAACAACTGTCACCGTCTCCTCA | 9 | | |
| JH6 171 | GACTATTACTATTATGGAATGGATGTATGGGGCCAGGGAACAACTGTCACCGTCTCCTCA | 9 | | |
| JH6 172 | CATTATTATTATTACGGAATGGACGTTTGGGGCCAGGGAACAACTGTCACCGTCTCCTCA | 9 | | |
| JH6 173 | TTCTATTACTATTATGGCATGGATGTGTGGGGCCAGGGAACAACTGTCACCGTCTCCTCA | 9 | | |
| JH6 174 | AGCTACTACTATTATGGTATGGACGTGTGGGGCCAGGGAACAACTGTCACCGTCTCCTCA | 9 | | |
| JH6 175 | AGATATTACTACTATGGCATGGATGTCTGGGGCCAGGGAACAACTGTCACCGTCTCCTCA | 9 | | |
| JH6 176 | TTATACTACTATTACGGCATGGATGTCTGGGGCCAGGGAACAACTGTCACCGTCTCCTCA | 9 | | |
| JH6 177 | CCCTATTATTACTACGGAATGGACGTTTGGGGCCAGGGAACAACTGTCACCGTCTCCTCA | 9 | | |
| JH6 178 | ATCTATTACTATTATGGCATGGATGTATGGGGCCAGGGAACAACTGTCACCGTCTCCTCA | 9 | | |
| JH6 179 | ACCTATTACTACTATGGCATGGACGTTTGGGGCCAGGGAACAACTGTCACCGTCTCCTCA | 9 | | |
| JH6 180 | GGCTACTATTACTATGGGATGGACGTCTGGGGCCAGGGAACAACTGTCACCGTCTCCTCA | 9 | | |
| JH6 181 | GTCTACTATTATTATGGCATGGACGTATGGGGCCAGGGAACAACTGTCACCGTCTCCTCA | 9 | | |
| JH6 182 | GCTTACTATTACTACGGCATGGACGTATGGGGCCAGGGAACAACTGTCACCGTCTCCTCA | 9 | | |
| JH6 183 | GATTATTATTATTACTATGGTATGGATGTGTGGGGCCAGGGAACAACTGTCACCGTCTCCTCA | 10 | | |
| JH6 184 | TACTATTACTACTATTATGGCATGGACGTCTGGGGCCAGGGAACAACTGTCACCGTCTCCTCA | 10 | | |
| JH6 185 | CACTACTACTATTATTATGGGATGGACGTATGGGGCCAGGGAACAACTGTCACCGTCTCCTCA | 10 | | |
| JH6 186 | TTCTATTATTATTATTACGGAATGGACGTCTGGGGCCAGGGAACAACTGTCACCGTCTCCTCA | 10 | | |
| JH6 187 | AGCTACTATTACTACTATGGGATGGATGTGTGGGGCCAGGGAACAACTGTCACCGTCTCCTCA | 10 | | |
| JH6 188 | AGATATTACTATTACTATGGTATGGACGTTTGGGGCCAGGGAACAACTGTCACCGTCTCCTCA | 10 | | |
| JH6 189 | TTGTACTATTACTATTATGGAATGGACGTTTGGGGCCAGGGAACAACTGTCACCGTCTCCTCA | 10 | | |
| JH6 190 | CCTTACTATTATTATTATGGGATGGATGTCTGGGGCCAGGGAACAACTGTCACCGTCTCCTCA | 10 | | |
| JH6 191 | ATATATTACTATTACTACGGGATGGATGTTTGGGGCCAGGGAACAACTGTCACCGTCTCCTCA | 10 | | |
| JH6 192 | ACCTACTATTATTATTACGGGATGGACGTTTGGGGCCAGGGAACAACTGTCACCGTCTCCTCA | 10 | | |
| JH6 193 | GGTTACTATTATTACTACGGGATGGACGTGTGGGGCCAGGGAACAACTGTCACCGTCTCCTCA | 10 | | |
| JH6 194 | GTGTATTACTATTACTACGGGATGGACGTGTGGGGCCAGGGAACAACTGTCACCGTCTCCTCA | 10 | | |
| JH6 195 | GCCTATTACTACTACTATGGGATGGATGTGTGGGGCCAGGGAACAACTGTCACCGTCTCCTCA | 10 | | |
| JH6 196 | AATTATTATTACTATTACGGTATGGACGTGTGGGGCCAGGGAACAACTGTCACCGTCTCCTCA | 10 | | |
| JH6 197 | KACTACTACTACTACTACTACATGGACGTATGGGGCAAGGGTACAACTGTCACCGTCTCCTCA | 10 | YES | |

**Table 32. Theoretical segment pool of unique H3-JH polypeptide segments encoded by the oligonucleotides of Table 31.**

| **Name** | **Sequence** | **Length** | **SEQ ID NO** |
|---|---|---|---|
| PJH4_001 | | 0 | |
| PJH1_002 | H | 1 | |
| PJH1_003 | I | 1 | |
| PJH1_004 | Y | 1 | |
| PJH1_005 | p | 1 | |
| PJH1_006 | V | 1 | |
| PJH1_007 | D | 1 | |
| PJH1_008 | F | 1 | |
| PJH1_009 | N | 1 | |
| PJH1_010 | S | 1 | |
| PJH1_011 | T | 1 | |
| PJH1_200 | A | 1 | |
| PJH4_013 | DY | 2 | |
| PJH4_016 | AY | 2 | |
| PJH4_017 | FY | 2 | |
| PJH4_018 | GY | 2 | |
| PJH4_019 | HY | 2 | |
| PJH4_023 | SY | 2 | |
| PJH4_024 | VY | 2 | |
| PJH4_025 | YY | 2 | |
| PJH3_012A | NI | 2 | |
| PJH3_012B | DI | 2 | |
| PJH3_015A | NV | 2 | |
| PJH3_015B | DV | 2 | |
| PJH4_020A | LY | 2 | |
| PJH4_020B | IY | 2 | |
| PJH4_021A | NY | 2 | |
| PJH4_021B | TY | 2 | |
| PJH4_022A | PY | 2 | |
| PJH4_022B | RY | 2 | |
| PJH5_014A | NP | 2 | |
| PJH5_014B | DP | 2 | |
| PJH4_029 | FDY | 3 | |
| PJH4_030 | IDY | 3 | |
| PJH4_031 | VDY | 3 | |
| PJH4_032 | LDY | 3 | |
| PJH4_033 | SDY | 3 | |
| PJH4_034 | HDY | 3 | |
| PJH4_035 | RDY | 3 | |
| PJH4_036 | PDY | 3 | |
| PJH4_037 | NDY | 3 | |
| PJH4_038 | TDY | 3 | |
| PJH4_039 | DDY | 3 | |
| PJH4_040 | GDY | 3 | |
| PJH4_041 | ADY | 3 | |
| PJH4_042 | YDY | 3 | |
| PJH5_043 | FDP | 3 | |
| PJH6_044 | MDV | 3 | |
| PJH1_026A | LQH | 3 | |
| PJH1_026B | FQH | 3 | |
| PJH2_027A | FDL | 3 | |
| PJH2_027B | LDL | 3 | |
| PJH3_028A | FDI | 3 | |
| PJH3_028B | LDI | 3 | |
| PJH3_046A | VDV | 3 | |
| PJH3_046B | LDV | 3 | |
| PJH5_045A | LDP | 3 | |
| PJH5_045B | SDP | 3 | |
| PJH3_049 | AFDI | 4 | |
| PJH4_051 | YFDY | 4 | |
| PJH4_052 | NFDY | 4 | |
| PJH4_053 | DFDY | 4 | |
| PJH4_054 | HFDY | 4 | |
| PJH4_055 | FFDY | 4 | |
| PJH4_056 | SFDY | 4 | |
| PJH4_057 | RFDY | 4 | |
| PJH4_058 | LFDY | 4 | |
| PJH4_059 | PFDY | 4 | |
| PJH4_060 | IFDY | 4 | |
| PJH4_061 | TFDY | 4 | |
| PJH4_062 | GFDY | 4 | |
| PJH4_063 | VFDY | 4 | |
| PJH4_064 | AFDY | 4 | |
| PJH5_065 | WFDP | 4 | |
| PJH6_068 | GMDV | 4 | |
| PJH1_047A | YFQH | 4 | |
| PJH1_047B | DFQH | 4 | |
| PJH2_048A | DFDL | 4 | |
| PJH2_048B | YFDL | 4 | |
| PJH3_050A | PFDI | 4 | |
| PJH3_050B | SFDI | 4 | |
| PJH3_198A | VFDI | 4 | |
| PJH3_198B | LFDI | 4 | |
| PJH5_066A | RFDP | 4 | |
| PJH5_066B | SFDP | 4 | |
| PJH5_067A | GFDP | 4 | |
| PJH5_067B | AFDP | 4 | |
| PJH6_069A | YMDV | 4 | |
| PJH6_069B | DMDV | 4 | |
| PJH6_070A | PMDV | 4 | |
| PJH6_070B | SMDV | 4 | |
| PJH2_072 | WYFDL | 5 | |
| PJH3_075 | DAFDI | 5 | |
| PJH3_076 | YAFDI | 5 | |
| PJH3_077 | HAFDI | 5 | |
| PJH3_078 | FAFDI | 5 | |
| PJH3_079 | SAFDI | 5 | |
| PJH3_080 | RAFDI | 5 | |
| PJH3_081 | LAFDI | 5 | |
| PJH3_082 | GAFDI | 5 | |
| PJH4_086 | DYFDY | 5 | |
| PJH4_087 | YYFDY | 5 | |
| PJH4_088 | HYFDY | 5 | |
| PJH4_089 | FYFDY | 5 | |
| PJH4_090 | SYFDY | 5 | |
| PJH4_091 | RYFDY | 5 | |
| PJH4_092 | LYFDY | 5 | |
| PJH4_093 | PYFDY | 5 | |
| PJH4_095 | GYFDY | 5 | |
| PJH4_096 | VYFDY | 5 | |
| PJH4_097 | AYFDY | 5 | |
| PJH4_098 | NYFDY | 5 | |
| PJH5_099 | NWFDP | 5 | |
| PJH5_100 | DWFDP | 5 | |
| PJH5_101 | YWFDP | 5 | |
| PJH5_102 | HWFDP | 5 | |
| PJH5_103 | FWFDP | 5 | |
| PJH5_104 | SWFDP | 5 | |
| PJH5_105 | RWFDP | 5 | |
| PJH5_107 | GWFDP | 5 | |
| PJH5_109 | AWFDP | 5 | |
| PJH6_110 | YGMDV | 5 | |
| PJH6_112 | DGMDV | 5 | |
| PJH1_071A | EYFQH | 5 | |
| PJH1_071B | KYFQH | 5 | |
| PJH2_073A | SYFDL | 5 | |
| PJH2_073B | GYFDL | 5 | |
| PJH2_074A | RYFDL | 5 | |
| PJH2_074B | LYFDL | 5 | |
| PJH3_083A | IAFDI | 5 | |
| PJH3_083B | VAFDI | 5 | |
| PJH3_084A | PAFDI | 5 | |
| PJH3_084B | AAFDI | 5 | |
| PJH3_085A | NAFDI | 5 | |
| PJH3_085B | TAFDI | 5 | |
| PJH4_094A | IYFDY | 5 | |
| PJH4_094B | TYFDY | 5 | |
| PJH5_106A | PWFDP | 5 | |
| PJH5_106B | TWFDP | 5 | |
| PJH5_108A | IWFDP | 5 | |
| PJH5_108B | VWFDP | 5 | |
| PJH6_111A | YYMDV | 5 | |
| PJH6_111B | DYMDV | 5 | |
| PJH6_113A | HGMDV | 5 | |
| PJH6_113B | I3GMDV | 5 | |
| PJH6_114A | SGMDV | 5 | |
| PJH6_114B | AGMDV | 5 | |
| PJH2_116 | YWYFDL | 6 | |
| PJH2_117 | DWYFDL | 6 | |
| PJH5_120 | DNWFDP | 6 | |
| PJH5_121 | YNWFDP | 6 | |
| PJH5_122 | HNWFDP | 6 | |
| PJH5_123 | FNWFDP | 6 | |
| PJH5_124 | SNWFDP | 6 | |
| PJH5_125 | RNWFDP | 6 | |
| PJH5_126 | LNWFDP | 6 | |
| PJH5_127 | PNWFDP | 6 | |
| PJH5_128 | INWFDP | 6 | |
| PJH5_129 | TNWFDP | 6 | |
| PJH5_130 | GWFDP | 6 | |
| PJH5_131 | VNWFDP | 6 | |
| PJH5_132 | ANWFDP | 6 | |
| PJH5_133 | NNWFDP | 6 | |
| PJH6_134 | YYGMDV | 6 | |
| PJH6_136 | DYGMDV | 6 | |
| PJH6_137 | FYGMDV | 6 | |
| PJH6_138 | GYGMDV | 6 | |
| PJH6_139 | GYGMDV | 6 | |
| PJH6_140 | NYGMDV | 6 | |
| PJH1_115A | AEYFQH | 6 | |
| PJH1_115B | PEYFQH | 6 | |
| PJH2_118A | LWYFDL | 6 | |
| PJH2_118B | HWYFDL | 6 | |
| PJH2_119A | NWYFDL | 6 | |
| PJH2_119B | SWYFDL | 6 | |
| PJH6_135A | DYYMDV | 6 | |
| PJH6_135B | YYYMDV | 6 | |
| PJH6_141A | AYGMDV | 6 | |
| PJH6_141B | PYGMDV | 6 | |
| PJH6_142A | SYGMDV | 6 | |
| PJH6_142B | GYGMDV | 6 | |
| PJH6_143A | TYGMDV | 6 | |
| PJH6_143B | RYGMDV | 6 | |
| PJH6_199A | GYGMDV | 6 | |
| PJH6_199B | VYGMDV | 6 | |
| PJH6_144 | YYYGMDV | 7 | |
| PJH6_145 | YYYYMDV | 7 | |
| PJH6_146 | DYYGMDV | 7 | |
| PJH6_148 | NYYGMDV | 7 | |
| PJH6_147A | LYYGMDV | 7 | |
| PJH6_147B | HYYGMDV | 7 | |
| PJH6_149A | SYYGMDV | 7 | |
| PJH6_149B | PYYGMDV | 7 | |
| PJH6_150A | NYYYMDV | 7 | |
| PJH6_150B | DYYYMDV | 7 | |
| PJH6_151 | YYYYGMDV | 8 | |
| PJH6_152 | NYYYGMDV | 8 | |
| PJH6_153 | DYYYGMDV | 8 | |
| PJH6_154 | HYYYGMDV | 8 | |
| PJH6_155 | FYYYGMDV | 8 | |
| PJH6_156 | SYYYGMDV | 8 | |
| PJH6_157 | RYYYGMDV | 8 | |
| PJH6_158 | LYYYGMDV | 8 | |
| PJH6_159 | PYYYGMDV | 8 | |
| PJH6_160 | TYYYGMDV | 8 | |
| PJH6_161 | GYYYGMDV | 8 | |
| PJH6_163 | AYYYGMDV | 8 | |
| PJH6_164 | YYYYYMDV | 8 | |
| PJH6_165 | NYYYYMDV | 8 | |
| PJH6_166 | DYYYYMDV | 8 | |
| PJH6_162A | VYYYGMDV | 8 | |
| PJH6_162B | IYYYGMDV | 8 | |
| PJH6_167A | HYYYYMDV | 8 | |
| PJH6_167B | PYYYYMDV | 8 | |
| PJH6_168A | SYYYYMDV | 8 | |
| PJH6_168B | GYYYYMDV | 8 | |
| PJH6_169 | YYYYYGMDV | 9 | |
| PJH6_170 | NYYYYGMDV | 9 | |
| PJH6_171 | DYYYYGMDV | 9 | |
| PJH6_172 | HYYYYGMDV | 9 | |
| PJH6_173 | PYYYYGMDV | 9 | |
| PJH6_174 | SYYYYGMDV | 9 | |
| PJH6_175 | RYYYYGMDV | 9 | |
| PJH6_176 | LYYYYGMDV | 9 | |
| PJH6_177 | PYYYYGMDV | 9 | |
| PJH6_178 | IYYYYGMDV | 9 | |
| PJH6_179 | TYYYYGMDV | 9 | |
| PJH6_180 | GYYYYGMDV | 9 | |
| PJH6_181 | VYYYYGMDV | 9 | |
| PJH6_182 | AYYYYGMDV | 9 | |
| PJH6_183 | DYYYYYGMDV | 10 | |
| PJH6_184 | YYYYYYGMDV | 10 | |
| PJH6_185 | HYYYYYGMDV | 10 | |
| PJH6_186 | FYYYYYGMDV | 10 | |
| PJH6_187 | YYYYYGMDV | 10 | |
| PJH6_188 | RYYYYYGMDV | 10 | |
| PJH6_189 | LYYYYYGMDV | 10 | |
| PJH6_190 | PYYYYYGMDV | 10 | |
| PJH6_191 | IYYYYYGMDV | 10 | |
| PJH6_192 | TYYYYYGMDV | 10 | |
| PJH6_193 | GYYYYYGMDV | 10 | |
| PJH6_194 | VYYYYYGMDV | 10 | |
| PJH6_195 | AYYYYYGMDV | 10 | |
| PJH6_196 | NYYYYYGMDV | 10 | |
| PJH6_197A | DYYYYYYMDV | 10 | |
| PJH6_197B | YYYYYYYMDV | 10 | |

### EQUIVALENTS

Those skilled in the art will recognize, or be able to ascertain using no more than routine experimentation, many equivalents to the specific embodiments and methods described herein. Such equivalents are intended to be encompassed by the scope of the following claims.

### APPENDIX A

### GI NUMBERS OF 3,571 SEQUENCES IN THE HEALTHY PREIMMUNE SET (HPS)

| | | | | |
|---|---|---|---|---|
| 33628 | 587301 | 1154807 | 1685210 | 1770797 |
| 37745 | 587304 | 1154808 | 1685220 | 1770799 |
| 37747 | 587306 | 1154809 | 1685222 | 1770800 |
| 37749 | 587308 | 1154810 | 1685228 | 1770801 |
| 37751 | 587311 | 1154811 | 1685234 | 1770805 |
| 37753 | 587313 | 1154813 | 1685238 | 1770806 |
| 37755 | 587315 | 1154818 | 1685240 | 1770807 |
| 37757 | 587317 | 1154820 | 1685242 | 1770808 |
| 37759 | 1052611 | 1154822 | 1685246 | 1770809 |
| 37761 | 1052620 | 1154824 | 1685248 | 1770810 |
| 37763 | 1052622 | 1154825 | 1685250 | 1770811 |
| 37765 | 1052626 | 1154834 | 1685252 | 1770812 |
| 37767 | 1052627 | 1154837 | 1685254 | 1770813 |
| 37769 | 1052634 | 1154838 | 1685256 | 1770814 |
| 37773 | 1052637 | 1154839 | 1685258 | 1770815 |
| 37777 | 1052639 | 1154840 | 1685260 | 1770816 |
| 38383 | 1052640 | 1154841 | 1685264 | 1770817 |
| 38391 | 1052642 | 1154843 | 1685266 | 1770818 |
| 38393 | 1052644 | 1154844 | 1685268 | 1770820 |
| 38397 | 1052655 | 1154845 | 1770744 | 1770822 |
| 38401 | 1052656 | 1154847 | 1770746 | 1770824 |
| 185292 | 1052657 | 1154848 | 1770747 | 1770826 |
| 264183 | 1052658 | 1197299 | 1770751 | 1770829 |
| 297147 | 1052659 | 1197300 | 1770755 | 1770830 |
| 306949 | 1052662 | 1197304 | 1770756 | 1770831 |
| 306951 | 1052668 | 1197307 | 1770758 | 1770833 |
| 306953 | 1052669 | 1197308 | 1770759 | 1770835 |
| 483332 | 1052671 | 1197309 | 1770761 | 1770836 |
| 483333 | 1052674 | 1197312 | 1770763 | 1770837 |
| 483335 | 1052676 | 1197313 | 1770765 | 1770839 |
| 483336 | 1052683 | 1197314 | 1770766 | 1770840 |
| 483338 | 1052685 | 1197315 | 1770770 | 1770843 |
| 483339 | 1052691 | 1197316 | 1770771 | 1770844 |
| 483348 | 1052692 | 1197318 | 1770772 | 1770845 |
| 483350 | 1052693 | 1197319 | 1770775 | 1770846 |
| 510999 | 1052695 | 1197321 | 1770776 | 1770847 |
| 547164 | 1154682 | 1197322 | 1770777 | 1770848 |
| 587252 | 1154691 | 1197323 | 1770779 | 1770851 |
| 587254 | 1154698 | 1197324 | 1770780 | 1770852 |
| 587266 | 1154699 | 1197325 | 1770783 | 1770853 |
| 587276 | 1154706 | 1197326 | 1770784 | 1770854 |
| 587278 | 1154710 | 1197327 | 1770785 | 1770855 |
| 587280 | 1154713 | 1197328 | 1770789 | 1770860 |
| 587286 | 1154715 | 1495508 | 1770791 | 1770861 |
| 587288 | 1154724 | 1495511 | 1770792 | 1770865 |
| 587291 | 1154754 | 1495512 | 1770793 | 1770866 |
| 587293 | 1154769 | 1495516 | 1770794 | 1770867 |
| 587295 | 1154770 | 1495518 | 1770795 | 1770869 |
| 587299 | 1154805 | 1592729 | 1770796 | 1770870 |
| 1770872 | 1770969 | 1791028 | 1791182 | 3170670 |
| 1770874 | 1770971 | 1791030 | 1791184 | 3170686 |
| 1770875 | 1770972 | 1791032 | 1791186 | 3170688 |
| 1770876 | 1770974 | 1791034 | 1791190 | 3170692 |
| 1770877 | 1770976 | 1791036 | 1791194 | 3170694 |
| 1770878 | 1770979 | 1791040 | 1791196 | 3170696 |
| 1770879 | 1770981 | 1791042 | 1791200 | 3170702 |
| 1770880 | 1770982 | 1791046 | 1791204 | 3170704 |
| 1770881 | 1770983 | 1791050 | 1791206 | 3170712 |
| 1770882 | 1770989 | 1791052 | 1869905 | 3170714 |
| 1770883 | 1770992 | 1791054 | 1869907 | 3170716 |
| 1770884 | 1770994 | 1791058 | 1869912 | 3170720 |
| 1770885 | 1770995 | 1791060 | 1869913 | 3170722 |
| 1770887 | 1770997 | 1791062 | 1869915 | 3170726 |
| 1770888 | 1770998 | 1791064 | 1869918 | 3170728 |
| 1770891 | 1771002 | 1791072 | 1869919 | 3170730 |
| 1770892 | 1771004 | 1791074 | 1934921 | 3170734 |
| 1770893 | 1771008 | 1791076 | 2367538 | 3170736 |
| 1770894 | 1771010 | 1791078 | 2388836 | 3170738 |
| 1770895 | 1771014 | 1791080 | 2388837 | 3170740 |
| 1770896 | 1771016 | 1791082 | 2388839 | 3170748 |
| 1770898 | 1771017 | 1791084 | 2388840 | 3170752 |
| 1770902 | 1771018 | 1791086 | 2388841 | 3170754 |
| 1770904 | 1771022 | 1791088 | 2388842 | 3170756 |
| 1770905 | 1771026 | 1791090 | 2388843 | 3170758 |
| 1770906 | 1771027 | 1791096 | 2388846 | 3170760 |
| 1770908 | 1771029 | 1791098 | 2388847 | 3170762 |
| 1770909 | 1771033 | 1791100 | 2388848 | 3170764 |
| 1770910 | 1771034 | 1791104 | 2388851 | 3170766 |
| 1770911 | 1771035 | 1791106 | 2388852 | 3170768 |
| 1770912 | 1771036 | 1791108 | 2388853 | 3170772 |
| 1770913 | 1771038 | 1791110 | 2388856 | 3170774 |
| 1770914 | 1771039 | 1791112 | 2388859 | 3170778 |
| 1770915 | 1771042 | 1791114 | 2388861 | 3170782 |
| 1770916 | 1771044 | 1791116 | 2388862 | 3170784 |
| 1770918 | 1771045 | 1791118 | 2388863 | 3170786 |
| 1770922 | 1771055 | 1791122 | 2388864 | 3170788 |
| 1770932 | 1771057 | 1791124 | 2388865 | 3170794 |
| 1770936 | 1771058 | 1791130 | 2388868 | 3170796 |
| 1770937 | 1771059 | 1791132 | 2388871 | 3170802 |
| 1770950 | 1771060 | 1791134 | 2388873 | 3170808 |
| 1770952 | 1771061 | 1791136 | 2388875 | 3170810 |
| 1770954 | 1771063 | 1791142 | 2388876 | 3170812 |
| 1770958 | 1791008 | 1791144 | 2388878 | 3170816 |
| 1770961 | 1791010 | 1791152 | 2773082 | 3170820 |
| 1770962 | 1791012 | 1791154 | 3170658 | 3170822 |
| 1770963 | 1791018 | 1791160 | 3170662 | 3170824 |
| 1770964 | 1791020 | 1791164 | 3170664 | 3170826 |
| 1770967 | 1791026 | 1791176 | 3170668 | 3170830 |
| 3170832 | 3170974 | 4995349 | 4995496 | 5834071 |
| 3170834 | 3170978 | 4995351 | 4995498 | 5834073 |
| 3170836 | 3170980 | 4995353 | 4995500 | 5834075 |
| 3170840 | 3170984 | 4995355 | 4995502 | 5834077 |
| 3170842 | 3170986 | 4995357 | 4995504 | 5834079 |
| 3170844 | 3170988 | 4995359 | 4995506 | 5834081 |
| 3170846 | 3170990 | 4995361 | 4995508 | 5834083 |
| 3170848 | 3170992 | 4995365 | 4995510 | 5834089 |
| 3170852 | 3171006 | 4995367 | 4995512 | 5834091 |
| 3170854 | 3171008 | 4995375 | 4995514 | 5834093 |
| 3170856 | 3171010 | 4995383 | 4995516 | 5834095 |
| 3170858 | 3171016 | 4995385 | 4995520 | 5834097 |
| 3170862 | 3171018 | 4995389 | 4995524 | 5834099 |
| 3170864 | 3171020 | 4995391 | 4995530 | 5834101 |
| 3170866 | 3171022 | 4995393 | 4995535 | 5834103 |
| 3170868 | 3171024 | 4995397 | 4995537 | 5834105 |
| 3170870 | 3171026 | 4995399 | 4995539 | 5834107 |
| 3170872 | 3171028 | 4995400 | 4995549 | 5834109 |
| 3170874 | 3171030 | 4995404 | 4995555 | 5834113 |
| 3170876 | 3171038 | 4995406 | 4995557 | 5834115 |
| 3170878 | 3171040 | 4995408 | 4995563 | 5834119 |
| 3170880 | 3171042 | 4995410 | 4995569 | 5834121 |
| 3170882 | 3171044 | 4995418 | 4995575 | 5834123 |
| 3170884 | 3171242 | 4995422 | 4995581 | 5834125 |
| 3170890 | 3608440 | 4995426 | 4995589 | 5834127 |
| 3170894 | 3608462 | 4995428 | 4995591 | 5834129 |
| 3170898 | 3954953 | 4995430 | 5833973 | 5834131 |
| 3170902 | 3954955 | 4995432 | 5833980 | 5834133 |
| 3170908 | 4530538 | 4995434 | 5833984 | 5834135 |
| 3170910 | 4530544 | 4995436 | 5833986 | 5834137 |
| 3170916 | 4753741 | 4995438 | 5834003 | 5834139 |
| 3170918 | 4959477 | 4995440 | 5834011 | 5834141 |
| 3170922 | 4995315 | 4995442 | 5834015 | 5834143 |
| 3170924 | 4995317 | 4995446 | 5834019 | 5834145 |
| 3170926 | 4995319 | 4995456 | 5834031 | 5834147 |
| 3170930 | 4995321 | 4995462 | 5834035 | 5834149 |
| 3170932 | 4995323 | 4995466 | 5834037 | 5834151 |
| 3170934 | 4995325 | 4995470 | 5834039 | 5834153 |
| 3170936 | 4995327 | 4995474 | 5834041 | 5834155 |
| 3170938 | 4995329 | 4995476 | 5834043 | 5834159 |
| 3170944 | 4995331 | 4995478 | 5834047 | 5834161 |
| 3170946 | 4995333 | 4995480 | 5834049 | 5834163 |
| 3170954 | 4995335 | 4995482 | 5834051 | 5834165 |
| 3170958 | 4995337 | 4995484 | 5834053 | 5834169 |
| 3170960 | 4995339 | 4995486 | 5834055 | 5834175 |
| 3170964 | 4995341 | 4995488 | 5834057 | 5834177 |
| 3170966 | 4995343 | 4995490 | 5834059 | 5834179 |
| 3170968 | 4995345 | 4995492 | 5834065 | 5834183 |
| 3170970 | 4995347 | 4995494 | 5834069 | 5834185 |
| 5834187 | 6723583 | 8249790 | 11137303 | 11137445 |
| 5834191 | 6723595 | 8249812 | 11137305 | 11137448 |
| 5834193 | 6723597 | 8249816 | 11137307 | 11137450 |
| 5834195 | 6723599 | 8249826 | 11137309 | 11137452 |
| 5834197 | 7161042 | 8249828 | 11137313 | 11137454 |
| 5834199 | 7161061 | 8249838 | 11137315 | 11137460 |
| 5834201 | 7161129 | 8250248 | 11137317 | 11137462 |
| 5834203 | 7161136 | 8250255 | 11137319 | 11137467 |
| 5834205 | 7161164 | 8489274 | 11137322 | 11137470 |
| 5834207 | 8249510 | 8489276 | 11137326 | 11137474 |
| 5834209 | 8249514 | 8489278 | 11137329 | 11137476 |
| 5834213 | 8249518 | 8489280 | 11137333 | 11137480 |
| 5834215 | 8249524 | 8489282 | 11137335 | 11137482 |
| 6013039 | 8249528 | 8489284 | 11137339 | 11137487 |
| 6013043 | 8249538 | 8489286 | 11137343 | 11137494 |
| 6013045 | 8249546 | 8489289 | 11137348 | 11137500 |
| 6531445 | 8249552 | 8489291 | 11137350 | 11137502 |
| 6531457 | 8249554 | 11137164 | 11137352 | 11137507 |
| 6531461 | 8249558 | 11137170 | 11137354 | 11137509 |
| 6531465 | 8249560 | 11137172 | 11137359 | 13171905 |
| 6531481 | 8249562 | 11137174 | 11137361 | 13171909 |
| 6531489 | 8249566 | 11137178 | 11137363 | 13171911 |
| 6531493 | 8249568 | 11137183 | 11137365 | 13171913 |
| 6531495 | 8249608 | 11137186 | 11137367 | 13171915 |
| 6531507 | 8249622 | 11137188 | 11137369 | 13171917 |
| 6531509 | 8249632 | 11137196 | 11137371 | 13171921 |
| 6531511 | 8249650 | 11137200 | 11137373 | 13171923 |
| 6531513 | 8249652 | 11137205 | 11137375 | 13171925 |
| 6531517 | 8249654 | 11137215 | 11137377 | 13171927 |
| 6531521 | 8249656 | 11137219 | 11137379 | 13171929 |
| 6531525 | 8249662 | 11137229 | 11137382 | 13171931 |
| 6531533 | 8249674 | 11137231 | 11137386 | 13171935 |
| 6531537 | 8249682 | 11137242 | 11137388 | 13171937 |
| 6531539 | 8249698 | 11137251 | 11137392 | 13171939 |
| 6531554 | 8249712 | 11137253 | 11137399 | 13171941 |
| 6531600 | 8249716 | 11137261 | 11137403 | 13171945 |
| 6723523 | 8249718 | 11137262 | 11137407 | 13171947 |
| 6723525 | 8249730 | 11137274 | 11137411 | 13171949 |
| 6723527 | 8249738 | 11137276 | 11137413 | 13171951 |
| 6723531 | 8249740 | 11137279 | 11137415 | 13171953 |
| 6723535 | 8249744 | 11137281 | 11137418 | 13171955 |
| 6723537 | 8249754 | 11137283 | 11137420 | 13171957 |
| 6723543 | 8249756 | 11137285 | 11137422 | 13171959 |
| 6723545 | 8249760 | 11137289 | 11137426 | 13171961 |
| 6723549 | 8249772 | 11137290 | 11137428 | 13171965 |
| 6723551 | 8249778 | 11137293 | 11137430 | 13171967 |
| 6723558 | 8249784 | 11137295 | 11137432 | 13171969 |
| 6723565 | 8249786 | 11137298 | 11137439 | 13171971 |
| 6723581 | 8249788 | 11137301 | 11137441 | 13171973 |
| 13171975 | 14289057 | 21702275 | 47846432 | 47846574 |
| 13171977 | 14289061 | 21702277 | 47846434 | 47846578 |
| 13171981 | 14289065 | 21702281 | 47846438 | 47846580 |
| 13171987 | 14289067 | 21702282 | 47846442 | 47846582 |
| 13171999 | 14289071 | 21702287 | 47846446 | 47846586 |
| 13172003 | 14289073 | 21702289 | 47846448 | 47846588 |
| 13172005 | 14289079 | 21702291 | 47846450 | 47846590 |
| 13172007 | 14289097 | 21702293 | 47846456 | 47846594 |
| 13172009 | 14289099 | 21702295 | 47846458 | 47846596 |
| 13172013 | 14289109 | 21702297 | 47846466 | 47846598 |
| 13172019 | 14289111 | 21702299 | 47846468 | 47846600 |
| 13172021 | 16075408 | 21702301 | 47846472 | 47846602 |
| 13172025 | 16075410 | 21702303 | 47846476 | 47846604 |
| 13172027 | 16075412 | 21702305 | 47846478 | 47846606 |
| 13172033 | 16075414 | 21702307 | 47846482 | 47846612 |
| 13172037 | 16075416 | 21702309 | 47846484 | 47846614 |
| 13172043 | 16075418 | 21702311 | 47846486 | 47846618 |
| 13172045 | 16075420 | 21702313 | 47846488 | 47846620 |
| 13172053 | 16075422 | 21702314 | 47846490 | 47846626 |
| 13172061 | 16075424 | 21702315 | 47846492 | 47846632 |
| 13172065 | 16075426 | 23337033 | 47846494 | 47846644 |
| 13172069 | 16075428 | 27370812 | 47846498 | 47846646 |
| 13172073 | 16075430 | 31076438 | 47846506 | 47846658 |
| 13172083 | 16075432 | 33873883 | 47846508 | 47846660 |
| 13172091 | 16075434 | 33989177 | 47846510 | 47846664 |
| 13172093 | 16075436 | 37987904 | 47846512 | 47846666 |
| 13172099 | 16075438 | 37987932 | 47846514 | 47846674 |
| 13172117 | 16075440 | 37987938 | 47846516 | 47846678 |
| 13172125 | 16075442 | 37987960 | 47846518 | 47846680 |
| 13172129 | 16075444 | 37987970 | 47846520 | 47846684 |
| 13172133 | 16075448 | 39644659 | 47846524 | 47846690 |
| 13172135 | 16075450 | 39645530 | 47846526 | 47846692 |
| 13172137 | 16075452 | 47846366 | 47846528 | 47846696 |
| 13172141 | 16075454 | 47846370 | 47846530 | 47846698 |
| 13172143 | 16075456 | 47846372 | 47846532 | 47846716 |
| 13172147 | 16075458 | 47846376 | 47846534 | 47846718 |
| 13172149 | 16075460 | 47846378 | 47846538 | 47846724 |
| 13172151 | 16075464 | 47846380 | 47846540 | 47846728 |
| 13172155 | 16075466 | 47846386 | 47846542 | 47846730 |
| 13172157 | 16076270 | 47846388 | 47846544 | 47846734 |
| 13172159 | 16076286 | 47846394 | 47846546 | 47846750 |
| 13172163 | 17511791 | 47846398 | 47846548 | 47846752 |
| 13172169 | 18044958 | 47846416 | 47846550 | 47846756 |
| 13172177 | 19171939 | 47846418 | 47846558 | 47846762 |
| 13623574 | 19550754 | 47846420 | 47846562 | 47846764 |
| 14289029 | 19848531 | 47846422 | 47846564 | 47846768 |
| 14289035 | 19848533 | 47846426 | 47846566 | 47846778 |
| 14289037 | 19848543 | 47846428 | 47846570 | 47846782 |
| 14289049 | 19848545 | 47846430 | 47846572 | 47846784 |
| 47846786 | 54779180 | 54779286 | 54780237 | 54780833 |
| 49256420 | 54779182 | 54779288 | 54780239 | 54780835 |
| 49256426 | 54779184 | 54779290 | 54780243 | 54780837 |
| 49258105 | 54779186 | 54779292 | 54780247 | 54780839 |
| 49523831 | 54779188 | 54779296 | 54780251 | 54780841 |
| 49523833 | 54779190 | 54779298 | 54780253 | 54780843 |
| 49523835 | 54779192 | 54779300 | 54780259 | 54780845 |
| 49523837 | 54779194 | 54779302 | 54780709 | 54780847 |
| 49523841 | 54779196 | 54779306 | 54780711 | 54780853 |
| 49523843 | 54779198 | 54779308 | 54780713 | 54780857 |
| 49523849 | 54779200 | 54779310 | 54780715 | 54780859 |
| 49523851 | 54779204 | 54779314 | 54780717 | 54780861 |
| 49523853 | 54779206 | 54779316 | 54780719 | 54780863 |
| 49523855 | 54779208 | 54779318 | 54780721 | 55228577 |
| 49523861 | 54779210 | 54779320 | 54780723 | 55228579 |
| 49523865 | 54779212 | 54779322 | 54780731 | 55228584 |
| 49523871 | 54779214 | 54779324 | 54780733 | 55228638 |
| 49523873 | 54779218 | 54779328 | 54780735 | 55228640 |
| 49523879 | 54779220 | 54779330 | 54780741 | 55228646 |
| 49523881 | 54779222 | 54779332 | 54780745 | 55228650 |
| 49523887 | 54779224 | 54779334 | 54780753 | 55228651 |
| 49523895 | 54779226 | 54779336 | 54780757 | 55228652 |
| 49523905 | 54779228 | 54779338 | 54780759 | 60688113 |
| 49523919 | 54779230 | 54779340 | 54780761 | 74095346 |
| 49523921 | 54779232 | 54779342 | 54780763 | 74095348 |
| 49523923 | 54779234 | 54779344 | 54780765 | 74095350 |
| 49523927 | 54779236 | 54779350 | 54780767 | 74095355 |
| 49523929 | 54779238 | 54779354 | 54780771 | 74095358 |
| 49523931 | 54779240 | 54779356 | 54780775 | 91979763 |
| 49523946 | 54779242 | 54779358 | 54780777 | 91979789 |
| 49523950 | 54779244 | 54779360 | 54780779 | 91979839 |
| 54779136 | 54779248 | 54779362 | 54780781 | 91979849 |
| 54779140 | 54779250 | 54779364 | 54780783 | 111918091 |
| 54779142 | 54779252 | 54780155 | 54780785 | 111918116 |
| 54779144 | 54779256 | 54780163 | 54780787 | 111918127 |
| 54779146 | 54779258 | 54780167 | 54780791 | 111918184 |
| 54779148 | 54779260 | 54780171 | 54780793 | 111918251 |
| 54779150 | 54779262 | 54780177 | 54780795 | 111918262 |
| 54779152 | 54779264 | 54780179 | 54780801 | 111918647 |
| 54779156 | 54779266 | 54780185 | 54780803 | 121488404 |
| 54779158 | 54779268 | 54780187 | 54780805 | 145910925 |
| 54779160 | 54779270 | 54780191 | 54780807 | 145910934 |
| 54779162 | 54779272 | 54780193 | 54780809 | 145910938 |
| 54779166 | 54779274 | 54780209 | 54780815 | 145910942 |
| 54779168 | 54779276 | 54780211 | 54780817 | 145910945 |
| 54779170 | 54779278 | 54780213 | 54780821 | 145910949 |
| 54779172 | 54779280 | 54780227 | 54780825 | 145910952 |
| 54779174 | 54779282 | 54780229 | 54780827 | 145910955 |
| 54779178 | 54779284 | 54780235 | 54780831 | 145910958 |
| 145910966 | 145911186 | 145911397 | 145911823 | 145912409 |
| 145910969 | 145911190 | 145911400 | 145911832 | 145912418 |
| 145910972 | 145911193 | 145911403 | 145911840 | 145912436 |
| 145910975 | 145911199 | 145911407 | 145911849 | 145912446 |
| 145910983 | 145911202 | 145911410 | 145911857 | 145912456 |
| 145910986 | 145911205 | 145911413 | 145911883 | 145912466 |
| 145910989 | 145911214 | 145911416 | 145911892 | 145912470 |
| 145910992 | 145911217 | 145911421 | 145911914 | 145912479 |
| 145910995 | 145911220 | 145911427 | 145911936 | 145912495 |
| 145910998 | 145911223 | 145911436 | 145911938 | 145912504 |
| 145911001 | 145911226 | 145911442 | 145911940 | 145912508 |
| 145911004 | 145911235 | 145911451 | 145911942 | 145912528 |
| 145911013 | 145911238 | 145911457 | 145911944 | 145912547 |
| 145911017 | 145911248 | 145911466 | 145911946 | 145912566 |
| 145911020 | 145911257 | 145911482 | 145911948 | 145912575 |
| 145911023 | 145911287 | 145911491 | 145911950 | 145912587 |
| 145911026 | 145911291 | 145911498 | 145911953 | 145912589 |
| 145911029 | 145911294 | 145911502 | 145911959 | 145912591 |
| 145911032 | 145911298 | 145911510 | 145911968 | 145912595 |
| 145911038 | 145911301 | 145911517 | 145911983 | 145912598 |
| 145911041 | 145911305 | 145911523 | 145911992 | 145912614 |
| 145911044 | 145911308 | 145911536 | 145912001 | 145912624 |
| 145911047 | 145911311 | 145911544 | 145912009 | 145912635 |
| 145911050 | 145911314 | 145911553 | 145912023 | 145912647 |
| 145911053 | 145911317 | 145911561 | 145912037 | 145912674 |
| 145911061 | 145911320 | 145911568 | 145912044 | 145912682 |
| 145911064 | 145911323 | 145911576 | 145912059 | 145912691 |
| 145911072 | 145911326 | 145911585 | 145912100 | 145912700 |
| 145911075 | 145911329 | 145911597 | 145912107 | 145912707 |
| 145911081 | 145911332 | 145911604 | 145912114 | 145912717 |
| 145911086 | 145911335 | 145911611 | 145912123 | 145912725 |
| 145911090 | 145911338 | 145911618 | 145912132 | 145912735 |
| 145911092 | 145911341 | 145911621 | 145912152 | 145912744 |
| 145911096 | 145911344 | 145911655 | 145912167 | 145912753 |
| 145911102 | 145911347 | 145911663 | 145912176 | 145912760 |
| 145911105 | 145911350 | 145911679 | 145912186 | 145912780 |
| 145911108 | 145911353 | 145911687 | 145912211 | 145912790 |
| 145911111 | 145911356 | 145911695 | 145912220 | 145912799 |
| 145911133 | 145911359 | 145911703 | 145912229 | 145912814 |
| 145911150 | 145911362 | 145911713 | 145912238 | 145912824 |
| 145911156 | 145911365 | 145911722 | 145912249 | 145912844 |
| 145911159 | 145911368 | 145911746 | 145912260 | 145912853 |
| 145911162 | 145911371 | 145911748 | 145912278 | 145912861 |
| 145911165 | 145911374 | 145911750 | 145912308 | 145912868 |
| 145911171 | 145911377 | 145911752 | 145912353 | 145912879 |
| 145911174 | 145911384 | 145911754 | 145912361 | 145912888 |
| 145911177 | 145911388 | 145911768 | 145912371 | 145912898 |
| 145911180 | 145911391 | 145911786 | 145912381 | 145912909 |
| 145911183 | 145911394 | 145911795 | 145912399 | 145912919 |
| 145912930 | 145913650 | 145913995 | 145938680 | 145939231 |
| 145912940 | 145913660 | 145914000 | 145938689 | 145939237 |
| 145912949 | 145913670 | 145914004 | 145938698 | 145939252 |
| 145912958 | 145913682 | 145914011 | 145938706 | 145939271 |
| 145912978 | 145913687 | 145914017 | 145938713 | 145939285 |
| 145912996 | 145913722 | 145914020 | 145938721 | 145939302 |
| 145913026 | 145913730 | 145914023 | 145938730 | 145939309 |
| 145913035 | 145913746 | 145914026 | 145938737 | 145939317 |
| 145913042 | 145913752 | 145914038 | 145938755 | 145939331 |
| 145913066 | 145913757 | 145914045 | 145938771 | 145939338 |
| 145913107 | 145913766 | 145914049 | 145938808 | 145939346 |
| 145913138 | 145913772 | 145914056 | 145938830 | 145939356 |
| 145913155 | 145913777 | 145914060 | 145938837 | 145939367 |
| 145913181 | 145913782 | 145914063 | 145938865 | 145939384 |
| 145913209 | 145913787 | 145938277 | 145938874 | 145939392 |
| 145913219 | 145913792 | 145938293 | 145938892 | 145939407 |
| 145913232 | 145913797 | 145938315 | 145938899 | 145939416 |
| 145913236 | 145913803 | 145938332 | 145938906 | 145939432 |
| 145913241 | 145913808 | 145938348 | 145938916 | 145939449 |
| 145913248 | 145913813 | 145938356 | 145938926 | 145939459 |
| 145913257 | 145913840 | 145938362 | 145938944 | 145939470 |
| 145913268 | 145913852 | 145938375 | 145938952 | 145939475 |
| 145913278 | 145913856 | 145938384 | 145938969 | 145939484 |
| 145913288 | 145913867 | 145938391 | 145938986 | 145939501 |
| 145913297 | 145913875 | 145938403 | 145938995 | 145939514 |
| 145913308 | 145913879 | 145938411 | 145939005 | 145939566 |
| 145913344 | 145913883 | 145938421 | 145939023 | 145939578 |
| 145913354 | 145913888 | 145938426 | 145939030 | 145939586 |
| 145913377 | 145913893 | 145938430 | 145939044 | 145939593 |
| 145913386 | 145913898 | 145938438 | 145939053 | 145939602 |
| 145913394 | 145913902 | 145938446 | 145939061 | 145939609 |
| 145913404 | 145913915 | 145938454 | 145939069 | 145939634 |
| 145913415 | 145913919 | 145938462 | 145939083 | 145939643 |
| 145913425 | 145913921 | 145938470 | 145939085 | 145939651 |
| 145913433 | 145913923 | 145938490 | 145939087 | 145939657 |
| 145913470 | 145913927 | 145938504 | 145939093 | 145939670 |
| 145913480 | 145913925 | 145938513 | 145939095 | 145939678 |
| 145913489 | 145913932 | 145938531 | 145939097 | 145939686 |
| 145913518 | 145913943 | 145938537 | 145939106 | 145939694 |
| 145913528 | 145913955 | 145938553 | 145939132 | 145939699 |
| 145913539 | 145913961 | 145938562 | 145939147 | 145939704 |
| 145913549 | 145913965 | 145938570 | 145939155 | 145939707 |
| 145913569 | 145913969 | 145938577 | 145939161 | 145939711 |
| 145913578 | 145913973 | 145938596 | 145939169 | 145939718 |
| 145913588 | 145913977 | 145938621 | 145939181 | 145939724 |
| 145913596 | 145913980 | 145938629 | 145939189 | 145939730 |
| 145913608 | 145913984 | 145938639 | 145939197 | 145939738 |
| 145913620 | 145913988 | 145938647 | 145939206 | 145939747 |
| 145913640 | 145913991 | 145938674 | 145939215 | 145939753 |
| 145939760 | 145940379 | 145940735 | 145941262 | 145941758 |
| 145939766 | 145940387 | 145940742 | 145941276 | 145941764 |
| 145939768 | 145940399 | 145940748 | 145941296 | 145941791 |
| 145939770 | 145940404 | 145940756 | 145941328 | 145941806 |
| 145939776 | 145940411 | 145940762 | 145941336 | 145941819 |
| 145939778 | 145940416 | 145940774 | 145941349 | 145941822 |
| 145939782 | 145940428 | 145940783 | 145941358 | 145941824 |
| 145939788 | 145940439 | 145940789 | 145941365 | 145941828 |
| 145939805 | 145940441 | 145940797 | 145941373 | 145941837 |
| 145939817 | 145940445 | 145940804 | 145941380 | 145941854 |
| 145939824 | 145940448 | 145940818 | 145941388 | 145941863 |
| 145939834 | 145940450 | 145940825 | 145941393 | 145941877 |
| 145939844 | 145940452 | 145940832 | 145941399 | 145941886 |
| 145939858 | 145940456 | 145940838 | 145941425 | 145941908 |
| 145939865 | 145940461 | 145940846 | 145941459 | 145941915 |
| 145939872 | 145940468 | 145940853 | 145941466 | 145941933 |
| 145939879 | 145940482 | 145940858 | 145941474 | 145942086 |
| 145939900 | 145940489 | 145940865 | 145941483 | 145942146 |
| 145939910 | 145940494 | 145940877 | 145941488 | 145942158 |
| 145939921 | 145940498 | 145940884 | 145941499 | 145942175 |
| 145939940 | 145940508 | 145940891 | 145941505 | 145942206 |
| 145939949 | 145940510 | 145940902 | 145941512 | 145942223 |
| 145939970 | 145940515 | 145940907 | 145941518 | 145942261 |
| 145939986 | 145940520 | 145940912 | 145941539 | 145942265 |
| 145940002 | 145940530 | 145940917 | 145941544 | 145942309 |
| 145940029 | 145940535 | 145940921 | 145941550 | 145942383 |
| 145940036 | 145940541 | 145940926 | 145941558 | 145942405 |
| 145940043 | 145940547 | 145940940 | 145941571 | 145942487 |
| 145940052 | 145940552 | 145941075 | 145941577 | 145942497 |
| 145940070 | 145940557 | 145941079 | 145941588 | 145942506 |
| 145940091 | 145940567 | 145941083 | 145941597 | 145942509 |
| 145940115 | 145940573 | 145941090 | 145941605 | 145942544 |
| 145940124 | 145940583 | 145941097 | 145941618 | 145942565 |
| 145940133 | 145940593 | 145941111 | 145941634 | 145942606 |
| 145940152 | 145940597 | 145941118 | 145941639 | 148717962 |
| 145940167 | 145940602 | 145941131 | 145941644 | 148717964 |
| 145940173 | 145940613 | 145941134 | 145941650 | 148717966 |
| 145940190 | 145940631 | 145941137 | 145941657 | 148910865 |
| 145940218 | 145940636 | 145941143 | 145941669 | 159034187 |
| 145940226 | 145940645 | 145941151 | 145941674 | 159034188 |
| 145940239 | 145940650 | 145941158 | 145941680 | 159034189 |
| 145940269 | 145940656 | 145941167 | 145941685 | 159034190 |
| 145940316 | 145940662 | 145941176 | 145941698 | 159034191 |
| 145940325 | 145940675 | 145941194 | 145941704 | 159034192 |
| 145940332 | 145940681 | 145941226 | 145941717 | 159034193 |
| 145940340 | 145940700 | 145941231 | 145941724 | 159034194 |
| 145940354 | 145940706 | 145941239 | 145941731 | 159034195 |
| 145940362 | 145940711 | 145941247 | 145941745 | 159034196 |
| 145940370 | 145940727 | 145941255 | 145941752 | 159034197 |
| 159034198 | 159034268 | 159034340 | 159034404 | 159034476 |
| 159034200 | 159034273 | 159034341 | 159034405 | 159034479 |
| 159034202 | 159034274 | 159034342 | 159034408 | 159034481 |
| 159034203 | 159034276 | 159034343 | 159034410 | 159034482 |
| 159034204 | 159034277 | 159034344 | 159034414 | 159034484 |
| 159034205 | 159034278 | 159034345 | 159034415 | 159034485 |
| 159034207 | 159034279 | 159034346 | 159034417 | 159034486 |
| 159034208 | 159034280 | 159034347 | 159034419 | 159034490 |
| 159034209 | 159034282 | 159034348 | 159034420 | 159034492 |
| 159034211 | 159034283 | 159034349 | 159034421 | 159034493 |
| 159034212 | 159034284 | 159034350 | 159034422 | 159034494 |
| 159034213 | 159034285 | 159034354 | 159034423 | 159034495 |
| 159034214 | 159034286 | 159034355 | 159034424 | 159034497 |
| 159034215 | 159034287 | 159034356 | 159034425 | 159034499 |
| 159034216 | 159034288 | 159034358 | 159034426 | 159034500 |
| 159034217 | 159034290 | 159034359 | 159034429 | 159034501 |
| 159034218 | 159034291 | 159034362 | 159034430 | 159034502 |
| 159034219 | 159034293 | 159034364 | 159034431 | 159034503 |
| 159034222 | 159034296 | 159034365 | 159034433 | 159034504 |
| 159034223 | 159034297 | 159034366 | 159034434 | 159034511 |
| 159034224 | 159034298 | 159034367 | 159034435 | 159034512 |
| 159034225 | 159034299 | 159034368 | 159034436 | 159034515 |
| 159034226 | 159034300 | 159034369 | 159034438 | 159034516 |
| 159034227 | 159034301 | 159034370 | 159034439 | 159034518 |
| 159034228 | 159034302 | 159034372 | 159034440 | 159034521 |
| 159034230 | 159034303 | 159034373 | 159034441 | 159034522 |
| 159034231 | 159034304 | 159034375 | 159034443 | 159034523 |
| 159034233 | 159034305 | 159034376 | 159034444 | 159034524 |
| 159034235 | 159034306 | 159034378 | 159034445 | 159034526 |
| 159034236 | 159034307 | 159034379 | 159034446 | 159034527 |
| 159034238 | 159034308 | 159034381 | 159034447 | 159034529 |
| 159034239 | 159034309 | 159034383 | 159034448 | 159034530 |
| 159034240 | 159034310 | 159034384 | 159034449 | 159034531 |
| 159034241 | 159034311 | 159034385 | 159034450 | 159034532 |
| 159034242 | 159034313 | 159034386 | 159034451 | 159034534 |
| 159034243 | 159034315 | 159034387 | 159034453 | 159034535 |
| 159034244 | 159034316 | 159034388 | 159034455 | 159034536 |
| 159034245 | 159034318 | 159034389 | 159034460 | 159034537 |
| 159034249 | 159034320 | 159034390 | 159034461 | 159034538 |
| 159034250 | 159034323 | 159034392 | 159034462 | 159034539 |
| 159034252 | 159034324 | 159034393 | 159034463 | 159034540 |
| 159034253 | 159034325 | 159034395 | 159034464 | 159034541 |
| 159034254 | 159034328 | 159034396 | 159034465 | 159034542 |
| 159034258 | 159034329 | 159034397 | 159034466 | 159034543 |
| 159034259 | 159034330 | 159034398 | 159034467 | 159034545 |
| 159034260 | 159034331 | 159034399 | 159034468 | 159034546 |
| 159034262 | 159034335 | 159034400 | 159034471 | 159034547 |
| 159034266 | 159034337 | 159034402 | 159034472 | 159034549 |
| 159034267 | 159034339 | 159034403 | 159034474 | 159034550 |
| 159034552 | 159034636 | 159034706 | 159034773 | 159034838 |
| 159034554 | 159034637 | 159034708 | 159034774 | 159034839 |
| 159034556 | 159034638 | 159034709 | 159034778 | 159034840 |
| 159034559 | 159034640 | 159034710 | 159034779 | 159034842 |
| 159034562 | 159034641 | 159034711 | 159034780 | 159034843 |
| 159034563 | 159034642 | 159034712 | 159034781 | 159034844 |
| 159034564 | 159034643 | 159034713 | 159034782 | 159034845 |
| 159034565 | 159034647 | 159034714 | 159034783 | 159034846 |
| 159034566 | 159034648 | 159034717 | 159034784 | 159034847 |
| 159034568 | 159034649 | 159034718 | 159034786 | 159034848 |
| 159034570 | 159034650 | 159034720 | 159034787 | 159034849 |
| 159034571 | 159034651 | 159034721 | 159034788 | 159034852 |
| 159034572 | 159034652 | 159034722 | 159034789 | 159034853 |
| 159034573 | 159034653 | 159034725 | 159034790 | 159034856 |
| 159034575 | 159034654 | 159034726 | 159034791 | 159034858 |
| 159034576 | 159034657 | 159034728 | 159034793 | 159034859 |
| 159034578 | 159034658 | 159034729 | 159034794 | 159034860 |
| 159034580 | 159034659 | 159034730 | 159034795 | 159034861 |
| 159034581 | 159034660 | 159034731 | 159034796 | 159034862 |
| 159034582 | 159034661 | 159034732 | 159034798 | 159034863 |
| 159034584 | 159034664 | 159034733 | 159034799 | 159034864 |
| 159034587 | 159034665 | 159034734 | 159034801 | 159034866 |
| 159034588 | 159034668 | 159034736 | 159034802 | 159034869 |
| 159034589 | 159034669 | 159034737 | 159034804 | 159034871 |
| 159034591 | 159034672 | 159034738 | 159034805 | 159034872 |
| 159034596 | 159034673 | 159034741 | 159034809 | 159034874 |
| 159034599 | 159034676 | 159034742 | 159034811 | 159034876 |
| 159034600 | 159034677 | 159034743 | 159034812 | 159034877 |
| 159034601 | 159034678 | 159034744 | 159034813 | 159034879 |
| 159034602 | 159034679 | 159034745 | 159034815 | 159034880 |
| 159034604 | 159034680 | 159034747 | 159034816 | 159034882 |
| 159034607 | 159034681 | 159034748 | 159034817 | 159034883 |
| 159034609 | 159034683 | 159034749 | 159034818 | 159034885 |
| 159034611 | 159034686 | 159034750 | 159034819 | 159034886 |
| 159034612 | 159034687 | 159034751 | 159034820 | 159034887 |
| 159034613 | 159034688 | 159034752 | 159034821 | 159034888 |
| 159034617 | 159034689 | 159034754 | 159034822 | 159034889 |
| 159034619 | 159034690 | 159034756 | 159034823 | 159034890 |
| 159034620 | 159034692 | 159034758 | 159034824 | 159034892 |
| 159034621 | 159034693 | 159034759 | 159034825 | 159034893 |
| 159034622 | 159034695 | 159034760 | 159034827 | 159034894 |
| 159034625 | 159034696 | 159034762 | 159034828 | 159034895 |
| 159034626 | 159034697 | 159034763 | 159034829 | 159034897 |
| 159034628 | 159034698 | 159034764 | 159034831 | 159034898 |
| 159034629 | 159034699 | 159034765 | 159034832 | 159034899 |
| 159034631 | 159034700 | 159034766 | 159034833 | 159034900 |
| 159034632 | 159034701 | 159034767 | 159034834 | 159034901 |
| 159034634 | 159034704 | 159034768 | 159034835 | 159034902 |
| 159034635 | 159034705 | 159034771 | 159034837 | 159034903 |
| 159034904 | 159034974 | 159035041 | 159035110 | 159035177 |
| 159034905 | 159034975 | 159035042 | 159035111 | 159035179 |
| 159034906 | 159034976 | 159035043 | 159035112 | 159035180 |
| 159034907 | 159034978 | 159035044 | 159035113 | 159035181 |
| 159034908 | 159034980 | 159035045 | 159035114 | 159035182 |
| 159034909 | 159034981 | 159035046 | 159035115 | 159035183 |
| 159034910 | 159034982 | 159035047 | 159035116 | 159035185 |
| 159034911 | 159034983 | 159035049 | 159035117 | 159035186 |
| 159034912 | 159034984 | 159035050 | 159035118 | 159035187 |
| 159034913 | 159034985 | 159035051 | 159035120 | 159035188 |
| 159034914 | 159034987 | 159035052 | 159035122 | 159035191 |
| 159034917 | 159034988 | 159035053 | 159035123 | 159035192 |
| 159034918 | 159034989 | 159035054 | 159035124 | 159035193 |
| 159034919 | 159034991 | 159035055 | 159035125 | 159035194 |
| 159034920 | 159034992 | 159035056 | 159035126 | 159035195 |
| 159034923 | 159034993 | 159035057 | 159035128 | 159035199 |
| 159034925 | 159034995 | 159035058 | 159035129 | 159035201 |
| 159034926 | 159034996 | 159035060 | 159035130 | 159035204 |
| 159034928 | 159034997 | 159035061 | 159035131 | 159035205 |
| 159034929 | 159034998 | 159035063 | 159035133 | 159035206 |
| 159034931 | 159035002 | 159035064 | 159035134 | 159035207 |
| 159034934 | 159035003 | 159035065 | 159035135 | 159035208 |
| 159034935 | 159035005 | 159035066 | 159035136 | 159035209 |
| 159034936 | 159035007 | 159035067 | 159035137 | 159035210 |
| 159034937 | 159035008 | 159035072 | 159035138 | 159035211 |
| 159034938 | 159035009 | 159035073 | 159035139 | 159035212 |
| 159034939 | 159035010 | 159035074 | 159035140 | 159035213 |
| 159034940 | 159035013 | 159035075 | 159035143 | 159035215 |
| 159034942 | 159035014 | 159035076 | 159035145 | 159035218 |
| 159034945 | 159035015 | 159035079 | 159035146 | 159035221 |
| 159034946 | 159035016 | 159035080 | 159035147 | 159035224 |
| 159034947 | 159035017 | 159035081 | 159035149 | 159035226 |
| 159034948 | 159035018 | 159035083 | 159035150 | 159035227 |
| 159034950 | 159035021 | 159035084 | 159035151 | 159035230 |
| 159034952 | 159035022 | 159035085 | 159035152 | 159035232 |
| 159034953 | 159035023 | 159035086 | 159035155 | 159035234 |
| 159034954 | 159035024 | 159035087 | 159035158 | 159035235 |
| 159034955 | 159035025 | 159035089 | 159035159 | 159035238 |
| 159034957 | 159035026 | 159035090 | 159035160 | 159035239 |
| 159034959 | 159035027 | 159035092 | 159035162 | 159035240 |
| 159034961 | 159035028 | 159035097 | 159035163 | 159035241 |
| 159034962 | 159035031 | 159035100 | 159035164 | 159035242 |
| 159034963 | 159035033 | 159035101 | 159035166 | 159035245 |
| 159034964 | 159035034 | 159035102 | 159035170 | 159035247 |
| 159034965 | 159035035 | 159035103 | 159035172 | 159035250 |
| 159034967 | 159035036 | 159035104 | 159035173 | 159035253 |
| 159034970 | 159035038 | 159035106 | 159035174 | 159035256 |
| 159034971 | 159035039 | 159035108 | 159035175 | 159035257 |
| 159034973 | 159035040 | 159035109 | 159035176 | 159035260 |
| 159035261 | 159035334 | 159035424 | 159035507 | 159035576 |
| 159035262 | 159035335 | 159035425 | 159035508 | 159035577 |
| 159035263 | 159035338 | 159035426 | 159035512 | 159035581 |
| 159035264 | 159035342 | 159035427 | 159035513 | 159035582 |
| 159035265 | 159035343 | 159035430 | 159035514 | 159035583 |
| 159035267 | 159035344 | 159035432 | 159035515 | 159035584 |
| 159035268 | 159035346 | 159035433 | 159035516 | 159035585 |
| 159035269 | 159035348 | 159035435 | 159035519 | 159035586 |
| 159035270 | 159035349 | 159035436 | 159035520 | 159035589 |
| 159035272 | 159035350 | 159035437 | 159035523 | 159035590 |
| 159035273 | 159035351 | 159035439 | 159035524 | 159035591 |
| 159035274 | 159035353 | 159035441 | 159035525 | 159035592 |
| 159035275 | 159035360 | 159035445 | 159035526 | 159035593 |
| 159035277 | 159035361 | 159035446 | 159035527 | 159035594 |
| 159035279 | 159035362 | 159035447 | 159035528 | 159035595 |
| 159035280 | 159035363 | 159035449 | 159035529 | 159035596 |
| 159035282 | 159035364 | 159035450 | 159035531 | 159035597 |
| 159035284 | 159035366 | 159035451 | 159035532 | 159035598 |
| 159035285 | 159035368 | 159035452 | 159035533 | 159035602 |
| 159035287 | 159035369 | 159035453 | 159035535 | 159035603 |
| 159035288 | 159035370 | 159035454 | 159035536 | 159035604 |
| 159035289 | 159035372 | 159035456 | 159035537 | 159035605 |
| 159035290 | 159035373 | 159035457 | 159035538 | 159035609 |
| 159035292 | 159035374 | 159035458 | 159035541 | 159035610 |
| 159035293 | 159035378 | 159035459 | 159035542 | 159035611 |
| 159035294 | 159035380 | 159035461 | 159035546 | 159035612 |
| 159035296 | 159035382 | 159035463 | 159035548 | 159035614 |
| 159035299 | 159035384 | 159035464 | 159035549 | 159035618 |
| 159035302 | 159035385 | 159035466 | 159035550 | 159035619 |
| 159035303 | 159035386 | 159035468 | 159035552 | 159035624 |
| 159035305 | 159035390 | 159035470 | 159035553 | 159035626 |
| 159035307 | 159035391 | 159035472 | 159035554 | 159035627 |
| 159035308 | 159035393 | 159035473 | 159035556 | 159035628 |
| 159035309 | 159035394 | 159035475 | 159035557 | 159035630 |
| 159035310 | 159035395 | 159035476 | 159035559 | 159035631 |
| 159035312 | 159035400 | 159035477 | 159035560 | 159035632 |
| 159035313 | 159035401 | 159035479 | 159035562 | 159035633 |
| 159035314 | 159035402 | 159035483 | 159035563 | 159035634 |
| 159035315 | 159035403 | 159035484 | 159035564 | 159035636 |
| 159035316 | 159035404 | 159035490 | 159035565 | 159035637 |
| 159035318 | 159035405 | 159035492 | 159035566 | 159035638 |
| 159035319 | 159035406 | 159035493 | 159035567 | 159035640 |
| 159035320 | 159035407 | 159035496 | 159035568 | 159035641 |
| 159035321 | 159035408 | 159035497 | 159035569 | 159035642 |
| 159035323 | 159035413 | 159035498 | 159035570 | 159035644 |
| 159035327 | 159035418 | 159035499 | 159035571 | 159035646 |
| 159035329 | 159035420 | 159035501 | 159035573 | 159035649 |
| 159035330 | 159035422 | 159035502 | 159035574 | 159035650 |
| 159035331 | 159035423 | 159035503 | 159035575 | 159035653 |
| 159035655 | 162950148 | 162950291 | 162950352 | 162950431 |
| 159035656 | 162950152 | 162950292 | 162950353 | 162950432 |
| 159035658 | 162950188 | 162950293 | 162950355 | 162950433 |
| 159035659 | 162950189 | 162950294 | 162950359 | 162950434 |
| 159035661 | 162950191 | 162950295 | 162950360 | 162950435 |
| 159035662 | 162950199 | 162950296 | 162950361 | 162950437 |
| 159035663 | 162950202 | 162950297 | 162950363 | 162950438 |
| 159035664 | 162950204 | 162950298 | 162950365 | 162950439 |
| 162950025 | 162950205 | 162950299 | 162950367 | 162950440 |
| 162950028 | 162950210 | 162950300 | 162950369 | 162950441 |
| 162950034 | 162950211 | 162950301 | 162950372 | 162950442 |
| 162950035 | 162950213 | 162950302 | 162950373 | 162950444 |
| 162950039 | 162950227 | 162950303 | 162950374 | 162950445 |
| 162950043 | 162950241 | 162950305 | 162950375 | 162950446 |
| 162950050 | 162950243 | 162950306 | 162950377 | 162950447 |
| 162950051 | 162950244 | 162950308 | 162950381 | 162950449 |
| 162950052 | 162950245 | 162950309 | 162950382 | 162950450 |
| 162950054 | 162950248 | 162950310 | 162950384 | 162950451 |
| 162950055 | 162950249 | 162950311 | 162950385 | 162950453 |
| 162950057 | 162950250 | 162950312 | 162950386 | 162950454 |
| 162950065 | 162950251 | 162950313 | 162950387 | 162950456 |
| 162950067 | 162950252 | 162950314 | 162950388 | 162950461 |
| 162950077 | 162950253 | 162950315 | 162950390 | 162950463 |
| 162950097 | 162950254 | 162950316 | 162950391 | 162950464 |
| 162950098 | 162950255 | 162950317 | 162950392 | 162950465 |
| 162950100 | 162950257 | 162950318 | 162950393 | 162950466 |
| 162950111 | 162950258 | 162950319 | 162950394 | 162950467 |
| 162950114 | 162950260 | 162950321 | 162950396 | 162950469 |
| 162950115 | 162950261 | 162950323 | 162950397 | 162950470 |
| 162950120 | 162950263 | 162950325 | 162950398 | 162950471 |
| 162950121 | 162950265 | 162950326 | 162950399 | 162950474 |
| 162950123 | 162950266 | 162950327 | 162950400 | 162950476 |
| 162950125 | 162950267 | 162950328 | 162950401 | 162950481 |
| 162950126 | 162950269 | 162950329 | 162950404 | 162950483 |
| 162950127 | 162950270 | 162950330 | 162950405 | 162950484 |
| 162950128 | 162950272 | 162950333 | 162950406 | 162950485 |
| 162950129 | 162950273 | 162950334 | 162950408 | 162950487 |
| 162950130 | 162950275 | 162950335 | 162950410 | 162950489 |
| 162950132 | 162950277 | 162950337 | 162950411 | 162950490 |
| 162950133 | 162950278 | 162950338 | 162950416 | 162950491 |
| 162950134 | 162950279 | 162950339 | 162950418 | 162950492 |
| 162950137 | 162950282 | 162950341 | 162950419 | 162950493 |
| 162950138 | 162950284 | 162950342 | 162950420 | 162950494 |
| 162950139 | 162950285 | 162950343 | 162950421 | 162950496 |
| 162950140 | 162950286 | 162950344 | 162950423 | 162950498 |
| 162950141 | 162950287 | 162950345 | 162950425 | 162950500 |
| 162950143 | 162950288 | 162950347 | 162950426 | 162950503 |
| 162950146 | 162950289 | 162950348 | 162950428 | 162950504 |
| 162950147 | 162950290 | 162950350 | 162950430 | 162950514 |
| 162950515 | 162950583 | 162950650 | | |
| 162950516 | 162950584 | 162950651 | | |
| 162950517 | 162950585 | 162950652 | | |
| 162950518 | 162950586 | 162950654 | | |
| 162950519 | 162950587 | 162950655 | | |
| 162950520 | 162950589 | 162950656 | | |
| 162950522 | 162950590 | 162950659 | | |
| 162950525 | 162950591 | 162950660 | | |
| 162950526 | 162950592 | 162950661 | | |
| 162950527 | 162950593 | 162950662 | | |
| 162950528 | 162950594 | 162950665 | | |
| 162950529 | 162950596 | 162950666 | | |
| 162950530 | 162950597 | 162950671 | | |
| 162950531 | 162950598 | 162950673 | | |
| 162950532 | 162950599 | 162950674 | | |
| 162950534 | 162950600 | 162950675 | | |
| 162950535 | 162950602 | 162950676 | | |
| 162950536 | 162950604 | 162950685 | | |
| 162950537 | 162950605 | 162950686 | | |
| 162950539 | 162950606 | 162950688 | | |
| 162950540 | 162950609 | 162950689 | | |
| 162950542 | 162950610 | 162950691 | | |
| 162950543 | 162950611 | 162950692 | | |
| 162950546 | 162950613 | 162950693 | | |
| 162950547 | 162950614 | 162950694 | | |
| 162950551 | 162950615 | 162950695 | | |
| 162950552 | 162950617 | 162950696 | | |
| 162950555 | 162950618 | 162950697 | | |
| 162950556 | 162950619 | 162950698 | | |
| 162950557 | 162950620 | 162950710 | | |
| 162950558 | 162950621 | 162950714 | | |
| 162950559 | 162950622 | 162950716 | | |
| 162950562 | 162950626 | 162950720 | | |
| 162950564 | 162950627 | 162950724 | | |
| 162950565 | 162950628 | 162950725 | | |
| 162950566 | 162950629 | 162950726 | | |
| 162950568 | 162950631 | 162950728 | | |
| 162950570 | 162950632 | 162950729 | | |
| 162950571 | 162950633 | 194719560 | | |
| 162950572 | 162950634 | 194719575 | | |
| 162950573 | 162950635 | 218454113 | | |
| 162950574 | 162950640 | 218454117 | | |
| 162950575 | 162950641 | 219937557 | | |
| 162950577 | 162950642 | | | |
| 162950578 | 162950644 | | | |
| 162950579 | 162950645 | | | |
| 162950580 | 162950646 | | | |
| 162950581 | 162950647 | | | |
| 162950582 | 162950649 | | | |

## Claims

1. A method of making a library of synthetic polynucleotides encoding polypeptides comprising CDRH3 sequences, the method comprising:
(a) providing a theoretical segment pool containing TN1 , DH, N2, and H3-JH segments;
(b) providing a reference set ofpreimmune CDRH3 sequences having sequence diversities and length diversities similar to naturally occurring human antibody sequences before these sequences have undergone negative selection and/or hypermutation;
(c) utilizing combinations of the TN1, DH, N2, and H3-JH segments contained in the theoretical segment pool of (a) to identify the closest match(es) to each CDRH3 sequence in the reference set of (b);
(d) selecting segments from the closest match(es) identified in step (c) for inclusion in a synthetic library; and
(e) synthesizing the synthetic CDRH3 library.

2. The method of claim 1, wherein
(a) the segments selected for inclusion in the synthetic library are selected according to their segment usage weight in the reference set of CDRH3 sequences,
(b) the segments selected for inclusion in the synthetic library are selected according to one or more physicochemical properties,
(c) the method further comprising selecting additional TNI and N2 segments occurring in the reference set but not in the theoretical segment pool,
(d) stop codons are reduced or eliminated from the library,
(e) the unpaired Cys residues, N-linked glycosylation motifs, and deamidation motifs are reduced or eliminated in the translation products of the library,
(f) the DH segments and H3-JH segments are progressively truncated prior to matching with the CDRH3 sequences in the reference set,
(g) the method further comprising introducing one or two degenerate codons in a segment selected from the group consisting ofDH and N2, or combinations thereof, or
(h) the method further comprising introducing one degenerate codon in the H3-JH segment.

3. A library of synthetic polynucleotides comprising at least about 10⁴ polynucleotides encoding polypeptides comprising different CDRH3 sequences with the structure:
[TN1]-[DH]-[N2]-[H3-JH],
wherein:
TN 1 is a polypeptide comprising any of the TN1 polypeptides of Tables 10 and 18-26, or a polypeptide produced by translation of any of the TN1 polynucleotides of Tables 25-26;
DH is a polypeptide comprising any of the DH polypeptides of Tables 11, 17-25 and 28, or a polypeptide produced by translation of any of the DH-encoding polynucleotides of Tables 16, 25 and 27;
N2 is a polypeptide comprising any of the N2 polypeptides of Tables 12, 18-25 and 30, or a polypeptide produced by translation of any of the N2-encoding polynucleotides of Tables 25 and 29; and
H3-JH is a polypeptide comprising any of the H3-JH polypeptides of Tables 13, 15, 18-25 and 32, or a polypeptide produced by translation of any of the H3-JH-encoding polynucleotides of Tables 14, 25 and 31;
wherein the library was made according to the method of claim 1.

4. The library of claim 3, wherein the polynucleotides encode CDRH3 polypeptides produced by the sets of TN1, DH, N2, and H3-JH polypeptides provided in any one of Tables 23-25.

5. The library of claim 3, wherein the polynucleotides encode CDRH3 polypeptides produced by the set of TN1 polypeptides provided in Table 26, the set of DH polypeptides provided in Table 28, the set of N2 polypeptides provided in Table 30 and the set of H3-JH polypeptides provided in Table 32.

6. The library of claim 3 wherein number of N-linked glycosylation sites, deamidation motifs, and/or Cys residues are reduced or eliminated in comparison to libraries produced by amplification of a repertoire from a biological source.

7. The library of claim 3, further comprising a polynucleotide encoding one or more light chain variable domain polypeptides.

8. The library of claim 7, wherein the polypeptides are expressed as full-length IgGs.

9. The polypeptide expression products of the library of any one of claim 3 to 8.

10. A method of producing an antibody of interest by expressing the library of any one of claims 3 to 8 in a host cell.

11. A library of vectors containing the library of any one of claim 3 to 8.

12. A library of host cells containing the library of vectors of claim 11.

13. The library of host cells of claim 12, wherein the host cells are yeast cells.

14. Use of the library of any one of claim 3 to 8 to isolate an antibody binding an antigen.

15. A kit containing the library of any one of claims 3 to 8.

16. The library of claim 7, wherein the polypeptides are expressed as intact antibodies.

## Patentansprüche

1. Ein Verfahren zur Herstellung einer Bibliothek synthetischer Polynucleotide, die Polypeptide mit CDRH3-Sequenzen kodieren, wobei das Verfahren umfasst:
(a) Bereitstellen eines theoretischen Segmente-Pools, enthaltend TN1-, DH-, N2- und H3-JH-Segmente;
(b) Bereitstellen eines Referenzsets von präimmunen CDRH3-Sequenzen mit Sequenzdiversitäten und Längendiversitäten ähnlich wie natürlich vorkommende humane Antikörpersequenzen vorweisen bevor diese Sequenzen negative Selektion und/oder Hypermutation erfahren haben;
(c) Nutzen von Kombinationen der in dem theoretischen Segmente-Pool von (a) enthaltenen TN1-, DH-, N2- und H3-JH-Segmente, um die größte(n) Übereinstimmung(en) mit jeder CDRH3-Sequenz in dem Referenzset von (b) zu identifizieren;
(d) Auswählen von Segmenten aus der/den in Schritt (c) identifizierten größten Übereinstimmung(en) für die Aufnahme in eine synthetische Bibliothek; und
(e) Synthetisieren der synthetischen CDRH3-Bibliothek.

2. Das Verfahren nach Anspruch 1, wobei
(a) die für die Aufnahme in die synthetische Bibliothek ausgewählten Segmente nach ihrem Segmentnutzungsgewicht in dem Referenzset von CDRH3-Sequenzen ausgewählt werden,
(b) die für die Aufnahme in die synthetische Bibliothek ausgewählten Segmente nach einer oder mehreren physikalisch-chemischen Eigenschaften ausgewählt werden,
(c) das Verfahren weiterhin das Auswählen zusätzlicher TN1- und N2-Segmente, die in dem Referenzset, aber nicht in dem theoretischen Segmente-Pool vorkommen, umfasst,
(d) Stoppcodons aus der Bibliothek verringert oder eliminiert werden,
(e) die ungepaarten Cys-Reste, N-verknüpften Glykosylierungsmotive und Deamidierungsmotive in den Translationsprodukten der Bibliothek verringert oder eliminiert werden,
(f) die DH-Segmente und H3-JH-Segmente vor dem Abgleichen mit den CDRH3-Sequenzen in dem Referenzset schrittweise gekürzt werden,
(g) das Verfahren weiter das Einführen von einem oder zwei degenerierten Codons in einem Segment, ausgewählt aus der Gruppe bestehend aus DH und N2, oder Kombinationen davon, umfasst, oder
(h) das Verfahren weiter das Einführen von einem degenerierten Codon in dem H3-JH-Segment umfasst.

3. Eine Bibliothek synthetischer Polynucleotide, umfassend mindestens etwa 10⁴ Polynucleotide, die Polypeptide mit verschiedenen CDRH3-Sequenzen mit der folgenden Struktur kodieren: wobei:
[TN1]-[DH]-[N2]-[H3-JH],
wobei:
TN1 ein Polypeptid ist, umfassend ein beliebiges TNl-Polypeptid der Tabellen 10 und 18-26, oder ein Polypeptid, das durch die Translation eines der TN1-Polynucleotide der Tabellen 25-26 gebildet wurde, ist;
DH ein Polypeptid ist, umfassend ein beliebiges DH-Polypeptid der Tabellen 11, 17-25 und 28, oder ein Polypeptid, das durch die Translation eines der DH-kodierenden Polynucleotide der Tabellen 16, 25 und 27 gebildet wurde, ist;
N2 ein Polypeptid ist, umfassend ein beliebiges N2-Polypeptid der Tabellen 12, 18-25 und 30, oder ein Polypeptid, das durch die Translation eines der N2-kodierenden Polynucleotide der Tabellen 25 und 29 gebildet wurde,ist; und
H3-JH ein Polypeptid ist, umfassend ein beliebiges H3-JH-Polypeptid der Tabellen 13, 15, 18-25 und 32, oder ein Polypeptid, das durch die Translation eines der H3-JH-kodierenden Polynucleotide der Tabellen 14, 25 und 31 gebildet wurde, ist;
wobei die Bibliothek gemäß dem Verfahren nach Anspruch 1 hergestellt wurde.

4. Die Bibliothek nach Anspruch 3, wobei die Polynucleotide CDRH3-Polypeptide kodieren, die von einem beliebigen Set von TN1-, DH-, N2- und H3-JH-Polypeptiden der Tabellen 23-25 produziert werden.

5. Die Bibliothek nach Anspruch 3, wobei die Polynucleotide CDRH3-Polypeptide kodieren, die von dem Set von TN1 Polypeptiden in Tabelle 26, dem Set von DH-Polypeptiden in Tabelle 28, dem Set von N2-Polypeptided in Tabelle 30 und dem Set von H3-JH-Polypeptiden in Tabelle 32, produziert werden.

6. Die Bibliothek nach Anspruch 3, wobei die Zahl von N-verknüpften Glykosylierungsstellen, Deamidierungsmotiven und/oder Cys-Resten im Vergleich zu Bibliotheken, die durch Amplifikation eines Repertoires aus einer biologischen Quelle erzeugt werden, verringert oder eliminiert ist.

7. Die Bibliothek nach Anspruch 3, weiter umfassend ein Polynucleotid, das ein oder mehrere Polypeptide mit variablen Domänen der leichten Ketten kodiert.

8. Die Bibliothek nach Anspruch 7, wobei die Polypeptide als IgGs in voller Länge exprimiert sind.

9. Die Polypeptidexpressionsprodukte der Bibliothek von einem beliebigen der Ansprüche 3 bis 8.

10. Ein Verfahren zur Herstellung eines Antikörpers von Interesse durch Exprimieren der Bibliothek von einem beliebigen der Ansprüche 3 bis 8 in einer Wirtszelle.

11. Eine Bibliothek von Vektoren, enthaltend die Bibliothek nach einem beliebigen der Ansprüche 3 bis 8.

12. Eine Bibliothek von Wirtszellen, enthaltend die Bibliothek von Vektoren nach Anspruch 11.

13. Die Bibliothek von Wirtszellen nach Anspruch 12, wobei die Wirtszellen Hefezellen sind.

14. Verwendung der Bibliothek nach einem beliebigen der Ansprüche 3 bis 8, um einen an ein Antigen bindenden Antikörper zu isolieren.

15. Ein Kit, enthaltend die Bibliothek nach einem beliebigen der Ansprüche 3 bis 8.

16. Die Bibliothek nach Anspruch 7, wobei die Polypeptide als intakte Antikörper exprimiert sind.

## Revendications

1. Procédé de création d'une banque de polynucléotides synthétiques encodant des polypeptides comprenant des séquences CDRH3, le procédé consistant à :
(a) fournir un groupe de segments théoriques contenant des segments TN1, DH, N2 et H3-JH ;
(b) fournir un ensemble de séquences CDRH3 préimmunitaires possédant une diversité de séquences et une diversité de longueurs similaires aux séquences d'anticorps humains survenant naturellement avant que ces séquences aient subi une sélection et/ou une hypermutation négative ;
(c) utiliser des combinaisons des segments TN1, DH, N2 et H3-JH contenus dans le groupe de segments théoriques de (a) pour identifier la ou les correspondances les plus proches de chaque séquence CDRH3 dans l'ensemble de référence de (b) ;
(d) sélectionner des segments parmi la ou les correspondances les plus proches identifiées à l'étape (c) pour les inclure dans une banque synthétique ; et
(e) synthétiser la banque de CDRH3 synthétiques.

2. Le procédé selon la revendication 1, dans lequel
(a) les segments sélectionnés pour être inclus dans la banque synthétique sont sélectionnés en fonction de leur poids d'usage de segments dans l'ensemble de référence des séquences CDRH3,
(b) les segments sélectionnés pour être inclus dans la banque synthétique sont sélectionnés en fonction d'une ou plusieurs propriétés physico-chimiques,
(c) le procédé comprenant en outre la sélection de segments supplémentaires TN1 et N2 survenant dans l'ensemble de référence mais pas dans le groupe de segments théoriques,
(d) les codons-stop sont réduits ou éliminés de la banque,
(e) les résidus Cys non appariés, les motifs de glycosylation à liaison N et les motifs de désamidation sont réduits ou éliminés dans les produits de traduction de la banque,
(f) les segments DH et H3-JH sont progressivement tronqués avant d'être mis en correspondance avec les séquences CDRH3 dans l'ensemble de référence,
(g) le procédé comprenant en outre l'introduction d'un ou deux codons dégénérés dans un segment sélectionné parmi le groupe constitué de DH et N2 ou des combinaisons de ceux-ci, ou
(h) le procédé comprenant en outre l'introduction d'un seul codon dégénéré dans le segment H3-JH.

3. Une banque de polynucléotides synthétiques comprenant au moins 10⁴ polynucléotides encodant des polypeptides comprenant des séquences CDRH3 différentes de structure :
[TN1]-[DH]-[N2]-[H3-JH],
dans laquelle :
TN1 est un polypeptide comprenant n'importe lesquels des polypeptides TN1 des tableaux 10 et 18 à 26, ou un polypeptide produit par traduction de n'importe lesquels des polynucléotides TN1 des tableaux 25 à 26 ;
DH est un polypeptide comprenant n'importe lesquels des polypeptides DH des tableaux 11, 17 à 25 et 28, ou un polypeptide produit par traduction de n'importe lesquels des polynucléotides encodant DH des tableaux 16, 25 et 27 ;
N2 est un polypeptide comprenant n'importe lesquels des polypeptides N2 des tableaux 12, 18 à 25 et 30, ou un polypeptide produit par traduction de n'importe lesquels des polynucléotides encodant N2 des tableaux 25 et 29 ; et
H3-JH est un polypeptide comprenant n'importe lesquels des polypeptides H3-JH des tableaux 13, 15, 18 à 25 et 32, ou un polypeptide produit par traduction de n'importe lesquels des polynucléotides encodant H3-JH des tableaux 14, 25 et 31 ;
la banque étant réalisée conformément au procédé selon la revendication 1.

4. La banque selon la revendication 3,dans laquelle les polynucléotides encodent les polypeptides CDRH3 produits par les ensembles de polypeptides TN1, DH, N2 et H3-JH prévus dans l'un quelconque des tableaux 23 à 25.

5. La banque selon la revendication 3,dans laquelle les polynucléotides encodent les polypeptides CDRH3 produits par l'ensemble des polypeptides TN1 prévus dans le tableau 26, l'ensemble des polypeptides DH prévus dans le tableau 28, l'ensemble des polypeptides N2 prévus dans le tableau 30 et l'ensemble des polypeptides H3-JH prévus dans le tableau 32.

6. La banque selon la revendication 3, dans laquelle le nombre de sites de glycosylation à liaison N, de motifs de désamidation et/ou de résidus Cys sont réduits ou éliminés par comparaison aux banques produites par amplification d'un répertoire provenant d'une source biologique.

7. La banque selon la revendication 3 comprenant en outre un polynucléotide encodant un ou plusieurs polypeptides de domaine variable à chaîne légère.

8. La banque selon la revendication 7, dans laquelle les polypeptides sont exprimés comme des IgG de longueur complète.

9. Les produits d'expression de polypeptides de la banque selon l'une quelconque des revendications 3 à 8.

10. Un procédé de production d'un anticorps d'intérêt par expression de la banque selon l'une quelconque des revendications 3 à 8 dans une cellule hôte.

11. Une banque de vecteurs contenant la banque selon l'une quelconque des revendications 3 à 8.

12. Une banque de cellules hôtes contenant la banques des vecteurs selon la revendication 11.

13. La banque de cellules hôtes selon la revendication 12, dans laquelle les cellules hôtes sont des cellules de levure.

14. Utilisation de la banque selon l'une quelconque des revendications 3 à 8 pour isoler un anticorps liant un antigène.

15. Un kit contenant la banque selon l'une quelconque des revendications 3 a 8.

16. La banque selon la revendication 7, dans laquelle les polypeptides sont exprimés comme des anticorps intacts.
